# EUROPEAN PATENT APPLICATION

(11) **EP 4 613 276 A2**
(43) Date of publication of application: **10.09.2025**
(21) Application number: 25169541.7
(22) Date of filing: 12.11.2020
(51) Int. Cl.: A61K 35/28

(54) **IMMUNOSTIMULATORY BACTERIA DELIVERY PLATFORMS AND THEIR USE FOR DELIVERY OF THERAPEUTIC PRODUCTS**

(30) Priority: 12.11.2019 US 201962934503 P; 16.01.2020 US 202062962162 P; 16.03.2020 US 202062990404 P
(62) Divisional of application: 20820673.0
(71) Applicant: Actym Therapeutics, Inc., Berkeley, CA 94710 (US)
(72) Inventor: THANOS, Christopher D., Tiburon, CA 94920 (US); GLICKMAN, Laura Hix, Oakland, CA 94611 (US); IANNELLO, Alexandre Charles Michel, Oakland, CA 94618 (US); RAE, Chris, Richmond, CA 94805 (US); KEHOE, Haixing, Berkeley, CA 94709 (US); PETERSON, Bret Nicholas, Oakland, CA 94602 (US); CHEUNG, Chingnam, Oakland, CA 94619 (US)
(74) Representative: Hoffmann Eitle

(57) **Abstract**

Provided are attenuated immunostimulatory bacteria with genomes that are modified to, for example, reduce toxicity and improve the anti-tumor activity, such as by increasing accumulation in the tumor microenvironment, particularly in tumor-resident myeloid cells, improving resistance to complement inactivation, reducing immune cell death, promoting adaptive immunity, and enhancing T-cell function. The increase in colonization of phagocytic cells improves the delivery of encoded therapeutic products to the tumor microenvironment and tumors, and permits, among other routes, systemic administration of the immunostimulatory bacteria.

## Description

### RELATED APPLICATIONS

Benefit of priority is claimed to co-pending U.S. Provisional Application Serial No. 62/990,404, filed on March 16, 2020, entitled "TUMOR-SPECIFIC IMMUNOSTIMULATORY BACTERIA DELIVERY PLATFORM," to Applicant Actym Therapeutics, Inc., and inventors Laura Hix Glickman, Christopher D. Thanos, Alexandre Charles Michel Iannello, Chris Rae, and Haixing Kehoe.

Benefit of priority also is claimed to co-pending U.S. Provisional Application Serial No. 62/962,162, filed on January 16, 2020, entitled "TUMOR-SPECIFIC IMMUNOSTIMULATORY BACTERIA DELIVERY PLATFORM," to Applicant Actym Therapeutics, Inc., and inventors Laura Hix Glickman, Christopher D. Thanos, Alexandre Charles Michel Iannello, Chris Rae, and Haixing Kehoe.

Benefit of priority also is claimed to co-pending U.S. Provisional Application Serial No. 62/934,503, filed on November 12, 2019, entitled "TUMOR-SPECIFIC IMMUNOSTIMULATORY BACTERIA DELIVERY PLATFORM," to Applicant Actym Therapeutics, Inc., and inventors Laura Hix Glickman, Christopher D. Thanos, Alexandre Charles Michel Iannello, Chris Rae, and Haixing Kehoe.

This application is related to co-pending International Application No. PCT/US2020/020240, filed on February 27, 2020, entitled "IMMUNOSTIMULATORY BACTERIA ENGINEERED TO COLONIZE TUMORS, TUMOR-RESIDENT IMMUNE CELLS, AND THE TUMOR MICROENVIRONMENT," to Applicant Actym Therapeutics, Inc., and inventors Christopher D. Thanos, Laura Hix Glickman, Justin Skoble, Alexandre Charles Michel Iannello, and Haixing Kehoe.

This application also is related to co-pending U.S. Provisional Application Serial No. 62/962,140, filed on January 16, 2020, entitled "IMMUNOSTIMULATORY BACTERIA ENGINEERED TO COLONIZE TUMORS, TUMOR-RESIDENT IMMUNE CELLS, AND THE TUMOR MICROENVIRONMENT," to Applicant Actym Therapeutics, Inc., and inventors Christopher D. Thanos, Laura Hix Glickman, Justin Skoble, Alexandre Charles Michel Iannello, and Haixing Kehoe.

This application also is related to co-pending U.S. Provisional Application Serial No. 62/934,478, filed on November 12, 2019, entitled "IMMUNOSTIMULATORY BACTERIA ENGINEERED TO COLONIZE TUMORS AND THE TUMOR MICROENVIRONMENT," to Applicant Actym Therapeutics, Inc., and inventors Christopher D. Thanos, Laura Hix Glickman, Justin Skoble, and Alexandre Charles Michel Iannello.

This application also is related to International Application No. PCT/US2018/041713, filed on July 11, 2018 and published as WO 2019/014398 on January 17, 2019, and to co-pending U.S. Patent Application Serial No. 16/033,187, filed on July 11, 2018 and published as U.S. Publication No. 2019/0017050 A1 on January 17, 2019, each entitled "ENGINEERED IMMUNOSTIMULATORY BACTERIAL STRAINS AND USES THEREOF," and each of which claims priority to U.S. Provisional Application Serial Nos. 62/531,327, filed on July 11, 2017, and 62/648,380, filed on March 26, 2018. Where permitted, the subject matter of each of these applications is incorporated by reference in its entirety.

This application also is related to co-pending International Application No. PCT/US2019/041489, filed on July 11, 2019, and published as WO 2020/014543 on January 16, 2020, and to co-pending U.S. Patent Application Serial No. 16/520,155, filed on July 23, 2019, each entitled "ENGINEERED IMMUNOSTIMULATORY BACTERIAL STRAINS AND USES THEREOF," and each of which claims priority to U.S. Provisional Application Serial No. 62/789,983, filed on January 08, 2019, and to U.S. Provisional Application Serial No. 62/828,990, filed on April 03, 2019.

This application also is related to U.S. Provisional Application Serial Nos. 62/811,521, filed on February 27, 2019, and 62/828,990, filed on April 03, 2019.

The immunostimulatory bacteria provided in each of these applications can be modified as described in this application, and such bacteria are incorporated by reference herein. Where permitted, the subject matter of each of these applications is incorporated by reference in its entirety.

### INCORPORATION BY REFERENCE OF SEQUENCE LISTING PROVIDED ELECTRONICALLY

An electronic version of the Sequence Listing is filed herewith, the contents of which are incorporated by reference in their entirety. The electronic file was created on November 11, 2020, is 687 kilobytes in size, and is titled 1707SEQPC1.txt.

### FIELD OF THE INVENTION

Provided are attenuated immunostimulatory bacteria with genomes that are modified to, for example, reduce toxicity and improve the anti-tumor activity, such as by increasing accumulation in the tumor microenvironment, particularly in tumor-resident myeloid cells, by improving resistance to complement inactivation, by reducing immune cell death, by promoting adaptive immunity, and by enhancing T-cell function. The increase in colonization of phagocytic cells improves the delivery of encoded therapeutic products to the tumor microenvironment and tumors, and permits, among other routes, systemic administration of the immunostimulatory bacteria.

### BACKGROUND

The field of cancer immunotherapy has made great strides, as evidenced by the clinical successes of anti-CTLA-4, anti-PD-1 and anti-PD-L1 immune checkpoint antibodies (see, *e.g.,* Buchbinder et al. (2015) J. Clin. Invest. 125:3377-3383; Hodi et al. (2010) N. Engl. J. Med. 363(8):711-723; and Chen et al. (2015) J. Clin. Invest. 125:3384-3391). Tumors have evolved a profoundly immunosuppressive environment. They initiate multiple mechanisms to evade immune surveillance, reprogram anti-tumor immune cells to suppress immunity, and continually mutate resistance to the latest cancer therapies (see, *e.g.,* Mahoney et al. (2015) Nat. Rev. Drug Discov. 14(8):561-584). Designing immunotherapies and cancer therapies that overcome immune tolerance and escape, while limiting the autoimmune-related toxicities of current immunotherapies, challenges the field of immuno-oncology. Hence, additional and innovative immunotherapies and other therapies are needed.

### SUMMARY

Provided are immunostimulatory bacteria that contain genome modifications, and a plasmid that encodes one or more therapeutic products, such as anti-cancer therapeutics or associated treatments. The genome modifications result in immunostimulatory bacteria that accumulate in the tumor microenvironment and in tumor-resident immune cells, where they express the encoded therapeutic products. The immunostimulatory bacteria provided herein encoded one or a plurality of complementary products that stimulate or induce or result in a robust anti-cancer response in the subject.

Provided herein are immunostimulatory bacteria that contain a plasmid encoding a therapeutic product or combinations of therapeutic products, under control of a eukaryotic promoter. The genomes of the bacteria contain modifications, such as one, two, or more modifications, selected from among:
a) deletion or disruption or inactivation of all or of a sufficient portion of a gene or genes, whereby the bacterium has been modified to generate penta-acylated lipopolysaccharide (LPS), wherein:
   the genome of the immunostimulatory bacterium is modified by deletion or disruption of all or of a sufficient portion of a gene or genes, whereby the bacterium has been modified to generate penta-acylated lipopolysaccharide; and/or
   hexa-acylated lipopolysaccharide is substantially reduced, by at least 10-fold, compared to the wild-type bacterium, or is absent;
b) deletion or disruption or inactivation of all or of a sufficient portion of a gene or genes, whereby the bacterium has attenuated recognition by Toll-like Receptors (TLRs): TLR2, TLR4, and TLR5;
c) deletion or disruption or inactivation of all or of a sufficient portion of a gene or genes, whereby the bacterium does not activate the synthesis of curli fimbriae and/or cellulose;
d) deletion or disruption or inactivation of all or of a sufficient portion of a gene or genes, whereby the bacterium does not activate the synthesis of secreted asparaginase;
e) deletion or disruption or inactivation of all or of a sufficient portion of a gene or genes, whereby the bacterium is auxotrophic for purines, adenosine, or ATP;
f) deletion or disruption or inactivation of all or of a sufficient portion of a gene or genes, whereby the bacterium lacks flagella;
g) deletion or disruption or inactivation of all or of a sufficient portion of a gene or genes, whereby the bacterium has been modified to specifically infect tumor-resident myeloid cells;
h) deletion or disruption or inactivation of all or of a sufficient portion of a gene or genes, whereby the bacterium has been modified to specifically infect tumor-resident myeloid cells, and is unable to replicate in tumor-resident myeloid cells; and
i) deletion or disruption or inactivation of either or both of *lppA* and *lppB,* to decrease or eliminate lipoprotein expression in the membrane, whereby expression of an encoded therapeutic protein is increased in the tumor microenvironment and/or in tumor-resident immune cells.

For example, the immunostimulatory bacteria contain modifications, including deletions, insertions, and replacements, of a), d), and f), or modifications c) and d), or modifications a), c), d), e), and f), or modifications a), c), d), e), f), and i), or modifications a), d) f), and i), or modifications c), d), and i), or modifications f) and i), or modifications a)-i), or modifications a), b), d), and f), or modifications a), b), c), and d), and other combinations of a)-i).

In all embodiments, the immunostimulatory bacteria also can comprise or further comprise deletion of or disruption of the genes encoding the flagella, whereby the bacterium is flagellin⁻ *(fliC⁻*/*fljB⁻*) and does not produce flagella, wherein the wild-type bacterium has flagella. The immunostimulatory bacteria can be auxotrophic for purines, such as auxotrophic for adenosine, or auxotrophic for adenosine, adenine, and/or ATP. The immunostimulatory bacteria also can be *purI⁻.* The immunostimulatory bacteria also can be *pagP⁻.* The immunostimulatory bacteria also can be *asd⁻* (aspartate-semialdehyde dehydrogenase⁻), such as where the bacterium is *asd⁻* by virtue of disruption or deletion of all or a portion of the endogenous gene encoding aspartate-semialdehyde dehydrogenase (*asd*)*,* whereby endogenous *asd* is not expressed. The bacteria can encode aspartate-semialdehyde dehydrogenase (*asd*) on the plasmid under control of a bacterial promoter. The immunostimulatory bacteria also can be *msbB⁻,* or can be *pagP⁻*/*msbB⁻.* For example, the immunostimulatory bacteria can be *αsd⁻, purI⁻, msbB⁻,* flagellin⁻ (*fliC⁻*/*fljB⁻*)*,* and *pagP⁻,* or they can be *asd⁻ , csgD⁻, purI⁻, msbB⁻,* flagellin⁻ (*fliC*/*fljB⁻*)*,* and *pagP⁻.* In some embodiments, the immunostimulatory bacteria are *ansB⁻, asd⁻, csgD⁻, purI⁻, msbB⁻,* flagellin⁻ (*fliC*/*fljB⁻*)*,* and *pagP⁻.*

Provided are immunostimulatory bacteria that contain a plasmid encoding a therapeutic product under control of a eukaryotic promoter, or that encode a plurality of products under control of a plurality of eukaryotic promoters or a single promoter. The genome of the immunostimulatory bacteria is modified by deletion of a sufficient portion of, or by the disruption of genes, whereby the bacterium is one or more of *ansB⁻, asd⁻, csgD⁻, purI⁻, msbB⁻,* flagellin⁻ (*fliC⁻*/*fljB⁻*)*,* and *pagP⁻.* The immunostimulatory bacteria provided herein also include those that have the genes *lppA* (*lppl*) and/or *lppB (lpp2),* which encode major outer membrane lipoproteins Lpp1 (LppA) and Lpp2 (LppB), respectively, deleted or disrupted, to eliminate or substantially reduce expression of the encoded lipoprotein(s). In particular, the bacteria are *lppA⁻* and *lppB⁻.* Provided are immunostimulatory bacteria that contain a plasmid encoding an anti-cancer therapeutic under control of eukaryotic regulatory sequences, and that are *lppA⁻* and *lppB⁻.* For example, the immunostimulatory bacteria can be *ansB⁻, asd⁻, csgD⁻, purI⁻, msbB⁻,* flagellin⁻ (*fliC⁻*/*fljB⁻*)*, pagP⁻, lppA⁻,* and *lppB⁻.*

In embodiments herein, the therapeutic product is an anti-cancer therapeutic or a therapeutic used in cancer therapy. The encoded product(s) can be operably linked to nucleic acid encoding a secretion signal, whereby, when expressed, the therapeutic product is secreted, such as secreted from a tumor-resident immune cell.

Any of the immunostimulatory bacteria also can have one or more genes or operons involved in *Salmonella* pathogenicity island 1 (SPI-1) invasion deleted or inactivated, whereby the immunostimulatory bacteria do not invade or infect epithelial cells. For example, the one or more genes/operons are selected from among *avrA, hilA, hilD, invA, invB, invC, invE, invF, invG, invH, invI, invJ, iacP, iagB, spaO, spaQ, spaR, spaS, orgA, orgB, orgC, prgH, prgI, prgJ, prgK, sicA, sicP, sipA, sipB, sipC, sipD, sirC, sopB, sopD, sopE, sopE2, sprB,* and *sptP.*

The plasmid in the immunostimulatory bacteria can be present in low copy number or medium copy number. The plasmid can contain a medium-to-low copy number origin of replication, such as a low copy number origin of replication. In some embodiments, the plasmid is present in higher copy number. Generally, medium copy number is less than 150 or less than about 150, and more than 20 or about 20, or is between 20 or 25 and 150; and low copy number is less than 25, or less than 20, or less than about 25, or less than about 20 copies. In particular, low to medium copy number is less than about 150 copies, or less than 150 copies; low copy number is less than about 25 copies, or less than 25 copies.

Provided are nucleic acid constructs, which are nucleic acid molecules that encode products, such as proteins, that are designed to be introduced into a cell or into a plasmid for expression of the encoded product. The constructs contain nucleic acid encoding a plurality of anti-cancer products as a polycistronic sequence under control of a single promoter. The promoter can be a eukaryotic promoter. Other eukaryote regulatory sequences, such as enhancers, and nucleic acid encoding protein trafficking signals, such as secretion signals, and other regulatory sequences, such as terminators, including bacterial terminators to prevent read-through from bacterial promoters on the constructs, and particular configurations of elements and the order of the product-encoding nucleic acid open reading frames and/or genes, are provided and described herein. In the constructs the polycistronic sequence can include signals or encoded signals, such as peptides, that result in expression of discrete products encoded by the polycistronic construct. Exemplary of such peptides are the 2A family of viral peptides. The constructs include such peptides or other signal between each open reading frame encoding each product. The 2A peptide include one or more of T2A, P2A, E2A, or F2A.

Included among the anti-cancer products are any that are used for treatment of cancer or to promote or aid or stimulate or help an anti-cancer response in a subject. Generally the anti-cancer products are proteins. The encoded products include one or more immunostimulatory protein(s) that confer(s )or contributes to an anti-tumor immune response in a tumor microenvironment. Exemplary encoded products is/are immunostimulatory protein(s) that confer(s) or contribute(s) to an anti-tumor immune response in the tumor microenvironment, such as, for example, any selected from among one or more of: IL-2, IL-7, IL-12p70 (IL-12p40 + IL-12p35), IL-15, IL-2 that has attenuated binding to IL-2Ra, IL-15/IL-15R alpha chain complex, IL-18, IL-21, IL-23, IL-36γ, IL-2 modified so that it does not bind to IL-2Ra, CXCL9, CXCL10, CXCL11, interferon-α, interferon-β, interferon-γ, CCL3, CCL4, CCL5, proteins that are involved in or that effect or potentiate recruitment/persistence of T-cells, co-stimulatory proteins, such as, CD40, CD40 ligand (CD40L), CD28, OX40, OX40 ligand (OX40L), 4-1BB, 4-1BB ligand (4-1BBL), including forms of the co-stimulatory proteins that are cytoplasmic domain deleted or truncated to eliminate the immunosuppressive reverse signaling, with optional modifications to promote correct orientation (*i.e*., cytoplasmic domain in the cytoplasm) in a cell; members of the B7-CD28 family, CD47 antagonists, an anti-IL-6 antibodies or IL-6 binding decoy receptors, TGF-beta polypeptide antagonists, including soluble TGF-beta receptors and TGF-beta antagonists, and members of the tumor necrosis factor receptor (TNFR) superfamily.

Others of the products include an immunostimulatory protein that confers or contributes to an anti-tumor immune response in the tumor microenvironment that is selected from among one or more of: IFN-α, IFN-β, GM-CSF, IL-2, IL-7, IL-12, IL-15, IL-18, IL-21, IL-23, IL-12p70 (IL-12p40 + IL-12p35), IL-15/IL-15R alpha chain complex, IL-36 gamma, IL-2 that has attenuated binding to IL-2Ra, IL-2 that is modified so that it does not bind to IL-2Ra, CXCL9, CXCL10 (IP-10), CXCL11, CCL3, CCL4, CCL5, molecules involved in the potential recruitment and/or persistence of T-cells, CD40, CD40 ligand (CD40L), OX40, OX40 ligand (OX40L), 4-1BB, 4-1BB ligand (4-1BBL), 4-1BBL with a deleted cytoplasmic domain (4-1BBLΔcyt) or with a partially deleted cytoplasmic domain, which is deleted or truncated to eliminate the immunosuppressive reverse signaling, members of the B7-CD28 family, and members of the tumor necrosis factor receptor (TNFR) superfamily. For example, the construct can contain nucleic acid encoding 4-1BBL with a deleted, or partially deleted cytoplasmic domain, or a partially deleted cytoplasmic domain and optionally including amino acid modifications, whereby the resulting 4-1BBL assumes the proper orientation when expressed in a cell (see, SEQ ID NOs:389-392 and detailed description below providing exemplary modified 4-1BBL variants with truncated cytoplasmic domains, where residues are replaced with positively charged residues (*i.e*., K and L) to confer proper orientation when expressed in a cell. The cytoplasmic domain is truncated sufficiently to eliminate or reduce immunosuppressive reverse signaling. Hence, provided are constructs containing nucleic acid encoding a 4-1BBL with a deleted or partially deleted cytoplasmic domain, or a modified 4-1BBL with a truncated and modified cytoplasmic domain, wherein the sequence of 4-1BBL is set forth in SEQ ID NOs:389-392, and exemplified sequences below in the detailed description and Examples. Constructs also can contain nucleic acid encoding any of the following products and combinations of products:
one or more of IL-12, or IL-15, or IL12p70, or IL-15/IL-15R alpha chain complex;
a cytokine and a STING pathway agonist;
a cytokine, a sting pathway agonist, and either a co-stimulatory molecule or an immune checkpoint inhibitor;
a cytokine and a STING pathway agonist, and a TGF-beta polypeptide antagonist;
a cytokine, a STING pathway agonist, a TGF-beta polypeptide antagonist, and either a co-stimulatory molecule (receptor or ligand) or an immune checkpoint inhibitor,
wherein a STING pathway agonist is any product that increases type I interferon expression via activation of the STING pathway. Exemplary are constructs that encode a Stimulator of Interferon Genes (STING) polypeptide, or a variant thereof or chimera thereof, as described in detail herein. Among the constructs are those that encode a combination of therapeutic products, selected from among the following combinations:
   an anti-CTLA-4 antibody and a STING polypeptide,
   IL-15 and a STING polypeptide,
   4-1BBL and a STING polypeptide,
   A TGF-beta decoy receptor or polypeptide antagonist, and a STING polypeptide,
   IL-12 and STING polypeptide
   an anti-CTLA-4 antibody, IL-15, and a STING polypeptide,
   4-1BBL, IL-15, and a STING polypeptide,
   TGF-beta decoy receptor or polypeptide antagonist, and IL-15, and a STING polypeptide,
   an anti-CTLA-4 antibody, and IL-12, and a STING polypeptide,
   4-1BBL, IL-12, and a STING polypeptide,
   a TGF-beta decoy receptor or polypeptide antagonist, IL-12, and a STING polypeptide,
   an anti-CTLA-4 antibody, IL-15, a TGF-beta decoy receptor or polypeptide antagonist, and STING polypeptide,
   4-1BBL, and IL-15, a TGF-beta decoy receptor or polypeptide antagonist, and a STING polypeptide,
   an anti-CTLA-4 antibody, and IL-12, a TGF-beta decoy receptor or polypeptide antagonist, and STING polypeptide,
   4-1BBL, and IL-12, a TGF-beta decoy receptor or polypeptide antagonist, and a STING polypeptide,
   an anti-CTLA-4 antibody, IL-12, IL-15, and a STING polypeptide,
   4-1BBL, IL-12, IL-15, and a STING polypeptide,
   a TGF-beta decoy receptor or polypeptide antagonist, IL-12, IL-15, and a STING polypeptide,
   a TGF-beta decoy receptor or polypeptide antagonist, IL-12, IL-15, and a STING polypeptide,
   an anti-CTLA-4 antibody, IL-12, IL-15, a TGF-beta decoy receptor or polypeptide antagonist, and a STING polypeptide,
   4-1BBL, IL-12, IL-21, a TGF-beta decoy receptor or polypeptide antagonist, and a STING polypeptide,
   an anti-CTLA-4 antibody, IL-12, IL-15, and a TGF-beta decoy receptor or polypeptide antagonist,
   4-1BBL, IL-12, IL-21, and a TGF-beta decoy receptor or polypeptide antagonist,
   IL-12, IL-15, and a STING polypeptide,
   IL-15, IL-21, and a STING polypeptide,
   IL-12, IL-21, and a STING polypeptide,
   an anti-CTLA-4 antibody, IL-15, IL-21, and a STING polypeptide,
   an anti-CTLA-4 antibody, IL-12, IL-21, and a STING polypeptide,
   4-1BBL, IL-15, IL-21, and a STING polypeptide,
   4-1BBL, IL-12, IL-21, and a STING polypeptide,
   an anti-CTLA-4 antibody, and IL-15,
   an anti-CTLA-4 antibody, IL-15, and a TGF-beta decoy receptor or polypeptide antagonist,
   4-1BBL and IL-15,
   4-1BBL, IL-15, and a TGF-beta decoy receptor or polypeptide antagonist,
   an anti-CTLA-4 antibody and IL-12,
   an anti-CTLA-4 antibody, and IL-12, and a TGF-beta decoy receptor or polypeptide antagonist,
   4-1BBL and IL-12,
   4-1BBL, IL-12, and a TGF-beta decoy receptor or polypeptide antagonist,
   an anti-CTLA-4 antibody and a TGF-beta decoy receptor or polypeptide antagonist,
   4-1BBL and a TGF-beta decoy receptor or polypeptide antagonist,
   IL-15 and a TGF-beta decoy receptor or polypeptide antagonist,
   IL-12 and a TGF-beta decoy receptor or polypeptide antagonist,
   IL-12, IL-15, and a TGF-beta decoy receptor or polypeptide antagonist, and
   IL-15, and IL-21, and a TGF- decoy receptor or polypeptide antagonist, wherein:
      the an anti-CTLA-4 antibody is an scFv or an scFv-Fc;
   STING polypeptides include a wild-type STING, or a variant STING polypeptide, or a chimeric STING polypeptide, and a chimeric STING protein with amino acid replacements that confer, for example, a gain-of-function; and
   4-1BBL is 4-1BBL with a deleted cytoplasmic domain, 4-1BBL with a modified cytoplasmic domain, 4-1BBL with a truncated cytoplasmic domain, or 4-1BBL with a truncated and modified cytoplasmic domain.

Other constructs include those that encode a combination of therapeutic products selected from among:
IL-2 and IL-12p70;
IL-2 and IL-21;
IL-2, IL-12p70, and a STING gain-of-function (GOF) variant;
IL-2, IL-21, and a STING GOF variant;
IL-2, IL-12p70, a STING GOF variant, and 4-1BBL (including 4-1BBLΔcyt), where Δcyt is a deleted cytoplasmic domain;
IL-2, IL-21, a STING GOF variant, and 4-1BBL (including 4-1BBLΔcyt);
IL-15/E-15Rα, and a STING GOF variant;
IL-15/E-15Rα, a STING GOF variant, and 4-1BBL (including 4-1BBLΔcyt);
IL-15/IL-15Rα and IL-12p70;
IL-15/IL-15Rα and IL-21;
IL-15/E-15Rα, IL-12p70, and a STING GOF variant;
IL-15/IL-15Rα, IL-21, and a STING GOF variant;
IL-15/E-15Rα, IL-12p70, a STING GOF variant, and 4-1BBL (including 4-1BBLΔcyt);
IL-15/E-15Rα, IL-21, a STING GOF variant, and 4-1BBL (including 4-1BBLΔcyt);
IL-12p70 and IL-21;
IL-12p70, IL-21, and a STING GOF variant;
IL-12p70, IL-21, a STING GOF variant, and 4-1BBL (including 4-1BBLΔcyt);
IL-12p70 and a STING GOF variant;
IL-12p70, a STING GOF variant, and 4-1BBL (including 4-1BBLΔcyt);
IL-12p70 and IL-18;
IL-12p70, IL-18, and a STING GOF variant;
IL-12p70, IL-18, a STING GOF variant, and 4-1BBL (including 4-1BBLΔcyt);
a TGF-β decoy receptor or antagonist polypeptide, IL-2, and IL-12p70;
a TGF-β decoy receptor or antagonist polypeptide, IL-2, and IL-21;
a TGF-β decoy receptor or antagonist polypeptide, IL-2, IL-12p70, and a STING GOF variant;
a TGF-β decoy receptor or antagonist polypeptide, IL-2, IL-21, and a STING GOF variant;
a TGF-β decoy receptor or antagonist polypeptide, IL-2, IL-12p70, a STING GOF variant, and 4-1BBL (including 4-1BBLΔcyt);
a TGF-β decoy receptor or antagonist polypeptide, IL-2, IL-21, a STING GOF variant, and 4-1BBL (including 4-1BBLΔcyt);
a TGF-β decoy receptor or antagonist polypeptide, IL-15/E-15Rα, and a STING GOF variant;
a TGF-β decoy receptor antagonist polypeptide, IL-15/IL-15Rα, a STING GOF variant, and 4-1BBL (including 4-1BBLΔcyt);
a TGF-β decoy receptor or antagonist polypeptide, IL-15/IL-15Rα, and IL-12p70;
a TGF-β decoy receptor or antagonist polypeptide, IL-15/E-15Rα, and IL-21;
a TGF-β decoy receptor or antagonist polypeptide, IL-15/E-15Rα, IL-12p70, and a STING GOF variant;
a TGF-β decoy receptor antagonist polypeptide, IL-15/E-15Rα, IL-21, and a STING GOF variant;
a TGF-β decoy receptor or antagonist polypeptide, IL-15/E-15Rα, IL-12p70, a STING GOF variant, and 4-1BBL (including 4-1BBLΔcyt);
a TGF-β decoy receptor or antagonist polypeptide, IL-15/E-15Rα, IL-21, a STING GOF variant, and 4-1BBL (including 4-1BBLΔcyt);
a TGF-β decoy receptor or antagonist polypeptide, IL-12p70, and IL-21;
a TGF-β decoy receptor or antagonist polypeptide, IL-12p70, IL-21, and a STING GOF variant;
a TGF-β decoy receptor or antagonist polypeptide, IL-12p70, IL-21, a STING GOF variant, and 4-1BBL (including 4-1BBLΔcyt);
a TGF-β decoy receptor or antagonist polypeptide and IL-12p70;
a TGF-β decoy receptor or antagonist polypeptide, IL-12p70, and a STING GOF variant;
a TGF-β decoy receptor or antagonist polypeptide, IL-12p70, a STING GOF variant, and 4-1BBL (including 4-1BBLΔcyt);
a TGF-β decoy receptor or antagonist polypeptide, IL-12p70, and IL-18;
a TGF-β decoy receptor or antagonist polypeptide, IL-12p70, IL-18, and a STING GOF variant;
a TGF-β decoy receptor or antagonist polypeptide, IL-12p70, IL-18, a STING GOF variant, and 4-1BBL (including 4-1BBLΔcyt);
a TGF-β decoy receptor and a STING GOF variant;
an anti-CTLA-4 antibody, IL-2, and IL-12p70;
an anti-CTLA-4 antibody, IL-2, and IL-21;
an anti-CTLA-4 antibody, IL-2, IL-12p70, and a STING GOF variant;
an anti-CTLA-4 antibody, IL-2, IL-21, and a STING GOF variant;
an anti-CTLA-4 antibody, IL-2, IL-12p70, a STING GOF variant, and 4-1BBL (including 4-1BBLΔcyt);
an anti-CTLA-4 antibody, IL-2, IL-21, a STING GOF variant, and 4-1BBL (including 4-1BBLΔcyt);
an anti-CTLA-4 antibody, IL-15/E-15Rα, and a STING GOF variant;
an anti-CTLA-4 antibody, IL-15/E-15Rα, a STING GOF variant, and 4-1BBL (including 4-1BBLΔcyt);
an anti-CTLA-4 antibody, IL-15/E-15Rα, and IL-12p70;
an anti-CTLA-4 antibody, IL-15/E-15Rα, and IL-21;
an anti-CTLA-4 antibody, IL-15/E-15Rα, IL-12p70, and a STING GOF variant;
an anti-CTLA-4 antibody, IL-15/E-15Rα, IL-21, and a STING GOF variant;
an anti-CTLA-4 antibody, IL-15/E-15Rα, IL-12p70, a STING GOF variant, and 4-1BBL (including 4-1BBLΔcyt);
an anti-CTLA-4 antibody, IL-15/E-15Rα, IL-21, a STING GOF variant, and 4-1BBL (including 4-1BBLΔcyt);
an anti-CTLA-4 antibody, IL-12p70, and IL-21;
an anti-CTLA-4 antibody, IL-12p70, IL-21, and a STING GOF variant;
an anti-CTLA-4 antibody, IL-12p70, IL-21, a STING GOF variant, and 4-1BBL (including 4-1BBLΔcyt);
an anti-CTLA-4 antibody and IL-12p70;
an anti-CTLA-4 antibody, IL-12p70, and a STING GOF variant;
an anti-CTLA-4 antibody, IL-12p70, a STING GOF variant, and 4-1BBL (including 4-1BBLΔcyt);
an anti-CTLA-4 antibody, IL-12p70, and IL-18;
an anti-CTLA-4 antibody, IL-12p70, IL-18, and a STING GOF variant;
an anti-CTLA-4 antibody, IL-12p70, IL-18, a STING GOF variant, and 4-1BBL (including 4-1BBLΔcyt);
an anti-CTLA-4 antibody and a STING GOF variant;
a CD40 agonist, IL-2, and IL-12p70;
a CD40 agonist, IL-2, and IL-21;
a CD40 agonist, IL-2, IL-12p70, and a STING GOF variant;
a CD40 agonist, IL-2, IL-21, and a STING GOF variant;
a CD40 agonist, IL-2, IL-12p70, a STING GOF variant, and 4-1BBL (including 4-1BBLΔcyt);
a CD40 agonist, IL-2, IL-21, a STING GOF variant, and 4-1BBL (including 4-1BBLΔcyt);
a CD40 agonist, IL-15/E-15Rα, and a STING GOF variant;
a CD40 agonist, IL-15/E-15Rα, a STING GOF variant, and 4-1BBL (including 4-1BBLΔcyt);
a CD40 agonist, IL-15/E-15Rα, and IL-12p70;
a CD40 agonist, IL-15/IL-15Rα, and IL-21;
a CD40 agonist, IL-15/E-15Rα, IL-12p70, and a STING GOF variant;
a CD40 agonist, IL-15/E-15Rα, IL-21, and a STING GOF variant;
a CD40 agonist, IL-15/E-15Rα, IL-12p70, a STING GOF variant, and 4-1BBL (including 4-1BBLΔcyt);
a CD40 agonist, IL-15/E-15Rα, IL-21, a STING GOF variant, and 4-1BBL (including 4-1BBLΔcyt);
a CD40 agonist, IL-12p70, and IL-21;
a CD40 agonist, IL-12p70, IL-21, and a STING GOF variant;
a CD40 agonist, IL-12p70, IL-21, a STING GOF variant, and 4-1BBL (including 4-1BBLΔcyt);
a CD40 agonist and IL-12p70;
a CD40 agonist, IL-12p70, and a STING GOF variant;
a CD40 agonist, IL-12p70, a STING GOF variant, and 4-1BBL (including 4-1BBLΔcyt);
a CD40 agonist, IL-12p70, and IL-18;
a CD40 agonist, IL-12p70, IL-18, and a STING GOF variant;
a CD40 agonist, IL-12p70, IL-18, a STING GOF variant, and 4-1BBL (including 4-1BBLΔcyt); and
a CD40 agonist and a STING GOF variant, wherein:
4-1BBL is 4-1BBL with a deleted cytoplasmic domain (4-1BBLΔcyt), 4-1BBL with a modified cytoplasmic domain, 4-1BBL with a truncated cytoplasmic domain, or 4-1BBL with a truncated and modified cytoplasmic domain; and
an anti-CTLA-4 antibody is an scFv or an scFv-Fc.

Exemplary of the STING polypeptides are those in which the STING polypeptide is modified to result in increased or constitutive expression of a type I interferon, or is a chimeric polypeptide comprising a human STING polypeptide with a C-terminal tail from a different species that has lower NF-κB signaling activity than the NF-κB signaling activity of human STING, and where, for example: the TRAF6 binding site in the CTT optionally is deleted; and the human STING protein has the sequence set forth in any of SEQ ID NOs:305-309. Particular combinations of encoded products include those where the encoded therapeutic proteins comprise IL-12p70 and a chimeric human STING polypeptide with a CTT from Tasmanian devil and an amino acid replacement that results in increased or constitutive expression of type I interferon, or is a STING polypeptide with an amino acid replacement that results in increased or constitutive expression of type I interferon, where a mutation that results in increased or constitutive expression of type I interferon is a gain-of-function mutation. Exemplary are STING proteins or polypeptides with replacements described in the detailed description, such as where the amino replacement in the STING polypeptide corresponds to R284G or N154S/R284G with reference, for alignment, to any of SEQ ID NOs: 305-309, which set forth human STING proteins. These constructs can additional encode IL-36γ and/or an immune checkpoint inhibitor antibody. As described herein antibodies include an antigen-binding portions thereof, and any of the various forms of antibodies, such as, but not limited to, scFvs, and scFv-Fc (generally IgG Fc), where the presence of the Fc multimerizes the resulting product so that it has two chains. Immune checkpoints targeted include, but are not limited to, CTLA-4 or PD-1 or PD-L1, and antibody forms of include scFV and is an scFV-Fc two-chain polypeptides.

Provided are plasmids that contain the constructs described above and throughout the disclosure herein. Plasmids include a bacterial plasmid, where the construct is operatively linked to eukaryotic transcriptional regulatory sequences.

Provided are compositions, such as pharmaceutical compositions, that contain the mixture of anti-cancer protein products encoded by the construct or plasmid as the only anti-cancer proteins in the compositions. Hence, among these are provided compositions that contain complementary combinations of therapeutic proteins; the mixtures include unique combinations of agents. These include the combinations of agents provided and described herein.

Also provided are immunostimulatory bacteria that contain any of the constructs and/or plasmids. The constructs and plasmids can be provided in or otherwise introduced into any of the immunostimulatory bacteria provided herein, including any discussed above and below. The constructs and plasmids also can be introduced into suitable bacteria known in the art, such as bacteria described in publications, such as International Application Publication Nos. WO2020/172461 and WO2020/172462, and U.S. Patent Nos. 10,449,237, 10,286,051, and 9,616,114.

The immunostimulatory bacteria provided herein include genome modifications, such as deletions, disruptions, alterations, such as changing the orientation of all or part of the gene, so that functional gene products in not expressed. Among the immunostimulatory bacteria provided those that are modified so that the resulting bacteria are *msbB⁻*/*purI⁻.* In some embodiment the bacteria are *msbB⁻* and *purI⁻,* whereby the full length of at least the coding portion of the *msbB⁻* and/or *purI⁻* genes are/is deleted. The genome of the bacteria also can be modified so that bacteria lack flagella. This is effected in bacteria that normally express flagella. In such bacteria for example the genes in Salmonella or equivalent genes in other species to *fliC⁻*/*fljB⁻* can be deleted or other modified so that functional gene product is not expressed. The bacteria also can be modified so that they are adenosine auxotrophs, and/or are *msbB⁻*/*pagP⁻.* Also provided are immunostimulatory bacteria and pharmaceutical compositions containing them, where the bacteria do not express L-asparaginase II, whereby the bacteria *ansB⁻.*

Provided are immunostimulatory bacteria that contain a plasmid encoding a therapeutic product under control of a eukaryotic promoter, where the genome of the immunostimulatory bacterium is modified by deletion or disruption of all or of a sufficient portion of a gene or genes, whereby the bacterium does not activate the synthesis of secreted asparaginase. Exemplary of such bacteria are those in which the asparaginase is L-asparaginase II encoded by the gene *ansB.*

It shown herein that in parental strain VNP20009, which is *msbB⁻* and *purI⁻,* the genes are not completely deleted. In immunostimulatory bacteria provided the genome of the bacterium is modified so that the full length of at least the coding portion of the *msbB⁻* and *purI⁻* genes is deleted. These strains are more fit, grow faster and/or to a greater extent than the parental strain. In all embodiments herein, the bacteria can be modified so that the native asd gene product is inactive or not expressed. To aid in producing the strain, the *asd* gene is encoded on a plasmid under control of a prokaryotic promoter, such as an inducible promoter.

In embodiments, the strains include modifications so that bacteria that lack flagella, and are *pagP⁻, ansB⁻,* and *csgD⁻.* In addition the bacteria are *purI⁻* and *asd⁻.* Thus provided are strains, include modified parental strains that already are *msbB⁻* and *purI⁻,* and/or have other modifications, particularly those that modify the LPS, that also are Δ*asd*/Δ*FLG*/Δ*pagP*/Δ*ansB*/Δ*csgD.* The strains also can be an adenosine or adenosine and adenine auxotroph.

Encoded therapeutic products include nucleic acids and proteins. The plasmid can encode two or more therapeutic products. Exemplary products include, but are not limited to, a cytokine, a protein that constitutively induces a type I interferon (IFN), and a co-stimulatory receptor or ligand. Further exemplary combinations are described below. In some embodiments, the co-stimulatory molecule lacks all or a portion of the cytoplasmic domain for expression on an antigen-presenting cell (APC), whereby the truncated molecule is capable of constitutive immuno-stimulatory signaling to a T-cell through co-stimulatory receptor engagement, and is unable to counter-regulatory signal to the antigen-presenting cell (APC), due to the deleted or truncated or otherwise modified cytoplasmic domain or portion thereof. Other products include enzymes that activate therapeutic proteins, such as those that activate prodrugs. As described herein and below, the immunostimulatory bacteria provided herein encode anti-cancer therapeutics, including combinations of therapeutic products that combine to provide a robust anti-cancer response. Among the proteins encoded are each of the products listed above and below, and combinations of products from different classes. Included are the co-stimulatory proteins, such as 4-1BBL, particularly those with truncated or deleted cytoplasmic domains to eliminate immunosuppressive reverse signaling, and also any compensatory mutations to ensure that the resulting protein is correctly oriented in the cell membrane when expressed in a cell. Other products include STING pathway agonists to induce or result in constitutive expression of type I interferons. These products include STING protein and, particularly, the modified and chimeric STING proteins provided and described herein. One or more cytokines, such as IL-12, IL-15, IL-21, L-12p70 (IL-12p40 + IL-12p35), IL-2 that has attenuated binding to IL-2Ra, IL-15/IL-15R alpha chain complex, and others, such as IL-18, IL-21, IL-23, IL-36γ, also are encoded on the plasmids. In addition to the co-stimulatory products, the STING pathway agonist proteins, such as STING, and the cytokines, antibodies, such as checkpoint inhibitor antibodies, including anti-CTLA-4 antibodies, can be encoded on the plasmids. The antibodies can be scFvs and also scFvs-Fc two-chain forms, as well as other forms. Additionally, among other products, TGF-beta antagonists and TGF-beta receptor decoys can be included.

The encoded therapeutic products can be operatively linked to nucleic acid encoding regulatory sequences recognized by a eukaryotic host, such as, for example, secretion signals to effect secretion from a cell comprising the bacterium or plasmid. In embodiments where the immunostimulatory bacteria encode two or more products, expression of each product can be under control of a separate promoter. Alternatively, two or more products can be expressed under control of a single promoter, and each product is separated by nucleic acid encoding, for example, an internal ribosomal entry site (IRES), or a 2A peptide, to effect separate expression of each encoded therapeutic product. Exemplary 2A peptides are T2A, F2A, E2A, or P2A, which can flank nucleic acids encoding the therapeutic products, to effect separate expression of the therapeutic products expressed under control of a single promoter. The therapeutic products are expressed under control of a eukaryotic promoter, such as an RNA polymerase II promoter, or an RNA polymerase III promoter. These include an RNA polymerase II promoter that is a viral promoter, or a mammalian RNA polymerase II promoter, such as, but not limited to, as a cytomegalovirus (CMV) promoter, an SV40 promoter, an Epstein-Barr virus (EBV) promoter, a herpes virus promoter, an adenovirus promoter, an elongation factor-1 (EF-1) alpha promoter, a UBC promoter, a PGK promoter, a CAGG promoter, an adenovirus 2 or 5 late promoter, an EIF4A1 promoter, a CAG promoter, or a CD68 promoter. The plasmids further can include other eukaryotic regulatory sequences, such as terminators and/or promoters selected from among SV40, human growth hormone (hGH), bovine growth hormone (BGH or bGH), MND (a synthetic promoter that contains the U3 region of a modified MoMuLV LTR with myeloproliferative sarcoma virus enhancer), chicken beta-globulin, and rbGlob (rabbit globulin) genes, to control expression of the therapeutic product(s). Other regulatory sequences include a polyA tail, a Woodchuck Hepatitis Virus (WHP) Posttranscriptional Regulatory Element (WPRE), and a Hepatitis B virus Posttranscriptional Regulatory Element (HPRE). Additional regulatory elements, such as bacterial terminators inserted in appropriate loci, as described herein, to reduce or eliminate read-through from bacterial promoters, can be included.

The encoded therapeutic products include any described herein and in the original claims, such as nucleic acid encoding a protein that is part of a cytosolic DNA/RNA sensor pathway that leads to expression of type I interferon (IFN), or a variant thereof. Type I IFNs include interferon-α and interferon-β. Variants include those that, when expressed in a subject, lead to constitutive expression of type I IFN. These include a gain-of-function (GOF) variant that does not require cytosolic nucleic acids, nucleotides, dinucleotides, or cyclic dinucleotides to result in expression of type I IFN. Exemplary of these proteins is a protein selected from among STING, RIG-I, MDA-5, IRF-3, IRF-7, TRIM56, RIP1, Sec5, TRAF3, TRAF2, TRAF6, STAT1, LGP2, DDX3, DHX9, DDX1, DDX9, DDX21, DHX15, DHX33, DHX36, DDX60, and SNRNP200, and variants thereof that have increased activity, or that result in constitutive expression of type I interferon (IFN). Variants include a variant of STING, RIG-I, IRF-3, or MDA5, in which one or more serine (S) or threonine (T) residue(s) that is/are phosphorylated as a consequence of viral infection, is/are replaced with an aspartic acid (D), whereby the resulting variant is a phosphomimetic that constitutively induces type I IFN, and any known to those of skill in the art and/or described herein. Variants include, for example, those wherein the mutations are selected as follows: a) in STING, with reference to SEQ ID NOs: 305-309, one or more selected from among: S102P, V147L, V147M, N154S, V155M, G166E, C206Y, G207E, S102P/F279L, F279L, R281Q, R284G, R284S, R284M, R284K, R284T, R197A, D205A, R310A, R293A, T294A, E296A, R197A/D205A, S272A/Q273A, R310A/E316A, E316A, E316N, E316Q, S272A, R293A/T294A/E296A, D231A, R232A, K236A, Q273A, S358A/E360A/S366A, D231A/R232A/K236A/R238A, S358A, E360A, S366A, R238A, R375A, and S324A/S326A; b) in MDA5, with reference to SEQ ID NO:310, one or more of: T331I, T331R, A489T, R822Q, G821S, A946T, R337G, D393V, G495R, R720Q, R779H, R779C, L372F, and A452T; c) in RIG-I, with reference to SEQ ID NO:311, one or both of E373A and C268F; and d) in IRF-3, with reference to SEQ ID NO:312, S396D, such as a variant STING that contains one or more amino replacement(s) selected, with reference to SEQ ID NOs: 305-309, from among: S102P, V147L, V147M, N154S, V155M, G166E, C206Y, G207E, S102P/F279L, F279L, R281Q, R284G, R284S, R284M, R284K, R284T, R197A, D205A, R310A, R293A, T294A, E296A, R197A/D205A, S272A/Q273A, R310A/E316A, E316A, E316N, E316Q, S272A, R293A/T294A/E296A, D231A, R232A, K236A, Q273A, S358A/E360A/S366A, D231A/R232A/K236A/R238A, S358A, E360A, S366A, R238A, R375A, N154S/R284G, and S324A/S326A, and conservative replacements thereof, and combinations thereof.

The immunostimulatory bacteria also can encode an immunostimulatory protein that confers or contributes to an anti-tumor immune response in the tumor microenvironment. These include, but are not limited to, a cytokine, a chemokine, or a co-stimulatory molecule. Exemplary of these is a protein selected from among one or more of: IL-2, IL-7, IL-12p70 (IL-12p40 + IL-12p35), IL-15, IL-36 gamma, IL-2 that has attenuated binding to IL-2Ra, IL-15/IL-15R alpha chain complex, IL-18, IL-21, IL-23, IL-2 modified so that it does not bind to IL-2Ra, CXCL9, CXCL10, CXCL11, interferon-α, interferon-β, interferon-γ, CCL3, CCL4, CCL5, proteins that are involved in or that effect or potentiate the recruitment and/or persistence of T-cells, CD40, CD40 ligand (CD40L), CD28, OX40, OX40 ligand (OX40L), 4-1BB, 4-1BB ligand (4-1BBL), members of the B7-CD28 family, CD47 antagonists, an anti-IL-6 antibody or IL-6 binding decoy receptor, TGF-beta polypeptide antagonists, and members of the tumor necrosis factor receptor (TNFR) superfamily. The co-stimulatory molecule, selected from among CD40, CD40 ligand, CD28, OX40, OX40 ligand, 4-1BB, and 4-1BB ligand, can be truncated, such that the molecule lacks a cytoplasmic domain (or a portion thereof) for expression on an antigen-presenting cell (APC); and the truncated gene product is capable of constitutive immunostimulatory signaling to a T-cell through co-stimulatory receptor engagement, and is unable to counter-regulatory signal to the antigen-presenting cell (APC), due to the deleted cytoplasmic domain, or partially deleted or truncated cytoplasmic domain, to eliminate the immunosuppressive reverse signaling. Other such proteins are TGF-beta polypeptide antagonists, such as an anti-TGF-beta antibody or antibody fragment, an anti-TGF-beta receptor antibody or antibody fragment, a soluble TGF-beta antagonist polypeptide, or a TGF-beta binding decoy receptor.

The plasmids can encode a therapeutic antibody or antigen-binding fragment thereof, such as, for example, a Fab, Fab', F(ab')₂, single-chain Fv (scFv), scFv-Fc, Fv, dsFv, nanobody, diabody fragment, or a single-chain antibody. Examples include, but are not limited to, an antagonist of PD-1, PD-L1, CTLA-4, VEGF, VEGFR2, or IL-6.

In some embodiments, the immunostimulatory bacteria provided herein contain a plasmid that encodes two or more therapeutic proteins selected from among: a) an immunostimulatory protein that confers or contributes to an anti-tumor immune response in the tumor microenvironment; b) one or more of a protein that is part of a cytosolic DNA/RNA sensor pathway that leads to expression of type I interferon (IFN), or a variant thereof that has increased activity to increase expression of type I IFN, or a variant thereof that results in constitutive expression of a type I IFN; and c) an anti-cancer antibody or antigen-binding portion thereof. For example, the immunostimulatory protein can be a co-stimulatory molecule that is one that lacks a cytoplasmic domain or a sufficient portion thereof, for expression on an antigen-presenting cell (APC), whereby the truncated co-stimulatory molecule is capable of constitutive immunostimulatory signaling to a T-cell through co-stimulatory receptor engagement, and is unable to counter-regulatory signal to the antigen presenting cell (APC). In some embodiments, the immunostimulatory bacteria encode at least two therapeutic products selected from among a cytokine, a protein that constitutively induces a type I IFN, a co-stimulatory molecule, and an anti-cancer antibody or antigen-binding portion thereof, which can be under control of a single promoter. For example, expression of the nucleic acid encoding at least two or all of the products is under control of a single promoter, and the nucleic acid encoding each product is separated by nucleic acid encoding 2A polypeptides, whereby, upon translation, each product is separately expressed. The nucleic acid encoding each product can be operatively linked to nucleic acid encoding a sequence that directs secretion of the expressed product from a cell.

Provided are immunostimulatory bacteria that encode two or more therapeutic products, wherein at least one product is selected from a), and at least one is selected from b), and a) is IL-2, IL-7, IL-12p70 (IL-12p40 + IL-12p35), IL-15, IL-23, IL-36 gamma, IL-2 that has attenuated binding to IL-2Ra, IL-15/IL-15R alpha chain complex, IL-18, IL-2 modified so that it does not bind to IL-2Ra, CXCL9, CXCL10, CXCL11, interferon-α, interferon-β, CCL3, CCL4, CCL5, proteins that are involved in or that effect or potentiate the recruitment and/or persistence of T cells, CD40, CD40 Ligand (CD40L), OX40, OX40 Ligand (OX40L), 4-1BB, 4-1BB Ligand (4-1BBL), members of the B7-CD28 family, TGF-beta polypeptide antagonists, or members of the tumor necrosis factor receptor (TNFR) superfamily; and b) is STING, RIG-I, MDA-5, IRF-3, IRF-5, IRF-7, TRIM56, RIP1, Sec5, TRAF3, TRAF2, TRAF6, STAT1, LGP2, DDX3, DHX9, DDX1, DDX9, DDX21, DHX15, DHX33, DHX36, DDX60, or SNRNP200. They also can encode one or more of a TGF-beta inhibitory antibody, a TGF-beta binding decoy receptor, an anti-IL-6 antibody, and an IL-6 binding decoy receptor.

Exemplary of combinations of encoded therapeutic products are any of the following combinations of therapeutic products: IL-2 and IL-12p70; IL-2 and IL-21; IL-2, IL-12p70, and a STING GOF variant; IL-2, IL-21, and a STING GOF variant; IL-2, IL-12p70, a STING GOF variant, and 4-1BBL (including 4-1BBLΔcyt, where Δcyt is a deleted cytoplasmic domain, and 4-1BBL with a truncated cytoplasmic domain (4-1BBLcyt trunc)); IL-2, IL-21, a STING GOF variant, and 4-1BBL (including 4-1BBLΔcyt, and 4-1BBL with a truncated cytoplasmic domain); IL-15/E-15Rα, and a STING GOF variant; IL-15/E-15Rα, a STING GOF variant, and 4-1BBL (including 4-1BBLΔcyt and 4-1BBLcyt trunc); IL-15/IL-15Rα and IL-12p70; IL-15/IL-15Rα and IL-21; IL-15/E-15Rα, IL-12p70, and a STING GOF variant; IL-15/E-15Rα, IL-21, and a STING GOF variant; IL-15/E-15Rα, IL-12p70, a STING GOF variant, and 4-1BBL (including 4-1BBLΔcyt and 4-1BBLcyt trunc); IL-15/E-15Rα, IL-21, a STING GOF variant, and 4-1BBL (including 4-1BBLΔcyt and 4-1BBLcyt trunc); IL-12p70 and IL-21; IL-12p70, IL-21, and a STING GOF variant; IL-12p70, IL-21, a STING GOF variant, and 4-1BBL (including 4-1BBLΔcyt and 4-1BBLcyt trunc); IL-12p70 and a STING GOF variant; IL-12p70, a STING GOF variant, and 4-1BBL (including 4-1BBLΔcyt and 4-1BBLcyt trunc); IL-12p70 and IL-18; IL-12p70, IL-18, and a STING GOF variant; IL-12p70, IL-18, a STING GOF variant, and 4-1BBL (including 4-1BBLΔcyt and 4-1BBLcyt trunc); a TGF-β decoy receptor, IL-2, and IL-12p70; a TGF-β decoy receptor, IL-2, and IL-21; a TGF-β decoy receptor, IL-2, IL-12p70, and a STING GOF variant; a TGF-β decoy receptor, IL-2, IL-21, and a STING GOF variant; a TGF-β decoy receptor, IL-2, IL-12p70, a STING GOF variant, and 4-1BBL (including 4-1BBLΔcyt and 4-1BBLcyt trunc); a TGF-β decoy receptor, IL-2, IL-21, a STING GOF variant, and 4-1BBL (including 4-1BBLΔcyt and 4-1BBLcyt trunc); a TGF-β decoy receptor, IL-15/IL-15Rα, and a STING GOF variant; a TGF-β decoy receptor, IL-15/E-15Rα, a STING GOF variant, and 4-1BBL (including 4-1BBLΔcyt and 4-1BBLcyt trunc); a TGF-β decoy receptor, IL-15/E-15Rα, and IL-12p70; a TGF-β decoy receptor, IL-15/IL-15Rα, and IL-21; a TGF-β decoy receptor, IL-15/E-15Rα, IL-12p70, and a STING GOF variant; a TGF-β decoy receptor, IL-15/E-15Rα, IL-21, and a STING GOF variant; a TGF-β decoy receptor, IL-15/E-15Rα, IL-12p70, a STING GOF variant, and 4-1BBL (including 4-1BBLΔcyt and 4-1BBLcyt trunc); a TGF-β decoy receptor, IL-15/IL-15Rα, IL-21, a STING GOF variant, and 4-1BBL (including 4-1BBLΔcyt and 4-1BBLcyt trunc); a TGF-β decoy receptor, IL-12p70, and IL-21; a TGF-β decoy receptor, IL-12p70, IL-21, and a STING GOF variant; a TGF-β decoy receptor, IL-12p70, IL-21, a STING GOF variant, and 4-1BBL (including 4-1BBLΔcyt and 4-1BBLcyt trunc); a TGF-β decoy receptor and IL-12p70; a TGF-β decoy receptor, IL-12p70, and a STING GOF variant; a TGF-β decoy receptor, IL-12p70, a STING GOF variant, and 4-1BBL (including 4-1BBLΔcyt and 4-1BBLcyt trunc); a TGF-β decoy receptor, IL-12p70, and IL-18; a TGF-β decoy receptor, IL-12p70, IL-18, and a STING GOF variant; a TGF-β decoy receptor, IL-12p70, IL-18, a STING GOF variant, and 4-1BBL (including 4-1BBLΔcyt and 4-1BBLcyt trunc); a TGF-β decoy receptor and a STING GOF variant; an anti-CTLA-4 antibody, IL-2, and IL-12p70; an anti-CTLA-4 antibody, IL-2, and IL-21; an anti-CTLA-4 antibody, IL-2, IL-12p70, and a STING GOF variant; an anti-CTLA-4 antibody, IL-2, IL-21, and a STING GOF variant; an anti-CTLA-4 antibody, IL-2, IL-12p70, a STING GOF variant, and 4-1BBL (including 4-1BBLΔcyt and 4-1BBLcyt trunc); an anti-CTLA-4 antibody, IL-2, IL-21, a STING GOF variant, and 4-1BBL (including 4-1BBLΔcyt); an anti-CTLA-4 antibody, IL-15/E-15Rα, and a STING GOF variant; an anti-CTLA-4 antibody, IL-15/IL-15Rα, a STING GOF variant, and 4-1BBL (including 4-1BBLΔcyt and 4-1BBL with a truncated cytoplasmic domain); an anti-CTLA-4 antibody, IL-15/IL-15Rα, and IL-12p70; an anti-CTLA-4 antibody, IL-15/E-15Rα, and IL-21; an anti-CTLA-4 antibody, IL-15/E-15Rα, IL-12p70, and a STING GOF variant; an anti-CTLA-4 antibody, IL-15/E-15Rα, IL-21, and a STING GOF variant; an anti-CTLA-4 antibody, IL-15/E-15Rα, IL-12p70, a STING GOF variant, and 4-1BBL (including 4-1BBLΔcyt and 4-1BBLcyt trunc); an anti-CTLA-4 antibody, IL-15/E-15Rα, IL-21, a STING GOF variant, and 4-1BBL (including 4-1BBLΔcyt and 4-1BBLcyt trunc); an anti-CTLA-4 antibody, IL-12p70, and IL-21; an anti-CTLA-4 antibody, IL-12p70, IL-21, and a STING GOF variant; an anti-CTLA-4 antibody, IL-12p70, IL-21, a STING GOF variant, and 4-1BBL (including 4-1BBLΔcyt and 4-1BBLcyt trunc); an anti-CTLA-4 antibody and IL-12p70; an anti-CTLA-4 antibody, IL-12p70, and a STING GOF variant; an anti-CTLA-4 antibody, IL-12p70, a STING GOF variant, and 4-1BBL (including 4-1BBLΔcyt and 4-1BBLcyt trunc); an anti-CTLA-4 antibody, IL-12p70, and IL-18; an anti-CTLA-4 antibody, IL-12p70, IL-18, and a STING GOF variant; an anti-CTLA-4 antibody, IL-12p70, IL-18, a STING GOF variant, and 4-1BBL (including 4-1BBLΔcyt and 4-1BBLcyt trunc); an anti-CTLA-4 antibody and a STING GOF variant; a CD40 agonist, IL-2, and IL-12p70; a CD40 agonist, IL-2 and IL-21; a CD40 agonist, IL-2, IL-12p70, and a STING GOF variant; a CD40 agonist, IL-2, IL-21, and a STING GOF variant; a CD40 agonist, IL-2, IL-12p70, a STING GOF variant, and 4-1BBL (including 4-1BBLΔcyt and 4-1BBLcyt trunc); a CD40 agonist, IL-2, IL-21, a STING GOF variant, and 4-1BBL (including 4-1BBLΔcyt and 4-1BBLcyt trunc); a CD40 agonist, IL-15/E-15Rα, and a STING GOF variant; a CD40 agonist, IL-15/E-15Rα, a STING GOF variant, and 4-1BBL (including 4-1BBLΔcyt and 4-1BBLcyt trunc); a CD40 agonist, IL-15/E-15Rα, and IL-12p70; a CD40 agonist, IL-15/E-15Rα, and IL-21; a CD40 agonist, IL-15/E-15Rα, IL-12p70, and a STING GOF variant; a CD40 agonist, IL-15/E-15Rα, IL-21, and a STING GOF variant; a CD40 agonist, IL-15/E-15Rα, IL-12p70, a STING GOF variant, and 4-1BBL (including 4-1BBLΔcyt and 4-1BBLcyt trunc); a CD40 agonist, IL-15/IL-15Rα, IL-21, a STING GOF variant, and 4-1BBL (including 4-1BBLΔcyt and 4-1BBLcyt trunc); a CD40 agonist, IL-12p70, and IL-21; a CD40 agonist, IL-12p70, IL-21, and a STING GOF variant; a CD40 agonist, IL-12p70, IL-21, a STING GOF variant, and 4-1BBL (including 4-1BBLΔcyt and 4-1BBLcyt trunc); a CD40 agonist and IL-12p70; a CD40 agonist, IL-12p70, and a STING GOF variant; a CD40 agonist, IL-12p70, a STING GOF variant, and 4-1BBL (including 4-1BBLΔcyt and 4-1BBLcyt trunc); a CD40 agonist, IL-12p70, and IL-18; a CD40 agonist, IL-12p70, IL-18, and a STING GOF variant; a CD40 agonist, IL-12p70, IL-18, a STING GOF variant, and 4-1BBL (including 4-1BBLΔcyt and 4-1BBLcyt trunc); and a CD40 agonist and a STING GOF variant.

In all combinations including 4-1BBL, the 4-1BBL molecule can be a full-length protein (see, *e.g.,* SEQ ID NOs:389 and 393, for human and mouse 4-1BBL, respectively); a 4-1BBL variant with the cytoplasmic domain deleted (4-1BBLΔcyt; see *e.g.,* SEQ ID NOs:390 and 394, for human and murine 4-1BBLΔcyt, respectively); a 4-1BBL variant with a truncated (*i.e*., not fully deleted) cytoplasmic domain (4-1BBLcyt trunc; see, *e.g.,* SEQ ID NOs:391-392 and SEQ ID NOs:395-396, for exemplary human and mouse 4-1BBLcyt trunc variants); or a 4-1BBL molecule with a modified cytoplasmic domain, in which one or more Ser residues, which act as phosphorylation sites, are replaced at an appropriate locus or loci, such as, for human 4-1BBL, with reference to SEQ ID NO:389, Ser5 and Ser8, with a residue that reduces or eliminates reverse signaling. Additionally, all combinations including an anti-CTLA-4 antibody, can include an anti-CTLA-4 antibody fragment, such as an anti-CTLA-4 scFv (see, *e.g.,* SEQ ID NOs:403 and 404, for exemplary human and mouse anti-CTLA-4 scFv fragments, respectively), or an anti-CTLA-4 scFv-Fc (see, *e.g.,* SEQ ID NOs:402 and 405, for exemplary human and mouse anti-CTLA-4 scFv-Fc fragments, respectively).

Also provided are modified non-human Stimulator of Interferon Genes (STING) proteins, and STING protein chimeras, as well as delivery vehicles, including any described herein, pharmaceutical compositions, cells encoding or containing these STING proteins, and uses thereof, and methods of treatment of cancers. In particular, the immunostimulatory bacteria provided herein encode the modified non-human STING proteins, non-human STING proteins, and chimeras, as described herein. These STING proteins that are encoded by the immunostimulatory bacteria are provided herein and described throughout. Provided herein are:
1. Modified non-human STING proteins, where the non-human STING protein is one that has lower NF-κB activation than the human STING protein, and, optionally, higher type I interferon activation activity compared to the wild-type (WT) human STING protein. These non-human STING proteins are modified to include a mutation or mutations so that they have increased activity, or act constitutively in the absence of cytosolic nucleic acid signaling. The mutations are typically amino acid mutations that occur in interferonopathies in humans, such as those described above for human STING. The corresponding mutations are introduced into the non-human species STING proteins, where corresponding amino acid residues are identified by alignment. Also, in some embodiments, the TRAF6 binding site in the C-terminal tail (CTT) of the STING protein is deleted, reducing NF-κB signaling activity.
2. Modified STING proteins, particularly human STING proteins, that are chimeras, in which the CTT (C-terminal tail) region in the STING protein from one species, such as human, is replaced with the CTT from a STING protein of another species that has lower NF-κB signaling activity and/or higher type I IFN signaling activity than human STING. Also, the TRAF6 binding site is optionally deleted in these chimeras.
3. The modified STING proteins of 2 that also include the mutations of 1.
4. Delivery vehicles, such as immunostimulatory bacteria, any provided herein or known to those of skill in the art, including, for example, exosomes, nanoparticles, minicells, cells, liposomes, lysosomes, oncolytic viruses, and other viral vectors, that encode the modified STING proteins of any of 1-3.
5. Delivery vehicles, such as immunostimulatory bacteria, any provided herein or known to those of skill in the art, including, for example, exosomes, nanoparticles, minicells, cells, liposomes, lysosomes, oncolytic viruses, and other viral vectors, that encode unmodified STING from a non-human species whose STING protein has reduced NF-κB signaling activity compared to that of human STING, and optionally, increased type I interferon stimulating/signaling activity compared to that of human STING.
6. Cells (non-zygotes, if human), such as cells used for cell therapy, such as T-cells and stem cells, and cells used to produce the STING proteins of any of 1-3.
7. Pharmaceutical compositions that contain the STING proteins of any of 1-3, or the delivery vehicles of 4 and 5, or the cells of 6.
8. Uses and methods of treatment of cancer by administering any of 1-7, as described herein for the immunostimulatory bacteria.

Assays and methods to assess NF-κB activity (signaling activity), and type I interferon stimulating activity or interferon-β stimulating activity of STING are described herein, and also are known to those of skill in the art. Methods include those described, for example, in de Oliveira Mann et al. (2019) Cell Reports 27:1165-1175, which describes, *inter alia,* the interferon-β and NF-κB signaling activities of STING proteins from various species, including human, thereby identifying STING proteins from various species that have lower NF-xB activity than human STING, and those that also have comparable or higher interferon-β activity than human STING. de Oliveira Mann *et al.* (2019) provides species alignments and identifies domains of STING in each species, including the CTT domain (see, also, the Supplemental Information for de Oliveira Mann *et al.* (2019)).

The non-human STING proteins can be, but are not limited to, STING proteins from the following species: Tasmanian devil (*Sarcophilus harrisii*; SEQ ID NO:349), marmoset (*Callithrixjacchus*; SEQ ID NO:359), cattle (*Bos taurus*; SEQ ID NO:360), cat (*Felis catus*; SEQ ID NO:356), ostrich (*Struthio camelus australis*; SEQ ID NO:361), crested ibis (*Nipponia nippon*; SEQ ID NO:362), coelacanth (*Latimeria chalumnae*; SEQ ID NOs:363-364), boar (*Sus scrofa*; SEQ ID NO:365), bat (*Rousettus aegyptiacus*; SEQ ID NO:366), manatee (*Trichechus manatus latirostris*; SEQ ID NO:367), ghost shark (*Callorhinchus milii*; SEQ ID NO:368), and mouse (*Mus musculus*; SEQ ID NO:369). These vertebrate STING proteins readily activate immune signaling in human cells, indicating that the molecular mechanism of STING signaling is shared in vertebrates (see, de Oliveira Mann et al. (2019) Cell Reports 27:1165-1175).

It is shown herein that the immunostimulatory bacteria provided herein, by virtue of the ability to infect myeloid cells, such as tumor-resident and tissue-resident macrophages, and to retain viability for at least a limited time, and/or that deliver plasmids that encode therapeutic products that result in expression of type I IFN and/or other immune-stimulating products, such as gain-of-function (GOF) variants that do not require cytosolic nucleic acids, nucleotides, dinucleotides, or cyclic dinucleotides to result in expression of type I IFN, can convert macrophages that have the M2 phenotype into M1 or M1-like, with immunosuppressive properties reduced or eliminated, and immune-stimulating, anti-tumor or anti-viral properties enhanced or added, macrophages. Provided are immunostimulatory bacteria that contain a plasmid encoding a therapeutic product, where infection of a macrophage, including human macrophages, by the bacterium, converts an M2 macrophage to an M1 phenotype or M1-like phenotype macrophage. Provided are immunostimulatory bacteria that contain a plasmid encoding a therapeutic product whose expression in a macrophage results in the conversion of, or converts, M2 macrophages, such as human M2 macrophages, to an M1 or M1-like phenotype. The immunostimulatory bacteria with such properties include any of the bacteria provided herein that contain genome modifications that result in infection of tumor-resident (in subjects with cancer), and tissue-resident myeloid cells. These genome modifications include those that result in bacteria that do not have flagella, wherein the wild-type bacterium has flagella, and others, such as those that result in bacteria that are *pagP⁻*/*msbB⁻.* Other modifications include those that result in elimination of the asparaginase activity, such as modifications that result in bacteria that are *ansB⁻,* in the bacteria that infect myeloid cells, which thereby enhances T-cell activities, and other modifications that alter the lipopolysaccharide (LPS).

Included are immunostimulatory bacteria that encode therapeutic products in macrophages that facilitate or result in the conversion of, or that convert M2 macrophages to an M1 or M1-like phenotype. Exemplary of the therapeutic products are those that are part of a cytosolic DNA/RNA sensor pathway that leads to expression of type I interferon (IFN), particularly constitutive expression. This includes the gain-of-function (GOF) variants of therapeutic products that are part of the cytosolic DNA/RNA sensor pathway, and that do not require cytosolic nucleic acids, nucleotides, dinucleotides, or cyclic dinucleotides to result in expression of type I IFN, such as the variant and non-human STING proteins as described and provided herein. The immunostimulatory bacteria include any that can be modified as described herein, including the species listed herein, such as *Salmonella* species and strains.

Also provided are immunostimulatory bacteria in which an encoded therapeutic product, such as a protein, is linked to a moiety that confers an improved pharmacological property, such a pharmacokinetic or pharmacodynamic property, such as increased serum half-life. Hence, provided are immunostimulatory bacteria, where an encoded therapeutic product comprises an Fc domain, or a half-life extending moiety, such as human serum albumin, or a portion thereof. Half-life extension modalities or methods include, for example, PEGylation, modification of glycosylation, sialylation, PASylation (modification with polymers of PAS amino acids that are about 100-200 residues in length), ELPylation (see, *e.g.,* Floss et al. (2010) Trends Biotechnol. 28(1):37-45), HAPylation (modification with a glycine homopolymer), fusion to human serum albumin, fusion to GLK, fusion to CTP, GLP fusion, fusion to the constant fragment (Fc) domain of a human immunoglobulin (IgG), fusion to transferrin, fusion to non-structured polypeptides, such as XTEN (also referred to as rPEG, which is a genetic fusion of non-exact repeat peptide sequences, containing A, E, G, P, S, and T; see, *e.g.,* Schellenberger et al. (2009) Nat. Biotechnol. 27(12):1186-1190), and other such modifications and fusions that increase the size, increase the hydrodynamic radius, alter the charge, or target to receptors for recycling rather than clearance, and combinations of such modifications and fusions.

Also provided are immunostimulatory bacteria, where the encoded therapeutic product comprises the B7 protein transmembrane domain, or where the therapeutic product is GPI-anchored by virtue of an endogenous or added GPI anchor. The encoded therapeutic product can comprise a fusion to collagen.

The immunostimulatory bacteria in any and all embodiments can be any suitable species. Where reference is made to particular genes and gene modifications, the genes and modifications are those that correspond to the genes and modifications referenced with respect to *Salmonella,* as an exemplary species. Species and strains include, for example, a strain of *Rickettsia, Klebsiella, Bordetella, Neisseria, Aeromonas, Francisella, Corynebacterium, Citrobacter, Chlamydia, Haemophilus, Brucella, Mycobacterium, Mycoplasma, Legionella, Rhodococcus, Pseudomonas, Helicobacter, Vibrio, Bacillus, and Erysipelothrix.* For example, *Rickettsia rickettsiae, Rickettsia prowazekii, Rickettsia tsutsugamuchi, Rickettsia mooseri, Rickettsia sibirica, Bordetella bronchiseptica, Neisseria meningitidis, Neisseria gonorrhoeae, Aeromonas eucrenophila, Aeromonas salmonicida, Francisella tularensis, Corynebacterium pseudotuberculosis, Citrobacter freundii, Chlamydia pneumoniae, Haemophilus somnus, Brucella abortus, Mycobacterium intracellulare, Legionella pneumophila, Rhodococcus equi, Pudomonas aeruginosa, Helicobacter mustelae, Vibrio cholerae, Bacillus subtilis, Erysipelothrix rhusiopathiae, Yersinia enterocolitica, Rochalimaea quintana,* and *Agrobacterium tumerfacium.*

The bacteria can be attenuated, or rendered of low toxicity or non-toxic, by virtue of the modifications described herein. Exemplary of bacteria are species of *Salmonella,* such as a *Salmonella typhimurium* strain. The immunostimulatory bacteria provided herein include those that endogenously encode and express, or are modified to encode and express, a gene encoding resistance to complement killing *(rck),* such as a *Salmonella rck* gene. Therapeutic *E. coli* are modified to encode *rck* so that they can be administered systemically. Also provided, as described herein, and as set forth in the claims, are delivery vehicles, cells, pharmaceutical compositions, methods, uses, and treatments of cancer, particularly in humans. Also provided are companion diagnostics and methods for selection of subjects for treatment, and methods for monitoring treatment. These are described below and also in the claims, which are incorporated in their entirety into this section.

### BRIEF DESCRIPTION OF THE DRAWINGS

**Figure 1** depicts the alignment of wild-type human and Tasmanian devil STING proteins.
**Figure 2** depicts the alignment of wild-type human and marmoset STING proteins.
**Figure 3** depicts the alignment of wild-type human and cattle STING proteins.
**Figure 4** depicts the alignment of wild-type human and cat STING proteins.
**Figure 5** depicts the alignment of wild-type human and ostrich STING proteins.
**Figure 6** depicts the alignment of wild-type human and crested ibis STING proteins.
**Figure 7** depicts the alignment of wild-type human and coelacanth (SEQ ID NO:345) STING proteins.
**Figure 8** depicts the alignment of wild-type human and zebrafish STING proteins.
**Figure 9** depicts the alignment of wild-type human and boar STING proteins.
**Figure 10** depicts the alignment of wild-type human and bat STING proteins.
**Figure 11** depicts the alignment of wild-type human and manatee STING proteins.
**Figure 12** depicts the alignment of wild-type human and ghost shark STING proteins.
**Figure 13** depicts the alignment of wild-type human and mouse STING proteins.
**Figure 14** depicts an exemplary construct containing the *asd* expression cassette, including the bacterial promoter and any other bacterial regulatory sequence(s), placed in the opposite orientation of the cassette encoding the payload(s) under control of the eukaryotic promoter, and including bacterial terminators flanking the nucleic acid encoding the payload(s), and in the orientation to terminate any readthrough transcripts, from the prokaryotic promoter.

### DETAILED DESCRIPTION

### OUTLINE

**A. DEFINITIONS**
**B. OVERVIEW OF IMMUNOSTIMULATORY BACTERIA FOR CANCER THERAPY**
   **1. Bacterial Cancer Immunotherapy**
   **2. Prior Therapies that Target the Tumor Microenvironment**
      **a. Limitations of Autologous T-Cell Therapies**
      **b. Viral Vaccine Platforms**
      **c. Bacterial Cancer Therapies**
         **i. *Listeria***
         **ii. *Salmonella* Species**
         **iii. VNP20009**
         **iv. Wild-Type Strains**
   **3. Limitations of Existing Bacterial Cancer Immunotherapies**
**C. MODIFICATIONS AND ENHANCEMENTS OF IMMUNOSTIMULATORY BACTERIA TO INCREASE THERAPEUTIC INDEX AND TO INCREASE ACCUMULATION IN TUMOR-RESIDENT MYELOID CELLS**
   **1. Deletions in Genes in the LPS Biosynthetic Pathway**
      **a. *msbB* Deletion**
      **b. *pagP* Deletion**
   **2. Nutrient Auxotrophy**
      **a. *purI* Deletion/Disruption**
      **b. Adenosine Auxotrophy**
   **3. Plasmid Maintenance and Delivery**
      **a. *asd* Deletion**
      **b. *endA* Deletion/Disruption**
   **4. Flagellin Knockout Strains**
   **5. Engineering Bacteria to Promote Adaptive Immunity and Enhance T-Cell Function**
      **L-asparaginase II (*ansB*) Deletion/Disruption**
   **6. Deletions/Disruptions in *Salmonella* Genes Required for Curli Fimbriae Expression**
   **7. Improving Resistance to Complement**
      ***Rck* Expression**
   **8. Deletions of Genes Required for Lipoprotein Expression in *Salmonella* and Other Gram-Negative Bacteria**
   **9. Robust Immunostimulatory Bacteria Whose Genomes are Modified to be Optimized for Anti-Tumor Therapy, and that Encode Therapeutic Products, Including a Plurality Thereof**
   **10. Conversion of M2 Phenotype Macrophages into M1 and M1-Like Phenotype Macrophages**
**D. IMMUNOSTIMULATORY BACTERIA WITH ENHANCED THERAPEUTIC INDEX ENCODING GENETIC PAYLOADS THAT STIMULATE THE IMMUNE RESPONSE IN THE TUMOR MICROENVIRONMENT**
   **1. Immunostimulatory Proteins**
      **a. Cytokines and Chemokines**
      **b. Co-Stimulatory Molecules**
   **2. Molecules that Activate Prodrugs**
   **3. Constitutively Active Proteins that Stimulate the Immune Response and/or Type I IFN, Non-Human STING Proteins, Chimeras, and Modified Forms**
      **a. Constitutive STING Expression and Gain-of-Function Mutations**
      **b. Constitutive IRF3 Expression and Gain-of-Function Mutations**
      **c. Non-Human STING Proteins, and Variants Thereof with Increased or Constitutive Activity, and STING Chimeras, and Variants Thereof with Increased or Constitutive Activity**
      **d. Other Gene Products that Act as Cytosolic DNA/RNA Sensors and Constitutive Variants Thereof**
         **i. RIG-I**
         **ii. MDA5/IFIH1**
         **iii. IRF7**
      **e. Other Type I IFN Regulatory Proteins**
   **4. Antibodies and Antibody Fragments**
      **a. TGF-β**
      **b. Bispecific scFvs and T-Cell Engagers**
      **c. Anti-PD-1/Anti-PD-L1 Antibodies**
      **d. Anti-CTLA-4 Antibodies**
      **e. Additional Exemplary Checkpoint Targets**
   **5. Combinations of Immunomodulatory Proteins can have Synergistic Effects and/or Complementary Effects**
   **6. Immunostimulatory Bacteria that Deliver Combination Therapies**
**E. CONSTRUCTING EXEMPLARY PLASMIDS ENCODING THERAPEUTIC PRODUCTS FOR BACTERIAL DELIVERY**
   **1. Constitutive Promoters for Heterologous Expression of Proteins**
   **2. Multiple Therapeutic Product Expression Cassettes**
      **a. Single Promoter Constructs**
      **b. Dual/Multiple Promoter Constructs**
   **3. Regulatory Elements**
      **a. Post-Transcriptional Regulatory Elements**
      **b. Polyadenylation Signal Sequences and Terminators**
      **c. Enhancers**
      **d. Secretion Signals**
      **e. Improving Bacterial Fitness**
   **4. Origin of Replication and Plasmid Copy Number**
   **5. CpG Motifs and CpG Islands**
   **6. Plasmid Maintenance/Selection Components**
   **7. DNA Nuclear Targeting Sequences**
**F. PHARMACEUTICAL PRODUCTION, COMPOSITIONS, AND FORMULATIONS**
   **1. Manufacturing**
      **a. Cell Bank Manufacturing**
      **b. Drug Substance Manufacturing**
      **c. Drug Product Manufacturing**
   **2. Compositions**
   **3. Formulations**
      **a. Liquids, Injectables, Emulsions**
      **b. Dried Thermostable Formulations**
   **4. Compositions for Other Routes of Administration**
   **5. Dosages and Administration**
   **6. Packaging and Articles of Manufacture**
**G. METHODS OF TREATMENT AND USES**
   **1. Diagnostics for Patient Selection for Treatment and for Monitoring Treatment**
      **a. Patient Selection**
      **b. Diagnostics to Assess or Detect Activity of the Immunostimulatory Bacteria are Indicative of the Effectiveness of Treatment**
   **2. Tumors**
   **3. Administration**
   **4. Monitoring**
**H. EXAMPLES**

### A. DEFINITIONS

Unless defined otherwise, all technical and scientific terms used herein have the same meaning as is commonly understood by one of skill in the art to which the invention(s) belong. All patents, patent applications, published applications and publications, GenBank sequences, databases, websites and other published materials referred to throughout the entire disclosure herein, unless noted otherwise, are incorporated by reference in their entirety. In the event that there are a plurality of definitions for terms herein, those in this section prevail. Where reference is made to a URL or other such identifier or address, it is understood that such identifiers can change and particular information on the internet can come and go, but equivalent information can be found by searching the internet. Reference thereto evidences the availability and public dissemination of such information.

As used herein, "therapeutic bacteria" are bacteria that effect therapy, such as anti-cancer or anti-tumor therapy, when administered to a subject, such as a human.

As used herein, "immunostimulatory bacteria" are therapeutic bacteria that, when introduced into a subject, accumulate in immunoprivileged tissues and cells, such as tumors, the tumor microenvironment and tumor-resident immune cells, and replicate and/or express products that are immunostimulatory or that result in immunostimulation. For example, the immunostimulatory bacteria are attenuated in the host by virtue of reduced toxicity or pathogenicity and/or by virtue of encoded products that reduce toxicity or pathogenicity, as the immunostimulatory bacteria cannot replicate and/or express products (or have reduced replication/product expression), except primarily in immunoprivileged environments. Immunostimulatory bacteria provided herein are modified to encode a product or products or exhibit a trait or property that renders them immunostimulatory. Such products, properties and traits include, but are not limited to, for example, at least one of: an immunostimulatory protein, such as a cytokine, chemokine, or co-stimulatory molecule; a cytosolic DNA/RNA sensor or gain-of-function or constitutively active variant thereof (*e*.*g*., STING, IRF3, IRF7, MDA5, RIG-I); RNAi, such as siRNA (shRNA and microRNA), or CRISPR, that targets, disrupts, or inhibits a checkpoint gene, such as TREX1, PD-1, CTLA-4 and/or PD-L1; antibodies and fragments thereof, such as an anti-immune checkpoint antibody, an anti-IL-6 antibody, an anti-VEGF antibody, or a TGF-β inhibitory antibody; other antibody constructs such as bi-specific T-cell engagers (BiTEs^{®}); soluble TGF-β receptors that act as decoys for binding TGF-β, or TGF-β antagonizing polypeptides; and IL-6 binding decoy receptors. Immunostimulatory bacteria also can include a modification that renders the bacterium auxotrophic for a metabolite that is immunosuppressive or that is in an immunosuppressive pathway, such as adenosine.

As used herein, the strain designations VNP20009 (see, *e.g*., International PCT Application Publication No. WO 99/13053, see, also U.S. Patent No. 6,863,894), YS1646 and 41.2.9 are used interchangeably, and each refer to the strain deposited with the American Type Culture Collection (ATCC) and assigned Accession No. 202165. VNP20009 is a modified attenuated strain of *Salmonella typhimurium,* which contains deletions in *msbB* and *purI,* and was generated from wild-type strain ATCC #14028.

As used herein, the strain designations YS1456 and 8.7 are used interchangeably and each refer to the strain deposited with the American Type Culture Collection (ATCC) and assigned Accession No. 202164 (see, U.S. Patent No. 6,863,894).

As used herein, recitation that a bacterium is "derived from" a particular strain means that such strain can serve as a starting material and can be modified to result in the particular bacterium.

As used herein, an "expression cassette" refers to a nucleic acid construct that includes regulatory sequences for gene expression, operatively linked to nucleic acid encoding open reading frames (ORFs) that encode payloads, such as therapeutic products, or other proteins.

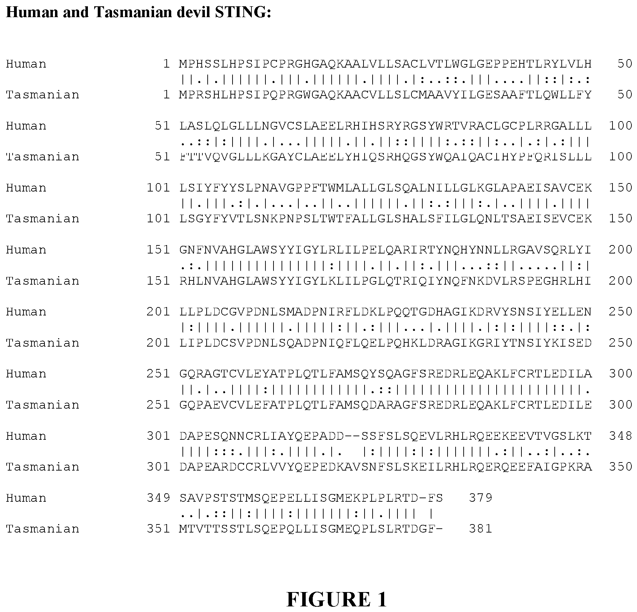

As used herein, 2A peptides are 18-22 amino-acid (aa)-long viral oligopeptides that mediate cleavage of polypeptides during translation in eukaryotic cells. The designation "2A" refers to a specific region of the viral genome, and different viral 2As have generally been named after the virus they were derived from. Exemplary of these are F2A (foot-and-mouth disease virus 2A), E2A (equine rhinitis A virus), P2A (porcine teschovirus-1 2A), and T2A (*Thosea asigna* virus 2A). See, *e.g.,* Liu et al. (2017) Scientific Reports 7:2193, Fig. 1, for encoding sequences. See, also, SEQ ID NOs:327-330. These peptides generally share a core sequence motif of DxExNPGP, and occur in a large number of viral families. They help break apart polyproteins by causing the ribosome to fail at making a peptide bond. The 2A peptides provide for multicistronic vectors, in which a plurality of proteins are expressed from a single open reading frame (ORF). For purposes herein, the 2A peptides include those that are naturally occurring, and any modified forms thereof, such as any having at 97%, 98%, or 99% sequence identity with any naturally-occurring 2A peptide, including those disclosed herein, that result in single polypeptides being transcribed and translated from a transcript comprising a plurality (2 or more) of open reading frames.

As used herein, an "interferonopathy" refers to a disorder associated with an upregulation of interferon by virtue of a mutation in a gene product involved in a pathway that regulates or induces expression of interferon. The activity of the products normally is regulated by a mediator, such as cytosolic DNA or RNA or nucleotides; when the protein product is mutated, the activity is constitutive. Type I interferonopathies include a spectrum of conditions, including the severe forms of Aicardi-Goutières Syndrome (AGS), and the milder Familial Chilblain Lupus (FCL). Nucleic acid molecules encoding mutated products with these properties can be produced *in vitro,* such as by selecting for mutations that result in a gain-of-function in the product, compared to the product of an allele that has normal activity, or has further gain-of-function compared to the disease-associated gain-of-function mutants described herein.

As used herein, a "gain-of-function mutation" is one that increases the activity of a protein compared to the same protein that does not have the mutation. For example, if the protein is a receptor, it will have increased affinity for a ligand; if it is an enzyme, it will have increased activity, including constitutive activity.

As used herein, an "origin of replication" is a sequence of DNA at which replication is initiated on a chromosome, or plasmid, or in a virus. For small DNA, including bacterial plasmids and small viruses, a single origin is sufficient.

The origin of replication determines the vector copy number, which depends upon the selected origin of replication. For example, if the expression vector is derived from the low-copy-number plasmid pBR322, the copy number is between about 15-20 copies/cell, and if derived from the high-copy-number plasmid pUC, it can be 500-700 copies/cell.

As used herein, medium copy number of a plasmid in cells is about or is 150 or less than 150, and low copy number is 5-30, such as 20 or less than 20. Low to medium copy number is less than 150 copies/cell. High copy number is greater than 150 copies/cell.

As used herein, a "CpG motif" is a pattern of bases that includes an unmethylated central CpG ("p" refers to the phosphodiester link between consecutive C and G nucleotides), surrounded by at least one base flanking (on the 3' and the 5' side of) the central CpG. A CpG oligodeoxynucleotide is an oligodeoxynucleotide that is at least about ten nucleotides in length and includes an unmethylated CpG. At least the C of the 5' CG 3' is unmethylated.

As used herein, a "RIG-I binding sequence" refers to a 5'triphosphate (5'ppp) structure directly, or that which is synthesized by RNA pol III from a poly(dA-dT) sequence, which, by virtue of interaction with RIG-I, can activate type I IFN via the RIG-I pathway. The RNA includes at least four A ribonucleotides (A-A-A-A); it can contain 4, 5, 6, 7, 8, 9, 10, or more. The RIG-I binding sequence is introduced into a plasmid in the bacterium for transcription into the polyA.

As used herein, "cytokines" are a broad and loose category of small proteins (~5-20 kDa) that are important in cell signaling. Cytokines include chemokines, interferons, interleukins, lymphokines, and tumor necrosis factors. Cytokines are cell signaling molecules that aid cell to cell communication in immune responses, and stimulate the movement of cells towards sites of inflammation, infection and trauma.

As used herein, "chemokines" refer to chemoattractant (chemotactic) cytokines that bind to chemokine receptors and include proteins isolated from natural sources as well as those made synthetically, as by recombinant means or by chemical synthesis. Exemplary chemokines include, but are not limited to, IL-8, IL-10, GCP-2, GRO-α, GRO-β, GRO-γ, ENA-78, PBP, CTAP III, NAP-2, LAPF-4, MIG (CXCL9), CXCL10 (IP-10), CXCL11, PF4, SDF-1α, SDF-1β, SDF-2, MCP-1, MCP-2, MCP-3, MCP-4, MCP-5, MIP-1α (CCL3), MIP-1β (CCL4), MIP-1γ (CCL9), MIP-2, MIP-2α, MIP-3α, MIP-3β, MIP-4, MIP-5, MDC, HCC-1, ALP, lungkine, Tim-1, eotaxin-1, eotaxin-2, I-309, SCYA17, TRAC, RANTES (CCL5), DC-CK-1, lymphotactin, and fractalkine, and others known to those of skill in the art. Chemokines are involved in the migration of immune cells to sites of inflammation, as well as in the maturation of immune cells and in the generation of adaptive immune responses.

As used herein, an "immunostimulatory protein" is a protein that exhibits or promotes an anti-tumor immune response in the tumor microenvironment. Exemplary of such proteins are cytokines, chemokines, and co-stimulatory molecules, such as, but not limited to, IFN-α, IFN-β, GM-CSF, IL-2, IL-7, IL-12, IL-15, IL-18, IL-21, IL-23, IL-12p70 (IL-12p40 + IL-12p35), IL-15/IL-15R alpha chain complex, IL-36 gamma, IL-2 that has attenuated binding to IL-2Ra, IL-2 that is modified so that it does not bind to IL-2Ra, CXCL9, CXCL10 (IP-10), CXCL11, CCL3, CCL4, CCL5, molecules involved in the potential recruitment and/or persistence of T-cells, CD40, CD40 ligand (CD40L), OX40, OX40 ligand (OX40L), 4-1BB, 4-1BB ligand (4-1BBL), 4-1BBL with a deleted cytoplasmic domain (4-1BBLΔcyt) or with a partially deleted (truncated) cytoplasmic domain, members of the B7-CD28 family, and members of the tumor necrosis factor receptor (TNFR) superfamily.

Among the immunostimulatory proteins are truncated co-stimulatory molecules, such as, for example, 4-1BBL, CD80, CD86, CD27L, B7RP1 and OX40L, each with a full or partial cytoplasmic domain deletion, for expression on an antigen presenting cell (APC). These truncated gene products, such as those with deletions or partial deletions of the cytoplasmic domain, are capable of constitutive immunostimulatory signaling to a T-cell through co-stimulatory receptor engagement, but are unable to counter-regulatory signal to the APC, due to a truncated or deleted cytoplasmic domain.

As used herein, a "cytoplasmic domain deletion" is a deletion in all, or a portion of, the amino acid residues that comprise the cytoplasmic, or intracellular, domain of the protein, where the deletion is sufficient to effect constitutive immunostimulatory signaling to a T-cell through co-stimulatory receptor engagement, and is sufficient to inhibit counter-regulatory signaling to the APC. For example, the cytoplasmic domain of human 4-1BBL (also known as TNFSF9) comprises amino acid residues 1-28 of SEQ ID NO:342. The cytoplasmic domain of human CD80 comprises amino acid residues 264-288 of the protein; the cytoplasmic domain of human CD86 comprises amino acid residues 269-329 of the protein; the cytoplasmic domain of human CD27L (also known as CD70) comprises amino acid residues 1-17 of the protein; the cytoplasmic domain of human B7RP1 (also known as ICOSLG or ICOS ligand) comprises amino acid residues 278-302 of the protein; and the cytoplasmic domain of human OX40L (also known as TNFSF4 or CD252) comprises amino acid residues 1-23 of the protein.

As used herein, a "decoy receptor" is a receptor that can specifically bind to specific growth factors or cytokines efficiently, but is not structurally able to signal or activate the intended receptor complex. The decoy receptor acts as an inhibitor by binding to a ligand and preventing it from binding to its cognate receptor.

For example, TGF-β family receptors include the cell-surface serine/threonine kinase receptors type I (TβRI or TGFβR1) and type II (TβRII or TGFβR2), which form heteromeric complexes in the presence of dimerized ligands, as well as the type III receptor betaglycan (TβRIII or TGFβR3). Soluble decoy receptors for TGF-β, which prevent the binding of TGF-β to its receptors, include the soluble extracellular domains (the TGF-β binding regions) of TβRI, TβRII, or TβRIII (βglycan), which can be fused with other molecules, such as an Fc domain. Additionally, BAMBI (bone morphogenetic protein (BMP) and activin membrane-bound inhibitor) is structurally related to type I receptors and acts as a decoy that inhibits receptor activation. A dominant negative TGFβR2 (DN-TGFβR2), which comprises the extracellular domain of TGFβR2 and the transmembrane region, but which lacks the cytoplasmic domain required for signaling, also can be used as a TGF-β decoy receptor (see, *e.g.,* International Application Publication No. WO 2018/138003).

As used herein, a co-stimulatory molecule agonist is a molecule that, upon binding to the co-stimulatory molecule, activates it or increases its activity. For example, the agonist can be an agonist antibody. CD40 agonist antibodies include, for example, CP-870,893, dacetuzumab, ADC-1013 (mitazalimab), and Chi Lob 7/4.

As used herein, a cytosolic DNA/RNA sensor pathway is one that is initiated by the presence of DNA, RNA, nucleotides, dinucleotides, cyclic nucleotides and/or cyclic dinucleotides or other nucleic acid molecules, that leads to production of type I interferon. The nucleic acid molecules in the cytosol occur from viral or bacterial or radiation or other such exposure, leading to activation of an immune response in a host.

As used herein, a "type I interferon pathway protein" is a protein that induces an innate immune response, such as the induction of type I interferon.

As used herein, a "cytosolic DNA/RNA sensor," is a protein that is part of a cytosolic DNA/RNA sensor pathway that leads to expression of an immune response mediator, such as type I interferon. A "cytosolic DNA/RNA sensor," includes type I interferon pathway proteins. For example, as described herein and known to those of skill in the art, cytosolic DNA is sensed by cGAS, leading to the production of cGAMP and subsequent STING/TBK1/IRF3 signaling, and type I IFN production. Bacterial cyclic dinucleotides (such as bacterial cyclic di-AMP) also activate STING. Hence, STING is an immunomodulatory protein that induces type I interferon. 5'-triphosphate RNA and double stranded RNA are sensed by RIG-I and either MDA-5 alone, or MDA-5/LGP2. This leads to polymerization of mitochondrial MAVS (mitochondrial antiviral-signaling protein), and also activates TANK-binding kinase 1 (TBK1) and interferon regulatory factor 3 (IRF3). The proteins in such pathways are immunostimulatory and lead to expression of innate immune response mediators, such as type I interferon. The immunomodulatory proteins in the DNA/RNA sensor pathways can be modified so that they have increased activity, or act constitutively in the absence of cytosolic nucleic acids, to lead to the immune response, such as the expression of type I interferon.

As used herein, the "carboxy-terminal tail" or "C-terminal tail" (CTT) of the innate immune protein STING refers to the C-terminal portion of a STING protein that, in a wild-type STING protein, is tethered to the cGAMP-binding domain by a flexible linker region. The CTT includes an IRF3 binding site, a TBK1 binding site, and a TRAF6 binding site. STING promotes the induction of interferon beta (IFN-β) production via the phosphorylation of the STING protein C-terminal tail (CTT) by TANK-binding kinase 1 (TBK1). The interaction between STING and TBK1 is mediated by an evolutionarily conserved stretch of eight amino-acid residues in the carboxy-terminal tail (CTT) of STING. TRAF6 catalyzes the formation of K63-linked ubiquitin chains on STING, leading to the activation of the transcription factor NF-κB and the induction of an alternative STING-dependent gene expression program. Deletion or disruption of the TRAF6 binding site in the CTT can reduce activation of NF-κB signaling. Substitution of the human STING CTT (or portions thereof), with the CTT (or corresponding portion thereof) from the STING protein of a species with low NF-κB activation, can decrease NF-κB activation by the resulting modified human STING protein. The STING CTT is an unstructured stretch of ~40 amino acids that contains sequence motifs required for STING phosphorylation and recruitment of IRF3 (see, de Oliveira Mann et al. (2019) Cell Reports 27:1165-1175). Human STING residue S366 has been identified as a primary TBK1 phosphorylation site that is part of an LxIS motif shared among innate immune adaptor proteins that activate interferon signaling (see, de Oliveira Mann et al. (2019) Cell Reports 27:1165-1175). The human STING CTT contains a second PxPLR motif that includes the residue L374, which is required for TBK1 binding; the LxIS and PxPLR sequences are conserved among vertebrate STING alleles (see, de Oliveira Mann et al. (2019) Cell Reports 27:1165-1175). Exemplary STING CTT sequences, and the IRF3, TBK1 and TRAF6 binding sites, are set forth in the following table:

| **Species** | **C-terminal Tail (CTT) Sequence** | **SEQ ID NO.** | **IRF3 Binding Site** | **TBK1 Binding Site** | **TRAF6 Binding Site** |
|---|---|---|---|---|---|
| Human | EKEEVTVGSLKTSAVPSTSTMS QEPELLISGMEKPLPLRTDFS | 370 | PELLIS | PLPLRT | DFS |
| Tasmanian devil | RQEEFAIGPKRAMTVTTSSTLS QEPQLLISGMEQPLSLRTDGF | 371 | PQLLIS | PLSLRT | DGF |
| Marmoset | EEEEVTVGSLKTSEVPSTSTMS QEPELLISGMEKPLPLRSDLF | 372 | PELLIS | PLPLRS | DLF |
| Cattle | EREVTMGSTETSVMPGSSVLS QEPELLISGLEKPLPLRSDVF | 373 | PELLIS | PLPLRS | DVF |
| Cat | EREVTVGSVGTSMVRNPSVLS QEPNLLISGMEQPLPLRTDVF | 374 | PNLLIS | PLPLRT | DVF |
| Ostrich | RQEEYTVCDGTLCSTDLSLQIS ESDLPQPLRSDCL | 375 | LSLQIS | PQPLRS | DCL |
| Boar | EREVTMGSAETSVVPTSSTLSQ EPELLISGMEQPLPLRSDIF | 376 | PELLIS | PLPLRS | DIF |
| Bat | EKEEVTVGTVGTYEAPGSSTL HQEPELLISGMDQPLPLRTDIF | 377 | PELLIS | PLPLRT | DIF |
| Manatee | EREEVTVGSVGTSVVPSPSSPS TSSLSQEPKLLISGMEQPLPLRT DVF | 378 | PKLLIS | PLPLRT | DVF |
| Crested ibis | CHEEYTVYEGNQPHNPSTTLH STELNLQISESDLPQPLRSDCF | 379 | LNLQIS | PQPLRS | DCF |
| Coelacanth (variant 1) | QKEEYFMSEQTQPNSSSTSCLS TEPQLMISDTDAPHTLKRQVC | 380 | PQLMIS | PHTLK R | QVC |
| Coelacanth (variant 2) | QKEEYFMSEQTQPNSSSTSCLS TEPQLMISDTDAPHTLKSGF | 381 | PQLMIS | PHTLKS | GF |
| Zebrafish | DGEIFMDPTNEVHPVPEEGPV GNCNGALQATFHEEPMSDEPT LMFSRPQSLRSEPVETTDYFNP SSAMKQN | 382 | PTLMFS | PQSLRS | EPVETT DY |
| Ghost shark | LTEYPVAEPSNANETDCMSSE PHLMISDDPKPLRSYCP | 383 | PHLMIS | PKPLRS | YCP |
| Mouse | EKEEVTMNAPMTSVAPPPSVL SQEPRLLISGMDQPLPLRTDLI | 384 | PRLLIS | PLPLRT | DLI |

As used herein, a "STING pathway agonist" is any product that increases type I interferon (IFN) expression via activation of the STING pathway. Exemplary of such agonists are the gain-of-function STING polypeptide variants provided herein, as well as gain-of-function variants of other cytosolic DNA/RNA sensors and type I IFN pathway proteins, such as variants of IRF-3, IRF-7, MDA5, and RIG-I, that increase or render expression of type I IFN constitutive, via the STING pathway.

As used herein, a bacterium that is modified so that it "induces less cell death in tumor-resident immune cells" or "induces less cell death in immune cells" is one that is less toxic than the bacterium without the modification, or one that has reduced virulence compared to the bacterium without the modification. Exemplary of such modifications are those that eliminate pyroptosis in phagocytic cells and that alter lipopolysaccharide (LPS) profiles on the bacterium. These modifications include disruption of or deletion of flagellin genes, *pagP,* or one or more components of the SPI-1 pathway, such as *hilA,* rod protein (*e.g., prgJ*)*,* needle protein (*e.g., prgI*)*,* and QseC.

As used herein, a bacterium that is "modified so that it preferentially infects tumor-resident immune cells" or "modified so that it preferentially infects immune cells" has a modification in its genome that reduces its ability to infect cells other than immune cells. Exemplary of such modifications are modifications that disrupt the type 3 secretion system or type 4 secretion system or other genes or systems that affect the ability of a bacterium to invade a non-immune cell. For example, modifications include disruption/deletion of an SPI-1 component, which is needed for infection of cells, such as epithelial cells, but does not affect infection of immune cells, such as phagocytic cells, by *Salmonella.*

As used herein, a "modification" is in reference to modification of a sequence of amino acids of a polypeptide, or a sequence of nucleotides in a nucleic acid molecule, and includes deletions, insertions, and replacements of amino acids or nucleotides, respectively. Methods of modifying a polypeptide are routine to those of skill in the art, such as by using recombinant DNA methodologies.

As used herein, a modification to a bacterial genome, or to a plasmid, or to a gene includes deletions, replacements, and insertions of nucleic acid.

As used herein, RNA interference (RNAi) is a biological process in which RNA molecules inhibit gene expression or translation, by neutralizing targeted mRNA molecules to inhibit translation, and thereby expression, of a targeted gene.

As used herein, RNA molecules that act via RNAi are referred to as inhibitory by virtue of their silencing of the expression of a targeted gene. Silencing expression means that expression of the targeted gene is reduced, or suppressed, or inhibited.

As used herein, gene silencing via RNAi is said to inhibit, suppress, disrupt, or silence expression of a targeted gene. A targeted gene contains sequences of nucleotides that correspond to the sequences in the inhibitory RNA, whereby the inhibitory RNA silences expression of target mRNA.

As used herein, inhibiting, suppressing, disrupting, or silencing a targeted gene refers to processes that alter expression, such as translation, of the targeted gene, whereby activity or expression of the product encoded by the targeted gene is reduced. Reduction includes a complete knock-out or a partial knockout, whereby, with reference to the immunostimulatory bacteria provided herein and administration herein, treatment is effected.

As used herein, small interfering RNAs (siRNAs) are small pieces of doublestranded (ds) RNA, usually about 21 nucleotides long, with 3' overhangs (2 nucleotides) at each end that can be used to "interfere" with the translation of proteins by binding to and promoting the degradation of messenger RNA (mRNA) at specific sequences. In doing so, siRNAs prevent the production of specific proteins based on the nucleotide sequences of their corresponding mRNAs. The process is called RNA interference (RNAi), and also is referred to as siRNA silencing, or siRNA knockdown.

As used herein, a short-hairpin RNA or small-hairpin RNA (shRNA) is an artificial RNA molecule with a tight hairpin turn that can be used to silence target gene expression via RNA interference (RNAi). Expression of shRNA in cells is typically accomplished by delivery of plasmids, or through viral or bacterial vectors.

As used herein, a tumor microenvironment (TME) is the cellular environment in which the tumor exists, including surrounding blood vessels, immune cells, fibroblasts, bone marrow-derived inflammatory cells, lymphocytes, signaling molecules and the extracellular matrix (ECM). Conditions that exist include, but are not limited to, increased vascularization, hypoxia, low pH, increased lactate concentration, increased pyruvate concentration, increased interstitial fluid pressure, and altered metabolites or metabolism, such as higher levels of adenosine, which are indicative of a tumor.

As used herein, "bactofection" refers to the bacteria-mediated transfer of genes or plasmid DNA into eukaryotic cells, such as mammalian cells.

As used herein, human type I interferons (IFNs) are a subgroup of interferon proteins that regulate the activity of the immune system. All type I IFNs bind to a specific cell surface receptor complex, such as the IFN-α receptor. Type I interferons include IFN-α and IFN-β, among others. Myeloid cells are the primary producers of IFN-α and IFN-β, which have antiviral activity that is involved mainly in innate immune responses. Two types of IFN-β are IFN-β1 (IFNB1) and IFN-β3 (IFNB3).

As used herein, M1 macrophage phenotype and M2 macrophage phenotype refer to the two broad groups into which macrophage phenotype is divided: M1 (classically activated macrophages) and M2 (alternatively activated macrophages). The role of M1 macrophages is to secrete pro-inflammatory cytokines and chemokines, and to present antigens, so that they participate in the positive immune response and function as an immune monitor. The main pro-inflammatory cytokines they produces are IL-6, IL-12 and TNF-alpha. M2 macrophages primarily secrete arginase-I, IL-10, TGF-β, and other anti-inflammatory cytokines, which have the function of reducing inflammation, and contributing to tumor growth and immunosuppressive function. A macrophage with an M1-like phenotype secretes pro-inflammatory cytokines, and does not have the immunosuppressive activity(ies) of an M2 macrophage. Conversion of an M2 macrophage into a macrophage with an M1 or M1-like phenotype converts an M2 macrophage into one that is not immunosuppressive, but participates in an anti-tumor response. An M2 macrophage that is converted into a macrophage with an M1 or M1-like phenotype exhibits more pro-inflammatory cytokines/chemokines and receptors, such as CD80 and CCR7, and chemokines, such as IFNγ and CXCL10. M1 phenyotypic markers include, but are not limited to, one or more of CD80, CD86, CD64, CD16, and CD32. The expression of nitric oxide synthase (iNOS) in M1 also can serve as a phenotypic marker. CD163 and CD206 are major markers for the identification of M2 macrophages. Other surface markers for M2-type cells also include CD68. A reduction or elimination of any of the M2 markers, and an increase in cytokines/chemokines indicative of M1 macrophages, reflect a conversion from an M2 phenotype into an M1 or M1-like phenotype. The sections below, and the working examples regarding M2 to M1-like or M1 phenotype conversion, describe exemplary cytokine profiles and markers that are induced.

As used herein, recitation that a nucleic acid or encoded RNA targets a gene means that it inhibits or suppresses or silences expression of the gene by any mechanism. Generally, such nucleic acid includes at least a portion complementary to the targeted gene, where the portion is sufficient to form a hybrid with the complementary portion.

As used herein, "deletion," when referring to a nucleic acid or polypeptide sequence, refers to the deletion of one or more nucleotides or amino acids compared to a sequence, such as a target polynucleotide, or polypeptide, or a native, or wild-type sequence.

As used herein, "insertion," when referring to a nucleic acid or amino acid sequence, describes the inclusion of one or more additional nucleotides or amino acids, within a target, native, wild-type or other related sequence. Thus, a nucleic acid molecule that contains one or more insertions compared to a wild-type sequence, contains one or more additional nucleotides within the linear length of the sequence.

As used herein, "additions" to nucleic acid and amino acid sequences describe addition of nucleotides or amino acids onto either termini compared to another sequence.

As used herein, "substitution" or "replacement" refers to the replacing of one or more nucleotides or amino acids in a native, target, wild-type or other nucleic acid or polypeptide sequence with an alternative nucleotide or amino acid, without changing the length (as described in numbers of nucleotides or residues) of the molecule. Thus, one or more substitutions in a molecule does not change the number of nucleotides or amino acid residues of the molecule. Amino acid replacements compared to a particular polypeptide can be expressed in terms of the number of the amino acid residue along the length of the polypeptide sequence.

As used herein, "at a position corresponding to," or recitation that nucleotides or amino acid positions "correspond to" nucleotides or amino acid positions in a disclosed sequence, such as set forth in the Sequence Listing, refers to nucleotides or amino acid positions identified upon alignment with the disclosed sequence to maximize identity using a standard alignment algorithm, such as the GAP algorithm. By aligning the sequences, one skilled in the art can identify corresponding residues, for example, using conserved and identical amino acid residues as guides. In general, to identify corresponding positions, the sequences of amino acids are aligned so that the highest order match is obtained (see, *e.g.,* Computational Molecular Biology, Lesk, A.M., ed., Oxford University Press, New York, 1988; Biocomputing: Informatics and Genome Projects, Smith, D.W., ed., Academic Press, New York, 1993*;* Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; Sequence Analysis Primer, Gribskov, M. and Devereux, J., eds., M Stockton Press, New York, 1991; and Carrillo et al. (1988) SIAM J. Applied Math 48:1073)*.*

As used herein, alignment of a sequence refers to the use of homology to align two or more sequences of nucleotides or amino acids. Typically, two or more sequences that are related by 50% or more identity are aligned. An aligned set of sequences refers to 2 or more sequences that are aligned at corresponding positions and can include aligning sequences derived from RNAs, such as ESTs and other cDNAs, aligned with a genomic DNA sequence. Related or variant polypeptides or nucleic acid molecules can be aligned by any method known to those of skill in the art. Such methods typically maximize matches, and include methods, such as using manual alignments, and by using the numerous alignment programs available (*e*.*g*., BLASTP) and others known to those of skill in the art. By aligning the sequences of polypeptides or nucleic acids, one skilled in the art can identify analogous portions or positions, using conserved and identical amino acid residues as guides. Further, one skilled in the art also can employ conserved amino acid or nucleotide residues as guides to find corresponding amino acid or nucleotide residues between and among human and non-human sequences. Corresponding positions also can be based on structural alignments, for example by using computer simulated alignments of protein structure. In other instances, corresponding regions can be identified. One skilled in the art also can employ conserved amino acid residues as guides to find corresponding amino acid residues between and among human and non-human sequences.

As used herein, a "property" of a polypeptide, such as an antibody, refers to any property exhibited by a polypeptide, including, but not limited to, binding specificity, structural configuration or conformation, protein stability, resistance to proteolysis, conformational stability, thermal tolerance, and tolerance to pH conditions. Changes in properties can alter an "activity" of the polypeptide. For example, a change in the binding specificity of the antibody polypeptide can alter the ability to bind an antigen, and/or various binding activities, such as affinity or avidity, or *in vivo* activities of the polypeptide.

As used herein, an "activity" or a "functional activity" of a polypeptide, such as an antibody, refers to any activity exhibited by the polypeptide. Such activities can be empirically determined. Exemplary activities include, but are not limited to, the ability to interact with a biomolecule, for example, through antigen-binding, DNA binding, ligand binding, or dimerization, or enzymatic activity, for example, kinase activity, or proteolytic activity. For an antibody (including antibody fragments), activities include, but are not limited to, the ability to specifically bind a particular antigen, affinity of antigen-binding (*e*.*g*., high or low affinity), avidity of antigen-binding (*e*.*g*., high or low avidity), on-rate, off-rate, effector functions, such as the ability to promote antigen neutralization or clearance, virus neutralization, and *in vivo* activities, such as the ability to prevent infection or invasion of a pathogen, or to promote clearance, or to penetrate a particular tissue or fluid or cell in the body. Activity can be assessed *in vitro* or *in vivo* using recognized assays, such as ELISA, flow cytometry, surface plasmon resonance, or equivalent assays to measure on-rate or off-rate, immunohistochemistry and immunofluorescence histology and microscopy, cell-based assays, and binding assays (*e*.*g*., panning assays).

As used herein, "bind," "bound," or grammatical variations thereof, refers to the participation of a molecule in any attractive interaction with another molecule, resulting in a stable association in which the two molecules are in close proximity to one another. Binding includes, but is not limited to, non-covalent bonds, covalent bonds (such as reversible and irreversible covalent bonds), and includes interactions between molecules such as, but not limited to, proteins, nucleic acids, carbohydrates, lipids, and small molecules, such as chemical compounds, including drugs.

As used herein, "antibody" refers to immunoglobulins and immunoglobulin fragments, whether natural, or partially or wholly synthetically, such as recombinantly produced, including any fragment thereof containing at least a portion of the variable heavy chain and light region of the immunoglobulin molecule that is sufficient to form an antigen-binding site and, when assembled, to specifically bind an antigen. Hence, an antibody includes any protein having a binding domain that is homologous or substantially homologous to an immunoglobulin antigen-binding domain (antibody combining site). For example, an antibody refers to an antibody that contains two heavy chains (which can be denoted H and H') and two light chains (which can be denoted L and L'), where each heavy chain can be a full-length immunoglobulin heavy chain or a portion thereof sufficient to form an antigen-binding site (*e*.*g*., heavy chains include, but are not limited to, V_{H} chains, V_{H}-C_{H}1 chains, and V_{H}-C_{H}1-C_{H}2-C_{H}3 chains), and each light chain can be a full-length light chain or a portion thereof sufficient to form an antigen-binding site (*e.g.,* light chains include, but are not limited to, V_{L} chains and V_{L}-C_{L} chains). Each heavy chain (H and H') pairs with one light chain (L and L', respectively). Typically, antibodies minimally include all or at least a portion of the variable heavy (V_{H}) chain and/or the variable light (V_{L}) chain. The antibody also can include all or a portion of the constant region.

For purposes herein, the term antibody includes full-length antibodies and portions thereof including antibody fragments, such as anti-CTLA-4 antibody fragments. Antibody fragments, include, but are not limited to, Fab fragments, Fab' fragments, F(ab')₂ fragments, Fv fragments, disulfide-linked Fvs (dsFv), Fd fragments, Fd' fragments, single-chain Fvs (scFvs), scFv-Fc fragments (in which the V_{H} domain in the scFv is linked to an Fc, such as a human IgG1 Fc, for example), single-chain Fabs (scFabs), diabodies, anti-idiotypic (anti-Id) antibodies, or antigen-binding fragments of any of the above. Antibody also includes synthetic antibodies, recombinantly produced antibodies, multi-specific antibodies (*e*.*g*., bispecific antibodies), human antibodies, non-human antibodies, humanized antibodies, chimeric antibodies, and intrabodies. Antibodies provided herein include members of any immunoglobulin class (*e.g.,* IgG, IgM, IgD, IgE, IgA and IgY), any subclass (*e.g.,* IgG1, IgG2, IgG3, IgG4, IgA1, and IgA2), or sub-subclass (*e*.*g*., IgG2a and IgG2b). Antibodies for human therapy generally are human antibodies or are humanized.

As used herein, "antibody fragment(s)" refers to (i) monovalent and monospecific antibody derivatives that contain the variable heavy and/or light chains, or functional fragments of an antibody and lack an Fc part; and (ii) BiTEs^{®} (tandem scFvs), DARTs, diabodies, and single-chain diabodies (scDbs). Thus, an antibody fragment includes alan: Fab, Fab', scFab, scFv, scFv-Fc, Fv fragment, nanobody (see, *e.g.,* antibodies derived from *Camelus bactriamus, Camelus dromedarius*, or *Lama paccos*) (see, *e.g.,* U.S. Pat. No. 5,759,808; and Stijlemans et al. (2004) J. Biol. Chem. 279:1256-1261), V_{HH}, dAb (single-domain antibody), minimal recognition unit, single-chain diabody (scDb), BiTE^{®}, and DART. The recited antibody fragments have a molecular weight below 60 kDa.

As used herein, "nucleic acid" refers to at least two linked nucleotides or nucleotide derivatives, including a deoxyribonucleic acid (DNA) and a ribonucleic acid (RNA), joined together, typically by phosphodiester linkages. Also included in the term "nucleic acid" are analogs of nucleic acids, such as peptide nucleic acid (PNA), phosphorothioate DNA, and other such analogs and derivatives, or combinations thereof. Nucleic acids also include DNA and RNA derivatives containing, for example, a nucleotide analog or a "backbone" bond other than a phosphodiester bond, for example, a phosphotriester bond, a phosphoramidate bond, a phosphorothioate bond, a thioester bond, or a peptide bond (peptide nucleic acid). The term also includes equivalents, derivatives, variants and analogs of either RNA or DNA made from nucleotide analogs, and single-stranded (sense or antisense) and double-stranded nucleic acids. Deoxyribonucleotides include deoxyadenosine, deoxycytidine, deoxyguanosine, and deoxythymidine. For RNA, the uracil base is uridine.

As used herein, an isolated nucleic acid molecule is one which is separated from other nucleic acid molecules which are present in the natural source of the nucleic acid molecule. An "isolated" nucleic acid molecule, such as a cDNA molecule, can be substantially free of other cellular material, or culture medium when produced by recombinant techniques, or substantially free of chemical precursors or other chemicals when chemically synthesized. Exemplary isolated nucleic acid molecules provided herein include isolated nucleic acid molecules encoding an antibody or antigen-binding fragments provided herein.

As used herein, "operably linked" or "operatively linked," with reference to nucleic acid sequences, regions, elements, or domains, means that the nucleic acid regions are functionally related to each other. It refers to a juxtaposition whereby the components so described are in a relationship permitting them to function in their intended manner. For instance, a promoter is operably linked to a coding sequence if the promoter effects or affects its transcription or expression. For example, a nucleic acid encoding a leader peptide can be operably linked to a nucleic acid encoding a polypeptide, whereby the nucleic acids can be transcribed and translated to express a functional fusion protein, wherein the leader peptide effects secretion of the fusion polypeptide. In some instances, the nucleic acid encoding a first polypeptide (*e.g*., a leader peptide) is operably linked to a nucleic acid encoding a second polypeptide, and the nucleic acids are transcribed as a single mRNA transcript, but translation of the mRNA transcript can result in one of two polypeptides being expressed. For example, an amber stop codon can be located between the nucleic acid encoding the first polypeptide and the nucleic acid encoding the second polypeptide, such that, when introduced into a partial amber suppressor cell, the resulting single mRNA transcript can be translated to produce either a fusion protein containing the first and second polypeptides, or can be translated to produce only the first polypeptide. In another example, a promoter can be operably linked to nucleic acid encoding a polypeptide, whereby the promoter regulates or mediates the transcription of the nucleic acid.

As used herein, "synthetic," with reference to, for example, a synthetic nucleic acid molecule or a synthetic gene or a synthetic peptide, refers to a nucleic acid molecule, or gene, or polypeptide molecule that is produced by recombinant methods and/or by chemical synthesis methods.

As used herein, the residues of naturally occurring α-amino acids are the residues of those 20 α-amino acids found in nature which are incorporated into a protein by the specific recognition of the charged tRNA molecule with its cognate mRNA codon in humans.

As used herein, a "polypeptide" refers to two or more amino acids covalently joined. The terms "polypeptide" and "protein" are used interchangeably herein.

As used herein, a "peptide" refers to a polypeptide that is from 2 to about or 40 amino acids in length.

As used herein, an "amino acid" is an organic compound containing an amino group and a carboxylic acid group. A polypeptide contains two or more amino acids. For purposes herein, amino acids contained in the antibodies and immunostimulatory proteins provided include the twenty naturally-occurring amino acids (see Table below), non-natural amino acids, and amino acid analogs (*e.g*., amino acids wherein the α-carbon has a side chain). As used herein, the amino acids, which occur in the various amino acid sequences of polypeptides appearing herein, are identified according to their well-known, three-letter or one-letter abbreviations (see Table below). The nucleotides, which occur in the various nucleic acid molecules and fragments, are designated with the standard single-letter designations used routinely in the art.

As used herein, "amino acid residue" refers to an amino acid formed upon chemical digestion (hydrolysis) of a polypeptide at its peptide linkages. The amino acid residues described herein are generally in the "L" isomeric form. Residues in the "D" isomeric form can be substituted for any L-amino acid residue, as long as the desired functional property is retained by the polypeptide. NH₂ refers to the free amino group present at the amino terminus of a polypeptide. COOH refers to the free carboxy group present at the carboxyl terminus of a polypeptide. In keeping with standard polypeptide nomenclature described in J. Biol. Chem., 243:3557-59 (1968) and adopted at 37 C.F.R. §§ 1.821-1.822, abbreviations for amino acid residues are shown in the following Table:

**Table of Correspondence**

| **SYMBOL** | | |
|---|---|---|
| **1-Letter** | **3-Letter** | **AMINO ACID** |
| Y | Tyr | Tyrosine |
| G | Glv | Glycine |
| F | Phe | Phenylalanine |
| M | Met | Methionine |
| A | Ala | Alanine |
| S | Ser | Serine |
| I | Ile | Isoleucine |
| L | Leu | Leucine |
| T | Thr | Threonine |
| V | Val | Valine |
| P | Pro | Proline |
| K | Lys | Lysine |
| H | His | Histidine |
| Q | Gln | Glutamine |
| E | Glu | Glutamic acid |
| Z | Glx | Glutamic Acid and/or Glutamine |
| W | Trp | Tryptophan |
| R | Arg | Arginine |
| D | Asp | Aspartic acid |
| N | Asn | Asparagine |
| B | Asx | Aspartic Acid and/or Asparagine |
| C | Cys | Cysteine |
| X | Xaa | Unknown or other |

All sequences of amino acid residues represented herein by a formula have a left to right orientation in the conventional direction of amino-terminus to carboxyl-terminus. The phrase "amino acid residue" is defined to include the amino acids listed in the above Table of Correspondence, as well as modified, non-natural, and unusual amino acids. A dash at the beginning or end of an amino acid residue sequence indicates a peptide bond to a further sequence of one or more amino acid residues, or to an amino-terminal group such as NH₂, or to a carboxyl-terminal group such as COOH.

In a peptide or protein, suitable conservative substitutions of amino acids are known to those of skill in the art and generally can be made without altering a biological activity of a resulting molecule. Those of skill in the art recognize that, in general, single amino acid substitutions in non-essential regions of a polypeptide do not substantially alter biological activity (see, *e.g.,* Watson et al., Molecular Biology of the Gene, 4th Edition, 1987, The Benjamin/Cummings Pub. Co., p. 224).

Such substitutions can be made in accordance with the exemplary substitutions set forth in the following Table:

### Exemplary Conservative Amino Acid Substitutions

| **Original Residue** | **Exemplary Conservative Substitution(s)** |
|---|---|
| Ala (A) | Gly; Ser |
| Arg (R) | Lys |
| Asn (N) | Gln; His |
| Cys (C) | Ser |
| Gln (Q) | Asn |
| Glu (E) | Asp |
| Gly (G) | Ala; Pro |
| His (H) | Asn; Gln |
| Ile (I) | Leu; Val |
| Leu (L) | Ile: Val |
| Lys (K) | Arg; Gln; Glu |
| Met (M) | Leu; Tyr; Ile |
| Phe (F) | Met; Leu; Tyr |
| Ser (S) | Thr |
| Thr (T) | Ser |
| Trp (W) | Tyr |
| Tyr (Y) | Trp; Phe |
| Val (V) | Ile; Leu |

Other substitutions also are permissible and can be determined empirically or in accord with other known conservative or non-conservative substitutions.

As used herein, "naturally occurring amino acids" refer to the 20 L-amino acids that occur in polypeptides.

As used herein, the term "non-natural amino acid" refers to an organic compound that has a structure similar to a natural amino acid, but that has been modified structurally to mimic the structure and reactivity of a natural amino acid. Non-naturally occurring amino acids thus include, for example, amino acids or analogs of amino acids other than the 20 naturally occurring amino acids and include, but are not limited to, the D-stereoisomers of amino acids. Exemplary non-natural amino acids are known to those of skill in the art, and include, but are not limited to, 2-Aminoadipic acid (Aad), 3-Aminoadipic acid (bAad), β-alanine/β-Amino-propionic acid (Bala), 2-Aminobutyric acid (Abu), 4-Aminobutyric acid/piperidinic acid (4Abu), 6-Aminocaproic acid (Acp), 2-Aminoheptanoic acid (Ahe), 2-Aminoisobutyric acid (Aib), 3-Aminoisobutyric acid (Baib), 2-Aminopimelic acid (Apm), 2,4-Diaminobutyric acid (Dbu), Desmosine (Des), 2,2'-Diaminopimelic acid (Dpm), 2,3-Diaminopropionic acid (Dpr), N-Ethylglycine (EtGly), N-Ethylasparagine (EtAsn), Hydroxylysine (Hyl), allo-Hydroxylysine (Ahyl), 3-Hydroxyproline (3Hyp), 4-Hydroxyproline (4Hyp), Isodesmosine (Ide), allo-Isoleucine (Aile), N-Methylglycine, sarcosine (MeGly), N-Methylisoleucine (MeIle), 6-N-Methyllysine (MeLys), N-Methylvaline (MeVal), Norvaline (Nva), Norleucine (Nle), and Ornithine (Om).

As used herein, a DNA construct is a single- or double-stranded, linear or circular DNA molecule that contains segments of DNA combined and juxtaposed in a manner not found in nature. DNA constructs exist as a result of human manipulation, and include clones and other copies of manipulated molecules.

As used herein, a DNA segment is a portion of a larger DNA molecule having specified attributes. For example, a DNA segment encoding a specified polypeptide is a portion of a longer DNA molecule, such as a plasmid or plasmid fragment, which, when read from the 5' to 3' direction, encodes the sequence of amino acids of the specified polypeptide.

As used herein, the term polynucleotide means a single- or double-stranded polymer of deoxyribonucleotides or ribonucleotide bases read from the 5' to the 3' end. Polynucleotides include RNA and DNA, and can be isolated from natural sources, synthesized *in vitro,* or prepared from a combination of natural and synthetic molecules. The length of a polynucleotide molecule is given herein in terms of nucleotides (abbreviated "nt"), or base pairs (abbreviated "bp"). The term nucleotides is used for single- and double-stranded molecules where the context permits. When the term is applied to double-stranded molecules, it is used to denote overall length and will be understood to be equivalent to the term base pairs. It will be recognized by those skilled in the art that the two strands of a double-stranded polynucleotide can differ slightly in length and that the ends thereof can be staggered; thus, all nucleotides within a double-stranded polynucleotide molecule cannot be paired. Such unpaired ends will, in general, not exceed 20 nucleotides in length.

As used herein, production by recombinant methods refers to the use of the well-known methods of molecular biology for expressing proteins encoded by cloned DNA.

As used herein, "heterologous nucleic acid" is nucleic acid that encodes products (*i.e.*, RNA and/or proteins) that are not normally produced *in vivo* by the cell in which it is expressed, or nucleic acid that is in a locus in which it does not normally occur, or that mediates or encodes mediators that alter expression of endogenous nucleic acid, such as DNA, by affecting transcription, translation, or other regulatable biochemical processes. Heterologous nucleic acid, such as DNA, also is referred to as foreign nucleic acid. Any nucleic acid, such as DNA, that one of skill in the art would recognize or consider as heterologous or foreign to the cell in which it is expressed, is herein encompassed by heterologous nucleic acid; heterologous nucleic acid includes exogenously added nucleic acid that is also expressed endogenously. Heterologous nucleic acid is generally not endogenous to the cell into which it is introduced, but has been obtained from another cell, or prepared synthetically, or is introduced into a genomic locus in which it does not occur naturally, or its expression is under the control of regulatory sequences or a sequence that differs from the natural regulatory sequence or sequences.

Examples of heterologous nucleic acid herein include, but are not limited to, nucleic acid that encodes a protein in a DNA/RNA sensor pathway or a gain-of-function or constitutively active variant thereof, or an immunostimulatory protein, such as a cytokine, chemokine or co-stimulatory molecule, that confers or contributes to anti-tumor immunity in the tumor microenvironment. Other products, such as antibodies and fragments thereof, BiTEs^{®}, decoy receptors, antagonizing polypeptides and RNAi, that confer or contribute to anti-tumor immunity in the tumor microenvironment, also are included. In the immunostimulatory bacteria, the heterologous nucleic acid generally is encoded on the introduced plasmid, but it can be introduced into the genome of the bacterium, such as a promoter that alters expression of a bacterial product. Heterologous nucleic acid, such as DNA, includes nucleic acid that can, in some manner, mediate expression of DNA that encodes a therapeutic product, or it can encode a product, such as a peptide or RNA, that in some manner mediates, directly or indirectly, expression of a therapeutic product.

As used herein, cell therapy involves the delivery of cells to a subject to treat a disease or condition. The cells, which can be allogeneic or autologous to the subject, are modified *ex vivo*, such as by infection of cells with immunostimulatory bacteria provided herein, so that they deliver or express products when introduced to a subject.

As used herein, genetic therapy involves the transfer of heterologous nucleic acid, such as DNA, into certain cells, such as target cells, of a mammal, particularly a human, with a disorder or condition for which such therapy is sought. The nucleic acid, such as DNA, is introduced into the selected target cells in a manner such that the heterologous nucleic acid, such as DNA, is expressed, and a therapeutic product(s) encoded thereby is (are) produced. Genetic therapy can also be used to deliver nucleic acid encoding a gene product that replaces a defective gene or supplements a gene product produced by the mammal or the cell in which it is introduced. The introduced nucleic acid can encode a therapeutic compound, such as a growth factor or inhibitor thereof, or a tumor necrosis factor or inhibitor thereof, such as a receptor thereof, that is not normally produced in the mammalian host or that is not produced in therapeutically effective amounts or at a therapeutically useful time. The heterologous nucleic acid, such as DNA, encoding the therapeutic product, can be modified prior to introduction into the cells of the afflicted host in order to enhance or otherwise alter the product or expression thereof. Genetic therapy can also involve delivery of an inhibitor or repressor or other modulator of gene expression.

As used herein, "expression" refers to the process by which polypeptides are produced by transcription and translation of polynucleotides. The level of expression of a polypeptide can be assessed using any method known in art, including, for example, methods of determining the amount of the polypeptide produced from the host cell. Such methods can include, but are not limited to, quantitation of the polypeptide in the cell lysate by ELISA, Coomassie blue staining following gel electrophoresis, Lowry protein assay, and Bradford protein assay.

As used herein, a "host cell" is a cell that is used to receive, maintain, reproduce and/or amplify a vector. A host cell also can be used to express the polypeptide encoded by the vector. The nucleic acid contained in the vector is replicated when the host cell divides, thereby amplifying the nucleic acid.

As used herein, a "vector" is a replicable nucleic acid from which one or more heterologous proteins can be expressed when the vector is transformed into an appropriate host cell. Reference to a vector includes those vectors into which a nucleic acid encoding a polypeptide or fragment thereof can be introduced, typically by restriction digest and ligation. Reference to a vector also includes those vectors that contain nucleic acid encoding a polypeptide, such as a modified anti-CTLA-4 antibody. The vector is used to introduce the nucleic acid encoding the polypeptide into the host cell for amplification of the nucleic acid, or for expression/display of the polypeptide encoded by the nucleic acid. The vectors typically remain episomal, but can be designed to effect integration of a gene or portion thereof into a chromosome of the genome. Also contemplated are vectors that are artificial chromosomes, such as yeast artificial chromosomes and mammalian artificial chromosomes. Selection and use of such vehicles are well-known to those of skill in the art. A vector also includes "virus vectors" or "viral vectors." Viral vectors are engineered viruses that are operatively linked to exogenous genes to transfer (as vehicles or shuttles) the exogenous genes into cells.

As used herein, an "expression vector" includes vectors capable of expressing DNA that is operatively linked with regulatory sequences, such as promoter regions, that are capable of effecting expression of such DNA fragments. Such additional segments can include promoter and terminator sequences, and optionally can include one or more origins of replication, one or more selectable markers, an enhancer, a polyadenylation signal, and the like. Expression vectors are generally derived from plasmid or viral DNA, or can contain elements of both. Thus, an expression vector refers to a recombinant DNA or RNA construct, such as a plasmid, a phage, recombinant virus or other vector that, upon introduction into an appropriate host cell, results in expression of the cloned DNA. Appropriate expression vectors are well-known to those of skill in the art and include those that are replicable in eukaryotic cells and/or prokaryotic cells and those that remain episomal or those which integrate into the host cell genome.

As used herein, "primary sequence" refers to the sequence of amino acid residues in a polypeptide, or the sequence of nucleotides in a nucleic acid molecule.

As used herein, "sequence identity" refers to the number of identical or similar amino acids or nucleotide bases in a comparison between a test and a reference polypeptide or polynucleotide. Sequence identity can be determined by sequence alignment of nucleic acid or protein sequences to identify regions of similarity or identity. For purposes herein, sequence identity is generally determined by alignment to identify identical residues. The alignment can be local or global. Matches, mismatches and gaps can be identified between compared sequences. Gaps are null amino acids or nucleotides inserted between the residues of aligned sequences so that identical or similar characters are aligned. Generally, there can be internal and terminal gaps. When using gap penalties, sequence identity can be determined with no penalty for end gaps (*e.g*., terminal gaps are not penalized). Alternatively, sequence identity can be determined without taking into account gaps, as the number of identical positions/length of the total aligned sequence x 100.

As used herein, a "global alignment" is an alignment that aligns two sequences from beginning to end, aligning each letter in each sequence only once. An alignment is produced, regardless of whether or not there is similarity or identity between the sequences. For example, 50% sequence identity based on "global alignment" means that in an alignment of the full sequence of two compared sequences each of 100 nucleotides in length, 50% of the residues are the same. It is understood that global alignment also can be used in determining sequence identity even when the length of the aligned sequences is not the same. The differences in the terminal ends of the sequences will be taken into account in determining sequence identity, unless the "no penalty for end gaps" is selected. Generally, a global alignment is used on sequences that share significant similarity over most of their length. Exemplary algorithms for performing global alignment include the Needleman-Wunsch algorithm (Needleman et al. (1970) J. Mol. Biol. 48:443-453). Exemplary programs for performing global alignment are publicly available and include the Global Sequence Alignment Tool available at the National Center for Biotechnology Information (NCBI) website (ncbi.nlm.nih.gov/), and the program available at deepc2.psi.iastate.edu/aat/align/align.html.

As used herein, a "local alignment" is an alignment that aligns two sequences, but only aligns those portions of the sequences that share similarity or identity. Hence, a local alignment determines if sub-segments of one sequence are present in another sequence. If there is no similarity, no alignment will be returned. Local alignment algorithms include BLAST or the Smith-Waterman algorithm (*Adv. Appl. Math.* 2:482 (1981)). For example, 50% sequence identity based on "local alignment" means that in an alignment of the full sequence of two compared sequences of any length, a region of similarity or identity of 100 nucleotides in length has 50% of the residues that are the same in the region of similarity or identity.

For purposes herein, sequence identity can be determined by standard alignment algorithm programs used with default gap penalties established by each supplier. Default parameters for the GAP program can include: (1) a unary comparison matrix (containing a value of 1 for identities and 0 for non-identities) and the weighted comparison matrix of Gribskov et al. (1986) Nucl. Acids Res. 14:6745-6763, as described by Schwartz and Dayhoff, eds., Atlas of Protein Sequence and Structure, National Biomedical Research Foundation, pp. 353-358 (1979); (2) a penalty of 3.0 for each gap and an additional 0.10 penalty for each symbol in each gap; and (3) no penalty for end gaps. Whether any two nucleic acid molecules have nucleotide sequences, or any two polypeptides have amino acid sequences that are at least 80%, 85%, 90%, 95%, 96%, 97%, 98%, or 99% "identical," or other similar variations reciting a percent identity, can be determined using known computer algorithms based on local or global alignment (see, *e.g.,* wikipedia.org/wiki/Sequence_alignment_software, providing links to dozens of known and publicly available alignment databases and programs). Generally, for purposes herein sequence identity is determined using computer algorithms based on global alignment, such as the Needleman-Wunsch Global Sequence Alignment tool available from NCBI/BLAST (blast.ncbi.nlm.nih.gov/Blast.cgi?CMD=Web&Page_TYPE=BlastHome); LAlign (William Pearson implementing the Huang and Miller algorithm (Adv. Appl. Math. (1991) 12:337-357)); and the program from Xiaoqui Huang, available at deepc2.psi.iastate.edu/aat/align/align.html. Typically, the full-length sequence of each of the compared polypeptides or nucleotides is aligned across the full-length of each sequence in a global alignment. Local alignment also can be used when the sequences being compared are substantially the same length.

Therefore, as used herein, the term "identity" represents a comparison or alignment between a test and a reference polypeptide or polynucleotide. In one nonlimiting example, "at least 90% identical to" refers to percent identities from 90% to 100% relative to the reference polypeptide or polynucleotide. Identity at a level of 90% or more is indicative of the fact that, assuming for exemplification purposes a test and reference polypeptide or polynucleotide length of 100 amino acids or nucleotides are compared, no more than 10% (*i.e.*, 10 out of 100) of amino acids or nucleotides in the test polypeptide or polynucleotide differ from those of the reference polypeptide or polynucleotide. Similar comparisons can be made between a test and reference polynucleotide. Such differences can be represented as point mutations randomly distributed over the entire length of an amino acid sequence, or they can be clustered in one or more locations of varying length up to the maximum allowable, *e.g.,* 10/100, amino acid differences (approximately 90% identity). Differences also can be due to deletions or truncations of amino acid residues. Differences are defined as nucleic acid or amino acid substitutions, insertions, or deletions. Depending on the length of the compared sequences, at the level of homologies or identities above about 85-90%, the result can be independent of the program and gap parameters set; such high levels of identity can be assessed readily, often without relying on software.

As used herein, a "disease or disorder" refers to a pathological condition in an organism resulting from a cause or condition, including, but not limited to, infections, acquired conditions, and genetic conditions, and that is characterized by identifiable symptoms.

As used herein, "treating" a subject with a disease or condition means that the subject's symptoms are partially or totally alleviated, or remain static following treatment.

As used herein, "treatment" refers to any effects that ameliorate symptoms of a disease or disorder. Treatment encompasses prophylaxis, therapy and/or cure. Treatment also encompasses any pharmaceutical use of any immunostimulatory bacterium or composition provided herein.

As used herein, "prophylaxis" refers to prevention of a potential disease and/or a prevention of worsening of symptoms or of progression of a disease.

As used herein, "prevention" or prophylaxis, and grammatically equivalent forms thereof, refers to methods in which the risk or probability of developing a disease or condition is reduced.

As used herein, a "pharmaceutically effective agent" includes any therapeutic agent or bioactive agent, including, but not limited to, for example, anesthetics, vasoconstrictors, dispersing agents, and conventional therapeutic drugs, including small molecule drugs and therapeutic proteins.

As used herein, a "therapeutic effect" means an effect resulting from treatment of a subject that alters, typically improves or ameliorates, the symptoms of a disease or condition, or that cures a disease or condition.

As used herein, a "therapeutically effective amount" or a "therapeutically effective dose" refers to the quantity of an agent, compound, material, or composition containing a compound that is at least sufficient to produce a therapeutic effect following administration to a subject. Hence, it is the quantity necessary for preventing, curing, ameliorating, arresting, or partially arresting a symptom of a disease or disorder.

As used herein, "therapeutic efficacy" refers to the ability of an agent, compound, material, or composition containing a compound to produce a therapeutic effect in a subject to whom the agent, compound, material, or composition containing a compound has been administered.

As used herein, a "prophylactically effective amount" or a "prophylactically effective dose" refers to the quantity of an agent, compound, material, or composition containing a compound that, when administered to a subject, will have the intended prophylactic effect, *e.g*., preventing or delaying the onset or reoccurrence, of disease or symptoms, reducing the likelihood of the onset or reoccurrence, of disease or symptoms, or reducing the incidence of viral infection. The full prophylactic effect does not necessarily occur by administration of one dose, and can occur only after administration of a series of doses. Thus, a prophylactically effective amount can be administered in one or more administrations.

As used herein, amelioration of the symptoms of a particular disease or disorder by a treatment, such as by administration of a pharmaceutical composition or other therapeutic, refers to any lessening, whether permanent or temporary, lasting or transient, of the symptoms, that can be attributed to or associated with administration of the composition or therapeutic.

As used herein, an "anti-cancer agent" or "an anti-cancer therapeutic" refers to any agent or therapeutic that is destructive or toxic, either directly or indirectly, to malignant cells and tissues. For example, anti-cancer agents include agents that kill cancer cells or otherwise inhibit or impair the growth of tumors or cancer cells. Exemplary anti-cancer agents are chemotherapeutic agents, and immunotherapeutic agents.

As used herein, "therapeutic activity" refers to the *in vivo* activity of a therapeutic product, such as a polypeptide, a nucleic acid molecule, and other therapeutic molecules. Generally, the therapeutic activity is the activity that is associated with treatment of a disease or condition.

As used herein, the term "subject" refers to an animal, including a mammal, such as a human being.

As used herein, a patient refers to a human subject.

As used herein, "animal" includes any animal, such as, but not limited to, primates, including humans, gorillas and monkeys; rodents, such as mice and rats; fowl, such as chickens; ruminants, such as goats, cows, deer, and sheep; and pigs and other animals. Non-human animals exclude humans as the contemplated animal. The polypeptides provided herein are from any source, animal, plant, prokaryotic and fungal. Most polypeptides are of animal origin, including mammalian origin.

As used herein, a "composition" refers to any mixture. It can be a solution, suspension, liquid, powder, paste, aqueous, non-aqueous, or any combination thereof.

As used herein, a "combination" refers to any association between or among two or more items. The combination can be two or more separate items, such as two compositions or two collections, a mixture thereof, such as a single mixture of the two or more items, or any variation thereof. The elements of a combination are generally functionally associated or related.

As used herein, "combination therapy" refers to administration of two or more different therapeutics. The different therapeutic agents can be provided and administered separately, sequentially, intermittently, or can be provided in a single composition.

As used herein, a "kit" is a packaged combination that optionally includes other elements, such as additional reagents and instructions for use of the combination or elements thereof, for a purpose including, but not limited to, activation, administration, diagnosis, and assessment of a biological activity or property.

As used herein, a "unit dose form" refers to physically discrete units suitable for human and animal subjects and packaged individually, as is known in the art.

As used herein, a "single dosage formulation" refers to a formulation for direct administration.

As used herein, a "multi-dose formulation" refers to a formulation that contains multiple doses of a therapeutic agent and that can be directly administered to provide several single doses of the therapeutic agent. The doses can be administered over the course of minutes, hours, weeks, days, or months. Multi-dose formulations can allow dose adjustment, dose-pooling and/or dose-splitting. Because multi-dose formulations are used over time, they generally contain one or more preservatives to prevent microbial growth.

As used herein, an "article of manufacture" is a product that is made and sold. As used throughout this application, the term is intended to encompass any of the compositions provided herein contained in articles of packaging.

As used herein, a "fluid" refers to any composition that can flow. Fluids thus encompass compositions that are in the form of semi-solids, pastes, solutions, aqueous mixtures, gels, lotions, creams, and other such compositions.

As used herein, an isolated or purified polypeptide or protein (*e.g.*, an isolated antibody or antigen-binding fragment thereof) or a biologically-active portion thereof (*e.g.*, an isolated antigen-binding fragment), is substantially free of cellular material or other contaminating proteins from the cell or tissue from which the polypeptide or protein is derived, or substantially free from chemical precursors or other chemicals when chemically synthesized. Preparations can be determined to be substantially free if they appear free of readily detectable impurities as determined by standard methods of analysis, such as thin layer chromatography (TLC), gel electrophoresis and high performance liquid chromatography (HPLC), that are used by those of skill in the art to assess such purity, or are sufficiently pure such that further purification does not detectably alter the physical and chemical properties, such as enzymatic and biological activities, of the substance. Methods for purification of the compounds to produce substantially chemically pure compounds are known to those of skill in the art. A substantially chemically pure compound, however, can be a mixture of stereoisomers. In such instances, further purification might increase the specific activity of the compound.

As used herein, a "cellular extract" or "lysate" refers to a preparation or fraction which is made from a lysed or disrupted cell.

As used herein, a "control" refers to a sample that is substantially identical to the test sample, except that it is not treated with a test parameter, or, if it is a plasma sample, it can be from a normal volunteer not affected with the condition of interest. A control also can be an internal control.

As used herein, the singular forms "a," "an" and "the" include plural referents unless the context clearly dictates otherwise. Thus, for example, reference to a polypeptide, comprising "an immunoglobulin domain" includes polypeptides with one or a plurality of immunoglobulin domains.

As used herein, the term "or" is used to mean "and/or" unless explicitly indicated to refer to alternatives only, or the alternatives are mutually exclusive.

As used herein, ranges and amounts can be expressed as "about" a particular value or range. "About" also includes the exact amount. Hence, "about 5 amino acids" means "about 5 amino acids" and also "5 amino acids."

As used herein, "optional" or "optionally" means that the subsequently described event or circumstance does or does not occur, and that the description includes instances where said event or circumstance occurs and instances where it does not. For example, an optionally variant portion means that the portion is variant or non-variant.

As used herein, the abbreviations for any protective groups, amino acids and other compounds, are, unless indicated otherwise, in accord with their common usage, recognized abbreviations, or the IUPAC-IUB Commission on Biochemical Nomenclature (see, Biochem. (1972) 11(9):1726-1732).

For clarity of disclosure, and not by way of limitation, the detailed description is divided into the subsections that follow.

### B. OVERVIEW OF IMMUNOSTIMULATORY BACTERIA FOR CANCER THERAPY

The recognition that bacteria have anti-cancer activity goes back to the 1800s, when several physicians observed the regression of tumors in patients infected with *Streptococcus pyogenes.* William Coley began the first study utilizing bacteria for the treatment of end-stage cancers, and developed a vaccine composed of *S. pyogenes* and *Serratia marcescens*, which was successfully used to treat a variety of cancers, including sarcomas, carcinomas, lymphomas and melanomas. Since then, a number of bacterial species, including *Clostridium*, *Mycobacterium*, *Bifidobacterium*, *Listeria monocytogenes and Escherichia*, have been studied as sources of anti-cancer vaccines (See, *e.g*., International PCT Application Publication Nos. WO 1999/013053 and WO 2001/025399; Bermudes et al. (2002) Curr. Opin. Drug Discov. Devel. 5:194-199; Patyar et al. (2010) Journal of Biomedical Science 17:21; and Pawlek et al. (2003) Lancet Oncol. 4:548-556).

As a therapeutic platform, bacteria have several advantages over other therapies such as oncolytic viruses. Some bacterial species can be engineered to be orally and systemically (intravenously; IV) administered, they propagate readily *in vitro* and *in vivo,* and they can be stored and transported in a lyophilized state. Bacterial chromosomes readily can be manipulated as they lack exons, and the complete genomes for numerous strains have been fully characterized (Felgner et al. (2016) mBio 7(5):e01220-16). Many types of bacteria are cheaper and easier to produce than viruses, and proper delivery of engineered bacteria can be favorable over viral delivery because they do not permanently integrate into host cell genomes, they preferentially infect myeloid cells over epithelial cells, and they can be rapidly eliminated by antibiotics if necessary, rendering them safe.

Provided herein are immunostimulatory bacteria that are modified to exploit these advantageous properties. The bacteria provided herein are modified so that they infect and accumulate in the tumor microenvironment, particularly in tumor-resident immune cells (myeloid cells), such as tumor-associated macrophages (TAMs), dendritic cells (DCs), and myeloid-derived suppressor cells (MDSCs), and also are designed to express and deliver high levels of therapeutic proteins and combinations, particularly complementary combinations, thereof. The immunostimulatory bacteria provided herein have advantageous properties that are superior to existing bacterial therapies, and also cell therapies, oncolytic virus therapies, and prior bacterial therapies. The immunostimulatory bacteria provided herein, while they can be administered by any suitable route, are suitable for systemic, such as intravenous, administration. As shown and described herein, the immunostimulatory bacteria provided herein can target major immune pathways.

The bacteria provided herein are designed and engineered to maintain the beneficial scaffold properties of bacteria, and to have a viral-like immune signature. This is advantageous for use as an anti-cancer therapeutic. The following table summarizes some of the immune and scaffold properties of bacteria and viruses; the immunostimulatory bacteria provided herein retain the feasibility of the bacterial scaffold, but result in a viral-like immune response in a treated subject (discussed in more detail in section C below).

| | **Bacteria** | **Viruses** | **Immunostimulatory Bacteria Provided Herein** |
|---|---|---|---|
| **Feasibility as a Therapeutic Scaffold** | Easy to manufacture; | Difficult to manufacture; | Engineered to retain and improve feasibility properties |
| | Stable Shelf Life; | Requires -80 °C for strorage; | |
| | Easy to engineer; | | |
| | Reversible with antibiotics; | Can be difficult to engineer; Immunogenic; | |
| | Not immunogenic | Complement can inactivate | |
| **Inflammatory Profile** | Recognized by TLR2, TLR4, and TLR5; Downstream targets suppress adaptive immunity | Recognized by TLR3, TLR7/8, and STING; Downstream targets promote adaptive immunity | Engineered to produce a viral-like immune response |
| **Chemokine Gradients** | Attract neutrophils to clear infection | Attract T-cells to clear infection | |
| **Generation of durable immunity** | No | Yes, but only to the virus | |

In *Salmonella* species and other bacterial species, the flagella contribute to TRL5-mediated inflammation, the LPS results in TLR4-mediated inflammatory responses, and the adhesive curli fimbriae result in TLR2-mediated inflammatory responses. The genomes of the immunostimulatory bacteria provided herein are modified so that the bacteria lack flagella and adhesive curli fimbriae, and have modified LPS, resulting in the reduction or elimination of TLR4-mediated inflammatory responses. As a result, the immunostimulatory bacteria provided herein induce a viral-like anti-tumor immune response. Elimination or modification of these components confers other advantageous properties, such as those discussed in detail below. The immunostimulatory bacteria deliver therapeutic products, such as anti-cancer therapeutics, and particularly, complementary combinations of products. The immunostimulatory bacteria provided herein deliver encoded genetic payloads in a tumor-specific manner to tumor-resident myeloid cells.

Provided is an anti-cancer therapeutic product, an immunostimulatory bacterium, that delivers a genetic payload encoding one or a plurality of therapeutic products. Included is a truncated co-stimulatory molecule (receptor or ligand; *e.g.*, 4-1BBL, CD80, CD86, CD27L, B7RP1, OX40L), with a complete or a partial cytoplasmic domain deletion, for expression on an antigen presenting cell (APC), where the truncated gene product is capable of constitutive immunostimulatory signaling to a T-cell through co-stimulatory receptor engagement, and is unable to counter-regulatory signal to the APC due to a truncated or deleted cytoplasmic domain.

The immunostimulatory bacteria can encode and express one or more of IL-2, IL-7, IL-12p70 (IL-12p40 + IL-12p35), IL-12, IL-15, IL-15/IL-15Rα chain complex, IL-18, IL-21, IL-23, IL-36y, interferon-α, interferon-β, IL-2 that has attenuated binding to IL-2Ra, IL-2 that is modified so that it does not bind to IL-2Ra, CXCL9, CXCL10, CXCL11, CCL3, CCL4, CCL5, cytosolic DNA/RNA sensors or type I IFN pathway proteins, such as gain-of-function or constitutively active STING, IRF3, IRF7, MDA5, or RIG-I variants (that induce type I IFN), inhibitors of TGF-beta, such as TGF-β inhibitory antibodies, TGF-beta polypeptide antagonists, and TGF-beta binding decoy receptors, antibodies and fragments thereof, such as those targeting immune checkpoints and other anti-cancer targets, such as VEGF and IL-6, co-stimulatory receptors/molecules, such as 4-1BBL, including 4-1BBL with the cytoplasmic domain deleted or truncated or otherwise eliminated, and others. The immunostimulatory bacteria also can encode and express a truncated co-stimulatory molecule (*e.g*., 4-1BBL, CD80, CD86, CD27L, B7RP1, OX40L), with a partial or complete cytoplasmic domain deletion, for expression on an antigen-presenting cell (APC), where the truncated gene product is capable of constitutive immuno-stimulatory signaling to a T-cell through co-stimulatory receptor engagement, and is unable to counter-regulatory signal to the APC, due to a deleted or truncated cytoplasmic domain. Combinations of such therapeutic products and agents can be expressed in a single therapeutic composition. By virtue of the modifications of the bacterial genome, the immunostimulatory bacteria exhibit tumor-specific localization and enrichment, and provide intravenous (IV) administration for activation of anti-tumor immune pathways that are otherwise toxic if systemically activated.

The immunostimulatory bacteria provided herein are genetically designed to be safe and to target tumors, the tumor microenvironment, and/or tumor-resident immune cells. The immunostimulatory bacteria provided herein include a combination of genomic modifications and other modifications, as well as encoded therapeutic products, that function in concert to provide immunostimulatory bacteria that accumulate in tumor-resident immune cells and that persist sufficiently long to deliver therapeutic products, particularly combinations that induce or promote anti-cancer immune stimulation in tumors and the tumor microenvironment, without toxic side-effects, or with limited toxic side-effects. When delivered systemically, such as intravenously (IV), the immunostimulatory bacteria enrich in tumors, including in metastatic lesions; they provide efficient genetic transfer of immune payloads, specifically to tumor-resident myeloid cells, including tumor-associated macrophages (TAMs), myeloid-derived suppressor cells (MDSCs), and dendritic cells (DCs); they induce powerful, local immune responses, destroying tumors and vaccinating against future recurrence; and, when therapy is finished, they are naturally eliminated, such as by phagocytosis and destruction by the infected cells, or they can be destroyed rapidly by a course of antibiotics.

The immunostimulatory bacteria provided herein exhibit preferential accumulation in the tumor microenvironment and/or in tumor-resident immune cells due to a designed purine/adenosine auxotrophy, and exhibit an inability to replicate inside of phagocytic cells. Immunostimulatory bacteria that avoid inactivation by serum complement allow for the delivery of a variety of immunotherapeutic agents and therapeutic products at high concentrations, directly within the tumor microenvironment, while minimizing toxicity to normal tissues, and are provided herein.

For example, as described in more detail in section C.8., the immunostimulatory bacteria provided herein include modifications of the genome that render them *msbB⁻*/*pagP*⁻, which alters the lipid A in LPS, resulting in penta-acylation (wild-type lipid A has 6-7 fatty acid chains), reducing the TLR4 affinity; are adenonsine/adenine auxotrophs, such as *purI*⁻; are asparaginase II⁻ (*ansB*⁻), which improves T-cell quality; are *csgD*⁻, which, among other properties, removes curli fimbriae; and include other optional genomic modifications, such as insertions, deletions, disruptions, and any other modification, so that the encoded product(s) is(are) not produced in active form, as discussed in detail herein. The immunostimulatory bacteria include a plasmid that encodes one or more therapeutic products, particularly anti-cancer products, under control of a eukaryotic promoter.

The immunostimulatory bacteria provided herein, that deliver therapeutic products (such as constitutively active STING variants and other immunomodulatory proteins and products), to the tumor-resident myeloid cells promote adaptive immunity and enhance T-cell function. The immunostimulatory bacteria lead to a complete remodeling of the immunosuppressive tumor microenvironment, towards an adaptive anti-tumor phenotype, and away from a bacterial phenotype, which is characterized by the promotion of innate immunity and the suppression of adaptive immunity.

The immunostimulatory bacteria provided herein include genomic modifications whereby they target or accumulate in tumor-resident immune cells, particularly tumor-resident myeloid cells, such as macrophages, MDSCs (myeloid derived suppressor cells), and DCs (dendritic cells), in which they deliver payloads of encoded therapeutic products expressed under control of regulatory sequences recognized by the hose cell (eukaryotic) transcriptional/translational machinery. The encoded products are expressed in the myeloid cells, and, as appropriate, delivered into the tumor microenvironment. The bacteria generate anti-tumor immunity, and also can deliver anti-tumor products that directly treat tumors, and products that can activate prodrugs.

Immunostimulatory bacteria provided herein can exhibit at least about 100,000-fold greater tumor infiltration and enrichment compared to unmodified bacteria. The immunostimulatory bacteria are consumed by tumor-resident immune cells, and deliver the plasmid encoding therapeutic products, which are expressed and produced in the immune cells and tumor microenvironment, to generate anti-tumor immunity.

### 1. Bacterial Cancer Immunotherapy

Many solid tumor types have evolved a profoundly immunosuppressive microenvironment that renders them highly refractory to approved checkpoint therapies, such as anti-CTLA-4, anti-PD-1 and anti-PD-L1 therapies. One mechanism by which tumors have evolved resistance to checkpoint therapies is through their lack of intratumoral T-cells and tumor antigen cross-presenting dendritic cells (DCs), described as T-cell excluded, non-inflamed, or "cold tumors" (Sharma et al. (2017) Cell 168(4):707-723). For the small number of patients whose tumors are T-cell inflamed and respond to checkpoint immunotherapies, they often experience severe autoimmune toxicities, and many will eventually relapse and become checkpoint refractory (see, *e.g.,* Buchbinder et al. (2015) J. Clin. Invest. 125:3377-3383; Hodi et al. (2010) N. Engl. J. Med. 363(8):711-723; and Chen et al. (2015) J. Clin. Invest. 125:3384-3391). Tumors initiate multiple mechanisms to evade immune surveillance, reprogram anti-tumor immune cells to suppress immunity, exclude and inactivate anti-tumor T-cells, and develop emerged resistance to the targeted cancer therapies (see, *e.g.,* Mahoney et al. (2015) Nat. Rev. Drug Discov. 14(8):561-584). Solving this problem will require immunotherapies that can properly inflame these tumors, and generate anti-tumor immunity that can provide long-lasting tumor regressions. In addition, intratumoral therapies are intractable and will be quite limiting in a metastatic disease setting. Systemically-administered therapies that properly inflame each individual metastatic lesion and overcome multiple pathways of immunosuppression are required. By virtue of their ability to specifically target tumor-resident immune cells, and to express multiple complementary genetic payloads/therapeutic products, the immunostimulatory bacteria provided herein are designed to address these issues.

### 2. Prior Therapies that Target the Tumor Microenvironment

A number of therapies that target the tumor microenvironment (TME) and attempt to promote anti-tumor immunity have been developed. Each has its own challenges and shortcomings, which are addressed by the immunostimulatory bacteria provided herein.

### a. Limitations of Autologous T-cell Therapies

Several systemically-administered therapeutic platforms have been investigated clinically, with the goal of accessing the highly immunosuppressive tumor microenvironment and inducing the proper immune responses to inflame tumors and promote anti-tumor immunity. These platforms include chimeric antigen receptors T-cells (CAR-T cells), which are produced by harvesting T-cells from patients and re-engineering them to fuse the T-cell receptor to an antibody Ig variable extracellular domain specific for a particular tumor antigen. This confers upon the cells the antigen-recognition properties of antibodies, with the cytolytic properties of activated T-cells (see, *e.g.,* Sadelain et al. (2015) J. Clin. Invest. 125(9):3392-3400). Despite the promise and potency of this technology, such as the FDA approvals of the CD19 CAR-Ts tisagenlecleucel (such as those under the trademark Kymriah^{®}) and axicabtagene ciloleucel (under the trademark Yescarta^{®}), success has been limited to CD19⁺ hematopoietic malignancies, and at the cost of deadly immune-related adverse events (see, *e.g.,* Jackson et al. (2016) Nat. Rev. Clin. Oncol. 13(6):370-383). Tumors can mutate rapidly to downregulate the targeted tumor antigens for solid tumors, including the antigen CD19, thereby fostering immune escape (see, *e.g.,* Mardiana et al. (2019) Sci. Transl. Med. 11(495):eaaw2293). There is not a plethora of tumor-specific target antigens. Solid tumor targets that are not expressed in healthy tissue are a major impediment to CAR-T therapy. Beyond that, CAR-T therapies suffer from other impediments to accessing solid tumor microenvironments, due to the lack of sufficient T-cell chemokine gradients, which are required for proper T-cell infiltration into tumors. In addition, once they have infiltrated tumors, they are rapidly inactivated (see, *e.g.,* Brown et al. (2019) Nat. Rev. Immunol. 19(2):73-74). Should the safety of CAR-T cells be significantly improved and the efficacy expanded to solid tumors, the feasibility and costs associated with these labor-intensive therapies still limit their broader adoption.

### b. Viral Vaccine Platforms

Oncolytic viruses (OVs) have natural and engineered properties to induce tumor cell lysis, recruit T-cells to the tumor, and deliver genetic material that can be read by tumor cells to produce immunomodulatory proteins. For example, the oncolytic virus designated Talimogene laherparepvec (T-VEC), is a modified herpes simplex virus encoding anti-melanoma antigens and the cytokine GM-CSF (granulocyte-macrophage colony-stimulating factor), that is intratumorally administered. It is FDA-approved for metastatic melanoma (see, *e.g.,* Bastin et al. (2016) Biomedicines 4(3):21). T-VEC has demonstrated clinical benefit for some melanoma patients, and with fewer immune toxicities than the immune checkpoint antibodies or the FDA-approved systemic cytokines, such as IL-2 and interferon-alpha (see, *e.g.,* Kim et al. (2006) Cytokine Growth Factor Rev. 17(5):349-366; and Paul et al. (2015) Gene 567(2):132-137).

Oncolytic viruses (OVs) possess a number of limitations as anti-cancer therapies. First, oncolytic viruses are rapidly inactivated by the human complement system in blood. It has proven difficult to deliver enough virus through systemic administration to have a desired therapeutic effect. Intratumoral delivery is limiting in a metastatic setting (where lesions are spread throughout the body), is intractable for most solid tumor types (*e.g*., lung and visceral lesions), and requires interventional, guided radiology for injection, which limits repeat dosing. Viruses can be difficult to manufacture at commercial scale and to store. Most OV-based vaccines, such as those based on paramyxovirus, reovirus and picornavirus, among others, have similar limitations (see, *e.g.,* Chiocca et al. (2014) Cancer Immunol. Res. 2(4):295-300). Oncolytic viruses are inherently immunogenic and rapidly cleared from human blood, and T-cells that traffic into the tumor have a much higher affinity for viral antigens over weaker tumor neoantigens (see, *e.g.,* Aleksic et al. (2012) Eur. J. Immunol. 42(12):3174-3179). Thus, in addition to the recognized technical limitations of the platform, OVs thus far have limited capacity to stimulate durable anti-tumor immunity.

### c. Bacterial Cancer Therapies

A number of bacterial species have demonstrated preferential replication within solid tumors when injected from a distal site in preclinical animal studies. These include, but are not limited to, species of *Salmonella*, *Bifodobacterium, Clostridium*, and *Escherichia.* The tumor-homing properties of the bacteria, combined with the host's innate immune response to the bacterial infection, can mediate an anti-tumor response. This tumor tissue tropism reduces the size of tumors to varying degrees. One contributing factor to the tumor tropism of these bacterial species is the ability to replicate in anoxic and hypoxic environments. A number of these naturally tumor-tropic bacteria have been further engineered to increase the potency of the anti-tumor response (reviewed in Zu et al. (2014) Crit. Rev. Microbiol. 40(3):225-235; and Felgner et al. (2017) Microbial Biotechnology 10(5):1074-1078). Despite proof-of-concept in animal studies, complement factors in human serum, that are not present in animal models, can inactivate the bacteria, limiting their use as therapies to treat cancer.

To be administered orally or systemically, the bacterial strains are attenuated so that they do not cause systemic disease and/or septic shock, but still maintain some level of infectivity for effective tumor colonization, and resistance to inactivation by complement. A number of different bacterial species, including *Clostridium* (see, *e.g.,* Dang et al. (2001) Proc. Natl. Acad. Sci. U.S.A. 98(26):15155-15160; U.S. Patent Publication Nos. 2017/0020931 and 2015/0147315; and U.S. Patent Nos. 7,344,710 and 3,936,354), *Mycobacterium* (see, *e.g.,* U.S. Patent Publication Nos. 2015/0224151 and 2015/0071873), *Bifidobacterium* (see, *e.g.,* Dang *et al.* (2001); and Kimura et al. (1980) Cancer Res. 40:2061-2068), *Lactobacillus* (see, *e.g.,* Dang *et al.* (2001)), *Listeria monocytogenes* (see, *e.g.,* Le et al. (2012) Clin. Cancer Res. 18(3):858-868; Starks et al. (2004) J. Immunol. 173:420-427; and U.S. Patent Publication No. 2006/0051380) and *Escherichia coli* (see, *e.g.,* U.S. Patent No. 9,320,787), have been studied as possible agents for anti-cancer therapy.

The immunostimulatory bacteria provided herein include genome modifications that address problems with prior bacteria developed for treating tumors. The modifications improve the targeting or accumulation of bacteria in the tumor microenvironment, and in particular, are designed so that the bacteria infect tumor-resident immune cells and not healthy tissues, thereby decreasing toxicity and improving delivery of encoded products. The immunostimulatory bacteria also are designed to deliver therapeutic products, including combinations thereof, designed to eliminate immune suppressive effects of tumors, enhance a host's anti-tumor response, and provide anti-tumor products.

### i. Listeria

*Listeria monocytogenes*, a live attenuated intracellular bacterium capable of inducing potent CD8⁺ T-cell priming to expressed tumor antigens in mouse models of cancer, has also been explored as a bacterial cancer vector (see, *e.g.,* Le et al. (2012) Clin. Cancer Res. 18(3):858-868). In a clinical trial of the *L. monocytogenes-*based vaccine incorporating the tumor antigen mesothelin, together with an allogeneic pancreatic cancer-based GVAX vaccine in a prime-boost approach, a median survival of 6.1 months was noted in patients with advanced pancreatic cancer, versus a median survival of 3.9 months for patients treated with the GVAX vaccine alone (see, *e.g.,* Le et al. (2015) J. Clin. Oncol. 33(12):1325-1333). These results were not replicated in a larger phase 2b study, however, pointing to the difficulties in humans of subverting peripheral immune surveillance towards low affinity tumor neoantigens. *L*. *monocytogenes* also has shown limited immune responses to the encoded tumor antigens due to the requirement for bacteria to be lysed after phagocytosis, a prerequisite to efficient plasmid transfer, which has not been demonstrated to occur by *L*. *monocytogenes* in human macrophages.

### ii, Salmonella Species

*Salmonella enterica* serovar Typhimurium (*S. typhimurium*) is exemplary of a bacterial species for use as an anti-cancer therapeutic. *S. typhimurium* is a Gram-negative facultative anaerobe, which preferentially accumulates in hypoxic and necrotic areas due to the availability of nutrients from tissue necrosis, the leaky tumor vasculature, and their increased likelihood to survive in the immunosuppressed tumor microenvironment (see, *e.g.,* Baban et al. (2010) Bioengineered Bugs 1(6):385-394). As a facultative anaerobe, *S. typhimurium* is able to grow under aerobic and anaerobic conditions, and is therefore able to colonize both small tumors that are less hypoxic, and large tumors that are more hypoxic.

*S. typhimurium* transmission through the fecal-oral route causes localized gastrointestinal infections. The bacterium can also enter the bloodstream and lymphatic system, infecting systemic tissues such as the liver, spleen and lungs. Systemic administration of wild-type *S. typhimurium* overstimulates TNF-α and IL-6, leading to a cytokine cascade and septic shock, which, if left untreated, can be fatal. As a result, pathogenic bacterial strains, such as *S. typhimurium,* must be attenuated to prevent systemic infection, without completely suppressing their ability to effectively colonize tumor tissues. Attenuation often is achieved by mutating a cellular structure that can elicit an immune response through pathogen pattern recognition, such as the bacterial outer membrane, or by limiting the bacterium's ability to replicate in the absence of supplemental nutrients.

*S. typhimurium* is an intracellular pathogen that is rapidly taken up by phagocytic myeloid cells such as macrophages, or it can directly invade non-phagocytic cells, such as epithelial cells, through its *Salmonella* pathogenicity island 1 (SPI-1)-encoded type III secretion system (T3SS1). Once inside cells, it can replicate within a *Salmonella-*containing vacuole (SCV) through SPI-2 regulation, and can also escape into the cytosol of some epithelial cells (see, *e.g.,* Agbor et al. (2011) Cell Microbiol. 13(12):1858-1869; and Galan and Wolf-Watz (2006) Nature 444:567-573). Genetically modified bacterial strains of *S. typhimurium* have been described as anti-tumor agents to elicit direct tumoricidal effects and/or to deliver tumoricidal molecules (see, *e.g.,* Clairmont et al. (2000) J. Infect. Dis. 181:1996-2002; Bermudes, D. et al. (2002) Curr. Opin. Drug Discov. Devel. 5:194-199; Zhao, M. et al. (2005) Proc. Natl. Acad. Sci. U.S.A. 102:755-760; and Zhao, M. et al. (2006) Cancer Res. 66:7647-7652).

Various methods for attenuation of bacterial pathogens are known in the art. Auxotrophic mutations, for example, render bacteria incapable of synthesizing an essential nutrient, and deletions/mutations in genes such as *aro*, *pur*, *gua*, *thy*, *nad* and *asd* (see, *e.g.,* U.S. Patent Publication No. 2012/0009153) are used. Nutrients produced by the biosynthesis pathways involving these genes are often unavailable in host cells, and as such, bacterial survival is challenging. For example, attenuation of *Salmonella* and other species can be achieved by deletion or disruption of the *aroA* gene, which is part of the shikimate pathway, connecting glycolysis to aromatic amino acid biosynthesis (see, *e.g.*, Felgner et al. (2016) mBio 7(5):e01220-16). Deletion or disruption of *aroA* results in bacterial auxotrophy for aromatic amino acids and subsequent attenuation (see, *e.g.*, U.S. Patent Publication Nos. 2003/0170276, 2003/0175297, 2012/0009153 and 2016/0369282; and International Application Publication Nos. WO 2015/032165 and WO 2016/025582). Similarly, other enzymes involved in the biosynthesis pathway for aromatic amino acids, including *aroC* and *aroD*, have been deleted to achieve attenuation (see, *e.g.*, U.S. Patent Publication No. 2016/0369282; and International Application Publication No. WO 2016/025582). For example, *S. typhimurium* strain SL7207 is an aromatic amino acid auxotroph (*aroA*⁻ mutant), and strains A1 and A1-R are leucine-arginine auxotrophs.

Mutations that attenuate bacteria also include, but are not limited to, mutations in genes that alter the biosynthesis of lipopolysaccharide (LPS), such as *rfaL*, *rfaG*, *rfaH, rfaD*, *rfaP, rFb, rfa*, *msbB, htrB*, *firA, pagL*, *pagP, lpxR, arnT*, *eptA, and lpxT;* mutations that introduce a suicide gene, such as *sacB*, *nuk*, *hok*, *gef*, *kil*, or*phlA*; mutations that introduce a bacterial lysis gene, such as *hly* and *cly*; mutations in genes that encode virulence factors, such as *IsyA*, *pag*, *prg*, *iscA*, *virG*, *plc*, and *act*; mutations in genes that modify the stress response, such as *recA*, *htrA*, *htpR*, *hsp*, and *groEL*; mutations in genes that disrupt the cell cycle, such as *min*; and mutations in genes that disrupt or inactivate regulatory functions, such as *cya*, *crp*, *phoP*/*phoQ*, and *ompR* (see, *e.g.,* U.S. Patent Publication Nos. 2012/0009153, 2003/0170276, and 2007/0298012; U.S. Patent No. 6,190,657; International Application Publication No. WO 2015/032165; Felgner et al. (2016) Gut Microbes 7(2):171-177; Broadway et al. (2014) J. Biotechnology 192:177-178; Frahm et al. (2015) mBio 6(2):e00254-15; Kong et al. (2011) Infection and Immunity 79(12):5027-5038; and Kong et al. (2012) Proc. Natl. Acad. Sci. U.S.A. 109(47):19414-19419). In general, attenuating mutations are gene deletions to prevent spontaneous compensatory mutations that might result in reversion to a virulent phenotype.

Another way to attenuate *S. typhimurium* for safety is to use the PhoP/PhoQ operon system, which is a typical bacterial two-component regulatory system, composed of a membrane-associated sensor kinase (PhoQ), and a cytoplasmic transcriptional regulator (PhoP) (see, *e.g.,* Miller, S. I. et al. (1989) Proc. Natl. Acad. Sci. U.S.A. 86:5054-5058; and Groisman, E. A. et al. (1989) Proc. Natl. Acad. Sci. U.S.A. 86:7077-7081). PhoP/PhoQ is required for virulence; its deletion results in poor survival of this bacterium in macrophages, and a marked attenuation in mice and humans (see, *e.g.,* Miller, S. I. et al. (1989) Proc. Natl. Acad. Sci. U.S.A. 86:5054-5058; Groisman, E. A. et al. (1989) Proc. Natl. Acad. Sci. U.S.A. 86:7077-7081; Galan, J. E. and Curtiss, R. III. (1989) Microb. Pathog. 6:433-443; and Fields, P. I. et al. (1986) Proc. Natl. Acad. Sci. U.S.A. 83:5189-5193). PhoP/PhoQ deletion strains have been employed as vaccine delivery vehicles (see, *e.g.,* Galan, J. E. and Curtiss, R. III. (1989) Microb. Pathog. 6:433-443; Fields, P. I. et al. (1986) Proc. Natl. Acad. Sci. U.S.A. 83:5189-5193; and Angelakopoulos, H. and Hohmann, E. L. (2000) Infect. Immun. 68:2135-2141). As described herein, however, it is disadvantageous for a strain to have limited survival in macrophages if the bacteria are not attempting to transfer plasmids.

These attenuated bacterial strains have been found to be safe in mice, pigs, and monkeys when administered intravenously (IV) (see, *e.g.,* Zhao, M. et al. (2005) Proc. Natl. Acad. Sci. U.S.A. 102:755-760; Zhao, M. et al. (2006) Cancer Res. 66:7647-7652; Tjuvajev J. et al. (2001) J. Control. Release 74:313-315; and Zheng, L. et al. (2000) Oncol. Res. 12:127-135), and certain live attenuated *Salmonella* strains have been shown to be well tolerated after oral administration in human clinical trials (see, *e.g.,* Chatfield, S. N. et al. (1992) Biotechnology 10:888-892; DiPetrillo, M. D. et al. (1999) Vaccine 18:449-459; Hohmann, E. L. et al. (1996) J. Infect. Dis. 173:1408-1414; and Sirard, J. C. et al. (1999) Immunol. Rev. 171:5-26).

Other strains of *S. typhimurium* that have been attenuated for therapy are, for example, the leucine-arginine auxtroph A-1 (see, *e.g.,* Zhao et al. (2005) Proc. Natl. Acad. Sci. U.S.A. 102(3):755-760; Yu et al. (2012) Scientific Reports 2:436; U.S. Patent No. 8,822,194; and U.S. Patent Publication No. 2014/0178341), and its derivative AR-1 (see, *e.g.,* Yu et al. (2012) Scientific Reports 2:436; Kawaguchi et al. (2017) Oncotarget 8(12):19065-19073; Zhao et al. (2006) Cancer Res. 66(15):7647-7652; Zhao et al. (2012) Cell Cycle 11(1): 187-193; Tome et al. (2013) Anticancer Research 33:97-102; Murakami et al. (2017) Oncotarget 8(5):8035-8042; Liu et al. (2016) Oncotarget 7(16):22873-22882; and Binder et al. (2013) Cancer Immunol. Res. 1(2):123-133); the *aroA*⁻ mutant *S. typhimurium* strain SL7207 (see, *e.g.*, Guo et al. (2011) Gene Therapy 18:95-105; and U.S. Patent Publication Nos. 2012/0009153, 2016/0369282 and 2016/0184456), and its obligate anaerobe derivative YB1 (see, *e.g.,* International Application Publication No. WO 2015/032165; Yu et al. (2012) Scientific Reports 2:436; and Leschner et al. (2009) PLoS ONE 4(8):e6692); the *aroA⁻* /*aroD*⁻ mutant *S. typhimurium* strain BRD509, a derivative of the SL1344 (wild-type) strain (see, *e.g.,* Yoon et al. (2017) Eur. J. Cancer 70:48-61); the *asd*⁻/*cya*⁻/*crp*⁻ mutant *S. typhimurium* strain χ4550 (see, *e.g.,* Sorenson et al. (2010) Biologics: Targets & Therapy 4:61-73) and the *phoP*⁻/*phoQ*⁻ *S. typhimurium* strain LH430 (see, *e.g.,* International Application Publication No. WO 2008/091375).

Attenuation, however, impacts the ability of the bacteria to accumulate in tumor-resident immune cells, the tumor microenvironment, and tumor cells. This problem is solved herein. The immunostimulatory bacteria, such as the *Salmonella* strains exemplified herein, are attenuated by virtue of modifications, that can include some of those described above, but also have other modifications and properties described herein that enhance the effectiveness as a cancer therapeutic.

Attenuated strains of *S. typhimurium* possess the innate ability to deliver DNA following phagocytosis and degradation (see, *e.g.,* Weiss et al. (2003) Int. J. Med. Microbiol. 41(7):3413-3414). They have been used as vectors for gene therapy. For example, *S. typhimurium* strains have been used to deliver and express a variety of genes, including those that encode cytokines, angiogenesis inhibitors, toxins, and prodrug-converting enzymes (see, *e.g.,* U.S. Patent Publication No. 2007/0298012; Loeffler et al. (2008) Cancer Gene Ther. 15(12):787-794; Loeffler et al. (2007) Proc. Natl. Acad. Sci. U.S.A. 104(31):12879-12883; Loeffler et al. (2008) J. Natl. Cancer Inst. 100:1113-1116; Clairmont, C. et al. (2000) J. Infect. Dis. 181:1996-2002; Bermudes, D. et al. (2002) Curr. Opin. Drug Discov. Devel. 5:194-199; Zhao, M. et al. (2005) Proc. Natl. Acad. Sci. U.S.A. 102:755-760; Zhao, M. et al. (2006) Cancer Res. 66:7647-7652; and Tjuvajev J. et al. (2001) J. Control. Release 74:313-315)*.*

*S. typhimurium* has been modified to deliver the tumor-associated antigen (TAA) survivin (SVN) to antigen presenting cells (APCs) to prime adaptive immunity (see, *e.g.,* U.S. Patent Publication No. 2014/0186401; and Xu et al. (2014) Cancer Res. 74(21):6260-6270). SVN is an inhibitor of apoptosis protein (IAP), which prolongs cell survival and provides cell cycle control, and is overexpressed in all solid tumors and poorly expressed in normal tissues. This technology uses SPI-2 and its type III secretion system to deliver the TAAs into the cytosol of APCs, which then are activated to induce TAA-specific CD8⁺T-cells and anti-tumor immunity (see, *e.g.,* Xu et al. (2014) Cancer Res. 74(21):6260-6270). Similar to the *Listeria-*based TAA vaccines, this approach has shown promise in mouse models, but has not demonstrated effective tumor antigen-specific T-cell priming in humans.

In addition to the delivery of DNA that encodes proteins, *S. typhimurium* also has been used for the delivery of small interfering RNAs (siRNAs) and short hairpin RNAs (shRNAs) for cancer therapy. For example, attenuated *S. typhimurium* has been modified to express certain shRNAs, such as those that target the immunosuppressive gene indolamine dioxygenase (IDO). Silenced IDO expression in a murine melanoma model resulted in tumor cell death and significant tumor infiltration by neutrophils (see, *e.g.,* Blache et al. (2012) Cancer Res. 72(24):6447-6456; International Application Publication No. WO 2008/091375; and U.S. Patent No. 9,453,227). Coadministration of this vector with a hyaluronidase showed positive results in the treatment of murine pancreatic ductal adenocarcinoma (see, *e.g.,* Manuel et al. (2015) Cancer Immunol. Res. 3(9):1096-1107; and U.S. Patent Publication No. 2016/0184456). In another study, an *S. typhimurium* strain attenuated by a *phoP*/*phoQ* deletion, and expressing a signal transducer and activator of transcription 3 (STAT3)-specific shRNA, inhibited tumor growth and reduced the number of metastatic organs, extending the life of C57BL/6 mice (see, *e.g.,* Zhang et al. (2007) Cancer Res. 67(12):5859-5864). In another example, *S. typhimurium* strain SL7207 has been used for the delivery of shRNA targeting *CTNNB1*, the gene that encodes β-catenin (see, *e.g.,* Guo et al. (2011) Gene Therapy 18:95-105; and U.S. Patent Publication Nos. 2009/0123426 and 2016/0369282). The *S. typhimurium* strain VNP20009 has been used for the delivery of shRNA targeting STAT3 (see, *e.g.,* Manuel et al. (2011) Cancer Res. 71(12):4183-4191; U.S. Patent Publication Nos. 2009/0208534, 2014/0186401 and 2016/0184456; and International Application Publication Nos. WO 2008/091375 and WO 2012/149364). siRNAs targeting the autophagy genes *Atg5* and *Beclin1* have been delivered to tumor cells using *S. typhimurium* strains A1-R and VNP20009 (see, *e.g.,* Liu et al. (2016) Oncotarget 7(16):22873-22882).

It has been found, however, that these strains do not effectively stimulate an anti-tumor immune response, nor effectively colonize tumors for delivery of therapeutic doses of encoded products. Improvement of such strains is needed so that they more effectively stimulate an anti-tumor immune response, such as the immunostimulatory bacteria provided herein. Further and alternative modifications of various bacteria have been described in published International PCT Application No. WO 2019/014398 and in U.S. Publication No. 2019/0017050 A1. The bacteria described in each of these publications, also described herein, can be modified as described herein to further improve their immunostimulatory and tumor-targeting properties.

### iii. VNP20009

Exemplary of a therapeutic bacterium that can be used as a starting strain for modification as described herein is the strain designated as VNP20009 (ATCC # 202165, YS1646). This virus was a clinical candidate. VNP20009 (ATCC # 202165, YS1646) was at least 50,000-fold attenuated for safety by deletion of the *msbB* and *purl* genes (see, *e.g.,* Clairmont et al. (2000) J. Infect. Dis. 181:1996-2002; Low et al. (2003) Methods in Molecular Medicine, Vol. 90, Suicide Gene Therapy: Methods and Reviews, pp. 47-59; and Lee et al. (2000) International Journal of Toxicology 19:19-25). Deletion or disruption to prevent expression of the *msbB* gene alters the composition of the lipid A domain of lipopolysaccharide, the major component of Gram-negative bacterial outer membranes (see, *e.g.,* Low et al. (1999) Nat. Biotechnol. 17(1):37-41). This prevents lipopolysaccharide-induced septic shock, attenuating the bacterial strain and lowering systemic toxicity, while reducing the potentially harmful production of TNFα (see, *e.g.,* Dinarello, C.A. (1997) Chest 112(6 Suppl):321S-329S; and Low et al. (1999) Nat. Biotechnol. 17(1):37-41). Deletion or disruption to prevent expression of the *purI* gene renders the bacteria auxotrophic for purines, which further attenuates the bacteria and enriches them in the tumor microenvironment (see, *e.g.,* Pawelek et al. (1997) Cancer Res. 57:4537-4544; and Broadway et al. (2014) J. Biotechnology 192:177-178). As shown herein, VNP20009 also is auxotrophic for the immunosuppressive nucleoside adenosine. Adenosine can accumulate to pathologically high levels in the tumor and contribute to an immunosuppressive tumor microenvironment (see, *e.g.,* Peter Vaupel and Arnulf Mayer, Oxygen Transport to Tissue XXXVII, Advances in Experimental Medicine and Biology 876 chapter 22, pp. 177-183).

When VNP20009 was administered into mice bearing syngeneic or human xenograft tumors, the bacteria accumulated preferentially within the extracellular components of tumors at ratios exceeding 300-1000 to 1, and demonstrated tumor growth inhibition, as well as prolonged survival compared to control mice (see, *e.g.,* Clairmont et al. (2000) J. Infect. Dis. 181:1996-2002). VNP20009 demonstrated success in tumor targeting and tumor growth suppression in animal models, while eliciting very little toxicity (see, *e.g.,* Broadway et al. (2014) J. Biotechnology 192:177-178; Loeffler et al. (2007) Proc. Natl. Acad. Sci. U.S.A. 104(31):12879-12883; Luo et al. (2002) Oncology Research 12:501-508; and Clairmont et al. (2000) J. Infect. Dis. 181:1996-2002).

Results from the Phase 1 clinical trial in human metastatic melanoma revealed that, while VNP20009 was relatively safe and well tolerated, very limited anti-tumor activity was observed (see, *e.g.,* Toso et al. (2002) J. Clin. Oncol. 20(1):142-152). While the use of VNP20009 resulted in no significant changes in metastatic disease burden, it did demonstrate evidence of tumor colonization at the maximum tolerated dose (MTD). Higher doses, which would be required to effect any anti-tumor activity, were not possible due to toxicity that correlated with high levels of pro-inflammatory cytokines.

The immunostimulatory bacteria provided herein provide numerous improvements and advantages that strain VNP20009 lacks. The immunostimulatory bacteria deliver encoded genetic payloads in a tumor-specific manner, to tumor-resident myeloid cells. The immunostimulatory bacteria, by virtue of genomic modifications, such as deletions or disruptions of genes, and other modifications of the genome, exhibit reduced TLR2-, TLR4-, and TLR5-mediated inflammation, for example, by virtue of the elimination of the flagella, the modifications of the LPS, and the elimination of the curl fimbriae and reduced biofilm formation. The immunostimulatory bacteria enhance T-cell function, such as by virtue of the elimination of the expression of L-asparaginase II, and facilitate, provide, permit, and support plasmid maintenance. The bacteria accumulate in (or target) only, or substantially only, myeloid cells, particularly tumor-resident myeloid cells, providing highly efficient plasmid delivery after phagocytosis. The immunostimulatory bacteria provided herein colonize the tumor microenvironment, and can be administered systemically. The immunostimulatory bacteria provided herein exhibit at least 15-fold improved LD₅₀ compared to VNP20009. Thus, a much higher dose, if needed, of the immunostimulatory bacteria provided herein can be administered without toxic effects, compared to VNP20009 (see, the table below in the section F.5. describing dosages and administration).

It is shown and described herein that immunostimulatory bacteria modified as described herein, including elimination of flagella, LPS modifications, and other modifications, preferentially accumulate in or target myeloid cells, particularly tumor-resident myeloid cells. The Examples demonstrate that the immunostimulatory bacteria accumulate in such cells following systemic, such as intravenous, administration. The Examples also describe and show plasmid transfer from the immunostimulatory bacteria into tumor-resident myeloid cells, and durable protein expression following bacterial cell death, thereby delivering therapeutic products, including products that result in an anti-cancer response and phenotype.

### iv. Wild-Type Strains

Accumulation of VNP20009 in tumors results from a combination of factors including: the inherent invasiveness of the parental strain, ATCC 14028, its ability to replicate in hypoxic environments, and its requirement for high concentrations of purines that are present in the interstitial fluid of tumors. As described herein, it is not necessary to use an attenuated strain, such as VNP20009, as a starting bacterial strain. By virtue of the modifications described herein, the bacteria are rendered non-toxic or attenuated. The parental strain, ATCC 14028, or another wild-type strain, can be used as a starting strain, and modified as described herein.

### 3. Limitations of Existing Bacterial Cancer Immunotherapies

Many classes of immunotherapies have significant limitations that limit their safety and efficacy, as well as complicated platforms that are not likely to be widely used. Bacteria have numerous advantageous properties for use as anti-cancer therapeutics, compared to, for example, oncolytic viruses. These include the ease with which they can be propagated, manufactured, stored, and eliminated from a host when treatment is completed. Viruses, however, also have advantageous properties, including the host response. The response to a bacterial infection is an innate inflammatory response, which is not advantageous for an anti-cancer therapeutic. The response to a viral infection is similar to an anti-cancer response. This is summarized in the following table (see, also, the Overview, above):

| | **Bacteria** | **Viruses** |
|---|---|---|
| Innate Recognition by: | TLR2, TLR4 and TLR5 | TLR3, TLR7/8, and STING |
| Inflammatory Cytokine Profile: | Promote innate immunity; | Promote innate immunity; |
| | Suppress adaptive immunity | Promote adaptive immunity |
| Chemokine Gradients: | Attract neutrophils to clear infection | Attract T-cells, monocytes to clear infection |
| Generation of Immunity: | No | Yes |
| Immunogenicity: | Not immunogenic | Highly immunogenic |

A limitation of bacteria as a microbial anti-cancer platform, thus, derives from the specific immune program that is initiated upon sensing of bacteria, even intracellular bacteria, by the immune system, compared to viral-sensing pathways, which are more akin to anti-cancer pathways. The sensing programs that recognize viruses permit the generation of highly effective vaccines and durable adaptive immunity. Vaccinating against bacteria, however, has been met with limited success. For example, the FDA-approved vaccine for typhoid fever against *Salmonella typhi* is only 55% effective (see, *e.g.,* Hart et al. (2016) PLoS ONE 11(1):e0145945), despite *S. typhi* containing a highly immunogenic Vi capsule and O:9 antigen, which do not occur in less immunogenic bacterial strains, such as *L. monocytogenes* and *S*. *typhimurium,* against which there are no vaccines.

Bacteria and viruses contain conserved structures known as Pathogen-Associated Molecular Patterns (PAMPs), which are sensed by host cell Pattern Recognition Receptors (PRRs). Recognition of PAMPs by PRRs triggers downstream signaling cascades that result in the induction of cytokines and chemokines, and initiation of a specific immune response (see, *e.g*., Iwasaki and Medzhitov (2010) Science 327(5963):291-295). The manner in which the innate immune system is engaged by PAMPs, and from what type of infectious agent, determines whether an appropriate innate or adaptive response is generated to combat the invading pathogen.

A class of PRRs, known as Toll-Like Receptors (TLRs), recognize PAMPs derived from bacterial and viral origins, and are located in various compartments within the cell. TLRs recognize a variety of ligands, including lipopolysaccharide (TLR4), lipoproteins (TLR2), flagellin (TLR5), unmethylated CpG motifs in DNA (TLR9), double-stranded RNA (TLR3), and single-stranded RNA (TLR7 and TLR8) (see, *e.g.,* Akira et al. (2001) Nat. Immunol. 2(8):675-680; and Kawai and Akira (2005) Curr. Opin. Immunol. 17(4):338-344). DNA and RNA-based viruses can be sensed either in host cytosolic compartments after phagocytosis, or directly in the cytosol. Type I interferons (IFN-α, IFN-β) are the signature cytokines induced by host recognition of single-stranded and double-stranded DNA and RNA, either of viral origin, or from the uptake of damaged host cell DNA. For example, the synthetic dsRNA analog polyinosinic:polycytidylic acid (poly(I:C)) is an agonist for endosomal TLR3 and a powerful inducer of type I IFN, and its more stable version, poly ICLC (such as that sold under the trademark Hiltonol^{®}), has been in clinical development (see, *e.g.,* Caskey et al. (2011) J. Exp. Med. 208(12):2357-2366). Similarly, single-stranded RNA (ssRNA) in the endosome is sensed by TLR7 and TLR8 (only in humans), and its known synthetic ligands, resiquimod and imiquimod, are FDA-approved topical cancer immunotherapies.

In the cytosol, double-stranded RNA (dsRNA) is sensed by RNA helicases, such as retinoic acid-inducible gene I (RIG-I) and melanoma differentiation-associated gene 5 (MDA-5), leading to induction of type I IFN (see, *e.g.,* Ireton and Gale (2011) Viruses 3(6):906-919). The cytosolic sensor for dsDNA is mediated through Stimulator of Interferon Genes (STING), an ER-resident adaptor protein that is the central mediator for sensing cytosolic dsDNA from infectious pathogens or aberrant host cell damage (see, *e.g.,* Barber (2011) Immunol. Rev. 243(1):99-108). STING signaling activates the TANK-binding kinase 1 (TBK1)/interferon regulatory factor 3 (IRF3) axis, and the NF-κB signaling axis, resulting in the induction of IFN-β and other pro-inflammatory cytokines and chemokines that strongly activate innate and adaptive immunity (see, *e.g.,* Burdette et al. (2011) Nature 478(7370):515-518). Sensing of cytosolic dsDNA through STING requires cyclic GMP-AMP synthase (cGAS), a host cell nucleotidyl transferase that directly binds dsDNA, and in response, synthesizes a cyclic dinucleotide (CDN) second messenger, cyclic GMP-AMP (cGAMP), which binds and activates STING (see, *e.g.,* Sun et al. (2013) Science 339(6121):786-791; and Wu et al. (2013) Science 339(6121):826-830).

STING also can bind to bacterially-derived CDNs, such as c-di-AMP produced from intracellular *L. monocytogenes*, or c-di-GMP from *S. typhimurium.* It was later discovered that cGAS produces a non-canonical CDN that can activate human STING alleles that are non-responsive to bacterially-derived canonical CDNs. Unlike the CDNs produced by bacteria, in which the two purine nucleosides are joined by a phosphate bridge with 3'-3' linkages, the internucleotide phosphate bridge in the cGAMP synthesized by cGAS is joined by a non-canonical 2'-3' linkage. These 2'-3' molecules bind STING with 300-fold better affinity than bacterial 3'-3' c-di-GMP, and thus, are more potent physiological ligands of STING (see, *e.g.,* Civril et al. (2013) Nature 498(7454):332-337; Diner et al. (2013) Cell Rep. 3(5):1355-1361; Gao et al. (2013) Sci. Signal 6(269):pl1; and Ablasser et al. (2013) Nature 503(7477):530-534). The cGAS/STING signaling pathway in humans appears to have evolved to preferentially respond to viral pathogens over bacterial pathogens.

Thus, viral-sensing PRRs and TLRs, such as STING, RIG-I, TLR3 and TLR7/8, induce type I IFN, and the cytokines and chemokines that lead to effective T-cell mediated adaptive immunity. In the tumor setting, type I IFN signaling is required to induce T-cell trafficking chemokines, such as CXCL10, and also to activate DC cross-presentation of tumor antigens to prime CD8⁺ T-cells (see, *e.g.,* Diamond et al. (2011) J. Exp. Med. 208(10):1989-2003; and Fuertes et al. (2011) J. Exp. Med. 208(10):2005-2016).

In contrast, host surveillance of bacteria, such as *S*. *typhimurium,* is largely mediated through TLR2, TLR4, and TLR5 (see, *e.g.,* Arpaia et al. (2011) Cell 144(5):675-688). These TLRs signal through MyD88 (myeloid differentiation primary response protein 88) and TRIF (Toll/interleukin-1 receptor (TIR)-domain-containing adapter-inducing interferon-β) adaptor molecules to mediate induction of the NF-κB-dependent pro-inflammatory cytokines TNF-α and IL-6 (see, *e.g*., Pandey et al. (2015) Cold Spring Harb. Perspect. Biol. 7(1):a016246). *S. typhimurium* was shown to activate the NLRP3 inflammasome pathway, resulting in the cleavage of caspase-1 and the induction of the pro-inflammatory cytokines IL-1β and IL-18 that lead to pyroptotic cell death. Engagement of TLR2, TLR4 and TLR5, and inflammasome activation, induces chemokines and cytokines that lead to bacterial clearance by neutrophils and macrophages. Evidence that *S. typhimurium* is cleared by T-cells is limited, and antibodies that are generated against it are non-neutralizing (see, *e.g.,* McSorley (2014) Immunol. Rev. 260(1):168-182). Further, *S. typhimurium* has mechanisms to directly suppress T-cell function, impairing any potential anti-tumor T-cell response from being generated (see, *e.g.,* Kullas et al. (2012) Cell Host Microbe. 12(6)791-798). As a result, bacterial cancer therapies, such as *S. typhimurium,* lead to recruitment and clearance by neutrophils and macrophages, which are not the T-cells that are required to generate adaptive anti-tumor immunity. It is described herein that these differences can explain why prior bacterial anti-cancer vaccines, even those harboring host tumor antigens, are poor T-cell priming vectors in humans.

These problems are among those addressed by the immunostimulatory bacteria provided herein. The immunostimulatory bacteria provided herein are engineered to have advantageous properties that were previously only provided by viral therapeutics, and also, to retain the advantageous properties of bacterial therapeutics. The bacteria provided herein can be systemically administered, can localize to tumors, tumor-resident immune cells, and/or the tumor microenvironment, overcome immunosuppression, and properly activate anti-tumor immunity, while also limiting the autoimmune-related toxicities of existing systemic immunotherapies. The immunostimulatory bacteria provided herein effectively localize to tumor-resident immune cells, and encode therapeutic anti-cancer products, and can encode a plurality of such products. For example, the bacteria provided herein can encode complementary therapeutic products.

Provided herein is a superior microbial anti-cancer platform, engineered to retain the beneficial properties of bacteria, while eliciting a viral-like immune response that induces effective adaptive immunity. As described herein, bacteria, such as strains of *Salmonella* and other species, can be modified as described herein to have reduced inflammatory effects, and thus, to be less toxic. As a result, for example, higher dosages can be administered. Any of these strains of *Salmonella,* as well as other species of bacteria, known to those of skill in the art and/or listed above and herein, can be modified as described herein. The immunostimulatory bacteria provided herein are modified to have increased colonization of the tumor microenvironment, tumor-resident immune cells, and tumors. They are engineered so that they have reduced toxicity, and other properties that target them to the tumor microenvironment, including adenosine auxotrophy. The strains provided herein also are engineered so that they are not inactivated by complement.

Provided is an anti-cancer therapeutic product that delivers a genetic payload encoding a truncated co-stimulatory molecule (receptor or ligand; *e.g*., 4-1BBL, CD80, CD86, CD27L, B7RP1, OX40L), with a full or truncated or partial cytoplasmic domain deletion, for expression on an antigen presenting cell (APC), where the truncated gene product is capable of constitutive immuno-stimulatory signaling to a T-cell through co-stimulatory receptor engagement, and is unable to counter-regulatory signal to the APC due to a deleted or truncated cytoplasmic domain. The co-stimulatory molecules also can be modified to include residues (such as positive residues) in the truncated cytoplasmic domain, to ensure that they are expressed in the correct orientation in the cell membrane (the Examples below describe this in more detail; see, *e.g*., Example 19).

The bacterial strains provided herein are engineered to deliver therapeutic products. The bacterial strains herein deliver immunostimulatory proteins, including cytokines, chemokines and co-stimulatory molecules, as well as modified gain-of-function cytosolic DNA/RNA sensors that can constitutively evoke or induce type I IFN expression, and other therapeutic products, such as, but not limited to, antibodies and fragments thereof, TGF-β and IL-6 binding decoy receptors, TGF-β polypeptide antagonists, bispecific T-cell engagers (BiTEs^{®}), RNAi, and complementary combinations thereof, that promote an anti-tumor immune response in the tumor microenvironment. The bacterial strains also include genomic modifications that reduce pyroptosis of phagocytic cells, thereby providing for a more robust immune response, and/or reduce or eliminate the ability to infect/invade epithelial cells, but retain the ability to infect/invade phagocytic cells, so that they accumulate more effectively in tumors, the tumor microenvironment and in tumor-resident immune cells. The bacterial strains also can be modified to be resistant to inactivation by complement factors in human serum. The bacterial strains also can be modified to encode therapeutic products, including, alone or in combinations, for example, cytokines, chemokines, co-stimulatory molecules, constitutively active inducers of type I IFN, and monoclonal antibodies (and fragments thereof) to immune checkpoints, and also to other such targets.

### C. MODIFICATIONS AND ENHANCEMENTS OF IMMUNOSTIMULATORY BACTERIA TO INCREASE THERAPEUTIC INDEX AND TO INCREASE ACCUMULATION IN TUMOR-RESIDENT MYELOID CELLS

Provided herein are enhancements, including modifications to the bacterial genome, or to the immunostimulatory bacteria, that, for example, reduce toxicity and improve the anti-tumor activity, such as by increasing accumulation in tumor-resident myeloid cells, improving resistance to complement inactivation, reducing immune cell death, promoting adaptive immunity, and enhancing T-cell function. The modifications are described with respect to *Salmonella,* particularly *S. typhimurium*; it is understood that the skilled person can effect similar enhancements/modifications in other bacterial species and other *Salmonella* strains. Exemplary of such enhancements/modifications are the following.

### 1. Deletions in Genes in the LPS Biosynthetic Pathway

The lipopolysaccharide (LPS) of Gram-negative bacteria is the major component of the outer leaflet of the bacterial membrane. It is composed of three major parts, lipid A, a non-repeating core oligosaccharide, and the O antigen (or O polysaccharide). O antigen is the outermost portion on LPS and serves as a protective layer against bacterial permeability, however, the sugar composition of O antigen varies widely between strains. The lipid A and core oligosaccharide vary less, and are more typically conserved within strains of the same species. Lipid A is the portion of LPS that contains endotoxin activity. It is typically a disaccharide decorated with multiple fatty acids. These hydrophobic fatty acid chains anchor the LPS into the bacterial membrane, and the rest of the LPS projects from the cell surface. The lipid A domain is responsible for much of the toxicity of Gram-negative bacteria. Typically, LPS in the blood is recognized as a significant pathogen associated molecular pattern (PAMP), and induces a profound pro-inflammatory response. LPS is the ligand for a membrane-bound receptor complex comprising CD14, MD2, and TLR4. TLR4 is a transmembrane protein that can signal through the MyD88 and TRIF pathways to stimulate the NF-κB pathway and result in the production of pro-inflammatory cytokines, such as TNF-α and IL-6, the result of which can be endotoxic shock, which can be fatal. LPS in the cytosol of mammalian cells can bind directly to the CARD domains of caspases 4, 5, and 11, leading to autoactivation and pyroptotic cell death (see, *e.g.,* Hagar et al. (2015) Cell Research 25:149-150). The composition of lipid A and the toxigenicity of lipid A variants is well documented. For example, a monophosphorylated lipid A is much less inflammatory than lipid A with multiple phosphate groups. The number and length of the acyl chains on lipid A also can have a profound impact on the degree of toxicity. Canonical lipid A from *E. coli* has six acyl chains, and this hexa-acylation is potently toxic. *S. typhimurium* lipid A is similar to that of *E. coli*; it is a glucosamine disaccharide that carries four primary and two secondary hydroxyacyl chains (see, *e.g.,* Raetz et al. (2002) Annu. Rev. Biochem. 71:635-700).

### a. msbB Deletion

The enzyme lipid A biosynthesis myristoyltransferase, encoded by the *msbB* gene in *S. typhimurium,* catalyzes the addition of a terminal myristoyl group to the lipid A domain of lipopolysaccharide (LPS) (see, *e.g.,* Low et al. (1999) Nat. Biotechnol. 17(1):37-41). Deletion of *msbB* thus alters the acyl composition of the lipid A domain of LPS, the major component of the outer membranes of Gram-negative bacteria. For example, deletion of *msbB* in the *S. typhimurium* strain VNP20009 results in the production of a predominantly penta-acylated lipid A, which is less toxic than native hexa-acylated lipid A, and allows for systemic delivery without the induction of toxic shock (see, *e.g.,* Lee et al. (2000) International Journal of Toxicology 19:19-25). This modification significantly reduces the ability of the LPS to induce septic shock, attenuating the bacterial strain, and thus, increasing the therapeutic index of *Salmonella-*based immunotherapeutics (see, *e.g.,* U.S. Patent Publication Nos. 2003/0170276, 2003/0109026, 2004/0229338, 2005/0255088, and 2007/0298012). Importantly, *msbB* mutants that do no express the *msbB* product are unable to replicate intracellularly, as exemplified herein (see, *e.g*., Example 2), which is a requirement for *Salmonella* virulence (see, *e.g.,* Leung et al. (1991) Proc. Natl. Acad. Sci. U.S.A. 88:11470-11474).

Other LPS mutations, including replacements, deletions, or insertions, that alter LPS expression can be introduced into the bacterial strains provided herein, including the *Salmonella* strains, that dramatically reduce virulence, and thereby provide for lower toxicity, and permit the administration of higher doses.

Corresponding genes, encoding homologs or orthologs of lipid A biosynthesis myristoyltransferase in other bacterial species, also can be deleted or disrupted to achieve similar results. These genes include, but are not limited to, for example, *lpxM*, encoding myristoyl-acyl carrier protein-dependent acyltransferase in *E. coli*; and *msbB,* encoding lipid A acyltransferase in *S. typhi.*

### b. pagP Deletion

As described above, *msbB* mutants of *S. typhimurium* cannot undergo the terminal myristoylation of LPS, and produce predominantly penta-acylated lipid A that is significantly less toxic than hexa-acylated lipid A. The modification of lipid A with palmitate is catalyzed by the enzyme lipid A palmitoyltransferase (PagP). Transcription of the *pagP* gene is under control of the PhoP/PhoQ system which is activated by low concentrations of magnesium, *e.g*., inside the SCV. Thus, the acyl content of *S. typhimurium* lipid A is variable, and with wild-type bacteria, it can be hexa- or penta-acylated. The ability of *S. typhimurium* to palmitate its lipid A increases resistance to antimicrobial peptides that are secreted into phagolysosomes.

In wild-type *S. typhimurium,* expression of *pagP* results in a lipid A that is hepta-acylated. In an *msbB* mutant (in which the terminal acyl chain of the lipid A cannot be added), the induction of *pagP* results in a hexa-acylated lipid A (see, *e.g.,* Kong et al. (2011) Infection and Immunity 79(12):5027-5038). Hexa-acylated lipid A has been shown to be the most pro-inflammatory. While groups have sought to exploit this pro-inflammatory signal, for example, by deletion or disruption of *pagP* to allow only hexa-acylated lipid A to be produced (see, *e.g.,* Felgner et al. (2016) Gut Microbes 7(2):171-177; and Felgner et al. (2018) Oncoimmunology 7(2):e1382791), this can lead to poor tolerability, due to the TNF-α-mediated pro-inflammatory nature of the LPS, and paradoxically less adaptive immunity (see, *e.g.,* Kocijancic et al. (2017) Oncotarget 8(30):49988-50001).

LPS is a potent TLR4 agonist that induces TNF-α and IL-6. The dose-limiting toxicities in the LV. VNP20009 clinical trial (see, *e.g.,* Toso et al. (2002) J. Clin. Oncol. 20(1):142-152), at 1E9 CFUs/m², were cytokine mediated (fever, hypotension), with TNF-α levels > 100,000 pg/ml, and IL-6 levels > 10,000 pg/ml in serum at 2 hours. Despite the *msbB* deletion in VNP20009 and its reduced pyrogenicity, the LPS still can be toxic at high doses, possibly due to the presence of hexa-acylated lipid A. Thus, a *pagP*⁻/*msbB*⁻ strain, which cannot produce hexa-acylated lipid A, and produces only penta-acylated lipid A, resulting in lower induction of pro-inflammatory cytokines, is better tolerated at higher doses, and will allow for dosing in humans at or above 1E9 CFUs/m². Higher dosing leads to increased colonization of tumors, tumor-resident immune cells, and the tumor microenvironment, enhancing the therapeutic efficacy of the immunostimulatory bacteria. Because of the resulting change in bacterial membranes and structure, the host immune response, such as complement activity, is altered so that the bacteria are not eliminated upon systemic administration. For example, it is shown herein (see, *e.g.,* Example 5) that *pagP*⁻/*msbB*⁻ mutant strains have increased resistance to complement inactivation, and enhanced stability in human serum.

Provided herein are immunostimulatory bacteria, exemplified by live attenuated *Salmonella* strains, such as the exemplary strain of *S. typhimurium,* that only can produce LPS with penta-acylated lipid A, that contain a deletion of the *msbB* gene, and that further are modified by deletion or disruption of *pagP.* As discussed above, deletion of *msbB* expression prevents the terminal myristoylation of lipid A, while deletion of *pagP* expression prevents palmitoylation. A strain modified to produce LPS with penta-acylated lipid A results in lower levels of pro-inflammatory cytokines, improved stability in the blood, resistance to complement fixation, increased sensitivity to antimicrobial peptides, enhanced tolerability, and increased anti-tumor immunity when further modified to express heterologous genetic payloads that stimulate the immune response in the tumor microenvironment.

Corresponding genes, encoding homologs and orthologs of lipid A palmitoyltransferase (PagP) in other bacterial species, also can be deleted or disrupted to achieve similar results. These genes include, but are not limited to, for example, *pagP,* encoding Lipid IVA palmitoyltransferase in *E*. *coli*; and *pagP,* encoding antimicrobial peptide resistance and lipid A acylation protein in *S. typhi.*

### 2. Nutrient Auxotrophy

The immunostimulatory bacteria provided herein can be attenuated by rendering them auxotrophic for one or more essential nutrients, such as purines (for example, adenine), nucleosides (for example, adenosine), amino acids (for example, aromatic amino acids, arginine and leucine), adenosine triphosphate (ATP), or other nutrients as known and described in the art.

### a. purI Deletion/Disruption

Phosphoribosylaminoimidazole synthetase, an enzyme encoded by the *purI* gene (synonymous with the *purM* gene), is involved in the biosynthesis pathway of purines. Disruption or deletion or inactivation of the *purI* gene thus renders the bacteria auxotrophic for purines. In addition to being attenuated, *purI*⁻ mutants are enriched in the tumor environment and have significant anti-tumor activity (see, *e.g.,* Pawelek et al. (1997) Cancer Research 57:4537-4544). It was previously described that this colonization results from the high concentration of purines present in the interstitial fluid of tumors as a result of their rapid cellular turnover. Since the *purI*⁻ bacteria are unable to synthesize purines, they require an external source of adenine, and it was thought that this would lead to their restricted growth in the purine-enriched tumor microenvironment (see, *e.g.,* Rosenberg et al. (2002) J. Immunotherapy 25(3):218-225). While the VNP20009 strain was initially reported to contain a deletion of the *purl* gene (see, *e.g.,* Low et al. (2003) Methods in Molecular Medicine Vol. 90, Suicide Gene Therapy: Methods and Reviews, pp. 47-59), subsequent analysis of the entire genome of VNP20009 demonstrated that the *purI* gene is not deleted, but is disrupted by a chromosomal inversion (see, *e.g*., Broadway et al. (2014) Journal of Biotechnology 192:177-178). The entire *purI* gene is contained within two parts of the VNP20009 chromosome that is flanked by insertion sequences, one of which has an active transposase. While disruption of the *purI* gene limits replication to the tumor tissue/microenvironment, it still permits intracellular replication and virulence. Deletion or disruption of each of the *msbB* and the *purI* genes, as exemplified herein (see, Example 2), is required to limit growth to the extracellular space in tumor tissue, and prevent intracellular replication. Provided herein are strains in which the coding portion of these genes are completely deleted to eliminate any possible reversion to wild-type by recombination.

Besides *purI* gene deletions or disruptions, nutrient auxotrophy can be introduced into the immunostimulatory bacteria by deletions/mutations in genes such as *aro*, *gua*, *thy*, *nad* and *asd*, for example. Nutrients produced by the biosynthesis pathways involving these genes are often unavailable in host cells, and as such, bacterial survival is challenging. For example, attenuation of *Salmonella* and other bacterial species can be achieved by deletion of the *aroA* gene, which is part of the shikimate pathway, connecting glycolysis to aromatic amino acid biosynthesis (see, *e.g.,* Felgner et al. (2016) mBio 7(5):e01220-16). Deletion of *aroA* results in bacterial auxotrophy for aromatic amino acids and subsequent attenuation (see, *e.g.,* U.S. Patent Publication Nos. 2003/0170276, 2003/0175297, 2012/0009153, and 2016/0369282; and International Application Publication Nos. WO 2015/032165 and WO 2016/025582). Similarly, other enzymes involved in the biosynthesis pathway for aromatic amino acids, including *aroC* and *aroD,* have been deleted to achieve attenuation (see, *e.g.,* U.S. Patent Publication No. 2016/0369282; and International Application Publication No. WO 2016/025582). For example, *S. typhimurium* strain SL7207 is an aromatic amino acid auxotroph (*aroA*⁻ mutant); strains A1 and A1-R are leucine-arginine auxotrophs; and VNP20009/YS1646 is a purine auxotroph (*purI*⁻ mutant). As shown herein, VNP20009/YS1646 is also auxotrophic for the immunosuppressive nucleoside adenosine, and for ATP (see, *e.g*., Example 1).

Corresponding genes, encoding homologs or orthologs of phosphoribosylaminoimidazole synthetase (PurI), and other genes required for purine synthesis in other bacterial species, also can be deleted or disrupted to achieve similar results. These genes include, but are not limited to, for example, *purM*, encoding phosphori-bosylformylglycinamide cyclo-ligase in *E. coli*; *purM*, encoding phosphoribosylformylglycinamidine cyclo-ligase in *S. typhi*; *purA*, encoding adenylosuccinate synthetase, *purQ*, encoding phosphoribosylformylglycinamidine synthase II, and *purS*, encoding phosphoribosylformylglycinamidine synthase subunit PurS in *L. monocytogenes*; *purM* (BL1122)*,* encoding phosphoribosylformylglycinamidine cyclo-ligase in *Bifidobacterium longum*; and NT01CX_RS09765, encoding AIR synthase, and NT01CX_RS07625 (*purM*), encoding phosphoribosylformylglycinamidine cyclo-ligase in *Clostridium novyi.*

### b. Adenosine Auxotrophy

Metabolites derived from the tryptophan and adenosine triphosphate (ATP)/adenosine pathways are major drivers in forming an immunosuppressive environment within the tumor/tumor microenvironment (TME). Adenosine, which exists in the free form inside and outside of cells, is an effector of immune function. Adenosine decreases T-cell receptor induced activation of NF-κB, and inhibits IL-2, IL-4, and IFN-γ. Adenosine decreases T-cell cytotoxicity, increases T-cell anergy, and increases T-cell differentiation to Foxp3⁺ or Lag3⁺ regulatory T-cells (T-reg cells, Tregs, or Tregs). On natural killer (NK) cells, adenosine decreases IFN-γ production, and suppresses NK cell cytotoxicity. Adenosine blocks neutrophil adhesion and extravasation, decreases phagocytosis, and attenuates levels of superoxide and nitric oxide. Adenosine also decreases the expression of TNF-α, IL-12, and MIP-1α (CCL3) on macrophages, attenuates major histocompatibility complex (MHC) Class II expression, and increases levels of IL-10 and IL-6. Adenosine immunomodulation activity occurs after its release into the extracellular space of the tumor and activation of adenosine receptors (ADRs) on the surface of target immune cells, cancer cells, or endothelial cells. The high adenosine levels in the tumor microenvironment result in local immunosuppression, which limits the capacity of the immune system to eliminate cancer cells.

Extracellular adenosine is produced by the sequential activities of membrane associated ectoenzymes CD39 (ecto-nucleoside triphosphate diphosphohydrolasel, or NTPDase1) and CD73 (ecto-5'-nucleotidase), which are expressed on tumor stromal cells, together producing adenosine by phosphohydrolysis of ATP or ADP produced from dead or dying cells. CD39 converts extracellular ATP (or ADP) to 5'-AMP, which is converted to adenosine by CD73. Expression of CD39 and CD73 on endothelial cells is increased under the hypoxic conditions of the tumor microenvironment, thereby increasing levels of adenosine. Tumor hypoxia can result from inadequate blood supply and disorganized tumor vasculature, impairing delivery of oxygen (see, *e.g.,* Carroll and Ashcroft (2005) Expert. Rev. Mol. Med. 7(6), DOI: 10.1017/S1462399405009117). Hypoxia, which occurs in the tumor microenvironment, also inhibits adenylate kinase (AK), which converts adenosine to AMP, leading to very high extracellular adenosine concentrations. The extracellular concentration of adenosine in the hypoxic tumor microenvironment has been measured at 10-100 µM, which is up to about 100-1000 fold higher than the typical extracellular adenosine concentration of approximately 0.1 µM (see, *e.g.,* Vaupel et al. (2016) Adv. Exp. Med. Biol. 876:177-183; and Antonioli et al. (2013) Nat. Rev. Can. 13:842-857). Since hypoxic regions in tumors are distal from microvessels, the local concentration of adenosine in some regions of the tumor can be higher than in others.

To direct effects to inhibit the immune system, adenosine also can control cancer cell growth and dissemination by effects on cancer cell proliferation, apoptosis, and angiogenesis. For example, adenosine can promote angiogenesis, primarily through the stimulation of A_{2A} and A_{2B} receptors. Stimulation of the receptors on endothelial cells can regulate the expression of intercellular adhesion molecule 1 (ICAM-1) and E-selectin on endothelial cells, maintain vascular integrity, and promote vessel growth (see, *e.g.,* Antonioli et al. (2013) Nat. Rev. Can. 13:842-857). Activation of one or more of A_{2A}, A_{2B}, or A₃ on various cells by adenosine can stimulate the production of the pro-angiogenic factors, such as vascular endothelial growth factor (VEGF), interleukin-8 (IL-8) or angiopoietin 2 (see, *e.g.,* Antonioli et al. (2013) Nat. Rev. Can. 13:842-857).

Adenosine also can directly regulate tumor cell proliferation, apoptosis, and metastasis through interaction with receptors on cancer cells. For example, studies have shown that the activation of A₁ and A_{2A} receptors promote tumor cell proliferation in some breast cancer cell lines, and activation of A_{2B} receptors have cancer growth-promoting properties in colon carcinoma cells (see, *e.g.,* Antonioli et al. (2013) Nat. Rev. Can. 13:842-857). Adenosine also can trigger apoptosis of cancer cells, and various studies have correlated this activity to activation of the extrinsic apoptotic pathway through A₃, or the intrinsic apoptotic pathway through A_{2A} and A_{2B} (see, *e.g.,* Antonioli *et al.* (2013)). Adenosine can promote tumor cell migration and metastasis, by increasing cell motility, adhesion to the extracellular matrix, and expression of cell attachment proteins and receptors to promote cell movement and motility.

The extracellular release of adenosine triphosphate (ATP) occurs from stimulated immune cells, and damaged, dying, or stressed cells. The NLR family pyrin domain-containing 3 (NLRP3) inflammasome, when stimulated by this extracellular release of ATP, activates caspase-1 and results in the secretion of the cytokines IL-1β and IL-18, which in turn activate innate and adaptive immune responses (see, *e.g.*, Stagg and Smyth (2010) Oncogene 29:5346-5358). ATP can accumulate to concentrations exceeding 100 mM in tumor tissue, whereas levels of ATP found in healthy tissues are very low (~1-5 µM) (see, *e.g.,* Song et al. (2016) Am. J. Physiol. Cell Physiol. 310(2):C99-C114). ATP is catabolized into adenosine by the enzymes CD39 and CD73. Activated adenosine acts as a highly immunosuppressive metabolite via a negative-feedback mechanism and has a pleiotropic effect against multiple immune cell types in the hypoxic tumor microenvironment (see, *e.g.,* Stagg and Smyth (2010) Oncogene 29:5346-5358). Adenosine receptors A_{2A} and A_{2B} are expressed on a variety of immune cells and are stimulated by adenosine to promote cAMP-mediated signaling changes, resulting in immunosuppressive phenotypes of T-cells, B-cells, NK cells, dendritic cells (DCs), mast cells, macrophages, neutrophils, and natural killer T (NKT) cells. As a result, adenosine levels can accumulate to over one hundred times their normal concentration in pathological tissues, such as solid tumors, which have been shown to overexpress ecto-nucleotidases, such as CD73. Adenosine also has been shown to promote tumor angiogenesis and development. An engineered bacterium that is auxotrophic for adenosine would thus exhibit enhanced tumor-targeting and colonization.

Immunostimulatory bacteria, such as *Salmonella typhi*, can be made auxotrophic for adenosine by, for example, deletion of the *tsx* gene (see, *e.g.,* Bucarey et al. (2005) Infection and Immunity 73(10):6210-6219) or by deletion of *purD* (see. *e.g.,* Husseiny (2005) Infection and Immunity 73(3):1598-1605). In the Gram-negative bacteria *Xanthomonas oryzae*, a *purD* gene knockout was shown to be auxotrophic for adenosine (see, *e.g.,* Park et al. (2007) FEMS Microbiol. Lett. 276:55-59). As exemplified herein, *S. typhimurium* strain VNP20009 is auxotrophic for adenosine due to its *purI* modification; hence, further modification to render it auxotrophic for adenosine is not required. Hence, embodiments of the immunostimulatory bacterial strains, as provided herein, are auxotrophic for adenosine. Such auxotrophic bacteria selectively replicate in the tumor microenvironment, further increasing accumulation and replication of the administered bacteria in tumors, and decreasing the levels of adenosine in and around tumors, thereby reducing or eliminating the immunosuppression caused by the accumulation of adenosine. Exemplary of such bacteria, provided herein, is a modified strain of *S. typhimurium* containing *purI*⁻/*msbB*⁻ mutations to provide adenosine auxotrophy. For other strains and bacteria, the *purI* gene can be disrupted as it has been in VNP20009, or it can contain a deletion of all or a portion of the *purI* gene, which ensures that there cannot be a reversion to a wild-type gene. As described elsewhere herein, in strain VNP20009, the *purI*⁻ gene was inactivated by inversion. Similarly, the *msbB* gene in VNP20009 was not completely deleted. As exemplified herein, strains in which the *purI* and *msbB* genes have been completely deleted to eliminate any risk of reversion, demonstrate superior fitness as assessed by growth of cultures *in vitro.*

Immunostimulatory bacteria modified by rendering them auxotrophic for one or more essential nutrients, such as purines (for example, adenine), nucleosides (for example, adenosine), amino acids (for example, aromatic amino acids, arginine, and leucine), or adenosine triphosphate (ATP), are employed. In particular, in embodiments of the immunostimulatory bacteria provided herein, such as strains of *S*. *typhimurium,* the bacteria are rendered auxotrophic for adenosine, and optionally, for ATP, and preferentially accumulate in tumor microenvironments (TMEs). Hence, strains of immunostimulatory bacteria described herein are attenuated because they require purines, adenosine, and/or ATP for growth, and they preferentially colonize TMEs, which, as discussed below, have an abundance of these metabolites. Because adenosine accumulation in the tumor microenvironment of some tumors is immunosuppressive, adenosine auxotrophy eliminates the immunosuppression from adenosine that accumulates in the tumor microenvironment of certain cancers.

### 3. Plasmid Maintenance and Delivery

### a. asd Deletion

The *asd* gene in bacteria encodes an aspartate-semialdehyde dehydrogenase. *asd⁻* mutants of *S. typhimurium* have an obligate requirement for diaminopimelic acid (DAP), which is required for cell wall synthesis, and will undergo lysis in environments deprived of DAP. This DAP auxotrophy can be used for plasmid selection and maintenance of plasmid stability *in vivo,* without the use of antibiotics, when the *asd* gene is complemented *in trans* on a plasmid in the bacterium. Non-antibiotic-based plasmid selection systems are advantageous and allow for 1) use of administered antibiotics as a rapid clearance mechanism in the event of adverse symptoms, and 2) for antibiotic-free scale up of production, where such use is commonly avoided. The *asd* gene complementation system provides for such non-antibiotic-based plasmid selection (see, *e.g.,* Galán et al. (1990) Gene 94(1):29-35). The use of the *asd* gene complementation system to maintain plasmids in the tumor microenvironment is expected to increase the potency of *S. typhimurium* engineered to deliver plasmids encoding genetic payloads/therapeutic products, such as immunostimulatory proteins (*e.g*., cytokines, chemokines, co-stimulatory molecules); cytosolic DNA/RNA sensors that induce type I IFN, such as STING and IRF3, and gain-of-function/constitutively active mutants thereof; antibodies and fragments thereof (*e.g*., checkpoint inhibitors, or anti-IL-6 or anti-VEGF antibodies); bi-specific T-cell engagers (sold under the trademark BiTEs^{®}); interfering RNAs; and other therapeutic products as discussed elsewhere herein and known in the art; and complementary combinations of all of the preceding therapeutic products.

An alternative use for an *asd* mutant of *S. typhimurium* is to exploit the DAP auxotrophy to produce an autolytic (or suicidal) strain, for delivery of therapeutic products/macromolecules to infected cells without the ability to persistently colonize host tumors. Deletion of the *asd* gene makes the bacteria auxotrophic for DAP when grown *in vitro* or *in vivo.* An example described herein, provides an *asd* deletion strain that is auxotrophic for DAP and that contains a plasmid suitable for delivery of immunomodulatory proteins, that does not contain an *asd* complementing gene, resulting in a strain that is defective for replication *in vivo.* This strain is propagated *in vitro* in the presence of DAP, and grows normally, and then is administered as an immunotherapeutic agent to a mammalian host where DAP is not present. The suicidal strain is able to invade host cells, but is not be able to replicate due to the absence of DAP in mammalian tissues, lysing automatically and delivering its cytosolic contents (*e.g*., plasmids or proteins).

Corresponding genes, encoding homologs or orthologs of aspartate-semialdehyde dehydrogenase (asd) in other bacterial species, also can be deleted or disrupted to achieve similar results. These genes include, but are not limited to, for example, *asd*, encoding aspartate-semialdehyde dehydrogenase in *E. coli*; *asd* (STY4271), encoding aspartate-semialdehyde dehydrogenase in *S. typhi*; *asd* (Imo1437), encoding aspartate-semialdehyde dehydrogenase in *L. monocytogenes*; *asd* (BL0492), encoding aspartate-semialdehyde dehydrogenase in *Bifidobacterium longum;* and NT01CX_RS04325 (*asd*), encoding aspartate-semialdehyde dehydrogenase in *Clostridium novyi.*

### b. endA Deletion/Disruption

The *endA* gene (see, for example, SEQ ID NO:250) encodes an endonuclease (DNA-specific endonuclease I; see, for example, SEQ ID NO:251) that mediates degradation of double-stranded DNA (dsDNA) in the periplasm of Gram-negative bacteria. Most common strains of laboratory *E. coli* are *endA*⁻, as a mutation in the *endA* gene allows for higher yields of plasmid DNA. This gene is conserved among species. To facilitate intact plasmid DNA delivery, the *endA* gene of the engineered immunostimulatory bacteria is deleted or mutated to prevent its endonuclease activity. Exemplary of such mutations is an E208K amino acid substitution (see, *e.g.,* Durfee et al. (2008) J. Bacteriol. 190(7):2597-2606), or a corresponding mutation in the species of interest. *endA,* including residue E208, is conserved among bacterial species, including *Salmonella.* Thus, the E208K mutation can be used to eliminate endonuclease activity in other species, including *Salmonella* species. Those of skill in the art can introduce other mutations or deletions to eliminate *endA* activity. Effecting this mutation, or deleting or disrupting the gene to eliminate activity of *endA* in the immunostimulatory bacteria herein, such as in *Salmonella,* increases efficiency of intact plasmid DNA delivery, thereby increasing expression of any one, or two, or more, immunomodulatory proteins/therapeutic products encoded on the plasmid, and enhancing the anti-tumor immune response and anti-tumor efficacy.

### 4. Flagellin Knockout Strains

Flagella are organelles on the surface of bacteria that are composed of a long filament that is attached, via a hook, to a rotary motor that can rotate in a clockwise or counterclockwise manner to provide a means for locomotion. Flagella, for example, in *S. typhimurium,* are important for chemotaxis and for establishing an infection via the oral route, due to the ability to mediate motility across the mucous layer in the gastrointestinal tract. While flagella have been demonstrated to be required for chemotaxis to and colonization of tumor cylindroids *in vitro* (see, *e.g.,* Kasinskas and Forbes (2007) Cancer Res. 67(7):3201-3209), and motility has been shown to be important for tumor penetration (see, *e.g.,* Toley and Forbes (2012) Integr. Biol. (Camb) 4(2):165-176), flagella are not required for tumor colonization in animals when the bacteria are administered intravenously (see, *e.g.,* Stritzker et al. (2010) International Journal of Medical Microbiology 300:449-456). Each flagellar filament is composed of tens of thousands of flagellin subunits. The *S. typhimurium* chromosome contains two genes, *fliC* and *fljB*, that encode antigenically distinct flagellin monomers. Mutants defective for both *fliC* and *fljB* are nonmotile and avirulent when administered via the oral route of infection, but maintain virulence when administered parenterally.

Flagellin is a major pro-inflammatory determinant of *Salmonella* (see, *e.g.,* Zeng et al. (2003) J. Immunol. 171:3668-3674), and is directly recognized by TLR5 on the surface of cells, and by NLCR4 in the cytosol (see, *e.g.,* Lightfield et al. (2008) Nat. Immunol. 9(10):1171-1178). Both pathways lead to pro-inflammatory responses resulting in the secretion of cytokines, including IL-1β, IL-18, TNF-α, and IL-6. Attempts have been made to make *Salmonella-*based cancer immunotherapy more potent by increasing the pro-inflammatory response to flagellin by engineering the bacteria to secrete *Vibrio vulnificus* flagellin B, which induces greater inflammation than flagellin encoded by *fliC* and *fljB* (see, *e.g.,* Zheng et al. (2017) Sci. Transl. Med. 9(376):eaak9537).

Herein, *Salmonella* bacteria, such as *S. typhimurium,* are engineered to lack both flagellin subunits *fliC* and *fljB*, to reduce TLR5-mediated pro-inflammatory signaling. Other bacteria that contain flagella can be similarly engineered to eliminate flagella. For example, as shown herein, a *Salmonella* strain lacking *msbB* and/or *pagP,* which results in reduced TNF-alpha induction, is combined with *fliC* and *fljB* knockouts. This results in a *Salmonella* strain that has a combined reduction in TNF-alpha induction and a reduction in TLR5 recognition. These bacterial modifications, *msbB⁻, pagP⁻, fliC⁻,* and *fljB*⁻, can be combined with an immunostimulatory plasmid, optionally containing CpGs, encoding therapeutic products, such as immunomodulatory proteins, alone or in combinations thereof. The resulting bacteria have reduced pro-inflammatory signaling, but robust anti-tumor activity. These genome modifications can be combined with others of the genome modifications described herein as well.

For example, as exemplified and provided herein, a *fliC* and *fljB* double mutant was constructed in the *asd*-deleted strain of *S. typhimurium,* VNP20009. VNP20009, which is attenuated for virulence by disruption of *purI*/*purM,* contains a modification of the *msbB* gene (a partial deletion) that results in production of a lipid A subunit that is less toxigenic than wild-type lipid A. This results in reduced TNF-α production in a mouse model after intravenous administration, compared to strains with wild-type lipid A. The resulting strain is exemplary of strains that are attenuated for bacterial inflammation by modification of lipid A to reduce TLR2/4 signaling, and deletion of expression of the flagellin subunits to reduce TLR5 recognition and inflammasome induction.

Pathogenesis in certain bacterial species, including *Salmonella* species, such as *S. typhimurium,* involves a cluster of genes referred to as *Salmonella* pathogenicity islands (SPIs). *Salmonella* invades non-phagocytic intestinal epithelial cells using a type 3 secretion system (T3SS) encoded by the *Salmonella* pathogenicity island 1 (SPI-1), which forms a needle-like structure that injects effector proteins directly into the cytosol of host cells. These effector proteins lead to rearrangement of the eukaryotic cell cytoskeleton to facilitate invasion of the intestinal epithelium, and also induces proinflammatory cytokines. The SPI designated SPI-1 mediates invasion of epithelial cells. SPI-1 genes include, but are not limited to: *avrA, hilA, hilD, invA, invB, invC, invE, invF, invG, invH, invI, invJ, iacP, iagB, spaO, spaP, spaQ, spaR, spaS, orgA, orgB, orgC, prgH, prgI, prgJ, prgK, sicA, sicP, sipA, sipB, sipC, sipD, sirC, sopB, sopD, sopE, sopE2, sprB,* and *sptP.* Deletion of one or more of these genes reduces or eliminates the ability of the bacterium to infect epithelial cells, but does not affect their ability to infect or invade phagocytic cells, including phagocytic immune cells. For example, it was demonstrated that deletion of both the *fliC* and *fljB* genes significantly reduced expression of SPI-1 genes, such as *hilA, hilD, invA, invF* and *sopB,* thereby reducing the ability to invade non-phagocytic cells (see, *e.g.,* Elhadad et al. (2015) Infect. Immun. 83(9):3355-3368).

In bacteria such as *Salmonella,* flagellin, in addition to the SPI-1 type 3 secretion system (T3SS), is necessary for triggering pyroptosis in macrophages, and can be detected by the macrophage NLRC4 inflammasome. Elimination of flagellin subunits decreases pyroptosis in macrophages. For example, *S. typhimurium* with deletions in *fliC* and *fljB* results in significantly reduced IL-1β secretion compared to the wild-type strain, whereas cellular uptake and intracellular replication of the bacterium remains unaffected. This demonstrates that flagellin plays a significant role in inflammasome activation. Additionally, *S. typhimurium* strains engineered to constitutively express *fliC* were found to induce macrophage pyroptosis (see, *e.g.,* Li et al. (2016) Scientific Reports 6:37447; Fink and Cookson (2007) Cellular Microbiology 9(11):2562-2570; and Winter et al. (2015) Infect. Immun. 83(4):1546-1555).

The genome of the immunostimulatory bacteria herein can be modified to delete or mutate the flagellin genes *fliC* and *fljB* in *S. typhimurium,* leading to decreased cell death of tumor-resident immune cells, such as macrophages, and enhancing the anti-tumor immune response of the immunostimulatory bacteria. Deletion of the flagellin subunits, combined with modification of the LPS, allows for greater tolerability in the host, limits uptake into only phagocytic cells and decreases their pyroptotic cell death, and directs the immunostimulatory response towards delivery of therapeutic products, such as immunomodulatory proteins, to the TME, particularly tumor-resident myeloid cells. The resulting immunostimulatory bacteria elicit an anti-tumor response and promote an adaptive immune response to the tumor.

Corresponding genes, encoding flagellin in other bacterial species, also can be deleted to achieve similar results. These genes include, but are not limited to, for example, *fliC*, encoding flagellar filament structural protein, and *fliE,* encoding flagellar basal-body protein FliE in *E. coli*; *fliC,* encoding flagellin, and *flgB,* encoding flagellar basal-body rod protein FlgB, in *S. typhi*; *flaA* encoding flagellin, *fliE,* encoding flagellar hook-basal body protein FliE, and flgB, encoding flagellar basal-body rod protein FlgB, in *L. monocytogenes*; and NT01CX_RS04995, NT01CX_RS04990, NT01CX_RS05070, and NT01CX_RS05075, encoding flagellin, NT01CX_RS05080 (*flgB*), encoding flagellar basal body rod protein FlgB, NT01CX_RS05085 (*flgC*), encoding flagellar basal body rod protein FlgC, and NT01CX_RS05215 (*flgG*)*,* encoding flagellar basal body rod protein FlgG, in *Clostridium novyi.*

### 5. Engineering Bacteria to Promote Adaptive Immunity and Enhance T-Cell Function

### L-asparaginase II (ansB) Deletion/Disruption

L-asparaginase II is an enzyme that catalyzes conversion of L-asparagine to ammonia and aspartic acid. Several bacterial strains, such as *E. coli* and *S*. *typhimurium,* utilize L-asparaginase to scavenge fructose-asparagine as a carbon and nitrogen source (see, *e.g.,* Sabag-Daigle et al. (2018) Appl. Environ. Microbiol. 84(5):e01957-17). Malignant T-cells, such as in acute lymphoblastic leukemia (ALL), require asparagine as they lack the enzymes to synthesize it. Administration of L-asparaginases has been a frontline therapy for ALL since the early 1970's (see, *e.g.,* Batool et al. (2016) Appl. Biochem. Biotechnol. 178(5):900-923). Production of L-asparaginase II by *S. typhimurium* is both necessary and sufficient for T-cell inhibition, as it directly induces T-cell receptor (TCR) downregulation, decreases T-cell cytokine production, and inhibits tumor cytolytic function (see, *e.g.,* Kullas et al. (2012) Cell Host Microbe. 12(6)791-798; and van der Velden et al. (2005) Proc. Natl. Acad. Sci. U.S.A. 102(49):17769-17774). Under rapid clonal expansion conditions, such as those that occur during T-cell activation in the tumor microenvironment, asparagine is required, and its depletion by L-asparaginase II leads to T-cell suppression. L-asparaginase II, thus, has been used as an anti-cancer therapeutic for cancers in which T-cell suppression is a therapeutic modality.

In contrast to the prior uses of L-asparaginase as an anti-cancer therapeutic, it is shown herein that elimination of L-asparaginase activity in the immunostimulatory bacteria provided herein enhances the function of T-cells in the tumor microenvironment. Elimination of L-asparaginase activity can be effected by modifying the bacterial genome to eliminate expression of active enzyme. Modifications include insertions, deletions, inversions, and replacements of nucleic acids, so that the resulting encoded enzyme is not active, or not expressed, or is eliminated. It is shown herein that deletion of all or of a part of the gene that encodes L-asparaginase II, *ansB,* or disruption thereof, to eliminate expression of the encoded enzyme in the immunostimulatory bacteria, enhances the function of T-cells in a bacterially-colonized tumor microenvironment. Inhibition of L-asparaginase II activity is accomplished by deletion of all or of a part of, or interruption/disruption of, the gene *ansB* in the immunostimulatory bacteria, whereby L-asparaginase II is not produced. Thus, provided are immunostimulatory bacteria whose genomes are modified so that L-asparaginase II is not produced. Immunostimulatory bacteria provided herein are employed to colonize tumor-resident immune cells to enhance the anti-tumor immune response; included among the genome modifications are deletions, insertions, disruptions, and/or other modifications that eliminate expression of L-asparaginase II.

As shown herein, the genome of the immunostimulatory bacteria herein can be modified to delete *ansB,* or to disrupt it or otherwise modify it, to result in inactive encoded L-asparaginase II, or to eliminate the asparginase, preventing T-cell suppression and enhancing anti-tumor T-cell function *in vivo.* It is shown herein that strains in which *ansB* is intact induce profound T-cell immunosuppression in T-cells infected with the strain. Strains in which *ansB* is deleted do not induce immunosuppression, thus, solving another problem in the art in using bacteria to deliver encoded therapeutic products to tumors. Thus, immunostimulatory bacteria that combine deletions or disruptions of the *ansB* gene, whereby functional encoded enzyme is not expressed, with other modifications described herein that result in increased accumulation in the tumor microenvironment and/or in tumor-resident immune cells, provide a superior therapeutic immunostimulatory bacteria.

Corresponding genes, encoding homologs or orthologs of L-asparaginase II (ansB) in other bacterial species, also can be deleted or disrupted to achieve similar results. These genes include, but are not limited to, for example, *ansB,* encoding L-asparaginase 2 in *E*. *coli; ansB* (STY3259), encoding L-asparaginase in *S. typhi; ansB* (lmo1663), encoding asparagine synthetase in *L. monocytogenes;* and BL1142, encoding an L-asparaginase precursor in *Bifidobacterium longum.*

### 6. Deletions/Disruptions in Salmonella Genes Required for Curli Fimbriae Expression

Bacteria and fungi are capable of forming multicellular structures called biofilms. Bacterial biofilms are encased within a mixture of secreted and cell wall-associated polysaccharides, glycoproteins, and glycolipids, as well as extracellular DNA, known collectively as extracellular polymeric substances. These extracellular polymeric substances protect the bacteria from multiple insults, such as cleaning agents, antibiotics, and antimicrobial peptides. Bacterial biofilms allow for colonization of surfaces, and are a cause of significant infection of prosthetics, such as injection ports and catheters. Biofilms also can form in tissues during the course of an infection, which leads to increases in the duration of bacterial persistence and shedding, and limits the effectiveness of antibiotic therapies. Chronic persistence of bacteria in biofilms is associated with increased tumorigenesis, for example in *S. typhi* infection of the gall bladder (see, *e.g.,* Di Domenico et al. (2017) Int. J. Mol. Sci. 18:1887).

In *Salmonella,* such as *S. typhimurium,* biofilm formation is regulated by *csgD,* which activates the *csgBAC* operon and results in increased production of the curli fimbriae subunits CsgA and CsgB (see, *e.g.,* Zakikhany et al. (2010) Molecular Microbiology 77(3):771-786). CsgA is recognized as a PAMP by TLR2 and induces production of IL-8 from human macrophages (see, *e.g.,* Tukel et al. (2005) Molecular Microbiology 58(1):289-304). Also, *csgD* indirectly increases cellulose production by activating the *adrA* gene that encodes for di-guanylate cyclase. The small molecule cyclic di-guanosine monophosphate (c-di-GMP), generated by *adrA,* is a ubiquitous secondary messenger that occurs in almost all bacterial species. Increases in c-di-GMP enhance expression of the cellulose synthase gene *bcsA,* which in turn increases cellulose production via stimulation of the *besABZC* and *bcsEFG* operons, leading to cellulose biofilm formation. As a result, bacteria, such as *S. typhimurium,* can form biofilms in solid tumors as protection against phagocytosis by host immune cells. Bacterial mutants, such as *Salmonella* mutants, that cannot form biofilms, are taken up more rapidly by host phagocytic cells and are more readily cleared from infected tumors (see, *e.g.,* Crull et al. (2011) Cellular Microbiology 13(8):1223-1233). This increase in intracellular localization within phagocytic cells can reduce the persistence of extracellular bacteria, and, as shown herein, can enhance the effectiveness of plasmid delivery of therapeutic products, such as immunomodulatory proteins and other anti-cancer therapeutics, as described herein. Reduction in the capability of immunostimulatory bacteria, such as *S. typhimurium,* to form biofilms, can be achieved through deletion or disruption of genes involved in biofilm formation, such as, for example, *csgD, csgA, csgB, adrA, bcsA, bcsB, bcsZ, bcsE, bcsF, bcsG, dsbA,* or *dsbB* (see, *e.g.,* Anwar et al. (2014) PLoS ONE 9(8):e106095).

It is shown herein that engineering the immunostimulatory bacteria to reduce biofilm formation increases clearance rates from tumors/tissues, increasing tolerability of the therapy, and prevents colonization of prosthetics in patients, thereby increasing the therapeutic benefit of these strains. It is known that adenosine mimetics inhibit *S. typhimurium* biofilm formation, indicating that the high adenosine concentration in the tumor microenvironment can contribute to tumor-associated biofilm formation (see, *e.g.,* Koopman et al. (2015) Antimicrob. Agents Chemother. 59:76-84). It is shown herein that *csgD*-deleted strains demonstrate improved anti-tumor efficacy because of greater bacterial uptake into tumor-resident myeloid cells.

Corresponding genes, encoding homologs and orthologs of *csgD,* and other genes that are required for curli fimbriae and biofilm formation in other bacterial species, also can be deleted or disrupted or otherwise modified to achieve similar results. These genes include, but are not limited to, for example, *csgD,* encoding DNA-binding transcriptional dual regulator CsgD in *E. coli; csgD* (STY1179), encoding regulatory protein CsgD in *S. typhi;* and *lcp,* encoding the *Listeria* cellulose binding protein that is involved in biofilm formation in *L. monocytogenes.*

Modification of the bacterial genome, such as by deletion or disruption of genes to render the bacteria *csgD⁻,* results in elimination of curli fimbriae and inflammatory cyclic dinucleotides (CDNs), and removes cellulose secretion. This eliminates inflammatory and immunosuppressive elements, prevents TLR4 recognition through altered LPS acylation, eliminates cellulose secretion, and, thus, possible biofilm formation, thereby increasing safety and efficacy.

As described herein, bacterial strains, such as *S. typhimurium* strains, that are engineered to be auxotrophic for adenosine; and are reduced in their ability to induce pro-inflammatory cytokines by modification of the LPS and/or deletion of flagellin; and/or that do not express L-asparaginase II to improve T-cell function; and/or that contain deletions of genes required for biofilm formation; and/or that are further modified to maintain significant plasmid copy number per cell, at least low to medium copy number or higher, in the absence of antibiotic selection; and that deliver genetic expression cassettes encoding therapeutic products, promote robust anti-tumor immune responses. The plasmids include regulatory sequences to promote secretion of the encoded therapeutic products into the tumor microenvironment.

### 7. Improving Resistance to Complement

The complement system is the first line of immune defense against invading pathogens that directly activate the lectin pathway or the alternative pathway (AP) cascades in the human host. The complement system involves more than 30 soluble and cell-membrane bound proteins that function in the innate immune response to recognize and kill pathogens, such as bacteria, virus-infected cells, and parasites, and also play a role in the antibody-mediated immune response. Activation of the complement cascade leads to opsonization of foreign microbes, release of chemotactic peptides, and finally, to disruption of bacterial cell membranes. Three homologous glycoproteins in the complement system, C3, C4 and C5, play a central role in complement function and interact with other complement components. C3b and C4b, generated from C3 and C4, respectively, are important components of convertases that promote activation of the complement cascade. The cleavage fragments of C5 are C5a, which induces migration of phagocytes into the infection site, and C5b, which initiates the formation of the membrane attack complex and bacterial lysis (see, *e.g.,* Ramu et al. (2007) FEBS Letters 581:1716-1720).

To survive, pathogens have developed strategies to prevent deleterious consequences of complement activation. For example, members of the Ail/Lom family of outer membrane proteins provide protection from complement-dependent killing for a number of pathogenic bacteria. Members of the Ail/Lom family, which include Ail (attachment invasion locus) of *Yersinia* species, *e.g., Y. enterocolitica* and *Y. pseudotuberculosis,* Rck (resistance to complement killing) and *PagC* of *Salmonella* species, and OmpX of *Escherichia coli,* are outer membrane proteins that share significant amino acid sequence similarity and identity, and have similar membrane topologies. While members of this family of proteins exhibit diverse functions, several of them, including Ail of *Y. enterocolitica* and *Y. pseudotuberculosis,* as well as Rck of *S. enterica,* function, at least in part, to protect bacteria from complement-mediated lysis (see, *e.g.,* Bartra et al. (2008) Infection and Immunity 76:612-622*).*

Another bacterial product that aids in avoiding or mitigating complement is the surface protease, designated PgtE (outer membrane serine protease) in *Salmonella,* and other members of the omptin family. The surface protease PgtE of *S. enterica* belongs to the omptin family of enterobacterial outer membrane aspartate proteases. PgtE and other omptins require rough LPS to be active, but are sterically inhibited by the O-antigen. Expression *of pgtE* is upregulated during the growth of *Salmonella* inside macrophages, and the bacteria released from macrophages exhibit strong PgtE-mediated proteolytic activity. PgtE proteolytically activates the mammalian plasma proenzyme plasminogen to plasmin, inactivates the main physiological inhibitor of plasmin, alpha 2-antiplasmin, and mediates bacterial adhesion to extracellular matrices of human cells. This way, PgtE mediates the degradation of extracellular matrix components and generates potent, localized proteolytic activity, which can promote migration of *Salmonella* across extracellular matrices. PgtE also degrades alpha-helical antimicrobial peptides which can be important during intracellular growth of *Salmonella.* The omptin Pla of *Yersinia pestis* is a close ortholog of PgtE and shares functions with PgtE. Pla cleaves C3, and PgtE increases serum resistance of *Salmonella* by cleaving complement components C3b, C4b, and C5. The gene *pgtE,* and orthologs thereof from other bacterial species, can be included in the immunostimulatory bacteria herein to increase resistance to complement.

It is shown herein that the effects of complement in human serum explain the failure of therapeutic immunostimulatory bacteria, such as the *Salmonella* strain VNP20009, which had been shown to effectively colonize tumors in rodent models. Systemic administration of VNP20009 resulted in colonization of mouse tumors (see, *e.g.,* Clairmont et al. (2000) J. Infect. Dis. 181:1996-2002; and Bermudes et al. (2001) Biotechnol. Genet. Eng. Rev. 18:219-33); whereas systemic administration of VNP20009 in human patients resulted in very little colonization. In the Phase 1 Study in advanced melanoma patients, very little VNP20009 was detected in human tumors after a 30 minute intravenous infusion (see, Toso et al. (2002) J. Clin. Oncol. 20:142-52). Patients that entered into a follow-up study evaluating a longer, four hour infusion of VNP20009, also demonstrated a lack of detectable VNP20009 after tumor biopsy (see, Heimann et al. (2003) J. Immunother. 26:179-180). Following intratumoral administration, colonization of a derivative of VNP20009 was detected (see, Nemunaitis et al. (2003) Cancer Gene Ther. 10:737-744). Direct intratumoral administration of VNP20009 to human tumors resulted in much higher tumor colonization, indicating that human tumors can be colonized at a high level, and that the difference in tumor colonization between mice and humans occurs only after systemic administration.

It is shown and described herein, that, while not previously known to occur in wild-type *S. typhimurium,* VNP20009 is inactivated by human complement, which explains the low tumor colonization observed in humans upon systemic administration of VNP20009. Strains provided herein exhibit resistance to complement. They can be modified to express Rck and other proteins involved in mediating complement resistance or avoidance, such as Ail of *Yersinia enterocolitica,* or PgtE of *Salmonella typhimurium,* or, if they natively express such a protein, they can be modified to overexpress Rck and/or other such proteins. Rck can be introduced into bacteria, such as *E. coli,* that lack a homolog.

### Rck Expression

Rck (resistance to complement killing) is a 17 kDa outer membrane protein encoded by the large virulence plasmid of *Salmonella* species, such as *S. enteritidis* and *S. typhimurium,* that induces adhesion to and invasion of epithelial cells. The Rck protein has been shown to protect *S. enterica* from complement by inhibiting C9 polymerization and subsequent assembly of a functional membrane attack complex. An *rck* mutant exhibited a 2-3 fold decrease in epithelial cell invasion compared to the wild-type strain, while *rck* overexpression in wild-type leads to increased invasion. The Rck protein induces cell entry by a receptor-mediated process, promoting local actin remodeling, and weak and closely adherent membrane extensions. Thus, *Salmonella* can enter cells by two distinct mechanisms: the Trigger mechanism mediated by the T3SS-1 complex, and a Zipper mechanism induced by *rck* (see, *e.g.,* Manon et al. (2012), Salmonella, Chapter 17, eds. Annous and Gurtler, Rijeka, pp. 339-364). Expression of *rck* on the *Salmonella* virulence plasmid confers a high level of resistance to neutralization by human complement, by preventing the formation of the membrane attack complex. When the *S. typhimurium* virulence plasmid containing *rck was* expressed in a highly serum-sensitive strain of *E. coli,* Rck was able to restore complement resistance.

The immunostimulatory bacteria provided herein retain, or are provided with, Rck to confer resistance to human complement. It is shown herein that immunostimulatory bacteria, such as *E*. *coli,* can be modified by encoding *rck* on a plasmid in the bacteria to thereby confer resistance to complement. Immunostimulatory bacteria provided herein encode *rck,* either endogenously, or can be modified to encode it in order to increase resistance to complement. Methods for conferring resistance to complement also are provided. For example, the therapeutic *E. coli* species described in U.S. Patent Application Publication Nos. 2018/0325963 and 2018/0273956, and U.S. Patent Nos. 9,889,164 and 9,688,967 can be improved by modifying the bacteria therein, such as by introducing nucleic acid encoding the *Salmonella rck* gene on a plasmid therein, to thereby improve or provide resistance to complement. Bacteria that are resistant to complement can be systemically administered, and sufficient bacteria can survive to be therapeutically effective. Nucleic acids encoding the *Salmonella rck* gene are introduced into bacteria, such as therapeutic *E*. *coli,* to thereby confer or increase complement resistance.

Other orthologs and homologs of *rck* from other bacterial species, similarly can be expressed in the immunostimulatory bacteria. For example, Ail is an Rck homolog from *Yersinia enterocolitica,* which enhances complement resistance under heterologous expression. PgtE is an *S. typhimurium* surface protease that has also been shown to enhance complement resistance under heterologous expression.

### 8. Deletions of Genes Required for Lipoprotein Expression in Salmonella and Other Gram-Negative Bacteria

The LPS and Braun (murein) lipoprotein (Lpp) are major components of the outer membrane of Gram-negative enteric bacteria that function as potent stimulators of inflammatory and immune responses. Braun (murein) lipoprotein (Lpp) is one of the most abundant components of the outer membrane in *S. typhimurium,* and leads to TLR2 induction of pro-inflammatory cytokines, such as TNFα, IL-6 and IL-8 (in humans). Two functional copies of the lipoprotein gene (*lppA* (SEQ ID NO:387) and *lppB* (SEQ ID NO:388)), that are located on the bacterial chromosome of *Salmonella,* contribute to bacterial virulence. Deletion of the *lppA* and *lppB* genes, and elimination of lipoprotein expression, reduces virulence and decreases pro-inflammatory cytokine production (see, *e.g.,* Sha et al. (2004) Infect. Immun. 72(7):3987-4003; Fadl et al. (2005) Infect. Immun. 73(2):1081-1096). Deletion of the Lpp genes would be expected to reduce infection of cells, and, thus, decrease plasmid delivery and expression of the encoded therapeutic products or proteins. As shown in Example 18 below, however, while deletion of these genes did reduce tumor colonization, the amount of plasmid delivered to the targeted cells, the tumor-resident immune cells, particularly macrophages, significantly was increased. As shown herein, deletion or disruption of these genes (*lppA* and *lppB),* thus, resulted in decreased virulence due to the inability to survive in infected macrophages, but resulted in enhanced plasmid delivery of the immunostimulatory bacteria, thereby increasing expression of encoded therapeutic genes in the targeted cells, *i.e.,* the tumor-resident immune cells, particularly macrophages.

### 9. Robust Immunostimulatory Bacteria Whose Genomes are Modified to be Optimized for Anti-Tumor Therapy, and that Encode Therapeutic Products, Including a Plurality Thereof

As described herein, bacterial strains, such as *S. typhimurium* strains, that are engineered to be adenosine auxotrophic, and are reduced in their ability to induce pro-inflammatory cytokines by modification of the LPS and/or deletion of flagellin, and/or are modified by deletion or elimination of L-asparaginase II expression to improve T-cell function, and/or are modified by deletion or disruption of genes required for biofilm formation, and/or that demonstrate enhanced human serum survival due to increased *rck* expression, are further modified to deliver therapeutic products, such as immunomodulatory proteins, and promote robust anti-tumor immune responses.

The table below summarizes the bacterial genotypes/modifications, their functional effects, and some of the effects/benefits achieved herein.

| **Genotype/Modification** | **Functional effect** | **Effect/Benefit** |
|---|---|---|
| Δ*asd* (in genome) | Plasmid maintenance | Improves plasmid delivery |
| | | Plasmid maintenance *in vivo* via *asd* cassette on plasmid |
| Δ*purI* | Purine/adenosine auxotrophy | Tumor-specific enrichment |
| | | Limited replication in healthy tissue |
| Δ*msbB* | LPS surface coat modification | Decreases TLR4 recognition |
| | | Reduces immunosuppressive cytokine profile (TNF-α) |
| | | Improves safety |
| | | Prevents intracellular replication |
| Δ*fliC*/Δ*fljB* (ΔFLG) | Flagella knockout | Removes major inflammatory and immune-suppressive element |
| | | Eliminates TLR5 recognition |
| | | Reduces immunosuppressive cytokine profile |
| | | Improves safety |
| | | Reduces ability to invade non-phagocytic cells (*e.g*., stromal and tumor cells) |
| Δ*pagP* | LPS surface coat modification | Removes major inflammatory and immunosuppressive element |
| | | Decreases TLR4 recognition |
| | | Reduces IL-6 production |
| | | Improves safety |
| Δ*ansB* | L-asparaginase II knockout | Enhances tumor T-cell function |
| Δ*csgD* | Removes curli fimbriae, cellulose production, c-di-GMP | Reduces inflammation |
| | | Prevents possible biofilm formation |
| | | Enhances phagocytic cell uptake |
| Plasmid | Expresses gene products under control of host-recognized promoter | Eukaryotic promoter limits expression to cells containing the plasmid |
| | | Long term expression in the TME (*i.e., asd* encoded on plasmid under control of host-recognized promoter) |
| | | Expression of any combination of therapeutic product(s) with large capacity |
| | | CpGs to induce proper viral-like innate immune response |

Strains provided herein are ΔFLG, and/or Δ*pagP,* and/or Δ*ansB,* and/or Δ*csgD.* Additionally, the strains are one or more of Δ*purI* (Δ*purM*)*, ΔmsbB,* and Δ*asd* (in the bacterial genome). In particular, the strains are Δ*purI* (Δ*purM*)*, ΔmsbB,* Δ*pagP,* and Δ*ansB,* and Δ*asd.* The strains also can be *lppA⁻* and/or *lppB⁻,* particularly *lppA⁻*/*lppB⁻.* The plasmid is modified to encode therapeutic products under control of host-recognized promoters (*e.g.*, eukaryotic promoters, such as RNA polymerase II promoters, including those from eukaryotes, and animal viruses). The plasmids can encode *asd* to permit bacterial replication *in vivo,* and can encode nucleic acids with other beneficial functions (such as CpGs), and can encode gene products, as described elsewhere herein.

The immunostimulatory bacteria provided herein can be modified to eliminate the ability to infect epithelial cells, such as by elimination of the flagella. Elimination of the ability to infect epithelial cells, as described elsewhere herein, also can be achieved by inactivating SPI-1-dependent invasion, through inactivation or knockout of one or more genes involved in the SPI-1 pathway. These genes include, but are not limited to, one more of: *avrA, hilA, hilD, invA, invB, invC, invE, invF, invG, invH, invI, invJ, iacP, iagB, spaO, spaP, spaQ, spaR, spaS, orgA, orgB, orgC, prgH, prgI, prgJ, prgK, sicA, sicP, sipA, sipB, sipC, sipD, sirC, sopB, sopD, sopE, sopE2, sprB,* and *sptP.* Additionally or alternatively, the immunostimulatory bacteria can contain knockouts or deletions in genes to inactivate products involved in SPI-1-independent infection/invasion, such as one or more of the genes *fljB, fliC, rck, pagN, hlyE, pefI, srgD, srgA, srgB,* and *srgC,* and/or the immunostimulatory bacteria can contain knockouts or deletions to inactivate products of genes that induce cell death of tumor-resident immune cells, such as genes that encode proteins that are directly recognized by the inflammasome, including *fljB*, *fliC*, *prgI* (needle protein), and *prgJ* (rod protein). The *rck* gene, however, is desirable because it protects against inactivation against complement. Bacteria that do not endogenously encode *rck,* can be modified to encode a heterologous *rck* gene.

The immunostimulatory bacteria are derived from suitable bacterial strains. Bacterial strains can be attenuated strains, or strains that are attenuated by standard methods, or that, by virtue of the modifications provided herein, are attenuated in that their ability to colonize is limited primarily to immunoprivileged tissues and organs, particularly tumor-resident immune cells, the TME, and tumor cells, including solid tumors. Bacteria include, but are not limited to, for example, strains of *Salmonella, Shigella, Listeria, E. coli,* and *Bifidobacteriae.* For example, species include *Shigella sonnei, Shigella flexneri, Shigella dysenteriae, Listeria monocytogenes, Salmonella typhi, Salmonella typhimurium, Salmonella gallinarum,* and *Salmonella enteritidis.* Other suitable bacterial species include *Rickettsia, Klebsiella, Bordetella, Neisseria, Aeromonas, Francisella, Corynebacterium, Citrobacter, Chlamydia, Haemophilus, Brucella, Mycobacterium, Mycoplasma, Legionella, Rhodococcus, Pseudomonas, Helicobacter, Vibrio, Bacillus,* and *Erysipelothrix.* For example, *Rickettsia rickettsii, Rickettsia prowazekii, Rickettsia tsutsugamuchi, Rickettsia mooseri, Rickettsia sibirica, Bordetella bronchiseptica, Neisseria meningitidis, Neisseria gonorrhoeae, Aeromonas eucrenophila, Aeromonas salmonicida, Francisella tularensis, Corynebacterium pseudotuberculosis, Citrobacter freundii, Chlamydia pneumoniae, Haemophilus somnus, Brucella abortus, Mycobacterium intracellulare, Legionella pneumophila, Rhodococcus equi, Pseudomonas aeruginosa, Helicobacter mustelae, Vibrio cholerae, Bacillus subtilis, Erysipelothrix rhusiopathiae, Yersinia enterocolitica, Rochalimaea quintana,* and *Agrobacterium tumerfacium.*

Exemplary of the immunostimulatory bacteria provided herein are species of *Salmonella.* Exemplary of bacteria for modification as described herein are wild-type strains of *Salmonella,* such as the strain that has all of the identifying characteristics of the strain deposited in the American Type Culture Collection (ATCC) as accession #14028. Engineered strains of *Salmonella typhimurium,* such as strain YS1646 (ATCC catalog # 202165, also referred to as VNP20009; see, also, International PCT Application Publication No. WO 99/13053), is engineered with plasmids to complement an *asd* gene knockout and to allow for antibiotic-free plasmid maintenance. The strains then are modified to delete the flagellin genes, and/or to delete *pagP.* The combination of flagella knockout and *pagP* deletion renders the strain highly resistant to human serum complement. The strains also are rendered auxotrophic for purines, particularly adenosine, and are *asd⁻* and *msbB⁻.* As exemplified, strains in which *purI* and *msbB* are completely deleted are more fit (grow faster) that strain VNP20009, in which these genes are not deleted, but are modified to eliminate expression. The *asd* gene can be provided on a plasmid for *in vivo* replication in the eukaryotic host. The strains also have a modification, such as a deletion, disruption, or other modification, in the *ansB* gene, preventing them from producing immunosuppressive L-asparaginase II, and improving tumor T-cell function. The strains also are modified to eliminate biofilm production, such as by a *csgD* deletion, which renders them unable to produce curli fimbriae, cellulose, and c-di-GMP, reducing unwanted inflammatory responses, and preventing them from forming biofilms.

These genomic deletions and plasmids are described and exemplified elsewhere herein. Any of the nucleic acid encoding therapeutic products, such as immunostimulatory proteins and other products, described elsewhere herein and/or known to those of skill in the art, can be included on the plasmid. The plasmid generally is present in low to medium copy number, as described elsewhere herein. Therapeutic products include gain-of-function mutants of cytosolic DNA/RNA sensors, that can constitutively evoke/induce type I IFN expression, and other immunostimulatory proteins, such as cytokines, chemokines, and co-stimulatory molecules, that promote an anti-tumor immune response in the tumor microenvironment, and other such products described herein. The plasmids also can encode antibodies, and fragments thereof, *e.g*., single chain antibodies, that target immune checkpoints and other cancer targets, such as VEGF, IL-6, and TGF-β, and other molecules, such as bispecific T-cell engagers, or BiTEs^{®}. The plasmids also can encode IL-6 binding decoy receptors, TGF-beta binding decoy receptors, and TGF-beta polypeptide antagonists. As described below, the plasmid can encode one or a plurality of therapeutic products/genetic payloads (*i.e.,* multiplexed), for delivery of anti-cancer therapeutic products to the tumor/tumor microenvironment. The products can be operatively linked to trafficking signals, such as signals for secretion. The products also can be designed for expression on a cell surface, such as in tumor-resident myeloid cells.

### 10. Conversion of M2 Phenotype Macrophages into M1 and M1-Like Phenotype Macrophages

As described herein, the immunostimulatory bacteria provided herein accumulate in and/or target macrophages. Macrophages are phagocytic immune cells; they play a role in clearing senescent and apoptotic cells, as well as in the phagocytosis of immune-related complexes and pathogens, and in the maintenance of homeostasis. The phenotype and function of macrophages can be polarized by the microenvironment. There are two types: M1-type (classically activated macrophage), and M2-type (alternatively activated macrophage).

The role of M1 macrophages is to secrete pro-inflammatory cytokines and chemokines, and to present antigens, and thus, to participate in the positive immune response and function as an immune monitor. **M1** macrophages produce pro-inflammatory cytokines, including IL-6, IL-12, and TNF-α. M2 macrophages secrete arginase 1, IL-10, TGF-β, and other anti-inflammatory cytokines, which have the function of reducing inflammation, and contributing to tumor growth and immunosuppressive function. Thus, for treatment of cancers and other such diseases and disorders, the M1 or M1-like phenotype is advantageous.

M2 macrophages can be converted into M1 macrophages or into macrophages with an M1-like phenotype. Immunostimulatory bacteria provided herein, which infect macrophages, can convert M2 macrophages into an M1 or M1-like phenotype. M1 macrophage phenotypic markers include CD80 (also known as B7, B7.1, or BB1), CD86 (also known as B7.2), CD64 (also known as high affinity immunoglobulin gamma Fc receptor I), CD16, and CD32 (also known as low affinity immunoglobulin gamma Fc receptor IIb). Expression of nitric oxide synthase (iNOS) in M1 macrophages also can serve as a phenotypic marker. CD163 and CD206 are markers for the identification of M2 macrophages. Arginase 1 (Arg1) and DECTIN-1 also are ideal phenotypic indicators for the identification of M2 macrophages. Thus, the conversion can be monitored or assessed by virtue of expression of these markers.

Tumor-associated macrophages (TAMs) are associated with an immunosuppressive M2 phenotype. Immunostimulatory bacteria provided herein can convert such macrophages into an M1 or M1-like phenotype. The immunostimulatory bacteria provided herein, that encode a therapeutic product that leads to expression of type I interferon (IFN), can effect such conversion. This is a property unique to the immunostimulatory bacteria provided herein, and exploits the ability of the bacteria that include genomic modifications that result in the infection of macrophages. The encoded therapeutic products include those that are part of a cytosolic DNA/RNA sensor pathway, such as the STING variants (described in detail herein). The encoding immunostimulatory bacteria can effect conversion to an M1 phenotype (or an M1-like phenotype) upon infection of the tumor-resident macrophages, and expression of the therapeutic product(s). This ability to convert macrophage phenotypes is demonstrated and exemplified in Example 12 below. The expression of a modified STING protein by immunostimulatory bacteria provided herein that infect macrophages and express the STING protein, converts the phenotype of M1 macrophages to M2 macrophages.

Immunostimulatory bacteria provided herein, that include genome modifications as descrbied herein, such as the elimination of flagella and LPS modification, convert infected M2 macrophages into those that induce cytokine profiles of M1 macrophages. Immunostimulatory bacteria that express a variant STING protein that results in constitutive type I IFN expression in human primary M2 macrophages, convert these cells to M1-like (having phenotypic markers and/or expression profiles typical of M1 macrophages) type I IFN producing cells.

The Examples demonstrate this change from an M2 to an M1-like or M1 phenotype. A comparison between the cytokine profiles in uninfected M2 macrophage with the induced cytokines in M2 macrophage infected with a salmonella strain that is Δ*asd*/Δ*FLG*/Δ*pagP*/Δ*ansB*/Δ*csgD* M2 induced high level of IFNγ, CXCL10 and CXCL11 secretions. Infection with the same strain that was transformed with plasmids encoding huSTING tazCTT N154S + R284G variant, WT huIL-12 and huSTING tazCTT N154S + R284G variant or WT huIL-15 induced higher CXCL10 and CXCL11 secretions than the untransformed Δ*FLG*/Δ*pagP*/Δ*ansB*/Δ*csgD* strain not containing a plasmid. Cytokine profiles, characteristic of M1 or M1-like phenotypes, were induced with a variety of different payloads in the strains. The exemplary results are detailed in the Examples.

### D. IMMUNOSTIMULATORY BACTERIA WITH ENHANCED THERAPEUTIC INDEX ENCODING GENETIC PAYLOADS THAT STIMULATE THE IMMUNE RESPONSE IN THE TUMOR MICROENVIRONMENT

The immunostimulatory bacteria provided herein are modified so that they accumulate in the tumor microenvironment, and in tumor-resident myeloid cells, where therapeutic products, under the control of eukaryotic promoters, are expressed. The bacteria encode therapeutic products, particularly anti-cancer products, including products that stimulate the immune system and/or that reverse or mitigate the immunosuppressive effects of tumors. As described herein, the bacteria can encode a plurality of products, where expression of each product is under control of a separate promoter, or they are under control of one promotor, and can include sequences that result in expression of the discrete products, and, where appropriate, include regulatory sequences to ensure secretion of the encoded products into the tumor microenvironment. The immunostimulatory bacteria express encoded therapeutic products on the plasmid. As discussed, the plasmid can encode one product or a plurality thereof. Each product can be under control of a different eukaryotic promoter, or multiple encoded products can be expressed under control of a single promoter, such as by including 2A self-cleaving peptides between the coding portions, such as T2A (SEQ ID NO:327), P2A (SEQ ID NO:328), E2A (SEQ ID NO:329), and F2A (SEQ ID NO:330). The encoded products include those described herein, and they can be anti-cancer immune stimulating products whose activities are complementary. The immunostimulatory bacteria provided herein permit the combinatorial administration of multiple immunomodulatory products or payloads (multiplexed payloads) that would otherwise be too toxic if systemically administered. Exemplary of multiplexed payloads include one or more cytokine(s), an immunostimulatory protein to stimulate or induce expression of type I IFN, such as STING or a variant thereof that has increased activity or that is constitutively active, and a co-stimulatory molecule, such as an engineered 4-1BBL co-stimulatory molecule. Provided herein is a modified 4-1BBL polypeptide, and encoding nucleic acid, that exhibits improved expression and activity when encoded on a plasmid in the immunostimulatory bacteria provided herein that deliver the plasmids to myeloid cells for expression under control of the host transcriptional and translational machinery.

The immunostimulatory bacteria provided herein have strong anti-tumor effects, including provision of cures, such as after IV dosing with the multiplexed payloads or single agent payloads. The immunostimulatory bacteria, when systemically administered, infiltrate and enrich in solid tumors, the TME, and tumor-resident myeloid cells, in which the encoded therapeutic products are expressed and then locally delivered to the tumor microenvironment. Upon consumption (phagocytosis) by tumor-resident myeloid cells, the bacteria deliver a genetic payload-encoding plasmid, which allows for ectopic, single or multiplexed payload expression in a tumor-specific manner.

### 1. Immunostimulatory Proteins

The immunostimulatory bacteria herein can be modified to encode one or more of an immunostimulatory protein that promotes, induces, or enhances an anti-tumor response. As exemplified and described in the Examples, the order in which the encoding nucleic acids are arranged on the plasmid can improve overall expression, and modifications to the plasmids can improve the fitness of the bacteria that contain the plasmids encoding the proteins.

The immunostimulatory protein can be encoded on a plasmid in the bacterium, under the control of a eukaryotic promoter, such as a promoter recognized by RNA polymerase II, for expression in a eukaryotic subject, particularly the subject for whom the immunostimulatory bacterium is to be administered, such as a human. The nucleic acid encoding the immunostimulatory protein(s) can include, in addition to the eukaryotic promoter, other regulatory signals for expression or trafficking in the cells, such as for secretion or expression on the surface of a cell.

Immunostimulatory proteins are those that, in the appropriate environment, such as a tumor microenvironment (TME), can promote, or participate in, or enhance, an anti-tumor response by the subject to whom the immunostimulatory bacterium is administered. Immunostimulatory proteins include, but are not limited to, cytokines, chemokines, and co-stimulatory molecules. These include cytokines, such as, but not limited to, IL-2, IL-7, IL-12, IL-15, IL-18, IL-21, IL-23, IL-12p70 (IL-12p40 + IL-12p35), IL-15/IL-15R alpha chain complex, IL-36y, GM-CSF, IFNα, IFNβ, IL-2 that has attenuated binding to IL-2Ra, and IL-2 that is modified so that it does not bind to IL-2Ra; chemokines, such as, but not limited to, CCL3, CCL4, CCL5, CXCL9, CXCL10, and CXCL11; and/or co-stimulatory molecules, such as, but not limited to, CD40, CD40L, OX40, OX40L, 4-1BB, 4-1BBL, 4-1BBL with the cytoplasmic domain truncated or deleted (4-1BBLΔcyt), members of the TNF/TNFR superfamily (*e.g.,* CD27 and CD27L), and members of the B7-CD28 family (*e.g.,* CD80, CD86, ICOS, and ICOS ligand (B7RP1)).

Other such immunostimulatory proteins, that are used for the treatment of tumors, or that can promote, enhance or otherwise increase or evoke an anti-tumor response, known to those of skill in the art, are contemplated for encoding in the immunostimulatory bacteria provided herein. For example, the immunostimulatory bacteria can deliver a genetic payload encoding a truncated co-stimulatory molecule (*e.g.,* 4-1BBL, CD80, CD86, CD27L, B7RP1, and OX40L), with a full or partial cytoplasmic domain deletion, for expression on an APC, where the truncated gene product is capable of constitutive immuno-stimulatory signaling to a T-cell through co-stimulatory receptor engagement, and is unable to counter-regulatory signal to the APC due to a deleted or truncated cytoplasmic domain. As described elsewhere herein, the modified truncated cytoplasmic domain, for example, of 4-1BBL, contains particular residues to ensure proper orientation of the protein domains, which increases expression of the protein. Deletion (full or partial) and modification of the cytoplasmic domain of co-stimulatory molecules, as described herein, potentiates the activation of the co-stimulatory molecule, without the immunosuppressive reverse signaling. This is exemplified with respect to 4-1BBL as described in the Examples and as follows; the same modifications, including replacement of residues in the truncated cytoplasmic domain to ensure proper orientation in the membrane, can be applied to any of the co-stimulatory molecules, as well as other transmembrane polypeptides.

The full-length sequence of human 4-1BBL (SEQ ID NO:389 see also, Uniprot P41273) is: 
MEYASDASLDPEAPWPPAPRARACRVLPWALVAGLLLLLLLAAACAVFLACPWAVS
GARASPGSAASPRLREGPELSPDDPAGLLDLRQGMFAQLVAQNVLLIDGPLSWYSDP
GLAGVSLTGGLSYKEDTKELVVAKAGVYYVFFQLELRRVVAGEGSGSVSLALHLQP
LRSAAGAAALALTVDLPPASSEARNSAFGFQGRLLHLSAGQRLGVHLHTEARARHA
WQLTQGATVLGLFRVTPEIPAGLPSPRSE.
 where the ctoplasmic domain corresponds to amino acids 1-28 (italicized), the transmembrane domain corresponds to amino acids 29-49 (bold), and the extracellular domain corresponds to amino acids 50-254 (underlined). The human 4-1BBLΔcyt sequence (see, SEQ ID NO:390) is: 
MWALVAGLLLLLLLAAACAVFLACPWAVSGARASPGSAASPRLREGPELSPDDPA
GLLDLRQGMFAQLVAQNVLLIDGPLSWYSDPGLAGVSLTGGLSYKEDTKELVVAKA
GVYYVFFQLELRRVVAGEGSGSVSLALHLQPLRSAAGAAALALTVDLPPASSEARNS
AFGFQGRLLHLSAGQRLGVHLHTEARARHAWQLTQGATVLGLFRVTPEIPAGLPSPR
SE.
 which is the same as the full-length protein, but lacking the cytoplasmic domain, so that the transmembrane domain corresponds to amino acid residues 2-22 (bold), and the extracellular domain corresponds to amino acid residues 23-227 (underlined).

An exemplary human 4-1BBL, with a truncated cytoplasmic domain is as follows (see, SEQ ID NO:391): 
MRVLPWALVAGLLLLLLLAAACAVFLACPWAVSGARASPGSAASPRLREGPELSPD
DPAGLLDLRQGMFAQLVAQNVLLIDGPLSWYSDPGLAGVSLTGGLSYKEDTKELVV
AKAGVYYVFFQLELRRVVAGEGSGSVSLALHLQPLRSAAGAAALALTVDLPPASSEA
 
RNSAFGFQGRLLHLSAGQRLGVHLHTEARARHAWQLTQGATVLGLFRVTPEIPAGLP
SPRSE.
 where the truncated cytoplasmic domain corresponds to residues RLVP (in *italics*)*,* with the initiating M, the transmembrane domain corresponds to residues 6-26 (bold), and the extracellular domain corresponds to residues 27-231 (underlined).

With respect to the full-length 4-1BBL, positively charged amino acids, such as R and K, tend to be positioned in the cytoplasm (inside/cytoplasmic domain), which orients the transmembrane domain so that N-terminus is inside. But when the cytoplasmic domain is truncated, this alters the charge balance, so that there are only positive charges on the outside (in the extracellular domain). This favors a configuration in which the N-terminus of the protein is on the outside, not towards the cytoplasm, resulting in an "inside out configuration." If this is observed, such as by apparent lower activity or expression or other parameters, the 4-1BBL variant with the truncated cytoplasmic domain can be modified to include positive residues, to ensure the proper orientation of the protein in the cell membrane upon expression. Exemplary of possible modifications of 4-1BBL are those in which residues are replaced with positively charged residues, or a c-myc tag is included. The skilled person can envision other similar replacements/additions to achieve the same result.

Exemplary modified human 4-1BBL variants with a truncated cytoplasmic domain include the following, in which extra positive residues (Arginine (R), Lysine (K), italicized) are included in the cytoplasmic domain region, as follows, so that the resulting protein, when expressed in a cell, is properly oriented (has the correct configuration and not the "inside out" configuration). See, SEQ ID NOs:391 and 392, respectively:

### Truncated cytoplasmic domain:

MRVLPWALVAGLLLLLLLAAACAVFLACPWAVSGARASPGSAASPRLREGPELSP
DDPAGLLDLRQGMFAQLVAQNVLLIDGPLSWYSDPGLAGVSLTGGLSYKEDTKELV
VAKAGVYYVFFQLELRRVVAGEGSGSVSLALHLQPLRSAAGAAALALTVDLPPASSE
ARNSAFGFQGRLLHLSAGQRLGVHLHTEARARHAWQLTQGATVLGLFRVTPEIPAG
LPSPRSE

This adds a positive charge back to the N-terminus (*R*), which favors a configuration in which the N-terminus is correctly oriented inside the cytoplasm. In another example a MYC tag is added.

### Truncated cytoplasmic domain with a MYC tag:

MEQKLISEEDLRVLPWALVAGLLLLLLLAAACAVFLACPWAVSGARASPGS
AASPRLREGPELSPDDPAGLLDLRQGMFAQLVAQNVLLIDGPLSWYSDPGLA
GVSLTGGLSYKEDTKELVVAKAGVYYVFFQLELRRVVAGEGSGSVSLALHL
QPLRSAAGAAALALTVDLPPASSEARNSAFGFQGRLLHLSAGQRLGVHLHTE
ARARHAWQLTQGATVLGLFRVTPEIPAGLPSPRSE

For sequences of full-length mouse 4-1BBL, and exemplary sequences of mu4-1BBLΔcyt (murine 4-1BBL with a deletion of the cytoplasmic domain), mu4-1BBL with a truncated cytoplasmic domain, and mu4-1BBL with a truncated cytoplasmic domain and a MYC tag, see Example 19 below; see, also, SEQ ID NOs:393-396, respectively.

Additional or alternative amino acid replacements can be included in the co-stimulatory molecules to ensure proper orientation of the expressed protein in the membrane. The skilled person readily can prepare other similar modifications to ensure proper orientation of a transmembrane protein with a truncated cytoplasmic domain.

In addition to deletion or truncation of the cytoplasmic domain, co-stimulatory molecules (e.g., 4-1BBL, CD80, CD86, CD27L, B7RP1, and OX40L), for expression on an APC, also can be modified by introducing amino acid modifications, such as insertions, deletions, and/or replacements, to the cytoplasmic domain, such that the modified gene product is capable of constitutive immuno-stimulatory signaling to a T-cell through co-stimulatory receptor engagement, and is unable to counter-regulatory signal to the APC due to the modifications to the cytoplasmic domain. For example, the immunosuppressive reverse (intracellular) signaling can be eliminated by modifying the cytoplasmic domain phosphorylation sites, such as by replacing one or more Ser residues, at an appropriate locus or loci, with a residue that reduces or eliminates reverse signaling. For example, for human 4-1BBL, the immunosuppressive reverse (intracellular) signaling can be eliminated by modifying the cytoplasmic domain phosphorylation sites, including Ser5 and Ser8, with reference to the sequence of full-length human 4-1BBL (SEQ ID NO:389). The serine residues in the cytoplasmic domain can be replaced by any other residue that reduces or eliminates reverse signaling.

Additional or alternative amino acid replacements can be included in the co-stimulatory molecules to eliminate immunosuppressive intracellular (reverse) signaling. The skilled person readily can prepare other similar modifications to eliminate immunosuppressive reverse signaling, while still maintaining the co-stimulatory molecule's ability to activate constitutive immuno-stimulatory signaling to a T-cell through co-stimulatory receptor engagement.

### a. Cytokines and Chemokines

In some embodiments, the immunostimulatory bacteria herein are engineered to express cytokines to stimulate the immune system, including, but not limited to, IL-2, IL-7, IL-12, IL-12p70 (IL-12p40 + IL-12p35), IL-15 (and the IL-15:IL-15R alpha chain complex), IL-18, IL-21, IL-23, IL-36γ, IL-2 that has attenuated binding to IL-2Ra, IL-2 that is modified so that it does not bind to IL-2Ra, IFN-α, and IFN-β. Cytokines stimulate immune effector cells and stromal cells at the tumor site, and enhance tumor cell recognition by cytotoxic cells. In some embodiments, the immunostimulatory bacteria can be engineered to express chemokines, such as, for example, CCL3, CCL4, CCL5, CXCL9, CXCL10, and CXCL11.

### IL-2

Interleukin-2 (IL-2), which was the first cytokine approved for the treatment of cancer, is implicated in the activation of the immune system by several mechanisms, including the activation and promotion of cytotoxic T lymphocyte (CTL) growth, the generation of lymphokine-activated killer (LAK) cells, the promotion of Treg cell growth and proliferation, the stimulation of tumor-infiltrating lymphocytes (TILs), and the promotion of T-cell, B cell and NK cell proliferation and differentiation. Recombinant IL-2 (rIL-2) is FDA-approved for the treatment of metastatic renal cell carcinoma (RCC) and metastatic melanoma (see, *e.g.,* Sheikhi et al. (2016) Iran J. Immunol. 13(3):148-166).

### IL-7

IL-7, which is a member of the IL-2 superfamily, is implicated in the survival, proliferation and homeostasis of T-cells. Mutations in the IL-7 receptor have been shown to result in the loss of T-cells, and the development of severe combined immunodeficiency (SCID), highlighting the critical role that IL-7 plays in T-cell development. IL-7 is a homeostatic cytokine that provides continuous signals to resting naive and memory T-cells, and which accumulates during conditions of lymphopenia, leading to an increase in both T-cell proliferation and T-cell repertoire diversity. In comparison to IL-2, IL-7 is selective for expanding CD8⁺ T-cells over CD4⁺FOXP3⁺ regulatory T-cells. Recombinant IL-7 has been shown to augment antigen-specific T-cell responses following vaccination, and adoptive cell therapy in mice. IL-7 also can play a role in promoting T-cell recovery following chemotherapy of hematopoietic stem cell transplantation. Early phase clinical trials on patients with advanced malignancy have shown that recombinant IL-7 is well-tolerated and has limited toxicity at biologically active doses (*i.e.,* in which the numbers of circulating CD4⁺ and CD8⁺ T-cells is increased by 3-4 fold) (see, *e.g.,* Lee, S. and Margolin, K. (2011) Cancers 3:3856-3893). IL-7 has been shown to possess antitumor effects in tumors such as gliomas, melanomas, lymphomas, leukemia, prostate cancer, and glioblastoma, and the *in vivo* administration of IL-7 in murine models resulted in decreased cancer cell growth. IL-7 also has been shown to enhance the antitumor effects of IFN-γ in rat glioma tumors, and to induce the production of IL-1α, IL-1β and TNF-α by monocytes, which results in the inhibition of melanoma growth. Additionally, administration of recombinant IL-7 following the treatment of pediatric sarcomas resulted in the promotion of immune recovery (see, *e.g.,* Lin et al. (2017) Anticancer Research 37:963-968).

### IL-12 (IL-12p70 (IL-12p40 + IL-12p35))

Bioactive IL-12 (IL-12p70), which promotes cell-mediated immunity, is a heterodimer, composed of p35 and p40 subunits, whereas IL-12p40 monomers and homodimers act as IL-12 antagonists. IL-12, which is secreted by antigen-presenting cells, promotes the secretion of IFN-γ from NK and T-cells, inhibits tumor angiogenesis, results in the activation and proliferation of NK cells, CD8⁺ T-cells and CD4⁺ T-cells, enhances the differentiation of naive CD4⁺ T-cells into Th1 cells, and promotes antibody-dependent cell-mediated cytotoxicity (ADCC) against tumor cells. IL-12 has been shown to exhibit anti-tumor effects in murine models of melanoma, colon carcinoma, mammary carcinoma, and sarcoma (see, *e.g.,* Kalinski et al. (2001) Blood 97:3466-3469; Sheikhi et al. (2016) Iran J. Immunol. 13(3):148-166; and Lee, S. and Margolin, K. (2011) Cancers 3:3856-3893).

### IL-15 and IL-15:IL-15Rα

IL-15 is structurally similar to IL-2, and while both IL-2 and IL-15 provide early stimulation for the proliferation and activation of T-cells, IL-15 blocks IL-2 induced apoptosis, which is a process that leads to the elimination of stimulated T-cells and induction of T-cell tolerance, limiting memory T-cell responses and potentially limiting the therapeutic efficacy of IL-2 alone. IL-15 also supports the persistence of memory CD8⁺ T-cells for maintaining long-term anti-tumor immunity, and has demonstrated significant anti-tumor activity in pre-clinical murine models via the direct activation of CD8⁺ effector T-cells in an antigen-independent manner. In addition to CD8⁺ T-cells, IL-15 is responsible for the development, proliferation and activation of effector natural killer (NK) cells (see, *e.g.,* Lee, S. and Margolin, K. (2011) Cancers 3:3856-3893; and Han et al. (2011) Cytokine 56(3):804-810*).*

IL-15 and IL-15 receptor alpha (IL-15Rα) are coordinately expressed by antigen-presenting cells, such as monocytes and dendritic cells, and IL-15 is presented *in trans* by IL-15Rα to the IL-15Rβγc receptor complex expressed on the surfaces of CD8⁺ T-cells and NK cells. Soluble 1L-15:IL15-Rα complexes have been shown to modulate immune responses via the IL-15Rβγc complex, and the biological activity of IL-15 has been shown to be increased 50-fold by administering it in a preformed complex of IL-15 and soluble IL-15Rα, which has an increased half-life compared to IL-15 alone. This significant increase in the therapeutic efficacy of IL-15 by pre-association with E-15Rα has been demonstrated in murine tumor models (see, *e.g.,* Han et al. (2011) Cytokine 56(3):804-810*).*

### IL-18

IL-18 induces the secretion of IFN-γ by NK and CD8⁺ T-cells, enhancing their toxicity. IL-18 also activates macrophages and stimulates the development of Th1 helper CD4⁺ T-cells. IL-18 has shown promising anti-tumor activity in several preclinical mouse models. For example, administration of recombinant IL-18 (rIL-18) resulted in the regression of melanoma or sarcoma in syngeneic mice through the activation of CD4⁺ T-cells and/or NK cell-mediated responses. Other studies showed that IL-18 anti-tumor effects were mediated by IFN-γ, and involved antiangiogenic mechanisms. The combination of IL-18 with other cytokines, such as IL-12, or with co-stimulatory molecules, such as CD80, enhances the IL-18-mediated anti-tumor effects. Phase I clinical trials in patients with advanced solid tumors and lymphomas showed that IL-18 administration was safe, and that it resulted in immune modulatory activity and in the increase of serum IFN-γ and GM-CSF levels in patients, and in modest clinical responses. Clinical trials showed that IL-18 can be combined with other anti-cancer therapeutic agents, such as monoclonal antibodies, cytotoxic drugs, or vaccines (see, *e.g.,* Fabbi et al. (2015) J. Leukoc. Biol. 97:665-675; and Lee, S. and Margolin, K. (2011) Cancers 3:3856-3893).

It was found that an attenuated strain of *Salmonella typhimurium,* engineered to express IL-18, inhibited the growth of subcutaneous (S.C.) tumors or pulmonary metastases in syngeneic mice without any toxic effects following systemic administration. Treatment with this engineered bacterium induced the accumulation of T-cells, NK cells and granulocytes in tumors, and resulted in the intratumoral production of cytokines (see, *e.g.,* Fabbi et al. (2015) J. Leukoc. Biol. 97:665-675).

### Chemokines

Chemokines are a family of small cytokines that mediate leukocyte migration to areas of injury or inflammation, and are involved in mediating immune and inflammatory responses. Chemokines are classified into four subfamilies, based on the position of cysteine residues in their sequences, namely XC-, CC-, CXC-, and CX3C-chemokine ligands, or XCL, CCL, CXCL, and CX3CL. The chemokine ligands bind to their cognate receptors and regulate the circulation, homing and retention of immune cells, with each chemokine ligand-receptor pair selectively regulating a certain type of immune cell. Different chemokines attract different leukocyte populations, and form a concentration gradient *in vivo,* with attracted immune cells moving through the gradient towards the higher concentration of chemokine (see, *e.g.,* Argyle D. and Kitamura, T. (2018) Front. Immunol. 9:2629; and Dubinett et al. (2010) Cancer J. 16(4):325-335). Chemokines can improve the anti-tumor immune response by increasing the infiltration of immune cells into the tumor, and facilitating the movement of antigen-presenting cells (APCs) to tumor-draining lymph nodes, which primes naive T-cells and B cells (see, *e.g.,* Lechner et al. (2011) Immunotherapy 3(11):1317-1340). The immunostimulatory bacteria herein can be engineered to encode chemokines, including, but not limited to, CCL3, CCL4, CCL5, CXCL9, CXCL10, and CXCL11.

### CCL3, CCL4, CCL5

CCL3, CCL4, and CCL5 share a high degree of homology, and bind to CCR5 (CCL3, CCL4 and CCL5) and CCR1 (CCL3 and CCL5) on several cell types, including immature DCs and T-cells, in both humans and mice. Therapeutic T-cells have been shown to induce chemotaxis of innate immune cells to tumor sites, via the tumor-specific secretion of CCL3, CCL4, and CCL5 (see, *e.g.,* Dubinett et al. (2010) Cancer J. 16(4):325-335).

The induction of the T helper cell type 1 (Th1) response releases CCL3. *In vivo* and *in vitro* studies of mice have indicated that CCL3 is chemotactic for both neutrophils and monocytes; specifically, CCL3 can mediate myeloid precursor cell (MPC) mobilization from the bone marrow, and has MPC regulatory and stimulatory effects. Human ovarian carcinoma cells transfected with CCL3 showed enhanced T-cell infiltration and macrophages within the tumor, leading to an improved anti-tumor response, and indicated that CCL3-mediated chemotaxis of neutrophils suppressed tumor growth. DCs transfected with the tumor antigen human melanoma-associated gene (MAGE)-1 that were recruited by CCL3 exhibited superior anti-tumor effects, including increased lymphocyte proliferation, cytolytic capacity, and survival, and decreased tumor growth, in a mouse model of melanoma. A combinatorial use of CCL3 with an antigen-specific platform for MAGE-1 has also been used in the treatment of gastric cancer. CCL3 production by CT26, a highly immunogenic murine colon tumor, slowed *in vivo* tumor growth; this process was driven by the CCL3-dependent accumulation of natural killer (NK) cells, and thus, IFNγ, resulting in the production of CXCL9 and CXLC10 (see, *e.g.,* Allen et al. (2017) Oncoimmunology 7(3):e1393598; and Schaller et al. (2017) Expert Rev. Clin. Immunol. 13(11):1049-1060).

CCL3 has been used as an adjuvant for the treatment of cancer. Administration of a CCL3 active variant, ECI301, after radiofrequency ablation in mouse hepatocellular carcinoma increased tumor-specific responses, and this mechanism was further shown to be dependent on the expression of CCR1. CCL3 has also shown success as an adjuvant in systemic cancers, whereby mice vaccinated with CCL3 and IL-2 or granulocyte-macrophage colony-stimulating factor (GM-CSF), in a model of leukemia/lymphoma, exhibited increased survival (see, *e.g.,* Schaller et al. (2017) Expert Rev. Clin. Immunol. 13(11):1049-1060).

CCL3 and CCL4 play a role in directing CD8⁺ T-cell infiltration into primary tumor sites in melanoma and colon cancers. Tumor production of CCL4 leads to the accumulation of CD103⁺ DCs; suppression of CCL4 through a WNT/β-catenin-dependent pathway prevented CD103⁺ DC infiltration of melanoma tumors (see, *e.g.,* Spranger et al. (2015) Nature 523(7559):231-235). CCL3 was also shown to enhance CD4⁺ and CD8⁺ T-cell infiltration to the primary tumor site in a mouse model of colon cancer (see, *e.g.,* Allen et al. (2017) Oncoimmunology 7(3):e1393598).

The binding of CCL3 or CCL5 to their receptors (CCR1 and CCR5), moves immature DCs, monocytes, and memory and T effector cells from the circulation into sites of inflammation or infection. For example, CCL5 expression in colorectal tumors contributes to T lymphocyte chemoattraction and survival. CCL3 and CCL5 have been used alone or in combination therapy to induce tumor regression and immunity in several preclinical models. For example, studies have shown that the subcutaneous injection of Chinese hamster ovary cells genetically modified to express CCL3, resulted in tumor inhibition and neutrophilic infiltration. In another study, a recombinant oncolytic adenovirus expressing *CCL5* (Ad-RANTES-E1A) resulted in primary tumor regression, and blocked metastasis in a mammary carcinoma murine model (see, *e.g.,* Lechner et al. (2011) Immunotherapy 3(11):1317-1340).

In a translational study of colorectal cancer, CCL5 induced an "antiviral response pattern" in macrophages. As a result of CXCR3-mediated migration of lymphocytes at the invasive margin of liver metastases in colorectal cancer, CCL5 is produced. Blockade of CCR5, the CCL5 receptor, results in tumor death, driven by macrophages producing IFN and reactive oxygen species. While macrophages are present in the tumor microenvironment, CCR5 inhibition induces a phenotypic shift from an M2 to an M1 phenotype. CCR5 blockade also leads to clinical responses in colorectal cancer patients (see, *e.g.,* Halama et al. (2016) Cancer Cell 29(4):587-601).

CCL3, CCL4, and CCL5 can be used for treating conditions, including lymphatic tumors, bladder cancer, colorectal cancer, lung cancer, melanoma, pancreatic cancer, ovarian cancer, cervical cancer, or liver cancer (see, *e.g.,* U.S. Patent Publication No. US 2015/0232880; and International Application Publication Nos. WO 2015/059303, WO 2017/043815, WO 2017/156349 and WO 2018/191654).

### CXCL9, CXCL10, CXCL11

CXCL9 (MIG), CXCL10 (IP10), and CXCL11 (ITAC) are induced by the production of IFN-γ. These chemokines bind CXCR3, preferentially expressed on activated T-cells, and function both angiostatically, and in the recruitment and activation of leukocytes. Prognosis in colorectal cancer is strongly correlated to tumor-infiltrating T-cells, particularly Th1 and CD8⁺ effector T-cells; high intratumoral expression of CXCL9, CXCL10 and CXCL11 is indicative of good prognosis. For example, in a sample of 163 patients with colon cancer, those with high levels of CXCL9 or CXCL11 showed increased post-operative survival, and patients with high CXC expression had significantly higher numbers of CD3⁺ T-cells, CD4⁺ T-helper cells, and CD8⁺ cytotoxic T-cells. In liver metastases of colorectal cancer patients, CXCL9 and CXCL10 levels were increased at the invasive margin, and correlated with effector T-cell density. The stimulation of lymphocyte migration via the action of CXCL9 and CXCL10 on CXCR3 leads to the production of CCL5 at the invasive margin (see, *e.g.,* Halama et al. (2016) Cancer Cell 29(4):587-601; and Kistner et al. (2017) Oncotarget 8(52):89998-90012).

*In vivo,* CXCL9 functions as a chemoattractant for tumor-infiltrating lymphocytes (TILs), activated peripheral blood lymphocytes, natural killer (NK) cells, and Th1 lymphocytes. CXCL9 also is critical for T-cell-mediated suppression of cutaneous tumors. For example, when combined with systemic IL-2, CXCL9 has been shown to inhibit tumor growth via the increased intratumoral infiltration of CXCR3⁺ mononuclear cells. In a murine model of colon carcinoma, a combination of the huKS1/4-IL-2 fusion protein with CXCL9 gene therapy achieved a superior anti-tumor effect and prolonged lifespan through the chemoattraction and activation of CD8⁺ and CD4⁺ T lymphocytes (see, *e.g.,* Dubinett et al. (2010) Cancer J. 16(4):325-335; and Ruehlmann et al. (2001) Cancer Res. 61(23):8498-8503).

CXCL10, produced by activated monocytes, fibroblasts, endothelial cells, and keratinocytes, is chemotactic for activated T-cells, and can act as an inhibitor of angiogenesis *in vivo.* Expression of CXCL10 in colorectal tumors has been shown to contribute to cytotoxic T lymphocyte chemoattraction and longer survival. The administration of immunostimulatory cytokines, such as IL-12, has been shown to enhance the anti-tumor effects generated by CXCL10. A dendritic cell (DC) vaccine primed with a tumor cell lysate, and transfected with CXCL10, had increased immunological protection and effectiveness in mice; the animals showed a resistance to a tumor challenge, a slowing of tumor growth, and longer survival time. *In vivo* and *in vitro* studies in mice using the CXCL10-mucin-GPI fusion protein resulted in tumors with higher levels of recruited NK cells compared to tumors not treated with the fusion protein. Interferons (which can be produced by plasmacytoid dendritic cells; these cells are associated with primary melanoma lesions and can be recruited to a tumor site by CCL20) can act on tumor DC subsets, for example, CD103⁺ DCs, which have been shown to produce CXCL9/10 in a mouse melanoma model, and have been associated with CXCL9/10 in human disease. CXCL10 also has shown higher expression in human metastatic melanoma samples relative to primary melanoma samples. Therapeutically, adjuvant IFN-α melanoma therapy upregulates CXCL10 production, whereas the chemotherapy agent cisplatin induces CXCL9 and CXCL10 (see, *e.g.,* Dubinett et al. (2010) Cancer J. 16(4):325-335; Kuo et al. (2018) Front. Med. (Lausanne) 5:271; Li et al. (2007) Scand. J. Immunol. 65(1):8-13; and Muenchmeier et al. (2013) PLoS One 8(8):e72749).

CXCL10/11 and CXCR3 expression has been established in human keratinocytes derived from basal cell carcinomas (BCCs). CXCL11 also is capable of promoting immunosuppressive indoleamine 2,3-dioxygenase (IDO) expression in human basal cell carcinoma, as well as enhancing keratinocyte proliferation, which could reduce the anti-tumor activity of any infiltrating CXCR3⁺ effector T-cells (see, *e.g.,* Kuo et al. (2018) Front. Med. (Lausanne) 5:271*).*

CXCL9, CXCL10 and CXCL11 can be encoded in oncolytic viruses for treating cancer (see, *e.g.,* U.S. Patent Publication No. 2015/0232880; and International Application Publication No. WO 2015/059303). Pseudotyped oncolytic viruses or a genetically engineered bacterium encoding the gene for CXCL10 also can be used to treat cancer (see, e.g., International Application Publication Nos. WO 2018/006005 and WO 2018/129404).

### b. Co-Stimulatory Molecules

Co-stimulatory molecules enhance the immune response against tumor cells, and co-stimulatory pathways are inhibited by tumor cells to promote tumorigenesis. The immunostimulatory bacteria herein can be engineered to express co-stimulatory molecules, such as, for example, CD40, CD40L, 4-1BB, 4-1BBL, 4-1BBL with a deletion of the cytoplasmic domain (4-1BBLΔcyt), 4-1BBL with a truncated cytoplasmic domain, OX40 (CD134), OX40L (CD252), other members of the TNFR superfamily (*e.g*., CD27, CD27 ligand, GITR, CD30, Fas receptor, TRAIL-R, TNF-R, HVEM, and RANK), B7, CD80, CD86, ICOS, ICOS ligand (B7RP1), and CD28. Additionally, the immunostimulatory bacteria can encode and express truncated co-stimulatory molecules (*e.g*., 4-1BBL, CD80, CD86, CD27L, B7RP1, OX40L), with a full or partial (complete, or truncated, or modified to ensure proper orientation when expressed in a cell) cytoplasmic domain deletion, for expression on an antigen presenting cell (APC). It is shown herein that the gene product with a truncated cytoplasmic domain, including a full deletion, provides constitutive immunostimulatory signaling to a T-cell through co-stimulatory receptor engagement, and is unable to counter-regulatory signal to the APC due to a truncated or deleted (or otherwise modified as described herein) cytoplasmic domain. The truncation is sufficient to provide the signaling, and to be unable to counter-regulatory signal to the APC. The complete or partial deletion of the cytoplasmic domain of a co-stimulatory molecule, as described herein, potentiates the activation of the co-stimulatory molecule, without the immunosuppressive reverse signaling. The partial deletion (or truncation) of the cytoplasmic domain is a sufficient deletion to achieve these effects, without affecting the expression of the co-stimulatory molecule, or the orientation of the expressed co-stimulatory molecule.

The co-stimulatory molecules also can be modified to eliminate or reduce the immunosuppressive intracellular/reverse signaling by modifications to the amino acids in the cytoplasmic domain, including insertions, deletions, and/or replacements. In particular, the co-stimulatory molecules are modified by modification, such as by replacement, of cytoplasmic domain phosphorylation sites. For example, replacing one or more Ser residues at an appropriate locus or loci, such as, for human 4-1BBL, with reference to SEQ ID NO:389, Ser5 and Ser8, with a residue that reduces or eliminates reverse signaling.

The immunostimulatory bacteria herein also can be engineered to express agonistic antibodies against co-stimulatory molecules (*e.g*., 4-1BB) to enhance the anti-tumor immune response.

### TNF Receptor Superfamily

The TNF superfamily of ligands (TNFSF) and their receptors (TNFRSF) are involved in the proliferation, differentiation, activation and survival of tumor and immune effector cells. Members of this family include CD30, Fas-L, TRAIL-R, and TNF-R, which induce apoptosis, and CD27, OX40L, CD40L, GITR-L, and 4-1BBL, which regulate B and T-cell immune responses. Other members include herpesvirus entry mediator (HVEM). The expression of TNFSF and TNFRSF by the immunostimulatory bacteria herein can enhance the anti-tumor immune response. It has been shown, for example, that the expression of 4-1BBL in murine tumors enhances immunogenicity, and that intratumoral injection of dendritic cells (DCs) with increased expression of OX40L can result in tumor rejection in murine models. Studies have also shown that injection of an adenovirus expressing recombinant GITR into B16 melanoma cells promotes T-cell infiltration and reduces tumor volume. Stimulatory antibodies against molecules such as 4-1BB, OX40 and GITR also can be encoded by the immunostimulatory bacteria to stimulate the immune system. For example, agonistic anti-4-1BB monoclonal antibodies have been shown to enhance anti-tumor CTL responses, and agonistic anti-OX40 antibodies have been shown to increase anti-tumor activity in transplantable tumor models. Additionally, agonistic anti-GITR antibodies have been shown to enhance anti-tumor responses and immunity (see, *e.g.,* Lechner et al. (2011) Immunotherapy 3(11):1317-1340; and Peggs et al. (2009) Clinical and Experimental Immunology 157:9-19*).*

### CD40 and CD40L

CD40, which is a member of the TNF receptor superfamily, is expressed by APCs and B cells, while its ligand, CD40L (CD154), is expressed by activated T-cells. Interaction between CD40 and CD40L stimulates B cells to produce cytokines, resulting in T-cell activation and tumor cell death. Studies have shown that anti-tumor immune responses are impaired with reduced expression of CD40L on T-cells, or CD40 on dendritic cells. CD40 is expressed on the surface of several B-cell tumors, such as follicular lymphoma, Burkitt lymphoma, lymphoblastic leukemia, and chronic lymphocytic leukemia, and its interaction with CD40L has been shown to increase the expression of B7-1/CD80, B7-2/CD86, and human leukocyte antigen (HLA) class II molecules in the CD40⁺ tumor cells, as well as enhance their antigen-presenting abilities. Transgenic expression of CD40L in a murine model of multiple myeloma resulted in the induction of CD4⁺ and CD8⁺ T-cells, local and systemic anti-tumor immune responses, and reduced tumor growth. Anti-CD40 agonistic antibodies also induced anti-tumor T-cell responses (see, *e.g.,* Marin-Acevedo et al. (2018) Journal of Hematology & Oncology 11:39; Dotti et al. (2002) Blood 100(1):200-207; and Murugaiyan et al. (2007) J. Immunol. 178:2047-2055).

### 4-1BB and 4-1BBL

4-1BB (CD137) is an inducible co-stimulatory receptor that is expressed primarily by T-cells and NK cells; it binds its ligand 4-1BBL that is expressed on APCs, including DCs, B-cells, and monocytes, to trigger immune cell proliferation and activation. 4-1BB results in longer and more widespread responses of activated T-cells. Anti-4-1BB agonists and 4-1BBL fusion proteins have been shown to increase immune-mediated anti-tumor activity, for example, against sarcoma and mastocytoma tumors, mediated by CD4⁺ Th1 and tumor-specific CTL activity (see, *e.g.,* Lechner et al. (2011) Immunotherapy 3(11):1317-1340; and Marin-Acevedo et al. (2018) Journal of Hematology & Oncology 11:39). 4-1BBL is negatively regulated by its cytoplasmic signaling domain. In the late-phase of 4-1BBL ligation on macrophages to T-cells, reverse signaling of the 4-1BBL cytoplasmic domain induces surface translocation of 4-1BBL to bind to form a signaling complex with TLR4. This induces high levels of TNF-α, comparable to LPS activation of TLR4, that leads to immunosuppression of the adaptive immune response (see, *e.g.,* Ma et al. (2013) Sci. Signaling 295(6):1-11).

4-1BBL, a member of the TNF superfamily, is expressed in B-cells, dendritic cells, activated T-cells and macrophages. 4-1BBL binds to its receptor, 4-1BB, and provides a co-stimulatory signal for T-cell activation and expansion. The human 4-1BBL gene encodes a 254 amino acid type II transmembrane protein containing a 28 amino acid cytoplasmic domain, a 21 amino acid transmembrane protein domain, and a 205 amino acid extracellular domain (see, SEQ ID NO:389). Deletion of all or of a portion of the cytoplasmic domain of 4-1BBL (corresponding to amino acid residues 1-28 of SEQ ID NO:342 or 389), as described herein, potentiates the activation of 4-1BBL without the immunosuppressive reverse signaling. The portion that is deleted is sufficient to potentiate the activation of 4-1BBL, but without the immunosuppressive reverse signaling. As described below, the truncated cytoplasmic domain can include amino acids or replacements to ensure proper orientation of the expressed protein in the cell membrane (similar modifications can be effected in others of the membrane-spanning proteins in which the cytoplasmic domain is truncated or deleted). Provided herein are nucleic acid molecules encoding a 4-1BBL variant that lacks the cytoplasmic domain, or that has a truncated cytoplasmic domain, to eliminate the immunosuppressive reverse signaling. An example of such nucleic acid and encoded protein is described in the Examples (see, *e.g.,* SEQ ID NOs: 391 and 395). The receptors also can be cytoplasmically truncated or deleted.

### OX40 and OX40L

OX40 (CD134) is a member of the TNF receptor superfamily that is expressed on activated effector T-cells, while its ligand, OX40L is expressed on APCs, including DCs, B cells and macrophages, following activation by TLR agonists and CD40-CD40L signaling. OX40-OX40L signaling results in the activation, potentiation, proliferation and survival of T-cells, as well as the modulation of NK cell function and inhibition of the suppressive activity of Tregs. Signaling through OX40 also results in the secretion of cytokines (IL-2, IL-4, IL-5, and IFN-γ), boosting Th1 and Th2 cell responses. The recognition of tumor antigens by tumor-infiltrating lymphocytes (TILs) results in increased expression of OX40 by the TILs, which has been correlated with improved prognosis. Studies have demonstrated that treatment with anti-OX40 agonist antibodies or Fc-OX40L fusion proteins results in enhanced tumor-specific CD4⁺ T-cell responses and increased survival in murine models of melanoma, sarcoma, colon carcinoma, and breast cancer, while Fc-OX40L incorporated into tumor cell vaccines protected mice from subsequent challenge with breast carcinoma cells (see, *e.g.,* Lechner et al. (2011) Immunotherapy 3(11):1317-1340; and Marin-Acevedo et al. (2018) Journal of Hematology & Oncology 11:39).

### B7-CD28 Family

CD28 is a co-stimulatory molecule expressed on the surface of T-cells that acts as a receptor for B7-1 (CD80) and B7-2 (CD86), which are co-stimulatory molecules expressed on antigen-presenting cells. CD28-B7 signaling is required for T-cell activation and survival, and for the prevention of T-cell anergy, and results in the production of interleukins, such as IL-6.

Optimal T-cell priming requires two signals: (1) T-cell receptor (TCR) recognition of MHC-presented antigens, and (2) co-stimulatory signals resulting from the ligation of T-cell CD28 with B7-1 (CD80) or B7-2 (CD86) expressed on APCs. Following T-cell activation, CTLA-4 receptors are induced, which then outcompete CD28 for binding to B7-1 and B7-2 ligands. Antigen presentation by tumor cells is poor due to their lack of expression of co-stimulatory molecules, such as B7-1/CD80 and B7-2/CD86, resulting in a failure to activate the T-cell receptor complex. As a result, upregulation of these molecules on the surfaces of tumor cells can enhance their immunogenicity. Immunotherapy of solid tumors and hematologic malignancies has been successfully induced by B7, for example, via tumor cell expression of B7, or soluble B7-immunoglobulin fusion proteins. The viral-mediated tumor expression of B7, in combination with other co-stimulatory ligands, such as ICAM-3 and LFA-3, has been successful in preclinical and clinical trials for the treatment of chronic lymphocytic leukemia and metastatic melanoma. Additionally, soluble B7 fusion proteins have demonstrated promising results in the immunotherapy of solid tumors as single agent immunotherapies (see, *e.g.,* Lechner et al. (2011) Immunotherapy 3(11):1317-1340; and Dotti et al. (2002) Blood 100(1):200-207).

### 2. Molecules that Activate Prodrugs

The plasmids in the immunostimulatory bacteria provided herein can include nucleic acids that encode molecules, such as enzymes, that activate, such as by cleavage of a portion of, therapeutic products, such as prodrugs, including chemotherapeutic prodrugs, particularly toxins, that are activated by enzymatic cleavage. As a result, the inactive prodrug can be administered systemically, and is inactive. The plasmid-encoded activating molecule, such as an enzyme, is expressed in the tumor microenvironment after delivery of the immunostimulatory bacteria provided herein, so that the inactive prodrug is activated in the tumor microenvironment, where it exerts its anti-tumor effect. There are many examples of such prodrugs, including certain nucleosides, and toxin conjugates. Many such prodrugs and enzymes are known (see, *e.g.,* Malekshah et al., (2016) Curr. Pharmacol. Rep. 2:299-308). These include prodrugs of 5-fluorouricil, axazaphosorines, platinum drugs, and enzymes such as deaminases, nitroreductases, phosphorylases, cytochrome P450 enzymes, and many others.

### 3. Constitutively Active Proteins that Stimulate the Immune Response and/or Type I IFN, Non-Human STING Proteins, Chimeras, and Modified Forms

Type I interferons (IFNs; also referred to as interferon type 1), include IFN-α and IFN-β, and are pleiotropic cytokines with antiviral, anti-tumor, and immunoregulatory activities. IFN-β is produced by most cell types; IFN-α primarily is produced by hematopoietic cells, particularly plasmacytoid dendritic cells. Type I IFNs are produced following the sensing of pathogen-associated molecular patterns (PAMPs) by pattern recognition receptors (PRRs). They are involved in the innate immune response against pathogens, mainly viral, and are potent immunomodulators that promote antigen presentation, mediate dendritic cell (DC) maturation, activate cytotoxic T lymphocytes (CTLs), natural killer (NK) cells and macrophages, and activate the adaptive immune system by promoting the development of high-affinity antigen-specific T-cell and B-cell responses and immunological memory.

Type I IFNs exhibit anti-proliferative and pro-apoptotic effects on tumors and have anti-angiogenic effects on tumor neovasculature. They induce the expression of MHC class I molecules on tumor cell surfaces, increase the immunogenicity of tumor cells, and activate cytotoxicity against them. Type I IFN has been used as a therapeutic for the treatment of cancers and viral infections. For example, IFN-α (sold under the trademark Intron^{®}/Roferon^{®}-A) is approved for the treatment of hairy cell leukemia, malignant melanoma, AIDS-related Kaposi's sarcoma, and follicular non-Hodgkin's lymphoma; it also is used in the treatment of chronic myelogenous leukemia (CML), renal cell carcinoma, neuroendocrine tumors, multiple myeloma, non-follicular non-Hodgkin's lymphoma, desmoid tumors, and cutaneous T-cell lymphoma, although use is limited due to systemic immunotoxicity (see, *e.g.,* Ivashkiv and Donlin (2014) Nat. Rev. Immunol. 14(1):36-49; Kalliolias and Ivashkiv (2010) Arthritis Research & Therapy 12(Suppl 1):S1; and Lee, S. and Margolin, K. (2011) Cancers 3:3856-3893).

Expression of type I interferons in tumors and the tumor microenvironment is among the immune responses that the immunostimulatory bacteria herein are designed to evoke. Inducing or evoking type I interferon provides anti-tumor immunity for the treatment of cancer.

### a. Constitutive STING Expression and Gain-of-Function Mutations

The induction of type I IFNs, proinflammatory cytokines and chemokines is necessary for mounting an immune response that prevents or inhibits infection by viral pathogens. This response also can be effective as an anti-tumor agent. The immunostimulatory bacteria provided herein encode proteins that constitutively induce type I IFNs. Among these proteins are those that occur in individuals with various diseases or disorders that involve the over-production of immune response modulators. For example, over-production or excessive production, or defective negative regulation of type I IFNs and pro-inflammatory cytokines, can lead to undesirable effects, such as inflammatory and autoimmune diseases. Disorders involving the overproduction, generally chronic, of type I IFNs, are referred to as interferonopathies (see, *e.g.,* Lu and MacDougall (2017) Front. Genet. 8:118; and Konno et al. (2018) Cell Reports 23:1112-1123). Disorders and clinical phenotypes associated with type I interferonopathies include Aicardi-Goutiéres syndrome (AGS), STING-associated vasculopathy with onset in infancy (SAVI), Singleton-Merten syndrome (SMS), atypical SMS, familial chilblain lupus (FCL), systemic lupus erythematosus (SLE), bilateral striatal necrosis (BSN), cerebrovascular disease (CVD), dyschromatosis symmetrica hereditaria (DSH), spastic paraparesis (SP), X-linked reticulate pigmentary disorder (XLPDR), proteasome-associated auto-inflammatory syndrome (PRAAS), intracranial calcification (ICC), Mendelian susceptibility to mycobacterial disease (MSMD), and spondyloenchondrodysplasia (SPENCD) (see, *e.g.,* Rodero et al. (2016) J. Exp. Med. 213(12):2527-2538). These phenotypes are associated with particular genotypes, involving mutations in genes that lead to constitutive activities of products involved in the induction of type I IFNs.

The sustained activation of interferon signaling can be due to: 1) loss-of-function mutations leading to increased cytosolic DNA (*e.g.,* mutations in *TREX1* and *SAMHDI*)*,* or increased cytosolic RNA/DNA hybrids (*e.g.,* mutations in *RNASEH2A, RNASEH2B, RNASEH2C,* and *POLA1*); 2) loss-of-function mutations resulting in a defect in RNA editing and abnormal sensing of self-nucleic acid RNA species in the cytosol (*e.g.,* mutations in *ADAR1*); 3) gain-of-function mutations leading to constitutive activation of cytosolic IFN signaling pathways/increased sensitivity to cytosolic nucleic acid ligands (*e.g*., mutations in RIG-I, MDA5 and STING); 4) loss-of-function mutations leading to aberrant RNA signaling via MAVS caused by a disturbance of the unfolded protein response (*e.g.,* mutations in *SKIV2L*); 5) loss-of-function mutations in molecules responsible for limiting IFN receptor (IFNAR1/2) signaling, leading to uncontrolled IFN-stimulated gene (ISG) production (*e.g*., mutations in *USP18* and *ISG15*); 6) proteasomal dysfunction, leading to increased IFN signaling through an unknown mechanism (*e.g.,* mutations in *PSMA3, PSMB4* and *PSMB8*); and 7) loss-of-function mutations in TRAP/*ACPP5* and C1q, where the mechanisms leading to type I IFN signaling remain unclear (see, *e.g.,* Rodero et al. (2016) J. Exp. Med. 213(12):2527-2538).

Of interest herein are mutations that lead to gain-of-function (GOF). There are known mutations in STING, MDA5 and RIG-I, that are associated with constitutive activation of the encoded proteins, and/or enhanced sensitivity or increased affinity or binding to endogenous ligands. GOF mutations in STING, for example, are linked to SAVI and FCL; GOF mutations in MDA5 are linked to AGS and SMS; and GOF mutations in RIG-I are linked to atypical SMS.

### TMEM173 STING Alleles

Stimulator of interferon genes (STING) is encoded by the transmembrane protein 173 (*TMEM173*) gene, which is a ~7 kb-long gene. The human *TMEM173* gene is characterized by significant heterogeneity and population stratification of alleles. The most common human *TMEM173* allele is referred to as *R232* (referencing the amino acid present at residue 232; see, *e.g.,* SEQ ID NOs:305-309, setting forth the sequences of various human *TMEM173* alleles). More than half the American population is not R232/R232. The second most common allele is *R71H-G230A-R293Q (HAQ).* Other common alleles include *AQ (G230A-R293Q), Q (Q293)* and *R232H* (named REF after the reference STING allele first identified and catalogued in the database by Glen Barber).

*R232*/*R232* is the most common genotype in Europeans, while *HAQ*/*R232* is the most common genotype in East Asians. Africans have no *HAQ*/*HAQ* genotypes, but have the *Q* allele, and ~4% of Africans are *AQ*/*AQ,* which is absent in other ethnic populations (see, *e.g.,* Patel and Jin (2018) Genes & Immunity, doi:10.1038/s41435-018-0029-9). The REF, *AQ* and *Q* alleles are highly refractory to bacterially-derived CDNs, such as 3'3' c-di-GMP (see, *e.g.,* Corrales et al. (2015) Cell Reports 11:1018-1030).

### STING Gain-of-Function Mutations

Several activating or gain-of-function (GOF) mutations in *TMEM173,* the gene for STING, inherited and *de novo,* have been linked to the rare auto-inflammatory disease SAVI (STING-associated vasculopathy with onset in infancy). SAVI is an autosomal dominant disease and is characterized by systemic inflammation, interstitial lung disease, cutaneous vasculitis, and recurrent bacterial infection. SAVI with *de novo TMEM173* mutations typically is characterized by an early-onset (< 8 weeks) and severe phenotype, while familial mutations result in late-onset (teens to adults) and milder clinical symptoms. Inherited *TMEM173* activating mutations include G166E and V155M, whereas *de novo* mutations include N154S, V155M, V147M, V147L, C206Y, R284G, R281Q and S102P/F279L (see, *e.g.,* Patel and Jin (2019) Genes & Immunity 20:82-89). Other activating *TMEM173* mutations that have been identified include R284M, R284K, R284T, E316Q, and R375A (see, *e.g.,* U.S. Patent Publication No. 2018/0311343). Another gain-of-function mutation in *TMEM173* is R284S, which results in a highly constitutively active STING, and was found to trigger innate immune signaling in the absence of activating CDNs, leading to chronic production of pro-inflammatory cytokines (see, *e.g.,* Konno et al. (2018) Cell Reports 23:1112-1123).

*TMEM173* mutations, such as N154S, V155M and V147L, and/or any of the mutations listed in the table below, singly or in any combination with these and any other such mutations, such as N154S/R284G, result in a gain-of-function STING that is constitutively active and does not require, or is hypersensitive to, ligand stimulation, leading to chronic activation of the STING-interferon pathway. This has been demonstrated (see, *e.g.,* Liu et al. (2014) N. Engl. J. Med. 371:507-518). Constructs of mutated *TMEM173* (with each of the replacements V147L, N154S, V155M, and the loss-of-function mutant V155R), and non-mutated *TMEM173,* were transfected into STING-negative HEK293T cells, and stimulated with the STING ligand, cGAMP. Cells transfected with the N154S, V155M and V147L mutants exhibited highly elevated *IFNB1* (the gene encoding IFN-β) reporter activity, which was not significantly boosted by stimulation with the STING ligand cGAMP. Cells that were transfected with the loss-of-function mutant (V155R), non-mutated *TMEM173,* or control plasmid, had no significant baseline activation. Stimulation with cGAMP resulted in a response in a dose-dependent manner in cells with non-mutated *TMEM173,* and resulted in a minimal response only at the highest cGAMP concentration, in cells expressing the loss-of-function mutant (see, *e.g.,* Liu et al. (2014) N. Engl. J. Med. 371:507-518). These results show that the activating *TMEM173* mutations result in constitutive activation of STING, even in the absence of stimulation by cGAMP.

G207E is another gain-of-function STING mutation that causes alopecia, photosensitivity, thyroid dysfunction, and SAVI-features. The G207E mutation causes constitutive activation of inflammation-related pathways in HEK cells, as well as aberrant interferon signature and inflammasome activation in patient peripheral blood mononuclear cells (PBMCs). Using STING variants with the R232 or H232 allele and the GOF mutation G207E, it was shown that after stimulation with CDN, the R232 + G207E variant resulted in slight increases of activity in the IFN-β and STAT1/2 pathways, while with the H232 + G207E variant, IFN-β levels remained constant, and STAT1/2 showed diminished activity. Both variants showed similar STAT3 and NF-xB pathway activation following stimulation. These results show that the residue R at position 232 is important for cGAMP binding and IFN induction, and show that G207E mutants result in constitutive activation of STING signaling pathways and ligand-dependent hyperactivation of the NF-κB pathway. Patients with the R232 allele and G207E mutation had more severe disease; this polymorphism strengthens the constitutive activation of the mutant STING, leading to the overexpression of downstream targets, such as IFN, IL1-β and IL-18 (see, *e.g.,* Keskitalo *et al.* (2018), available from: doi.org/10.1101/394353).

67 amino acids in murine STING (SEQ ID NO:369) were mutated (see, Burdette et al. (2011) Nature 478(7370):515-518) either individually or in groups, to identify amino acids involved in cyclic di-GMP (c-di-GMP) binding and/or IFN induction. Among the mutants identified were hyperactive mutants R196A/D204A, S271A/Q272A, R309A/E315A, E315A, E315N, E315Q, and S271A (corresponding to R197A/D205A, S272A/Q273A, R310A/E316A, E316A, E316N, E316Q, and S272A, respectively, with reference to the sequence of human STING as set forth in SEQ ID NOs:305-309), that spontaneously induced IFN at low levels of transfection and did not respond to c-di-GMP, and the mutants R374A, R292A/T293A/E295A/E299A, D230A, R231A, K235A, Q272A, S357A/E359A/S365A, D230A/R231A/K235A/R237A, and R237A (corresponding to R375A, R293A/T294A/E296A (there is no equivalent to E299 in human STING), D231A, R232A, K236A, Q273A, S358A/E360A/S366A, D231A/R232A/K236A/R238A, and R238A, respectively, with reference to human STING, as set forth in SEQ ID NOs:305-309), that induced IFN when overexpressed but did not respond to c-di-GMP. These alleles can still respond to the endogenous CDN 2'3' c-di-GAMP, as it was later discovered that some human STING mutations have low affinity for the 3'3' CDNs produced by bacteria, such as c-di-GMP (see, *e.g.,* Corrales et al. (2015) Cell Reports 11:1018-1030).

The immunostimulatory bacteria provided herein that encode these proteins with gain-of-function mutations exploit the constitutive activation of these proteins to increase production of type I IFNs and pro-inflammatory cytokines. Tumor-targeting immunostimulatory bacteria are provided herein that encode STING, IRF3, IRF5, IRF7, MDA5, and/or RIG-I, with gain-of-function mutations. The immunostimulatory bacteria increase the production of type I IFN-mediated cytokines and chemokines in the tumor microenvironment, potentiating the anti-tumor immune response and improving the therapeutic efficacy of the immunostimulatory bacteria. The gene encoding STING is referred to as *TMEM173,* the gene encoding MDA5 is *IFIHI,* and the gene encoding RIG-I is *DDX58.* There are numerous alleles for each gene, and known mutations that can occur in genes with any of the alleles, resulting in gain-of-function or constitutive activation. The mutations listed below can occur singly, or can be used in any combination. Other mutations that result in gain-of-function can be identified by routine screening/mutation protocols. The table below lists exemplary gain-of-function mutations in each of STING/TMEM173 (SEQ ID NOs:305-309), MDA5/IFIH1 (SEQ ID NO:310), RIG-I/DDX58 (SEQ ID NO:311), IRF3 (SEQ ID NO:312), and IRF7 (SEQ ID NO:313). Other mutations, such as deletion of, or replacement of, a phosphorylation site or sites, such as S324/L325/S326 → S324A/L325/S326A in STING, and other replacements to eliminate a phosphorylation site to reduce nuclear factor-κB (NF-κB) signaling in STING, or other proteins that employ such signaling, also can be introduced.

The resulting proteins can be encoded in the immunostimulatory bacteria provided herein. The proteins are encoded on plasmids in the immunostimulatory bacteria.

Administering nucleic acids encoding wild-type STING can induce an immune response; the administration of gain-of-function STING mutants, with constitutive activity as provided herein, in tumor-targeted immunostimulatory bacteria, leads to a more potent immune response and more effective anti-cancer therapeutic. The enhanced immune response by the tumor-targeted administration of constitutively active STING, or other such modified DNA/RNA sensors, such as gain-of-function mutants of MDA5, RIG-I, IRF3, or IRF7, as provided herein, provides a therapeutically more effective anti-cancer treatment. For example, as described herein, modifying the immunostimulatory bacteria so that they do not infect epithelial cells, but retain the ability to infect phagocytic cells, including tumor-resident immune cells, effectively targets the immunostimulatory bacteria to the tumor microenvironment, improving therapeutic efficiency and preventing undesirable systemic immune responses. These tumor-targeted bacteria are engineered to encode gain-of-function STING, MDA5, RIG-I, IRF3, or IRF7 mutants, which are constitutively active, for example, even in the absence of ligand stimulation, providing a potent type I IFN response to improve the anti-cancer immune response in the tumor microenvironment.

Thus, for example, the administration of constitutively activated STING can provide an alternative means to boost STING signaling for the immunotherapeutic treatment of cancer. In certain embodiments, the tumor-targeting immunostimulatory bacteria provided herein can be modified to encode STING/TMEM173 (SEQ ID NOs: 305-309) with gain-of-function mutations, selected from S102P, V147L, V147M, N154S, V155M, G166E, R197A, D205A, R197A/D205A, C206Y, G207E, D231A, R232A, K236A, R238A, D231A/R232A/K236A/R238A, S272A, Q273A, S272A/Q273A, F279L, S102P/F279L, R281Q, R284G, R284S, R284M, R284K, R284T, R293A, T294A, E296A, R293A/T294A/E296A, R310A, E316A, E316N, E316Q, R310A/E316A, S324A/S326A, S358A, E360A, S366A, S358A/E360A/S366A, N154S/R284G, and R375A, as well as conservative mutations thereof. In addition, combinations of STING gain-of-function mutations can have significantly boosted STING signaling over their individual mutation counterparts.

**Table of Exemplary Gain-Of-Function Mutants**

| **Gain-of-Function Mutations Resulting in the Persistent Expression of Type I IFN** | | | | |
|---|---|---|---|---|
| **STING** | **RIG-I** | **MDA5** | **IRF3** | **IRF7** |
| V147L | E373A | T331I | S396D | S477D/S479D |
| N154S | C268F | T331R | S396D/S398D | S475D/S476D/S477D/S479D/S483D/S487D |
| V155M | | A489T | S396D/S398D/ S402D/T404D/ S405D | Δ247-467 |
| G166E | | R822Q | | S475D/S477D/S479D |
| C206Y | | G821S | | |
| G207E | | A452T | | |
| R281Q | | A946T | | |
| R284G | | R337G | | |
| R284S | | D393V | | |
| R284M | | G495R | | |
| R284K | | R720Q | | |
| R284T | | R779H | | |
| S102P/ F279L | | R779C | | |
| S102P | | L372F | | |
| F279L | | | | |
| R197A | | | | |
| D205A | | | | |
| R197A/ D205A | | | | |
| S272A/ Q273A | | | | |
| R310A/ E316A | | | | |
| R310A | | | | |
| E316A | | | | |
| E316N | | | | |
| E316Q | | | | |
| S272A | | | | |
| R375A | | | | |
| R293A | | | | |
| T294A | | | | |
| E296A | | | | |
| R293A/ T294A/ E296A | | | | |
| D231A | | | | |
| R232A | | | | |
| K236A | | | | |
| Q273A | | | | |
| S358A | | | | |
| E360A | | | | |
| S366A | | | | |
| S358A/ E360A/ S366A | | | | |
| D231A/ R232A/ K236A/ R238A | | | | |
| R238A | | | | |
| V147M | | | | |
| S324A/ S326A | | | | |
| N154S/R284G | | | | |

Amino acid residues R197, D205, R310, R293, T294, E296, S272, Q273, E316, D231, R232, K236, S358, E360, S366, and R238, with reference to the sequence of human STING, as set forth in any of SEQ ID NOs:305-309, correspond to amino acid residues R196, D204, R309, R292, T293, E295, S271, Q272, E315, D230, R231, K235, S357, E359, S365, and R237, respectively, with reference to the sequence of murine STING, as set forth in SEQ ID NO:369.

It is shown herein that the combination of replacements N154S/R284G results in constitutive expression of type I interferon. Also included are conservative substitutions of each of the replacements (see, Table in the Definitions section, listing exemplary conservative mutations for each amino acid).

### b. Constitutive IRF3 Expression and Gain-of-Function Mutations

IRF3 (interferon regulatory factor 3, or IRF-3) and IRF7 (or IRF-7) are key activators of type I IFN genes. Following virus-induced C-terminal phosphorylation (by TBK1), activated IRF3 and IRF7 form homodimers, translocate from the cytoplasm to the nucleus, and bind to IFN-stimulated response elements (ISREs) to induce type I IFN responses. IRF3 is expressed constitutively in unstimulated cells, and exists as an inactive cytoplasmic form, while IRF7 is not constitutively expressed in cells, and is induced by IFN, lipopolysaccharide, and virus infection. Overexpression of IRF3 significantly increases the virus-mediated expression of type I IFN genes, resulting in the induction of an antiviral state. IRF3 activation also has been shown to up-regulate the transcription of the CC-chemokine RANTES (CCL5) following viral infection (see, *e.g.,* Lin et al. (1999) Mol. Cell Biol. 19(4):2465-2474).

Residues S385, S386, S396, S398, S402, T404, and S405 in the C-terminal domain of IRF3 are phosphorylated after virus infection, inducing a conformational change that results in the activation of IRF3. IRF3 activation is induced, not only by viral infection, but also by lipopolysaccharide (LPS) and poly(I:C). Of the seven residues that can be phosphorylated in the C-terminal cluster of IRF3, a single point mutation, S396D, is sufficient for the generation of a constitutively active form of IRF3. IRF3(S396D) enhances the transactivation of IFNα1, IFN-β, and RANTES promoters by 13-, 14-, and 11-fold, respectively, compared to wild-type IRF3. Another mutant, IRF3(S396D/S398D), enhances the transactivation of IFNα1, IFN-β, and RANTES promoters by 13-, 12-, and 12-fold, respectively, compared to wild-type IRF3. Another constitutively active mutant of IRF3 is IRF3(5D), in which the serine or threonine residues at positions 396, 398, 402, 404, and 405 are replaced by phosphomimetic aspartic acid residues (IRF3(S396D/S398D/S402D/T404D/S405D)). Similar gain-of-function mutations, leading to constitutive activity of immune response mediators, such as induction of type I interferon, can be achieved by mutating serine residues to phosphomimetic aspartic acid in other proteins, such as RIG-I, MDA5, and STING, that are in immune response signaling pathways.

IRF3(5D) displays constitutive DNA binding and transactivation activities, dimer formation, association with the transcription coactivators p300 (also called EP300, or E1A binding protein p300)/ CBP (also known as CREB-binding protein, or CREBBP), and nuclear localization. Its transactivation activity is not induced further by virus infection. IRF3(5D) is a very strong activator of IFN-β and ISG-15 gene expression; IRF3(5D) alone stimulates IFN-β expression as strongly as virus infection, and enhances transactivation of IFNα1, IFN-β, and RANTES promoters by 9-fold, 5.5-fold, and 8-fold, respectively, compared to wild-type IRF3 (see, *e.g.,* Lin et al. (2000) J. Biol. Chem. 275(44):34320-34327; Lin et al. (1998) Mol. Cell Biol. 18(5):2986-2996; and Servant et al. (2003) J. Biol. Chem. 278(11):9441-9447). Any of positions S385, S386, S396, S398, S402, T404, and S405 can be mutated, alone or in combination, to produce constitutively active IRF3 mutants in the immunostimulatory bacteria provided herein.

### c. Non-Human STING Proteins, and Variants Thereof with Increased or Constitutive Activity, and STING Chimeras, and Variants Thereof with Increased or Constitutive Activity

As discussed above, cytosolic double-stranded DNA (dsDNA) stimulates the production of type I interferon (IFN) through the endoplasmic reticulum (ER)-resident adaptor protein STING (stimulator of IFN genes), which activates the transcription factor interferon regulatory factor 3 (IRF3). The TANK binding kinase 1 (TBK1)/IRF3 axis results in the induction of type I IFNs, and the activation of dendritic cells (DCs) and cross-presentation of tumor antigens to activate CD8⁺ T cell-mediated anti-tumor immunity. STING signaling also activates the nuclear factor kappa-light-chain-enhancer of activated B cell (NF-κB) signaling axis, resulting in a pro-inflammatory response, but not in the activation of the DCs and CD8⁺ T cells that are required for anti-tumor immunity.

Upon recognition of 2'3' cGAMP, STING translocates from the endoplasmic reticulum through the Golgi apparatus, allowing the recruitment of TANK-binding kinase 1 (TBK1) and activation of the transcription factors IRF3 and NF-κB. The carboxyl-terminal tail (C-terminal tail or CTT) region of STING is necessary and sufficient to activate TBK1 and stimulate the phosphorylation of IRF3; it also is involved in NF-κB signaling. The CTT is an unstructured stretch of approximately 40 amino acids that contains sequence motifs required for STING phosphorylation and recruitment of IRF3. IRF3 and NF-κB downstream signaling is attributed to specific sequence motifs within the C-terminal tail (CTT) of STING that are conserved among vertebrate species. Modular motifs in the CTT, which include IRF3-, TBK1- and TRAF6-binding modules, control the strength and specificity of cell signaling and immune responses.

Depending on the species and the respective characteristics of their STING CTT discrete elements, the IRF3 and NF-κB downstream responses can be affected, and sometimes opposite. The STING CTT elements dictate and finely tune the balance between the two signaling pathways, resulting in different biological responses. In human and mouse immune cells, for example, STING-dependent IRF3 activation results predominantly in a type I interferon response. STING signaling in human cells also drives a pro-inflammatory response through canonical and possibly non-canonical NF-κB pathways via TRAF6 recruitment. Human STING residue S366 (see, *e.g.,* SEQ ID NOs:305-309) is a primary TBK1 phosphorylation site that is part of an LxIS motif in the CTT, which is required for IRF3 binding, while a second PxPLR motif, including residue L374, is required for TBK1 binding. The LxIS and PxPLR motifs are highly conserved in all vertebrate STING alleles. In other species, STING signaling results predominantly in the activation of the NF-κB signaling axis. For example, the zebrafish CTT, which is responsible for hyperactivation of NF-κB signaling, contains an extension with a highly conserved PxExxD motif at the extreme C-terminus that is not present in human and other mammalian STING alleles; this motif shares similarity with tumor necrosis factor receptor-associated factor 6 (TRAF6) binding sites. While the role of TRAF6 in human STING signaling is non-essential, TRAF6 recruitment is essential for zebrafish STING-induced NF-κB activation. A human-zebrafish STING chimera, in which human STING was engineered to contain the zebrafish STING CTT module DPVETTDY, induced more than 100-fold activation of NF-κB activation, indicating that this region is necessary and sufficient to direct enhanced NF-κB signal activation. The addition of the zebrafish CTT also resulted in an increased STING interferon response (see, de Oliveira Mann et al. (2019) Cell Reports 27:1165-1175).

The differences among species in the balance between IRF3 and NF-κB signaling is exploited herein to produce modified STING proteins that have reduced NF-κB signaling, and/or optionally, increased IRF3 signaling, so that when the STING protein is delivered to and expressed in the TME, the resulting response is an increased anti-tumor/anti-viral response, compared to the unmodified STING protein.

In some embodiments, STING proteins from species that have low or no NF-κB signaling activity are provided in delivery vehicles, including any of the immunostimulatory bacteria described herein or known to those of skill in the art, as well as in other delivery vehicles, such as viral vectors, including oncolytic vectors, minicells, exosomes, liposomes, and in cells, such as T-cells that are used in cell therapy and used to deliver vehicles, such as bacteria and oncolytic vectors.

The non-human STING proteins can be, but are not limited to, STING proteins from the following species: Tasmanian devil (*Sarcophilus harrisii*; SEQ ID NO:349), marmoset (*Callithrix jacchus;* SEQ ID NO:359), cattle (*Bos taurus;* SEQ ID NO:360), cat (*Felis catus;* SEQ ID NO:356), ostrich (*Struthio camelus australis;* SEQ ID NO:361), crested ibis (*Nipponia nippon;* SEQ ID NO:362), coelacanth (*Latimeria chalumnae;* SEQ ID NOs:363-364), boar (*Sus scrofa*; SEQ ID NO:365), bat (*Rousettus aegyptiacus*; SEQ ID NO:366), manatee (*Trichechus manatus latirostris*; SEQ ID NO:367), ghost shark (*Callorhinchus milii;* SEQ ID NO:368), and mouse (*Mus musculus*; SEQ ID NO:369). These vertebrate STING proteins readily activate immune signaling in human cells, indicating that the molecular mechanism of STING signaling is shared in vertebrates (see, de Oliveira Mann et al. (2019) Cell Reports 27:1165-1175).

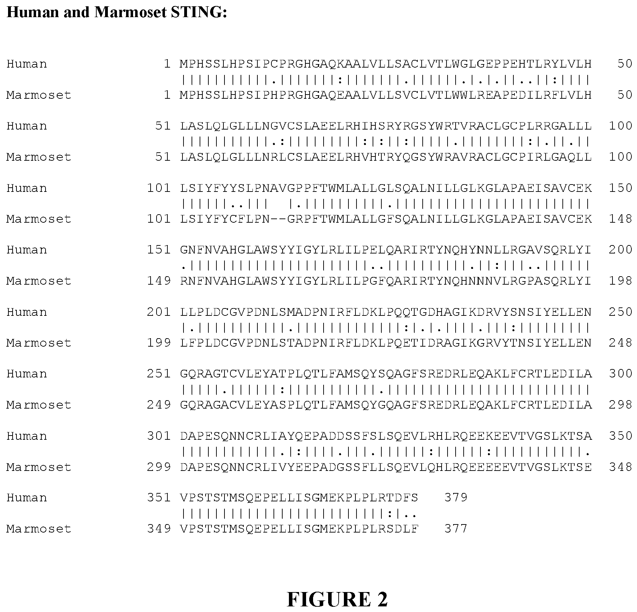

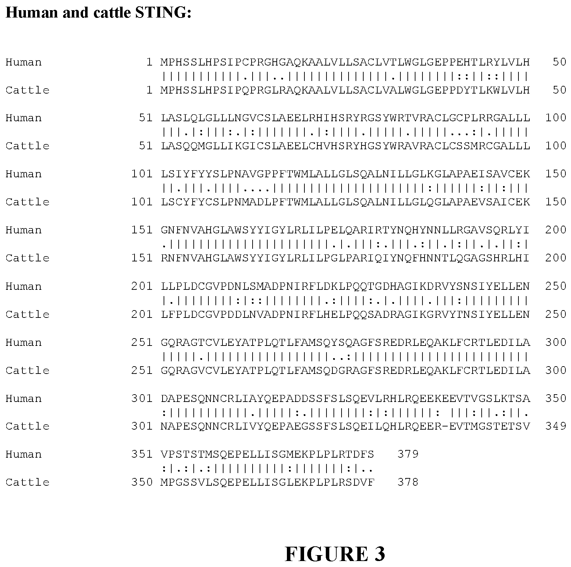

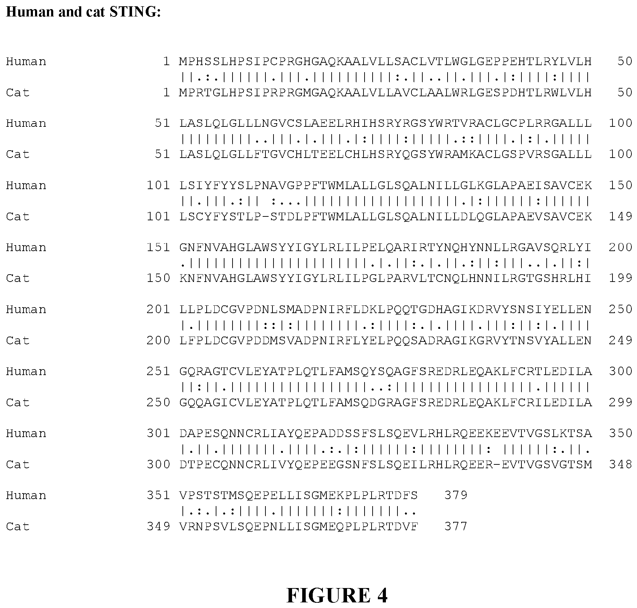

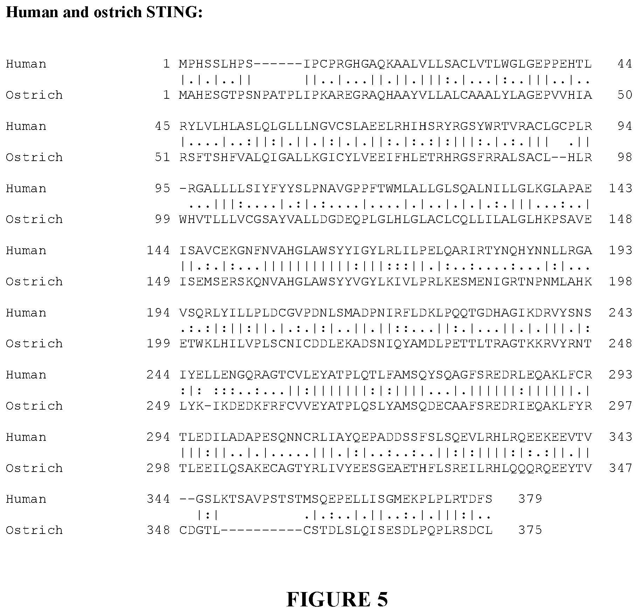

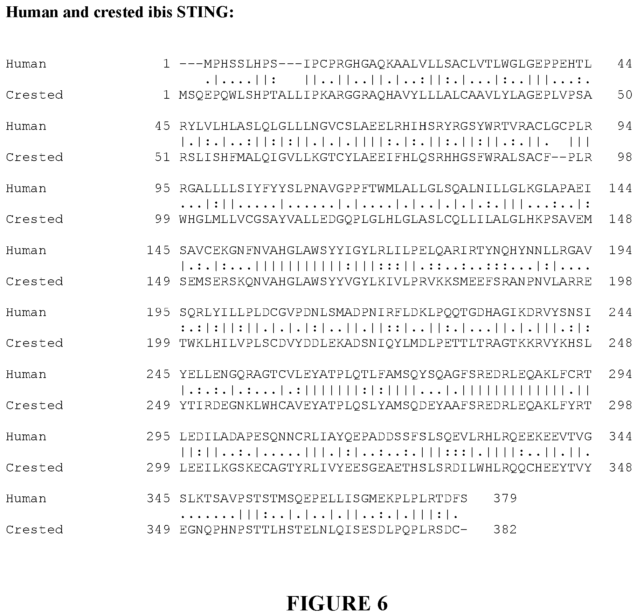

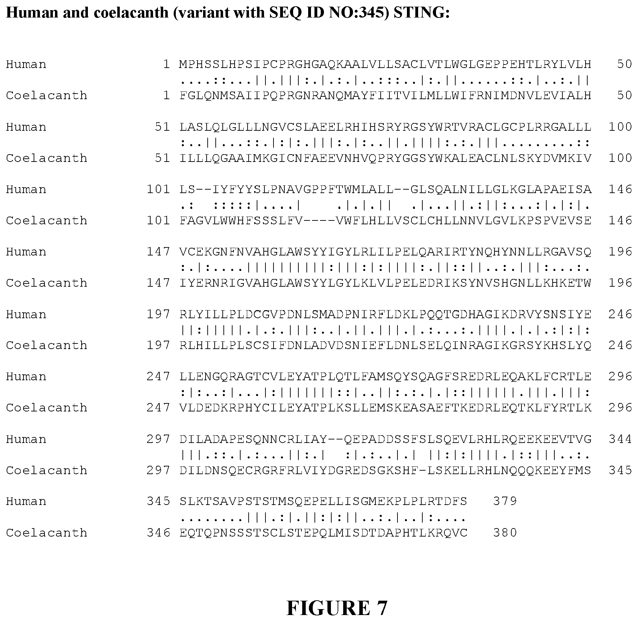

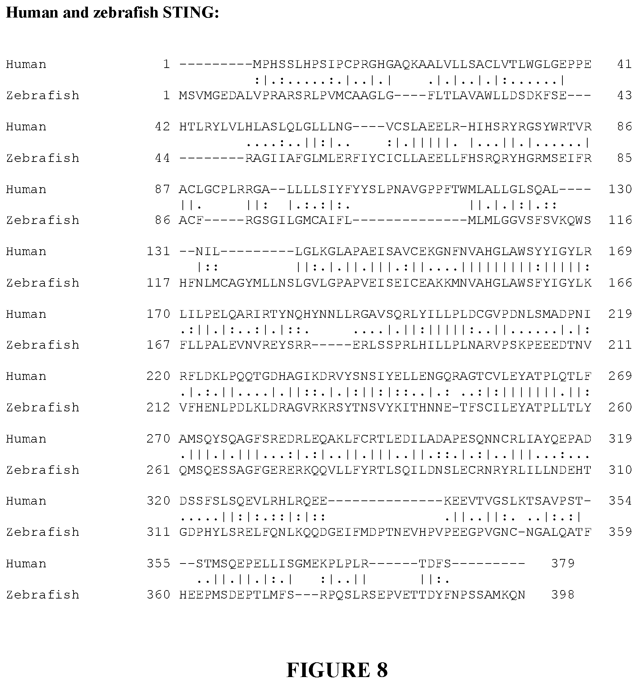

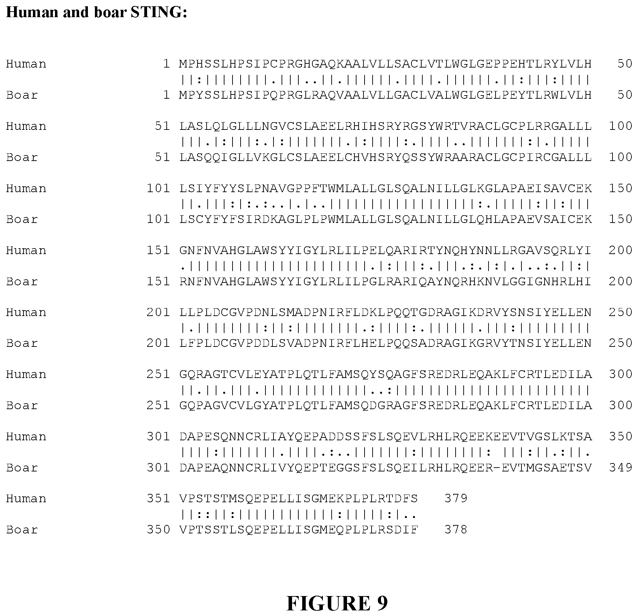

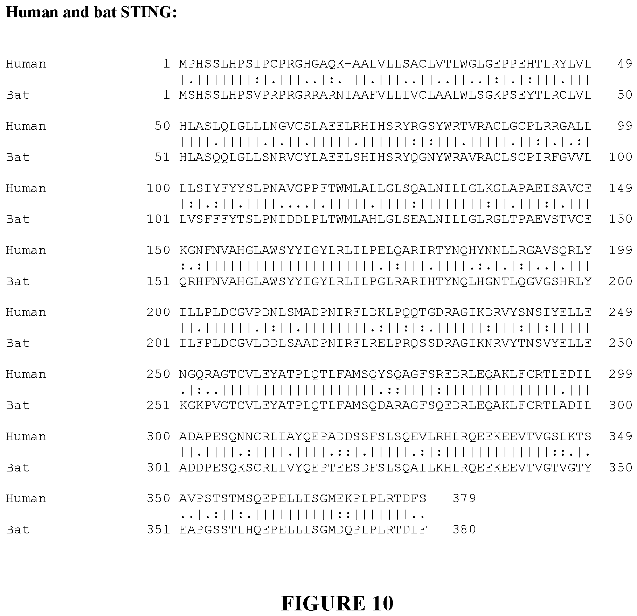

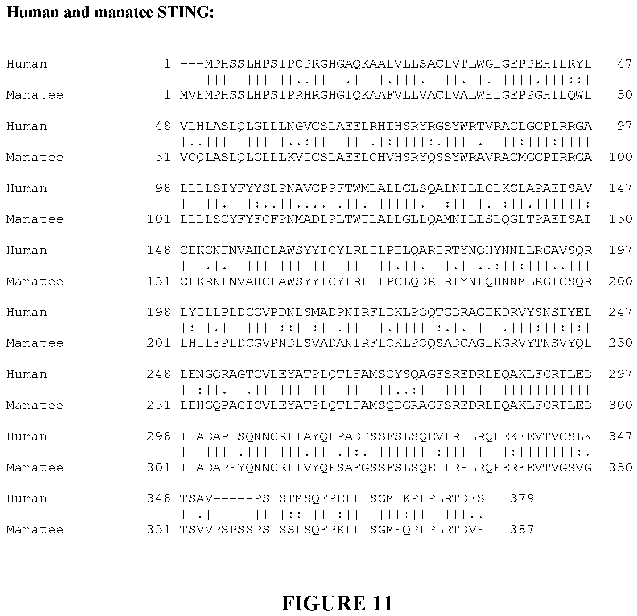

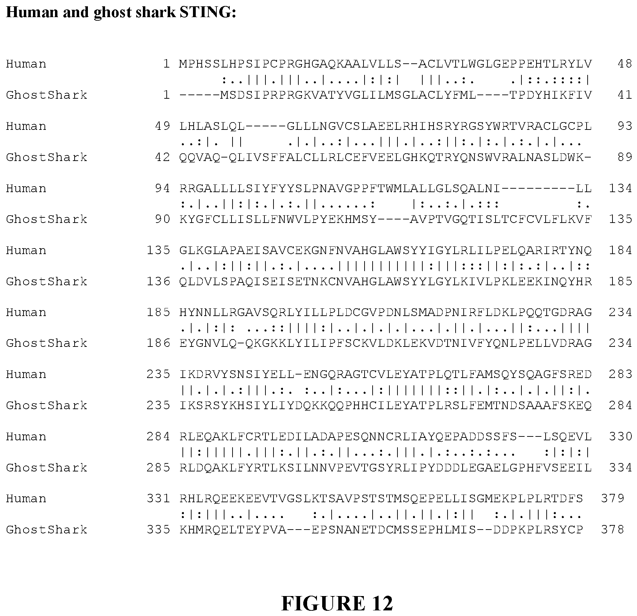

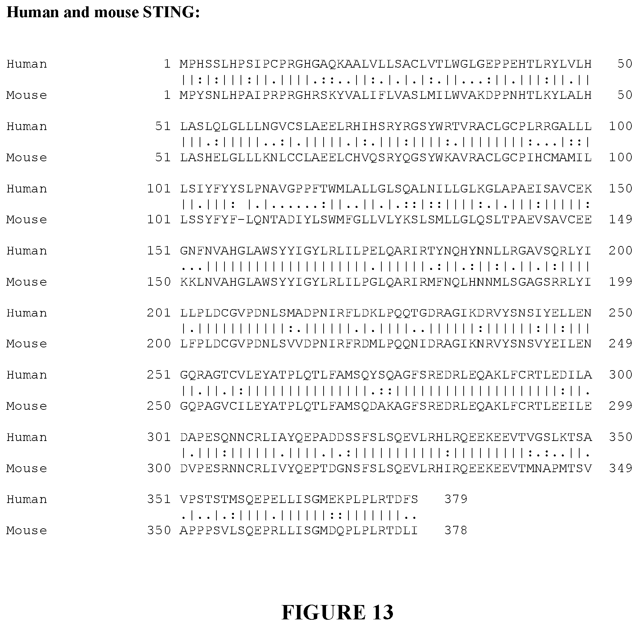

In other embodiments, the non-human STING proteins contain any of the constitutive STING activation and gain-of-function mutations, at corresponding loci in the non-human STING corresponding to those in human STING, described above (see, Example 17 below, which provides exemplary alignments and corresponding mutations in various species; see, also, Figures 1-13).

In other embodiments, chimeras of STING proteins are provided. In the chimeras, the CTT region, or portion(s) thereof that confers or participates in NF-κB signaling/activity, of a first species STING protein, is replaced with the corresponding CTT or portion(s) thereof from a second species, whose STING protein has lower or very little, less than human, NF-κB signaling activity. Generally, the first species is human, and the replacing CTT or portion(s) thereof is from the STING of a species such as Tasmanian devil, marmoset, cattle, cat, ostrich, boar, bat, manatee, crested ibis, coelacanth, and ghost shark, which have much lower NF-κB activity. This thereby results in a STING protein that induces type I interferon, which is important for anti-tumor activity, and that has limited or no NF-κB activity, which is not desirable in an anti-tumor therapy. The chimeras can further include the human constitutive STING activation and gain-of-function mutations in corresponding loci, to increase or render type I interferon activity constitutive. In all embodiments, the TRAF6 binding motif can be deleted to further decrease or eliminate activity that is not desirable in an anti-tumor therapeutic.

These non-human STING proteins, chimeras, and mutants are provided in delivery vehicles, such as any described herein or known to those of skill in the art, including oncolytic viral vectors, cells, such as stem cells and T-cells that are used in cell therapies, exosomes, minicells, liposomes, and the immunostimulatory bacteria provided herein, which accumulate in tumor-resident immune cells, and deliver encoded proteins to the tumor microenvironment and to tumors. The non-human STING proteins, modified STING proteins, and STING chimeras, are for use as therapeutics for the treatment of tumors as described herein, or for use in other methods known to those of skill in the art. Pharmaceutical compositions containing the STING proteins, delivery vehicles, and encoding nucleic acids also are provided.

### d. Other Gene Products that Act as Cytosolic DNA/RNA Sensors and Constitutive Variants Thereof

Other gene products that sense or interact with cytosolic nucleic acids are the retinoic acid-inducible gene I (RIG-I)-like receptors (RLRs), which include RIG-I and MDA5 (melanoma differentiation-associated protein 5). RLRs are cytoplasmic sensors of viral dsRNA and nucleic acids secreted by bacteria, and include RIG-I, MDA5, and LGP2 (laboratory of genetics and physiology 2). Upon the binding of a ligand, such as a viral dsRNA, RIG-I and MDA5 activate the mitochondrial antiviral-signaling adaptor protein, or MAVS, which recruits tumor necrosis factor (TNF) receptor-associated factors (TRAFs), to assemble a signaling complex at the outer membranes of the mitochondria. Downstream signaling components further are recruited by TRAFs, resulting in the phosphorylation and activation of IRF3 (interferon regulatory factor 3), IRF7, NF-κB (nuclear factor kappa-light-chain-enhancer of activated B cells), and AP-1 (activator protein 1). As a result, the expression of IFNs, proinflammatory cytokines, and other genes involved in pathogen clearance, is induced (see, *e.g.,* Lu and MacDougall (2017) Front. Genet. 8:118). Like STING, the constitutive activation of MDA5 and RIG-I due to gain-of-function mutations leads to the induction of type I IFNs, which can be leveraged to enhance the anti-tumor immune response in the immunostimulatory bacteria.

### i. RIG-I

Retinoic acid-inducible gene I (RIG-I), also known as DDX58 (DEXD/H-box helicase 58), is another protein whose constitutive activation has been linked to the development of interferonopathies, such as atypical Smith-Magenis syndrome. RIG-I, like MDA5/IFIH1, is a member of the RIG-I-like receptor (RLR) family, and is a 925-residue cytosolic pattern recognition receptor that functions in the detection of viral dsRNA. RIG-I initiates an innate immune response to viral RNA through independent pathways that promote the expression of type I and type III IFNs and proinflammatory cytokines (see, *e.g.,* Jang et al. (2015) Am. J. Hum. Genet. 96:266-274; and Lu and MacDougall (2017) Front. Genet. 8:118)*.*

Atypical Smith-Magenis syndrome, without hallmark dental anomalies, but with variable phenotypes, including glaucoma, aortic calcification, and skeletal abnormalities, has been found to be caused by mutations in the DEXD/H-box helicase 58 gene (*DDX58*), which encodes retinoic acid-inducible gene I (RIG-I). In particular, the mutations E373A and C268F in *DDX58* were identified as causing gain-of-function in RIG-I. Elevated amounts of mutated DDX58 were associated with a significant increase in the basal levels of NF-xB reporter gene activity, and this activity was further increased by stimulation with the dsRNA analog poly(I:C). The RIG-I mutations also induced IRF3 phosphorylation and dimerization at the basal level, and led to increased expression of *IFNB1,* interferon-stimulated gene 15 (*ISG15*)*,* and chemokine (C-C motif) ligand 5 (*CCL5*) in both basal, and poly(I:C) transfected HEK293FT cells. These results indicate that the mutated DDX58/RIG-I results in constitutive activation, leading to increased IFN activity and IFN-stimulated gene expression (see, *e.g.,* Jang et al. (2015) Am. J. Hum. Genet. 96:266-274; and Lu and MacDougall (2017) Front. Genet. 8:118). Tumor-targeting immunostimulatory bacteria provided herein can be modified to encode RIG-I/DDX58 (see, *e.g.,* SEQ ID NO:311) with gain-of-function mutations such as, but not limited to, E373A and C268F, singly and in combination.

### ii. MDA5/IFIH1

Another interferonopathy gene is the IFN-induced with helicase C domain-containing protein 1 (IFIH1), also known as melanoma differentiation-associated protein 5 (MDA5), which is a member of the RIG-I-like family of cytoplasmic DExD/H box RNA receptors. MDA5, encoded by *IFIH1,* is a 1,025 amino acid cytoplasmic pattern-recognition receptor that senses viral double-stranded RNA (dsRNA) and secreted bacterial nucleic acids in the cytoplasm, and activates type I IFN signaling through an adaptor molecule, MAVS (mitochondrial antiviral-signaling protein). MAVS recruits tumor necrosis factor (TNF) receptor-associated factors (TRAFs), which in turn recruit downstream signaling components, resulting in the phosphorylation and activation of IRF3 (interferon regulatory factor 3), IRF7, NF-κB (nuclear factor kappa-light-chain-enhancer of activated B cells), and AP-1 (activator protein 1). This results in the expression of IFNs, proinflammatory cytokines, and other genes involved in pathogen clearance (see, *e.g.,* Rutsch et al. (2015) Am. J. Hum. Genet. 96:275-282; Rice et al. (2014) Nat. Genet. 46(5):503-509; and Lu and MacDougall (2017) Front. Genet. 8:118).

Gain-of-function (GOF) *IFIH1* variants occur in subjects with autoimmune disorders, including Aicardi-Goutiéres syndrome (AGS) and Singleton-Merten syndrome (SMS), which are characterized by prominent vascular inflammation. AGS is an inflammatory disease particularly affecting the brain and skin, and is characterized by an upregulation of interferon-induced transcripts. AGS typically occurs due to mutations in any of the genes encoding DNA exonuclease TREX1, the three non-allelic components of the RNase H2 endonuclease complex, the deoxynucleoside triphosphate triphosphohydrolase SAMHD1, and the double-stranded RNA editing enzyme ADAR1. Some patients with AGS do not have mutations in any of these genes, but have GOF mutations in *IFIH1,* indicating that this gene also is implicated in AGS. Singleton-Merten syndrome is an autosomal-dominant disorder characterized by abnormalities in the blood vessels (*e.g*., calcification), teeth (*e.g.,* early-onset periodontitis, root resorption), and bones (*e.g.*, osteopenia, acro-osteolysis, osteoporosis). Interferon signature genes are upregulated in Singleton-Merten syndrome patients, which was linked to GOF mutations in *IFIH1* (see, *e.g.,* Rice et al. (2014) Nat. Genet. 46(5):503-509; and Rutsch et al. (2015) Am. J. Hum. Genet. 96:275-282).

The IFN-β reporter stimulatory activity of wild-type IFIH1, and six IFIH1 GOF mutants identified in AGS patients (R720Q, R779H, R337G, R779C, G495R, D393V), was compared in HEK293T cells, which express low levels of endogenous viral RNA receptors. Wild-type IFIH1 was induced upon binding of the long (> 1 kb) dsRNA analog polyinosinic-polycytidylic acid (poly(I:C)), but not by a short 162 bp dsRNA, and had minimal activity in the absence of exogenous RNA. The IFIH1 mutants displayed a significant induction of IFN signaling in response to the short 162 bp dsRNA, in addition to robust signaling in response to poly(I:C). The mutants also displayed a 4-10 fold higher level of baseline signaling activity in the absence of exogenous ligand (see, *e.g.,* Rice et al. (2014) Nat. Genet. 46(5):503-509).

Another gain-of-function *IFIH1* mutation, R822Q, was identified as causing Singleton-Merten syndrome by triggering type I IFN production, and leading to early arterial calcification, as well as dental inflammation and resorption. HEK293T cells (which have the lowest endogenous *IFIH1* expression levels) were used to overexpress wild-type and R822Q MDA5. Wild-type *IFIH1* expression led to an increase in the expression of *IFNB1* (interferon, beta 1, fibroblast) in a dose-dependent manner, whereas the mutated *IFIH1* led to approximately 20-fold more *IFNB1* expression. Following stimulation with the dsRNA analog poly(I:C), R822Q *IFIH1* resulted in higher levels of *IFNB1* expression than wild-type *IFIH1,* indicating that R822Q *IFIHI* is hyperactive to non-self dsRNA. There was also higher expression of interferon signature genes, such as *IFI27, IFI44L, IFIT1, ISG15, RSG15, RSAD2,* and *SIGLEC1,* in whole-blood samples from Singleton-Merten syndrome patients, which was in agreement with the higher expression level of *IFNB1* by R822Q *IFIH1* (see, *e.g.,* Rutsch et al. (2015) Am. J. Hum. Genet. 96:275-282).

The interferon signature observed in patients with another *IFIH1* GOF mutation, A489T, is indicative of a type I interferonopathy; *IFIH1* A489T is associated with increased interferon production and phenotypes resembling chilblain lupus, AGS and SMS (see, *e.g.,* Bursztejn et al. (2015) Br. J. Dermatol. 173(6):1505-1513). The A489T variant not only resulted in IFN induction following stimulation with the long dsRNA analog poly(I:C), but also with short dsRNA. Two additional gain-of-function mutations in *IFIH1,* T331I and T331R, were identified in patients with SMS phenotypes, who presented with a significant upregulation of IFN-induced transcripts. The T331I and T331R variants resulted in increased expression of IFN-β, even in the absence of exogenous dsRNA ligand, consistent with the observed constitutive activation of MDA5 (see, *e.g.,* Lu and MacDougall (2017) *Front. Genet.* 8:118).

A946T is another *IFIH1* GOF mutation that leads to the increased production of type I IFN, promoting inflammation and increasing the risk of autoimmunity. The A946T mutation in *IFIH1* results in additive effects when combined with the *TMEM173* R232 allele and G207E GOF mutation in STING, leading to a severe early-onset phenotype with features similar to SAVI (see, *e.g.,* Keskitalo *et al.* (2018) preprint, available from doi.org/10.1101/394353). G821S is a GOF mutation in *IFIH1* which has been shown to lead to spontaneously developed lupus-like autoimmune symptoms in a mouse model (see, *e.g.,* Rutsch et al. (2015) Am. J. Hum. Genet. 96:275-282), while the *IFIH1* missense mutations A452T, R779H and L372F, identified in individuals with AGS, were shown to cause type I interferon overproduction (see, *e.g.,* Oda et al. (2014) Am. J. Hum. Genet. 95:121-125).

The tumor-targeting immunostimulatory bacteria provided herein can be modified to encode MDA5/IFIH1 (see, *e.g.,* SEQ ID NO:310) with gain-of-function mutations selected from T331I, T331R, R337G, L372F, D393V, A452T, A489T, G495R, R720Q, R779H, R779C, G821S, R822Q, and A946T, singly or in any combination.

### iii. IRF7

Constitutively active forms of IRF7 (or IRF-7) include mutants in which different C-terminal serines are substituted by phosphomimetic Asp, including IRF7(S477D/S479D), IRF7(S475D/S477D/S479D), and IRF7(S475D/S476D/S477D/S479D/S483D/S487D). IRF7(S477D/S479D) is a strong transactivator for IFNA and RANTES gene expression, and stimulates gene expression, even in the absence of virus infection. IRF7(S475D/S477D/S479D), and IRF7(S475D/S476D/S477D/S479D/S483D/S487D) do not further augment the transactivation activity of IRF7(S477D/S479D), but the transactivation activity of all three mutants is stimulated further by virus infection. The mutant IRF7(Δ247-467), which localizes to the nucleus in uninfected cells, is a very strong constitutive form of IRF7; it activates transcription more than 1500-fold higher than wild-type IRF7 in unstimulated and virus-infected cells (see, *e.g.,* Lin et al. (2000) J. Biol. Chem. 275(44):34320-34327).

The immunostimulatory bacteria provided herein can encode and express constitutively active IRF7 mutants, including those with replacements at residues 475-477, 479, 483, and 487, and those with amino acid deletions. The immunostimulatory bacteria encode these proteins on plasmids under the control of promoters and any other desired regulatory signals recognized by mammalian hosts, including humans.

### e. Other Type I IFN Regulatory Proteins

Other proteins involved in the recognition of DNA/RNA that activate type I IFN responses can be mutated to generate constitutive type I IFN expression. The unmodified and/or modified proteins can be encoded in the immunostimulatory bacteria provided herein, to be used to deliver the protein to the tumor microenvironment, such as to tumor-resident immune cells, to increase expression of type I IFN.

These proteins include, but are not limited to, proteins designated TRIM56, RIP1, Sec5, TRAF2, TRAF3, TRAF6, STAT1, LGP2, DDX3, DHX9 (DDX9), DDX1, DDX21, DHX15, DHX33, DHX36, DDX60, and SNRNP200.

| **Gene** | **Encoded Protein** | **Activity/Function** |
|---|---|---|
| TRIM56 | Tripartite motif-containing protein 56/ E3 ubiquitin-protein ligase TRIM56 | Promotes dimerization of STING in response to dsDNA stimulation, resulting in production of IFN-β; potentiates extracellular dsRNA-induced expression of IFNB1 and IFN-stimulated genes *ISG15, IFIT1*/*ISG56, CXCL10, OASL* and *CCL5*; positive regulator of TLR3 signaling |
| RIP1/RIPK1 | Receptor-interacting serine/threonine protein (kinase) 1 | Transduces inflammatory and cell-death signals (programmed necrosis) following death receptor ligation, activation of pathogen recognition receptors and DNA damage; indirectly activates NF-κB; directs LPS-induced IFN-β synthesis in mice |
| Sec5 (EXOC2) | Exocyst complex component 2 | Component of exocyst complex, involved in docking of exocytic vesicles with fusion sites on plasma membrane; co-localizes with STING and TBK1 after intracellular DNA stimulation, inducing type I IFN production |
| TRAF2 | TNF receptor-associated factor 2 | Regulates activation of NF-κB and JNK/MAPK8; mediates type I IFN induction |
| TRAF3 | TNF receptor-associated factor 3 | Regulates activation of NF-κB and MAP kinases; mediates activation of IRF3; mediates type I IFN induction; mediates cytokine production |
| TRAF6 | TNF receptor-associated factor 6 | Activates NF-κB, JUN and AP-1; induces type I IFN production in response to viral infection and intracellular dsRNA; induces production of proinflammatory cytokines |
| STAT1 | Signal transducer and activator of transcription 1 | Forms part of ISGF3 transcription factor, which binds IFN-stimulated response elements (ISREs) to activate transcription of IFN-stimulated genes (ISGs) |
| LGP2 (DHX58) | Laboratory of genetics and physiology 2 / Probable ATP-dependent RNA helicase DHX58 | Regulates RIG-I/DDX58- and IFIH1/MDA5-mediated antiviral signaling |
| DDX3 (DDX3X) | ATP-dependent RNA helicase DDX3X | Promotes production of type I IFN; acts as viral RNA sensor; involved in TBK1 and IKBKE-dependent |
| | | IRF3 activation, leading to induction of IFNB; associates with IFNB promoters; associates with MAVS and RIG-I to induce signaling in early stages of infection; binds MDA5 to enhance its recognition of dsRNA |
| DHX9/DDX9 | DExD/H-box helicase 9 / ATP-dependent RNA helicase A | Senses viral nucleic acids; triggers host responses to non-self DNA in MyD88-dependent manner; interacts with MAVS to stimulate NF-κB-mediated innate immunity against virus infection and activate IRF3 and MAPK pathways; potentiates virus-triggered induction of IL-6 and IFN-β |
| DDX1 | ATP-dependent RNA helicase DDX1 | Component of a multi-helicase-TRIF complex that senses viral double-stranded RNA (dsRNA), activates the NF-κB signaling pathway, and induces production of type I IFN and proinflammatory cytokines |
| DDX21 | Nucleolar RNA helicase 2 | Component of a multi-helicase-TRIF complex that senses viral double-stranded RNA (dsRNA), activates the NF-κB signaling pathway, and induces production of type I IFN and proinflammatory cytokines |
| DHX15 (DDX15) | Pre-mRNA-splicing factor ATP-dependent RNA helicase DHX15 | Viral RNA sensor that interacts with MAVS to induce type I IFN and proinflammatory cytokine production; activates IRF3, NF-κB, and MAPK signaling |
| DHX33 (DDX33) | ATP-dependent RNA helicase DHX33 | Viral dsRNA sensor that interacts with MAVS and triggers type I IFN response; activates NF-κB, IRF3, and MAPK signaling pathways; activates NLRP3 inflammasome, resulting in secretion of proinflammatory cytokines |
| DHX36 (DDX36) | ATP-dependent DNA/RNA helicase DHX36 | Component of a multi-helicase-TRIF complex that senses viral double-stranded RNA (dsRNA), activates the NF-κB signaling pathway, and induces production of type I IFN and proinflammatory cytokines |
| DDX60 | Probable ATP-dependent RNA helicase DDX60 | Senses viral RNA and DNA; forms complex with RIG-I like receptors to promote antivirus activity; positively regulates RIG-I and MDAS-dependent type I IFN and IFN-inducible gene expression in response to viral infection; binds ssRNA, dsRNA, and dsDNA; promotes binding of RIG-I to dsRNA |
| SNRNP200 | U5 small nuclear ribonucleoprotein 200 kDa helicase | Senses/binds viral RNA and interacts with TBK1 to promote IRF3 activation and type I IFN production |

Gain-of-function variants can be produced, such as by screening and/or by mutagenesis. Site-directed mutagenesis can be performed *in vitro* to identify mutations with enhanced activity, that lead to higher level and/or constitutive type I IFN expression. Intact genomic DNA can be obtained from non-related patients experiencing auto-immune and auto-inflammatory symptoms, and from healthy individuals, to screen for and identify other products whose expression leads to increased or constitutive type I IFN expression. Whole exome sequencing can be performed, and introns and exons can be analyzed, such that proteins with mutations in the pathways associated with the increased or constitutive expression of type I interferon are identified. After identification of mutations, cDNA molecules encoding the full-length gene, with and without the identified mutation(s), are transfected into a reporter cell line that measures expression of type I interferon. For example, a reporter cell line can be generated where the expression of luciferase is placed under control of the promoter for IFN-β. A gain-of-function mutant that is constitutively active will promote the expression of IFN-β, whereas the unstimulated wild-type protein will not. Stimulation can be by virus infection, bacterial infection, bacterial nucleic acids, LPS, dsRNA, poly(I:C), or by increasing exogenous levels of the protein's ligand (*e.g*., CDNs). Identified proteins also include those that enhance an immune response to an antigen(s) of interest in a subject. The immune response comprises a cellular or humoral immune response characterized by one or more of: (i) stimulating type I interferon pathway signaling; (ii) stimulating NF-κB pathway signaling; (iii) stimulating an inflammatory response; (iv) stimulating cytokine production; (v) stimulating dendritic cell development, activity, or mobilization; (vi) any other responses indicative of a product whose expression enhances an immune response; and (vii) a combination of any of (i)-(vi).

### 4. Antibodies and Antibody Fragments

Advances in antibody engineering have led to the creation of recombinant antibody fragments that have many improvements over conventional monoclonal antibodies, especially in terms of manufacturing, tissue penetration, and ease of use. An example of these is the single-chain fragment variable (scFv), consisting of the variable regions of the heavy (V_{H}) and light (V_{L}) chains of the antibody binding site, joined together by a flexible peptide linker that is generally the (G₄S)₃ sequence (see, *e.g.,* Weisser et al. (2009) Biotechnol. Adv. 27(4):502-520). Other examples include scFv-Fc antibody fragments, in which the V_{H} domain of the scFv is linked to an Fc region. Antibody fragments such as this allow for targeting of antigens in a manner that can be encoded on a plasmid and delivered, as exemplified herein, by an immunostimulatory bacterium. Examples of potential antigens to target, include, but are not limited to, the following listed below.

### a. TGF-β

Transforming growth factor beta (TGF-β) is a pleiotropic cytokine with numerous roles in embryogenesis, wound healing, angiogenesis, and immune regulation. It exists in three isoforms in mammalian cells, TGF-β1, TGF-β2, and TGF-β3; TGF-β1 is the most predominant in immune cells (see, *e.g.,* Esebanmen et al. (2017) Immunol. Res. 65:987-994). TGF-β's role as an immunosuppressant is arguably its most dominant function. Its activation from a latent form in the tumor microenvironment, in particular, has profound immunosuppressive effects on DCs and their ability to tolerize antigen-specific T-cells. TGF-β also can directly convert Th1 CD4⁺ T-cells to immunosuppressive Tregs, further promoting tumor tolerance (see, *e.g.,* Travis et al. (2014) Annu. Rev. Immunol. 32:51-82). Based on its tumor-specific immunosuppressive functions, and irrespective of its known cancer cell growth and metastasis-promoting properties, inhibition of TGF-β is a cancer therapy target. High levels of TGF-β signaling have been demonstrated in several human tumor types, including colorectal cancer (CRC), hepatocellular carcinoma (HCC), pancreatic ductal adenocarcinoma (PDAC), and non-small-cell lung cancer (NSCLC) (see, *e.g.,* Colak et al. (2017) Trends Cancer 3(1):56-71). Systemic inhibition of TGF-β can lead to unacceptable autoimmune toxicities, and its inhibition should be localized to the tumor microenvironment. One way to accomplish this is to create a soluble TGF-β receptor that acts as a decoy for binding TGF-β (see, *e.g.,* Zhang et al. (2008) J. Immunol. 181:3690-3697). As such, a tumor-targeting immunostimulatory bacteria, containing a TGF-β receptor decoy, provided herein, can bind and remove TGF-β from the tumor microenvironment, thereby breaking tumor immune tolerance and stimulating anti-tumor immunity.

In addition to TGF-beta binding decoy receptors, other TGF-beta polypeptide antagonists, that can bind and remove TGF-β from the tumor microenvironment, thereby breaking tumor immune tolerance and stimulating anti-tumor immunity, include anti-TGF-beta antibodies or antibody fragments, anti-TGF-beta receptor antibodies or antibody fragments, and soluble TGF-beta antagonist polypeptides.

Provided herein are immunostimulatory bacteria, that accumulate in the tumor microenvironment, in tumors, and in particular, in tumor-resident immune cells, that contain plasmids encoding TGF-beta polypeptide antagonists, including, for example, TGF-beta binding decoy receptors (TGF-β receptor decoys), anti-TGF-beta antibodies or antibody fragments, anti-TGF-beta receptor antibodies or antibody fragments, and soluble TGF-beta antagonist polypeptides. The antibody fragments can include any known in the art, or described herein, such as, but not limited to, scFvs and scFv-Fcs.

### b. Bispecific scFvs and T-Cell Engagers

The use of scFvs has been improved by increasing the valency of binding to the target, often through the use of one or more scFv fragments (bi-specific, tri-specific, etc.), joined together by a long linker. Bi-specific T-cell engager (sold under the trademark BiTE^{®}) constructs are a class of artificial bispecific monoclonal antibodies that are utilized in cancer immunotherapy, and are formed by linking two single-chain variable fragments (scFvs), such that one scFv binds CD3 on the surface of cytotoxic T-cells, and the other binds a specific tumor-associated antigen. BiTEs^{®} thus target T-cells to tumor cells, stimulating T-cell activation, cytokine production, and tumor cell cytotoxicity, independently of MHC class I or co-stimulatory molecules. Two examples of BiTEs^{®} have been approved by the FDA, including catumaxomab, which is directed against the tumor antigen EpCAM, and CD3, and is used in the treatment of malignant ascites, and blinatumomab, a BiTE^{®} antibody against CD19 and CD3, which is used for the treatment of relapsed, refractory acute lymphoblastic leukemia (ALL) (see, *e.g.,* Ahamadi-Fesharaki et al. (2019) Mol. Ther. Oncolytics 14:38-56). Other BiTEs^{®} target other antigens, including carcinoembryonic antigen (CEA), prostate-specific membrane antigen (PSMA), EGFR, EphA2, Her2, ADAM17/TACE, prostate stem cell antigen (PSCA), and melanoma-associated chondroitin sulfate proteoglycan (MCSP). As exemplified herein, a BiTE^{®} antibody also can be expressed from a plasmid following delivery by an immunostimulatory bacterium.

### c. Anti-PD-1/Anti-PD-L1 Antibodies

Programmed cell death protein 1 (PD-1) is an immune-inhibitory receptor that is involved in the negative regulation of immune responses. Its cognate ligand, programmed death-ligand 1 (PD-L1), is expressed on antigen-presenting cells (APCs), and upon binding to PD-1 on T-cells, leads to loss of CD8⁺ T-cell effector function, inducing T-cell tolerance. The expression of PD-L1 is often associated with tumor aggressiveness and reduced survival in certain human cancers (see, *e.g.,* Gao et al. (2009) Clin. Cancer Res. 15(3):971-979).

Antibodies designed to block immune checkpoints, such as anti-PD-1 (for example, pembrolizumab, and nivolumab) and anti-PD-L1 (for example, atezolizumab, avelumab, and durvalumab) antibodies, can prevent T-cell anergy and break immune tolerance. Only a fraction of treated patients, however, exhibit clinical benefit, and those that do, often present with autoimmune-related toxicities (see, *e.g.,* Ribas (2015) N. Engl. J. Med. 373(16):1490-1492; and Topalian et al. (2012) N. Engl. J. Med. 366(26):2443-2454). Besides acquiring toxicity, anti-PD-1/anti-PD-L1 therapy often leads to resistance, and the concomitant use of anti-CTLA-4 antibodies (for example, ipilimumab) has shown limited success in clinical trials, with significantly additive toxicity. To limit the toxicity and enhance the potency of PD-1/PD-L1 blockade, an immunostimulatory bacterium, containing a plasmid encoding an antibody or antibody fragment, such as an scFv or scFv-Fc, and others known in the art or described herein, against PD-1 or against PD-L1, will synergize with activation of immune cells to potentiate anti-tumor immunity.

### d. Anti-CTLA-4 Antibodies

CTLA-4 (cytotoxic T-lymphocyte-associated protein 4), also known as CD152 (cluster of differentiation 152), is another immune-inhibitory receptor that functions as an immune checkpoint, and downregulates immune responses. CTLA-4 is constitutively expressed in regulatory T-cells (Tregs, or T_{regs}), and contributes to their inhibitory function, but is upregulated in conventional T-cells only after activation. CTLA-4 functions as an immune checkpoint by transmitting inhibitory signals to T-cells. CTLA-4 is homologous to the T-cell co-stimulatory protein, CD28, and both molecules bind to CD80 (also known as B7-1 or B7.1) and CD86 (also known as B7-2 or B7.2) ligands on antigen-presenting cells (APCs). The binding of CTLA-4 to the ligands transmits an inhibitory signal to T-cells, whereas the binding of CD28 transmits a stimulatory signal.

Following T-cell activation, CTLA-4 receptors are induced, which then outcompete CD28 receptors on T-cells, for binding to CD80 and CD86 ligands on the surfaces of APCs. CTLA-4 binds to CD80 and CD86 with greater affinity and avidity than CD28, thus enabling it to outcompete CD28 for its ligands, resulting in the transmittal of inhibitory signals to T-cells, and an immune inhibitory response. T-cell activation through the T-cell receptor and CD28 leads to increased expression of CTLA-4.

Optimal T-cell priming requires co-stimulatory signals resulting from the ligation of T-cell CD28 with CD80 and/or CD86. The blockade of CTLA-4 from binding to these ligands thus enhances T-cell priming, and allows for the induction of an anti-tumor immune response.

In some embodiments, the immunostimulatory bacterial strains provided herein contain plasmids encoding anti-CTLA-4 antibodies, including fragments thereof, such as, but not limited to, anti-CTLA-4 scFvs (see, *e.g.,* SEQ ID NO:403 for an exemplary human anti-CTLA-4 scFv fragment), and anti-CTLA-4 scFv-Fcs (see, *e.g.,* SEQ ID NO:402, for an exemplary human anti-CTLA-4 scFv-Fc fragment; see, also, Example 20).

### e. Additional Exemplary Checkpoint Targets

Exemplary immune checkpoint targets, for which an scFv, or any other recombinant antibody fragment against them can be prepared, or are exemplified herein include, but are not limited to, those listed in the table below:

| **Checkpoint target** |
|---|
| CTLA-4 |
| PD-L1 (B7-H1) |
| PD-L2 |
| PD-1, PD-2 |
| IDO1 |
| IDO2 |
| SIRP alpha (CD47) |
| VISTA (B7-H5) |
| LIGHT |
| HVEM |
| CD28 |
| LAG3, TIM3, TIGIT |
| Galectin-9 |
| CEACAM1, CD155, CD112, CD226, CD244 (2B4) |
| B7-H2, B7-H3, CD137, ICOS, GITR, B7-H4, B7-H6 |
| CD137, CD27, CD40, CD40L, CD48, CD70, CD80, CD86, CD137 (4-IBB), 4-IBBL, CD200, CD272 (BTLA), CD160 |
| A2a receptor, A2b receptor, HHLA2, ILT-2, ILT-4, gp49B, PIR-B |
| OX40, OX-40L, HLA-G, ILT-2/4 |
| KIR, TIM1, TIM4 |
| CLEVER-1/Stabilin-1 |

### 5. Combinations of Immunomodulatory Proteins can have Synergistic Effects and/or Complementary Effects

Cytokines are powerful modulators of the anti-tumor immune response. Cytokine combinations are known to have profound synergistic effects on different immune compartments involving T-cells, NK cells, and myeloid cells (including dendritic cells and macrophages). Cytokines are known to play major roles in antigen priming by dendritic cells, survival and proliferation of innate immune cells and antigen-specific T-cells, and the cytotoxic activity of NK and T-cells. Cytokine combinations must be properly chosen to maximize biological responses and enhance anti-tumor immunity. For example, in a murine model of hepatitis, IFN-α alone was found to enhance the CD8⁺ T-cell cytolytic function of virally infected cells, while IL-15 alone enhanced the proliferation of activated lymphocytes. Together, they maximally suppressed hepatitis B (HBV) infection (see *e.g.,* Di Scala et al. (2016) J. Virol. 90(19):8563-8574). In another example, combinations of the cytokines IL-15 + IL-18, and IL-15 + IL-21, were able to enhance the production of IFN-γ from human NK and T-cells (see, *e.g.,* Strengell et al. (2003) J. Immunol. 170(11):5464-5469). In another example, IL-2 + IL-18 synergized to enhance IFN-γ production and increase cytolytic function of CD4⁺ T-cells, CD8⁺ T cells, and NK lymphocytes (see, *e.g.,* Son et al. (2001) Cancer Res. 61(3):884-888). Additionally, IL-12 and IL-18 were found to synergize to promote antigen-CD3 T-cell ligation-independent production of IFN-γ from human T-cells (see, *e.g.,* Tominaga et al. (2000) Int. Immunol. 12(2):151-160). Combinations of cytokines are powerful enhancers of T-cell function, but the FDA-approved anti-cancer cytokines are too toxic to be dosed systemically and are thus rarely used, and combinations of systemically-administered cytokines only compound the toxicity (see, *e.g.,* Conlon et al. (2019) J. Interferon Cytokine Res. 39(1):6-21).

The immunostimulatory bacteria provided herein solve these problems. Provided are immunostimulatory bacteria containing plasmids encoding multiple therapeutic products, such as immunomodulatory proteins, that allow for tumor-specific delivery of cytokine combinations, and/or combinations with other therapeutic products, such as the inducers of type I interferon discussed herein, and others, including co-stimulatory molecules, chemokines, and antibodies and fragments thereof. These immunostimulatory bacteria achieve powerful and synergistic immuno-activation without the systemic toxicities and pharmacokinetic (PK) liabilities associated with direct IV administration of the cytokines and other therapeutic products.

Combinations of therapeutic products that can be encoded on the plasmids in the immunostimulatory bacteria provided herein include, but are not limited to, for example, two or more cytokines; one or more cytokines and an inducer of type I IFN (*e.g*., STING, IRF3, IRF7, MDA5, RIG-I, and constitutively active, GOF variants thereof), and/or a co-stimulatory molecule (*e*.*g*., 4-1BBL, 4-1BBLΔcyt, and other variants of 4-1BBL discussed herein); a TGF-β decoy receptor and one or more cytokines; a TGF-β decoy receptor and an inducer of type I IFN; a TGF-β decoy receptor, one or more cytokines, and/or an inducer of type I IFN, and/or a co-stimulatory molecule; an antibody (*e*.*g*., against an immune checkpoint, such as CTLA-4) and one or more cytokines; an antibody and an inducer of type I IFN; an antibody, one or more cytokines, and/or an inducer of type I IFN, and/or a co-stimulatory molecule; a co-stimulatory molecule agonist *(e.g.,* a CD40 agonist) and one or more cytokines; a co-stimulatory molecule agonist and an inducer of type I IFN; and a co-stimulatory molecule agonist, one or more cytokines, and/or an inducer of type I IFN, and/or a co-stimulatory molecule.

As discussed below, the multiple therapeutic product expression cassettes can include single promoter constructs and/or dual/multiple promoter constructs, as well as post-transcriptional regulatory elements, and other regulatory elements, such as enhancers, polyadenylation signals, terminators, signal peptides, etc. The nucleic acid sequences can be codon optimized to increase protein expression, and generally, are under control of a eukaryotic promoter. Particular constructs and details thereof are described elsewhere herein.

Among the immunostimulatory bacteria provided herein are those that contain plasmids encoding immunostimulatory proteins (*e*.*g*., cytokines, chemokines, co-stimulatory molecules), and/or gene products with gain-of-function mutations that increase immune responses in the tumor microenvironment (*e*.*g*., cytosolic DNA/RNA sensors that induce type I IFN), and/or antibodies and fragments thereof, and/or other therapeutic products that enhance the anti-tumor response, such as TGF-β and/or IL-6 decoy receptors, and/or TGF-β antagonizing polypeptides. These immunostimulatory bacteria that encode the cytokines, gain-of-function products/type I IFN pathway proteins, and/or chemokines, and/or co-stimulatory molecules, and/or antibodies and fragments thereof, such as single-chain antibodies, and other therapeutic products discussed herein, include the immunostimulatory bacteria that preferentially infiltrate the tumor microenvironment, tumors, and tumor-resident immune cells. The immunostimulatory bacteria also include those in which the genome is modified so that they induce less cell death in tumor-resident immune cells, whereby the immunostimulatory bacteria accumulate in tumor-resident myeloid cells, to achieve high level ectopic expression of multiplexed genetic payloads in the target cells, and deliver the therapeutic products/immunomodulatory proteins to the tumor microenvironment (TME), to stimulate the immune response against the tumor. In particular, the immunostimulatory bacteria provided herein include up to about 8 or 8 modifications as described herein, including, but not limited to, adenosine auxotrophy, *csgD⁻, pagP⁻, msbB⁻,* flagellin⁻ (*fliC⁻*/*fljB⁻*)*, purI⁻, ansB⁻, asd⁻,* and any other modifications described herein or known to improve targeting to, or accumulation in, the tumor microenvironment and/or tumor-resident myeloid cells, or to improve safety and tolerability (allowing for a higher dose), reduce the immunosuppressive cytokine profile, improve T-cell quality and function, limit replication in healthy tissues, eliminate biofilms, and improve the anti-tumor immune response, or to impart any of the desirable and advantageous properties discussed elsewhere herein.

The immunostimulatory bacteria further can encode other therapeutic products, such as a tumor antigen from the subject's tumor, to enhance the response against the particular tumor. Any of the immunostimulatory bacteria provided herein and described above and below can be modified to encode the therapeutic products, such as cytokines, chemokines, co-stimulatory molecules, and gain-of-function type I IFN pathway product(s). The therapeutic products are encoded on a plasmid under control of a promoter recognized by the host, and any other desired regulatory sequences recognized in a eukaryotic, such as a human, or other animal, or mammalian, subject. Generally, the nucleic acid encoding the product is under the control of an RNA polymerase II promoter. Additionally, any of the bacteria described herein for modification, such as any of the strains of *Salmonella, Shigella, E. coli, Bifidobacteriae, Rickettsia, Vibrio, Listeria, Klebsiella, Bordetella, Neisseria, Aeromonas, Francisella, Cholera, Corynebacterium, Citrobacter, Chlamydia, Haemophilus, Brucella, Mycobacterium, Mycoplasma, Legionella, Rhodococcus, Pseudomonas, Helicobacter, Bacillus,* and *Erysipelothrix,* or an attenuated strain thereof, or a modified strain thereof, can be modified by introducing a plasmid containing, or encoding on a plasmid in the bacteria, nucleic acid encoding the therapeutic product(s) under control of an RNA polymerase promoter recognized by the host. The therapeutic products are expressed in the infected subject's cells. The immunostimulatory bacteria include those that are modified, as described herein, to accumulate in, or to preferentially infect, tumors, the TME and/or tumor-resident myeloid cells. For example, immunostimulatory bacteria that encode gain-of-function products leading to the expression of, or the constitutive expression of, type I interferon (IFN), such as IFN-beta, and/or other therapeutic products as discussed herein, further are modified to have reduced ability or no ability to infect epithelial cells, but are able to infect phagocytic cells, including tumor-resident immune cells, and/or the immunostimulatory bacteria are modified so that they do not kill the infected phagocytic cells.

As described herein, genes involved in the SPI-1 pathway, and flagella, activate the inflammasome in phagocytic cells (immune cells), triggering pyroptosis. Knocking out SPI-1 genes and genes that encode flagella, decreases or eliminates pyroptosis of phagocytic cells, and also, eliminates infection of epithelial cells, resulting in increased infection of phagocytic cells. Provided are immunostimulatory bacteria that accumulate in phagocytic cells, particularly tumor-resident immune cells, such as, for example, myeloid-derived suppresser cells (MDSCs), tumor-associated macrophages (TAMs), and dendritic cells (DCs), in which they express the genetic payloads/therapeutic products encoded on plasmids that are controlled by eukaryotic promoters, such as those recognized by RNA polymerase II, and include other eukaryotic regulatory signals, as discussed herein. Expressed therapeutic products include those that evoke immune responses, such as through pathways that increase or induce type I interferons, which increase the host response in the tumor microenvironment. The immunostimulatory bacteria also can encode immunostimulatory proteins, such as IL-2 and/or other cytokines, and/or other immunostimulatory proteins and therapeutic products, as discussed herein, further enhancing the immune response in the tumor microenvironment.

The immunostimulatory bacteria can encode products, referred to as cytosolic DNA/RNA sensors, that evoke immune responses when exposed to nucleic acids, such as RNA, DNA, nucleotides, dinucleotides, cyclic nucleotides, cyclic dinucleotides, and other such molecules, in the cytosol of cells. The immunostimulatory bacteria herein, encode modified therapeutic products that constitutively evoke immune responses, and do not require the presence of the DNA/RNA in the cytosol. Exemplary of such are components of pathways that induce type I interferon expression. The therapeutic products contemplated herein include modified forms of these cytosolic DNA/RNA sensors, that have constitutive activity or increased activity (*i.e*., gain-of-function products), such that type I interferon(s) is/are expressed or produced in the absence of nucleotides, dinucleotides, cyclic nucleotides, cyclic dinucleotides, and other such ligands, in the cytosol of cells. Expression of these modified products in cells, particularly in tumor cells, including tumor-resident immune cells, leads to constitutive expression of type I interferons, including interferon-β, in the tumor microenvironment. Because the immunostimulatory bacteria that express these gain-of-function products accumulate in or preferentially infect tumor cells/the TME/tumor-resident immune cells, the therapeutic products are expressed in the tumor microenvironment, resulting in increased immune responses in the tumor microenvironment.

Exemplary gene products that can be encoded in the immunostimulatory bacteria and other vehicles, include, but are not limited to, proteins that sense or are involved in innate pathways that recognize cytosolic DNA/RNA and activate type I interferon production. Proteins involved in innate DNA/RNA recognition that activate type I interferon include, but are not limited to: STING, RIG-I, MDA5, IRF3, IRF7, TRIM56, RIP1/RIPK1, Sec5/EXOC2, TRAF2, TRAF3, TRAF6, STAT1, LGP2/DHX58, DDX3/DDX3X, DHX9/DDX9, DDX1, DDX21, DHX15/DDX15, DHX33/DDX33, DHX36/DDX36, DDX60, and SNRNP200. Gain-of-function mutations in any of these proteins that result in constitutive type I interferon expression are known, or can be identified, and the mutants can be delivered by the immunostimulatory bacteria to the tumor microenvironment, such as by infection of phagocytic cells, or by targeting and binding to tumor cells.

The gain-of-function mutations include those identified from individuals with disorders resulting from constitutive type I interferon expression. Exemplary of gain-of-function products are those that occur in subjects with interferonopathies. As noted above, mutations can be identified by screening, to generate gain-of-function products as well.

The nucleic acids encoding the therapeutic products further can be modified to improve properties for expression. Modifications include, for example, codon optimization to increase transcriptional efficiency in a mammalian, particularly human, subject, such as reduction of GC content or CpG dinucleotide content, removal of cryptic splicing sites, adding or removing (generally removing) CpG islands to improve expression in eukaryotic cells, and replacement of TATA box and/or terminal signals to increase transcriptional efficiency. Codons can be optimized for increasing translation efficiency by altering codon usage bias, decreasing GC content, decreasing mRNA secondary structure, removing premature PolyA sites, removing RNA instability motifs (ARE), reducing stable free energy of mRNA, modifying internal chi sites and ribosomal binding sites, and reducing RNA secondary structures. Additional modifications to improve expression, and to maintain or enhance bacterial fitness have been incorporated into the immunostimulatory bacteria. These are described in sections below, and detailed and exemplified in the working Examples below.

As described above, type I interferon induction pathways, mediated by host recognition of cytosolic nucleic acids, such as single-stranded and double-stranded RNA, cyclic di-nucleotides (CDNs), and other such forms of nucleic acids, induce type I IFN. There also are Toll-Like Receptor (TLR)-independent type I IFN pathways, mediated by host recognition of single-stranded (ss) and double-stranded (ds) RNA in the cytosol. These are sensed by RNA helicases, including retinoic acid-inducible gene I (RIG-I), melanoma differentiation-associated gene 5 (MDA5), and through IFN-β promoter stimulator 1 (IPS-1) adaptor protein-mediated phosphorylation of the IRF3 transcription factor, leading to induction of IFN-β (see, *e.g.,* Ireton and Gale (2011) Viruses 3(6):906-919). As discussed herein, proteins in these pathways can be modified, or can exist as variants, that result in constitutive expression of type I interferons (also referred to as interferon type 1), which include IFN-α and IFN-β. Exemplary of such proteins are the modified STING polypeptides provided herein, which include those with mutations that result in constitutive expression of the type I interferons so that the interferons are expressed in the absence of induction, and also, chimeric STING proteins, such as those in which the a C-terminal tail (CTT) portion is replaced with a CTT portion from a STING protein from a second species, wherein the STING protein of the second species has lower NF-κB signaling activity than the NF-κB signaling activity of human STING, and the TRAF6 binding site in the CTT optionally is deleted.

Therapy with the immunostimulatory bacteria provided herein can be combined with any other anti-cancer therapy, including checkpoint inhibitor therapies and, as discussed above and elsewhere herein, other cancer treatments and chemotherapy.

### 6. Immunostimulatory Bacteria that Deliver Combination Therapies

The immunostimulatory bacteria herein can be used to provide more than one therapeutic product, particularly those that are for anti-cancer therapy. In general, the products are complementary products to enhance and re-program the anti-tumor immune response. The immunostimulatory bacteria, by virtue of the genomic modifications described herein, particularly the combination of several or all of *asd⁻,* flagellin⁻ (*fliC⁻*/*fljB⁻*)*, pagP⁻, csgD⁻, purI⁻,* adenosine auxotrophy, *msbB⁻, ansB⁻,* and any other modifications described elsewhere herein or known to those of skill in the art, accumulate in the tumor microenvironment (TME) and infect tumor-resident immune cells (myeloid cells). The immunostimulatory bacteria contain plasmids, encoding complementary therapeutic products, under control of a promoter or promoters recognized by the host, and any other desired regulatory sequences recognized in a eukaryotic, such as a human, or other animal, or mammalian, subject, to effect expression of the encoded products, and, also, secretion of the products. The immunostimulatory bacteria accumulate in the TME, particularly in tumor-resident immune cells, including myeloid-derived suppressor cells (MDSCs), tumor-associated macrophages (TAMs), and dendritic cells (DCs), where the encoded therapeutic products are expressed and then secreted into the tumor microenvironment to achieve an anti-tumor effect. By appropriate combination of products, the anti-tumor effect can be enhanced by virtue of interactions of the various products with the host immune system.

As discussed elsewhere herein, the immunostimulatory bacteria, containing plasmids encoding therapeutic products, with a single promoter and open reading frame (ORF), can express two (or more) proteins through the use of viral internal ribosomal entry sites (IRES), which are cap-independent, or through translational read-through of 2A peptides (*e.g*., T2A, P2A, E2A, or F2A), and subsequent self-cleavage into equally expressed co-proteins. Alternatively, the genetic payloads/therapeutic products can be expressed using dual or multiple promoter constructs, where each protein is expressed under the control of a separate promoter. A combination of single and dual/multiple promoter constructs, to express three or more proteins, also can be included on the plasmids. Generally, the nucleic acids encoding the therapeutic products are under the control of RNA polymerase II promoters. For example, promoters include, but are not limited to EF-1α, CMV, SV40, UBC, CBA, PGK, GUSB, GAPDH, EIF41A, CAG, CD68, and synthetic MND promoters. The plasmids can contain other regulatory elements, such as post-transcriptional regulatory elements (PREs; *e*.*g*., WPRE, HPRE), polyadenylation signal sequences, terminators, enhancers, secretion signals (also known as signal peptides/sequences, leader peptides/sequences), DNA nuclear targeting sequences (DTS), and other regulatory elements, described elsewhere herein or known to those of skill in the art, that can enhance or increase the expression and/or secretion of the encoded therapeutic products.

The genetic payloads or therapeutic products encoded on the plasmids include immunostimulatory proteins, such as cytokines, chemokines, and co-stimulatory molecules; cytosolic DNA/RNA sensors that induce type I IFN and gain-of-function/constitutively active mutants/variants thereof; antibodies and fragments thereof; bi-specific T-cell engagers (BiTEs^{®}); soluble TGF-β receptors that act as decoys for binding TGF-β, or TGF-β antagonizing polypeptides; IL-6 binding decoy receptors; interfering RNAs (*e*.*g*., siRNA, shRNA, miRNA); and other therapeutic products as discussed below and elsewhere herein, and as known in the art; and complementary combinations of all of the preceding therapeutic products. In some embodiments, the cytokines can be encoded on the plasmid within the immunostimulatory bacteria, with a membrane anchoring motif, such as a transmembrane domain, and a collagen-binding domain.

The immunostimulatory proteins, including cytokines, chemokines, and co-stimulatory molecules, that can be encoded on the plasmids include, but are not limited to, IL-2, IL-7, IL-12p70 (IL-12p40 + IL-12p35), IL-15, IL-15/IL-15Rα chain complex, IL-18, IL-21, IL-23, IL-36 gamma, interferon-α, interferon-β, IL-2 that has attenuated binding to IL-2Ra, IL-2 that is modified so that it does not bind to IL-2Ra, CXCL9, CXCL10, CXCL11, CCL3, CCL4, CCL5, proteins that are involved in or that effect or potentiate the recruitment and/or persistence of T-cells, CD40, CD40 Ligand (CD40L), OX40, OX40 Ligand (OX40L), 4-1BB, 4-1BB Ligand (4-1BBL), 4-1BBL with a deletion in the cytoplasmic domain (4-1BBLΔcyt), ICOS, CD27, members of the B7-CD28 family, and members of the tumor necrosis factor receptor (TNFR) superfamily. The immunostimulatory proteins also include truncated co-stimulatory molecules, such as, for example, 4-1BBL, CD80, CD86, CD27L, B7RP1 and OX40L, with a full-length cytoplasmic domain, or with a truncated, or partial, or partial with modifications to ensure proper orientation, cytoplasmic domain deletion, for expression on an antigen-presenting cell (APC), where the truncated gene product is capable of constitutive immunostimulatory signaling to a T-cell through co-stimulatory receptor engagement, and is unable to counter-regulatory signal to the APC due to a deleted cytoplasmic domain.

The cytosolic DNA/RNA sensors, that induce or activate type I IFN production include, but are not limited to, STING, RIG-I, MDA5, IRF3, IRF5, and IRF7, and gain-of-function (GOF) or constitutively active variants thereof. Other proteins involved in the recognition of DNA/RNA that activate type I IFN responses, that can be mutated to generate constitutive type I IFN expression and can be encoded on the plasmids, include, but are not limited to, TRIM56, RIP1, Sec5, TRAF2, TRAF3, TRAF6, STAT1, LGP2, DDX3, DHX9, DDX1, DDX21, DHX15, DHX33, DHX36, DDX60, and SNRNP200.

Other therapeutic products that can be encoded on the plasmids delivered by the immunostimulatory bacteria herein, or that can be co-administered with the bacteria, that enhance or increase the anti-tumor response, include, but are not limited to, antibodies and fragments thereof, for example, TGF-β inhibitory antibodies; anti-IL-6 antibodies; antibodies against checkpoint inhibitors, such as PD-1, PD-L1, and CTLA-4; and antibodies against, or inhibitors of, VEGF, CD73, CD38, Siglec-15, EGFR, Her2, Mesothelin, and BCMA. Also contemplated for expression on the plasmid, or for co-administration with the immunostimulatory bacteria herein, are bispecific T-cell engagers (BiTEs^{®}), IL-6 binding decoy receptors, TGF-beta binding decoy receptors, and TGF-beta polypeptide antagonists. Any of these antibodies, inhibitors, or decoy receptors can be co-administered with the immunostimulatory bacteria herein. In some embodiments, PARP (poly (ADP)-ribose polymerase) inhibitors, histone deacetylase (HDAC) inhibitors and/or chemotherapy, also can be co-administered with any of the therapeutic products listed above, alone or in any combination.

Exemplary of complementary combinations of therapeutic products that can be encoded on the plasmids in the immunostimulatory bacteria herein include, but are not limited to:
IL-2 and IL-12p70; IL-2 and IL-21; IL-2, IL-12p70, and a STING GOF variant; IL-2, IL-21, and a STING GOF variant; IL-2, IL-12p70, a STING GOF variant, and 4-1BBL (including 4-1BBLΔcyt); and IL-2, IL-21, a STING GOF variant, and 4-1BBL (including 4-1BBLΔcyt);
IL-15/IL-15Rα and a STING GOF variant; IL-15/IL-15Rα, a STING GOF variant, and 4-1BBL (including 4-1BBLΔcyt); IL-15/IL-15Rα and IL-12p70; IL-15/IL-15Rα and IL-21; IL-15/IL-15Rα, IL-12p70, and a STING GOF variant; IL-15/IL-15Rα, IL-21, and a STING GOF variant; IL-15/IL-15Rα, IL-12p70, a STING GOF variant, and 4-1BBL (including 4-1BBLΔcyt); and IL-15/IL-15Rα, IL-21, a STING GOF variant, and 4-1BBL (including 4-1BBLΔcyt);
IL-12p70 and IL-21; IL-12p70, IL-21, and a STING GOF variant; IL-12p70, IL-21, a STING GOF variant, and 4-1BBL (including 4-1BBLΔcyt); IL-12p70 and a STING GOF variant; IL-12p70, a STING GOF variant, and 4-1BBL (including 4-1BBLΔcyt); IL-12p70 and IL-18; IL-12p70, IL-18, and a STING GOF variant; and IL-12p70, IL-18, a STING GOF variant, and 4-1BBL (including 4-1BBLΔcyt);
a TGF-β decoy receptor, IL-2, and IL-12p70; a TGF-β decoy receptor, IL-2, and IL-21; a TGF-β decoy receptor, IL-2, IL-12p70, and a STING GOF variant; a TGF-β decoy receptor, IL-2, IL-21, and a STING GOF variant; a TGF-β decoy receptor, IL-2, IL-12p70, a STING GOF variant, and 4-1BBL (including 4-1BBLΔcyt); and a TGF-β decoy receptor, IL-2, IL-21, a STING GOF variant, and 4-1BBL (including 4-1BBLΔcyt);
a TGF-β decoy receptor, IL-15/IL-15Rα, and a STING GOF variant; a TGF-β decoy receptor, IL-15/IL-15Rα, a STING GOF variant, and 4-1BBL (including 4-1BBLΔcyt); a TGF-β decoy receptor, IL-15/IL-15Rα, and IL-12p70; a TGF-β decoy receptor, IL-15/IL-15Rα, and IL-21; a TGF-β decoy receptor, IL-15/IL-15Rα, IL-12p70, and a STING GOF variant; a TGF-β decoy receptor, IL-15/IL-15Rα, IL-21, and a STING GOF variant; a TGF-β decoy receptor, IL-15/IL-15Rα, IL-12p70, a STING GOF variant, and 4-1BBL (including 4-1BBLΔcyt); and a TGF-β decoy receptor, IL-15/IL-15Rα, IL-21, a STING GOF variant, and 4-1BBL (including 4-1BBLΔcyt);
a TGF-β decoy receptor, IL-12p70, and IL-21; a TGF-β decoy receptor, IL-12p70, IL-21, and a STING GOF variant; a TGF-β decoy receptor, IL-12p70, IL-21, a STING GOF variant, and 4-1BBL (including 4-1BBLΔcyt); a TGF-β decoy receptor and IL-12p70; a TGF-β decoy receptor, IL-12p70, and a STING GOF variant; a TGF-β decoy receptor, IL-12p70, a STING GOF variant, and 4-1BBL (including 4-1BBLΔcyt); a TGF-β decoy receptor, IL-12p70, and IL-18; a TGF-β decoy receptor, IL-12p70, IL-18, and a STING GOF variant; a TGF-β decoy receptor, IL-12p70, IL-18, a STING GOF variant, and 4-1BBL (including 4-1BBLΔcyt); and a TGF-β decoy receptor and a STING GOF variant;
an anti-CTLA-4 antibody, IL-2, and IL-12p70; an anti-CTLA-4 antibody, IL-2, and IL-21; an anti-CTLA-4 antibody, IL-2, IL-12p70, and a STING GOF variant; an anti-CTLA-4 antibody, IL-2, IL-21, and a STING GOF variant; an anti-CTLA-4 antibody, IL-2, IL-12p70, a STING GOF variant, and 4-1BBL (including 4-1BBLΔcyt); and an anti-CTLA-4 antibody, IL-2, IL-21, a STING GOF variant, and 4-1BBL (including 4-1BBLΔcyt);
an anti-CTLA-4 antibody, IL-15/IL-15Rα, and a STING GOF variant; an anti-CTLA-4 antibody, IL-15/IL-15Rα, a STING GOF variant, and 4-1BBL (including 4-1BBLΔcyt); an anti-CTLA-4 antibody, IL-15/IL-15Rα, and IL-12p70; an anti-CTLA-4 antibody, IL-15/IL-15Rα, and IL-21; an anti-CTLA-4 antibody, IL-15/IL-15Rα, IL-12p70, and a STING GOF variant; an anti-CTLA-4 antibody, IL-15/IL-15Rα, IL-21, and a STING GOF variant; an anti-CTLA-4 antibody, IL-15/IL-15Rα, IL-12p70, a STING GOF variant, and 4-1BBL (including 4-1BBLΔcyt); and an anti-CTLA-4 antibody, IL-15/IL-15Rα, IL-21, a STING GOF variant, and 4-1BBL (including 4-1BBLΔcyt);
an anti-CTLA-4 antibody, IL-12p70, and IL-21; an anti-CTLA-4 antibody, IL-12p70, IL-21, and a STING GOF variant; an anti-CTLA-4 antibody, IL-12p70, IL-21, a STING GOF variant, and 4-1BBL (including 4-1BBLΔcyt); an anti-CTLA-4 antibody and IL-12p70; an anti-CTLA-4 antibody, IL-12p70, and a STING GOF variant; an anti-CTLA-4 antibody, IL-12p70, a STING GOF variant, and 4-1BBL (including 4-1BBLΔcyt); an anti-CTLA-4 antibody, IL-12p70, and IL-18; an anti-CTLA-4 antibody, IL-12p70, IL-18, and a STING GOF variant; an anti-CTLA-4 antibody, IL-12p70, IL-18, a STING GOF variant, and 4-1BBL (including 4-1BBLΔcyt); and an anti-CTLA-4 antibody and a STING GOF variant;
a CD40 agonist, IL-2, and IL-12p70; a CD40 agonist, IL-2, and IL-21; a CD40 agonist, IL-2, IL-12p70, and a STING GOF variant; a CD40 agonist, IL-2, IL-21, and a STING GOF variant; a CD40 agonist, IL-2, IL-12p70, a STING GOF variant, and 4-1BBL (including 4-1BBLΔcyt); and a CD40 agonist, IL-2, IL-21, a STING GOF variant, and 4-1BBL (including 4-1BBLΔcyt);
a CD40 agonist, IL-15/IL-15Rα, and a STING GOF variant; a CD40 agonist, IL-15/IL-15Rα, a STING GOF variant, and 4-1BBL (including 4-1BBLΔcyt); a CD40 agonist, IL-15/IL-15Rα, and IL-12p70; a CD40 agonist, IL-15/IL-15Rα, and IL-21; a CD40 agonist, IL-15/IL-15Rα, IL-12p70, and a STING GOF variant; a CD40 agonist, IL-15/IL-15Rα, IL-21, and a STING GOF variant; a CD40 agonist, IL-15/IL-15Rα, IL-12p70, a STING GOF variant, and 4-1BBL (including 4-1BBLΔcyt); and a CD40 agonist, IL-15/IL-15Rα, IL-21, a STING GOF variant, and 4-1BBL (including 4-1BBLΔcyt); and
a CD40 agonist, IL-12p70, and IL-21; a CD40 agonist, IL-12p70, IL-21, and a STING GOF variant; a CD40 agonist, IL-12p70, IL-21, a STING GOF variant, and 4-1BBL (including 4-1BBLΔcyt); a CD40 agonist and IL-12p70; a CD40 agonist, IL-12p70, and a STING GOF variant; a CD40 agonist, IL-12p70, a STING GOF variant, and 4-1BBL (including 4-1BBLΔcyt); a CD40 agonist, IL-12p70, and IL-18; a CD40 agonist, IL-12p70, IL-18, and a STING GOF variant; a CD40 agonist, IL-12p70, IL-18, a STING GOF variant, and 4-1BBL (including 4-1BBLΔcyt); and a CD40 agonist and a STING GOF variant.

In all combinations including 4-1BBL, the 4-1BBL molecule can be a full-length protein (see, *e.g.,* SEQ ID NOs:389 and 393, for human and mouse 4-1BBL, respectively); a 4-1BBL variant with the cytoplasmic domain deleted (4-1BBLΔcyt; see *e.g.,* SEQ ID NOs:390 and 394, for human and murine 4-1BBLΔcyt, respectively); a 4-1BBL variant with a truncated (*i.e*., not fully deleted) cytoplasmic domain (4-1BBLcyt trunc; see, *e.g.,* SEQ ID NOs:391-392 and SEQ ID NOs:395-396, for exemplary human and mouse 4-1BBLcyt trunc variants); or a 4-1BBL molecule with a modified cytoplasmic domain, in which one or more Ser residues, which act as phosphorylation sites, are replaced at an appropriate locus or loci, such as, for human 4-1BBL, with reference to SEQ ID NO:389, Ser5 and Ser8, with a residue that reduces or eliminates reverse signaling. Additionally, all combinations including an anti-CTLA-4 antibody, can include an anti-CTLA-4 antibody fragment, such as an anti-CTLA-4 scFv (see, *e.g.,* SEQ ID NOs:403 and 404, for exemplary human and mouse anti-CTLA-4 scFv fragments, respectively), or an anti-CTLA-4 scFv-Fc (see, *e.g.,* SEQ ID NOs:402 and 405, for exemplary human and mouse anti-CTLA-4 scFv-Fc fragments, respectively). Additionally, a TGF-β receptor decoy can be replaced by other TGF-beta polypeptide antagonists, that can bind and remove TGF-β from the tumor microenvironment, including, for example, anti-TGF-beta antibodies or antibody fragments, anti-TGF-beta receptor antibodies or antibody fragments, and soluble TGF-beta antagonist polypeptides.

The following table lists exemplary products that can be encoded in plasmids in the immunostimulatory bacteria, and some effects/characteristics of such products.

| **Encoded Product** | **Effects/Characteristics** |
|---|---|
| Engineered STING - to increase Type I IFN expression | Chemokine gradients recruit T-cells. |
| | Induction of Type I IFN, T-cell activation, APC tumor antigen cross-presentation. |
| | Constitutive STING variants validated in SAVI patients with Type I Interferonopathies. |
| IL-12 | Strong Th1 immune response driver. |
| | Produced by activated APCs, particularly M1 Macrophages and DCs* |
| | Data indicate that it is a strong driver of IFN-γ |
| IL-15 | Stimulates T-cell and NK cell proliferation. |
| | Lacks T_{reg} stimulation seen with IL-2. |
| IL-21 | Pleiotropic cytokine that stimulates T-cells and NK cells. |
| | Converts M2 macrophages to M1 macrophages. |
| | Produced upon TLR3 activation (RNA viral sensing). |
| IL-36γ | Promotes Th1 APCs and IFN-γ production by T-cells, NK cells and γδT-cells. |
| Modified 4-1BBL | Provides T-cell co-stimulation, improves cytotoxicity and memory phenotype of T-cells; modified with deletion or truncation of the cytoplasmic domain, and addition of extra positive residues to the N-terminal truncated cytoplasmic domain. |
| Anti-CTLA-4 scFV-Fc | Enhances T-cell priming and activation against weaker tumor antigens. |
| TGF-β Decoy Receptor Trap | Binds and removes TGF-beta, relieves immunosuppression of T-cells and Th1 APCs. |

| | |
|---|---|
| *DC = Dendritic Cells | |

In any of the complementary combinations above, a TGF-β decoy receptor can be replaced with a TGF-β antagonizing polypeptide. As discussed above, TGF-β decoy receptors are any that act as decoys for binding TGF-β to remove it, or are TGF-β antagonizing polypeptides (e.g., anti-TGF-beta antibodies or antibody fragments, and anti-TGF-beta receptor antibodies or antibody fragments). The STING protein, or other DNA/RNA sensor that induces or activates type I IFN production, can be a GOF/constitutively active variant, or can be the wild-type protein, including the modified STING polypeptides and chimeric STING polypeptides described and provided herein. Any of the complementary combinations above also can be administered in combination with any one or more of: an anti-PD-1 antibody, an anti-CTLA-4 antibody, an anti-PD-L1 antibody, an anti-IL-6 antibody, an anti-Siglec-15 antibody, an anti-VEGF antibody, an anti-CD73 antibody, an anti-CD38 antibody, an anti-EGFR antibody, an anti-Her2 antibody, an anti-Mesothelin antibody, an anti-BCMA antibody, and antibody fragments thereof, as well as PARP inhibitors, HDAC inhibitors, or chemotherapy, and combinations thereof.

The plasmids and immunostimulatory bacteria provided herein encode combinations of therapeutic payloads. These include combinations of nucleic acid encoding any or all of the products listed in the table above.

Combinations of complementary payloads were assessed, and exemplary combinations and their effects are described in the Examples. The effects of various combinations of payloads on the activation of antigen-specific T-cells, and on the secretion of CXCL10 by myeloid cells, a key chemokine involved in the recruitment of anti-tumor T-cells, were assessed. For example, combinations of the payloads can induce a strong secretion of CXCL10 by bone marrow dendritic cells (BMDCs). Combining IL-36γ with IL-12p70 and STING R284G tazCTT led to higher secretion of CXCL10 and IFN-γ by BMDCs (see, Example 26). Many of the combinations induce the activation of CD8⁺ T-cell responses (*e.g.,* 4-1BB expression), and the secretion of IFN-γ. The results in the working Examples (see, Example 26) show that particular cytokine combinations can activate T-cells. For example, the combinations of IL-12p70 + IL-15; IL-12p70 + IL-15 + IFN-α2; IL-12p70 + IL-15 + anti-4-1BB agonistic antibody; IL-12p70 + IL-15 + IL-36γ; IL-12p70 + IL-15 + IL-21; IL-12p70 + IL-21 + IL-36γ; IL-12p70 + IL-36γ + IFN-α2; IL-12p70 + IL-36γ + anti-4-1BB agonistic antibody; IL-15 + IL-36γ + IFN-α2; and IL-15 + IL-36γ + anti-4-1BB agonistic antibody, result in the secretion of high levels of IFN-γ, but relatively low levels of IL-6, from T-cells, making them ideal combinations for optimal T-cell activation, for the induction of anti-tumor immunity in the tumor microenvironment.

Additionally, several combinations of cytokines (IL-12p70, IL-15, IL-21, and IL-36γ) and 4-1BB engagement, activate T-cells to secrete high levels of IFN-γ, with and without TCR stimulation by an anti-CD3ε agonistic antibody, for CD4⁺ and CD8⁺ T-cells. STING variants described herein as well as IL-12 can increase antigen specific activation of human CD8⁺ T-cells. Data (see, Example 24) also showed, in a mouse (mu) model of colorectal carcinoma, that immunostimulatory bacterial strains, expressing IL-15, or the combination of 4-1BBLΔcyt + IL-12 more potently inhibit tumor growth inhibition that the same strains expressing 4-1BBL(Δcyt) or IL-12 alone, and result in a high complete response rate (50% cure rate). Other combinations also were tested and shown to have potent anti-tumor activity *in vivo.*

Combinations of payloads can include a co-stimulatory molecule, such as an OX40L polypeptide, or a 4-1BBL polypeptide, or one of the cytoplasmic deleted or truncated variants thereof, and/or the modified forms thereof described and exemplified herein; or an anti-immune checkpoint antibody or fragment thereof, such as an anti-CTLA-4 scFv-Fc or an anti-CTLA-4 scFv (see, Example 20 and SEQ ID NOs:402 and 403, respectively); one or more cytokines/chemokines, such as IL-12, IL-15, IL-18, IL-21, IL-23, IL-36γ, IFN-β, IFN-α2, and CXCL10; TGF-β binding decoy receptors and other TGF-beta polypeptide antagonists, such as, for example, a human soluble TGFβ receptor II fused with a human IgG1 Fc (hu sTGFβRII-Fc; SEQ ID NO:407), anti-TGF-beta antibodies or antibody fragments, anti-TGF-beta receptor antibodies or antibody fragments, and soluble TGF-beta antagonist polypeptides; and one or more of a STING protein or a modified and/or chimeric STING protein, as described and exemplified herein.

As discussed herein, these payloads/products/polypeptides can be encoded as a polycistronic construct, under the control of a single promoter (*i.e*., a single promoter system) and, as required, other regulatory sequences, and also can include 2A polypeptides or other such polypeptides that result in the translation of individual products. The payloads also can be expressed on plasmids containing two separate open reading frames (ORFs), each under the control of a different promoter (*i.e*., *a* dual promoter system). Exemplary combinations of payloads, in the order they are encoded on a plasmid, and including the 2A peptide that is encoded in the polycistronic construct, are set forth in the following table.

### Exemplary Combinations of Products and Exemplary Order on the Plasmids

| **1^{st} Encoded Product** | **1^{st} 2A Peptide** | **2^{nd} Encoded Product** | **2^{nd} 2A Peptide** | **3^{rd} Encoded Product** | **3^{rd} 2A Peptide** | **4^{th} Encoded Product** |
|---|---|---|---|---|---|---|
| 4-1BBL* | T2A | IL-12p70 | | | | |
| 4-1BBL* | T2A | Chimeric STING** | | | | |
| 4-1BBL* | T2A | IL-12p70 | P2A | Chimeric STING** | | |
| IL-12p70 | T2A | Chimeric STING** | | | | |
| IL-12p70 | T2A | IL-15 | | | | |
| IL-12p70 | T2A | IL-21 | | | | |
| IL-12p70 | T2A | IL-15 | | | | |
| IL-12p70 | T2A | IL-21 | | | | |
| IL-21 | T2A | IL-12p70 | | | | |
| IL-12p70 | T2A | IL-36γ | | | | |
| IL-36γ | T2A | IL-12p70 | | | | |
| 4-1BBL* | T2A | IL-12p70 | P2A | IL-15 | | |
| 4-1BBL* | T2A | IL-12p70 | P2A | IL-21 | | |
| 4-1BBL* | T2A | IL-12p70 | P2A | IL-36γ | | |
| 4-1BBL* | T2A | IL-12p70 | P2A | IL-15 | T2A | Chimeric STING** |
| 4-1BBL* | T2A | IL-12p70 | P2A | IL-21 | T2A | Chimeric STING** |
| 4-1BBL* | T2A | IL-12p70 | P2A | IL-36γ | T2A | Chimeric STING** |
| IL-12p70 | T2A | IL-21 | P2A | Chimeric STING** | | |
| IL-21 | T2A | IL-12p70 | P2A | Chimeric STING** | | |
| IL-12p70 | T2A | IL-15 | P2A | Chimeric STING** | | |
| IL-12p70 | T2A | IL-36γ | P2A | Chimeric STING** | | |
| IL-36γ | T2A | IL-12p70 | P2A | Chimeric STING** | | |
| Anti-CTLA-4 scFv-Fc | T2A | IL-12p70 | | | | |
| Anti-CTLA-4 scFv-Fc | T2A | IL-12p70 | P2A | Chimeric STING** | | |
| Anti-CTLA-4 scFv-Fc | T2A | IL-12p70 | P2A | IL-15 | T2A | |
| Anti-CTLA-4 scFv-Fc | T2A | IL-12p70 | P2A | IL-21 | T2A | |
| Anti-CTLA-4 scFv-Fc | T2A | IL-12p70 | P2A | IL-36γ | T2A | |
| 4-1BBL* | T2A | sTGFβRIIFc^{#} | | | T2A | |
| sTGFβRIIFc^{#} | T2A | IL-12p70 | P2A | Chimeric STING** | | |
| 4-1BBL* | T2A | sTGFβRIIFc^{#} | P2A | IL-12p70 | T2A | |
| 4-1BBL* | T2A | IL-12p70 | P2A | sTGFβRIIFc^{#} | T2A | |
| IL-36γ | T2A | IL-23 | P2A | OX40L | | |

| | | | | | | |
|---|---|---|---|---|---|---|
| *4-1BBL = the modified 4-1BBL with the cytoplasmic truncation and residue modifications to render the remaining cytoplasmic domain more positive to retain correct orientation with respect to the cell membrane. **Chimeric STING = STING with the Tasmanian Devil CTT and the replacements R284G/N154S. sTGFβRIIFc^{#} = type II receptor betaglycan. | | | | | | |

The properties of the immunostimulatory bacteria provided herein, such as the accumulation in tumor-resident myeloid cells, and in the TME, and the combinations of products/payloads that can be expressed, can be selected to cover the cancer immunity cycle. Each step in the cycle, and the role of the immunostimulatory bacteria and payloads is summarized as follows:
1) Release of cancer cell antigens - the immunostimulatory bacteria accumulate in the tumor-resident myeloid cells;
2) Cancer antigen presentation - the immunostimulatory bacteria provided herein encode and express immune stimulators, such as the STING polypeptides and variants thereof, and IL-12, leading to the expression of type I interferons, including IFN-α and IFN-β;
3) Priming and activation - the immunostimulatory bacteria encode the STING polypeptides and variants thereof, and the co-stimulatory proteins, such as 4-1BBL, and IL-12;
4) Trafficking of the T-cells to the tumor - the encoded STING variants are expressed, leading to the consequent expression of IFN-α and IFN-β;
5) Infiltration of T-cells into the tumor - vascular leakage and repolarization of immunosuppressive myeloid cells;
6) Cancer cell recognition by T-cells - Type I IFN, and IFNγ, and upregulation of MHC; and
7) Killing of cancers cells - the combination of encoded cytokines/chemokines, such as IL-12, IL-15, IL-21, and/or IL-36γ, which induce T-cell proliferation and release of IFN-γ, and the expression of a soluble TGF-β decoy receptor.

A skilled person, based on the disclosure herein and their knowledge, can identify other product payload combinations and other orders of the products as encoded on a polycistronic construct, that have immune-activating and/or immune suppressing effects, to enhance the anti-tumor activities of the immunostimulatory bacteria provided herein.

### E. CONSTRUCTING EXEMPLARY PLASMIDS ENCODING THERAPEUTIC PRODUCTS FOR BACTERIAL DELIVERY

The immunostimulatory bacteria herein can be modified to encode one or more therapeutic products, including immunomodulatory proteins, that promote, or induce, or enhance an anti-tumor response. The therapeutic product can be encoded on a plasmid in the bacterium, under the control of a eukaryotic promoter, such as a promoter recognized by RNA polymerase II, for expression in a eukaryotic subject, particularly the subject for whom the immunostimulatory bacterium is to be administered, such as a human. The nucleic acid encoding the therapeutic product(s) can include, in addition to the eukaryotic promoter, other regulatory signals for expression or trafficking in the cells, such as for secretion or expression on the surface of a cell. Immunostimulatory proteins are those that, in the appropriate environment, such as a tumor microenvironment (TME), can promote, or participate in, or enhance an anti-tumor response in the subject to whom the immunostimulatory bacterium is administered. Immunostimulatory proteins include, but are not limited to, cytokines, chemokines, and co-stimulatory molecules. These include cytokines, such as, but not limited to, IL-2, IL-7, IL-12, IL-12p70 (IL-12p40 + IL-12p35), IL-15, IL-15/IL-15Rα chain complex, IL-18, IL-21, IL-23, IL-36γ, IL-2 that has attenuated binding to IL-2Ra, IL-2 that is modified so that it does not bind to IL-2Ra, IFN-α, and IFN-β; chemokines, such as, but not limited to, CCL3, CCL4, CCL5, CXCL9, CXCL10, and CXCL11; proteins that are involved in, or that effect or potentiate the recruitment and/or persistence of T-cells; and/or co-stimulatory molecules, such as, but not limited to, CD40, CD40L, OX40, OX40L, 4-1BB, 4-1BBL, 4-1BBL with a deletion of the cytoplasmic domain (4-1BBLΔcyt), 4-1BBL with a truncated cytoplasmic domain or otherwise modified truncated cytoplasmic domain, ICOS, ICOS ligand, CD27, CD27 ligand, CD80, CD86, members of the TNF/TNFR superfamily, and members of the B7-CD28 family. Other such immunostimulatory proteins that are used for treatment of tumors or that can promote, enhance, or otherwise increase or evoke an anti-tumor response, known to those of skill in the art, are contemplated for encoding in the immunostimulatory bacteria provided herein.

Other therapeutic products, encoded by the immunostimulatory bacteria herein, include cytosolic DNA/RNA sensors that induce or activate type I interferon production, including STING, MDA5, RIG-I, IRF3, and IRF7, as well as gain-of-function and constitutively active variants thereof. For example, the constitutively active STING variants include those with the mutations V147L, N154S, V155M, C206Y, R281Q, and/or R284G, such as N154S/R284G, and others described herein and known in the art, while the constitutively active IRF3 variants include those with the mutations S396D, S398D, S402D, T404D, and/or S405D, and others described herein and known in the art. Other therapeutic products, encoded by the immunostimulatory bacteria herein, include antibodies and antibody fragments, including single chain fragment variables (scFvs), Fab fragments, Fab' fragments, F(ab')₂ fragments, Fv fragments, disulfide-linked Fvs (dsFvs), Fd fragments, Fd' fragments, single-chain Fabs (scFabs), diabodies, anti-idiotypic (anti-Id) antibodies, synthetic antibodies, recombinantly produced antibodies, multi-specific antibodies (e.g., bi-specific antibodies), human antibodies, non-human antibodies, humanized antibodies, chimeric antibodies, and intrabodies, or antigen-binding fragments of any of the above. The antibodies can be directed against immune checkpoints, such as PD-1, PD-L1, CTLA-4, IDO 1 and 2, CTNNB1 (β-catenin), SIRPα, VISTA, and TREX-1, and others known in the art or described herein, or against other targets such as TGF-β, VEGF, HER2, EGFR, STAT3, and IL-6, and other such targets whose inhibition improves the anti-tumor response. The immunostimulatory bacteria also can encode RNAi, such as siRNA (shRNA and miRNA) against immune checkpoints, such as TREX1, and other targets whose inhibition, suppression, or disruption improves the anti-tumor response.

In some embodiments, the immunostimulatory bacteria herein are engineered to encode and express one or more cytokines to stimulate the immune system, including, but not limited to, IL-2, IL-7, IL-12 (IL-12p70 (IL-12p40 + IL-12p35)), IL-15 (and the IL-15:IL-15R alpha chain complex), IL-18, IL-21, IL-23, IL-36 gamma, IFN-alpha, and IFN-beta. Cytokines stimulate immune effector cells and stromal cells at the tumor site, and enhance tumor cell recognition by cytotoxic cells. In some embodiments, the immunostimulatory bacteria can be engineered to encode and express chemokines, such as, for example, one or more of CCL3, CCL4, CCL5, CXCL9, CXCL10 and CXCL11. Complementary combinations of any of the therapeutic products can be encoded and delivered to the tumor microenvironment, to enhance the anti-tumor efficacy of the immunostimulatory bacteria. These modifications, and the immunostimulatory bacteria encoding them, are discussed above, and exemplified below.

### 1. Constitutive Promoters for Heterologous Expression of Proteins

Plasmids provided herein are designed to encode a therapeutic product, such as an immunostimulatory protein, that, when expressed in a mammalian subject, confers or contributes to anti-tumor immunity in the tumor microenvironment; the immunostimulatory protein or other therapeutic product is encoded on a plasmid in the bacterium under control of a eukaryotic promoter, such as a promoter that is recognized by RNA polymerase II (RNAP II). Generally the promoter is a constitutive promoter, such as a late eukaryotic virus promoter. Exemplary promoters include, but are not limited to, a cytomegalovirus (CMV) promoter, an elongation factor-1 alpha (EF-1α) promoter, a ubiquitin C (UBC) promoter, a simian virus 40 (SV40) early promoter, a phosphoglycerate kinase 1 (PGK) promoter, a chicken β-actin (CBA) promoter and its derivative promoters CAGG or CAG, a β-glucuronidase (GUSB) promoter, the MND promoter (a synthetic promoter that contains the U3 region of a modified MoMuLV (Moloney murine leukemia virus) LTR with myeloproliferative sarcoma virus enhancer and deleted negative control region), a eukaryotic initiation factor 4A-I (EIF4A1) promoter, a CD68 promoter, and a GAPDH promoter, among others (see, *e.g.,* Powell et al. (2015) Discov. Med. 19(102):49-57). The CAG promoter consists of: (C) the cytomegalovirus (CMV) early enhancer element; (A) the promoter, the first exon, and the first intron of chicken beta-actin gene; and (G) the splice acceptor of the rabbit beta-globin gene. MND is a synthetic promoter that contains the U3 region of a modified MoMuLV (Moloney murine leukemia virus) LTR with myeloproliferative sarcoma virus enhancer and deleted negative control region (murine leukemia virus-derived MND promoter (myeloproliferative sarcoma virus enhancer, negative control region deleted, dl587rev primer-binding site substituted); see, *e.g.,* Li et al. (2010) J. Neurosci. Methods 189:56-64*).*

Two or more of these promoters can be encoded in multiple open reading frames (ORFs) on the plasmid. Certain promoters, including, but not limited to, CMV, contain multiple cAMP response element binding protein (CREB) sites. When plasmids containing these elements are released to the cytosol, for example those contained within *S. typhimurium* that are released into the cytosol following bacterial destruction, they can be efficiently shuttled to the nucleus using the CREB-mediated host microtubule machinery (see, *e.g.,* Bai et al. (2017) Biosci. Rep. 37(6):BSR20160616).

The plasmids can include multiple promoters, including bacterial promoters, such as for expression of *asd,* and eukaryotic promoters for expression of therapeutic products. Various configurations of the promoters and other regulatory sequences have been assessed to improve expression of therapeutic products and to improve bacterial growth and fitness. As shown in the examples below (see, Example 30), among the configurations tested, reversing the orientation of the eukaryotic expression cassette on the plasmid, and inclusion of one or more bacterial terminators, can increase the efficiency of encoded payload expression and can improve bacterial fitness.

### 2. Multiple Therapeutic Product Expression Cassettes

### a. Single Promoter Constructs

Expression of multiple genes in the same cell from a single construct can be achieved, and is advantageous when the co-expression of several proteins is required to elicit a desired biological effect, such as an anti-tumor response. Internal ribosome entry site (IRES) sequences have been used to separate two coding sequences under control of a single promoter, however, the expression level of the second protein can be reduced compared to the first protein, and the length of the IRES sequence can be prohibitive in certain cases, such as when using viruses with small packaging capacities. The discovery of short (~18-22 amino acid long), virus-derived peptide sequences, known as 2A peptides, that mediate a ribosome-skipping event, enables the generation of multiple separate peptide products, at similar levels, from a single mRNA. The 2A peptide coding sequence is included between the polypeptide-encoding transgenes (see, *e.g.,* Daniels et al. (2014) PLoS One 9(6):e100637).

IRES and 2A peptides use different mechanisms for co-expression of multiple genes in one transcript. For example, when using an IRES to express multiple genes in one mRNA, the gene directly downstream of the promoter is translated by the canonical cap-dependent mechanism, and those downstream of the IRES are translated by a cap-independent mechanism, which has a lower translation efficiency than the cap-dependent mechanism, resulting in unbalanced expression, with lower expression of the IRES-driven gene (see, *e.g.,* Chng et al. (2015) mAbs 7(2):403-412). 2A linked genes, on the other hand, are translated in one open reading frame (ORF). The cleavage of proteins separated by a 2A sequence occurs co-translationally, in an unconventional process, where a peptide bond often fails to form *(i.e.,* the peptide bond is "skipped") between the C-terminal glycine and proline in the 2A peptide. Despite this, translation proceeds, and two distinct proteins are produced in equal amounts. A short stretch, coding for approximately 20 amino acids, of the 2A peptide sequence, is sufficient to cause the bond-skipping. If the bond skipping does not occur, however, a fusion protein is generated that will not subsequently cleave (see, *e.g.,* Daniels et al. (2014) PLoS One 9(6):e100637).

Many of these 2A peptides have been described, including, but not limited to, T2A (SEQ ID NO:327) from *Thosea asigna* virus, P2A (SEQ ID NO:328) from porcine teschovirus-1, E2A (SEQ ID NO:329) from equine rhinitis A virus, and F2A (SEQ ID NO:330) from foot-and-mouth disease virus, among others. Different studies have reported conflicting cleavage efficiencies of the various 2A peptides, and the cleavage efficiency of a 2A peptide can be affected by the nature of the protein expressed, the order of genes flanking the 2A sequence, the length of the 2A peptide used, and the linker between the upstream protein and 2A peptide. Cleavage efficiency and enhanced protein expression can often be improved through the use of upstream viral cleavage sequences, such as, but not limited to, the peptide furin cleavage sequence, RRKR, as well as by inserting GSG and SGS peptide linkers, a V5 epitope tag (GKPUPNPLLGLDST), or a 3xFlag epitope tag immediately preceding the 2A peptide (see, *e.g.,* Chng et al. (2015) mAbs 7(2):403-412).

The immunostimulatory bacteria herein, containing plasmids encoding therapeutic products, such as immunomodulatory proteins, with a single promoter and ORF, can express two or more proteins through the use of viral internal ribosomal entry sites (IRES), which are cap-independent, or through translational read-through of 2A peptides, and subsequent self-cleavage into equally expressed co-proteins. The plasmids can contain other regulatory elements, as discussed below and elsewhere herein. For example, an exemplary construct (see, Example 14) is CMV-muIL-2 CO_T2A_muIFN-α2-WPRE, where codon optimized murine IL-2 is co-expressed with murine IFN-α2, using a CMV promoter, and a T2A peptide. Additionally, a Woodchuck Hepatitis Virus (WHP) Posttranscriptional Regulatory Element (WPRE) is included, to enhance expression. If, for example, a third therapeutic product is to be expressed by the plasmid, a 2A sequence is flanked by the first two proteins, which are expressed under the control of a first promoter, *e.g*., CMV, and a third protein is encoded under the control of a second promoter, *e.g*., EF-1α. Exemplary of such a construct is CMV-muIL-15Rα/IL-15sc_T2A_muSTING-R283G + EF-1α-muIL-18-WPRE, where murine 15Rα/IL-15sc and murine STING with the replacement R283G are co-expressed under control of a CMV promoter, using T2A, and murine IL-18 is expressed separately under control of an EF-1α promoter. This exemplary construct also includes a WPRE for enhanced expression.

### b. Dual/Multiple Promoter Constructs

Alternatively, the genetic payloads/therapeutic products can be expressed using dual or multiple promoter constructs, where each protein is expressed under the control of a separate promoter. Thus, plasmids encoding therapeutic products, such as immunomodulatory proteins, expressed in combinations, can contain multiple promoters, each controlling an individual intact ORF with proper stop codon processing (*i.e.,* dual/multiple promoter constructs); or multiple proteins can be expressed in a single ORF through the use of 2A peptides (*i.e.,* single promoter constructs); or the plasmid can contain a mixture of single and dual/multiple promoter constructs, to express three or more proteins, as described above.

### 3. Regulatory Elements

### a. Post-Transcriptional Regulatory Elements

In order to enhance expression of single and multiple therapeutic products/immunomodulatory proteins from a single plasmid, regulatory elements may be employed that enhance the transcription and translation of the protein(s) of interest. For example, the post-transcriptional regulatory element (PRE) of woodchuck hepatitis virus (WPRE), when inserted in the 3' untranslated region of the ORF, can enhance expression levels several fold (see, *e.g.,* Zufferey et al. (1999) J. Virol. 73(4):2886-2892). Similarly, other such elements, including, but not limited to, the Hepatitis B Virus PRE (HPRE), also can enhance expression. The combination of these can be used at the 3' ends of multiple ORFs to improve expression of multiple proteins on a single plasmid.

The PREs WPRE and HPRE are hepadnaviral cis-acting RNA elements that can increase the accumulation of cytoplasmic mRNA by promoting mRNA exportation from the nucleus, and can enhance post-transcriptional processing and stability.

### b. Polyadenylation Signal Sequences and Terminators

Other elements on the plasmid that can enhance protein expression include polyadenylation signal sequences and terminators. Polyadenylation is the post-transcriptional addition of a poly(A) tail to the 3' end of an mRNA transcript, which is part of the process that produces mature mRNA for translation. Polyadenylation signal sequences are important for nuclear export, mRNA stability, and efficient translation. A terminator is a sequence that defines the end of a transcript, creating a free 3' end, and initiates the release of the newly synthesized mRNA from the transcriptional machinery. The free 3' end is then available for the addition of the poly(A) tail. Terminators are found downstream of the gene to be transcribed, and typically occur directly after any 3' regulatory elements, such as the polyadenylation or poly(A) signal. Commonly used mammalian terminators in expression plasmids include the simian virus 40 (SV40), human growth hormone (hGH), bovine growth hormone (BGH or bGH), and rabbit beta-globin (rbGlob) polyA sequences, that include the sequence motif AAUAAA (SEQ ID NO:398), and promote both polyadenylation and termination.

When placed at the 3' end of the ORF, sequences such as the simian virus 40 poly A (SV40pA) or the bovine growth hormone poly A (bGHpA) signals, result in several-fold increased expression both *in vitro* and *in vivo* (see, *e.g.,* Powell et al. (2015) Discov. Med. 19(102):49-57). These and other such elements can further enhance the expression and translation of multiple therapeutic products, including immunomodulatory proteins, expressed from a single plasmid.

### c. Enhancers

Promoters and enhancers are found upstream of the multiple cloning site (MCS) in a plasmid, and cooperate to determine the rate of transcription. Enhancers are sequences that bind activator proteins, in order to loop the DNA, and bring a specific promoter to the initiation complex, thus increasing the rate of transcription. They can be adjacent to, or far from the promoter they influence, and include CMV, EF-1α, SV40, and synthetic enhancers, or the MND promoter, which is a synthetic promoter that contains the U3 region of a modified MoMuLV (Moloney murine leukemia virus) LTR with myeloproliferative sarcoma virus enhancer. The immunostimulatory bacteria herein contain plasmids that can comprise enhancer(s) to enhance the expression of the therapeutic products/proteins encoded on the plasmids.

### d. Secretion Signals

A secretion signal, also known as a signal sequence or peptide, a leader sequence or peptide, or a localization signal or sequence, is a short peptide at the N-terminus of a newly synthesized protein that is to be secreted. Signal peptides promote a cell to translocate a protein, usually to the cellular membrane. The efficiency of protein secretion is strongly determined by the signal peptide. Thus, the immunostimulatory bacteria herein contain plasmids that can comprise a signal peptide/secretion signal peptide, to facilitate and/or increase the expression or secretion of the encoded therapeutic product(s).

### e. Improving Bacterial Fitness

The plasmids in the immunostimulatory bacteria that encode the therapeutic products, include genes and regulatory elements that are provided for expression of bacterial genes, and also for expression of complex polycistronic eukaryotic payloads. The switch between such evolutionarily divergent organisms introduces challenges for proper functioning in prokaryotes and eukaryotes. The bacteria are cultured *in vitro,* then administered to a eukaryotic subject, where the plasmids are delivered to cells, particularly to tumor-resident myeloid cells, in cancer subjects, where the payloads are expressed, processed and trafficked. As described in the Examples (see, Example 30), transcriptional leakiness from the eukaryotic promoter, such as the CMV promoter, in bacteria, combined with large eukaryotic genes and regulatory sequences, can result in reduced bacterial fitness that manifests as low injection stock viability, and reduced growth rate in broth culture.

As shown in the Examples (see, Example 30), to minimize the negative impacts to bacterial fitness, while maintaining high ectopic expression in mammalian cells, the delivery plasmid was systematically modified to improve bacterial fitness, and to maintain or improve eukaryotic expression. The possible exemplary negative impacts and solutions include, for example, the following:
1) cryptic bacterial promoter sequences encoded within the CMV promoter enhancer region were identified using PromoterHunter (available online at phisite.org/promoterhunter/; see, *e.g.,* Klucar et al. (2010) Nucleic Acids Res. 38(Database issue):D366-D370), and these putative promoter sequences were replaced with CREB-binding sites and partial CREB-binding sites to promote efficient plasmid delivery;
2) to inhibit transcriptional leakiness from the CMV promoter, a number of bacterial terminators were inserted in the 5' UTR of ORF 1, to inhibit expression in bacteria (see table in Example 30, below);
3) to reduce the level of readthrough transcription from the origin of replication, the orientation of the expression cassette, from upstream of the CMV promoter to the end of the polyadenylation signal, was reversed, with and without a transcription terminator inserted between the expression cassette and the origin of replication; and
4) modifications from among 1)-3) above, that resulted in increased injection stock viability, with enhanced *in vitro* genetic payload expression, were combined for further improvement.

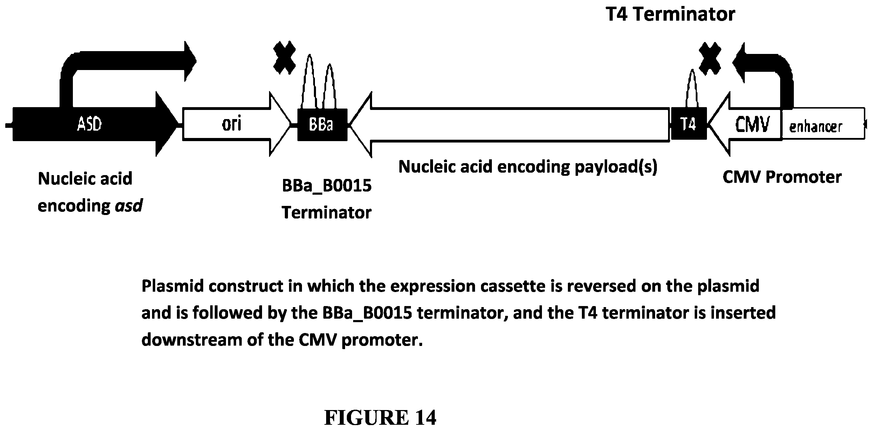

The results, detailed in the Examples (see, Example 30), show that, when the expression cassette was reversed on the plasmid and the BBa_B0015 bacterial terminator was inserted after the coding region, and the T4 bacterial terminator was inserted downstream of the CMV promoter (see, *e.g*., Figure 14), there was an increase in the bacterial cell viability, a reduced doubling time, growth to a higher stationary OD₆₀₀, and increased expression of the encoded payload *in vitro,* compared to the plasmid without the modifications.

The BBa_B0015 terminator is a composite terminator, in which a terminator derived from *E*. *coli* (BBa_B0010), and a terminator derived from the T7 phage (BBa_B0012), are joined.

Thus, provided herein are plasmids in which the eukaryotic promoter, such as a viral promoter, is in the opposite orientation from the bacterial promoter, such as the bacterial promoter controlling expression of the exogenous *asd* gene on the plasmid. For example, the exogenous *asd* cassette (includes the regulatory sequences for expression and the exogenous *asd* gene, encoded on the plasmid in the *asd⁻* bacteria) is in the opposite orientation from the eukaryotic regulatory sequences and operatively linked payload-encoding nucleic acid. This reduces readthrough (leakiness) of the eukaryotic promoter. These constructs also include bacterial terminators flanking the payload expression cassette that includes the eukaryotic promoter, which reduces readthrough from bacterial promoters. Figure 14, for example, provides an exemplary construct configuration.

Thus, bacterial fitness, if desired, can be improved by one or more of several strategies, including orienting expression cassettes including eukaryotic promoters in the opposite direction from those under the control of bacterial promoters, and the inclusion of bacterially-recognized terminators to terminate bacterial expression at strategic loci. Other such modifications can be included to enhance bacterial growth *in vitro,* and to favor expression, or to not interfere with expression, from eukaryotic promoters *in vivo* in the eukarytic host, such as a human. Thus, the constructs were improved by the inclusion of bacterial promoters; and by reversing the orientation of the nucleic acid encoding the payload, relative to the exogenous *asd*-encoding cassette.

### 4. Origin of Replication and Plasmid Copy Number

Plasmids are autonomously-replicating, extra-chromosomal, circular double-stranded DNA molecules that are maintained within bacteria by means of a replication origin. Copy number influences the plasmid stability. High copy number generally results in greater stability of the plasmid when the random partitioning occurs at cell division. A high copy number of plasmids generally decreases the growth rate, thus possibly allowing for bacterial cells with few plasmids to dominate the culture, since they grow faster. This can be ameliorated by using gene attenuation and gene dosing strategies, that limit the expression of certain genes on the plasmid that can be toxic to the bacteria when present in high copy numbers. The origin of replication also determines the plasmid's compatibility, *i.e.,* its ability to replicate in conjunction with another plasmid within the same bacterial cell. Plasmids that utilize the same replication system cannot co-exist in the same bacterial cell; they are said to belong to the same compatibility group. The introduction of a new origin, in the form of a second plasmid from the same compatibility group, mimics the result of replication of the resident plasmid. Thus, any further replication is prevented until after the two plasmids have been segregated to different cells to create the correct pre-replication copy number.

Numerous bacterial origins of replication are known to those of skill in the art. The origin can be selected to achieve a desired copy number. Origins of replication contain sequences that are recognized as initiation sites of plasmid replication via DNA-dependent DNA polymerases (see, *e.g.,* del Solar et al. (1998) Microbiol. Mol. Biol. Rev. 62(2):434-464). Different origins of replication provide for varying plasmid copy levels within each cell, and can range from one to hundreds of copies per cell. Commonly used bacterial plasmid origins of replication include, but are not limited to, pMB1 derived origins, which have very high copy derivatives, such as pUC, and lower copy derivatives, such as pBR322, as well as ColE1, p15A, and pSC101, and other origins, which have low copy numbers. Such origins are well-known to those of skill in the art. For example, the pUC19 origin results in copy numbers of 500-700 copies per cell. The pBR322 origin has a known copy number of 15-20 copies per cell. These origins only vary by a single base pair. The ColE1 origin copy number is 15-20, and derivatives, such as pBluescript, have copy numbers ranging from 300-500. The p15A origin that is in plasmid pACYC184, for example, results in a copy number of approximately 10. The pSC101 origins confer a copy number of approximately 5. Other low copy number vectors from which origins of replication can be obtained, include, for example, pWSK29, pWKS30, pWSK129, and pWKS130 (see, *e.g.,* Wang et al. (1991) Gene 100:195-199). Medium to low copy number is less than 150, or less than 100. Low copy number is less than 20, 25, or 30. Generally, less than medium copy number is less than 150 copies, and less than low copy number is less than about 25 or less than 25 copies, and generally, copy number refers to the average copies of plasmid per bacterium in a preparation. Those of skill in the art can identify plasmids with low, medium, or high copy numbers. For example, one method to determine experimentally if the copy number is high or low is to perform a miniprep. A high-copy plasmid should yield between 3-5 µg DNA per 1 ml LB culture; a low-copy plasmid will yield between 0.2-1 µg DNA per ml of LB culture. Sequences of bacterial plasmids, including identification of and sequence of the origin of replication, are well known (see, *e.g.,* snapgene.com/resources/plasmid_files/basic_cloning_vectors/pBR322/). Exemplary origins of replication, and their plasmid copy numbers, are summarized in the table below.

| **Origin of Replication** | **Copy Number** | **SEQ ID NO.** |
|---|---|---|
| pMB1 | Varies | 254 |
| p15A | 10-12 | 255 |
| pSC101 | ~5 | 256 |
| pBR322 | 15-20 | 243 |
| ColE1 | 15-20 | 257 |
| pPS10 | 15-20 | 258 |
| RK2 | ~5 | 259 |
| R6K (alpha origin) | 15-20 | 260 |
| R6K (beta origin) | 15-20 | 261 |
| R6K (gamma origin) | 15-20 | 262 |
| P1 (oriR) | Low | 263 |
| R1 | Low | 264 |
| pWSK | Low | 265 |
| ColE2 | 10-15 | 266 |
| pUC (pMB1) | 500-700 | 267 |
| F1 | 300-500 | 268 |

High copy plasmids are selected for heterologous expression of proteins *in vitro,* because the gene dosage is increased relative to chromosomal genes, there are higher specific yields of protein, and for therapeutic bacteria, higher therapeutic dosages of encoded therapeutics. It is shown, herein, however, that for delivery of plasmids encoding therapeutic products (*e*.*g*., immunomodulatory proteins), such as by *S. typhimurium,* in some embodiments, a high copy plasmid might be advantageous.

The requirement for bacteria to maintain the high copy plasmids can be a problem if the expressed molecule is toxic to the organism. The metabolic requirements for maintaining these plasmids can come at a cost of replicative fitness *in vivo.* Optimal plasmid copy number for delivery of therapeutic products can depend on the mechanism of attenuation of the strain engineered to deliver the plasmid. If needed, the skilled person, in view of the disclosure herein, can select an appropriate copy number for a particular immunostimulatory species and strain of bacteria.

### 5. CpG Motifs and CpG Islands

Unmethylated cytidine-phosphate-guanosine (CpG) motifs are prevalent in bacterial, but not in vertebrate, genomic DNA. Pathogenic DNA and synthetic oligodeoxynucleotides (ODNs) containing CpG motifs activate host defense mechanisms, leading to innate and acquired immune responses. The unmethylated CpG motifs contain a central unmethylated CG dinucleotide plus flanking regions. In humans, four distinct classes of CpG ODNs have been identified, based on differences in structure, and the nature of the immune response they induce. K-type ODNs (also referred to as B-type) contain from 1 to 5 CpG motifs, typically on a phosphorothioate backbone. D-type ODNs (also referred to as A-type) have a mixed phosphodiester/phosphorothioate backbone and have a single CpG motif, flanked by palindromic sequences that permit the formation of a stem-loop structure, as well as poly G motifs at the 3' and 5' ends. C-type ODNs have a phosphorothioate backbone, and contain multiple palindromic CpG motifs that can form stem loop structures or dimers. P-Class CpG ODNs have a phosphorothioate backbone, and contain multiple CpG motifs with double palindromes that can form hairpins at their GC-rich 3' ends (see, *e.g.,* Scheiermann et al. (2014) Vaccine 32(48):6377-6389). For purposes herein, the CpGs are encoded in the plasmid DNA; they can be introduced as a motif, or in a gene.

Toll-like receptors (TLRs) are key receptors for sensing pathogen-associated molecular patterns (PAMPs) and activating innate immunity against pathogens (see, *e.g.,* Akira et al. (2001) Nat. Immunol. 2(8):675-680). TLR9 recognizes hypomethylated CpG motifs in the DNA of prokaryotes that do not occur naturally in mammalian DNA (see, *e.g.,* McKelvey et al. (2011) J. Autoimmun. 36:76-86). Recognition of CpG motifs, upon phagocytosis of pathogens into endosomes in immune cell subsets, induces IRF7-dependent type I interferon signaling, and activates innate and adaptive immunity.

Immunostimulatory bacteria, such as *Salmonella* species, such as *S. typhimurium* strains, carrying plasmids containing CpG islands or motifs, are provided herein. These bacteria can activate TLR9, and induce type I IFN-mediated innate and adaptive immunity. As exemplified herein, bacterial plasmids that contain hypomethylated CpG islands can elicit innate and adaptive anti-tumor immune responses that, in combination with the therapeutic products encoded on the plasmid, such as immunostimulatory proteins and constitutively active variants of STING, IRF3, and other cytosolic DNA/RNA sensors, can have synergistic or enhanced anti-tumor activity. For example, the *asd* gene (SEQ ID NO:48) encodes a high frequency of hypomethylated CpG islands. CpG motifs can be included in combination with any of the therapeutic products, described or apparent from the description herein, in the immunostimulatory bacteria, to thereby enhance or improve anti-tumor immune responses in a treated subject.

Immunostimulatory CpGs can be included in the plasmids, by including a nucleic acid, typically from a bacterial gene, that encodes a gene product, and also, by adding a nucleic acid that encodes CpG motifs. The plasmids herein can include CpG motifs. Exemplary CpG motifs are known (see, *e.g.,* U.S. Patent Nos. 8,232,259, 8,426,375, and 8,241,844). These include, for example, synthetic immunostimulatory oligonucleotides, that are between 10 and 100, 10 and 20, 10 and 30, 10 and 40, 10 and 50, or 10 and 75, base pairs long, with the general formula: (CpG)ₙ, where n is the number of repeats. Generally, at least one or two repeats are used; non-CG bases can be interspersed. Those of skill in the art are very familiar with the general use of CpG motifs for inducing an immune response by modulating TLRs, particularly TLR9.

### 6. Plasmid Maintenance/Selection Components

The maintenance of plasmids in laboratory settings is usually ensured by the inclusion of an antibiotic resistance gene on the plasmid, and the use of antibiotics in the growth media. As described above, the use of an *asd* deletion mutant, complemented with a functional *asd* gene on the plasmid, allows for plasmid selection *in vitro* without the use of antibiotics, and allows for plasmid maintenance *in vivo.* The *asd* gene complementation system provides for such selection/maintenance (see, *e.g.,* Galan et al. (1990) Gene 94(1):29-35). The use of the *asd* gene complementation system to maintain plasmids in the tumor microenvironment increases the potency of *S. typhimurium* and other immunostimulatory bacterial strains, engineered to deliver plasmids encoding therapeutic products, such as immunostimulatory proteins, constitutively active cytosolic DNA/RNA sensors, antibodies, antibody fragments, or other such products as discussed herein.

### 7. DNA Nuclear Targeting Sequences

DNA nuclear targeting sequences (DTS), such as the SV40 DTS, mediate the translocation of DNA sequences through the nuclear pore complex. The mechanism of this transport is reported to be dependent on the binding of DNA binding proteins that contain nuclear localization sequences. The inclusion of a DTS on a plasmid to increase nuclear transport and expression has been demonstrated (see, *e.g.,* Dean, D.A. et al. (1999) Exp. Cell Res. 253(2):713-722), and has been used to increase gene expression from plasmids delivered by *S. typhimurium* (see, *e.g.,* Kong et al. (2012) Proc. Natl. Acad. Sci. U.S.A. 109(47):19414-19419).

Rho-independent or class I transcriptional terminators, such as the T1 terminator of the *rrnB* gene of *E. coli,* contain sequences of DNA that form secondary structures that cause dissociation of the transcription elongation complex. Transcriptional terminators are included in the plasmid in order to prevent expression of heterologous proteins by the *S. typhimurium* transcriptional machinery. This ensures that expression of the therapeutic products is confined to the host cell transcriptional machinery.

Plasmids used for transformation of *Salmonella,* such as *S. typhimurium,* as a cancer therapy described herein, contain all or some of the following attributes: 1) one or more constitutive promoters for heterologous expression of proteins; 2) one or more human immunomodulatory expression cassettes; 3) a bacterial origin of replication and optimized plasmid copy number; 4) immunostimulatory CpG islands; 5) an *asd* gene selectable marker for plasmid maintenance and selection; 6) DNA nuclear targeting sequences; and 7) transcriptional terminators.

### F. PHARMACEUTICAL PRODUCTION, COMPOSITIONS, AND FORMULATIONS

Provided herein are methods for manufacturing, and pharmaceutical compositions and formulations, containing any of the immunostimulatory bacteria provided herein and pharmaceutically acceptable excipients or additives. The pharmaceutical compositions can be used in the treatment of diseases, such as hyperproliferative diseases or conditions, such as a tumor or cancer. The immunostimulatory bacteria can be administered as a single agent therapy, or can be administered in a combination therapy with a further agent(s) or treatment(s). Combination therapy includes combining therapy with the immunostimulatory bacteria and/or other delivery vehicles provided herein, with any other anti-cancer therapy or treatment, including, but not limited to, immunotherapies, such as CAR-T therapy and checkpoint inhibitors, radiation, surgery, chemotherapeutic agents, such as nucleoside analogs and platinum compounds, and cellular therapies. The compositions can be formulated for single dosage administration, or for multiple dosage administration. The agents can be formulated for direct administration. The compositions can be provided as a liquid or dried formulation.

### 1. Manufacturing

### a. Cell Bank Manufacturing

As the active ingredient of the immunotherapeutic described herein is composed of engineered self-replicating bacteria, the selected composition will be expanded into a series of cell banks that will be maintained for long-term storage and as the starting material for manufacturing of the drug substance. Cell banks are produced under current good manufacturing practices (cGMP) in an appropriate manufacturing facility per the Code of Federal Regulations (CFR) 21 part 211, or other relevant regulatory authority. As the active agent of the immunotherapeutic is a live bacterium, the products described herein are, by definition, non-sterile and cannot be terminally sterilized. Care must be taken to ensure that aseptic procedures are used throughout the manufacturing process to prevent contamination. As such, all raw materials and solutions must be sterilized prior to use in the manufacturing process.

A master cell bank (MCB) is produced by sequential serial single colony isolation of the selected bacterial strain, to ensure no contaminants are present in the starting material. A sterile culture vessel containing sterile media (can be complex media, *e.g.,* LB or MSB, or defined media, *e.g.,* M9 supplemented with appropriate nutrients) is inoculated with a single well-isolated bacterial colony and the bacteria are allowed to replicate, *e.g*., by incubation at 37 °C with shaking. The bacteria are then prepared for cryopreservation by suspension in a solution containing a cryoprotective agent or agents.

Examples of cryoprotective agents include: proteins, such as human or bovine serum albumin, gelatin, and immunoglobulins; carbohydrates, including monosaccharides (*e.g.,* galactose, D-mannose, sorbose, *etc*.) and their non-reducing derivatives (*e.g*., methylglucoside), disaccharides (trehalose, sucrose, and others), cyclodextrins, and polysaccharides (*e.g*., raffinose, maltodextrins, dextrans, *etc*.); amino-acids (*e*.*g*., glutamate, glycine, alanine, arginine or histidine, tryptophan, tyrosine, leucine, phenylalanine, *etc*.); methylamines, such as betaine; polyols, such as trihydric or higher sugar alcohols, *e*.*g*., glycerin, erythritol, glycerol, arabitol, xylitol, sorbitol, and mannitol; propylene glycol; polyethylene glycol; surfactants, *e*.*g*., Pluronic^{®}; or organo-sulfur compounds, such as dimethyl sulfoxide (DMSO), and combinations thereof. Cryopreservation solutions can include one or more cryoprotective agents in a solution that also can contain salts (*e*.*g*., sodium chloride, potassium chloride, magnesium sulfate), and/or buffering agents, such as sodium phosphate, tris(hydroxymethyl)aminomethane (TRIS), 4-(2-hydroxyethyl)-1-piperazineethanesulfonic acid (HEPES), and other such buffering agents known to those of skill in the art.

Suspension of the bacteria in cryopreservation solution can be achieved either by addition of a concentrated cryoprotective agent or agents to the culture material to achieve a final concentration that preserves viability of the bacteria during the freezing and thawing process (*e.g*., 0.5% to 20% final concentration of glycerol), or by harvesting the bacteria (*e*.*g*., by centrifugation) and suspending in a cryopreservative solution containing the appropriate final concentration of cryoprotective agent(s). The suspension of bacteria in cryopreservation solution is then filled into appropriate sterile vials (plastic or glass) with a container closure system that is capable of maintaining closure integrity under frozen conditions (*e*.*g*., butyl stoppers and crimp seals). The vials of master cell bank are then frozen (either slowly by means of a controlled rate freezer, or quickly by means of placing directly into a freezer). The MCB is then stored frozen at a temperature that preserves long-term viability (*e*.*g*., at or below -60 °C). Thawed master cell bank material is thoroughly characterized to ensure identity, purity, and activity per regulation by the appropriate authorities.

Working cell banks (WCBs) are produced much the same way as the master cell bank, but the starting material is derived from the MCB. MCB material can be directly transferred into a fermentation vessel containing sterile media and expanded as above. The bacteria are then suspended in a cryopreservation solution, filled into containers, sealed, and frozen at or below -20°C. Multiple WCBs can be produced from MCB material, and WCB material can be used to make additional cell banks (*e.g*., a manufacturer's working cell bank (MWCB)). WCBs are stored frozen, and are characterized to ensure identity, purity, and activity. WCB material is typically the starting material used in the production of the drug substance of biologics such as engineered bacteria.

### b. Drug Substance Manufacturing

Drug substance is manufactured using aseptic processes under cGMP, as described above. Working cell bank material is typically used as starting material for manufacturing of drug substance under cGMP, however, other cell banks can be used (*e.g.,* MCB or MWCB). Aseptic processing is used for production of all cell therapies, including bacterial cell-based therapies. The bacteria from the cell bank are expanded by fermentation; this can be achieved by production of a pre-culture (*e.g*., in a shake flask), or by direct inoculation of a fermenter. Fermentation is accomplished in a sterile bioreactor or flask that can be single-use disposable, or re-usable. Bacteria are harvested by concentration (*e*.*g*., by centrifugation, continuous centrifugation, or tangential flow filtration). Concentrated bacteria are purified from media components and bacterial metabolites by exchange of the media with buffer (*e*.*g*., by diafiltration). The bulk drug product is formulated and preserved as an intermediate (*e*.*g*., by freezing or drying), or is processed directly into a drug product. Drug substance is tested for identity, strength, purity, potency, and quality.

### c. Drug Product Manufacturing

Drug product is defined as the final formulation of the active substance contained in its final container. Drug product is manufactured using aseptic processes under cGMP. Drug product is produced from drug substance. Drug substance is thawed or reconstituted if necessary, then formulated at the appropriate target strength. Because the active component of the drug product is live, engineered bacteria, the strength is determined by the number of colony forming units (CFUs) contained within the suspension. The bulk product is diluted in a final formulation appropriate for storage and use, as described below. Containers are filled and sealed with a container closure system, and the drug product is labeled. The drug product is stored at an appropriate temperature to preserve stability, and is tested for identity, strength, purity, potency, and quality, and released for human use if it meets specified acceptance criteria.

### 2. Compositions

Pharmaceutically acceptable compositions are prepared in view of approvals for a regulatory agency or other agency, and/or prepared in accordance with generally recognized pharmacopeia for use in animals and in humans. The compositions can be prepared as solutions, suspensions, powders, or sustained release formulations. Typically, the compounds are formulated into pharmaceutical compositions using techniques and procedures well-known in the art (see, *e.g.,* Ansel, Introduction to Pharmaceutical Dosage Forms, Fourth Edition, 1985, page 126). The formulation should suit the mode of administration.

Compositions can be formulated for administration by any route known to those of skill in the art, including intramuscular, intravenous, intradermal, intralesional, intraperitoneal, subcutaneous, intratumoral, epidural, nasal, oral, vaginal, rectal, topical, local, otic, inhalational, buccal (*e*.*g*., sublingual), and transdermal administration, or by any suitable route. Other modes of administration also are contemplated. Administration can be local, topical, or systemic, depending upon the locus of treatment. Local administration to an area in need of treatment can be achieved by, for example, but not limited to, local infusion during surgery, topical application, *e*.*g*., in conjunction with a wound dressing after surgery, by injection, by means of a catheter, by means of a suppository, or by means of an implant. Compositions also can be administered with other biologically active agents, either sequentially, intermittently, or in the same composition. Administration also can include controlled release systems, including controlled release formulations and device controlled release, such as by means of a pump.

The most suitable route in any given case depends on a variety of factors, such as the nature of the disease, the progress of the disease, the severity of the disease, and the particular composition which is used. Pharmaceutical compositions can be formulated in dosage forms appropriate for each route of administration. In particular, the compositions can be formulated into any suitable pharmaceutical preparations for systemic, local, intraperitoneal, oral, or direct administration. For example, the compositions can be formulated for administration subcutaneously, intramuscularly, intratumorally, intravenously, or intradermally. Administration methods can be employed to decrease the exposure of the active agent to degradative processes, such as immunological intervention via antigenic and immunogenic responses. Examples of such methods include local administration at the site of treatment, or continuous infusion.

The immunostimulatory bacteria can be formulated into suitable pharmaceutical preparations, such as solutions, suspensions, tablets, dispersible tablets, pills, capsules, powders, sustained release formulations, or elixirs, for oral administrations, as well as transdermal patch preparations, and dry powder inhalers. Typically, the compounds are formulated into pharmaceutical compositions using techniques and procedures well-known in the art (see, *e.g.,* Ansel, Introduction to Pharmaceutical Dosage Forms, Fourth Edition, 1985, page 126). Generally, the mode of formulation is a function of the route of administration. The compositions can be formulated in dried (lyophilized or other forms of vitrification) or liquid form. Where the compositions are provided in dried form, they can be reconstituted just prior to use by addition of an appropriate buffer, for example, a sterile saline solution.

### 3. Formulations

### a. Liquids, Injectables, Emulsions

The formulation generally is made to suit the route of administration. Parenteral administration, generally characterized by injection or infusion, either subcutaneously, intramuscularly, intratumorally, intravenously, or intradermally, is contemplated herein. Preparations of bacteria for parenteral administration include suspensions ready for injection (direct administration), frozen suspensions that are thawed prior to use, dry soluble products, such as lyophilized powders, ready to be combined with a resuspension solution just prior to use, and emulsions. Dried thermostable formulations, such as lyophilized formulations, can be used for storage of unit doses for later use.

The pharmaceutical preparation can be in a frozen liquid form, for example, a suspension. If provided in frozen liquid form, the drug product can be provided as a concentrated preparation to be thawed and diluted to a therapeutically effective concentration before use.

The pharmaceutical preparations also can be provided in a dosage form that does not require thawing or dilution for use. Such liquid preparations can be prepared by conventional means with pharmaceutically acceptable additives, as appropriate, such as suspending agents (*e*.*g*., sorbitol, cellulose derivatives, or hydrogenated edible fats); emulsifying agents (*e.g.,* lecithin or acacia); non-aqueous vehicles (*e.g.,* almond oil, oily esters, or fractionated vegetable oils); and preservatives suitable for use with microbial therapeutics. The pharmaceutical preparations can be presented in dried form, such as lyophilized or spray-dried, for reconstitution with water or other sterile suitable vehicle before use.

Suitable excipients are, for example, water, saline, dextrose, or glycerol. The solutions can be either aqueous or non-aqueous. If administered intravenously, suitable carriers include physiological saline or phosphate buffered saline (PBS), and other buffered solutions used for intravenous hydration. For intratumoral administration, solutions containing thickening agents, such as glucose, polyethylene glycol, and polypropylene glycol, oil emulsions, and mixtures thereof, can be appropriate to maintain localization of the injectant.

Pharmaceutical compositions can include carriers or other excipients. For example, pharmaceutical compositions provided herein can contain any one or more of a diluents(s), adjuvant(s), antiadherent(s), binder(s), coating(s), filler(s), flavor(s), color(s), lubricant(s), glidant(s), preservative(s), detergent(s), or sorbent(s), and a combination thereof, or a vehicle with which a modified therapeutic bacteria is administered. For example, pharmaceutically acceptable carriers or excipients used in parenteral preparations include aqueous vehicles, non-aqueous vehicles, isotonic agents, buffers, antioxidants, local anesthetics, suspending and dispersing agents, emulsifying agents, sequestering or chelating agents, and other pharmaceutically acceptable substances. Formulations, including liquid preparations, can be prepared by conventional means with pharmaceutically acceptable additives or excipients.

Pharmaceutical compositions can include carriers, such as a diluent, adjuvant, excipient, or vehicle, with which the compositions are administered. Examples of suitable pharmaceutical carriers are described in "Remington's Pharmaceutical Sciences" by E. W. Martin. Such compositions will contain a therapeutically effective amount of the compound or agent, generally in purified form or partially purified form, together with a suitable amount of carrier, so as to provide the form for proper administration to the patient. Such pharmaceutical carriers can be sterile liquids, such as water and oils, including those of petroleum, animal, vegetable, or synthetic origin, such as peanut oil, soybean oil, mineral oil, and sesame oil. Water is a typical carrier. Saline solutions and aqueous dextrose and glycerol solutions also can be employed as liquid carriers, particularly for injectable solutions. Compositions can contain, along with an active ingredient: a diluent, such as lactose, sucrose, dicalcium phosphate, or carboxymethylcellulose; a lubricant, such as magnesium stearate, calcium stearate, and talc; and a binder, such as starch, natural gums, such as gum acacia, gelatin, glucose, molasses, polyvinylpyrrolidine, celluloses and derivatives thereof, povidone, crospovidone, and other such binders known to those of skill in the art. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, and ethanol. For example, suitable excipients are, for example, water, saline, dextrose, glycerol, or ethanol. A composition, if desired, also can contain other minor amounts of non-toxic auxiliary substances, such as wetting or emulsifying agents, pH buffering agents, stabilizers, solubility enhancers, and other such agents, such as, for example, sodium acetate, sorbitan monolaurate, triethanolamine oleate, and cyclodextrins.

Pharmaceutically acceptable carriers used in parenteral preparations include aqueous vehicles, non-aqueous vehicles, antimicrobial agents, isotonic agents, buffers, antioxidants, local anesthetics, suspending and dispersing agents, emulsifying agents, sequestering or chelating agents, and other pharmaceutically acceptable substances. Examples of aqueous vehicles include Sodium Chloride Injection, Ringer's Injection, Isotonic Dextrose Injection, Sterile Water Injection, and Dextrose and Lactated Ringer's Injection. Non-aqueous parenteral vehicles include fixed oils of vegetable origin, cottonseed oil, corn oil, sesame oil, and peanut oil. Isotonic agents include sodium chloride and dextrose. Buffers include phosphate and citrate. Antioxidants include sodium bisulfate. Local anesthetics include procaine hydrochloride. Suspending and dispersing agents include sodium carboxymethylcellulose, hydroxypropyl methylcellulose, and polyvinylpyrrolidone. Emulsifying agents include, for example, polysorbates, such Polysorbate 80 (TWEEN 80). Sequestering or chelating agents of metal ions, such as EDTA, can be included. Pharmaceutical carriers also include polyethylene glycol and propylene glycol, for water miscible vehicles, and sodium hydroxide, hydrochloric acid, citric acid, or lactic acid, for pH adjustment. Non-anti-microbial preservatives can be included.

The pharmaceutical compositions also can contain other minor amounts of non-toxic auxiliary substances, such as wetting or emulsifying agents, pH buffering agents, stabilizers, solubility enhancers, and other such agents, such as, for example, sodium acetate, sorbitan monolaurate, triethanolamine oleate, and cyclodextrins. Implantation of a slow-release or sustained-release system, such that a constant level of dosage is maintained (see, *e.g.,* U.S. Patent No. 3,710,795), also is contemplated herein. The percentage of active compound contained in such parenteral compositions is highly dependent on the specific nature thereof, as well as the activity of the compound and the needs of the subject.

### b. Dried Thermostable Formulations

The bacteria can be dried. Dried thermostable formulations, such as lyophilized or spray dried powders and vitrified glass, can be reconstituted for administration as solutions, emulsions, and other mixtures. The dried thermostable formulations can be prepared from any of the liquid formulations, such as the suspensions, described above. The pharmaceutical preparations can be presented in lyophilized or vitrified form, for reconstitution with water or other suitable vehicle, before use.

The thermostable formulation is prepared for administration by reconstituting the dried compound with a sterile solution. The solution can contain an excipient which improves the stability or other pharmacological attribute of the active substance or reconstituted solution, prepared from the powder. The thermostable formulation is prepared by dissolving an excipient, such as dextrose, sorbitol, fructose, corn syrup, xylitol, glycerin, glucose, sucrose, or other suitable agent, in a suitable buffer, such as citrate, sodium, or potassium phosphate, or other such buffer known to those of skill in the art. Then, the drug substance is added to the resulting mixture, and stirred until it is mixed. The resulting mixture is apportioned into vials for drying. Each vial will contain a single dosage, containing 1x10⁵ to 1x10¹¹ CFUs per vial. After drying, the product vial is sealed with a container closure system that prevents moisture or contaminants from entering the sealed vial. The dried product can be stored under appropriate conditions, such as at -20 °C, 4 °C, or room temperature. Reconstitution of this dried formulation with water or a buffer solution provides a formulation for use in parenteral administration. The precise amount depends upon the indication treated and selected compound. Such amount can be empirically determined.

### 4. Compositions for Other Routes of Administration

Depending upon the condition treated, other routes of administration in addition to parenteral, such as topical application, transdermal patches, and oral and rectal administration, also are contemplated herein. The suspensions and powders described above can be administered orally, or can be reconstituted for oral administration. Pharmaceutical dosage forms for rectal administration are rectal suppositories, capsules, and tablets and gel capsules for systemic effect. Rectal suppositories include solid bodies for insertion into the rectum which melt or soften at body temperature, releasing one or more pharmacologically or therapeutically active ingredients. Pharmaceutically acceptable substances in rectal suppositories are bases or vehicles and agents to raise the melting point. Examples of bases include cocoa butter (theobroma oil), glycerin-gelatin, carbowax (polyoxyethylene glycol), and appropriate mixtures of mono-, di-, and triglycerides of fatty acids. Combinations of the various bases can be used. Agents to raise the melting point of suppositories include spermaceti and wax. Rectal suppositories can be prepared either by the compressed method, or by molding. The typical weight of a rectal suppository is about 2 to 3 grams. Tablets and capsules for rectal administration are manufactured using the same pharmaceutically acceptable substance and by the same methods as for formulations for oral administration. Formulations suitable for rectal administration can be provided as unit dose suppositories. These can be prepared by admixing the drug substance with one or more conventional solid carriers, for example, cocoa butter, and then shaping the resulting mixture.

For oral administration, pharmaceutical compositions can take the form of, for example, tablets or capsules prepared by conventional means with pharmaceutically acceptable excipients, such as binding agents (*e.g.,* pregelatinized maize starch, polyvinyl pyrrolidone, or hydroxypropyl methylcellulose); fillers (*e.g.,* lactose, microcrystalline cellulose, or calcium hydrogen phosphate); lubricants (*e.g.,* magnesium stearate, talc, or silica); disintegrants (*e.g.,* potato starch or sodium starch glycolate); or wetting agents (*e.g.,* sodium lauryl sulfate). The tablets can be coated by methods well-known in the art.

Formulations suitable for buccal (sublingual) administration include, for example, lozenges containing the active compound in a flavored base, usually sucrose and acacia or tragacanth; and pastilles containing the compound in an inert base, such as gelatin and glycerin, or sucrose and acacia.

Topical mixtures are prepared as described for local and systemic administration. The resulting mixtures can be solutions, suspensions, emulsions, or the like, and are formulated as creams, gels, ointments, emulsions, solutions, elixirs, lotions, suspensions, tinctures, pastes, foams, aerosols, irrigations, sprays, suppositories, bandages, dermal patches, or any other formulations suitable for topical administration.

The compositions can be formulated as aerosols for topical application, such as by inhalation (see, *e.g.,* U.S. Patent Nos. 4,044,126, 4,414,209, and 4,364,923, which describe aerosols for the delivery of a steroid useful for treatment of lung diseases). These formulations, for administration to the respiratory tract, can be in the form of an aerosol or solution for a nebulizer, or as a microfine powder for insufflation, alone or in combination with an inert carrier such as lactose. In such a case, the particles of the formulation will typically have diameters of less than 50 microns, or less than 10 microns.

The compounds can be formulated for local or topical application, such as for topical application to the skin and mucous membranes, such as in the eye, in the form of gels, creams, and lotions, and for application to the eye, or for intracisternal or intraspinal application. Topical administration is contemplated for transdermal delivery, and also for administration to the eyes or mucosa, or for inhalation therapies. Nasal solutions of the active compound alone, or in combination with other pharmaceutically acceptable excipients, also can be administered.

Formulations suitable for transdermal administration are provided. They can be provided in any suitable format, such as discrete patches adapted to remain in intimate contact with the epidermis of the recipient for a prolonged period of time. Such patches contain the active compound in an optionally buffered aqueous solution of, for example, 0.1 to 0.2 M concentration, with respect to the active compound. Formulations suitable for transdermal administration also can be delivered by iontophoresis (see, *e.g.,* Tyle, P. (1986) Pharmaceutical Research 3(6):318-326), and typically take the form of an optionally buffered aqueous solution of the active compound.

Pharmaceutical compositions also can be administered by controlled release formulations and/or delivery devices (see *e.g.,* U.S. Patent Nos. 3,536,809; 3,598,123; 3,630,200; 3,845,770; 3,916,899; 4,008,719; 4,769,027; 5,059,595; 5,073,543; 5,120,548; 5,591,767; 5,639,476; 5,674,533; and 5,733,566).

### 5. Dosages and Administration

The compositions can be formulated as pharmaceutical compositions for single dosage or multiple dosage administration. The immunostimulatory bacteria can be included in an amount sufficient to exert a therapeutically useful effect in the absence of undesirable side effects on the patient treated. For example, the concentration of the pharmaceutically active compound is adjusted so that an injection provides an effective amount to produce the desired pharmacological effect. The therapeutically effective concentration can be determined empirically by testing the immunostimulatory bacteria in known *in vitro* and *in vivo* systems, such as by using the assays described herein or known in the art. For example, standard clinical techniques can be employed. *In vitro* assays and animal models can be employed to help identify optimal dosage ranges. The precise dose, which can be determinied empirically, can depend on the age, weight, body surface area, and condition of the patient or animal, the particular immunostimulatory bacteria administered, the route of administration, the type of disease to be treated, and the seriousness of the disease.

Hence, it is understood that the precise dosage and duration of treatment is a function of the disease being treated, and can be determined empirically using known testing protocols, or by extrapolation from *in vivo* or *in vitro* test data. Concentrations and dosage values also can vary with the severity of the condition to be alleviated. It is to be further understood that, for any particular subject, specific dosage regimens should be adjusted over time according to the individual need and the professional judgment of the person administering or supervising the administration of the compositions, and that the concentration ranges set forth herein are exemplary only and are not intended to limit the scope or use of compositions and combinations containing them. The compositions can be administered hourly, daily, weekly, monthly, yearly, or once. Generally, dosage regimens are chosen to limit toxicity. It should be noted that the attending physician would know how to and when to terminate, interrupt, or adjust therapy to lower dosage due to toxicity, or bone marrow, liver, or kidney, or other tissue dysfunctions. Conversely, the attending physician would also know how to and when to adjust treatment to higher levels if the clinical response is not adequate (precluding toxic side effects).

The immunostimulatory bacteria are included in the composition in an amount sufficient to exert a therapeutically useful effect. For example, the amount is one that achieves a therapeutic effect in the treatment of a hyperproliferative disease or condition, such as cancer. An exemplary dose can be about 1 x 10⁹ CFU/m². As shown in the table below, and noted above, higher doses can be administered. The data below, from an experiment in a mouse model, show that strains with the genome modifications as described herein have significantly improved tolerability, at least about 15-fold, compared VNP20009, and, thus, can be dosed in higher amounts.

| **Strain Name** | **Strain Genotype** | **LD₅₀ in BALB/c Mice** | **Fold Attenuated** | **Fold Improved vs, VNP2009** | **Human Equivalent Dose (CFU/m²)** |
|---|---|---|---|---|---|
| VNP20009 | Δ*purI +* Δ*msbB* | 4.4E6 | ~44,000 | - | 6.33E+08 |
| ΔFLG | Δ*purI +* Δ*msbB +* Δ*asdA +* Δ*FLG* | 2.0E7 | 200,000 | 4.5 | 2.88E+09 |
| Δ*pagP* | Δ*purI +* Δ*msbB +* Δ*asdA +* Δ*pagP* | 1.4E7 | 139,000 | 3.2 | 2.00E+09 |
| ΔFLG/Δ*ρagP* | Δ*purI +* Δ*msbB +* Δ*asdA +* Δ*FLG +* Δ*pagP* | >6.2E7 | >620,000 | >14 | >8.91E+09 |

Pharmaceutically and therapeutically active compounds and derivatives thereof are typically formulated and administered in unit dosage forms or multiple dosage forms. Each unit dose contains a predetermined quantity of therapeutically active compound sufficient to produce the desired therapeutic effect, in association with the required pharmaceutical carrier, vehicle, or diluent. Unit dosage forms, include, but are not limited to, tablets, capsules, pills, powders, granules, parenteral suspensions, oral solutions or suspensions, and oil-in-water emulsions, containing suitable quantities of the compounds or pharmaceutically acceptable derivatives thereof. Unit dose forms can be contained in vials, ampoules and syringes, or individually packaged tablets or capsules. Unit dose forms can be administered in fractions or multiples thereof. A multiple dose form is a plurality of identical unit dosage forms packaged in a single container to be administered in segregated unit dose form. Examples of multiple dose forms include vials, bottles of tablets or capsules, or bottles of pints or gallons. Hence, multiple dose form is a multiple of unit doses that are not segregated in packaging. Generally, dosage forms or compositions containing active ingredient in the range of 0.005% to 100%, with the balance made up from non-toxic carrier, can be prepared. Pharmaceutical compositions can be formulated in dosage forms appropriate for each route of administration.

The unit-dose parenteral preparations are packaged in an ampoule, a vial, or a syringe with a needle. The volume of liquid solution or reconstituted powder preparation, containing the pharmaceutically active compound, is a function of the disease to be treated and the particular article of manufacture chosen for package. All preparations for parenteral administration must be sterile, as is known and practiced in the art.

As indicated, compositions provided herein can be formulated for any route known to those of skill in the art, including, but not limited to, subcutaneous, intramuscular, intravenous, intradermal, intralesional, intraperitoneal, epidural, vaginal, rectal, local, otic, or transdermal administration, or any route of administration. Formulations suited for such routes are known to one of skill in the art. Compositions also can be administered with other biologically active agents, either sequentially, intermittently, or in the same composition.

Pharmaceutical compositions can be administered by controlled release formulations and/or delivery devices (see, *e.g.,* U.S. Patent Nos. 3,536,809; 3,598,123; 3,630,200; 3,845,770; 3,847,770; 3,916,899; 4,008,719; 4,687,660; 4,769,027; 5,059,595; 5,073,543; 5,120,548; 5,354,556; 5,591,767; 5,639,476; 5,674,533; and 5,733,566). Various delivery systems are known and can be used to administer selected compositions, are contemplated for use herein, and such particles can be easily made.

### 6. Packaging and Articles of Manufacture

Also provided are articles of manufacture containing packaging materials, any pharmaceutical composition provided herein, and a label that indicates that the compositions are to be used for treatment of diseases or conditions as described herein. For example, the label can indicate that the treatment is for a tumor or for cancer.

Combinations of immunostimulatory bacteria described herein and another therapeutic agent also can be packaged in an article of manufacture. In one example, the article of manufacture contains a pharmaceutical composition containing the immunostimulatory bacteria composition and no further agent or treatment. In other examples, the article of manufacture contains another further therapeutic agent, such as a different anti-cancer agent. In this example, the agents can be provided together or separately, for packaging as articles of manufacture.

The articles of manufacture provided herein contain packaging materials. Packaging materials for use in packaging pharmaceutical products are well-known to those of skill in the art. See, for example, U.S. Patent Nos. 5,323,907, 5,052,558, and 5,033,252, each of which is incorporated herein in its entirety. Examples of pharmaceutical packaging materials include, but are not limited to, blister packs, bottles, tubes, inhalers, pumps, bags, vials, containers, syringes, bottles, and any packaging material suitable for a selected formulation and intended mode of administration and treatment. Exemplary of articles of manufacture are containers, including single chamber and dual chamber containers. The containers include, but are not limited to, tubes, bottles, and syringes. The containers can further include a needle for intravenous administration.

The choice of package depends on the agents, and whether such compositions will be packaged together or separately. In general, the packaging is non-reactive with the compositions contained therein. In other examples, some of the components can be packaged as a mixture. In other examples, all components are packaged separately. Thus, for example, the components can be packaged as separate compositions that, upon mixing just prior to administration, can be directly administered together. Alternatively, the components can be packaged as separate compositions for administration separately.

Selected compositions including articles of manufacture thereof also can be provided as kits. Kits can include a pharmaceutical composition described herein, and an item for administration provided as an article of manufacture. The compositions can be contained in the item for administration, or can be provided separately to be added later. The kit can, optionally, include instructions for application, including dosages, dosing regimens, and instructions for modes of administration. Kits also can include a pharmaceutical composition described herein and an item for diagnosis.

### G. METHODS OF TREATMENT AND USES

The methods provided herein include methods of administering or using the immunostimulatory bacteria, for treating subjects having a disease or condition whose symptoms can be ameliorated or lessened by administration of such bacteria, such as cancer. In particular examples, the disease or condition is a tumor or a cancer. Additionally, methods of combination therapies with one or more additional agents for treatment, such as an anti-cancer agent or an anti-hyaluronan agent, also are provided. The bacteria can be administered by any suitable route, including, but not limited to, parenteral, systemic, topical, and local, such as intra-tumoral, intravenous, rectal, oral, intramuscular, mucosal, and other routes. Because of the modifications of the bacteria described herein, problems associated with systemic administration are solved. Formulations suitable for each route of administration are provided. The skilled person can establish suitable regimens and doses, and can select routes of administration.

### 1. Diagnostics for Patient Selection for Treatment and for

### Monitoring Treatment

### a. Patient Selection

Biomarkers can be used to identify patients who are likely to respond to therapy with the immunostimulatory bacteria provided herein. For example, the Adenosine Signature and the Myeloid Signature can be assessed by NanoString gene expression panels, and T-cell infiltration of tumors can be assessed by the Immunoscore^{®} test, which is an *in vitro* diagnostic test used for predicting the risk of relapse in early stage colon cancer patients, by measuring the host immune response at a tumor site. Patients whose tumors or body fluids indicate an immune responsiveness or an immune response are more likely to respond to the treatment with the immunostimulatory bacteria provided herein.

Other biomarkers include tumor-infiltrating lymphocytes (TILs), CD73, CD39, TNAP (tissue-nonspecific alkaline phosphatase), CD38, CD68, PD-L1, and FoxP3. For example, tumors that can be treated with the immunostimulatory bacteria provided herein are T-cell excluded, exhibit high levels of purines/adenosine, and are unresponsive to PD-1/PD-L1 targeted therapies.

Gene expression profiles (GEPs), which can be determined using various NanoString gene expression panels, can be analyzed, for example, to identify the "adenosine signature" of tumors. High concentrations of adenosine are found in certain tumors, including colorectal carcinoma (CRC), non-small cell lung cancer (NSCLC), and pancreatic cancer, among others. Patients with tumors that exhibit high concentrations of purines/adenosine are likely to respond to therapy, since the immunostimulatory bacteria herein accumulate and replicate in purine/adenosine rich tumor microenvironments. Thus, the identification of tumors that express an "Adenosine Signature" can be used to predict patient response to therapy. Additionally, the immunostimulatory bacteria herein preferentially accumulate in and infect tumor-resident myeloid cells. Thus, the "Myeloid Signature" also can be used to predict patient response to therapy with the immunostimulatory bacteria. For example, it has been shown that the "Adenosine Signature" is nearly identical to the "Myeloid Signature" that is associated with poor response to atezolizumab (anti-PD-L1) monotherapy in renal cell carcinoma (RCC) patients, which is indicative of the role of adenosine in tumor escape from anti-PD-L1 therapy (see, *e.g.,* McDermott et al. (2018) Nature Medicine 24:749-757). Tumor-myeloid and tumor-adenosine NanoString signature panels are available and can be used for the selection of patients.

Macrophages limit T-cell infiltration into solid tumors and suppress their function, for example, in triple negative breast cancer (see, *e.g.,* Keren et al. (2018) Cell 174:1373-1387). In certain cancers, such as CRC, macrophages dominate the intratumoral immune population and promote T-cell exclusion, and as a result, tumor-associated macrophages are associated with poor prognosis in CRC (see, *e.g.*, Bindea et al. (2013) Immunity 39:782-795). Immunoscore^{®}, a method to estimate the prognosis of cancer patients, based on the immune cells that infiltrate the cancer and surround it, can be used to measure T-cell exclusion or T-cell infiltration. Immunoscore^{®} incorporates the effects of the host immune response into cancer classification and improves prognostic accuracy. It measures the density of two T lymphocyte populations (CD3/CD8, CD3/CD45RO, or CD8/CD45RO) in the center and at the periphery of the tumor, and provides a score ranging from 0 (I0), when low densities of both cell types are found in both regions, to Immunoscore 4 (I4), when high densities are found in both regions. Low infiltration of T lymphocytes results in a low Immunoscore^{®}, which correlates with high risk, while high infiltration of T lymphocytes results in a high Immunoscore^{®}, which correlates with low risk. Immunoscore^{®}, thus, can be evaluated as a prospective biomarker to identify patients that will respond to therapy with the immunostimulatory bacteria provided herein. For example, T-cell poor/uninflamed tumors can be treated, because the immunostimulatory bacteria provided herein induce T-cell infiltration in cold tumors. Such tumors represent a high, unmet need population that is refractory to checkpoint inhibition.

Extracellular adenosine is produced by the sequential activities of membrane associated ectoenzymes, CD39 (ecto-nucleoside triphosphate diphosphohydrolase 1, or NTPDase1) and CD73 (ecto-5'-nucleotidase), which are expressed on tumor stromal cells, together producing adenosine by phosphohydrolysis of ATP or ADP that is produced from dead or dying cells. CD39 converts extracellular ATP (or ADP) to 5'-AMP, which is converted to adenosine by CD73. Expression of CD39 and CD73 on endothelial cells is increased under the hypoxic conditions of the tumor microenvironment, thereby increasing levels of adenosine. Thus, CD39 and CD73 can be used as biomarkers that indicate adenosine-rich tumors that can be targeted with the immunostimulatory bacteria provided herein.

CD38, also known as cyclic ADP ribose hydrolase, is a glycoprotein that is found on the surface of many immune cells, including CD4⁺ T-cells, CD8⁺ T-cells, B lymphocytes, and natural killer cells. The loss of CD38, which is a marker of cell activation, is associated with impaired immune responses, and has been linked to leukemias, myelomas, and solid tumors. Additionally, increased expression of CD38 is an unfavorable diagnostic marker in chronic lymphocytic leukemia and is associated with increased disease progression. CD38 also is used as a target for daratumumab (Darzalex^{®}), which has been approved for the treatment of multiple myeloma. CD68 is highly expressed by monocytes, circulating macrophages, and by tissue macrophages (*e.g.,* Kupffer cells, microglia). FoxP3 is involved in immune system responses, and acts as a regulator in the development and function of regulatory T-cells (or Tregs), which are immunosuppressive. In cancer, an excess of regulatory T-cell activity can prevent the immune system from destroying cancer cells. Thus, CD38, CD68, and FoxP3 also can be used as biomarkers for the selection of patients that are likely to respond to therapy with the immunostimulatory bacteria herein.

### b. Diagnostics to Assess or Detect Activity of the

### Immunostimulatory Bacteria are Indicative of the Effectiveness of Treatment

Biomarkers can be used to monitor the immunostimulatory bacteria following treatment. Biomarkers occur in tumor samples and/or in body fluid samples, such as blood, plasma, urine, saliva, and other fluids. Validated, peripheral blood biomarkers are used to evaluate the immune status of patients prior to and during treatment, to determine changes in the immune status, which correlate with the effectiveness of treatment. A change to, or an increase in, anti-tumor immune response status indicates that treatment with the immunostimulatory bacteria is having an effect. Immunomodulatory activity of the immunostimulatory bacteria provided herein, for example, in dose escalation and expansion studies, can be assessed. Examination of biomarkers reveals prognostic and predictive factors relating to disease (*e.g.,* a tumor) status and its treatment, which can aid in monitoring treatment. Evaluating the tumor microenvironment, for example, provides insights into the mechanism of tumor responses to immunotherapies. Serum biomarkers to detect immunomodulatory activity of the immunostimulatory bacteria include, but are not limited to, CXCL10 (IP-10), CXCL9, interferon-β, interferon-γ, proinflammatory serum cytokines (*e.g.,* IL-6, TNF-α, MCP-1/CCL1), and IL-18 binding protein.

CXCL10 and CXCL9 are chemokines that are necessary for CD8⁺ T-cell activation and trafficking to tumors, for example, in response to immunotherapies. In a phase 3 trial of nivolumab, an anti-PD-1 immune checkpoint inhibitor, for the treatment of previously treated patients with metastatic renal cell carcinoma (mRCC), immune pharmacodynamic effects that were shared by the majority of patients, irrespective of the dose administered, were identified. Assessment of the IFN-γ regulated serum chemokines CXCL9 and CXCL10 was performed using a multiplex panel based on Luminex technology (Myriad^{®} Rules-Based Medicine (RBM)), and the results demonstrated that increased CXCL9 and CXCL10 serum levels, as well as increased transcription in the tumor, correlated with clinical response. Median increases in chemokine levels after treatment with nivolumab from baseline were 101% for CXCL9, and 37% for CXCL10, in peripheral blood (see, *e.g.,* Choueiri et al. (2016) Clin. Cancer Res. 22(22):5461-5471). Additionally, treatment of patients with advanced solid tumors or lymphomas with the MK-1454 STING agonist (Merck), resulted in a dose-dependent increase in serum CXCL10 after intratumoral dosing (see, *e.g.,* Harrington et al. ESMO Annual Meeting (2018)). Dose-dependent increases in serum levels of IFN-β were observed following intratumoral dosing of the ADU-S100 STING agonist (Aduro) (see, *e.g.,* Meric-Bernstam et al. ASCO Annual Meeting (2019)). Additionally, intravenous administration of VNP20009 induced a dose-dependent increase in the serum levels of the pro-inflammatory cytokines IL-6, TNF-α, IL-1β, and IL-12 (see, *e.g.,* Toso et al. (2002) J. Clin. Oncol. 20(1):142-152).

IL-18 participates in protective immune responses to intracellular bacteria, fungi and viruses, and has demonstrated anti-tumor activity in preclinical models of lung cancer, breast cancer, sarcoma, and melanoma. The biological activity of IL-18 is modulated in a negative feedback loop by IL-18 binding protein (IL-18BP), induced through IFN-γ. Thus, serum levels of IL-18BP are predictive of clinical IFN-γ activity. The intravenous administration of recombinant human IL-18 (rhIL-18) to patients with advanced cancers resulted in increased serum concentrations of IL-18 binding protein in a dose-dependent manner, as well as increases in IFN-γ, GM-CSF, and soluble Fas ligand (see, *e.g.,* Robertson et al. (2006) Clin. Cancer Res. 12(14):4265-4273). Additionally, the levels of IL-18BP in urine and serum were observed to correlate with tumor status in patients with prostate cancer; significant differences in urinary IL-18BP levels were found between cases with and without prostate cancer, and increased serum IL-18BP levels correlated with increasing prostate cancer Gleason score, demonstrating that elevated IL-18BP secretion from prostate cancer cells can be indicative of an attempt by cancer to escape immune surveillance (see, *e.g.,* Fujita et al. (2011) Int. J. Cancer 129(2):424-432). Thus, IL-18BP can be used as a biomarker for tumor immune responses.

### 2. Tumors

The immunostimulatory bacteria, combinations, uses, and methods provided herein are applicable to treating all types of tumors, including cancers, particularly solid tumors, including lung cancer, bladder cancer, non-small cell lung cancer, gastric cancers, head and neck cancers, ovarian cancer, liver cancer, pancreatic cancer, kidney cancer, breast cancer, colorectal cancer, and prostate cancer. The methods also can be used for treating hematological cancers.

Tumors and cancers subject to treatment by the immunostimulatory bacteria, compositions, combinations, uses, and methods provided herein include, but are not limited to, those that originate in the immune system, skeletal system, muscles and heart, breast, pancreas, gastrointestinal tract, central and peripheral nervous system, renal system, reproductive system, respiratory system, skin, connective tissue systems, including joints, fatty tissues, and the circulatory system, including blood vessel walls. Examples of tumors that can be treated with the immunostimulatory bacteria provided herein include carcinomas, gliomas, sarcomas (including liposarcoma), adenocarcinomas, adenosarcomas, and adenomas. Such tumors can occur in virtually all parts of the body, including, for example, the breast, heart, lung, small intestine, colon, spleen, kidney, bladder, head and neck, ovary, prostate, brain, pancreas, skin, bone, bone marrow, blood, thymus, uterus, testicles, cervix, or liver.

Tumors of the skeletal system include, for example, sarcomas and blastomas, such as osteosarcoma, chondrosarcoma, and chondroblastoma. Muscle and heart tumors include tumors of both skeletal and smooth muscles, *e.g.*, leiomyomas (benign tumors of smooth muscle), leiomyosarcomas, rhabdomyomas (benign tumors of skeletal muscle), rhabdomyosarcomas, and cardiac sarcomas. Tumors of the gastrointestinal tract include, *e.g.*, tumors of the mouth, esophagus, stomach, small intestine, and colon, and colorectal tumors, as well as tumors of gastrointestinal secretory organs, such as the salivary glands, liver, pancreas, and the biliary tract. Tumors of the central nervous system (CNS) include tumors of the brain, retina, and spinal cord, and can also originate in associated connective tissue, bone, blood vessels, or nervous tissue. Treatment of tumors of the peripheral nervous system are also contemplated. Tumors of the peripheral nervous system include malignant peripheral nerve sheath tumors. Tumors of the renal system include those of the kidneys, *e.g.*, renal cell carcinoma, as well as tumors of the ureters and bladder. Tumors of the reproductive system include tumors of the cervix, uterus, ovary, prostate, testes, and related secretory glands. Tumors of the immune system include both blood-based and solid tumors, including lymphomas, *e.g.*, both Hodgkin's and non-Hodgkin's lymphomas. Tumors of the respiratory system include tumors of the nasal passages, bronchi, and lungs. Tumors of the breast include, *e.g.*, both lobular and ductal carcinomas.

Other examples of tumors that can be treated by the immunostimulatory bacteria and methods provided herein include Kaposi's sarcoma, CNS neoplasms, neuroblastomas, capillary hemangioblastomas, meningiomas and cerebral metastases, melanoma, gastrointestinal and renal carcinomas and sarcomas, rhabdomyosarcoma, glioblastoma (such as glioblastoma multiforme), and leiomyosarcoma. Examples of other cancers that can be treated as provided herein include, but are not limited to, lymphoma, blastoma, neuroendocrine tumors, mesothelioma, schwannoma, meningioma, melanoma, and leukemia or lymphoid malignancies. Examples of such cancers include hematologic malignancies, such as Hodgkin's lymphoma, non-Hodgkin's lymphomas (Burkitt's lymphoma, small lymphocytic lymphoma, chronic lymphocytic leukemia, mycosis fungoides, mantle cell lymphoma, follicular lymphoma, diffuse large B-cell lymphoma, marginal zone lymphoma, hairy cell leukemia, and lymphoplasmacytic leukemia), tumors of lymphocyte precursor cells, including B-cell acute lymphoblastic leukemia/lymphoma, and T-cell acute lymphoblastic leukemia/lymphoma, thymoma, tumors of the mature T and NK cells, including peripheral T-cell leukemias, adult T-cell leukemia/T-cell lymphomas and large granular lymphocytic leukemia, Langerhans cell histiocytosis, myeloid neoplasias such as acute myelogenous leukemias, including acute myeloid leukemia (AML) with maturation, AML without differentiation, acute promyelocytic leukemia, acute myelomonocytic leukemia, and acute monocytic leukemias, myelodysplastic syndromes, and chronic myeloproliferative disorders, including chronic myelogenous leukemia; tumors of the central nervous system, such as glioma, glioblastoma, neuroblastoma, astrocytoma, medulloblastoma, ependymoma, and retinoblastoma; solid tumors of the head and neck (*e.g.,* nasopharyngeal cancer, salivary gland carcinoma, and esophageal cancer), lung (*e.g.,* small-cell lung cancer, non-small cell lung cancer, adenocarcinoma of the lung, and squamous carcinoma of the lung), digestive system (*e.g.,* gastric or stomach cancer, including gastrointestinal cancer, cancer of the bile duct or biliary tract, colon cancer, rectal cancer, colorectal cancer, and anal carcinoma), reproductive system (*e.g.,* testicular, penile, prostate, uterine, vaginal, vulval, cervical, ovarian, and endometrial cancers), skin (*e.g.,* melanoma, basal cell carcinoma, squamous cell cancer, actinic keratosis, and cutaneous melanoma), liver (*e.g.,* liver cancer, hepatic carcinoma, hepatocellular cancer, and hepatoma), bone (*e.g.,* osteoclastoma, and osteolytic bone cancers), additional tissues and organs (*e.g.,* pancreatic cancer, bladder cancer, kidney or renal cancer, thyroid cancer, breast cancer, cancer of the peritoneum, and Kaposi's sarcoma), tumors of the vascular system (*e.g.,* angiosarcoma and hemangiopericytoma), Wilms' tumor, retinoblastoma, osteosarcoma, and Ewing's sarcoma.

### 3. Administration

In practicing the uses and methods herein, immunostimulatory bacteria provided herein can be administered to a subject, including a subject having a tumor or having neoplastic cells, or a subject to be immunized. One or more steps can be performed prior to, simultaneously with, or after administration of the immunostimulatory bacteria to the subject, including, but not limited to, diagnosing the subject with a condition appropriate for administering immunostimulatory bacteria, determining the immunocompetence of the subject, immunizing the subject, treating the subject with a chemotherapeutic agent, treating the subject with radiation, or surgically treating the subject.

For embodiments that include administering immunostimulatory bacteria to a tumor-bearing subject for therapeutic purposes, the subject typically has previously been diagnosed with a neoplastic condition. Diagnostic methods also can include determining the type of neoplastic condition, determining the stage of the neoplastic condition, determining the size of one or more tumors in the subject, determining the presence or absence of metastatic or neoplastic cells in the lymph nodes of the subject, or determining the presence of metastases in the subject.

Some embodiments of the therapeutic methods for administering immunostimulatory bacteria to a subject can include a step of determining the size of the primary tumor or the stage of the neoplastic disease, and, if the size of the primary tumor is equal to or above a threshold volume, or if the stage of the neoplastic disease is at or above a threshold stage, an immunostimulatory bacterium is administered to the subject. In a similar embodiment, if the size of the primary tumor is below a threshold volume, or if the stage of the neoplastic disease is at or below a threshold stage, the immunostimulatory bacterium is not yet administered to the subject; such methods can include monitoring the subject until the tumor size or neoplastic disease stage reaches a threshold amount, and then administering the immunostimulatory bacterium to the subject. Threshold sizes can vary according to several factors, including rate of growth of the tumor, ability of the immunostimulatory bacterium to infect a tumor, and immunocompetence of the subject. Generally, the threshold size will be a size sufficient for an immunostimulatory bacterium to accumulate and replicate in or near the tumor, without being completely removed by the host's immune system, and will typically also be a size sufficient to sustain a bacterial infection for a time long enough for the host to mount an immune response against the tumor cells, typically about one week or more, about ten days or more, or about two weeks or more. Exemplary threshold stages are any stage beyond the lowest stage (*e.g.,* Stage I or equivalent), or any stage where the primary tumor is larger than a threshold size, or any stage where metastatic cells are detected.

Any mode of administration of a microorganism to a subject can be used, provided the mode of administration permits the immunostimulatory bacteria to enter a tumor or metastasis. Modes of administration can include, but are not limited to, intravenous, intraperitoneal, subcutaneous, intramuscular, topical, intratumoral, multipuncture, inhalation, intranasal, oral, intracavity (*e.g.,* administering to the bladder via a catheter, or administering to the gut by suppository or enema), aural, rectal, and ocular administration.

One skilled in the art can select any mode of administration compatible with the subject and the bacteria, and that also is likely to result in the bacteria reaching tumors and/or metastases. The route of administration can be selected by one skilled in the art according to any of a variety of factors, including the nature of the disease, the kind of tumor, and the particular bacteria contained in the pharmaceutical composition. Administration to the target site can be performed, for example, by ballistic delivery, or as a colloidal dispersion system, or systemic administration can be performed by injection into an artery.

The dosage regimen can be any of a variety of methods and amounts, and can be determined by one skilled in the art according to known clinical factors. A single dose can be therapeutically effective for treating a disease or disorder in which immune stimulation effects treatment. Exemplary of such stimulation is an immune response, that includes, but is not limited to, one or both of a specific immune response and non-specific immune response, both specific and non-specific responses, innate response, primary immune response, adaptive immunity, secondary immune response, memory immune response, immune cell activation, immune cell proliferation, immune cell differentiation, and cytokine expression.

As is known in the medical arts, dosages for a subject can depend on many factors, including the subject's species, size, body surface area, age, sex, immunocompetence, and general health, the particular bacteria to be administered, the duration and route of administration, the kind and stage of the disease, for example, the tumor size, and other compounds, such as drugs, being administered concurrently. In addition to the above factors, such levels can be affected by the infectivity of the bacteria and the nature of the bacteria, as can be determined by one skilled in the art. In the present methods, appropriate minimum dosage levels of bacteria can be levels sufficient for the bacteria to survive, grow, and replicate in a tumor or metastasis. Exemplary minimum levels for administering a bacterium to a 65 kg human can include at least about 5 x 10⁶ colony forming units (CFUs), at least about 1 x 10⁷ CFUs, at least about 5 x 10⁷ CFUs, at least about 1 x 10⁸ CFUs, or at least about 1 x 10⁹ CFUs. In the present methods, appropriate maximum dosage levels of bacteria can be levels that are not toxic to the host, levels that do not cause splenomegaly of 3x or more, or levels that do not result in colonies or plaques in normal tissues or organs after about 1 day, or after about 3 days, or after about 7 days. Exemplary maximum levels for administering a bacterium to a 65 kg human can include no more than about 5 x 10¹¹ CFUs, no more than about 1 x 10¹¹ CFUs, no more than about 5 x 10¹⁰ CFUs, no more than about 1 x 10¹⁰ CFUs, or no more than about 1 x 10⁹ CFUs.

The methods and uses provided herein can include a single administration of immunostimulatory bacteria to a subject, or multiple administrations of immunostimulatory bacteria to a subject, or others of a variety of regimens, including combination therapies with other anti-tumor therapeutics and/or treatments. These include, for example, cellular therapies, such as administration of modified immune cells; CAR-T therapy; CRISPR therapy; checkpoint inhibitors, such as antibodies (*e.g.,* anti-PD-1 antibodies, anti-PD-L1 antibodies, and anti-CTLA-4 antibodies, and other such immunotherapies); chemotherapeutic compounds, such as nucleoside analogs; surgery; and radiotherapy. Other cancer therapies also include anti-VEGF, anti-VEGFR, anti-VEGFR2, anti-TGF-β or anti-IL-6 antibodies, or fragments thereof, cancer vaccines, and oncolytic viruses.

In some embodiments, a single administration is sufficient to establish immunostimulatory bacteria in a tumor, where the bacteria can colonize, and can cause or enhance an anti-tumor response in the subject. In other embodiments, the immunostimulatory bacteria provided for use in the methods herein can be administered on different occasions, separated in time, typically by at least one day. Separate administrations can increase the likelihood of delivering a bacterium to a tumor or metastasis, where a previous administration may have been ineffective in delivering the bacterium to a tumor or metastasis. In embodiments, separate administrations can increase the locations on a tumor or metastasis where bacterial colonization/proliferation can occur, or can otherwise increase the titer of bacteria accumulated in the tumor, which can increase eliciting or enhancing a host's anti-tumor immune response.

When separate administrations are performed, each administration can be a dosage amount that is the same or different relative to other administration dosage amounts. In one embodiment, all administration dosage amounts are the same. In other embodiments, a first dosage amount can be a larger dosage amount than one or more subsequent dosage amounts, for example, at least 10x larger, at least 100x larger, or at least 1000x larger, than subsequent dosage amounts. In one example of a method of separate administrations, in which the first dosage amount is greater than one or more subsequent dosage amounts, all subsequent dosage amounts can be the same, smaller amount, relative to the first administration.

Separate administrations can include any number of two or more administrations, including two, three, four, five, or six administrations. One skilled in the art readily can determine the number of administrations to perform, or the desirability of performing one or more additional administrations, according to methods known in the art for monitoring therapeutic methods, and other monitoring methods provided herein. Accordingly, the methods provided herein include methods of providing to the subject one or more administrations of immunostimulatory bacteria, where the number of administrations can be determined by monitoring the subject, and, based on the results of the monitoring, determining whether or not to provide one or more additional administrations. Deciding whether or not to provide one or more additional administrations can be based on a variety of monitoring results, including, but not limited to, indication of tumor growth or inhibition of tumor growth, appearance of new metastases or inhibition of metastasis, the subject's anti-bacterial antibody titer, the subject's anti-tumor antibody titer, the overall health of the subject, and the weight of the subject.

The time period between administrations can be any of a variety of time periods. The time period between administrations can be a function of any of a variety of factors, including monitoring steps, as described in relation to the number of administrations, the time period for a subject to mount an immune response, the time period for a subject to clear bacteria from normal tissue, or the time period for bacterial colonization/proliferation in the tumor or metastasis. In one example, the time period can be a function of the time period for a subject to mount an immune response; for example, the time period can be more than the time period for a subject to mount an immune response, such as more than about one week, more than about ten days, more than about two weeks, or more than about a month. In another example, the time period can be less than the time period for a subject to mount an immune response, such as less than about one week, less than about ten days, less than about two weeks, or less than about a month. In another example, the time period can be a function of the time period for bacterial colonization/proliferation in the tumor or metastasis; for example, the time period can be more than the amount of time for a detectable signal to arise in a tumor or metastasis after administration of a microorganism expressing a detectable marker, such as about 3 days, about 5 days, about a week, about ten days, about two weeks, or about a month.

The methods used herein also can be performed by administering compositions, such as suspensions and other formulations, containing the immunostimulatory bacteria provided herein. Such compositions contain the bacteria and a pharmaceutically acceptable excipient or vehicle, as provided herein or known to those of skill in the art.

As discussed above, the uses and methods provided herein also can include administering one or more therapeutic compounds, such as anti-tumor compounds or other cancer therapeutics, to a subject, in addition to administering the immunostimulatory bacteria to the subject. The therapeutic compounds can act independently, or in conjunction with the immunostimulatory bacteria, for tumor therapeutic effects. Therapeutic compounds that can act independently include any of a variety of known chemotherapeutic compounds that can inhibit tumor growth, inhibit metastasis growth and/or formation, decrease the size of a tumor or metastasis, or eliminate a tumor or metastasis, without reducing the ability of the immunostimulatory bacteria to accumulate in a tumor, replicate in the tumor, and cause or enhance an anti-tumor immune response in the subject. Examples of such chemotherapeutic agents include, but are not limited to, alkylating agents, such as thiotepa and cyclophosphamide; alkyl sulfonates, such as busulfan, improsulfan, and piposulfan; androgens, such as calusterone, dromostanolone propionate, epitiostanol, mepitiostane, and testolactone; anti-adrenals, such as aminoglutethimide, mitotane, and trilostane; anti-androgens, such as flutamide, nilutamide, bicalutamide, leuprolide, and goserelin; antibiotics, such as aclacinomycin, actinomycin, anthramycin, azaserine, bleomycin, cactinomycin, calicheamicin, carubicin, carminomycin, carzinophilin, chromomycin, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, porfiromycin, puromycin, quelamycin, rodorubicin, streptonigrin, streptozocin, tubercidin, ubenimex, zinostatin, and zorubicin; antiestrogens, including, for example, tamoxifen, raloxifene, aromatase inhibiting 4(5)-imidazoles, 4-hydroxytamoxifen, trioxifene, keoxifene, LY 117018, onapristone, and toremifene (Fareston^{®}); anti-metabolites, such as methotrexate and 5-fluorouracil (5-FU); folic acid analogues, such as denopterin, methotrexate, pteropterin, and trimetrexate; aziridines, such as benzodepa, carboquone, meturedepa, and uredepa; ethylenimines and methylmelamines, including altretamine, triethylenemelamine, triethylenephosphoramide, triethylenethiophosphoramide, and trimethylol melamine; folic acid replenishers, such as folinic acid; nitrogen mustards, such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, and uracil mustard; nitrosoureas, such as carmustine, chlorozotocin, fotemustine, lomustine, nimustine, and ranimustine; platinum analogs, such as cisplatin and carboplatin; vinblastine; platinum; proteins, such as arginine deiminase and asparaginase; purine analogs, such as fludarabine, 6-mercaptopurine, thiamiprine, and thioguanine; pyrimidine analogs, such as ancitabine, azacitidine, 6-azauridine, carmofur, cytarabine, dideoxyuridine, doxifluridine, enocitabine, floxuridine, and 5-FU; taxanes, such as paclitaxel and docetaxel, and albuminated forms thereof (*i.e.,* nab-paclitaxel and *nab*-docetaxel); topoisomerase inhibitors, such as RFS-2000; thymidylate synthase inhibitors, such as Tomudex^{®}; and additional chemotherapeutics, including aceglatone; aldophosphamide glycoside; aminolevulinic acid; amsacrine; bestrabucil; bisantrene; edatrexate; defosfamide; demecolcine; diaziquone; difluoromethylomithine (DFMO); eflornithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK^{®}; razoxane; sizofiran; spirogermanium; tenuazonic acid; triaziquone; 2,2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; chlorambucil; gemcitabine; 6-thioguanine; mercaptopurine; methotrexate; etoposide (VP-16); ifosfamide; mitomycin C; mitoxantrone; vincristine; vinorelbine; Navelbine; Novantrone; teniposide; daunomycin; aminopterin; Xeloda^{®}; ibandronate; CPT-11; retinoic acid; esperamycins; capecitabine; and topoisomerase inhibitors, such as irinotecan. Pharmaceutically acceptable salts, acids, or derivatives of any of the above also can be used.

Therapeutic compounds that act in conjunction with the immunostimulatory bacteria include, for example, compounds that increase the immune response eliciting properties of the bacteria, *e.g.*, by increasing expression of encoded therapeutic products, such as cytokines, chemokines, co-stimulatory molecules, proteins that constitutively induce type I IFNs, RNAi molecules that inhibit, suppress, or disrupt expression of checkpoint gene(s), a checkpoint inhibitor antibody and antibodies or fragments thereof against other targets, or compounds that can further augment bacterial colonization/proliferation. For example, a gene expression-altering compound can induce or increase transcription of a gene in a bacterium, such as an exogenous gene encoded on the plasmid, thereby provoking an immune response. Any of a wide variety of compounds that can alter gene expression are known in the art, including IPTG and RU486. Exemplary genes whose expression can be up-regulated include those encoding proteins and RNA molecules, including toxins, enzymes that can convert a prodrug to an anti-tumor drug, cytokines, transcription regulating proteins, shRNA, siRNA, and ribozymes. In other embodiments, therapeutic compounds that can act in conjunction with the immunostimulatory bacteria to increase the colonization/proliferation or immune response eliciting properties of the bacteria, are compounds that can interact with a bacterially-encoded gene product, and such interaction can result in an increased killing of tumor cells, or an increased anti-tumor immune response in the subject. A therapeutic compound that can interact with a bacterially-encoded gene product can include, for example, a prodrug or other compound that has little or no toxicity, or other biological activity in its subject-administered form, but after interaction with a bacterially-encoded gene product, the compound can develop a property that results in tumor cell death, including but not limited to, cytotoxicity, the ability to induce apoptosis, or the ability to trigger an immune response. A variety of prodrug-like substances are known in the art, including ganciclovir, 5-fluorouracil, 6-methylpurine deoxyriboside, cephalosporin-doxorubicin, 4-[(2-chloroethyl)(2-mesuloxyethyl)amino]benzoyl-L-glutamic acid, acetaminophen, indole-3-acetic acid, CB1954, 7-ethyl-10-[4-(1-piperidino)-1-piperidino]carbonyloxycamptothecin, bis-(2-chloroethyl)amino-4-hydroxyphenylaminomethanone 28, 1-chloromethyl-5-hydroxy-1,2-dihyro-3H-benz[e]indole, epirubicin-glucuronide, 5'-deoxy5-fluorouridine, cytosine arabinoside, and linamarin.

### 4. Monitoring

The methods provided herein can further include one or more steps of monitoring the subject, monitoring the tumor, and/or monitoring the immunostimulatory bacteria administered to the subject. Any of a variety of monitoring steps can be included in the methods provided herein, including, but not limited to, monitoring tumor size, monitoring the presence and/or size of metastases, monitoring the subject's lymph nodes, monitoring the subject's weight or other health indicators, including blood or urine markers, monitoring anti-bacterial antibody titer, monitoring bacterial expression of a detectable gene product, and directly monitoring bacterial titer in a tumor, tissue, or organ of a subject.

The purpose of the monitoring can be simply for assessing the health state of the subject, or the progress of therapeutic treatment of the subject, or can be for determining whether or not further administration of the same or a different immunostimulatory bacterium is warranted, or for determining when or whether or not to administer a compound to the subject where the compound can act to increase the efficacy of the therapeutic method, or the compound can act to decrease the pathogenicity of the bacteria administered to the subject.

In some embodiments, the methods provided herein can include monitoring one or more bacterially-expressed genes. Bacteria, such as those provided herein or otherwise known in the art, can express one or more detectable gene products, including but not limited to, detectable proteins.

As provided herein, measurement of a detectable gene product expressed in a bacterium can provide an accurate determination of the level of bacteria present in the subject. As further provided herein, measurement of the location of the detectable gene product, for example, by imaging methods, including tomographic methods, can determine the localization of the bacteria in the subject. Accordingly, the methods provided herein that include monitoring a detectable bacterial gene product can be used to determine the presence or absence of the bacteria in one or more organs or tissues of a subject, and/or the presence or absence of the bacteria in a tumor or metastases of a subject. Further, the methods provided herein that include monitoring a detectable bacterial gene product can be used to determine the titer of bacteria present in one or more organs, tissues, tumors, or metastases. Methods that include monitoring the localization and/or titer of bacteria in a subject can be used for determining the pathogenicity of bacteria, since bacterial infection, and particularly the level of infection, of normal tissues and organs can indicate the pathogenicity of the bacteria. The methods that include monitoring the localization and/or titer of the immunostimulatory bacteria in a subject can be performed at multiple time points and, accordingly, can determine the rate of bacterial replication in a subject, including the rate of bacterial replication in one or more organs or tissues of a subject; accordingly, methods that include monitoring a bacterial gene product can be used for determining the replication competence of the bacteria. The methods provided herein also can be used to quantitate the amount of immunostimulatory bacteria present in a variety of organs or tissues, and tumors or metastases, and can thereby indicate the degree of preferential accumulation of the bacteria in a subject; accordingly, the bacterial gene product monitoring can be used in methods of determining the ability of the bacteria to accumulate in tumors or metastases, in preference to normal tissues or organs. Since the immunostimulatory bacteria used in the methods provided herein can accumulate in an entire tumor, or can accumulate at multiple sites in a tumor, and can also accumulate in metastases, the methods provided herein for monitoring a bacterial gene product can be used to determine the size of a tumor, or the number of metastases present in a subject. Monitoring such presence of a bacterial gene product in a tumor or metastasis over a range of time can be used to assess changes in the tumor or metastasis, including growth or shrinking of a tumor, or development of new metastases, or disappearance of metastases, and also can be used to determine the rate of growth or shrinking of a tumor, or the rate of development of new metastases or disappearance of metastases, or the change in the rate of growth or shrinking of a tumor, or the change in the rate of development of new metastases or disappearance of metastases. Accordingly, monitoring a bacterial gene product can be used for monitoring a neoplastic disease in a subject, or for determining the efficacy of treatment of a neoplastic disease, by determining the rate of growth or shrinking of a tumor, or the development of new metastases or disappearance of metastases, or the change in the rate of growth or shrinking of a tumor, or the development of new metastases or disappearance of metastases.

Any of a variety of detectable proteins can be detected by monitoring, exemplary of which are any of a variety of fluorescent proteins (*e.g.,* green fluorescent proteins), any of a variety of luciferases, transferrin, or other iron-binding proteins; or receptors, binding proteins, and antibodies, where a compound that specifically binds the receptor, binding protein or antibody can be a detectable agent, or can be labeled with a detectable substance (*e.g.,* a radionuclide or imaging agent).

Tumor and/or metastasis size can be monitored by any of a variety of methods known in the art, including external assessment methods, or tomographic or magnetic imaging methods. In addition to the methods known in the art, methods provided herein, for example, monitoring bacterial gene expression, can be used for monitoring tumor and/or metastasis size.

Monitoring size over several time points can provide information regarding the increase or decrease in the size of a tumor or metastasis, and can also provide information regarding the presence of additional tumors and/or metastases in the subject. Monitoring tumor size over several time points can provide information regarding the development of a neoplastic disease in a subject, including the efficacy of treatment of a neoplastic disease in a subject.

The methods provided herein also can include monitoring the antibody titer in a subject, including antibodies produced in response to administration of the immunostimulatory bacteria to a subject. The bacteria administered in the methods provided herein can elicit an immune response to endogenous bacterial antigens. The bacteria administered in the methods provided herein also can elicit an immune response to exogenous genes expressed by the bacteria. The bacteria administered in the methods provided herein also can elicit an immune response to tumor antigens. Monitoring antibody titer against bacterial antigens, bacterially-expressed exogenous gene products, or tumor antigens, can be used to monitor the toxicity of the bacteria, to monitor the efficacy of treatment methods, or to monitor the level of gene product(s) or antibodies for production and/or harvesting.

Monitoring antibody titer can be used to monitor the toxicity of the bacteria. Antibody titer against a bacteria can vary over the time period after administration of the bacteria to the subject, where at some particular time points, a low anti-(bacterial antigen) antibody titer can indicate a lower toxicity, while at other time points, a high anti-(bacterial antigen) antibody titer can indicate a higher toxicity. The bacteria used in the methods provided herein can be immunogenic, and can, therefore, elicit an immune response soon after administering the bacteria to the subject. Generally, immunostimulatory bacteria against which the immune system of a subject can mount a strong immune response can be bacteria that have low toxicity when the subject's immune system can remove the bacteria from all normal organs or tissues. Thus, in some embodiments, a high antibody titer against bacterial antigens soon after administering the bacteria to a subject can indicate low toxicity of the bacteria.

In other embodiments, monitoring antibody titer can be used to monitor the efficacy of treatment methods. In the methods provided herein, antibody titer, such as anti-(tumor antigen) antibody titer, can indicate the efficacy of a therapeutic method, such as a therapeutic method to treat neoplastic disease. Therapeutic methods provided herein can include causing or enhancing an immune response against a tumor and/or metastasis. Thus, by monitoring the anti-(tumor antigen) antibody titer, it is possible to monitor the efficacy of a therapeutic method in causing or enhancing an immune response against a tumor and/or metastasis.

In other embodiments, monitoring antibody titer can be used for monitoring the level of gene product(s) or antibodies for production and/or harvesting. As provided herein, methods can be used for producing proteins, RNA molecules, or other compounds, by expressing an exogenous gene in a microorganism that has accumulated in a tumor, in the tumor microenvironment, and/or in tumor-resident immune cells. Monitoring antibody titer against the protein, RNA molecule, or other compound can indicate the level of production of the protein, RNA molecule, or other compound by the tumor-accumulated microorganism, and also, can directly indicate the level of antibodies specific for such a protein, RNA molecule, or other compound.

The methods provided herein also can include methods of monitoring the health of a subject. Some of the methods provided herein are therapeutic methods, including neoplastic disease therapeutic methods. Monitoring the health of a subject can be used to determine the efficacy of the therapeutic method, as is known in the art. The methods provided herein also can include a step of administering to a subject an immunostimulatory bacterium, as provided herein. Monitoring the health of a subject can be used to determine the pathogenicity of an immunostimulatory bacterium administered to a subject. Any of a variety of health diagnostic methods for monitoring disease, such as neoplastic disease, infectious disease, or immune-related disease, can be monitored, as is known in the art. For example, the weight, blood pressure, pulse, breathing, color, temperature, or other observable state of a subject can indicate the health of a subject. In addition, the presence, or absence, or level of one or more components in a sample from a subject can indicate the health of a subject. Typical samples can include blood and urine samples, where the presence, or absence, or level of one or more components can be determined by performing, for example, a blood panel or a urine panel diagnostic test. Exemplary components indicative of a subject's health include, but are not limited to, white blood cell count, hematocrit, and c-reactive protein concentration.

The methods provided herein can include monitoring a therapy, where therapeutic decisions can be based on the results of the monitoring. Therapeutic methods provided herein can include administering to a subject immunostimulatory bacteria, where the bacteria can preferentially accumulate in a tumor, the tumor microenvironment, or in tumor-resident immune cells, and/or in metastases, and where the bacteria can cause or enhance an anti-tumor immune response. Such therapeutic methods can include a variety of steps, including multiple administrations of a particular immunostimulatory bacterium, administration of a second immunostimulatory bacterium, or administration of a therapeutic compound. Determination of the amount, timing, or type of immunostimulatory bacteria or compound to administer to the subject can be based on one or more results from monitoring the subject. For example, the antibody titer in a subject can be used to determine whether or not it is desirable to administer an immunostimulatory bacterium and, optionally, a compound, the quantity of bacteria and/or compound to administer, and the type of bacteria and/or compound to administer, where, for example, a low antibody titer can indicate the desirability of administering an additional immunostimulatory bacterium, a different immunostimulatory bacterium, and/or a therapeutic compound, such as a compound that induces bacterial gene expression, or a therapeutic compound that is effective independent of the immunostimulatory bacteria.

In another example, the overall health state of a subject can be used to determine whether or not it is desirable to administer an immunostimulatory bacterium and, optionally, a compound, the quantity of bacterium and/or compound to administer, and the type of bacterium and/or compound to administer, where, for example, determining that the subject is healthy can indicate the desirability of administering additional bacteria, different bacteria, or a therapeutic compound, such as a compound that induces bacterial gene/genetic payload/therapeutic product expression. In another example, monitoring a detectable bacterially-expressed gene product can be used to determine whether it is desirable to administer an immunostimulatory bacterium and, optionally, a compound, the quantity of bacterium and/or compound to administer, and the type of bacterium and/or compound to administer, where, for example, determining that the subject is healthy can indicate the desirability of administering additional bacteria, different bacteria, or a therapeutic compound, such as a compound that induces bacterial gene/genetic payload/therapeutic product expression. Such monitoring methods can be used to determine whether or not the therapeutic method is effective, whether or not the therapeutic method is pathogenic to the subject, whether or not the bacteria have accumulated in a tumor or metastasis, and whether or not the bacteria have accumulated in normal tissues or organs. Based on such determinations, the desirability and form of further therapeutic methods can be derived.

In another example, monitoring can determine whether or not immunostimulatory bacteria have accumulated in a tumor or metastasis of a subject. Upon such a determination, a decision can be made to further administer additional bacteria, a different immunostimulatory bacterium, and, optionally, a compound to the subject.

### H. EXAMPLES

The following examples are included for illustrative purposes only and are not intended to limit the scope of the invention.

### Example 1

### Auxotrophic Strains of S. typhimurium

### The Salmonella Strain YS1646 is Auxotrophic for Adenosine

Strains provided herein are engineered to be auxotrophic for adenosine. As a result, they are attenuated *in vivo* because they are unable to replicate in the low adenosine concentrations of normal tissue, and colonization occurs primarily in the solid tumor microenvironment (TME), where adenosine levels are high. The *Salmonella* strain YS1646 is a derivative of the wild-type strain ATCC 14028, and was engineered to be auxotrophic for purines due to disruption of the *purI* gene (synonymous with *purM*) (see, *e.g.,* Low et al. (2004) Methods Mol. Med. 90:47-60). Subsequent analysis of the entire genome of YS1646 demonstrated that the *purI* gene was not in fact deleted, but was instead disrupted by a chromosomal inversion (see, *e.g.,* Broadway et al. (2014) J. Biotechnol. 192:177-178), and that the entire gene is still contained within two parts of the YS1646 chromosome that is flanked by insertion sequences, one of which has an active transposase. The presence of the complete genetic sequence of the *purI* gene, disrupted by means of a chromosomal reengagement, leaves open the possibility of reversion to a wild-type gene. While it has previously been demonstrated that the purine auxotrophy of YS1646 was stable after >140 serial passages *in vitro,* it was not clear what the reversion rate is (see, *e.g.,* Clairmont et al. (2000) J. Infect. Dis. 181:1996-2002).

It is shown herein that, when provided with adenosine, strain YS1646 is able to replicate in minimal medium, whereas the wild-type parental strain, ATCC 14028, can grow in minimal media that is not supplemented with adenosine. Strain YS1646 was grown overnight in lysogeny broth (LB) medium, washed with M9 minimal medium, and diluted into M9 minimal medium containing no adenosine, or increasing concentrations of adenosine. Growth was measured using a SpectraMax^{®} M3 Spectrophotometer (Molecular Devices) at 37 °C, reading the OD₆₀₀ every 15 minutes.

The results showed that, unlike a wild-type strain (ATCC 14028), which was able to grow in all concentrations of adenosine, strain YS1646 only was able to replicate when adenosine was provided at concentrations ranging from 11 to 300 micromolar, and was completely unable to replicate in M9 alone, or in M9 supplemented with 130 nanomolar adenosine. These data demonstrate that *purI* mutants are able to replicate at concentrations of adenosine that are found in the tumor microenvironment, but not at concentrations found in normal tissues. Engineered adenosine auxotrophic strains exemplified herein include strains in which all or portions of the *purI* open reading frame are deleted from the chromosome to prevent reversion to wild-type. Such gene deletions can be achieved by any method known to one of skill in the art, including the lambda red system, as described below.

### The Salmonella Strain YS1646 is Auxotrophic for ATP

In addition to the purine and adenosine auxotrophy, it was determined whether the *purI-*deleted strain also can scavenge ATP. ATP accumulates to high levels in the tumor microenvironment, due to leakage from dying tumor cells. It is shown herein that, when provided with ATP, strain YS1646 is able to replicate in minimal media, but is unable to grow when not supplemented with ATP. To demonstrate this, strain YS1646 was grown overnight in LB medium, washed with M9 minimal medium, and diluted into M9 minimal medium containing no ATP, or increasing concentrations of ATP (Fisher). Growth was measured using a SpectraMax^{®} M3 Spectrophotometer (Molecular Devices) at 37 °C, reading the OD₆₀₀ every 15 minutes. The results demonstrated that strain YS1646 is able to replicate when ATP is provided at concentrations of 0.012 mM, but not in M9 alone.

### Example 2

### Defects in Intracellular Replication are Attributed to the msbB Mutation

The YS1646 strain contains mutations in *purI*, which limits replication to sites containing high concentrations of purines, adenosine, or ATP, and mutations in *msbB,* which alters the lipopolysaccharide (LPS) surface coat in order to reduce TLR4-mediated pro-inflammatory signaling. It also has been established that, unlike wild-type *Salmonella,* strain YS1646 is unable to replicate in macrophages. Experiments were performed to determine which of these genetic mutations is responsible for conferring that phenotype within the wild-type strain, ATCC 14028.

In this assay, mouse RAW macrophage cells (InvivoGen, San Diego, Ca.) were infected with wild-type *Salmonella* strains containing deletions in *purI, msbB,* or both, at a multiplicity of infection (MOI) of approximately 5 bacteria per cell for 30 minutes, then the cells were washed with PBS, and medium containing gentamicin was added to kill extracellular bacteria. Intracellular bacteria are not killed by gentamicin, as it cannot cross the cell membrane. At various time points after infection, cell monolayers were lysed by osmotic shock with water, and the cell lysates were diluted and plated on LB agar to enumerate surviving colony forming units (CFUs).

As shown in the table below, wild-type *Salmonella* strains containing only the *purI⁻* mutation still were able to replicate. This explains why there is only a modest improvement in tolerability observed with the *purI* deletion alone, while achieving a high degree of specificity to the tumor microenvironment. Strains containing only the *msbB⁻* mutation, as well as strains containing the *purI⁻* and *msbB⁻* mutations, were unable to replicate, and were rapidly cleared from cells within 48 hours.

| **Hours** | **CFUs/Well** | | | | | |
|---|---|---|---|---|---|---|
| | **ATCC 14028 Δ*purI*** | | **ATCC 14028 Δ*purI*/Δ*msbB*** | | **ATCC 14028 Δ*msbB*** | |
| **1** | 104000 | 108000 | 68000 | 68000 | 88000 | 40000 |
| **2.5** | 5600 | 6000 | 760 | 960 | 3200 | 3200 |
| **5** | 5600 | 4000 | 1120 | 880 | 800 | 680 |
| **27** | 11200 | 5600 | 4 | 4 | 20 | 4 |

### Example 3

### Salmonella asd Gene Knockout Strain Engineering and Characterization

Strain YS1646Δ*asd* was prepared. It is an attenuated *Salmonella typhimurium* strain derived from strain YS1646 (which can be purchased from ATCC, Catalog # 202165) that has been engineered to have a deletion in the *asd* gene. In this example, the *Salmonella typhimurium* strain YS1646Δ*asd* was engineered using modifications of the method of Datsenko and Wanner (Proc. Natl. Acad. Sci. U.S.A. 97:6640-6645 (2000)), as described below.

### Introduction of the Lambda Red Helper Plasmid into Strain YS1646

The YS1646 strain was prepared to be electrocompetent as described previously (Sambrook J. (1998) Molecular Cloning, A Laboratory Manual, 2nd Ed., Cold Spring Harbor, NY: Cold Spring Harbor Laboratory), by growing a culture in LB and concentrating 100-fold, and then washing three times with ice-cold 10% glycerol. The electrocompetent strain was electroporated with the Lambda red helper plasmid pKD46 (SEQ ID NO:218), using a 0.2 cm gap cuvette at the following settings: 2.5 kV, 186 ohms, and 50 µF. Transformants carrying pKD46 were grown in 5 mL SOC medium with ampicillin and 1 mM L-arabinose at 30 °C, and selected on LB agar plates containing ampicillin. A YS1646 clone containing the lambda red helper plasmid pKD46 then was made electrocompetent, as described above for strain YS1646.

### Construction of asd Gene Knockout Cassette

The *asd* gene from the genome of strain YS1646 (Broadway et al. (2014) J. Biotechnology 192:177-178) was used for designing the *asd* gene knockout cassette. A plasmid containing 204 and 203 base pairs (bps) of homology to the left hand and right hand regions, respectively, of the *asd* gene, was transformed into DH5-alpha competent cells (Thermo Fisher Scientific). A kanamycin gene cassette flanked by loxP sites was cloned into this plasmid. The *asd* gene knockout cassette then was PCR amplified using primers asd-1 and asd-2 (see, Table 1), and gel purified.

### Deletion of asd gene

The YS1646 strain carrying plasmid pKD46 was electroporated with the gel-purified linear *asd* gene knock-out cassette. Electroporated cells were recovered in SOC medium and plated onto LB agar plates supplemented with kanamycin (20 µg/mL) and diaminopimelic acid (DAP, 50 µg/mL). During this step, lambda red recombinase induces homologous recombination of the chromosomal *asd* gene with the *kan* cassette (due to the presence of homologous flanking sequences upstream and downstream of the chromosomal *asd* gene), and knockout of the chromosomal copy of the *asd* gene occurs. The presence of the disrupted *asd* gene in the selected kanamycin-resistant clones was confirmed by PCR amplification, with primers from the YS1646 genome flanking the sites of disruption (primer asd-3), and from the multi-cloning site (primer scFv-3) (see, Table 1). Colonies were also replica plated onto LB plates, with and without supplemental DAP, to demonstrate DAP auxotrophy. All clones with the *asd* gene deletion were unable to grow in the absence of supplemental DAP, demonstrating DAP auxotrophy.

**Table 1. Primer Information**

| **Primer Name** | **Primer Sequence** | **SEQ ID NO.** |
|---|---|---|
| asd-1 | ccttcctaacgcaaattccctg | 219 |
| asd-2 | ccaatgctctgcttaactcctg | 220 |
| asd-3 | gcctcgccatgtttcagtacg | 221 |
| asd-4 | ggtctggtgcattccgagtac | 222 |
| scFv-3 | cataatctgggtccttggtctgc | 223 |
| APR-001 | aaaaaaRcttgcagctctggcccgtg | 226 |
| APR-002 | aaaaaagcttttagaaaaactcatcgagcatcaaatga | 227 |
| APR-003 | acactagaaggacagtatttggtatctg | 228 |
| APR-004 | agccgtagttageccacc | 229 |
| flic-1 | cgttatcggcaatctggaggc | 232 |
| flic-2 | ccagcccttacaacagtggtc | 233 |
| flic-3 | gtctgtcaacaactggtctaacgg | 234 |
| flic-4 | agacggtcctcatccagataagg | 235 |
| fljb-1 | ttccagacgacaagagtatcgc | 236 |
| fljb-2 | cctttaggtttatccgaagccagaatc | 237 |
| fljb-3 | caccaggtttttcacgctgc | 238 |
| fljb-4 | acacgcatttacgcctgtcg | 239 |
| pagp-1 | gcgtgacggttctgagtgct | 315 |
| pagp-2 | cgtctttgctgccatcttccg | 316 |
| pagp-3 | acaataacgacgactccgataagg | 317 |
| pagp-4 | ctgctgaatgtgctgattaacctg | 318 |
| ansb-1 | accttagaagatagccgcaaagc | 319 |
| ansb-2 | cagagacatgacacccacgattatc | 320 |
| ansb-3 | gcaaaccgctatccagaacga | 321 |
| ansb-4 | agtttaagtatgccgtggtactgc | 322 |
| csgd-1 | cacttgctttaagatttgtaatggctag | 323 |
| csgd-2 | ggtgtattcgctttcccatttgtc | 324 |
| csgd-3 | tgtgctgtccaggttaatgcc | 325 |
| csgd-4 | gacgacggttttctcgaagtctc | 326 |

### Kanamycin Gene Cassette Removal

The *kan* selectable marker was removed by using the Cre/loxP site-specific recombination system. The YS1646Δ*asd* gene Kan^{R} mutant was transformed with pJW168, a temperature-sensitive plasmid expressing the Cre recombinase (SEQ ID NO:224). Amp^{R} colonies were selected at 30 °C; pJW168 was subsequently eliminated by growth at 42 °C. A selected clone was tested for loss of *kan* by replica plating on LB agar plates with and without kanamycin, and confirmed by PCR verification using primers from the YS1646 genome flanking the sites of disruption (primers asd-3 and asd-4; for primer sequence, see Table 1).

### Confirmation of Functional asd Deletion Mutant Strain YS1646Δasd (also designated AST-101)

The Δ*asd* mutant was unable to grow on LB agar plates at 37 °C, but was able to grow on LB plates containing 50 µg/mL diaminopimelic acid (DAP). The Δ*asd* mutant growth rate was evaluated in LB liquid media; it was unable to grow in liquid LB, but was able to grow in LB supplemented with 50 µg/mL DAP, as determined by measuring absorbance at 600 nM.

### Sequence Confirmation of the asd Locus Sequence in Strain YS1646Δasd After asd Gene Deletion

The *asd* gene deletion strain was verified by DNA sequencing using primers asd-3 and asd-4 (see, Table 1). Sequencing of the region flanking the *asd* locus was performed, and the sequence confirmed that the *asd* gene was deleted from the YS1646 chromosome.

### Complementation of asd Deletion by asd Expression From Plasmids

A plasmid, pATIU6 (SEQ ID NO:225), was chemically synthesized and assembled. The plasmid contained the following features: a high copy (pUC19) origin of replication, a U6 promoter for driving expression of a short hairpin, an ampicillin resistance gene flanked by HindIII restriction sites for subsequent removal, and the *asd* gene containing 85 base pairs of sequence upstream of the start codon (SEQ ID NO:246). Into this vector, shRNAs targeting murine TREX1 were introduced by restriction digestion with SpeI and XhoI, and ligation and cloning into *E. coli* DH5-alpha cells. The resulting plasmid was designated pATI-shTREX1.

### Electroporation of Plasmids into Immunostimulatory Bacterial Strains

Selected plasmids, containing expression cassettes encoding immunostimulatory proteins and a functional *asd* gene, were electroporated into *S. typhimurium* strains lacking the *asd* gene with a BTX^{®} ECM600 electroporator, using a 0.2 cm gap cuvette (BTX, San Diego, Calif.) at the following settings: 2.5 kV, 186 ohms, and 50 µF. Electroporated cells were added to 1 mL SOC supplemented with 50 µM diaminopimelic acid (DAP), incubated for 1 hour at 37 °C, and then spread onto agar plates that do not contain DAP, to select for strains that received plasmids with a functional *asd* gene. After single colony isolation, cell banks were produced by inoculating a flask of sterile lysogeny broth (LB) with a single well isolated colony of *S. typhimurium,* and incubating at 37 °C with agitation at 250 RPM. After the culture was grown to stationary phase, the bacteria were washed in PBS containing 10% glycerol, and stored in aliquots frozen at less than -60 °C.

The plasmid pATI-shTREX1 was amplified in *E. coli* and purified for transformation into the YS1646Δ*asd* strain by electroporation and clonal selection on LB Amp plates, to produce strain YS1646Δ*asd*-shTREX1. The YS1646Δ*asd* mutants complemented with pATIU6-derived plasmids were able to grow on LB agar and liquid media in the absence of DAP.

In a subsequent iteration, the ampicillin resistance gene (Amp^{R}) from pATI-shTREX1 was replaced with a kanamycin resistance gene. This was accomplished by digestion of the pATI-shTREX1 plasmid with HindIII, followed by gel purification to remove the Amp^{R} gene. The kanamycin resistance (Kan^{R}) gene was amplified by PCR using primers APR-001 and APR-002 (SEQ ID NO:226 and SEQ ID NO:227, respectively), followed by digestion with HindIII, and ligation into the gel purified, digested pATIU6 plasmid.

In subsequent iterations, a single point mutation was introduced into the pATIKan plasmid at the pUC19 origin of replication, using the Q5^{®} Site-Directed Mutagenesis Kit (New England Biolabs) and the primers APR-003 (SEQ ID NO:228) and APR-004 (SEQ ID NO:229), to change the nucleotide T at position 148 to a C. This mutation makes the origin of replication homologous to the pBR322 origin of replication, which is a low copy origin of replication, in order to reduce the plasmid copy number.

### Plasmid Maintenance Demonstrated In Vivo Using asd Complementation System

In this example, CT26 tumor-bearing mice were treated with strain YS1646, containing a plasmid that expresses an shRNA targeting TREX1 (YS1646-shTREX1), or with an *asd*-deleted strain of YS1646, containing a plasmid with a functional *asd* gene and an shRNA targeting TREX1 (YS1646Δ*asd*-shTREX1).

CT26 (Colon Tumor #26) is a tumor model that originated from exposing BALB/c mice to N-nitro-N-methylurethane (NMU), resulting in a highly metastatic carcinoma that recapitulates the aggressive, undifferentiated and checkpoint-refractory human colorectal carcinoma (see, *e.g.,* Castle et al. (2014) BMC Genomics 15(1):190). When implanted subcutaneously in the flank, as opposed to orthotopically in the colon, the tumor immunophenotype is much more immunosuppressive and checkpoint refractory. While largely lacking in T-cell infiltration, the tumor is rich in myeloid cells, such as macrophages and myeloid-derived suppressor cells (MDSCs) (see, *e.g.,* Zhao et al. (2017) Oncotarget 8(33):54775-54787). As this model more closely resembles human microsatellite stable (MSS) colorectal cancer, it is an ideal model to evaluate the therapeutic approach provided herein.

For this experiment, 6-8 week-old female BALB/c mice (3 mice per group) were inoculated subcutaneously (SC) in the right flank with CT26 (purchased from ATCC) tumor cells (2x10⁵ cells in 100 µL PBS). Mice bearing 8 day-old established flank tumors were intravenously (IV) injected with three doses of 5 x10⁶ CFUs of the YS1646Δ*asd-*shTREX1 strain, or the parental YS1646-shTREX1 strain, on days 8, 15, and 23. The plasmid encodes shTREX1 as an exemplary therapeutic product; any other desired therapeutic product or products can be substituted.

Body weights and tumors were measured twice weekly. Tumor measurements were performed using electronic calipers (Fowler, Newton, MA). Tumor volume was calculated using the modified ellipsoid formula, 1/2(length × width²). Mice were euthanized when tumor size reached >20% of body weight or became necrotic, as per IACUC regulations.

At 12 days after the final *Salmonella* injection, tumors were homogenized, and homogenates were serially diluted and plated on LB agar plates, to enumerate the total number of colony forming units (CFUs) present, or on LB plates containing kanamycin, to enumerate the number of kanamycin resistant colonies.

The results demonstrated that *S. typhimurium* strain YS1646-shTREX1 did not have selective pressure to maintain the shRNA plasmid, and demonstrated significant plasmid loss, as the percent of kanamycin resistant (Kan^{R}) colonies was less than 10%. The strain that used the *asd* gene complementation system for plasmid maintenance, YS1646Δ*asd*-shTREX1, had nearly identical numbers of kanamycin resistant and kanamycin sensitive CFUs. These data demonstrate that the *asd* gene complementation system is sufficient to maintain the plasmid in the context of the tumor microenvironment in mice.

### Enhanced Anti-Tumor Efficacy Using asd Complementation System

The *asd* complementation system is designed to prevent plasmid loss and potentiate the anti-tumor efficacy of the therapeutic product delivery by *S. typhimurium* strains *in vivo.* To test this, YS1646Δ*asd* strains, containing the shTREX1 plasmid (YS1646Δ*asd*-shTREX1), or scrambled control (YS1646Δ*asd-*shSCR), that contain a functional *asd* gene cassette, were compared for anti-tumor efficacy in a murine colon carcinoma model, to strain YS1646 containing plasmid pEQU6-shTREX1 (YS1646-shTREX1), a plasmid that lacks an *asd* gene cassette, and therefore, does not have a mechanism for plasmid maintenance. shTREX1 is an exemplary therapeutic product.

For this experiment, 6-8 week-old female BALB/c mice (8 mice per group) were inoculated SC in the right flank with CT26 cells (2x10⁵ cells in 100 µL PBS). Mice bearing established flank tumors were IV injected twice, on day 8 and on day 18, with 5x10⁶ CFUs of YS1646Δ*asd*-shTREX1, or YS1646-shTREX1, and compared to PBS control.

The YS1646-shTREX1 strain demonstrated enhanced tumor control compared to PBS (70% tumor growth inhibition (TGI), day 28) despite its demonstrated plasmid loss over time. The Δ*asd* strain containing the plasmid with the *asd* gene complementation system and shTREX1 (YS1646Δ*asd*-shTREX1) demonstrated superior tumor growth inhibition compared to PBS (82% TGI, *p* = 0.002, day 25). These data demonstrate that improved potency is achieved by preventing plasmid loss, using the *asd* complementation system, and delivery of shTREX1, as compared to YS1646 containing plasmids without the *asd* gene complementation system. Thus, strains with *asd* complementation systems are superior anti-cancer therapeutics.

### Example 4

### S. typhimurium Flagellin Knockout by Deletion of the fliC and fljB Genes Strain Engineering and Characterization

In the example herein, the live attenuated *S. typhimurium* YS1646 strain containing the *asd* gene deletion was further engineered to delete *the fliC* and*fljB* genes, in order to remove both flagellin subunits. This eliminates pro-inflammatory TLR5 activation, in order to reduce pro-inflammatory signaling and improve anti-tumor adaptive immunity.

### Deletion of fliC Gene

In this example, *fliC* was deleted from the chromosome of the YS1646Δ*asd* strain using modifications of the method of Datsenko and Wanner (Proc. Natl. Acad. Sci. U.S.A. 97:6640-6645 (2000)) as described in detail in the previous example. Briefly, synthetic *fliC* gene homology arm sequences, that contained 224 and 245 bases of homologous sequence flanking the *fliC* gene, were cloned into a plasmid called pSL0147 (SEQ ID NO:230). A kanamycin gene cassette flanked by cre/loxP sites then was cloned into plasmid pSL0147, and the *fliC* gene knockout cassette was then PCR amplified with primers flic-1 (SEQ ID NO:232) and flic-2 (SEQ ID NO:233), gel purified, and then introduced into the YS1646Δ*asd* strain carrying the temperature sensitive lambda red recombination plasmid pKD46, by electroporation. Electroporated cells were recovered in SOC + DAP medium, and plated onto LB agar plates supplemented with kanamycin (20 µg/mL) and diaminopimelic acid (DAP, 50 µg/mL). Colonies were selected and screened for insertion of the knockout fragment by PCR using primers flic-3 (SEQ ID NO:234) and flic-4 (SEQ ID NO:235). pKD46 then was cured by culturing the selected kanamycin resistant strain at 42 °C and screening for loss of ampicillin resistance. The kanamycin resistance marker then was cured by electroporation of a temperature-sensitive plasmid expressing the Cre recombinase (pJW168), and Amp^{R} colonies were selected at 30 °C; pJW168 was subsequently eliminated by growing cultures at 42 °C. Selected *fliC* knockout clones were then tested for loss of the kanamycin marker by PCR, using primers flanking the sites of disruption (flic-3 and flic-4), and evaluation of the electrophoretic mobility on agarose gels.

### Deletion of fljB Gene

The *fljB* gene was then deleted from the YS1646Δ*asd*/Δ*fliC* strain using modifications of the methods described above. Synthetic *fljB* gene homology arm sequences that contained 249 and 213 bases of the left hand and right hand sequence, respectively, flanking the *fljB* gene, were synthesized and cloned into a plasmid called pSL0148 (SEQ ID NO:231). A kanamycin gene cassette flanked by cre/loxP sites then was cloned into pSL0148, and the *fljB* gene knockout cassette was PCR amplified with primers fljb-1 (SEQ ID NO:236) and fljb-2 (SEQ ID NO:237) (see, Table 1), gel purified, and introduced into strain YS1646Δ*asd*/Δ*fliC* carrying the temperature sensitive lambda red recombination plasmid pKD46, by electroporation. The kanamycin resistance gene then was cured by Cre-mediated recombination, as described above, and the temperature-sensitive plasmids were cured by growth at non-permissive temperature. The *fliC* and *fljB* gene knockout sequences were amplified by PCR using primers flic-3 and flic-4, or fljb-3 (SEQ ID NO:238) and fljb-4 (SEQ ID NO:239), respectively, and verified by DNA sequencing. This mutant derivative of strain YS1646 was designated YS1646Δ*asd*/*ΔfliC*/*ΔfljB,* or YS1646Δ*asd*/ΔFLG for short.

### In Vitro Characterization of Engineered S. typhimurium Flagellin Knockout Strain

The YS1646-derived *asd⁻* mutant strain harboring the deletions of both*fliC* and *fljB*, herein referred to as YS1646Δ*asd*/ΔFLG, was evaluated for swimming motility by spotting 10 microliters of overnight cultures onto swimming plates (LB containing 0.3% agar and 50 mg/mL DAP). While motility was observed for the YS1646Δ*asd* strain, no motility was evident with the YS646Δ*asd*/ΔFLG strain. The YS1646Δ*asd*/ΔFLG strain then was electroporated with a plasmid containing an *asd* gene, and its growth rate in the absence of DAP was assessed. The YS1646Δ*asd*/ΔFLG strain, with an *asd* complemented plasmid, was able to replicate in LB in the absence of supplemental DAP, and grew at a rate comparable to the YS1646Δ*asd* strain containing an *asd* complemented plasmid. These data demonstrate that the elimination of flagellin does not decrease the fitness of *S. typhimurium in vitro.*

### Elimination of Flagella Decreases Pyroptosis in Murine Macrophages

5x10⁵ mouse RAW macrophage cells (InvivoGen, San Diego, Ca.) were infected with the YS1646Δ*asd*/ΔFLG strain, or the parental YS1646Δ*asd* strain, both harboring an *asd* complemented plasmid, at an MOI of approximately 100, in a gentamicin protection assay. After 24 hours of infection, culture supernatants were collected and assessed for lactate dehydrogenase release as a marker of macrophage cell death, using a Pierce^{™} LDH Cytotoxicity Assay Kit (Thermo Fisher Scientific, Waltham, Ma.). The YS1646Δ*asd* strain induced 75% maximal LDH release, while the YS 1646Δ*asd*/ΔFLG strain induced 54% maximal LDH release, demonstrating that deletion of the flagellin genes reduces the *S. typhimurium*-induced pyroptosis of infected macrophages.

### Flagella-Deleted Mutants Lead to Less Pyroptosis in Infected Human Monocytes

To demonstrate that the YS646Δ*asd*/ΔFLG strains are reduced in their ability to cause cell death in macrophages, THP-1 human macrophage cells (ATCC Catalog # 202165) were infected with the *S. typhimurium* strains YS1646 and YS1646Δ*asd*/ΔFLG, with the Δ*asd* strain containing plasmids encoding a functional *asd* gene to ensure plasmid maintenance. 5x10⁴ cells were placed in a 96-well dish with DMEM and 10% FBS. Cells were infected with washed log-phase cultures of *S. typhimurium* for 1 hour at an MOI of 100 CFUs per cell, then the cells were washed with PBS, and the media was replaced with media containing 50 µg/mL gentamicin to kill extracellular bacteria, and 50 ng/mL of IFNγ to convert the monocytes into a macrophage phenotype. After 24 hours, the THP-1 cells were stained with Cell Titer-Glo^{®} reagent (Promega), and the percentage of viable cells was determined using a luminescent cell viability assay using a SpectraMax^{®} M3 plate reader (Molecular Devices) to quantify the luminescence. The cells infected with the YS1646 strain had only 38% viability, while the cells infected with the YS1646Δ*asd*/ΔFLG strain had 51% viability, indicating that the deletion of the flagellin genes induced less cell death of human macrophages, despite a very high and supraphysiological MOI.

### Flagella is not Required for Tumor Colonization After Systemic Administration

To assess the impact of the flagellin knockout strains, administered in a murine model of colon carcinoma, 6-8 week-old female BALB/c mice (5 mice per group) were inoculated SC in the right flank with CT26 cells (2x10⁵ cells in 100 µL PBS). Mice bearing 10-day established flank tumors were IV injected with a single dose of 3 x10⁵ CFUs of the YS1646Δ*asd*/ΔFLG-shTREX1 strain, or the parental YS1646Δ*asd*-shTREX1 strain. At day 35 post tumor implantation, mice were euthanized, and tumors were homogenized and plated on LB plates to enumerate the number of colony forming units (CFUs) per gram of tumor tissue. The YS1646Δ*asd-*shTREX1 strain colonized tumors at a mean of 5.9 x10⁷ CFUs per gram of tumor tissue, while the flagella-deleted YS1646Δ*asd*/ΔFLG-shTREX1 strain colonized the tumors with almost a 2-fold increased mean of 1.1 x 10⁸ CFUs/g of tumor tissue. The splenic colonization of the YS1646Δ*asd-*shTREX1 strain was calculated as a mean of 1.5 x 10³ CFU/g of spleen tissue, whereas splenic colonization of the flagella-deleted YS1646Δ*asd*/ΔFLG-shTREX1 strain was slightly lower, at a mean of 1.2 x 10³ CFU/g of spleen tissue.

These data demonstrate that the absence of flagella not only does not negatively impact tumor colonization after IV administration, but it enhances tumor colonization compared to the flagella-intact strain. Importantly, deletion of the flagella slightly reduces splenic colonization, giving a tumor to spleen ratio of 100,000-fold. These data demonstrate that, contrary to the expectation from the art, not only are the flagella not required for tumor colonization, but their elimination enhances tumor colonization, while reducing splenic colonization.

### The Flagella-Deleted Strain Demonstrates Enhanced Anti-Tumor Activity in Mice

To assess the impact of the flagellin knockout strains, administered in a murine model of colon carcinoma, 6-8 week-old female BALB/c mice (5 mice per group) were inoculated SC in the right flank with CT26 cells (2x10⁵ cells in 100 µL PBS). Mice bearing established flank tumors were IV injected with a single dose of 3 x10⁵ CFUs of the YS1646Δ*asd*/ΔFLG-shTREX1 strain, or the YS1646Δ*asd-*shTREX1 strain, and compared to PBS control. Mice were monitored by caliper measurements for tumor growth.

The results demonstrated that the YS1646Δ*asd*/ΔFLG-shTREX1 strain, incapable of making flagella, showed enhanced tumor control compared to the parental YS1646Δ*asd*-shTREX1 strain (27% TGI, day 24), and significant tumor control compared to the PBS control (73% TGI, *p* = 0.04, day 24). These data demonstrate that, not only is the flagella not required for tumor colonization, but its loss can enhance anti-tumor efficacy.

### Flagella-Deleted Strains Demonstrate Enhanced Adaptive Immunity in a Murine Tumor Model

The impact of deletion of the flagellin on the immune response, and whether STING activation from tumor myeloid cell-delivery of shRNA to the STING checkpoint gene TREX1 would promote an adaptive type I IFN immune signature, was assessed. The CT26 murine model of colon carcinoma was used, where 6-8 week-old female BALB/c mice (5 mice per group) were inoculated SC in the right flank with CT26 cells (2x10⁵ cells in 100 µL PBS). Mice bearing established flank tumors were IV injected 11 days post tumor implantation with 5 x10⁶ CFUs of the YS1646Δ*asd*/ΔFLG-shTREX1 strain, or the parental YS1646Δ*asd*-shTREX1, or the scrambled plasmid control strain, YS1646Δ*asd*-shSCR, and compared to PBS control. Mice were bled 7 days post dosing on Sodium Heparin coated tubes (Becton Dickinson). Non-coagulated blood was then diluted in the same volume of PBS and peripheral blood mononuclear cells (PBMCs) were separated from the interphase layer of whole blood using Lympholyte^{®}-M cell separation reagent (Cedarlane). Isolated PBMCs were washed with PBS + 2% FBS by centrifugation at 1300 RPM for 3 minutes at room temperature, and resuspended in flow buffer. One million PBMCs were seeded per well of a V-bottom 96-well plate. Cells were centrifuged at 1300 RPM for 3 minutes at room temperature (RT) and resuspended in 100 µL of flow buffer containing fluorochrome-conjugated AH1 peptide:MHC class I tetramers (MBL International), and the cell surface flow cytometry antibodies CD4 FITC clone RM4-5; CD8a BV421 clone 53-6.7; F4/80 APC clone BM8; CD11b PE-Cy7 clone M1/70; CD45 BV570 clone 30-F11; CD3 PE clone 145-2C11; Ly6C BV785 clone HK1.4; I-A/I-E APC-Cy7 clone M5/114.15.2; Ly6G BV605 clone 1A8; and CD24 PercP-Cy5.5 clone M1/69 (all from BioLegend), for 45 minutes at room temperature and in the dark. After 45 min, the cells were washed twice with PBS + 2% FBS by centrifugation at 1200 RPM for 3 min. The cells were then resuspended in PBS + 2% FBS containing DAPI (4',6-diamino-2-phenylindole; dead/live stain), and data were immediately acquired using the NovoCyte^{®} flow cytometer (ACEA Biosciences, Inc.) and analyzed using FlowJo^{™} software (Tree Star, Inc.).

The following cell types were enumerated as a percentage of total live cells: CD11b⁺ Gr1⁺ neutrophils (possibly MDSCs, although further phenotyping in an *ex vivo* functional assay would be required), CD11b⁺ F4/80⁺ macrophages, CD8⁺ T-cells, and CD8⁺ T-cells that recognize the CT26 tumor rejection antigen gp70 (AH1), the product of the envelope gene of murine leukemia virus (MuLV)-related cell surface antigen (see, *e.g.,* Castle et al. (2014) BMC Genomics 15(1):190).

The results, summarized in the table below, show that the YS1646Δ*asd-*shSCR strain, containing a plasmid encoding a non-specific scrambled shRNA, elicits the typical anti-bacterial immune profile of significantly increased neutrophils, as compared to PBS (*p* = 0.02), to the flagella-intact YS1646Δ*asd*-shTREX1 strain (*p =* 0.02 ), and to the flagella-deleted strain YS1646Δ*asd*/ΔFLG-shTREX1 (*p* = 0.01), which had the lowest levels of circulating neutrophils. Similarly, bacterially-induced macrophages also were significantly elevated in the YS1646Δ*asd*-shSCR strain, as compared to PBS (*p* = 0.01), to the YS1646Δ*asd*-shTREX1 strain (*p* = 0.01), and to YS1646Δ*asd*/ΔFLG-shTREX1 strain (*p* = 0.01). Thus, both strains carrying type I IFN-inducing payloads were capable of overwriting the normal anti-bacterial immune response, which clears bacterial infections through neutrophils and macrophages, and does not induce adaptive T-cell-mediate immunity. However, while the overall circulating levels of CD8⁺ T-cells were similar across all groups, the flagella-deleted YS1646Δ*asd*/ΔFLG-shTREX1 strain demonstrated significantly increased percentages of AH1-tetramer⁺ CD8⁺ T-cells, as compared to PBS (*p* = 0.04).

These data demonstrate the feasibility of engineering a bacteria to deliver viral-like type I IFN-inducing plasmids to tumor-resident myeloid cells. This results in a dramatic reprogramming of the immune response towards a more viral, and less bacterial, immune profile. Deletion of the flagella further enhanced the shift away from bacterially-recruited neutrophils and macrophages, and towards significantly increased tumor antigen-specific CD8⁺ T-cells. Thus, eliminating bacterial TLR5-mediated inflammation can enhance adaptive immunity.

| **Immune Cells** | **% Live Cells Mean ± SD** | | | |
|---|---|---|---|---|
| | **PBS** | **YS1646Δ*asd-*shSCR** | **YS1646Δ*asd-*shTREX1** | **YS1646Δ*asd*/ ΔFLG shTREX1** |
| **Neutrophils** | 6.27 ± 2.62 | 19.21 ± 9.46 | 5.87 ± 3.94 | 4.01 ± 1.65 |
| **Macrophages** | 10.08 ± 2.11 | 23.14 ± 9.04 | 9.12 ± 3.84 | 7.39 ± 2.11 |
| **CD8⁺ T-cells** | 6.64 ± 0.56 | 7.17 ± 0.60 | 7.14 ± 2.30 | 6.44 + 1.43 |
| **AH1⁺ CD8⁺ T-cells** | 0.83 ± 0.12 | 1.06 ± 1.11 | 2.27 ± 1.44 | 4.12 ± 3.08 |

| | | | | |
|---|---|---|---|---|
| SD = Standard Deviation | | | | |

### Flagella-Deleted Strains are Restricted to the Phagocytic Myeloid Immune Cell Compartment In Vivo

According to the literature, Δ*fljB*/Δ*fliC* strains demonstrate suppression of many downstream genes associated with SPI-1-mediated entry into non-phagocytic cells. In order to determine whether the YS1646Δ*asd*/ΔFLG strain also is deficient for non-phagocytic cell uptake, a YS1646Δ*asd*/ΔFLG strain, constitutively expressing mCherry (a red fluorescent protein) under the bacterial *rpsM* promoter, was IV administered to MC38 subcutaneous flank tumor-bearing mice.

The MC38 (murine colon adenocarcinoma #38) model was derived similarly as the CT26 model using mutagenesis, but with dimethylhydralazine, and in a C57BL/6 mouse strain (see, *e.g.,* Corbett et al. (1975) Cancer Res. 35(9):2434-2439). Similarly to CT26, subcutaneous implantation results in a more T-cell excluded and immunosuppressive tumor microenvironment than when implanted orthotopically in the colon (see, *e.g.,* Zhao et al. (2017) Oncotarget 8(33):54775-54787). MC38 has a higher mutational burden than CT26, and a similar viral-derived gp70 antigen (p15E) that can be detected by CD8⁺ T-cells, although it is not considered a rejection antigen. While variants of MC38 have been found to be partially responsive to checkpoint therapy, most variants of the cell line are considered checkpoint refractory and T-cell excluded (see, *e.g.,* Mariathasan et al. (2018) Nature 555:544-548), including the MC38 cells used herein.

For this experiment, 6-8 week-old female C57BL/6 mice (5 mice per group) were inoculated SC in the right flank with MC38 cells (5x10⁵ cells in 100 µL PBS). Mice bearing large established flank tumors were IV injected on day 34 with 1x10⁶ CFUs of the YS 1646Δ*asd*/ΔFLG-mCherry strain. Tumors were resected 7 days post IV-dosing, and cut into 2-3 mm pieces into gentleMACS^{™} C tubes (Miltenyi Biotec) filled with 2.5 mL enzyme mix (RPMI-1640 containing 10% FBS with 1 mg/mL Collagenase IV and 20 µg/mL DNase I). The tumor pieces were dissociated using OctoMACS^{™} (Miltenyi Biotec) specific dissociation program (mouse implanted tumors), and the whole cell preparation was incubated with agitation for 45 minutes at 37 °C. After the 45 minute incubation, a second round of dissociation was performed using the OctoMACS^{™} (mouse implanted tumor) program, and the resulting single cell suspensions were filtered through a 70 µM nylon mesh into a 50 mL tube. The nylon mesh was washed once with 5 mL of RPMI-1640 + 10% FBS, and the cells were filtered a second time using a new 70 µM nylon mesh into a new 50 mL tube. The nylon mesh was washed with 5 mL of RPMI-1640 + 10% FBS, and the filtered cells were then centrifuged at 1000 RPM for 7 minutes. The resulting dissociated cells were resuspended in PBS and kept on ice before the staining process.

For the flow-cytometry staining, 100 µL of the single cell suspensions were seeded in wells of a V-bottom 96-well plate. PBS containing a dead/live stain (Zombie Aqua^{™}, BioLegend), and Fc Blocking reagents (BD Biosciences), were added at 100 µL per well and the cells were incubated on ice for 30 minutes in the dark. After 30 minutes, the cells were washed twice with PBS + 2% FBS by centrifugation at 1300 RPM for 3 minutes. Cells were then resuspended in PBS + 2% FBS containing fluorochrome-conjugated antibodies (CD4 FITC clone RM4-5; CD8a BV421 clone 53-6.7; F4/80 APC clone BM8; CD11b PE-Cy7 clone M1/70; CD45 BV570 clone 30-F11; CD3 PE clone 145-2C11; Ly6C BV785 clone HK1.4; I-A/I-E APC-Cy7 clone M5/114.15.2; Ly6G BV605 clone 1A8; and CD24 PercP-Cy5.5 clone M1/69, all from BioLegend), and incubated on ice for 30 minutes in the dark. After 30 minutes, the cells were washed twice with PBS + 2% FBS by centrifugation at 1300 RPM for 3 minutes, and resuspended in flow cytometry fixation buffer (Thermo Fisher Scientific). Flow cytometry data were acquired using the NovoCyte^{®} Flow Cytometer (ACEA Biosciences, Inc.), and analyzed using the FlowJo^{™} software (Tree Star, Inc.).

The results demonstrated that 7.27% of tumor-infiltrating monocytes had taken up the flagella-deleted mCherry strain in the tumor microenvironment. Similarly, 8.96% of the tumor-associated macrophage (TAM) population, and 3.33% of the tumor-infiltrating dendritic cells (DCs) had taken up the flagella-deleted mCherry strain. In contrast, within the CD45⁻ population, corresponding to stromal and tumor cells, only 0.076% showed positivity for mCherry expression (compared to 0.067% background staining). These data demonstrate that the flagella, and its downstream signaling impact on SPI-1, are necessary to enable epithelial cell infectivity, and that the lack thereof restricts uptake of the bacteria to only the phagocytic immune cell compartment of the tumor microenvironment (*i.e.,* tumor-resident immune/myeloid cells).

Deletion of the flagella confers multiple benefits to the immunostimulatory *S*. *typhimurium* strain, including eliminating TLR5-induced inflammatory cytokines that suppress adaptive immunity, reducing macrophage pyroptosis, as well as maintaining (or enhancing) tumor-specific enrichment upon systemic administration, where uptake is confined to tumor-resident phagocytic cells.

### Example 5

### Salmonella pagP Gene Knockout Strain Engineering and Characterization

In this example, the YS1646Δ*asd*/ΔFLG strain was further modified to delete *pagP.* The *pagP* gene is induced during the infectious life cycle of *S. typhimurium,* and encodes an enzyme (lipid A palmitoyltransferase) that modifies lipid A with palmitate. In wild-type *S. typhimurium,* expression of *pagP* results in a lipid A molecule that is hepta-acylated. In an *msbB⁻* mutant, in which the terminal acyl chain of lipid A cannot be added, the expression of *pagP* results in a hexa-acylated lipid A molecule. LPS with hexa-acylated lipid A has been shown to be highly pro-inflammatory and have a high affinity for TLR4 (hepta-acylated LPS, found in wild-type, has the highest affinity for TLR4). In this example, a strain deleted of *pagP* and *msbB* can produce only penta-acylated lipid A, allowing for lower pro-inflammatory cytokines due to low affinity for TLR4, enhanced tolerability, and increased adaptive immunity when the bacteria are engineered to deliver plasmids encoding immunomodulatory proteins.

### ΔpagP Strain Construction

The *pagP* gene was deleted from the YS1646Δ*asd*/ΔFLG strain using modifications of the methods described in the preceding examples. Synthetic *pagP* gene homology arm sequences that contain 203 and 279 bases of the left hand and right hand sequence, respectively, flanking the *pagP* gene, were synthesized and cloned into a plasmid called pSL0191 (SEQ ID NO:331). A kanamycin gene cassette flanked by cre/loxP sites then was cloned into pSL0191, and the *pagP* gene knockout cassette was PCR amplified with primers pagp-1 (SEQ ID NO:315) and pagp-2 (SEQ ID NO:316) (see, Table 1), gel purified, and introduced into strain YS1646Δ*asd*/ΔFLG, carrying the temperature sensitive lambda red recombination plasmid pKD46, by electroporation. The kanamycin resistance gene then was cured by Cre-mediated recombination, as described above, and the temperature-sensitive plasmids were cured by growth at non-permissive temperature. The *pagP* gene knockout sequences were amplified by PCR using primers pagp-3 (SEQ ID NO:317) and pagp-4 (SEQ ID NO:318), and verified by DNA sequencing. The resulting mutant derivative of YS1646 was designated YS1646Δ*asd*/ΔFLG/Δ*pagP.*

### pagP Deletion Mutants have Penta-Acylated LPS and Induce Reduced Inflammatory Cytokines

The *pagP* gene also was deleted from the YS1646Δ*asd* strain using the lambda-derived Red recombination system, as described in Datsenko and Wanner (Proc. Natl. Acad. Sci. U.S.A. 97:6640-6645 (2000)) and above, to generate the strain YS1646Δ*asd*/Δ*pagP.* This strain was then electroporated with a plasmid containing a functional *asd* gene, to complement the deleted *asd* gene and to ensure plasmid maintenance *in vivo.* The lipid A was then extracted from this strain and evaluated by Matrix-Assisted Laser Desorption/Ionization Mass Spectrometry (MALDI MS) and compared to lipid A from the wild-type *S. typhimurium* strain ATCC 14028, the YS1646 strain (which is deleted for *msbB* and *purI*), and the YS 1646Δ*asd* strain. Wild-type *Salmonella* had a minor lipid A peak with a mass of 2034, and a major peak with a mass of 1796, corresponding to the hepta-acylated and hexa-acylated species, respectively, due to the presence of a functional *msbB* gene. The *msbB-*deleted strains, YS1646 and YS1646Δ*asd*, had major peaks at 1828 and 1585, corresponding to a mixture of hexa-acylated and penta-acylated lipid A. The *msbB* and *pagP* deleted strain, YS1646Δ*asd*/Δ*pagP,* had only a single peak with a mass of 1585, corresponding to penta-acylated lipid A. These data demonstrate that deletion of *pagP* prevents palmitoylation of the lipid A, thereby restricting it to a single penta-acylated species.

To determine whether the LPS with the penta-acylated lipid A from the Δ*pagP* mutant strains reduced TLR4 signaling, 4 µg of purified LPS from the wild-type strain, the YS1646 strain, or the YS1646Δ*asd*/Δ*pagP* strain, was added to THP-1 human monocytic cells (ATCC Catalog # TIB-202), and the supernatants were evaluated 24 hours later for the presence of inflammatory cytokines using a Cytometric Bead Array (CBA) kit (BD Biosciences). The results showed that LPS from the YS1646Δ*asd*/Δ*pagP* strain induced 25% of the amount of TNFα, compared to wild-type LPS, and induced 7-fold less IL-6 than wild-type LPS. The LPS from the YS1646Δ*asd*/Δ*pagP* strain induced 22-fold less IL-6 than strain YS1646, demonstrating that the penta-acylated LPS species from a Δ*pagP* mutant is significantly less inflammatory in human cells, and indicating that the Δ*pagP* mutant would be better tolerated in humans.

### Deletion of pagP Induces Significantly Less IL-6 in Primary Human M2 Macrophages

To demonstrate that the YS1646Δ*asd*/ΔFLG/Δ*pagP* strain also elicits less inflammatory and dose-limiting IL-6 from primary human M2 macrophages, the strain was evaluated, and compared with the YS646Δ*asd*/ΔFLG and the parental YS1646 strains. The M2 macrophages derived from human donors are representative of the immunosuppressive phenotypes that are highly enriched in T-cell excluded solid tumors. Frozen human PBMCs, isolated from healthy human donors, were thawed in complete medium (RPMI-1640 + 1X non-essential amino acids + 5% human AB serum), and washed by centrifugation for 10 minutes at 800 RPM at room temperature. PBMCs were resuspended in PBS + 2% FBS, and monocytes were negatively isolated using a CD16 depletion kit (StemCell Technologies). Isolated untouched monocytes were then washed by centrifugation in PBS + 2% FBS and resuspended in complete medium containing 100 ng/mL human macrophage colony-stimulating factor (M-CSF) and 10 ng/mL human IL-4. Isolated monocytes (3e5 per well) were then seeded in a 24-well plate with a final volume of 750 µL. Two days after seeding, the cell culture media was entirely aspirated and replaced with fresh complete medium containing 100 ng/mL human M-CSF and 10 ng/mL human IL-4. Two days later (on day 4), 500 µL of complete medium containing 100 ng/mL human M-CSF and 10 ng/mL human IL-4 was added per well for 48 hours. On day 6, the cell culture media was entirely aspirated and replaced with fresh complete medium without cytokines, alone, or with media containing the log-phase cultures of the *S. typhimurium* strains at an MOI of 20. Cells were infected for 1 hour, then washed with PBS, and the media was replaced with fresh media containing 50 µg/mL gentamicin to kill extracellular bacteria. The wells were then washed and replaced with fresh media and allowed to incubate at 37 °C and 5% CO₂. After 48 hours, supernatants were harvested and assayed for cytokines using a human IL-6 cytometric bead array (CBA) kit (BD Biosciences), according to the manufacturer's instructions.

The results demonstrated that secreted IL-6 levels from human primary M2 macrophages, infected with parental strain YS1646, yielded an average of 14839 ± 926 pg/mL, while the IL-6 levels from the YS1646Δ*asd*/ΔFLG strain were significantly lower, at 2075 ± 723 pg/mL (*p* = 0.004). This further affirms the impact that the deletion of flagella, and elimination of TLR5 signaling, has on the induction of IL-6. The YS1646Δ*asd*/ΔFLG/Δ*pagP* strain elicited the lowest IL-6 levels, at 332 ± 100 pg /mL, demonstrating the reduced ability of this modified LPS coating to stimulate TLR4, and the resulting dramatically reduced inflammatory IL-6 production.

### The Combined Flagella and pagP Deletions Significantly Enhance Tolerability in Mice

To determine whether the modified strains described above are more attenuated than parental strain YS1646, a median lethal dose (LD₅₀) study was conducted. 6-8 week-old BALB/c mice (5 mice per group) were injected intravenously with a dose range of 3e5 to 3e7 CFUs of strain YS1646, or the derivative strains YS1646Δ*asd*/ΔFLG, YS1646Δ*asd*/Δ*pagP*, and YS1646Δ*asd*/ΔFLG/Δ*pagP.* Unlike strain YS1646, the derivative strains also carried a plasmid encoding murine IL-2, an FDA-approved cytokine that has demonstrated significant toxicity when systemically administered.

The LD₅₀ for strain YS1646 was found to be 4.4 x 10⁶ CFUs (average of two studies), in line with previously published LD₃₀ reports of YS1646, and a >1000-fold improvement compared to wild-type *S. typhimurium* (see, *e.g.,* Clairmont et al. (2000) J. Infect. Dis. 181:1996-2002). The LD₅₀ for the YS646Δ*asd*/ΔFLG strain was determined to be 2.07 x 10⁷ CFUs, demonstrating a greater than 4.5-fold reduction in virulence compared to strain YS1646. The LD₅₀ for the YS1646Δ*asd*/Δ*pagP* strain was determined to be 1.39 x 10⁶ CFUs, demonstrating at least a 3.2-fold reduction in virulence compared to strain YS1646, which is expected, given that the strain still has highly inflammatory flagella. The LD₅₀ for the YS1646Δ*asd*/ΔFLG/Δ*pagP* strain could not be established, as no mice died at the highest dose given, but was >6.2 x 10⁷ CFUs. The YS1646Δ*asd*/ΔFLG/Δ*pagP* strain therefore demonstrates a >14-fold reduction in virulence compared to parental YS1646 strain. These data demonstrate that the genetic modifications described above reduce the virulence of the clinical *S. typhimurium* strain, YS1646 (also known as VNP20009), and therefore, lead to increased tolerability in humans.

In the Phase I clinical trial of VNP20009 (see, *e.g.,* Toso et al. (2002) J. Clin. Oncol. 20(1):142-152), the presence of the bacteria in patients' tumors only partially was observed at the two highest doses tested, 3 x 10⁸ CFU/m² (33% presence), and 1 x 10⁹ CFU/m² (50% presence), indicating that the tolerable dose of VNP20009 was too low to achieve tumor colonization. By improving the tolerability of the strains through the modifications described above, >14-fold higher doses can be administered, if necessary, improving the percentage of patients whose tumors will be colonized, and increasing the level of therapeutic colonization per tumor, thereby solving the observed problems with VNP20009.

### The Combined Flagella and pagP Deletions Significantly Limit the Generation of Anti-S. typhimurium Antibodies in Mice

The surviving mice from the 3 x 10⁶ CFU dosing group described above (N=5, except for N=4 in the YS1646 dosing group) were kept for 40 days post IV-dosing, at which time they were bled for serum, and assessed for antibody titers to *S. typhimurium,* by a modified flow-based antibody titering system. Overnight cultures of the YS1646Δ*asd*/ΔFLG-mCherry strain were washed and fixed with flow cytometry fixation buffer. Sera from the previously-treated mice, and from naive control mice, were seeded in a 96-well plate, and serial dilutions were performed in PBS. Next, 25 µL of the YS1646Δ*asd*/ΔFLG-mCherry cultures, containing 1 x 10⁶ CFUs, were added to the sera and incubated for 25 minutes at room temperature. The bacteria were then washed twice with PBS by spinning them at 4000 RPM for 5 minutes. After the last wash, the bacteria were resuspended in PBS containing a secondary Goat anti-Mouse Fc AF488 antibody (1/400 dilution from stock), and incubated for 25 minutes at room temperature and protected from light. The bacteria were then washed three times with PBS by spinning them at 4000 RPM for 5 minutes. After the last wash, the bacteria were resuspended in PBS, and data were acquired using the NovoCyte^{®} flow cytometer (ACEA Biosciences, Inc.), and analyzed using the MFI FlowJo^{™} software (Tree Star, Inc.).

To evaluate the results by flow cytometry, the highest dilution with signal in all groups was chosen (the 1250X serum dilution), and the corresponding mean fluorescence intensity (MFI) values were plotted. The limit of detection (LOD) was chosen at an MFI of 1000, as that is the MFI obtained without staining, as well as with background staining with Goat anti-Mouse Fc AF488 antibody only. Therefore, an MFI greater than 1000 was considered a positive signal, and everything equal to or under this value was considered a negative result, despite having an MFI value.

The results of this assay revealed a high MFI titer of anti-*S*. *typhimurium* serum antibodies from mice treated with 3 x 10⁶ CFUs of the YS1646 strain (MFI of 29196.3 ± 20730), in line with previously published data that YS1646 is able to generate serum antibodies (that are non-neutralizing). Fewer antibodies were detected in the mice treated with the YS1646Δ*asd*/ΔFLG strain (MFI of 11257 ± 9290), which can be due to the lack of adjuvant activity from the flagella. In the mice treated with the YS1646Δ*asd*/Δ*pagP* strain, significantly fewer antibodies were generated (MFI of 4494 ± 3861), as compared to strain YS1646 (*p* = 0.033), which can be due to the altered LPS surface coating. The most significant reduction in serum antibodies was demonstrated in the YS1646Δ*asd*/ΔFLG/Δ*pagP* treatment group (MFI of 1930 ± 2445), where several of the mice had MFI titers under 1000, and were thus considered negative for serum antibodies (*p* = 0.021, *vs.* strain YS1646). Thus, the combined deletions of the flagella and the *pagP* gene enable both improved safety, as well as significantly reduced immunogenicity, which will enable repeat dosing of high CFUs in humans.

### pagP and Flagella Deleted Strains, and their Combination, Demonstrate Significantly Higher Viability in Human Serum Compared to Strain YS1646

Strain YS1646 exhibits limited tumor colonization in humans after systemic administration. It is shown herein that strain YS1646 is inactivated by complement factors in human blood. To demonstrate this, strains YS1646 and *E. coli* D10B were compared to exemplary immunostimulatory bacteria provided herein, that contain additional mutations that alter the surface of the bacteria. These exemplary modified strains were *YS1646Δasd*/*ΔpagP,* YS1646Δ*asd*/ΔFLG, and YS1646Δ*asd*/ΔFLG/Δ*pagP.* These three strains, in addition to YS1646 and *E. coli* D10B cultures, were incubated with serum, or heat-inactivated (HI) serum, from either pooled mouse blood, or pooled healthy human donors (n=3), for 3 hours at 37 °C. After incubation with serum, bacteria were serially diluted and plated on LB agar plates, and the colony forming units (CFUs) were determined.

In mouse serum, all strains remained 100% viable and were completely resistant to complement inactivation. In human serum, all strains were 100% viable in the heat-inactivated serum. The *E. coli* D10B strain was completely eliminated after 3 hours in whole human serum. In whole human serum, the YS1646 strain exhibited only 6.37% of live colonies, demonstrating that tumor colonization of the YS1646 clinical strain was limited due to complement inactivation in human blood. For the YS1646Δ*asd*/ΔFLG strain, 31.47% of live colonies remained, and for the YS1646Δ*asd*/Δ*pagP* strain, 72.9% of live colonies remained, after incubation with human serum for 3 hours. The combined YS1646Δ*asd*/ΔFLG/Δ*pagP* strain was completely resistant to complement in human serum.

These data explain why strain YS1646 (VNP20009) has very low tumor colonization when systemically administered. It is shown herein that strain YS1646 is highly sensitive to complement inactivation in human serum, but not in mouse serum. These data explain why limited tumor colonization was observed in humans, while mouse tumors were colonized at a high level. The *fljB*/*fliC* or *pagP* deletions, or the combination of these mutations, partially or completely rescues this phenotype. Thus, the enhanced stability observed in human serum with the YS1646Δ*asd*/Δ*pagP,* YS1646Δ*asd*/ΔFLG, and YS1646Δ*asd*/ΔFLG/Δ*pagP* strains provides for increased human tumor colonization.

### Example 6

### Salmonella ansB Gene Knockout Strain Engineering and Characterization

In this example, the YS1646Δ*asd*/ΔFLG/Δ*pagP* strain was further modified to delete *ansB,* the gene encoding bacterial L-asparaginase II. Secretion of L-asparaginase II by *S. typhimurium* in the presence of T-cells has been shown to directly impair T-cell function, by reducing T-cell receptor (TCR) expression and impairing cytolytic cytokine production. As a result, bacterially-derived asparaginases have been successfully used to treat acute lymphoblastic leukemia (ALL) for decades. Deletion of *ansB* eliminates the ability of the *S. typhimurium* to produce L-asparaginase II, thereby enhancing the function of T-cells in the bacterially-colonized tumor microenvironment.

### ΔansB Strain Construction

The *ansB* gene was deleted from the YS1646Δ*asd*/ΔFLG/Δ*pagP* strain using modifications of the methods described in the preceding examples. Synthetic *ansB* gene homology arm sequences that contained 236 and 251 bases of the left hand and right hand sequence, respectively, flanking the *ansB* gene, were synthesized and cloned into a plasmid called pSL0230 (SEQ ID NO:332). A kanamycin gene cassette flanked by cre/loxP sites then was cloned into plasmid pSL0230 and the *ansB* gene knockout cassette was PCR amplified with primers ansb-1 (SEQ ID NO:319) and ansb-2 (SEQ ID NO:320), gel purified, and introduced into strain YS1646Δ*asd*/ΔFLG/Δ*pagP,* carrying the temperature sensitive lambda red recombination plasmid pKD46, by electroporation. The kanamycin resistance gene then was cured by Cre-mediated recombination, as described above, and the temperature-sensitive plasmids were cured by growth at non-permissive temperature. The *ansB* gene knockout sequences were amplified by PCR using primers ansb-3 (SEQ ID NO:321) and ansb-4 (SEQ ID NO:322) (see, Table 1), and verified by DNA sequencing. The resulting mutant derivative of YS1646 was designated YS1646Δ*asd*/ΔFLG/Δ*pagP*/Δ*ansB.*

### Deletion of ansB Eliminates Asparaginase Activity In Vitro

In order to determine whether the *YS1646Δasd*/*ΔFLG*/*ΔpagP*/*ΔansB* strain produced less L-asparaginase II, cultures of this strain, along with the *ansB*-intact *YS1646Δasd*/*ΔFLG*/*ΔpagP* strain, were grown in LB and allowed to reach stationary phase. At this time, 50 µL of conditioned media from the cultures was analyzed for asparaginase activity, using a colorimetric Asparaginase assay kit (Sigma-Aldrich), per the manufacturer's instructions. After a 40 min incubation, the absorbance units were read on a SpectraMax^{®} M3 Spectrophotometer (Molecular Devices) at an absorbance of 570 nm.

Compared to the recombinant L-asparaginase II positive control, which gave an absorbance of 1.95, the absorbance of the *ansB*-intact YS1646Δasd/ΔFLG/ΔpagP strain was 0.82. Deletion of *ansB,* in the *YS1646Δasd*/*ΔFLG*/*ΔpagP*/*ΔansB* strain, however, resulted in background levels of asparaginase activity, detected at an absorbance of 0.109. These data confirm that the Δ*ansB* mutation completely eliminates asparaginase activity.

### Deletion of ansB Restores T-cell Function in an In Vitro Co-Culture Assay

In order to functionally characterize the *ansB*-deleted strain for the impact of reduced L-asparaginase II activity on T-cells, a co-culture assay was established using strain-infected murine primary bone marrow-derived macrophages (BMMs), in culture with splenic purified T-cells. For this assay, spleens from healthy BALB/c mice were isolated and dissociated, and splenic CD4⁺ and CD8⁺ T-cells were isolated using a mouse T-cell isolation kit (StemCell Technologies), per the manufacturer's instructions. From the isolated T-cells, 2e5 cells were added per well to a Flat-bottom 96-well plate that had been previously coated with 5 µg/ml of an anti-mouse CD3ε antibody (clone 145-2C11, Thermo Fisher Scientific). Conditioned LB media from the YS1646Δ*asd*/ΔFLG/Δ*pagP* and *YS1646Δasd*/*ΔFLG*/*ΔpagP*/*ΔansB* cultures, grown to stationary phase, were filtered through a 0.45 µM nylon mesh and added to the T-cells, with or without the addition of 10 µg/ml of an agonistic CD28 antibody for co-stimulation. Control groups containing recombinant asparaginase at 20 U/mL, and normal culture media, were used as controls in the assay. The plate was incubated at 37 °C in a 5% CO₂ incubator. At 24 hours post-incubation, 100 µL of the co-culture supernatants were harvested from the wells, and a murine Th1-specific cytokine bead array (CBA, BioLegend) was performed. Concurrently, T-cells were harvested and analyzed for surface T-cell receptor β (TCRβ) expression on CD4⁺ and CD8⁺ T-cells by flow cytometry, as well as for intracellular staining of IFNy, TNFα, and IL-2.

The results confirmed that the *ansB*-intact strain (YS *1646Δasd*/*ΔFLG*/*ΔpagP*) used to infect the macrophages, and subsequently co-cultured with T-cells, induces profound T-cell immunosuppression. This was exhibited by marked downregulation of TCRβ surface expression in both CD4⁺ and CD8⁺ T-cells (see table below), compared to media control, and to the positive control of recombinant asparaginase at 20 U/mL. Deletion of *ansB* in the *YS1646Δasd*/*ΔFLG*/*ΔpagP*/*ΔansB* strain significantly restored TCRβ surface expression in both CD4⁺ (*p* = 0.004) and CD8⁺ T-cells (*p* = 0.002), as compared to the parental YS1646Δ*asd*/ΔFLG/Δ*pagP* strain.

| **Treatment** | **TCRβ Expression (MFI Mean ± SD)** | |
|---|---|---|
| | **CD4⁺ T-Cells** | **CD8⁺ T-Cells** |
| **Media control** | 8141 ± 405.9 | 12655 ± 534.6 |
| **YS1646Δ*asd*/ΔFLG/Δ*pagP*** | 3817 ± 200.8 | 6492 ± 260.2 |
| ***YS1646Δasd*/*ΔFLG*/*ΔpagP*/*ΔansB*** | 7047.5 ± 204.4 | 13350 ± 339.4 |
| **Recombinant asparaginase II (20 U/mL)** | 4253.5 ± 576.3 | 6305 ± 687.3 |

| | | |
|---|---|---|
| SD = Standard deviation | | |

T-cell secretion of cytokines, 24 hours after co-culture, was measured as a marker of T-cell cytolytic function. As shown in the table below, T-cell production of the cytokines IFNγ, TNFα, and IL-2 was markedly lower after treatment with the YS1646Δ*asd*/ΔFLG/Δ*pagP* strain, as compared to the media control, and was significantly restored by *ansB* deletion in the YS1646Δ*asd*/ΔFLG/Δ*pagP*/Δ*ansB* strain (IFNγ (*p* = 0.05), TNFα (*p* = 0.012), and IL-2 (*p* = 0.006)). These data indicate that deletion of *ansB* in the YS 1646Δ*asd*/ΔFLG/Δ*pagP*/Δ*ansB* strain significantly restores T-cell cytolytic function, as compared to the parental YS *1646Δasd*/*ΔFLG*/*ΔpagP* strain.

| **Treatment** | **T-Cell Cytokine Expression (pg/mL, Mean ± SD)** | | |
|---|---|---|---|
| | **IFNγ** | **TNFα** | **IL-2** |
| **Media control** | 4176.1 ± 20.8 | 136.0 ± 11.1 | 3118.2 ± 154.3 |
| **YS1646Δ*asd*/ΔFLG/Δ*pagP*** | 238.1 ± 0.6 | 35.7 ± 7.1 | 134.9 ± 12.7 |
| **YS1646Δ*asd*/ΔFLG/Δ*pagP*/Δ*ansB*** | 1788.9 ± 515.5 | 120.5 ± 11.2 | 1947.6 + 190.5 |
| **Recombinant asparaginase II (20 U/mL)** | 166.2 ± 19.7 | 33.2 ± 3.4 | 114.5 ± 4.8 |

| | | | |
|---|---|---|---|
| SD = Standard deviation | | | |

### Deletion of ansB Restores Tumor-Resident T-cell TCRβ Expression In Vivo

In *in vitro* co-culture assays, expression of *ansB* demonstrated immunosuppressive effects on T-cell function, including downregulation of TCRβ on T-cells by flow cytometry. In order to assess whether this would similarly occur *in vivo,* the MC38 mouse model of colorectal cancer was utilized.

For this experiment, 6-8 week-old female C57BL/6 mice (4 mice per group) were inoculated SC in the right flank with MC38 cells (5x10⁵ cells in 100 µL PBS). Mice bearing large established flank tumors were IV injected on day 17 with 1x10⁷ CFUs of the YS1646Δasd/ΔFLG-mCherry strain. Tumors were resected 7 days post IV dosing and cut into 2-3 mm pieces into gentleMACS^{™} C tubes (Miltenyi Biotec) filled with 2.5 mL enzyme mix (RPMI-1640 containing 10% FBS with 1 mg/mL Collagenase IV and 20 µg/mL DNase I). The tumor pieces were dissociated using OctoMACS^{™} (Miltenyi Biotec) specific dissociation program (mouse implanted tumors), and the whole cell preparation was incubated with agitation for 45 minutes at 37 °C. After 45 minutes of incubation, a second round of dissociation was performed using the OctoMACS^{™} (mouse implanted tumor) program, and the resulting single cell suspensions were filtered through a 70 µM nylon mesh into a 50 mL tube. The nylon mesh was washed once with 5 mL of RPMI-1640 + 10% FBS, and the cells were filtered a second time using a new 70 µM nylon mesh, into a new 50 mL tube. The nylon mesh was washed with 5 mL of RPMI-1640 + 10% FBS, and the filtered cells were then centrifuged at 1000 RPM for 7 minutes. The resulting dissociated cells were resuspended in PBS and kept on ice before the staining process.

For the flow-cytometry staining, 100 µL of the single cell suspensions were seeded in wells of a V-bottom 96-well plate. PBS containing a dead/live stain (Zombie Aqua^{™}, BioLegend) and Fc Blocking reagents (BD Biosciences) were added at 100 µL per well, and the cells were incubated on ice for 30 minutes in the dark. After 30 minutes, the cells were washed twice with PBS + 2% FBS by centrifugation at 1300 RPM for 3 minutes. Cells were then resuspended in PBS + 2% FBS containing fluorochrome-conjugated antibodies (CD45 BV570 clone 30-F11; TCRβ PE clone H57-597; and CD4 FITC clone RM4-5; all from BioLegend) and DAPI (BioLegend), and incubated on ice for 30 minutes in the dark. After 30 minutes, the cells were washed twice with PBS + 2% FBS by centrifugation at 1300 RPM for 3 minutes, and resuspended in flow cytometry fixation buffer (Thermo Fisher Scientific). Flow cytometry data were acquired using the ACEA NovoCyte^{®} flow cytometer (ACEA Biosciences, Inc.), and analyzed using the FlowJo^{™} software (Tree Star, Inc.).

As shown in the table below, the average mean fluorescence intensity (MFI) for the surface expression of TCRβ on tumor-infiltrating CD4⁺ T-cells, following their interaction within tumors with the colonized parental YS *1646Δasd*/*ΔFLG*/*ΔpagP* strain, was significantly lower than with the *ansB*-deleted YS1646Δ*asd*/ΔFLG/Δ*pagP*/Δ*ansB* strain (*p* = 0.042), which was higher than even the PBS control-treated mice.

| | **MFI for TCRβ Expression on Tumor-Infiltrating CD4⁺ T-Cells** | | |
|---|---|---|---|
| | **PBS** | **YS1646 Δ*asd*/ΔFLG/Δ*pagP*** | ***YS1646Δasd*/*ΔFLG*/*ΔpagP*/*ΔansB*** |
| | 13980 | 13933 | 14412 |
| | 14543 | 13480 | 14957 |
| | 14177 | 12087 | 14844 |
| | 13931 | 14010 | 14233 |
| **AVG** | **14157.8** | **13377.5** | **14611.5** |
| **SD** | **278.0** | **891.5** | **344.7** |

| | | | |
|---|---|---|---|
| MFI = mean fluorescence intensity; AVG = average; SD = standard deviation | | | |

Taken together, these data confirm the necessity of deleting the *ansB* gene in order to restore T-cell function, due to the bacterial production of immunosuppressive L-asparaginase II, and demonstrate the enhanced T-cell function observed with the *ansB* deletion in the YS1646Δ*asd*/ΔFLG/Δ*pagP*/Δ*ansB* strain.

### Example 7

### Salmonella csgD Gene Knockout Strain Engineering and Characterization

The *YS1646Δasd*/*ΔFLG*/*ΔpagP*/*ΔansB* strain was further modified to delete *csgD,* a master gene that controls *S. typhimurium* curli fimbriae formation, cellulose production, and c-di-GMP production. The *csgD* deletion eliminates the possibility of cellulose-mediated biofilm formation, reduces pro-inflammatory signaling, and enhances uptake by host phagocytic cells. This increase in intracellular localization would thereby enhance the effectiveness of plasmid delivery and immunomodulatory protein production.

### AcsgD Strain Construction

The *csgD* gene was deleted from the *YS1646Δasd*/*ΔFLG*/*ΔpagP*/*ΔansB* strain, using modifications of the methods described in the preceding examples. Synthetic *csgD* gene homology arm sequences that contained 207 and 209 bases of the left hand and right hand sequence, respectively, flanking the *csgD* gene, were synthesized and cloned into a plasmid called pSL0196 (SEQ ID NO:333). A kanamycin gene cassette flanked by cre/loxP sites then was cloned into plasmid pSL0196, and the *csgD* gene knockout cassette was PCR amplified with primers csgd-1 (SEQ ID NO:323) and csgd-2 (SEQ ID NO:324), gel purified, and introduced into strain YS1646Δ*asd*/ΔFLG/Δ*pagP*/*ansB,* carrying the temperature sensitive lambda red recombination plasmid pKD46, by electroporation. The kanamycin resistance gene then was cured by Cre-mediated recombination as described above, and the temperature-sensitive plasmids were cured by growth at non-permissive temperature. The *csgD* gene knockout sequences were amplified by PCR, using primers csgd-3 (SEQ ID NO:325) and csgd-4 (SEQ ID NO:326), and verified by DNA sequencing. The resulting mutant derivative of parental strain YS1646 was designated YS1646Δ*asd*/ΔFLG/Δ*pagP*/Δ*ansB*/Δ*csgD.*

### csgD-Deleted Strains Cannot Form RDAR Colonies on Congo Red Plates

The ability to form Rough Dry And Red (RDAR) colonies after growth on Congo Red plates is a well-validated assay for bacterial biofilm formation. The Rough and Dry texture occurs through cellulose production, and the red is due to the accumulation of pigment by the curli fimbriae surface structures. For this assay, the YS1646Δ*asd*/ΔFLG/Δ*pagP*/Δ*ansB* strain was compared to the YS1646Δ*asd*/ΔFLG/Δ*pagP*/Δ*ansB*/Δ*csgD* strain for the ability to form the RDAR phenotype after incubation on Congo Red agar plates.

Congo Red agar plates were prepared with soytone (10 g/L) and yeast extract (5 g/L) (modified LB without NaCl), and complemented with Congo red (40 mg/L) and Coomassie brilliant blue G-250 (20 mg/L). Five microliters of a stationary phase bacterial culture was spotted onto Congo Red plates, and incubated at 37 °C for 16 hours, then transferred to 30 °C and incubated for an additional 120 hours. Visual analysis of colony morphology and color was performed and recorded daily to confirm presence or absence of the RDAR colony morphotype.

Comparing the colony morphotypes between the two strains, the YS1646Δ*asd*/ΔFLG/Δ*pagP*/Δ*ansB*/Δ*csgD* strain had a smooth phenotype, and the colonies lacked pigment. In comparison, the YS1646Δ*asd*/ΔFLG/Δ*pagP*/Δ*ansB* strain, still containing the *csgD* gene, exhibited the classic rough and dry appearance, and clear evidence of pigment uptake. Thus, the functional assay confirms that the Δ*csgD* strain is unable to form biofilms, as it lacks curli fimbriae and cellulose production.

### csgD-Deleted Strains Demonstrate Superior Anti-Tumor Efficacy in a Highly Refractory Mouse Model of Triple Negative Breast Cancer

The impact of the *csgD* deletion in models where the immunostimulatory bacterial therapy colonizes tumors, but has shown limited efficacy, was assessed. This can indicate the presence of bacterially-produced cellulose that can limit uptake into tumor-resident myeloid cells, thereby limiting therapeutic benefit (see, *e.g.,* Crull et al. (2011) Cellular Microbiology 13(8):1223-1233). The difficult-to-treat EMT6 model was utilized, which is a representative model of human triple negative breast cancer (see, *e.g.,* Yu et al. (2018) PLoS ONE 13(11):e0206223). When EMT6 tumor cells are administered orthotopically into the mammary fat pad, as opposed to subcutaneously in the flank, the model is T-cell excluded, highly metastatic, and highly refractory to immunotherapy, including to all approved checkpoint antibodies (see, *e.g.,* Mariathasan et al. (2018) Nature 554: 544-548).

For this experiment, 6-8 week-old female BALB/c mice (5 mice per group) were inoculated in the left mammary fat pad with EMT6 tumor cells (ATCC # CRL-2755) (2x10⁵ cells in 100 µL PBS). Mice bearing 13 day-old established mammary tumors (~55 mm³) were IV injected with a single dose of 1 x10⁷ CFUs of the *csgD-*deleted strain YS1646Δ*asd*/ΔFLG/Δ*pagP*/Δ*ansB*/Δ*csgD,* or the parental *YS1646Δasd*/*ΔFLG*/*ΔpagP*/*ΔansB* strain, and compared to PBS control. The bacterial strains contained a plasmid expressing a constitutively active murine STING (EF-1α muSTING R283G).

The tumors in the PBS-treated mice grew evenly, reaching a max tumor volume at day 35 (1199.0 ± 298.1 mm³). Mice treated with the csgD-intact strain, YS1646Δ*asd*/ΔFLG/Δ*pagP*/Δ*ansB*, did not demonstrate evidence of anti-tumor efficacy in this model, also reaching max tumor volume at day 35 (1689.1 ± 537.0). *Ex vivo* LB plating of these tumors revealed all tumors to be colonized. However, the *csgD-deleted* strain, *YS1646Δasd*/*ΔFLG*/*ΔpagP*/*ΔansB*/*ΔcsgD,* resulted in 3 out of 5 mice being completely cured of both their primary and any metastatic disease (day 60+). Overall tumor growth inhibition (TGI) was 45.7%, with one of the other two remaining tumors partially responding before eventually growing out. The two bacterial strains contained the same plasmid payload, yet only one demonstrated significant anti-tumor efficacy. Thus, in one of the most intractable and highly metastatic syngeneic tumor models, orthotopic EMT6, a strain with a *csgD* deletion was able to induce systemic anti-tumor efficacy, and result in 60% complete responses.

### csgD-Deleted Strains Demonstrate Enhanced Intracellular Uptake In Vivo

In order to determine whether the csgD-deleted strain demonstrated improved efficacy because of greater bacterial uptake into tumor-resident myeloid cells, an *ex vivo* gentamicin protection assay was performed (see, Crull et al. (2011) Cellular Microbiology 13(8):1223-1233). For this experiment, 6-8 week-old female C57BL/6 mice (4 mice per group) were inoculated SC in the right flank with MC38 cells (5x10⁵ cells in 100 µL PBS). Mice bearing large established flank tumors were IV injected on day 17 with 1x10⁷ CFUs of the csgD-deleted *YS1646Δasd*/*ΔFLG*/*ΔpagP*/*ΔansB*/*ΔcsgD* strain (N=12), or the parental YS1646 strain (N=4). Tumors were resected 7 days post IV dosing, weighed, and minced in RPMI supplemented with 1 mg/mL collagenase IV and 20 mg/mL DNase I, and incubated with shaking at 37 °C for 30 minutes to generate a single cell suspension. After 30 minutes, the suspension was passed through a 70 mm filter, and the recovered volume was divided into two separate, identical samples. Gentamicin (Thermo Fisher Scientific) was added at 200 mg/mL to one of each of the paired samples to kill extracellular bacteria, and the samples were incubated with shaking at 37 °C for 90 minutes. Cell suspension samples were then washed and lysed with 0.05% Triton X, and plated on LB agar plates to enumerate for CFUs.

The results demonstrate that, compared to the CFUs from YS1646-treated tumors without gentamicin treatment (11925 ± 19859 CFUs), gentamicin treatment resulted in very few CFUs detected from the tumors (51 ± 45 CFUs). This indicates that the bacteria reside largely extracellularly in these tumors, and are thus sensitive to gentamicin elimination. In the csgD-deleted YS1646Δ*asd*/ΔFLG/Δ*pagP*/Δ*ansB*/Δ*csgD* treatment group, the non-gentamicin treated tumors yielded high CFUs, as expected from well-colonized tumors, and treatment with gentamicin yielded less CFUs (1276 ± 2410 CFUs), and much more than in the parental YS1646 strain-treated tumors. This is due to more of the *csgD-*deleted bacteria residing intracellularly, and thus, being protected from gentamicin. These data demonstrate that the *csgD* deletion improves intracellular uptake of the bacteria, which can enhance plasmid delivery of immunomodulatory proteins *in vivo.*

### Example 8

### pATI-1.75 Vector Construction

A plasmid (pATI-1.75) was designed and synthesized that contains the following features: a pBR322 origin of replication, the *asd* gene, a kanamycin resistance gene flanked by HindIII sites for curing, and a multiple cloning site for expression cassette insertion. The expression cassette is composed of multiple elements, including eukaryotic promoters, open reading frames (ORFs), posttranscriptional regulatory elements, and polyadenylation signals, that are assembled in various configurations.

Exemplary promoters include the human cytomegalovirus (CMV) immediate early core promoter encoded directly downstream of the CMV immediate early enhancer sequence, and the core promoter for human elongation factor-1 alpha (EF-1α). Open reading frames (ORFs) can include one or more sequences that each are translated into a protein, and can be separated into distinct polypeptides by insertion of a 2A sequence, whereby eukaryotic ribosomes fail to insert a peptide bond between Gly and Pro residues within the 2A sequence. Examples of 2A sequences are the T2A peptide (SEQ ID NO:327) from the *Thosea asigna* virus (TaV) capsid protein, and the P2A peptide (SEQ ID NO:328) from porcine teschovirus (PTV). Upstream furin cleavage sites (RRKR), and other enhancer elements, are placed upstream to facilitate cleavage to separate the expressed proteins.

Examples of post-transcriptional regulatory elements (PREs) include the Woodchuck Hepatitis virus PRE (WPRE; SEQ ID NO:346), and the Hepatitis B virus PRE (HPRE; SEQ ID NO:347), which increase accumulation of the cytoplasmic mRNA of a gene by promoting mRNA nuclear export to the cytoplasm, enhancing 3' end processing and stability. Examples of polyadenylation signal sequences include the SV40 polyadenylation signal, and the bovine growth hormone polyadenylation signal, both of which are 3' regulatory elements that serve to promote transcriptional termination, and contain the sequence motif recognized by the RNA cleavage complex.

### Example 9

### Designed Heterologous Protein Expression Plasmids Induce Functional Protein Production from Human Cells

### Optimal Expression of Cytokines Established in Human Cells

In order to exemplify that immunostimulatory cytokines can be expressed from designed plasmids in human cells, a panel of cytokines were cloned into the pATI-1.75 plasmid, under the control of the EF-1α promoter. The cytokines include, but are not limited to, murine IL-2 (muIL-2), muIL-12p70, muIL-23, and human IL-2 (huIL-2). For the muIL-15 Receptor-α fused to an IL-15 single chain (muIL-15Rα-IL-15sc), an EF-1α and CMV promoter were tested. HEK293T STING Null cells (InvivoGen) were seeded in 24-well plates coated with poly-L-lysine at 200,000 cells per well, overnight at 37 °C in a 5% CO₂ incubator, to achieve 80% confluency. The following day, 200 ng of each cytokine plasmid DNA was diluted in serum-free media and added to FuGENE^{®} transfection reagent (Promega), at the proper reagent:DNA ratios, with untransfected wells as negative controls (in duplicates). Cell culture supernatants from each sample were collected at 24 hours post-transfection and assessed for protein expression by ELISAs specific for each cytokine.

The muIL-2 construct was evaluated in a murine IL-2 ELISA (R&D Systems), according to the manufacturer's instructions, and an additional version of muIL-2 with codon optimization (muIL-2 CO) also was evaluated. Concentrations of neat supernatant were tested, and yielded an average of 1680 pg/mL of muIL-2 for the muIL-2 construct, and 1812 pg/mL of muIL-2 for the muIL-2 CO construct. These data confirmed the functionality of the constructs, and demonstrated that yield could be improved with codon optimization. The muIL-12p70 construct was evaluated in a murine IL-12 ELISA (R&D Systems), according to the manufacturer's protocol. When supernatants were added neat, a mean of 400 pg/mL of secreted muIL-12p70 was measured, although this was outside the linear range. When the supernatants were diluted 5-fold, an average of 105 pg/mL of secreted muIL-12p70 was detected. For the muIL-23 plasmid, detection of protein was achieved using the murine IL-23 ELISA (BioLegend), per kit instructions. With the supernatant added neat, a mean of 966 pg/mL of muIL-23 was detected. For the human IL-2 plasmid, detection of protein was achieved using the human IL-2 ELISA (Invitrogen), per kit instructions. With the supernatant added neat, an average of 1422 pg/mL of huIL-2 was detected. For the muIL-15Rα-IL-15sc construct, expressed using either the EF-1α or CMV promoters, the murine IL-15 ELISA (eBioscience, Inc.) was used, per kit instructions. When added neat, the muIL-15Rα-IL-15sc plasmid with the EF-1α promoter resulted in an average of 131 pg/mL, while the muIL-15Rα-IL-15sc plasmid with the CMV promoter resulted in an average of 289 pg/mL.

These data validate the plasmid expression constructs encoding immunomodulatory cytokines, both mouse and human, in human cells. Further, they indicate that codon optimization, and the use of promoters such as CMV, can enhance protein expression.

### Post-Transcriptional Regulatory Elements Enhance Cytokine Expression

In order to determine whether post-transcriptional regulatory elements (PREs), added at the 3' end of the ORF, enhance expression of immunostimulatory cytokines in human cells, expression of huIL-2, under the control of the EF-1α promoter, was tested with or without the addition of a Woodchuck Hepatitis virus post-transcriptional regulatory element (WPRE) in the pATI-1.75 plasmid.

HEK293T STING Null cells (InvivoGen) were seeded in 24-well plates coated with poly-L-lysine at 200,000 cells per well, overnight at 37 °C in a 5% CO₂ incubator, to achieve 80% confluency. The following day, 200 ng of each cytokine plasmid DNA was diluted in serum-free media and added to FuGENE^{®} transfection reagent (Promega), at the proper reagent:DNA ratios, with untransfected wells as negative controls (in duplicates). Cell culture supernatants from each sample were collected at 24 hours post-transfection and assessed for activity by a human IL-2 ELISA (Invitrogen), according to the manufacturer's instructions. Supernatants were added neat, or were diluted 5-fold.

The results demonstrated that, when supernatant was added neat, compared to the huIL-2 construct without a WPRE, which secreted an average of 1540 pg/mL, the huIL-2 construct with the WPRE secreted 5511 pg/mL, a 3.6-fold increase. In the 5-fold diluted supernatants, the non-WPRE huIL-2 construct secreted 315 pg/mL of huIL-2, while the huIL-2 construct with the WPRE secreted 1441 pg/mL, a 4.6-fold increase. Thus, addition of 3' post-transcriptional regulatory elements, exemplified by, but not limited to, WPRE, can significantly improve protein expression in human cells.

### Promoter Optimization and Post-Transcriptional Regulatory Elements Enhance Cytokine Production in Primary M2 Macrophages

While expression of cytokines, such as muIL-15Rα-IL-15sc, was enhanced in human HEK293T cells by use of the CMV promoter, it was determined whether expression of cytokines could similarly be enhanced in donor-derived primary human M2 macrophages, the predominant macrophage phenotype in T-cell excluded, solid human tumors. Additionally, it was determined whether post-transcriptional regulatory elements, such as WPRE, could enhance expression in these cells.

In order to determine if protein expression could be improved, the promoters EF-1α and CMV were tested for controlling expression of muIL-2, and the WPRE post-transcriptional regulatory element was tested for expression of huIL-2. Frozen human PBMCs, isolated from healthy human donors, were thawed in complete medium (RPMI-1640 + 1X non-essential amino acids + 5% Human AB serum), and washed by centrifugation for 10 minutes at 800 RPM at room temperature. PBMCs were resuspended in PBS + 2% FBS, and monocytes were negatively isolated using a CD16 depletion kit (StemCell Technologies). The isolated monocytes were cultured for 6 days in RPMI media containing M-CSF and IL-4, to generate M2 macrophages. For this, isolated untouched monocytes were washed by centrifugation in PBS + 2% FBS, and resuspended in complete medium containing 100 ng/mL human M-CSF and 10 ng/mL human IL-4. Isolated monocytes (3e5 per well) were then seeded in a 24-well plate with a final volume of 750 microliters. Two days after the seeding, the cell culture media was entirely aspirated and replaced with fresh complete medium containing 100 ng/mL human M-CSF and 10 ng/mL human IL-4. Two days later (on day 4), 500 µL of complete medium containing 100 ng/mL human M-CSF and 10 ng/mL human IL-4 was added per well, and incubated for 48 hours. On day 6, the cell culture media was entirely aspirated, and replaced with fresh complete medium without cytokines, for transfection with the Viromer^{®} RED mRNA and plasmid transfection reagent (Lipocalyx).

Transfection with Viromer^{®} RED was performed according to the manufacturer's instructions. Briefly, 500 ng of plasmid DNA, containing the EF-1α-muIL-2 construct, the CMV-muIL-2 construct, the EF-1α-huIL-2 construct, or the EF-1α-huIL-2 + WPRE construct, as well as untransfected control, were diluted in the provided buffer, and mixed with 0.2 µL of Viromer^{®} RED, and incubated at room temperature for 15 minutes to allow the Viromer^{®} complexes to form. The DNA/Viromer^{®} RED complexes were then slowly added to each well of the 24-well plate (in duplicates), and the plate was incubated at 37 °C in a CO₂ incubator for 24 hours. Supernatants were harvested at 24 hours, and assayed for cytokines using either a murine IL-2 ELISA (R&D Systems), or a human IL-2 ELISA (Invitrogen), per kit instructions.

The results demonstrated that expression of muIL-2 from neat supernatants harvested from primary human M2 macrophages, transfected with the muIL-2 construct under control of the EF-1α promoter, resulted in the secretion of an average of 59.7 pg/mL of muIL-2. The muIL-2 construct with the CMV promoter yielded an average of 275 pg/mL muIL-2, an almost 5-fold increase. For the human IL-2 ELISA, neat supernatants from the cells transfected with the plasmid lacking the WPRE yielded an average of 170 pg/mL huIL-2. The huIL-2 construct containing the WPRE yielded an average of 219 pg/mL huIL-2. These data confirm that promoters such as CMV, and post-transcriptional regulatory elements such as WPRE, can significantly improve cytokine expression in multiple cells types, including primary human M2 macrophages.

### Co-Stimulatory Receptor Ligand 4-1BBL Expressed from Human Cells

Co-stimulatory molecules, such as 4-1BBL, when expressed on antigen-presenting cells (APCs), can engage 4-1BB expressed on T-cells to promote optimal T-cell function. 4-1BBL is negatively regulated by its cytoplasmic signaling domain. In the late-phase of 4-1BBL ligation of macrophages to T-cells, reverse signaling of the 4-1BBL cytoplasmic domain induces surface translocation of 4-1BBL to bind and form a signaling complex with TLR4. This induces high levels of TNF-α, comparable to LPS activation of TLR4, that leads to immunosuppression of the adaptive immune response (see, *e.g.,* Ma *et al.* (2013) *Sci. Signal.* 6(295):ra87).

In this example, the sequence encoding murine 4-1BBL was cloned into the pATI-1.75 vector. In order to maximally engage T-cells, the reverse signaling of the 4-1BBL cytoplasmic domain was eliminated by deleting the cytoplasmic domain (corresponding to amino acid residues 1-82 of SEQ ID NO:344), generating mu4-1BBLΔcyt. To determine whether mu4-1BBLΔcyt could be functionally expressed on the surface of human cells, HEK-293T cells were utilized. HEK293T STING Null cells (InvivoGen) were seeded in 24-well plates coated with poly-L-lysine at 200,000 cells per well, overnight at 37 °C in a CO₂ incubator, to achieve 80% confluency. The following day, 200 ng of plasmid DNA, encoding mu4-1BBLΔcyt, was diluted in serum-free media and added to FuGENE^{®} transfection reagent (Promega), at the proper reagent:DNA ratio, with untransfected wells as a negative control (in duplicates). After 48 hours, the cells were washed twice with PBS + 2% FBS by centrifugation at 1300 RPM for 3 minutes. The cells were then resuspended in PBS + 2% FBS, and stained with a PE-conjugated murine anti-4-1BBL antibody (clone TKS-1, BioLegend) and DAPI (dead/live stain). After 30 minutes, the cells were washed twice with PBS + 2% FBS by centrifugation at 1300 RPM for 3 minutes, and resuspended in PBS + 2% FBS. Flow cytometry data were acquired using the ACEA NovoCyte^{®} flow cytometer (ACEA Biosciences, Inc.) and analyzed using the FlowJo^{™} software (Tree Star, Inc.).

As a percentage of live cells, the untransfected control cells showed a percent positive staining for murine 4-1BBL of 14.6. In comparison, 93.4% of the cells that were transfected with the plasmid encoding mu4-1BBLΔcyt, were positive for surface expression of 4-1BBL. These data demonstrate that the pATI-1.75 plasmid can effectively be used to express 4-1BBL at high levels on the surface of human cells.

### Soluble TGFβ Receptor II Expressed from Human Cells

Soluble mouse TGFβ receptor II variants were designed by removing the cytoplasmic and transmembrane portions of the full TGFβ receptor II. Additionally, either a FLAG or Fc tag was added for detection. These variants were cloned into the pATI-1.75 vector under the control of a CMV promoter and a 3' WPRE element. The sequences were confirmed by Sanger sequencing. 1.5 x 10⁶ HEK293T cells were plated one day prior on 6-well plates coated with poly-L-lysine, to achieve 80% confluency. On the day of transfection, 3 µg of DNA was diluted in serum-free media and added to FuGENE^{®} transfection reagent (Promega) at the proper reagent:DNA ratios. Cell culture supernatants from each sample were collected after 48 hours of incubation. Some supernatant was concentrated in a 10 kDa spin column (Millipore). Direct ELISAs with mouse TGF-β1 (R&D systems) were performed on the supernatant of transfected HEK293T cells. ELISA data, with absorbance at 450 nm, is provided in the table below.

| **Construct** | **Absorbance at 450 nm** |
|---|---|
| Concentrated soluble mouse TGFβ receptor II-Fc | 1.522 ± 0.025 |
| Soluble mouse TGFβ receptor II-Fc | 1.508 ± 0.018 |
| Media (control) | 0.041 ± 0.002 |

The functionality of these constructs was tested in a T-cell assay. Mouse T-cells were harvested from the spleen using a magnetic isolation kit (StemCell Technologies). T-cells were incubated with anti-mouse CD3ε antibody, with or without the soluble receptor, at various concentrations of mouse TGF-beta. T-cell activation was quantified using the mouse TH1 CBA kit (BioLegend), and flow cytometry labeling of CD4, CD8, 4-1BB, and CD69.

These data demonstrate the ability to express heterologous molecules, such as extracellular receptors fused to an Fc domain, from the plasmid engineered for delivery by the immunostimulatory bacteria to eukaryotic, such as human, cells.

### Expression of a CD3xCD19 Bispecific T-cell Engager from Human Cells

A CD3xCD19 bispecific T-cell engager (BiTE^{®}), containing a FLAG tag and a His tag, was cloned into the pATI-1.75 vector, under the control of a CMV promoter and with a 3' WPRE element. The sequences were confirmed by Sanger sequencing. 1.5 x 10⁶ HEK293T cells were plated one day prior on 6-well plates coated with poly-L-lysine, to achieve 80% confluency. On the day of transfection, 3 µg of DNA was diluted in serum-free media and added to FuGENE^{®} transfection reagent (Promega) at the proper reagent:DNA ratios. Cell culture supernatants from each sample were collected after 48 hours of incubation. Some supernatant was concentrated in a 10 kDa spin column (Millipore).

The functionality of this construct was tested by binding of the CD3xCD19 BiTE^{®} to Raji and Jurkat-Lucia^{™} NFAT cells (InvivoGen). The BiTE^{®} was detected using an anti-FLAG-APC (BioLegend), using flow cytometry. One well of 50,000 cells was run for each condition. The mean fluorescence intensity (MFI) of the APC positive events, and the number of cells gated as APC positive, are provided in the table below.

| **Sample** | **Number of APC⁺ Cells** | **Mean Fluorescence Intensity (MFI) of APC⁺ Cells** |
|---|---|---|
| Raji cells with anti-FLAG-APC only | 41 | 1439 |
| Raji cells with CD3xCD19 BiTE^{®} | 10307 | 7408 |
| Raji cells with concentrated CD3xCD19 BiTE^{®} | 11248 | 16045 |
| Jurkat cells with anti-FLAG-APC only | 100 | 1519 |
| Jurkat cells with CD3xCD19 BiTE^{®} | 18482 | 4584 |
| Jurkat cells with concentrated CD3xCD19 BiTE^{®} | 17563 | 14089 |

Additionally, this construct was tested in a co-culture of Raji and Jurkat-Lucia^{™} NFAT cells. Cells were incubated with or without the CD3xCD19 BiTE^{®}, and the luminescence (corresponding to the NFAT reporter) was detected at 6 hours and 24 hours post-addition of BiTE^{®}. The luminescence readings of this assay are provided in the table below.

| **Experimental Conditions** | **Concentrated CD3xCD19 BiTE^{®} (Luminescence)** | **CD3xCD19 BiTE^{®} (Luminescence)** | **Medium (Luminescence)** |
|---|---|---|---|
| 2 Raji: 1 Jurkat, 6 hour time point | 12110.5 ± 837.9 | 3445.0 ± 717.0 | 44.5 ± 10.6 |
| 1 Raji: 1 Jurkat, 6 hour time point | 4337.0 ± 219.2 | 2057.5 ± 20.5 | 26.0 ± 15.6 |
| 2 Raji: 1 Jurkat, 24 hour time point | 47159.5 ± 1038.7 | 11274 ± 408.7 | 114.5 ± 57.3 |
| 1 Raji: 1 Jurkat, 24 hour time point | 18614 ± 1540.1 | 6017 ± 31.1 | 122 ± 15.6 |

These data demonstrate the ability to express heterologous molecules, such as scFvs, alternative antibody constructs, and bispecific T-cell engagers, in eukaryotic, such as human, cells, from the engineered plasmid that can be delivered by the immunostimulatory bacteria herein.

### Example 10

### Immunostimulatory Bacterial Strains Efficiently Deliver Plasmids and Express Cytokines in Human Cells

### Flagella-Deleted Strains Containing Plasmids Encoding Murine IL-2 Induce Functional IL-2 Protein Expression Following Infection in Human Monocytes

As described above, the flagellin genes, fljB and fliC, were deleted from the YS1646 strain of *S. typhimurium* with the *asd* gene deleted, generating the strain YS1646Δ*asd*/ΔFLG. This strain was electroporated with a plasmid containing an expression cassette with the EF-1α promoter and the murine cytokine IL-2 (muIL-2). In addition, the YS1646Δ*asd*/ΔFLG strain was electroporated with an expression plasmid encoding murine IL-15δ, as a control for a non-cognate cytokine. Additional constructs were created using the CMV promoter.

To determine whether these strains containing expression plasmids can infect human monocytes and induce the production of murine IL-2, THP-1 human monocytic cells were plated at 50,000 cells/well in RPMI 1640 (Gibco^{™}) + 10% Nu-Serum^{™} (Corning^{®}), one day prior to infection. The cells were infected with the various strains at an MOI of 50 for one hour in RPMI, then washed 3 times with PBS, and resuspended in RPMI + 100 µg/mL gentamicin (Sigma). Supernatants were collected 48 hours later from a 96-well plate, and assessed for the concentration of murine IL-2 by ELISA (R&D Systems).

The concentration of muIL-2 detected in the YS1646Δ*asd*/ΔFLG-IL15δ control wells was very low (6.52 pg/mL), as expected, and likely reflective of some cross-reactivity to the endogenous human IL-2 receptor. In contrast, the YS1646Δasd/ΔFLG-muIL-2 strain induced an average of 35.1 pg/mL of muIL-2. These data demonstrate the feasibility of expressing and secreting functional heterologous proteins, such as IL-2, from the *S. typhimurium* immunomodulatory platform strains, in human monocytes.

### Flagella-Deleted and pagP-Deleted Strains, Containing Plasmids Encoding Murine IL-2, Demonstrate Enhanced IL-2 Expression Compared to Transfected muIL-2 DNA in Primary Human M2 Macrophages

The relative efficiencies of transfection *(i.e.,* direct transfer of plasmid DNA) vs. bactofection (*i.e.,* transfer of plasmid DNA by the immunostimulatory bacterial strains herein), in primary human M2 macrophages, for expression of muIL-2, were compared. Frozen human PBMCs, isolated from healthy human donors, were thawed in complete medium (RPMI-1640 + 1X non-essential amino acids + 5% Human AB serum), and washed by centrifugation for 10 minutes at 800 RPM at room temperature. PBMCs were resuspended in PBS + 2% FBS, and monocytes were negatively isolated using a CD16 depletion kit (StemCell Technologies). Isolated untouched monocytes were then washed by centrifugation in PBS + 2% FBS, and resuspended in complete medium containing 100 ng/mL human M-CSF and 10 ng/mL human IL-4. Isolated monocytes (3e5 per well) were then seeded in a 24-well plate, with a final volume of 750 microliters. Two days after the seeding, the cell culture media was entirely aspirated and replaced with fresh complete medium containing 100 ng/mL human M-CSF and 10 ng/mL human IL-4. Two days later (on day 4), 500 µL of complete medium containing 100 ng/mL human M-CSF and 10 ng/mL human IL-4 was added per well, and incubated for 48 hours. On day 6, the cell culture media was entirely aspirated, and replaced with fresh complete medium without cytokines, for transfection with Viromer^{®} RED mRNA and plasmid transfection reagent (Lipocalyx).

Transfection with Viromer^{®} RED was performed according to the kit instructions. Briefly, 500 ng of plasmid DNA from the constructs encoding muIL-2 under control of the EF-1α promoter (EF-1α-muIL-2), or under control of the CMV promoter (CMV-muIL-2), or untransfected control, were diluted in the provided buffer, mixed with 0.2 µL of Viromer^{®} RED transfection reagent, and incubated at room temperature for 15 minutes to allow the DNA/Viromer^{®} RED complexes to form. The DNA/Viromer^{®} RED complexes were then slowly added to each well of the 24-well plate containing the monocytes (in duplicates), and the plate was incubated at 37 °C in a CO₂ incubator for 24 hours. Additional wells of cells were infected at an MOI of 450 with the YS1646Δ*asd*/ΔFLG/Δ*pagP* strain containing the EF-1α-muIL-2 construct, or the YS1646Δ*asd*/ΔFLG/Δ*pagP* strain containing the CMV-muIL-2 construct, for one hour in RPMI, then washed 3 times with PBS, and resuspended in RPMI + 100 µg/mL gentamicin (Sigma).

After 24 hours, the cells were lysed with 350 µL Buffer RLT with β-mercaptoethanol (β-ME) (Qiagen), and RNA extraction was performed using the Qiagen RNeasy Mini Kit with the following modifications. A genomic DNA elimination step, using an RNase-Free DNase kit (Qiagen) was included in the kit to remove genomic DNA from the total RNA. Total RNA concentration was measured using a NanoDrop^{™} One^{C} UV-Vis Spectrophotometer (Thermo Fisher Scientific). The purity of each sample also was assessed from the A₂₆₀/A₂₃₀ absorption ratio. RNA was stored at -80 °C without freeze-thawing until reverse-transcription was performed. cDNA synthesis was performed using 0.4-1 µg of template RNA using a C1000 Touch Thermal Cycler (Bio-Rad) and SuperScript^{™} VILO^{™} Master Mix (Invitrogen) in a 30 µL reaction, according to the manufacturer's instructions.

qPCR (quantitative polymerase chain reaction) was performed with a CFX96^{™} Real-Time PCR Detection System (Bio-Rad). SYBR^{®} primers for murine IL-2 (Assay ID: qMmuCED0060978) were purchased from Bio-Rad. The qPCR reaction (20 µL) was conducted per protocol, using the iTaq^{™} Universal SYBR^{®} Green Supermix (Bio-Rad). The standard thermocycling program on the Bio-Rad CFX96^{™} Real-Time System consisted of a 95 °C denaturation for 30 seconds, followed by 40 cycles of 95 °C for 5 seconds and 60 °C for 30 seconds. Reactions with template-free control were included for each set of primers on each plate. All samples were run in duplicate, and the mean C_{q} values were calculated. Quantification of the target mRNA was normalized using Gapdh (glyceraldehyde-3-phosphate dehydrogenase) reference mRNA (Bio-Rad, Assay ID: qMmuCED0027497). ΔC_{q} was calculated as the difference between target (mu-IL2) and reference (Gapdh) gene. ΔΔC_{q} was obtained by normalizing the ΔC_{q} values of the treatments, to the ΔC_{q} values of the non-treatment controls. Fold increase was calculated as 2^-ΔΔC_{q}. The fold increases relative to untransfected/uninfected control are shown in the table below.

| **Treatment Group** | **Fold Increase in muIL-2** |
|---|---|
| YS1646 | <1 |
| YS1646Δ*asd*/ΔFLG/Δ*pagP*, EF-1α-muIL-2 (infection) | 74 |
| YS1646Δ*asd*/ΔFLG/Δ*pagP,* CMV-muIL-2 (infection) | 668.2 |
| Transfection, EF-1α-muIL-2 | 249.4 |
| Transfection, CMV-muIL-2 | 1527 |

The results show that, with either transfection or bactofection, the CMV promoter demonstrated superior expression of muIL-2, compared to the EF-1α promoter, in primary human M2 macrophages. While transfection using the most efficient reagent currently available gave the highest levels of muIL-2 expression, bactofection also elicited high expression levels of muIL-2, demonstrating the high efficacy of heterologous gene transfer with the bacterial platforms provided herein.

### Example 11

### Bacterial Strains Efficiently Deliver Immunomodulatory Plasmids In Vivo and Demonstrate Potent Anti-tumor Activity

### Flagella-Deleted Strains Containing Plasmids Encoding Murine IL-2 Induce Potent Tumor Inhibition without Toxicity in a Mouse Model of Colorectal Carcinoma

In order to demonstrate that *S. typhimurium* strains containing the muIL-2 expression plasmids can induce anti-tumor efficacy without additional toxicity, the YS1646Δ*asd*/ΔFLG strain containing the muIL-2 plasmid was compared to PBS control for safety and efficacy in the subcutaneous flank MC38 colorectal adenocarcinoma model. For this study, 6-8 week-old female C57BL/6 mice (5 mice per group) were inoculated SC in the right flank with MC38 cells (5x10⁵ cells in 100 µL PBS). Mice bearing established flank tumors were IV injected on day 11 with 5x10⁵ CFUs of the YS1646Δasd/ΔFLG-muIL-2 strain, or with PBS vehicle control. Tumor measurements and body weights were recorded twice weekly.

The results revealed that the YS1646Δ*asd*/ΔFLG-muIL-2 strain demonstrated significant tumor growth inhibition (TGI) compared to PBS (76.7% TGI, *P* = 0.005, day 21), with tumors being well-controlled out to day 40 post-implantation, when the PBS mice were euthanized. The therapy was well tolerated, even without further strain attenuation, and the weight loss early on was transient and resulted in only a 3.4% reduction in body weight, compared to PBS control at day 40. Thus, the immunostimulatory strain expressing muIL-2 potently inhibits tumor growth inhibition, in a safe and non-toxic manner, in a model of colorectal carcinoma.

### Flagella-Deleted Strains Containing Plasmids Encoding Murine IL-2 Induce Tumor-Specific Production of IL-2 In Vivo

The level of tumor muIL-2 expression, relative to spleen, was determined in order to confirm the tumor-specific nature of delivery. 6-8 week-old female C57BL/6 mice (5 mice per group) were inoculated SC in the right flank with MC38 colorectal adenocarcinoma cells (5x10⁵ cells in 100 µL PBS). Mice bearing established flank tumors were IV injected on day 10 with 5x10⁵ CFUs of strain YS1646Δ*asd*/ΔFLG-muIL-2, or with PBS vehicle control. On day 31 post tumor implantation, tumors and spleens were excised and processed for tumor extracts using the GentleMACS^{™} Octo Dissociator and the M tubes (Miltenyi Biotec) molecule setting in 2 mL of PBS. The homogenates were spun down at 1300 RPM for 10 minutes, and the supernatant was collected and assayed using the muIL-2 CBA kit (BD Biosciences), according to the manufacturer's instructions. Results were quantified as pg/mL of muIL-2, and standardized to per gram of tissue.

The PBS control tumors exhibited background levels of muIL-2 in the tumor, with a mean of 134 pg/mL per gram of tumor tissue. The YS1646Δ*asd*/ΔFLG-muIL-2 treated tumors yielded a much higher mean of 389.9 pg/mL muIL-2 per gram of tumor tissue, demonstrating the ability to detect elevated muIL-2 levels due to plasmid delivery in the tumor-resident myeloid cells. The level of muIL-2 in the spleen, from mice injected with strain YS1646Δ*asd*/ΔFLG-muIL-2, was an average of 6.6 pg/mL per gram of tissue, which was lower than in the PBS controls. This specificity for the tumor enables delivery of immunomodulatory levels of IL-2, in a much safer manner than conventional cytokine therapies, which are not tumor-targeted.

### Attenuated Bacterial Strains Containing Plasmids Encoding Murine Co-stimulatory Receptor Ligand 4-1BBL Demonstrate Curative Effects In Vivo

In order to determine whether tumor-specific delivery of a co-stimulatory molecule, such as 4-1BBL, enhances anti-tumor efficacy, a bacterial strain containing a plasmid encoding 4-1BBL(Δcyt) (described above), under control of the CMV promoter and containing a 3' WPRE element, was assessed in the MC38 murine model of colorectal adenocarcinoma. For this study, 6-8 week-old female C57BL/6 mice (5 mice per group) were inoculated SC in the right flank with MC38 colorectal adenocarcinoma cells (5x10⁵ cells in 100 µL PBS). Mice bearing established flank tumors were IV injected on day 10 with 1x10⁷ CFUs of strain *YS1646Δasd*/*ΔFLG*/*ΔpagP*/*ΔansB* containing the CMV-4-1BBL(Δcyt)-WPRE plasmid, or with PBS vehicle control.

The therapy was very well tolerated, with only an initial weight loss of 2.2% that had fully recovered 3 days later. Compared to PBS, the 4-1BBL(Δcyt) therapy was highly effective and curative (90.7% TGI, 60% complete response (CR), day 30). These data demonstrate the potency and safety of delivering immunostimulatory bacteria containing plasmids encoding co-stimulatory molecules in a tumor-specific manner.

### Example 12

### Identification of Gain-of-Function Mutations in Genes That Promote Constitutive Type I Interferon Production

Instances of subjects presenting with severe auto-inflammatory conditions and vasculopathies of unknown etiology occur, and, often derive from mutations. The cause for these conditions has, and can be, identified. Steps to identify a mutational basis for such a pathology are as follows. In step one, intact genomic DNA is obtained from patients experiencing symptoms, and from healthy individuals. Whole exome sequencing is performed, then introns and exons are analyzed. Analysis of genes, and identification of mutations in products in the pathways associated with the expression of type I interferon (IFN), is performed. From this analysis, mutations are discovered in genes known to lead to constitutive functional activation of the encoded proteins, and subsequent persistent expression of type I IFN.

After identification of mutations, cDNAs encoding the full-length gene, with and without the identified mutation(s), are transfected into a reporter cell line that measures expression of type I IFN. For example, a reporter cell line can be generated where the expression of luciferase is placed under control of the promoter for IFN-β. A gain-of-function (GOF) mutant that is constitutively active will promote the expression of IFN-β, whereas the unstimulated wild-type (WT) protein will not. In the case of known STING SAVI (STING-associated vasculopathy with onset in infancy) mutants, the WT-STING stimulation of IFN-β requires the addition of increasing exogenous levels of cGAMP to directly activate WT-STING. Constitutively active mutations stimulate the expression of IFN-β in a cGAMP-independent manner. Exemplary gain-of-function mutations in each of STING, RIG-I, MDA5, IRF3, and IRF7, are set forth below in Example 15, and discussed elsewhere herein. Other such genes, in which gain-of-function mutations can be identified in subjects or produced by *in vitro* mutation and screening, include, but are not limited to TRIM56, RIP1, Sec5, TRAF3, TRAF2, TRAF6, STAT1, LGP2, DDX3, DHX9, DDX1, DDX9, DDX21, DHX15, DHX33, DHX36, DDX60, and SNRNP200.

### Expression of Functional Constitutive Type I IFN Mutants in Human Cells

Human STING (allele R232) and IRF3 gain-of-function (GOF) mutants (see, table below) were cloned into the pATI-1.75 vector, and the sequences were confirmed by PCR. To determine whether the STING and IRF3 GOF expression plasmids could induce functional type I IFN in human cells, the plasmids were assessed using HEK293T STING Null Reporter cells (InvivoGen), which do not contain endogenous STING. These cells express secreted embryonic alkaline phosphatase (SEAP), placed under the control of the endogenous IFN-stimulated response element (ISRE) promoter, where the coding sequence of ISRE has been replaced by the SEAP ORF using knock-in technology. Type I interferon activity can be assessed by monitoring Type I IFN-stimulated SEAP production in the cell supernatants.

To test the relative production of type I IFN by each of the GOF mutants, 1x10⁵ 293T-Dual^{™} Null cells (InvivoGen) were plated one day prior on plates coated with poly-L-lysine, to achieve 80% confluency, in a 24-well plate. On the day of transfection, 200 ng of plasmids encoding a panel of STING and IRF3 GOF mutants, including a STING wild-type (WT) and IRF3 WT control, and a negative control mutation that has been reported in the literature to be non-functional in human cells (STING V155R negative control (NC)), were diluted in serum-free media and added to FuGENE^{®} transfection reagent (Promega) at the proper reagent:DNA ratios. Cell culture supernatants from each sample were collected after overnight incubation, and 10 µL of the cell culture supernatants was added to 50 µL QUANTI-Blue^{™} reagent (InvivoGen), which is used for measuring SEAP. Type I interferon activation was determined by measuring ISRE-induced SEAP activity on a SpectraMax^{®} M3 Spectrophotometer (Molecular Devices), at an absorbance of 650 nm.

As shown in the table below, all GOF mutants were able to induce type I IFN activity in a STING ligand-independent manner in human cells, compared to the wild-type and negative controls, which did not induce type I IFN activity. The highest levels of type I IFN induction were observed with the human STING R284G variant, and the human IRF3 S396D phosphomimetic variant. These data support the ability of the plasmids encoding GOF mutants to produce functional, constitutive STING and constitutive phosphomimetic IRF3, that can induce type I IFN in a cGAMP-independent manner.

| **GOF Mutant** | **Mean Absorbance (650nm)** | **Standard Deviation** |
|---|---|---|
| Plasmid control | 0.049 | 0.002 |
| huSTING WT | 0.144 | 0.004 |
| huSTING V147L | 1.399 | 0.015 |
| huSTING N154S | 1.382 | 0.008 |
| huSTING V155M | 1.360 | 0.048 |
| huSTING C206Y | 1.566 | 0.121 |
| huSTING R281Q | 1.546 | 0.132 |
| huSTING R284G | 1.831 | 0.039 |
| huSTING V155R (NC) | 0.181 | 0.014 |
| huIRF3 WT | 0.781 | 0.073 |
| huIRF3 S396D | 1.922 | 0.131 |

### Infection of Flagella-Deleted Strains Containing Plasmids Encoding Constitutive Type I IFN Mutants Converts Human M2 Macrophages to Type I IFN-Producing M1 Macrophages

It was determined if primary human M2 macrophages, infected with flagella-deleted strains containing plasmids encoding constitutive type I IFN GOF variants, could be converted to producers of type I IFN and downstream chemokines, such as CXCL10 (also known as IP-10).

Frozen human PBMCs, isolated from healthy human donors, were thawed in complete medium (RPMI-1640 + 1X non-essential amino acids + 5% Human AB serum), and washed by centrifugation for 10 minutes at 800 RPM at room temperature. PBMCs were resuspended in PBS + 2% FBS, and monocytes were negatively isolated using a CD16 depletion kit (StemCell Technologies). To generate primary human M2 macrophages, isolated untouched monocytes were washed by centrifugation in PBS + 2% FBS, and resuspended in complete medium containing 100 ng/mL human M-CSF and 10 ng/mL human IL-4. Isolated monocytes (3e5 per well) were then seeded in a 24-well plate with a final volume of 750 microliters. Two days after the seeding, the cell culture media was entirely aspirated and replaced with fresh complete medium containing 100 ng/mL human M-CSF and 10 ng/mL human IL-4. Two days later (on day 4), 500 µL of complete medium containing the cytokines was added per well and incubated for 48 hours. On day 6, the cell culture media was entirely aspirated and replaced with fresh complete medium without cytokines. Duplicate wells were infected at an MOI of 450, for one hour in RPMI, with the following strains: YS1646Δ*asd*/ΔFLG containing a plasmid encoding wild-type (WT) human (hu) STING; YS1646Δ*asd*/ΔFLG containing a plasmid encoding the huSTING R284G variant; YS1646Δ*asd*/ΔFLG containing a plasmid encoding WT huIRF3; YS1646Δ*asd*/ΔFLG containing a plasmid encoding the huIRF3 S396D variant; or a strain containing a plasmid control. The cells were then washed 3 times with PBS, and resuspended in RPMI + 100 µg/mL gentamicin (Sigma). As a control, the STING agonist 3'5' RpRp c-di-AMP (InvivoGen), an analog of the clinical compound ADU-S100, was added to the cells at 10 µg/mL.

After 24 hours, the cells were lysed with 350 µL Buffer RLT with β-ME (Qiagen), and RNA extraction was performed using the Qiagen RNeasy Mini Kit with the following modification. A genomic DNA elimination step, using an RNase-Free DNase kit (Qiagen), was included to remove genomic DNA from the total RNA. Total RNA concentration was measured using a NanoDrop^{™} One^{C} UV-Vis Spectrophotometer (Thermo Scientific). The purity of each sample also was assessed from the A₂₆₀/A₂₃₀ absorption ratio. RNA was stored at -80 °C without freeze-thawing until reverse-transcription was performed. Synthesis of cDNA was performed from 0.4-1 µg of template RNA using a C1000 Touch Thermal Cycler (Bio-Rad) and SuperScript^{™} VILO^{™} Master Mix (Invitrogen) in a 30 µL reaction, according to the manufacturer's instructions.

qPCR was performed with a CFX96^{™} Real-Time System (Bio-Rad). SYBR^{®} primers for huCXCL10 (qHsaCED0046619), huIRF3 (qHsaCID0013122), huSTING (qHsaCID0010565), and huIFNβ1 (qHsaCED0046851) were purchased from Bio-Rad. The qPCR reaction (20 µL) was conducted per protocol, using the iTaq^{™} Universal SYBR^{®} Green Supermix (Bio-Rad). The standard thermocycling program on the BioRad CFX96^{™} Real-Time System consisted of a 95 °C denaturation for 30 seconds, followed by 40 cycles of 95 °C for 5 seconds and 60 °C for 30 seconds. Reactions with template free control were included for each set of primers on each plate. All samples were run in duplicate, and the mean C_{q} values were calculated. Quantification of the target mRNA was normalized using Gapdh reference mRNA (Bio-Rad, qMmuCED0027497). ΔC_{q} was calculated as the difference between the target and reference gene. ΔΔC_{q} was obtained by normalizing the ΔC_{q} values of the treatments to the ΔC_{q} values of the non-treatment control. Fold increase was calculated as 2^-ΔΔC_{q}. The values are shown in the table below, as the average of the duplicate wells.

As shown in the table below, compared to the infection of the plasmid control, strains of YS1646Δ*asd*/ΔFLG, containing plasmids encoding huSTING WT and huSTING R284G, induced high levels of STING expression, which were significantly higher compared to the plasmid control or the small molecule STING agonist. Similarly, the strains containing plasmids encoding WT huIRF3 and huIRF3-S396D induced high levels of IRF3 expression, which were significantly higher than the plasmid control, or the small molecule STING agonist. The bacterial strain containing a plasmid encoding the huSTING R284G variant induced much higher expression of IFNβ and CXCL10 as compared to the strain containing a plasmid encoding WT huSTING. This demonstrates the ability of the strain, containing a plasmid encoding a constitutive STING GOF variant, to convert a human primary, immunosuppressive M2 macrophage into an M1, type I IFN producing, cell. While the strains containing plasmids encoding WT huIRF3 and huIRF3-S396D both induced more, or similar levels of IFNβ, they induced less CXCL10 than the huSTING-R284G variant.

| **GOF Mutant** | **Fold Expression Over Untransfected Control** | | | |
|---|---|---|---|---|
| | **STING** | **IRF3** | **IFNβ** | **CXCL10** |
| Plasmid Control | 22.3 | 0 | ND | ND |
| huSTING WT | 24017.1 | ND | 3.4 | 3934.5 |
| huSTING R284G | 36542.7 | ND | 20 | 23484.5 |
| huIRF3 WT | 22.7 | 478.9 | 17.5 | 10766.2 |
| huIRF3-S396D | 30.8 | 346.4 | 26.3 | 15696.1 |
| 3'5' RpRp c-di-AMP | 244.8 | 1.11 | 1.77 | 594.1 |

| | | | | |
|---|---|---|---|---|
| ND = No Data | | | | |

These data demonstrate the expression of constitutive GOF type I IFN variants in human primary M2 macrophages, and converting these cells to M1-like, type I IFN producing, cells.

### Example 13

### Immunostimulatory Bacteria Containing Plasmids Encoding Constitutive Type I IFN Variants Demonstrate Potent Anti-tumor Immunity in a Murine Model of Colorectal Cancer Human GOF STING Mutants Show Anti-Tumor Activity in Mouse Models

To demonstrate that immunostimulatory bacterial strains, containing expression plasmids encoding constitutively active STING variants, induce anti-tumor efficacy, strain YS1646Δ*asd*/ΔFLG (knockout of both flagellin genes *fljB* and *fliC*) was electroporated with a plasmid containing an expression cassette for human STING with the allele R232 and the GOF mutation V155M (huSTING V155M), behind the human elongation factor-1 alpha (EF-1α) promoter, and was compared to strain YS1646 alone, and to a PBS vehicle control. The gene encoding huSTING V155M was generated using DNA synthesis and cloned into the pATI-1.75 vector. In order to evaluate whether a constitutive human STING variant could demonstrate anti-tumor activity in mice, 6-8 week-old female C57BL/6 mice (5 mice per group) were inoculated SC in the right flank with MC38 colorectal adenocarcinoma cells (5x10⁵ cells in 100 µL PBS). Mice bearing established flank tumors were IV injected on day 8 with 5x10⁵ CFUs of strain YS1646Δ*asd*/ΔFLG-huSTING V155M, with strain YS 1646, or with PBS control.

The results showed that the YS1646 parental strain was only mildly effective as an anti-tumor therapy and was not curative (35% TGI, *p* = NS (not significant), day 28), in line with previously published data. The more attenuated strain, containing a plasmid encoding constitutively active human STING, YS1646Δ*asd*/ΔFLG-huSTING V155M, however, elicited significant tumor control (60% TGI, *p* < 0.05, day 28) compared to PBS, and had a cure rate of 20%. Thus, an immunostimulatory bacterial strain that delivers a constitutively active STING variant potently inhibits tumor growth, and demonstrates curative effects in a model of colorectal adenocarcinoma.

### Murine Phosphomimetic IRF3 Shows Curative Effects In Vivo

The murine version of the phosphomimetic human IRF3 variant was designed, designated muIRF3-S388D, and evaluated in a murine model of colorectal adenocarcinoma. Strain YS1646Δ*asd*/ΔFLG was electroporated with a plasmid containing an expression cassette for murine IRF3 with the GOF mutation S388D (muIRF3-S388D), behind the human elongation factor-1 alpha (EF-1α) promoter, and was compared to PBS vehicle control. The gene encoding muIRF3-S388D was generated using DNA synthesis and cloned into the pATI-1.75 vector. 6-8 week-old female C57BL/6 mice (5 mice per group) were inoculated SC in the right flank with MC38 colorectal adenocarcinoma cells (5x10⁵ cells in 100 µL PBS). Mice bearing established flank tumors were IV injected on day 10 with 5 x10⁵ CFUs of strain YS1646Δ*asd*/ΔFLG-EF-1α-muIRF3-S388D, and compared to PBS vehicle control.

The therapy was very well tolerated, with an initial weight loss nadir of only 0.3%. Compared to PBS, the bacterial strain containing the plasmid encoding the muIRF3-S388D GOF mutant was highly effective and curative (81.8% TGI, 60% CR, day 42). These data demonstrate the potency and safety of delivering constitutive type I IFN inducing variants in a tumor-specific manner.

### Murine STING GOF Variants Show Potent and Curative Anti-Tumor Activity

A panel of murine orthologs of the human STING variants discovered in human patients, was designed. These orthologs differ by one codon from the human variants, and were cloned into the pATI-1.75 vector under the control of an EF-1α promoter, to yield the following set of mutants: muSTING N153S, muSTING V154M, muSTING R280Q, muSTING V146L, muSTING R283G, and muSTING C205Y, among others. The STING variants were evaluated in the MC38 model of murine adenocarcinoma for anti-tumor efficacy. For the studies, 6-8 week-old female C57BL/6 mice (5 mice per group) were inoculated SC in the right flank with MC38 colorectal adenocarcinoma cells (5x10⁵ cells in 100 µL PBS). Mice bearing established flank tumors were IV injected on day 10 with 5 x10⁵ CFUs of strain YS1646Δ*asd*/ΔFLG, containing a plasmid with EF-1α driving the expression of muSTING N153S, muSTING V154M, muSTING R280Q, muSTING V146L, or muSTING R283G, or a scrambled shRNA plasmid control, and compared to PBS vehicle control.

In this experiment, strain YS1646Δ*asd*/ΔFLG-EF-1α-shSCR (scrambled plasmid control) demonstrated anti-tumor efficacy as compared to PBS control (73% TGI, day 26), which was much more potent than the YS1646 parental strain has shown historically. This can be due to inherently immunostimulatory elements on the plasmid itself, such as CpGs and RNAi stimulatory elements. However, this therapy was the least well tolerated of the group, demonstrating a weight loss nadir of 9.9% that only resolved at the very end of the study. In contrast, the constitutively active murine STING mutants resulted in a lower weight loss that was transient and that resolved within days. The relative anti-tumor efficacy of these variants revealed differences in activity, with only two variants demonstrating curative effects and enhanced efficacy over the plasmid control, muSTING N153S and muSTING R283G.

| **Murine STING GOF Mutant** | **TGI vs. PBS, Day 26** | **Complete Response** | **Weight Loss Nadir and Day** |
|---|---|---|---|
| Plasmid Control | 73.0% | 0/5 | 9.9%, day 19 |
| muSTING N153S | 81.7% | 1/5 | 6.2%, day 12 |
| muSTING V154M | 69.4% | 0/5 | 4.3%, day 12 |
| muSTING R280Q | 68.7% | 0/5 | 5.4%, day 12 |
| muSTING V146L | 63.4% | 0/5 | 2.8%, day 12 |
| muSTING R283G | 81.2% | 1/5 | 6.9%, day 12 |

In a follow-up study, the murine STING C205Y variant was tested along with the R283G and N153S variants, to compare their anti-tumor efficacy. 6-8 week-old female C57BL/6 mice (5 mice per group) were inoculated SC in the right flank with MC38 colorectal adenocarcinoma cells (5x10⁵ cells in 100 µL PBS). Mice bearing established flank tumors were IV injected on day 9 with 5 x10⁵ CFUs of strain YS1646Δ*asd*/ΔFLG, containing a plasmid with EF-1α driving the expression of muSTING N153S, muSTING R283G, or muSTING C205Y, and compared to PBS vehicle control. As before, the STING variants were well tolerated, and only a transient dip in weight loss was observed that resolved quickly. This is likely due to on-target therapy, as it is also observed with the small molecule STING agonists. The efficacy of the two constitutively active murine STING variants, muSTING N153S and muSTING R283G, was nearly identical to the previous study, although the weight loss was much less, for reasons unclear. The muSTING C205Y variant also was highly effective, although not curative.

| **Murine STING GOF Mutant** | **TGI vs. PBS, Day 29** | **Complete Response** | **Weight Loss Nadir and Day** |
|---|---|---|---|
| muSTING C205Y | 79.4% | 0/5 | 2.6%, day 13 |
| muSTING N153S | 79.3% | 1/5 | 2.2%, day 13 |
| muSTING R283G | 85.1% | 1/5 | 1.8%, day 13 |

The STING-cured mice from these studies were re-challenged at day 40 post-initial tumor implantation on the opposite flank, SC, with MC38 colorectal adenocarcinoma cells (5x10⁵ cells in 100 µL PBS). Compared to naive mice (N=5), in which all tumors grew out, all of the STING-cured mice rejected the tumors, demonstrating the engagement of adaptive immunity.

These data validate the safety and potency of the murine versions of the human constitutively active STING variants in a murine model of colorectal carcinoma, and reveal a small subset of variants that have enhanced potency compared to the other STING variants. These highly active variants also elicit protective immunity, demonstrating the potency of tumor-specific production of type I interferon.

### Murine STING GOF Variants Demonstrate Significant Tumor Remodeling Following IV Dosing

It was next determined whether the bacterial strains containing plasmids encoding constitutive STING variants demonstrate differences in their ability to remodel the tumor microenvironment (TME) following IV dosing. To test this, 6-8 week-old female C57BL/6 mice (5 mice per group) were inoculated SC in the right flank with MC38 colorectal adenocarcinoma cells (5x10⁵ cells in 100 µL PBS). Mice bearing established flank tumors were IV injected on day 8 with 5 x10⁵ CFUs of strain YS1646Δ*asd*/ΔFLG, containing a plasmid with EF-1α driving the expression of muSTING N153S, muSTING V154M, muSTING R280Q, muSTING V146L, muSTING R283G, or plasmid control, and compared to PBS vehicle control.

At day 28 post tumor implantation, tumors were excised for analysis. Tumors were cut into 2-3 mm pieces into gentleMACS^{™} C tubes (Miltenyi Biotec) filled with 2.5 mL enzyme mix (RPMI-1640 + 10% FBS with 1 mg/mL Collagenase IV and 20 µg/mL DNase I). The tumor pieces were dissociated using OctoMACS^{™} (Miltenyi Biotec) specific dissociation program (mouse implanted tumors), and the whole cell preparation was incubated with agitation for 45 minutes at 37 °C. After 45 minutes of incubation, a second round of dissociation was performed using the OctoMACS^{™} (mouse implanted tumor) program, and the resulting single cell suspensions were filtered through a 70 µM nylon mesh into a 50 mL tube. The nylon mesh was washed once with 5 mL of RPMI-1640 + 10% FBS, and the cells were filtered a second time using a new 70 µM nylon mesh into a new 50 mL tube. The nylon mesh was washed with 5 mL of RPMI-1640 with 10% FBS, and the filtered cells were then centrifuged at 1000 RPM for 7 minutes. The resulting dissociated cells were resuspended in PBS and kept on ice before the staining process.

The percentage of live tumor-infiltrating leukocytes (TILs), including CD4⁺ Tregs, CD4⁺ Th1 cells, CD8⁺ T cells, neutrophils, monocytes, dendritic cells (DCs), M1 macrophages, and M2 macrophages, following the administration of strain YS1646Δ*asd*/ΔFLG, containing plasmids encoding the various GOF muSTING mutants, was determined by flow cytometry. For the flow-cytometry staining, 100 µL of the single cell suspensions were seeded in wells of a V-bottom 96-well plate. PBS containing a dead/live stain (Zombie Aqua^{™}, BioLegend) and Fc Blocking reagents (BD Biosciences) were added at 100 µL per well, and incubated on ice for 30 minutes in the dark. After 30 minutes, cells were washed twice with PBS + 2% FBS by centrifugation at 1300 RPM for 3 minutes. Cells were then resuspended in PBS + 2% FBS, containing fluorochrome-conjugated antibodies (CD4 FITC clone RM4-5; CD8a BV421 clone 53-6.7; F4/80 APC clone BM8; CD11b PE-Cy7 clone M1/70; CD45 BV570 clone 30-F11; CD3 PE clone 145-2C11; Ly6C BV785 clone HK1.4; I-A/I-E APC-Cy7 clone M5/114.15.2; Ly6G BV605 clone 1A8; and CD24 PercP-Cy5.5 clone M1/69; all from BioLegend), and incubated on ice for 30 minutes in the dark. After 30 minutes, cells were washed twice with PBS + 2% FBS by centrifugation at 1300 RPM for 3 minutes and resuspended in flow cytometry fixation buffer (Thermo Fisher Scientific). Flow cytometry data were acquired using the ACEA NovoCyte^{®} flow cytometer (ACEA Biosciences, Inc.) and analyzed using the FlowJo^{™} software (Tree Star, Inc.).

As shown in the tables below, the YS1646Δ*asd*/ΔFLG strain with the EF-1α plasmid control demonstrated predominantly high neutrophil infiltration, despite some CD8⁺ T-cell recruitment, likely due to immunostimulatory elements on the plasmid. In contrast, the different muSTING variants had unique tumor-infiltrating immune cell signatures, with some, such as muSTING V146L and muSTING R283G, resulting in fewer immunosuppressive neutrophils than the PBS control. The most favorable immune profiles were observed in the tumors from mice that were administered the muSTING R283G and muSTING N153S mutants, with high numbers of CD4⁺ Th1 cells and CD8⁺ T-cells, and low numbers of neutrophils, which indicates highly favorable conditions for generating an adaptive immune response. In addition, the muSTING R283G and muSTING N153S mutants had significantly higher p15e tumor-antigen-specific CD8⁺ T-cells in the tumor, as compared to PBS. These trends were also recapitulated in the total cell counts, as shown below. Thus, delivery of constitutively active STING variants to the tumor-resident myeloid cells leads to a complete remodeling of the immunosuppressive tumor microenvironment, towards an adaptive anti-tumor phenotype, and away from a bacterial phenotype, which is characterized by the promotion of innate immunity and the suppression of adaptive immunity.

**% of Live Tumor-Infiltratine Leukocytes (TILs)**

| **% Among TILs** | **GOF muSTING Mutants Encoded on Plasmids in IV-Administered Strain YS1646Δ*asd*/ΔFLG** | | | | | | |
|---|---|---|---|---|---|---|---|
| | **PBS** | **Plasmid Control** | **muSTING N153S** | **muSTING V154M** | **muSTING R280Q** | **muSTING V146L** | **muSTING R283G** |
| **CD4⁺ Tregs** | 2.9 ± 1.7 | 1.8 ± 0.5 | 3.3 ± 2.6 | 1.5 ± 0.5 | 2 ± 0.9 | 1.8 ± 0.5 | 1.5 ± 0.4 |
| **CD4⁺ Th1 cells** | 6.6 ± 3.2 | 13 ± 4.3 | 20.7 ± 10.4 | 13.2 ± 6.1 | 22.6 ± 7.4 | 19.5 ± 4.9 | 25.6 ± 7.8 |
| **CD8⁺ T cells** | 16.2 ± 10.2 | 24.5 ± 11.6 | 25.8 ± 9.2 | 16.5 ± 4.4 | 20.2 ± 7.2 | 20.8 ± 3.6 | 27.8 ± 8.7 |
| **Neutrophils** | 11.3 ± 14.2 | 30.6 ± 17.4 | 13.5 ± 12.4 | 21.4 ± 12.5 | 15.9 ± 11.6 | 9.1 ± 6.5 | 6.6 ± 5.9 |
| **Monocytes** | 16.6 ± 3.5 | 12.7 ± 2.7 | 13.7 ± 3.9 | 16.4 ± 5.4 | 15.8 ± 6.5 | 15.6 ± 2.2 | 13.5 ± 1.3 |
| **DCs** | 1.4 ± 0.4 | 0.5 ± 0.3 | 0.9 ± 0.7 | 0.4 ± 0.2 | 0.5 ± 0.3 | 0.5 ± 0.2 | 0.5 ± 0.3 |
| **M1 Macrophages** | 10.2 ± 6.2 | 3.1 ± 2.1 | 4.6 ± 2.1 | 9.4 ± 5 | 5.6 ± 4.2 | 8.6 ± 3.7 | 5.4 ± 2 |
| **M2 Macrophages** | 14.9 ± 10.6 | 4.6 ± 2.6 | 7.3 ± 3.1 | 11.6 ± 5.2 | 8.6 ± 6.3 | 12.8 ± 5 | 9.2 ± 4 |

### Total Cell Counts

| | **PBS** | **Plasmid Control** | **muSTING N153S** | **muSTING V154M** | **muSTING R280Q** | **muSTING V146L** | **muSTING R283G** |
|---|---|---|---|---|---|---|---|
| **CD4⁺ Tregs** | 437 ± 230 | 148 ± 102 | 530 ± 117 | 310 ± 114 | 520 ± 169 | 297 ± 207 | 438 ± 176 |
| **CD4⁺ Th1 cells** | 1108 ± 599 | 1059 ± 711 | 3765 ± 917 | 3349 ± 2869 | 5864 ± 1618 | 2961 ± 1800 | 7463 ± 3240 |
| **CD8⁺ T cells** | 2948 ± 3119 | 1571 ± 601 | 6152 ± 3820 | 3898 ± 2823 | 5446 ± 2454 | 3266 ± 1277 | 7566 ± 1782 |
| **Neutrophils** | 1531 ± 1604 | 2604 ± 1975 | 3699 ± 4400 | 4815 ± 3423 | 4301 ± 3502 | 1240 ± 1160 | 1698 ± 1485 |
| **Monocytes** | 2871 ± 1472 | 912 ± 369 | 3182 ± 1708 | 3350 ± 1183 | 4132 ± 1595 | 2524 ± 1420 | 3811 ± 996 |
| **DCs** | 233 ± 97 | 28 ± 18 | 161 ± 45 | 82 ± 31 | 130 ± 90 | 78 ± 48 | 135 ± 90 |
| **M1 Macrophages** | 2163 ± 2025 | 227 ± 213 | 881 ± 316 | 1797 ± 750 | 1421 ± 910 | 1325 ± 856 | 1524 ± 658 |
| **M2 Macrophages** | 3046 ± 2996 | 334 ± 275 | 1391 ± 373 | 2183 ± 608 | 2189 ± 1402 | 2043 ± 1237 | 2612 ±1330 |

### Example 14

### Expression of Multiple Immunomodulatory Proteins using Combinatorial Plasmid Expression Cassettes

### Multi-Modular Plasmid Expression Cassettes Demonstrate the Ability to Produce Multiple Immunomodulatory Proteins in Human Cells

Combinations of nucleic acids, encoding GOF Type I IFN inducing variants and cytokines, were cloned into the pATI-1.75 vector, using two separate ORFs, under the control of the CMV and EF-1α promoters (dual promoter system), or using T2A peptides within the ORFs and one promoter (single promoter system). The constructs also optionally included post-transcriptional regulatory elements (PREs), such as 3' WPRE or HPRE, and/or polyadenylation signal sequences, such as the SV40 or bovine growth hormone (bGH) polyadenylation signals. The constructs prepared included those encoding the cytokines muIL-2, muIL-2 with codon optimization (muIL-2 CO), muIL-21, muIL-12p70, muIL-15Rα-IL-15sc, muIL-18, and muIFN-α2, and combinations thereof; the muSTING variant with the mutation R283G; and/or the murine co-stimulatory molecule 4-1BBL with a deletion of the cytoplasmic domain (mu4-1BBLΔcyt). The sequences were confirmed by PCR.

To determine whether the combination plasmids, containing GOF Type I IFN inducing variants, can induce functional type I IFN in human cells, the plasmids were assessed using HEK293T STING Null Reporter cells (InvivoGen), which do not contain endogenous STING. These cells express secreted embryonic alkaline phosphatase (SEAP), placed under the control of the endogenous IFN-stimulated response element (ISRE) promoter, where the coding sequence of ISRE has been replaced by the SEAP ORF using knock-in technology. Type I interferon activity can be assessed by monitoring Type I IFN-stimulated SEAP production in the cell supernatants. In addition, supernatants were collected and evaluated for relative cytokine concentrations by ELISA.

To test the relative production of type I IFN and co-expressed cytokines, 2x10⁵ 293T-Dual^{™} Null cells (InvivoGen) were plated one day prior on 24-well plates coated with poly-L-lysine, to achieve 80% confluency. On the day of transfection, 500 ng of plasmids encoding a panel of GOF variants, cytokines, and co-stimulatory molecules, alone or in various combinations, were diluted in serum-free media and added to FuGENE^{®} transfection reagent (Promega), at the proper reagent:DNA ratios. Cell culture supernatants from each sample were collected after overnight incubation, and 20 µL of the cell culture supernatants was added to 180 µL QUANTI-Blue^{™} reagent (InvivoGen). Type I interferon activation was determined by measuring ISRE-induced SEAP activity on a SpectraMax^{®} M3 Spectrophotometer (Molecular Devices) at an absorbance of 650 nm. The muIL-2 constructs were evaluated for muIL-2 expression in a murine IL-2 ELISA (R&D Systems), according to the manufacturer's instructions. The muIL-12p70 constructs were evaluated in a murine IL-12 ELISA (R&D Systems), according to the manufacturer's recommendation. For the muIL-15Rα-IL-15sc constructs, the murine IL-15 ELISA (eBioscience) was used, per kit instructions. IFN-α2 was measured using the RAW-Lucia^{™} ISG reporter cell line (InvivoGen). Murine IL-18 and murine IL-21 were measured by ELISA (Invitrogen).

As shown in the table below, assays performed to detect the presence of secreted functional proteins demonstrated high expression of multiple combination payloads. These include cytokine combinations, as well as combinations with type I IFN inducing GOF variants, and/or co-stimulatory payloads. These data validate the ability of the platform provided herein to express multiple immunomodulatory proteins from a single expression cassette.

| **Construct** | **Protein Expression** | | | | | | | |
|---|---|---|---|---|---|---|---|---|
| | **STING (Abs 650 nm)** | **IL-2 (Abs 650 nm)** | **IL-12 (Abs 650 nm)** | **IL-15 (pg/mL)** | **IL-15 (Abs 650 nm)** | **IFN-α2 (Lumines cence)** | **IL-21 (OD at 450 nm)** | **IL-18 (pg/mL)** |
| CMV-mu-IL-2 | | 1.995 | | | | | | |
| CMV-muIL-2 CO-WPRE | | 2.0073 | | | | | | |
| EF-1α-muIL-21 | | | | | | | 1.138 ± 0.1853 | |
| CMV-muIL-21 | | | | | | | 0.4758 ± 0.0461 | |
| EF-1α-muIL-12p70 | | | 0.9052 | | | | | |
| CMV-muIL-15Rα-IL-15sc-WPRE | | | | 30140.8 ±535.5 | 0.8224 | | | |
| EF-1α-muIFN-α2 | | | | | | 14570 | | |
| CMV-muIFN-α2-WPRE | | | | | | 18658 | | |
| CMV-muIL-2 CO_T2A_m uIFN-α2-WPRE | | 2.0998 | | | | 13066 | | |
| CMV-muIL-15Rα-sc_T2A_ muIFN-α2-WPRE | | | | 29895.9 ±630.9 | 1.0125 | 8571 | | I |
| CMV-muIL-2 CO + EF-1α-muIFN-α2 | | 2.0589 | | | | 8297 | | |
| CMV-muIL-21 + EF-1α-muIFN-α2 | | | | | | 8682 | 0.3797 ± 0.0830 | |
| CMV-muIL-2 CO + EF-1α-muIL-21 | | 2.0085 | | | | | 0.7565 ± 0.1271 | |
| CMV-muIL-15Rα-IL-15sc + EF-1α-muIL-21 | | | | 12605 ± 278.6 | 0.2362 | | 0.2963 ± 0.0155 | |
| CMV-muIL-12p70 + EF-1α-muIL-21 | | | 1.0893 | | | | 0.1479 ± 0.001 | |
| CMV-muIL-2 CO- | | 1.207 ± 0.121 | | | | | | |
| HPRE-HPRE-bGHpolyA | | | | | | | | |
| EF-1α-muSTING-R283G | 0.4874 ± 0.0045 | | | | | | | |
| CMV-muSTING-R283G-WPRE | 0.4866 ± 0.0511 | | | | | | | |
| CMV-muIL-2 CO + EF-1α-muIL-12p70 | | 0.9971 ± 0.0104 | 0.3303 ± 0.0157 | | | | | |
| CMV-muIL-15Rα-IL-15sc + EF-1α-muIL-12p70 | | | 0.3629 ± 0.0242 | | 0.1464 ± 0.0034 | | | |
| CMV-muIL-I8 + EF-1α-muSTING-R283G-WPRE | 0.7096 ± 0.0169 | | | | | | | 5991.4 ± 1642.0 |
| CMV-muIL-12p70_T2A _muIFN-α2 + EF-1α-IL-18-WPRE | | | 0.6538 ± 0.0205 | | | 577.3 ± 122.5 | | 12685.0 ± 4402.8 |
| CMV-mulL-12p70_T2A _muSTING-R283G + EF-1α-muIL-18-WPRE | 0.454 ± 0.123 | | 0.4581 ± 0.0158 | | | | | 3805.8 ± 1369.5 |
| CMV-muIL-15Rα-IL-15sc_ T2A_muSTI NG-R283G + EF-1α-muIL-18-WPRE | 0.5867 ± 0.0333 | | | | 0.2326 ± 0.0049 | | | 3626 ± 1248 |
| CMV-muIL-15Rα-IL-15sc_ T2A_muIF N-α2 + EF-1α-muIL-18-WPRE | | | | | 0.3512 ± 0.0172 | 1802 ± 216.5 | | 6872 ± 1671 |
| CMV-4-1BBL(Δcyt) + EF-1α-muSTING- | 0.2525 ± 0.0144 | | | | | | | |
| R283G-WPRE | | | | | | | | |

### Constitutive Type I IFN Inducing Variants have Unique Cytokine and Chemokine Profiles

Supernatants harvested from HEK293T transfection experiments with a panel of human type I IFN inducing GOF variants, tested as described above, were evaluated for downstream signaling differences using a human anti-viral CBA panel (BD Biosciences), according to the manufacturer's protocol. Each transfection was performed in duplicate, and cytokine levels were measured. The average of two measurements was calculated. Fold increase in average cytokine secretion was calculated compared to untransfected wells, in which the average cytokine secretion was set as 1.00.

As shown in the table below, low levels of human IL-12p70 were produced in the cells. However, several human type I IFN inducing GOF variants induced the production of type I **IFN-α2** and/or IFN-β, including the phosphomimetic IRF3 variant (huIRF3-S396D), as well as several constitutively active STING variants. A number of these GOF variants produced high levels of secreted CXCL10, demonstrating the ability of these expressed variants to recruit T-cells into the tumor microenvironment. The highest levels of CXCL10 expression were observed following the expression of the huSTING variant with the mutation R284G.

These data validate the use of multiplexed plasmid expression constructs, containing type I IFN inducing GOF variants, for induction of functional downstream cytokines and chemokines, such as CXCL10/IP10. These variants all have unique signatures, and STING GOF variants induced the highest levels of CXCL10 secretion, particularly huSTING-R284G (corresponding to muSTING-R283G).

| **Constructs** | **Secreted Immunostimulatory Cytokines and Chemokines (Fold Relative to Untransfected)** | | | |
|---|---|---|---|---|
| | **IL-12p70** | **IFN-α2** | **IFN-β** | **CXCL10** |
| Untransfected control | 1.00 | 1.00 | 1.00 | 1.00 |
| EF-1α-mCherry Control | 1.50 | 1.59 | 1.50 | 2.05 |
| EF-1α-huSTING Negative Control | 1.32 | 2.10 | 1.43 | 2.16 |
| EF-1α-huSTING WT | 1.66 | 1.69 | 1.46 | 2.77 |
| EF-1α-huSTING-V147L | 1.78 | 2.35 | 21.35 | 332.14 |
| EF-1α-huSTING-N154S | 1.52 | 2.23 | 23.37 | 398.99 |
| EF-1α-huSTING-V155M | 2.00 | 2.09 | 6.02 | 121.38 |
| EF-1α-huSTING-C206Y | 1.39 | 2.02 | 16.51 | 218.38 |
| EF-1α-huSTING-R281Q | 1.35 | 1.35 | 8.17 | 301.18 |
| EF-1α-huSTING-R284G | 1.46 | 2.14 | 13.41 | 429.40 |
| EF-1α-huIRF3-WT | 1.50 | 1.49 | 1.50 | 1.11 |
| EF-1α-huIRF3-S396D | 1.93 | 9.37 | 38.06 | 65.89 |
| CMV-muIFN-α2-WPRE | 1.03 | 3.63 | 1.37 | 146.49 |
| CMV-muIL-2 CO_T2A_muIFN-α2-WPRE | 1.03 | 1.63 | 1.37 | 86.02 |
| CMV-muIL-15Rα-11-15sc_T2A_muIFN-α2-WPRE | 1.03 | 0.80 | 1.37 | 40.85 |

### Immune Cell Co-Culture Assays Identify Optimal Combinations of Immunomodulatory Targets

In order to determine the optimal combinations of cytokines to elicit T-cell recruitment and activation, a panel was tested in a macrophage and T-cell co-culture assay. Golden Ticket (STING deficient) murine primary bone marrow-derived macrophages (BMMs) were generated as described above (see, Example 6), using a 24-well plate. Each well was transfected with the appropriate DNA constructs using FuGENE^{®} transfection reagents (Promega). The constructs included those encoding muIL-2 CO, muIL-12p70, muSTING-R283G, muIL-2 CO + muIL-12p70, muIL-15Rα-IL-15sc + muIL-12p70, and muIL-12p70 + muSTING-R283G + muIL-18.

24 hours post-transfection, 100 µL of cell culture supernatants were harvested from the wells for a flow cytometry-based cytokine bead array (CBA). In parallel, two spleens from C57BL/6 mice were dissected, and splenic CD4⁺ and CD8⁺ T-cells were isolated following instructions from a mouse T-cell isolation kit (StemCell Technologies). 200,000 isolated T-cells per well were then added to the transfected cells, with or without CD3ε antibody (clone 145-2C11, BioLegend), at a final concentration of 0.5 µg/ml per well. At 24 and 48 hours post-addition of T-cells to the transfected cells, 100 µL of the co-culture supernatants were harvested from the wells for a flow-cytometry based cytokine bead array. Supernatants from transfected bone marrow macrophages (BMMs) and bone marrow macrophage/T-cell co-cultures were analyzed for their cytokine content, using murine anti-viral and murine Th1 specific cytokine bead arrays, respectively.

As shown in the table below, only the plasmids encoding muSTING-R283G elicited CXCL10 production from the macrophages, while plasmids encoding muIL-12p70, alone or in combination with other proteins, elicited the highest levels of IFNy from the co-cultured T-cells, which were greater than the background amount resulting from CD3ε stimulation. The combination of muIL-12p70 + muIL-18 + muSTING-R283G was able to induce both CXCL10 production from macrophages, and IFNy production from co-cultured T-cells.

These data demonstrate the feasibility of expressing multiple immunomodulatory payloads from a single plasmid, as well as the synergistic activities of these combinations.

| **Constructs** | **BMM CXCL10 Production (pg/mL)** | **T-Cell IFNγ Production (pg/mL)** |
|---|---|---|
| Untransfected | 3.82 | 3170.28 |
| CMV-muIL-2 CO-WPRE | 5.92 | 7633.19 |
| EF-1α-muIL-12p70 | 12.24 | 11495.65 |
| CMV-muSTING-R283G | 662.03 | 1060.82 |
| CMV-muIL-2 CO + EF-1α-muIL-12p70 | 4.59 | 8511.19 |
| CMV-muIL-15Rα-IL-15sc + EF-1α-muIL-12p70 | 5.82 | 11694.78 |
| CMV-muIL-12p70_T2A_muSTING-R283G + EF-1α-muIL-18 | 324.92 | 9331.51 |

### Combinatorial Immunotherapy Demonstrates Enhanced Anti-Tumor Activity in a Murine Model of Colorectal Adenocarcinoma

In order to determine whether tumor-specific delivery of a combination of cytokines enhances anti-tumor efficacy, a construct encoding the combination of muIL-12p70, muIL-18, and muSTING-R283G (CMV-muIL-12p70_T2A_muIL-18 + EF-1α-muSTING-R283G-WPRE), was assessed in the MC38 murine model of colorectal adenocarcinoma. For this study, 6-8 week-old female C57BL/6 mice (5 mice per group) were inoculated SC in the right flank with MC38 colorectal adenocarcinoma cells (5x10⁵ cells in 100 µL PBS). Mice bearing established flank tumors were IV injected on day 10 with 1x10⁷ CFUs of strain YS1646Δ*asd*/ΔFLG/Δ*pagP*/Δ*ansB,* containing a plasmid encoding the combination of muIL-12p70, muIL-18, and muSTING-R283G (CMV-mul-12p70_T2A_muIL-18 + EF-1α-muSTING-R283G-WPRE), or with PBS vehicle control.

The combination therapy was very well tolerated, with only an initial weight loss of 3.6% that had fully recovered 3 days later. This is in marked contrast to the toxicities observed with systemic administration of these cytokines (IL-12p70 and IL-18). Compared to PBS control, the combination therapy was highly effective and curative (92.3% TGI, 60% cure rate, day 30). These data demonstrate the potency and safety of delivering combinations of cytokines in a tumor-specific manner, using the immunostimulatory bacterial strains described herein.

### Example 15

### Protein Engineering Screening to Identify Improved Gain-of-Function Mutations in STING, RIG-I, MDA5, IRF3, IRF7, and other Interferon Pathway Genes

Gain-of-function (GOF) amino acid mutants that are constitutively active and that promote interferonopathies are identified from humans. Many GOF mutations occur due to single base pair nucleotide changes that alter the amino acid codon at that particular position in the gene. For example, in STING, the V147L mutation occurs due to a mutation at c.439G→C; N154S occurs due to a mutation at c.461A→G; and V155M occurs due to a mutation at c.463G→A. The purpose of the screening was to identify constitutively active mutants that lead to high levels of type I interferon expression. Designed mutations, at sites known to promote interferonopathies when mutated in human patients, allow for a greater number of amino acid substitutions to be tested. In this example, site-directed mutagenesis with designed amino acids is performed at the positions of known mutations (see, Table below, listing mutations in genes that promote interferonopathies), to identify mutations with enhanced activity, that lead to high level type I interferon expression.

| **Exemplary Normal Function Proteins in which the Mutations are Introduced** | **Gain-of-Function Mutations** |
|---|---|
| | S102P |
| | V147L |
| | V147M |
| | N154S |
| **STING/TMEM173 (SEQ ID NOs: 305-309)** | V155M |
| | G166E |
| | C206Y |
| | G207E |
| | S102P/F279L |
| | F279L |
| | R281Q |
| | R284G |
| | R284S |
| | R284M |
| | R284K |
| | R284T |
| | R197A |
| | D205A |
| | R310A |
| | R293A |
| | T294A |
| | E296A |
| | R197A/D205A |
| | S272A/Q273A |
| | R310A/E316A |
| | E316A |
| | E316N |
| | E316Q |
| | S272A |
| | R375A |
| | R293A/T294A/E296A |
| | D231A |
| | R232A |
| | K236A |
| | Q273A |
| | S358A/E360A/S366A |
| | D231A/R232A/K236A/R238A |
| | S358A |
| | E360A |
| | S366A |
| | R238A |
| | S324A/S326A |
| **MDA5/IFIH1 (SEQ ID NO: 310)** | T331I |
| | T331R |
| | A489T |
| | R822Q |
| | G821S |
| | A946T |
| | R337G |
| | D393V |
| | G495R |
| | R720Q |
| | R779H |
| | R779C |
| | L372F |
| | A452T |
| **RIG-I (SEQ ID NO: 311)** | E373A |
| | C268F |

Amino acid residues R197, D205, R310, R293, T294, E296, S272, Q273, E316, D231, R232, K236, S358, E360, S366, and R238, with reference to the sequence of human STING, as set forth in SEQ ID NOs:305-309, correspond to amino acid residues R196, D204, R309, R292, T293, E295, S271, Q272, E315, D230, R231, K235, S357, E359, S365 and R237, respectively, with reference to the sequence of murine STING, as set forth in SEQ ID NO:369.

PCR primers are generated with designed substitutions flanked on the 5' and 3' ends with homologous cDNA sequences from the gene. The QuikChange^{®} Site-Directed Mutagenesis kit (Agilent), or other comparable commercially available kit, is used to generate a PCR product incorporating the designed mutation. PCR amplified plasmids are treated with DpnI, then electroporated into competent *E. coli* cells. Individual clones are isolated, plasmid mini-preps are performed, and the sequence identity of the desired mutation is confirmed. Larger scale plasmid preparations are then performed (using a Qiagen Kit), and the DNA is transfected into HEK293T STING Reporter cells (InvivoGen), which do not contain endogenous STING. These cells express Lucia^{™} luciferase, a secreted luciferase, placed under the control of the endogenous IFN-β promoter, the coding sequence of IFN-β has been replaced by the Lucia^{™} luciferase ORF using knock-in technology. Constitutively activate mutants then are identified and ranked by measurement of IFN-β promoter induced expression of luciferase activity.

### Example 16

### Various Species of Immunostimulatory Bacteria with Inactivating Deletions Corresponding to the Deletions in Salmonella typhimurium

The Examples above describe exemplary modifications to the genome of *Salmonella typhimurium* to increase targeting to and accumulation of *Salmonella typhimurium* in immune-resident myeloid cells and in the tumor microenvironment, to deliver therapeutic products/payloads to tumors, and to reduce toxicity of the bacteria by eliminating their ability to infect other cell types. These genetic modifications similarly can be introduced into other bacterial strains and species, such as by deletion of the corresponding genes in other species, as described below.

### Escherichia coli

In-frame chromosomal deletions of the *lpxM, purM, asd, fliC, fliE, pagP, ansB* and *csgD* genes are made sequentially in *E. coli* strains using a technique based on the recombineering methods described by Datsenko and Wanner (Proc. Natl. Acad. Sci. U.S.A. 97:6640-6645 (2000)). The genes in *E. coli* are as follows:
*1) lpxM,* encoding myristoyl-acyl carrier protein-dependent acyltransferase [*E. coli* strain K-12, substrain MG1655; NCBI Gene ID: 945143];
2) *purM,* encoding phosphoribosylformylglycinamide cyclo-ligase *[E. coli* strain K-12, substrain MG1655; NCBI Gene ID: 946975];
*3) asd,* encoding aspartate-semialdehyde dehydrogenase *[E. coli* strain K-12, substrain MG1655; NCBI Gene ID: 947939];
4) *fliC*, encoding flagellar filament structural protein *[E. coli* strain K-12, substrain MG1655; NCBI Gene ID: 949101];
*5) fliE,* encoding flagellar basal-body protein FliE *[E. coli* strain K-12, substrain MG1655; NCBI Gene ID: 946446];
*6) pagP,* encoding Lipid IVA palmitoyltransferase *[E. coli* strain K-12, substrain MG1655; NCBI Gene ID: 946360];
*7) ansB,* encoding L-asparaginase 2 *[E. coli* strain K-12, substrain MG1655; NCBI Gene ID: 947454];
*8) csgD,* encoding DNA-binding transcriptional dual regulator CsgD *[E. coli* strain K-12, substrain MG1655; NCBI Gene ID: 949119]; and
*9) rpsM,* encoding 30S ribosomal subunit protein S13 (promoter) *[E. coli* strain K-12, substrain MG1655; NCBI Gene ID: 947791].

Briefly, a specific chromosomal sequence is replaced with a selectable antibiotic resistance marker flanked by homology arms, and is subsequently removed by a cre/loxP system. The 5' and 3' flanking sequences of each target gene are identified and cloned into a plasmid vector on opposing sides of an antibiotic resistance gene. The gene deletion cassette, containing the antibiotic resistance gene and flanking 5' and 3' homology arms is PCR amplified, gel purified, and introduced into the *E. coli* strain by electroporation. Electroporated cells are recovered, and transformants are selected for on antibiotic plates. The antibiotic marker is then cured using a cre/loxP recombination system, in which antibiotic resistant clones are transformed with a cre-expressing temperature-dependent plasmid. Colonies are selected at 30 °C and subsequently eliminated by serial passage at 42 °C, and then screened for loss of antibiotic resistance. Antibiotic sensitive clones are confirmed for gene deletion by colony PCR and sequence analysis.

### Increasing Resistance or Rendering E. coli Resistant to Human Complement

Expression of the *Salmonella typhimurium rck* (resistance to complement killing) gene, the *Yersinia enterocolitica* homolog *ail* (attachment invasion locus), or the *Salmonella typhimurium pgtE* (outer membrane serine protease) gene, in the *E. coli* Δ*lpxM*/Δ*purM*/Δ*asd*/Δ*fliC*/Δ*fliE*/Δ*pagP*/Δ*ansB*/Δ*csgD* strain is achieved by encoding the *rck, ail*, or *pgtE* gene sequence downstream from a constitutive promoter, such as *E. coli* or *S. typhimurium* rpsM, on a plasmid *(asd* complementation system compatible), or by insertion on the bacterial chromosome (at any of the *lpxM*, *purM, asd*, *fliC, fliE*, *pagP, ansB*, or *csgD* loci).

### Salmonella typhi

In-frame chromosomal deletions of the *msbB, purM, asd*, *fliC*, *figB, pagP, ansB*, and *csgD* genes are made sequentially in *S. typhi* strains using the technique described above for E. coli-based strains.

Expression of the *Salmonella typhimurium rck* (resistance to complement killing) gene, the *Yersinia enterocolitica* homolog *ail*, or the *Salmonella typhimurium pgtE* gene, in the *S. typhi* Δ*msbB*/Δ*purM*/Δ*asd*/Δ*fliC*/Δ*flgB*/Δ*pagP*/Δ*ansB*/Δ*csgD* strain is achieved by encoding the *rck, ail*, or *pgtE* gene sequence downstream of a constitutive promoter, such *as S. typhimurium* or *S. typhi* rpsM, on a plasmid *(asd* complementation system compatible), or by insertion on the bacterial chromosome (at any of the *msbB, purM, asd*, *fliC, flgB*, *pagP, ansB*, or *csgD* loci). The genes in *S. typhi* are as follows:
*1) msbB* (STY2097), encoding lipid A acyltransferase *[Salmonella enterica* subspecies *enterica* serovar Typhi, strain CT18; NCBI Gene ID: 1248440];
*2) purM* (STY2740), encoding phosphoribosylformylglycinamidine cyclo-ligase *[Salmonella enterica* subspecies *enterica* serovar Typhi, strain CT18; NCBI Gene ID: 1249054];
*3) asd* (STY4271), encoding aspartate-semialdehyde dehydrogenase *[Salmonella enterica* subspecies *enterica* serovar Typhi, strain CT18; NCBI Gene ID: 1250488];
*4) fliC* (STY2167), encoding flagellin *[Salmonella enterica* subspecies *enterica* serovar Typhi, strain CT18; NCBI Gene ID: 1248507];
5) *flgB* (STY1213), encoding flagellar basal-body rod protein FlgB *[Salmonella enterica* subspecies *enterica* serovar Typhi, strain CT18; NCBI Gene ID: 1247617];
6) *pagP* (STY0677), encoding antimicrobial peptide resistance and lipid A acylation protein *[Salmonella enterica* subspecies *enterica* serovar Typhi, strain CT18; NCBI Gene ID: 1247137];
*7) ansB* (STY3259), encoding L-asparaginase *[Salmonella enterica* subspecies *enterica* serovar Typhi, strain CT18; NCBI Gene ID: 1249541];
*8) csgD* (STY1179), encoding regulatory protein CsgD *[Salmonella enterica* subspecies *enterica* serovar Typhi, strain CT18; NCBI Gene ID: 1247585]; and
*9) rpsM* (STY4380), encoding 30S ribosomal subunit protein S13 (promoter) *[Salmonella enterica* subspecies *enterica* serovar Typhi, strain CT18; NCBI Gene ID: 1250594].

### Listeria monocytogenes

In-frame chromosomal deletions of the *purA, purQ, purS, asd*, *flaA*, *fliC*, *flgB,* and *ansB* genes in *Listeria monocytogenes* is achieved by an allelic exchange technique using a temperature-sensitive shuttle vector, such as pKSV7, that confers antibiotic resistance and allows replication at low temperatures (30 °C), but is unable to replicate at higher temperature (43 °C). The 5' and 3' flanking sequences of each target gene are identified and cloned in tandem into the pKSV7 vector, which is transformed into recipient *Listeria monocytogenes* and selected for by plating on agar plates with antibiotic. Chromosomal integration of the plasmid is induced by serial passage of antibiotic-resistant transformants at 42 °C under selection. Subsequent sequential subculturing of the strain at 30 °C results in subpopulations of cells in which the plasmids are excised through a second crossover event, producing reversions to the original wild-type gene, or incorporation of the 5' and 3' flanking sequence homology arms to generate the targeted deletion mutant. Antibiotic sensitive clones are screened at this step by colony PCR and sequence analysis.

To increase complement resistance, expression of the *Salmonella typhimurium rck* (resistance to complement killing) gene, the *Yersinia enterocolitica* homolog *ail*, or the *Salmonella typhimurium pgtE* gene in the *Listeria monocytogenes* Δ*purA*/Δ*purQ*/Δ*purS*/Δ*asd*/Δ*flaA*/Δ*fliC*/Δ*flgB*/Δ*ansB* strain is achieved by encoding the *rck, ail*, or *pgtE* gene sequence downstream of a constitutive promoter, such as P_{hyper} or Pₕₑₗₚₑᵣ, on a plasmid *(asd* complementation system compatible), or by insertion on the bacterial chromosome (at any of the *purA*, *purQ, purS, asd*, *flaA*, *fliC, flgB,* or *ansB* loci). The genes in *L. monocytogenes* are as follows:
1) *purA* (lmo0055), encoding adenylosuccinate synthetase [*Listeria monocytogenes* strain EGD-e; NCBI Gene ID: 986069];
2) *purQ* (lmo1769), encoding phosphoribosylformylglycinamidine synthase II *[Listeria monocytogenes* strain EGD-e; NCBI Gene ID: 985972];
*3) purS* (lmo1771), encoding phosphoribosylformylglycinamidine synthase subunit PurS *[Listeria monocytogenes* strain EGD-e; NCBI Gene ID: 985970];
*4) asd* (lmo1437), encoding aspartate-semialdehyde dehydrogenase [*Listeria monocytogenes* strain EGD-e; NCBI Gene ID: 986492];
5) *flaA* (lmo0690), encoding flagellin *[Listeria monocytogenes* strain EGD-e; NCBI Gene ID: 987167];
*6) fliE* (lmo0712), encoding flagellar hook-basal body protein FliE [ *Listeria monocytogenes* strain EGD-e; NCBI Gene ID: 985062];
7) *flgB* (lmo0710), encoding flagellar basal-body rod protein FlgB [*Listeria monocytogenes* strain EGD-e; NCBI Gene ID 985059]; and
*8) ansB* (lmo1663), encoding asparagine synthetase *[Listeria monocytogenes* strain EGD-e; NCBI Gene ID: 985663].

The genes *msbB* and *pagP* are absent in *Listeria monocytogenes,* which is a Gram-positive bacterium. CsgD also is absent in *Listeria monocytogenes.* Instead, *Listeria* express *lcp,* encoding the *Listeria* cellulose binding protein that is involved in biofilm formation, which also can be deleted.

### Bifidobacterium longum

In-frame chromosomal deletions of BL1122 (*purM*), BL0492 (*asd*) and BL1142 (encoding an L-asparaginase precursor) in *Bifidobacterium longum* is achieved by an allelic exchange technique using an incompatible plasmid vector system, in which a conditional-replication vector, such as pBS423-Δ*repA*, which lacks the plasmid replication gene, *repA*, is initially integrated into the genome, providing antibiotic resistance. A second plasmid, such as pTBR101-CM, encoding the *repA* gene, is subsequently transformed and facilitates a second crossover event that selects for excision of the initial integrant. The 5' and 3' flanking sequences of each target gene are identified and cloned in tandem into the conditional replication vector, lacking *repA*, and transformed into the *Bifidobacterium longum* ΔBL1122/ΔBL0492/ΔBL1142 strain. Crossover recombination events can occur at homology arm sequences, and successful plasmid integrants are selected for and isolated on antibiotic plates. Integrants are then transformed with an incompatible plasmid encoding a functional copy of *repA*, which facilitates a second crossover event and excision of the *repA* deficient plasmid, which is subsequently lost due to plasmid incompatibility. Genomic deletions are confirmed by colony PCR and sequence analysis, and the remaining plasmid is cured by removing selection and subsequent Rif treatment.

Expression of the *Salmonella typhimurium rck* (resistance to complement killing) gene, the *Yersinia enterocolitica* homolog *ail*, or the *Salmonella typhimurium pgtE* gene in the *Bifidobacterium longum* ΔBL1122/ΔBL0492/ΔBL1142 strain is achieved by encoding the *rck, ail*, or *pgtE* gene sequence downstream of a strong constitutive promoter, such as P_{gap}, on a plasmid *(asd* complementation system compatible), or by insertion on the bacterial chromosome (at any of the BL1122, BL0492, or BL1142 loci). The *Bifidobacterium longum* genes are as follows:
*1) purM* (BL1122), encoding phosphoribosylformylglycinamidine cyclo-ligase *[Bifidobacterium longum* strain NCC2705; NCBI Gene ID: 1022669];
*2) asd* (BL0492), encoding aspartate-semialdehyde dehydrogenase *[Bifidobacterium longum* strain NCC2705; NCBI Gene ID: 1023089];
3) BL1142, encoding an L-asparaginase precursor (Ntn_Asparaginase_2_like; L-Asparaginase type 2-like enzymes of the NTN-hydrolase superfamily) [*Bifidobacterium longum* strain NCC2705; NCBI Gene ID: 1023120]; and
4) BL1363 gap (promoter) *[Bifidobacterium longum* strain NCC2705; NCBI Gene ID: 1022828].

*Bifidobacterium longum* are non-motile and lack flagellin, and are Gram-positive and lack *msbB* and *pagP.* The *ansB* gene is present, but encodes aspartate ammonia-lyase, which catalyzes the formation of fumarate from aspartate (*aspA*/*ansB*) [BL0338, *Bifidobacterium longum* strain NCC2705; NCBI Gene ID: 1023259].

### Clostridium novyi

In-frame chromosomal deletions of NT01CX_ RS09765, NT01CX_RS07625, and NT01CX_RS04325 (*asd*); the flagellin genes NT01CX_RS04995, NT01CX_RS04990, NT01CX_RS05070, and NT01CX_RS05075; and the flagellar basal body rod protein genes NT01CX _RS05080 (*flgB*), NT01CX_RS05085 (*flgC*), and NT01CX_RS05215 (*flgG*) in *Clostridium* is achieved by an allelic exchange technique requiring a counter-selection method including toxin-antitoxin systems, which requires an inducible promoter and toxic gene, such as the *E. coli* mRNA interferase *mazF.* The 5' and 3' flanking sequences of each target gene are identified and cloned in a configuration on opposing sides of a frt-flanked antibiotic resistance cassette in an allelic exchange counter-selection-containing vector, and transformed into *Clostridium novyi.* The *mazF* gene is encoded under the control of an inducible lac promoter on the allelic exchange vector, and permits selection of a double crossover event by growth on lactose-supplemented agar plates. Genomic deletions are confirmed by colony PCR and sequence analysis. Flp-frt recombination can then be used to cure the antibiotic resistance cassette from the chromosome.

Expression of the *Salmonella typhimurium rck* (resistance to complement killing) gene, the *Yersinia enterocolitica* homolog *ail*, or the *Salmonella typhimurium pgtE* gene in the *Clostridium novyi* ΔNT01CX_RS09765/Δ NT01CX_RS07625/ΔNT01CX_RS04325/ΔNT01CX_RS04995/ΔNT01CX_RS04990 /ΔNT01CX_RS05070/ΔNT01CX_RS05075/ΔNT01CX_RS05080/ΔNT01CX_RS050 85/ΔNT01CX_RS05215 strain is achieved by encoding the *rck, ail*, or *pgtE* gene sequence downstream from a strong constitutive promoter, such as Pₜₕₗ, P_{ptb}, or other variants, on a plasmid (*asd* complementation system compatible), or by insertion on the bacterial chromosome (at any of the NT01CX_RS09765, NT01CX_RS07625, NT01CX_RS04325, NT01CX_RS04995, NT01CX_RS04990, NT01CX_RS05070, NT01CX_RS05075, NT01CX_RS05080, NT01CX_RS05085, or NT01CX_RS05215 loci). The *Clostridium novyi* genes are as follows:
1) NT01CX_RS09765, encoding AIR synthase *[Clostridium novyi* strain NT; NCBI Gene ID: 4541583];
2) NT01CX_RS07625, encoding phosphoribosylformylglycinamidine cyclo-ligase *[Clostridium novyi* strain NT; NCBI Gene ID: 4540669];
3) NT01CX_RS04325 (*asd*), encoding aspartate-semialdehyde dehydrogenase *[Clostridium novyi* strain NT; NCBI Gene ID: 4541762];
4) NT01CX_RS04995, encoding flagellin *[Clostridium novyi* strain NT; NCBI Gene ID: 4541703];
5) NT01CX _RS04990, encoding flagellin *[Clostridium novyi* strain NT; NCBI Gene ID: 4539984];
6) NT01CX_RS05070, encoding flagellin [ *Clostridium novyi* NT; NCBI Gene ID: 4539886];
7) NT01CX_RS05075, encoding flagellin *[Clostridium novyi* NT; NCBI Gene ID: 4539699];
8) NT01CX_RS05080 (*flgB*), encoding flagellar basal body rod protein FlgB *[Clostridium novyi* strain NT; NCBI Gene ID: 4540637];
9) NT01CX_RS05085 (*flgC*), encoding flagellar basal body rod protein FlgC *[Clostridium novyi* strain NT; NCBI Gene ID: 4540143]; and
10) NT01CX_RS05215 (*flgG*), encoding flagellar basal body rod protein FlgG *[Clostridium novyi* strain NT; NCBI Gene ID: 4540245].

*Clostridium novyi* is Gram-positive, and lacks *msbB* and *pagP.*

### Example 17

### Immunostimulatory Bacteria Modified to Express Vertebrate STING Variants that Induce Stronger Type I IFN Signaling and/or Weaker NF-κB Signaling than Human STING

STING signaling activates two signaling pathways. The first is the TANK binding kinase 1 (TBK1)/IRF3 axis, resulting in the induction of type I IFNs, and the activation of dendritic cells (DCs) and cross-presentation of tumor antigens to activate CD8⁺ T-cell-mediated anti-tumor immunity. The second is the nuclear factor kappa-light-chain-enhancer of activated B-cell (NF-κB) signaling axis, resulting in a pro-inflammatory response, but not in the activation of the DCs and CD8⁺ T-cells that are required for anti-tumor immunity. Bacterially-based cancer immunotherapies are limited in their ability to induce type I IFN to recruit and activate the CD8⁺ T-cells that are necessary to promote tumor antigen cross-presentation and durable anti-tumor immunity. Hence, provided are immunostimulatory bacteria herein that induce and/or increase type I IFN signaling, and that have decreased NF-κB signaling, thereby increasing the induction of CD8⁺ T-cell mediated anti-tumor immunity, and enhancing the therapeutic efficacy of the bacteria. The immunostimulatory bacteria described above encode modified STING proteins that are gain-of-function mutants of STING that can increase induction of type I IFN compared to wild-type STING, or render the expression of type I IFN constitutive. In this example (and also described in the detailed description), the STING protein is modified to reduce or eliminate NF-κB signaling activity, and to retain the ability to induce type I IFN, and/or is modified for increased or constitutive type I IFN expression. This results in immunostimulatory bacteria that induce anti-tumor immunity, and do not induce (or induce less) NF-xB signaling that normally results from infection by bacterial pathogens.

STING proteins from different species exhibit different levels of type I IFN and NF-κB signaling activities. For example, STING signaling in human and mouse cells results in a strong type I IFN response, and a weak pro-inflammatory NF-κB response. STING signaling in ray-finned fish, such as salmon and zebrafish, in comparison, elicits robust activation of a primarily NF-κB-driven response, that is more than 100-fold higher compared with the IRF3-driven *(i.e.,* type I IFN inducing) response. In other species, such as Tasmanian devil, STING signaling results in a type I IFN response, but essentially no NF-κB response. The immunostimulatory bacteria provided herein encode STING from non-human species, such as Tasmanian devil STING, in order to exploit the ability of STING to induce a type I IFN response, but without the concomitant induction of an NF-κB response. As described herein, these non-human STING proteins also are modified by mutation to increase the type I IFN response, or to render it constitutive. The identified mutations that have this effect in human STING are introduced into the non-human STING proteins. The corresponding residues are identified by alignment.

Also provided are chimeras in which the C-terminal tail (CTT) of STING is replaced in one species, such as human, with the CTT from a second (*e.g.,* non-human) species STING protein that exhibits little or no NF-κB signaling activity. The CTT is an unstructured stretch of approximately 40 amino acids that contains sequence motifs required for STING phosphorylation and recruitment of IRF3. It can shape downstream immunity by altering the balance between type I IFN and NF-κB signaling. This is controlled through independent modules in the CTT, including IRF3, TBK1 and TRAF6 binding modules. For example, human STING residue S366 (see, *e.g.,* SEQ ID NOs:305-309) is a primary TBK1 phosphorylation site that is part of an LxIS motif in the CTT, which is required for IRF3 binding, while a second PxPLR motif, including residue L374, is required for TBK1 binding. The LxIS and PxPLR motifs are highly conserved in all vertebrate STING alleles. Replacing the CTT of human STING with that of, for example, Tasmanian devil STING, produces a STING variant that induces a type I IFN response, but not an NF-κB response.

In this Example, the immunostimulatory bacteria are engineered to express a STING variant with increased type I IFN signaling, and/or reduced NF-κB signaling, compared to wild-type (WT) human STING (see, *e.g.,* SEQ ID NOs:305-309). The STING variants can be from a non-human vertebrate, such as a mammalian, bird, reptilian, amphibian, or fish species. Species from which the non-human STING proteins are derived include, but are not limited to, Tasmanian devil (*Sarcophilus harrisii;* SEQ ID NO:349), marmoset (*Callithrix jacchus;* SEQ ID NO:359), cattle (*Bos taurus;* SEQ ID NO:360), cat (*Felis catus;* SEQ ID NO:356), ostrich (*Struthio camelus australis;* SEQ ID NO:361), crested ibis (*Nipponia nippon;* SEQ ID NO:362), coelacanth (*Latimeria chalumnae;* SEQ ID NOs:363-364), boar (*Sus scrofa*; SEQ ID NO:365), bat (*Rousettus aegyptiacus*; SEQ ID NO:366), manatee (*Trichechus manatus latirostris*; SEQ ID NO:367), ghost shark (*Callorhinchus milii;* SEQ ID NO:368), and mouse (*Mus musculus;* SEQ ID NO:369). These vertebrate STING proteins readily activate immune signaling in human cells, indicating that the molecular mechanism of STING signaling is shared in vertebrates (see, *e.g.,* de Oliveira Mann et al. (2019) Cell Reports 27:1165-1175). STING proteins from these species induce less NF-κB signal activation and/or more type I IFN signal activation, than human STING (see, *e.g.,* de Oliveira Mann *et al.* (2019), Fig. 1A). Wild-type or modified STING proteins from different non-human species can be expressed by the immunostimulatory bacteria herein, as can chimeras of human and non-human STING proteins.

The various non-human STING proteins are modified, such that the non-human STING has lower NF-κB activation, and, optionally, higher type I interferon activation, than human STING. These non-human STING proteins are modified to include a mutation or mutations so that they have increased type I IFN activity, or act constitutively, in the absence of cytosolic nucleic acid ligands (e.g., CDNs). The mutations typically are amino acid mutations, such as gain-of-function mutations, that are associated with interferonopathies in humans. The corresponding mutations are introduced into the non-human species STING proteins, where corresponding amino acid residues are identified by alignment. For example, mutations include, but are not limited to, S102P, V147L, V147M, N154S, V155M, G166E, C206Y, G207E, S102P/F279L, F279L, R281Q, R284G, R284S, R284M, R284K, R284T, R197A, D205A, R310A, R293A, T294A, E296A, R197A/D205A, S272A/Q273A, R310A/E316A, E316A, E316N, E316Q, S272A, R293A/T294A/E296A, D231A, R232A, K236A, Q273A, S358A/E360A/S366A, D231A/R232A/K236A/R238A, S358A, E360A, S366A, R238A, R375A, and S324A/S326A, with reference to the sequence of human STING, as set forth in SEQ ID NOs:305-309. Corresponding mutations in STING from other species are listed in the tables below. The resulting variants of the non-human STING proteins include one or more of these mutations, and optionally, a CTT replacement, and optionally, a deletion in the TRAF6 binding site.

The STING variants include one or more replacements of the amino acid serine (S) or threonine (T) at a phosphorylation site, with aspartic acid (D), which is phosphomimetic, resulting in increased or constitutive activity. Other mutations include deletion or replacement of a phosphorylation site or sites, such as 324-326 SLS → ALA in STING, and other replacements to eliminate a phosphorylation site to reduce NF-κB signaling in STING. Additionally, chimeras of human STING with STING from other species are provided, in which the C-terminal tail (CTT) of human STING is replaced with the CTT of STING from another species that has lower NF-κB signaling activity, and/or higher type I IFN signaling activity. The variant STING proteins can include a deletion in the TRAF6 binding site of the CTT, to reduce NF-κB signaling.

| **Human STING (SEQ ID NOs: 305-309)** | **Tasmanian devil STING (SEQ ID NO:349)** | **Marmoset STING (SEQ ID NO:359)** | **Cattle STING (SEQ ID NO:360)** | **Cat STING (SEQ ID NO:356)** | **Ostrich STING (SEQ ID NO:361)** | **Crested ibis STING (SEQ ID NO:362)** | **Coelacanth STING (SEQ ID NO:363)** |
|---|---|---|---|---|---|---|---|
| S102P | S102P | S102P | S102P | S102P | C107P | V106P | A102P |
| V147L | V147L | V145L | I147L | V146L | M152L | M151L | I147L |
| V147M | V147M | V145M | I147M | V146M | - | - | I147M |
| N154S | N154S | N152S | N154S | N153S | N159S | N158S | G154S |
| V155M | V155M | V153M | V155M | V154M | V160M | V159M | V155M |
| G166E | G166E | G164E | G166E | G165E | G171E | G170E | G166E |
| C206Y | C206Y | C204Y | C206Y | C205Y | C211Y | C210Y | C206Y |
| G207E | S207E | G205E | G207E | G206E | N212E | D211E | S207E |
| S102P/ F279L | S102P/ F279L | S102P/ F277L | S102P/ F279L | S102P/ F278L | C107P/ F283L | V106P/ F283L | A102P/ F279L |
| F279L | F279L | F277L | F279L | F278L | F283L | F283L | F279L |
| R281Q | R281Q | R279Q | R281Q | R280Q | R285Q | R285Q | K281Q |
| R284G | R284G | R282G | R284G | R283G | R288G | R288G | R284G |
| R284S | R284S | R282S | R284S | R283S | R288S | R288S | R284S |
| R284M | R284M | R282M | R284M | R283M | R288M | R288M | R284M |
| R284K | R284K | R282K | R284K | R283K | R288K | R288K | R284K |
| R284T | R284T | R282T | R284T | R283T | R288T | R288T | R284T |
| R197A | R197A | R195A | R197A | R196A | K202A | K201A | R197A |
| D205A | D205A | D203A | D205A | D204A | S210A | S209A | S205A |
| R310A | R310A | R308A | R310A | R309A | R314A | R314A | R310A |
| R293A | R293A | R291A | R293A | R292A | R297A | R297A | R293A |
| T294A | T294A | T292A | T294A | 1293A | T298A | T298A | T294A |
| E296A | E296A | E294A | E296A | E295A | E300A | E300A | K296A |
| R197A/ D205A | R197A/ D205A | R195A/ D203A | R197A/ D205A | R196A/ D204A | K202A/ S210A | K201A/ S209A | R197A/ S205A |
| S272A/ Q273A | S272A/ Q273A | S270A/ Q271A | S272A/ Q273A | S271A/ Q272A | S276A/ Q277A | S276A/ Q277A | S272A/ K273A |
| R310A/ E316A | R310A/ E316A | R308A/ E314A | R310A/ E316A | R309A/ E315A | R314A/ E320A | R314A/ E320A | R310A/ E318A |
| E316A | E316A | E314A | E316A | E315A | E320A | E320A | E318A |
| E316N | E316N | E314N | E316N | E315N | E320N | E320N | E318N |
| E316Q | E316Q | E314Q | E316Q | E315Q | E320Q | E320Q | E318Q |
| S272A | S272A | S270A | S272A | S271A | S276A | S276A | S272A |
| R375A | R377A | R373A | R374A | R373A | R371A | R379A | K376A |
| R293A/ T294A/ E296A | R293A/ T294A/ E296A | R291A/ T292A/ E294A | R293A/ T294A/ E296A | R292A/ 1293A/ E295A | R297A/ T298A/ E300A | R297A/ T298A/ E300A | R293A/ T294A/ K296A |
| D231A | D231A | D229A | D231A | D230A | T236A | T235A | N231A |
| R232A | R232A | R230A | R232A | R231A | R237A | R236A | R232A |
| K236A | K236A | K234A | K236A | K235A | K241A | K240A | K236A |
| Q273A | Q273A | Q271A | Q273A | Q272A | Q277A | Q277A | K273A |
| S358A/ E360A/ S366A | S360A/ E362A/ S368A | S356A/ E358A/ S364A | S357A/ E359A/ S365A | S356A/ E358A/ S364A | S354A/ D356A/ S362A | S362A/ E364A/ S370A | S359A/ E361A/ S367A |
| D231A/ | D231A/ | D229A/ | D231A/ | D230A/ | T236A/ | T235A/ | N231A/ |
| R232A/ K236A/ R238A | R232A/ K236A/ R238A | R230A/ K234A/ R236A | R232A/ K236A/ R238A | **R231A/** K235A/ R237A | R237A/ K241A/ R243A | R236A/ K240A/ R242A | R232A/ K236A/ R238A |
| S358A | S360A | S356A | S357A | S356A | S354A | S362A | S359A |
| E360A | E362A | E358A | E359A | E358A | D356A | E364A | E361A |
| S366A | S368A | S364A | S365A | S364A | S362A | S370A | S367A |
| R238A | R238A | R236A | R238A | R237A | R243A | R242A | R238A |
| S324A/ S326A | S326A/ S328A | L322A/ S324A | S324A/ S326A | S323A/ S325A | F328A/ S330A | S328A/ S330A | S327A |

| **Human STING (SEQ ID NOs: 305-309)** | **Boar STING (SEQ ID NO:365)** | **Bat STING (SEQ ID NO:366)** | **Manatee STING (SEQ ID NO:367)** | **Ghost Shark STING (SEQ ID NO:368)** | **Mouse STING (SEQ ID NO:369)** |
|---|---|---|---|---|---|
| S102P | S102P | S103P | S105P | S98P | S102P |
| V147L | I147L | V148L | I150L | I148L | V146L |
| V147M | I147M | V148M | I150M | I148M | V146M |
| N154S | N154S | N155S | N157S | N155S | N153S |
| V155M | V155M | V156M | V158M | V156S | V154M |
| G166E | G166E | G167E | G169E | G167E | G165E |
| C206Y | C206Y | C207Y | C209Y | C206Y | C205Y |
| G207E | G207E | G208E | G210E | K207E | G206E |
| S102P/F279L | S102P/F279L | S103P/F280L | S105P/F282L | S98P/F280L | S102P/F278L |
| F279L | F279L | F280L | F282L | F280L | F278L |
| R281Q | R281Q | - | R284Q | K282Q | R280Q |
| R284G | R284G | R285G | R287G | R285G | R283G |
| R284S | R284S | R285S | R287S | R285S | R283S |
| R284M | R284M | R285M | R287M | R285M | R283M |
| R284K | R284K | R285K | R287K | R285K | R283K |
| R284T | R284T | R285T | R287T | R285T | R283T |
| R197A | R197A | R198A | R200A | K197A | R196A |
| D205A | D205A | D206A | D208A | S205A | D204A |
| R310A | R310A | R311A | R313A | R311A | R309A |
| R293A | R293A | R294A | R296A | R294A | R292A |
| T294A | T294A | T295A | T297A | T295A | T293A |
| E296A | E296A | - | E299A | K297A | E295A |
| R197A/D205A | R197A/D205A | R198A/D206A | R200A/D208A | K197A/S205A | R196A/D204A |
| S272A/Q273A | S272A/Q273A | S273A/Q274A | S275A/Q276A | T273A/N274A | S271A/Q272A |
| R310A/E316A | R310A/E316A | R311A/E317A | R313A/E319A | R311A/D317A | R309A/E315A |
| E316A | E316A | E317A | E319A | D317A | E315A |
| E316N | E316N | E317N | E319N | D317N | E315N |
| E316Q | E316Q | E317Q | E319Q | D317Q | E315Q |
| S272A | S272A | S273A | S275A | T273A | S271A |
| R375A | S374A | R376A | R383A | R374A | R374A |
| R293A/ T294A/ E296A | R293A/ T294A/ E296A | R294A/T295A | R296A/ T297A/ E299A | R294A/ T295A/ K297A | R292A/ T293A/ E295A |
| D231A | D231A | D232A | D234A | D231A | D230A |
| R232A | R232A | R233A | C235A | R232A | R231A |
| K236A | K236A | K237A | K239A | K236A | K235A |
| Q273A | Q273A | Q274A | Q276A | N274A | Q272A |
| S358A/ E360A/ S366A | S357A/ E359A/ S365A | H359A/ E361A/ S367A | S366A/ E368A/ S374A | S359A/ E361A/ S367A | S357A/ E359A/ S365A |
| D231A/ | D231A/ | D232A/ | D234A/ | D231A/ | D230A/ |
| R232A/ K236A/ R238A | R232A/ K236A/ R238A | R233A/ K237A/ R239A | C235A/ K239A/ R241A | R232A/ K236A/ R238A | R231A/ K235A/ R237A |
| S358A | S357A | H359A | S366A | S359A | S357A |
| E360A | E359A | E361A | E368A | E361A | E359A |
| S366A | S365A | S367A | S374A | S367A | S365A |
| R238A | R238A | R239A | R241A | R238A | R237A |
| S324A/S326A | S324A/S326A | S325A/S327A | S327A/S329A | G325A/S330A | S323A/S325A |

For example, modified STING variants include Tasmanian devil STING with the mutations C206Y (SEQ ID NO:350), or R284G (SEQ ID NO:351); a variant in which the CTT of human STING is replaced with the CTT of Tasmanian devil STING (SEQ ID NO:352); human STING with the mutation C206Y (SEQ ID NO:353), or R284G (SEQ ID NO:354), and where the CTT is replaced with the CTT of Tasmanian devil STING; wild-type human STING with a deletion in the TRAF6 binding domain (corresponding to residues 377-379 (DFS)) (SEQ ID NO:355); cat STING with the mutations C205Y (SEQ ID NO:357), or R283G (SEQ ID NO:358); and other such modified STING variants.

To determine the corresponding amino acid residues for the STING mutations, the wild-type STING protein sequences from various non-human species each were aligned with the wild-type human STING protein sequence (of the allelic variants of SEQ ID NO:305 (R232 allele) or SEQ ID NO:306 (H232 allele)). The alignments were performed using the Kalign sequence alignment tool, available from ebi.ac.uk/Tools/msa/kalign/, or the EMBOSS needle sequence alignment tool, available from ebi.ac.uk/Tools/psa/emboss_needle/. Figures 1-13 depict exemplary sequence alignments for human STING compared to STING proteins from Tasmanian devil, marmoset, cattle, cat, ostrich, crested ibis, coelacanth, zebrafish, boar, bat, manatee, ghost shark, and mouse species, respectively.

### STING GOF Hybrid Variants Demonstrate Significantly Enhanced Type I Interferon to NF-κB Activity Ratios in Dendritic Cells

In order to determine the optimal STING GOF mutant that would elicit the highest levels of the CD8⁺ T-cell chemokine CXCL10 in mice, a panel of STING mutants were tested in murine primary bone marrow-derived dendritic cells (BMDCs). These included the Tasmanian devil STING with the constitutive human GOF mutations C206Y (tazSTING C206Y; SEQ ID NO:350), or R284G (tazSTING R284G; SEQ ID NO:351); murine STING with the constitutive GOF mutants C205Y (muSTING C205Y; SEQ ID NO:399), or R283G (muSTING R283G; SEQ ID NO:400); cat STING with the constitutive GOF mutants C205Y (catSTING C205Y; SEQ ID NO:357), or R283G (catSTING R283G; SEQ ID NO:358); as well as variants in which the CTT of human STING (SEQ ID NO:370) was replaced with the CTT of Tasmanian devil STING (SEQ ID NO:371), and containing either wild-type human STING (huSTING tazCTT; see, *e.g.,* SEQ ID NO:352), or the human STING GOF mutations C206Y (huSTING C206Y tazCTT; see, *e.g.,* SEQ ID NO:353), or R284G (huSTING R284G tazCTT; see, *e.g.,* SEQ ID NO:354). Also included were human STING variants with the constitutive, GOF mutations C206Y (huSTING C206Y), or R284G (huSTING R284G).

To test these, murine bone marrow was isolated and flushed into 1.5 mL Eppendorf tubes, and spun at 1200 RPM for 5 minutes, to collect the bone marrow cells. Cells were washed once in RPMI-1640 + 10% FBS, then seeded in 96-well TC-treated plates in RPMI-1640 + 10% FBS with 20 ng/ml GM-CSF. Every 2 days, 50% of the medium was replaced with fresh complete media. After six days, non-adherent cells were pipetted off the wells and re-seeded at 1e5 cells per well in RPMI-1640 + 10% FBS in a 96-well plate for transfection. Cells were transfected using Viromer^{®} RED, according to the manufacturer's instructions. Briefly, 200 ng of plasmid DNA from a panel of STING GOF mutants, as well as untransfected control, were diluted in the provided buffer, and mixed with 0.08 µL of Viromer^{®} RED and incubated at room temperature for 15 minutes to allow the Viromer^{®} complexes to form. The DNA/Viromer^{®} RED complexes were then slowly added to each well of the 96-well plate (in duplicates), and the plate was incubated at 37 °C in a CO₂ incubator. Supernatants were harvested at 48 hours, and assayed for murine CXCL10 (IP-10) using a flow cytometry-based cytokine bead array (CBA), according to the manufacturer's protocol.

As shown in the table below, the construct that induced the highest expression of murine CXCL10 was human STING containing the GOF mutation R284G, and containing a replacement of the CTT of human STING with the CTT of Tasmanian devil STING (huSTING R284G tazCTT). The next highest expression of CXCL10 was induced by the human STING variant containing the GOF mutation C206Y (huSTING C206Y), and the Tasmanian devil STING construct containing the human STING GOF mutation R284G (tazSTING R284G). The human STING GOF mutants (huSTING C206Y and huSTING R284G) were more potent than the corresponding murine STING GOF mutants (muSTING C205Y and muSTING R283G, respectively), which were even less potent than the cat STING mutants containing the same GOF mutations (catSTING C205Y and catSTING R283G, respectively), in primary murine dendritic cells.

| **Construct** | **muCXCL10 (pg/mL)** |
|---|---|
| Untransfected | 11.48 ± 3.889 |
| huSTING C206Y | 861.0 ± 58.48 |
| huSTING R284G | 769.7 ± 95.16 |
| muSTING C205Y | 194 ± 27.15 |
| muSTING R283G | 230.1 ± 1.018 |
| huSTING C206Y tazCTT | 366.6 ± 42.61 |
| huSTING R284G tazCTT | 1326 ± 137.9 |
| tazSTING C206Y | 808.8 ± 95.78 |
| tazSTING R284G | 831.3 ± 30.15 |
| catSTING C205Y | 480.7 ± 24.94 |
| catSTING R283G | 376.2 ± 6.682 |

These data demonstrate that STING proteins obtained from other species, such as Tasmanian devil, can be combined with constitutive GOF human STING mutations, to elicit potent T-cell recruiting chemokines.

### STING GOF Hybrid Variants Demonstrate Significantly Enhanced Type I Interferon to NF-κB Activity Ratios in Human Monocytes

In order to demonstrate that the ratio of STING-induced type I interferon to NF-κB signaling can be altered using STING GOF hybrid variants from other species, a panel was tested in a human monocyte cell line. The panel included wild-type human STING (huSTING), and huSTING mutants with the constitutive human GOF mutations C206Y, or R284G; wild-type Tasmanian devil STING (tazSTING), and tazSTING mutants with the constitutive GOF mutations C206Y, or R284G; wild-type cat STING, and catSTING mutants with the constitutive GOF mutations C205, or R283G; murine STING mutants with the constitutive GOF mutations C205Y, or R283G; and the variants in which the CTT of human STING was replaced with the CTT of Tasmanian devil STING, and containing either wild-type human STING, or the human GOF STING mutations C206Y, or R284G. Also included were a wild-type human STING with a deletion in the TRAF6 binding domain (corresponding to residues 377-379, DFS, see, *e.g.,* SEQ ID NO:355), and wild-type zebrafish STING.

For this experiment, the THP1-Dual^{™} KO STING cells were utilized, which have been altered to lack endogenous STING, and to also express Lucia^{™} luciferase, a secreted luciferase, placed under the control of the endogenous IFN-β promoter. Constitutively active STING GOF mutants then were identified, and ranked by measurement of IFN-β promoter induced expression of luciferase activity. These cells also express secreted embryonic alkaline phosphatase (SEAP), placed under the control of the endogenous NF-κB promoter, where the coding sequence of NF-κB has been replaced by the SEAP ORF using knock-in technology. NF-κB activity induced by STING GOF mutants can be assessed by monitoring SEAP production in the cell supernatants.

For this experiment, THP1-Dual^{™} KO STING cells were transfected using Viromer^{®} RED, according to the manufacturer's instructions. Briefly, 200 ng of plasmid DNA from a panel of STING GOF mutants, as well as untransfected control, were diluted in the provided buffer, and mixed with 0.08 µL of Viromer^{®} RED and incubated at room temperature for 15 minutes to allow the Viromer^{®} complexes to form. The DNA/Viromer^{®} RED complexes were then slowly added to each well of the 96-well plate (in duplicates), and the plate was incubated at 37 °C in a CO₂ incubator. In addition, the wild-type STING variants were treated with or without the STING agonist 3'5' RpRp c-di-AMP (CDN, InvivoGen), an analog of the clinical compound ADU-S100, which was added to the cells after 24 hours of incubation at 10 µg/mL. Supernatants were harvested at 48 hours, and assayed for NF-κB-SEAP and IFN-Lucia reporter signals, according to the manufacturer's protocol. Briefly, 10 µL of the cell culture supernatants was added to 50 µL QUANTI-Blue^{™} reagent (InvivoGen) (which is used for measuring SEAP). NF-κB activation was determined by measuring NF-κB-induced SEAP activity on a SpectraMax^{®} M3 Spectrophotometer (Molecular Devices), at an absorbance (Abs) of 650 nm. For measuring type I interferon activity from IFN-Lucia, 10 µL of the cell culture supernatants was added to 50 µL QUANTI-Luc^{™}, containing the coelenterazine substrate for the luciferase reaction, which produces a light signal that is quantified using a SpectraMax^{®} M3 luminometer, and expressed as relative light units (RLUs).

As shown in the table below, the highest type I IFN responses were observed from the variant in which the CTT of human STING was replaced with the CTT of Tasmanian devil STING, and that contained the human STING GOF mutation R284G (huSTING R284G tazCTT), as well as from the wild-type zebrafish STING with the CDN STING agonist (zfSTING WT + CDN). However, unlike the wild-type zebrafish STING, which had very high NF-κB signaling, the huSTING R284G tazCTT variant had high type I IFN signaling with much lower NF-κB signaling activity. The best ratio of higher type I IFN to lower NF-κB signaling was found with the Tasmanian devil STING variant containing the human STING GOF mutation R284G (tazSTING R284G).

| **STING Variant** | **ISRE-Lucia (RLUs)** | **± SD** | **NF-κB-SEAP (Abs)** | **± SD** |
|---|---|---|---|---|
| Untransfected | 47.96 | 33.91 | 0.065 | 0.007 |
| huSTING WT + CDN | 170.8 | 38.15 | 0.100 | 0.014 |
| huSTING WT delTRAF6 + CDN | 164.8 | 8.48 | 0.120 | 0.014 |
| huSTING WT tazCTT + CDN | 31.47 | 10.59 | 0.060 | 0.000 |
| tazSTING WT + CDN | 143.9 | 4.24 | 0.090 | 0.014 |
| zfSTING WT + CDN | 310.2 | 23.31 | 0.690 | 0.028 |
| CMV catSTING WT WPRE + CDN | 202.3 | 6.36 | 0.125 | 0.007 |
| huSTING C206Y | 175.3 | 36.03 | 0.105 | 0.007 |
| huSTING R284G | 143.9 | 29.67 | 0.100 | 0.000 |
| huSTING C206Y tazCTT | 137.9 | 21.19 | 0.095 | 0.007 |
| huSTING R284G tazCTT | 301.2 | 61.46 | 0.250 | 0.127 |
| tazSTING C206Y | 199.3 | 2.12 | 0.120 | 0.000 |
| tazSTING R284G | 217.3 | 19.08 | 0.105 | 0.007 |
| muSTING C205Y | 43.46 | 2.12 | 0.070 | 0.000 |
| muSTING R283G | 32.97 | 4.24 | 0.070 | 0.000 |
| catSTING C205Y | 202.3 | 23.31 | 0.135 | 0.007 |
| catSTING R283G | 157.4 | 10.60 | 0.120 | 0.000 |

| | | | | |
|---|---|---|---|---|
| SD = Standard deviation | | | | |

These data further demonstrate using non-human STING proteins, such as the STING protein from Tasmanian devil, and combining them with human constitutive gain-of-function STING mutations, in order to enhance the beneficial type I interferon activity, while minimizing the immunosuppressive NF-κB activity, in human monocytes.

### Example 18

### S. typhimurium Lipoprotein Knockout by Deletion of the lppA and IppB Genes

The live attenuated *S. typhimurium* YS1646 strain, containing the Δ*asd*/ΔFLG/Δ*pagP*/Δ*ansB*/Δ*csgD* gene deletions, was engineered to delete the *lppA* (SEQ ID NO:387) and *lppB* (SEQ ID NO:388) genes, in order to remove membrane surface lipoproteins. This reduces pro-inflammatory TLR2 activation, which reduces immunosuppressive cytokines and improves anti-tumor adaptive immunity. As shown below, this also enhances plasmid delivery and encoded protein expression in the tumor.

### Strain Engineering and Characterization

### Deletion of lppA Gene

*IppA* was deleted from the chromosome of the YS1646Δ*asd*/ΔFLG/Δ*pagP*/Δ*ansB*/Δ*csgD* strain using modifications of the method of Datsenko and Wanner (Proc. Natl. Acad. Sci. U.S.A. 97:6640-6645 (2000)), as described in detail above. Synthetic *lppA* gene homology arm sequences that contained 231 and 200 bases of the left hand and right hand sequence, respectively, flanking the *lppA* gene, were synthesized and cloned into a plasmid called pSL0148 (SEQ ID NO:231). The sequence for the *lppA* gene is shown in SEQ ID NO:387; appropriate primers for PCR amplification were designed using the gene sequence. A kanamycin gene cassette flanked by cre/loxP sites then was cloned into plasmid pSL0148, and the *lppA* gene knockout cassette was then PCR amplified with primers lppA-1 and lppA-2, gel purified, and then introduced into the YS1646Δ*asd*/ΔFLG/Δ*pagP*/Δ*ansB*/Δ*csgD* strain carrying the temperature sensitive lambda red recombination plasmid pKD46, by electroporation. Electroporated cells were recovered in SOC + DAP medium, and plated onto LB agar plates supplemented with kanamycin (20 µg/mL) and diaminopimelic acid (DAP, 50 µg/mL). Colonies were selected and screened for insertion of the knockout fragment by PCR using primers lppA-3 and lppA-4. pKD46 then was cured by culturing the selected kanamycin resistant strain at 42 °C and screening for loss of ampicillin resistance. The kanamycin resistance marker then was cured by electroporation of a temperature-sensitive plasmid expressing the Cre recombinase (pJW168), and Amp^{R} colonies were selected at 30 °C; pJW168 was subsequently eliminated by growing cultures at 42 °C. Selected *lppA* knockout clones were then tested for loss of the kanamycin marker by PCR, using primers flanking the sites of disruption (lppA-3 and lppA-4), and evaluation of the electrophoretic mobility was performed on agarose gels. This mutant derivative of strain YS1646 was designated YS1646Δ*asd*/ΔFLG/Δ*pagP*/Δ*ansB*/Δ*csgD*/Δ*lppA*

### Deletion of lppB Gene

*lppB* then was deleted in the YS1646Δ*asd*/ΔFLG/Δ*pagP*/Δ*ansB*/Δ*csgD*/*lppA* strain using modifications of the methods described above. Synthetic *lppB* gene homology arm sequences that contained 224 and 231 bases of the left hand and right hand sequence, respectively, flanking the *lppB* gene, were synthesized and cloned into a plasmid called pSL0148 (SEQ ID NO:231). The sequence for the *lppB* gene is shown in SEQ ID NO:388; appropriate primers for PCR amplification were designed using the gene sequence. A kanamycin gene cassette flanked by cre/loxP sites then was cloned into plasmid pSL0148, and the *lppB* gene knockout cassette was PCR amplified with primers lppB-5 and lppB-6, gel purified, and introduced into strain YS1646Δ*asd*/ΔFLG/Δ*pagP*/Δ*ansB*/Δ*csgD*/*lppA* carrying the temperature-sensitive lambda red recombination plasmid pKD46 by electroporation. The kanamycin resistance gene then was cured by Cre-mediated recombination as described above, and the temperature-sensitive plasmids were cured by growth at non-permissive temperature. The *lppA* and *lppB* gene knockout sequences were amplified by PCR, using primers designated lppA-3 and lppA-4, and lppB-7 and lppB-8, respectively, and verified by DNA sequencing. This mutant derivative of strain YS1646 was designated YS1646Δ*asd*/ΔFLG/Δ*pagP*/Δ*ansB*/Δ*csgD*/Δ*lppAB*, or nicknamed YS1646Δ*lppAB.*

### In vitro Characterization of Engineered S. typhimurium Lipoprotein Knockout Strain

The YS1646Δ*asd*/ΔFLG/Δ*pagP*/Δ*ansB*/Δ*csgD*/Δ*lppAB* strain, harboring the deletions of *lppA* and *lppB,* was evaluated for growth by overnight cultures in LB. Growth was measured using a SpectraMax^{®} M3 Spectrophotometer (Molecular Devices) at 37 °C, reading the OD₆₀₀ every 15 minutes. The results demonstrated that strain YS1646Δ*asd*/ΔFLG/Δ*pagP*/Δ*ansB*/Δ*csgD*/Δ*lppAB* was able to replicate in LB at a growth rate comparable to the parental YS1646Δ*asd*/ΔFLG/Δ*pagP*/Δ*ansB*/Δ*csgD* strain. These data demonstrate that the elimination of lipoprotein does not decrease the fitness of *S. typhimurium in vitro.*

### Lipoprotein Deletion Enhances Plasmid Delivery to the Tumor After Systemic Administration

As TLR2 is expressed on vascular endothelial cells, and activation of TLR2 enhances vascular permeability, the effect of Δ*lppAB*, and the subsequent reduction in TLR2 agonism, on tumor colonization was assessed. The effect on payload expression also was assessed. As shown below, while colonization of tumors was somewhat reduced, plasmid delivery and encoded gene expression was significantly increased, following systemic administration.

To demonstrate the impact of the lipoprotein knockout strains in a murine model of triple-negative breast cancer, 6-8 week-old female BALB/c mice (4 mice per group) were inoculated orthotopically in the 4^{th} mammary fat pad with EMT6 cells (5x10⁵ cells in 100 µL PBS). Mice bearing 10-day established flank tumors were IV injected with a single dose of 1 x10⁷ CFUs of the YS1646Δ*asd*/ΔFLG/Δ*pagP*/Δ*ansB*/Δ*csgD*/Δ*lppAB* strain, or the parental YS1646Δ*asd*/ΔFLG/Δ*pagP*/Δ*ansB*/Δ*csgD* strain, each containing a plasmid encoding a luciferase protein (NanoLuciferase^{®} luciferase, or NanoLuc^{®}, Promega) that is secreted (secNanoLuc^{®}), under the control of the CMV promoter. At day 7 post IV dosing, mice were euthanized and tumors were homogenized and plated on LB plates, to enumerate the number of colony forming units (CFUs) per gram of tumor tissue. The parental YS1646Δ*asd*/ΔFLG/Δ*pagP*/Δ*ansB*/Δ*csgD* strain colonized tumors at a mean of 3.3 x10⁶ CFUs per gram of tumor tissue, while the lipoprotein-deleted YS1646Δ*asd*/ΔFLG/Δ*pagP*/Δ*ansB*/Δ*csgD*/Δ*lppAB* strain colonized the tumors with a 2-fold decreased mean of 1.18 x 10⁶ CFUs/g of tumor tissue.

In order to measure plasmid delivery to the tumor, and subsequent heterologous gene expression and protein secretion, the activity of the secNanoLuc^{®} was measured. For this, homogenized tumors were assessed for luciferase activity using the NanoGlo^{®} detection reagent (Promega), and read on a SpectraMax^{®} M3 Spectrophotometer/Luminometer (Molecular Devices). While the parental YS1646Δ*asd*/ΔFLG/Δ*pagP*/Δ*ansB*/Δ*csgD* strain induced average luminescence relative light units (RLUs) of 4482.6, the YS1646Δ*asd*/ΔFLG/Δ*pagP*/Δ*ansB*/Δ*csgD*/Δ*lppAB* strain induced a nearly 10-fold increased average of 33,926.6 RLUs. These data demonstrate the ability of the lipoprotein-deleted strain to improve tumor colonization, and to enhance payload expression.

These data reveal that, while deletion of lipoprotein somewhat reduces tumor colonization after IV dosing, it significantly enhances plasmid delivery and payload expression in the tumor. These data demonstrate that, contrary to the expectation from the art that deletion of these genes will decrease colonization, lipoprotein deletion enhances plasmid delivery and protein expression in the tumor microenvironment, and in tumors.

### Example 19

### Human and Murine 4-1BBL with a Truncated Cytosolic Domain are Expressed as Well as Wild-Type 4-1BBL

As discussed herein, variants of the co-stimulatory molecule 4-1BBL that lack the cytoplasmic domain (4-1BBLΔcyt), potentiate the activation of 4-1BBL, without the immunosuppressive reverse signaling that occurs through the molecule's cytoplasmic domain. It is shown in Example 9, that murine 4-1BBLΔcyt can be expressed on the surface of human cells. As shown below, 4-1BBL variants that completely lack the cytoplasmic domain are poorly expressed. To address this, provided are variants of 4-1BBL where the cytoplasmic domain is truncated, such that the immunosuppressive reverse signaling is eliminated.

Full-length human and mouse 4-1BBL contain positive charges within the cytoplasmic domain, which is at the N-terminus of the protein. This favors the N-terminal to be positioned within the cytoplasm, and allows for proper orientation of the transmembrane domain, and consequently, of the extracellular domain. Full-length human and mouse 4-1BBL are thus expressed at high levels. Upon deletion of the full cytoplasmic domain from human and mouse 4-1BBL, generating the 4-1BBLΔcyt variants, the positive charges at the N-terminal are removed, favoring the N-terminal to be positioned outside the cell. This results in an incorrect orientation of the transmembrane domain (which is now at the N-terminus), and an "inside out" configuration for the 4-1BBLΔcyt variants expressed on the cell surface. The 4-1BBLΔcyt variants are thus expressed at much lower levels than the full-length 4-1BBL proteins.

To improve 4-1BBL expression, without inducing immunosuppressive reverse signaling, human and mouse 4-1BBL variants were rationally designed with a truncation to the cytoplasmic domain, rather than a complete deletion of the cytoplasmic domain. The human and mouse 4-1BBL variants with the truncated cytoplasmic domains contain the last 4 (RVLP) and last 5 (SRHPK) amino acid residues, respectively, of the cytoplasmic domain, which are adjacent to the transmembrane domain. This re-introduces positively charged residues back into the N-terminal, favoring its position to be inside the cell, and correcting the orientation of the transmembrane domain and the "inside out" configuration. This results in increased expression of the 4-1BBL variants with the truncated cytoplasmic domains, compared to the expression of the 4-1BBLΔcyt variants, while still suppressing the immunosuppressive reverse signaling that occurs through the protein's cytoplasmic domain.

Additionally, variants of human and mouse 4-1BBL with truncated cytoplasmic domains, and with a Myc Tag added to the N-terminus, were generated, with the hypothesis that the addition of a Myc Tag would add extra positive residues to the N-terminal truncated cytoplasmic domain, correcting the inside out configuration and increasing protein expression.

The sequences of the full-length human and mouse 4-1BBL proteins, as well as the variants with the partially truncated cytoplasmic domains (CytTrunc), and the deleted cytoplasmic domains (Δcyt), are shown in the table below. The cytoplasmic domain is indicated in bold and is underlined; the transmembrane region is indicated in bold; the extracellular region is underlined; and the MycTag is double underlined.

### Sequences of Full-length Human and Mouse 4-1BBL, and of Variants with Truncated or Deleted Cytoplasmic Domains

| **Variant** | **Protein Sequence** | **SEQ ID NO.** |
|---|---|---|
| Human 4-1BBL, Full-length | MEY ASDASLDPEAPWPPAPRARACRVLPWALVAGLLLLLLLA AACAVFLACPWAVSGARASPGSAASPRLREGPELSPDDPAGLL DLRQGMFAQLVAQNVLLIDGPLSWYSDPGLAGVSLTGGLSYK EDTKELVVAKAGVYYVFFQLELRRVVAGEGSGSVSLALHLQPL RSAAGAAALALTVDLPPASSEARNSAFGFQGRLLHLSAGQRLG VHLHTEARARHAWQLTQGATVLGLFRVTPEIPAGLPSPRSE | 389 |
| Human 4-1BBLΔcyt | MWALVAGLLLLLLLAAACAVFLACPWAVSGARASPGSAASPR LREGPELSPDDPAGLLDLRQGMFAQLVAQNVLLIDGPLSWYSD PGLAGVSLTGGLSYKEDTKELVVAKAGVYYVFFQLELRRVVA GEGSGSVSLALHLQPLRSAAGAAALALTVDLPPASSEARNSAF GFQGRLLHLSAGQRLGVHLHTEARARHAWQLTQGATVLGLFR VTPEIPAGLPSPRSE | 390 |
| Human 4-1BBL CytTrunc | MRVLPWALVAGLLLLLLLAAACAVFLACPWAVSGARASPGSA ASPRLREGPELSPDDPAGLLDLRQGMFAQLVAQNVLLIDGPLS WYSDPGLAGVSLTGGLSYKEDTKELVVAKAGVYYVFFQLELR RVVAGEGSGSVSLALHLQPLRSAAGAAALALTVDLPPASSEAR NSAFGFQGRLLHLSAGQRLGVHLHTEARARHAWQLTQGATVL GLFRVTPEIPAGLPSPRSE | 391 |
| Human 4-1BBL CytTrunc + MycTag | MEQKLISEEDLRVLPWALVAGLLLLLLLAAACAVFLACPWAVS GARASPGSAASPRLREGPELSPDDPAGLLDLRQGMFAQLVAQN VLLIDGPLSWYSDPGLAGVSLTGGLSYKEDTKELVVAKAGVY YVFFQLELRRVVAGEGSGSVSLALHLQPLRSAAGAAALALTVD LPPASSEARNSAFGFQGRLLHLSAGQRLGVHLHTEARARHAW QLTQGATVLGLFRVTPEIPAGLPSPRSE | 392 |
| Mouse 4-1BBL, Full-length | MDQHTLDVEDTADARHPAGTSCPSDAALLRDTGLLADAALLS DTVRPTNAALPTDAAYPAVNVRDREAAWPPALNFCSRHPKLY GLVAL VLLLLIAACVPIFTRTEPRPALTITTSPNLGTRENNADQV TPVSHIGCPNTTQQGSPVFAKLLAKNQASLCNTTLNWHSQDGA GSSYLSQGLRYEEDKKELVVDSPGLYYVFLELKLSPTFTNTGH KVQGWVSLVLQAKPQVDDFDNLALTVELFPCSMENKLVDRS WSQLLLLKAGHRLSVGLRAYLHGAQDAYRDWELSYPNTTSFG LFLVKPDNPWE | 393 |
| Mouse 4-1BBLΔcyt | MLYGLVALVLLLLIAACVPIFTRTEPRPALTITTSPNLGTRENNA DQVTPVSHIGCPNTTQQGSPVFAKLLAKNQASLCNTTLNWHSQ DGAGSSYLSQGLRYEEDKKELVVDSPGLYYVFLELKLSPTFTN TGHKVQGWVSLVLQAKPQVDDFDNLALTVELFPCSMENKLVD RSWSQLLLLKAGHRLSVGLRAYLHGAQDAYRDWELSYPNTTS FGLFLVKPDNPWE | 394 |
| Mouse 4-1BBL CytTrunc | MSRHPKLYGLVALVLLLLIAACVPIFTRTEPRPALTITTSPNLGT RENNADQVTPVSHIGCPNTTQQGSPVFAKLLAKNQASLCNTTL NWHSQDGAGSSYLSQGLRYEEDKKELVVDSPGLYYVFLELKL SPTFTNTGHKVQGWVSLVLQAKPQVDDFDNLALTVELFPCSM ENKLVDRSWSQLLLLKAGHRLSVGLRAYLHGAQDAYRDWEL SYPNTTSFGLFLVKPDNPWE | 395 |
| Mouse 4-1BBL CytTrunc + Myc Tag | MEQKLISEEDLSRHPKLYGLVALVLLLLIAACVPIFTRTEPRPAL TITTSPNLGTRENNADQVTPVSHIGCPNTTQQGSPVFAKLLAKN QASLCNTTLNWHSQDGAGSSYLSQGLRYEEDKKELVVDSPGL YYVFLELKLSPTFTNTGHKVQGWVSLVLQAKPQVDDFDNLAL TVELFPCSMENKLVDRSWSQLLLLKAGHRLSVGLRAYLHGAQ DAYRDWELSYPNTTSFGLFLVKPDNPWE | 396 |

To compare the expression levels of the engineered 4-1BBL variants with those of the full-length 4-1BBL proteins, HEK293T cells were transfected with 500 ng of DNA encoding the full-length protein or variant, using the FuGENE^{®} transfection reagent (Promega), at the proper reagent:DNA ratios, with untransfected cells as negative controls. Forty-eight hours post-transfection, transfected HEK293T cells were harvested, and stained with either an anti-mouse 4-1BBL or anti-human 4-1BBL antibody, as appropriate. Staining for mouse 4-1BBL was performed using a biotinylated antibody against mouse 4-1BBL (clone TKS-1; BioLegend), followed by a streptavidin-allophycocyanin (APC) conjugate (BioLegend). Staining for human 4-1BBL was performed using an APC-conjugated antibody against human 4-1BBL (clone 5F4; BioLegend). The untransfected cells also were stained, to measure background fluorescence. The expression of membrane-bound 4-1BBL was measured by flow cytometry, and the results are provided in terms of the background-subtracted mean fluorescence intensity (dMFI) *(i.e.,* by subtracting the MFI of a negative control (stained untransfected cells), from the MFI of the sample).

As shown in the tables below, for both human and mouse 4-1BBL, expression of the variants with the truncated cytoplasmic domain was significantly higher than expression of the variants with a complete deletion of the cytoplasmic domain. Despite lacking the full-length cytoplasmic domain, the human 4-1BBL variant with the truncated cytoplasmic domain was expressed at 93% of the level of expression of full-length human 4-1BBL, whereas the human 4-1BBL variant with the full cytoplasmic domain deletion was expressed at 42% of the level of expression of full-length human 4-1BBL. For the orthologous mouse 4-1BBL, the variant with the truncated cytoplasmic domain was expressed at 63% of the level of expression of full-length mouse 4-1BBL, whereas the variant with the full cytoplasmic domain deletion was expressed at 13% of the level of expression of full-length mouse 4-1BBL.

For each of the truncated mouse and human 4-1BBL variants, a construct with a Myc tag was tested, to determine if adding additional positive amino acid residues to the truncated cytoplasmic portion of 4-1BBL would increase expression levels. As shown in the tables below, however, addition of the Myc tag to the variants with the truncated cytoplasmic domain decreased expression when compared to the variants with the truncated cytoplasmic domain and no Myc tag. It is undetermined why the Myc tag reduces expression, however, addition of the Myc tag could potentially result in a non-optimal sequence for 4-1BBL expression.

### Expression Levels of Human 4-1BBL Variants with Truncated or Deleted Cytoplasmic Domains

| **Human 4-1BBL Variants** | **Membrane-Bound 4-1BBL Expression in HEK293T Cells (dMFI)** | **% of Full-Length 4-1BBL Expression** |
|---|---|---|
| Human 4-1BBL, Full-length | 1.04E+04 | 100.00 |
| Human 4-1BBL, Cytoplasmic domain deleted | 4.39E+03 | 42.21 |
| Human 4-1BBL, Cytoplasmic domain truncated | 9.71E+03 | 93.37 |
| Human 4-1BBL, Cytoplasmic domain truncated + Myc tag | 1.77E+03 | 17.02 |

### Expression Levels of Murine 4-1BBL Variants with Truncated or Deleted Cytoplasmic Domains

| **Murine 4-1BBL Variants** | **Membrane-Bound 4-1BBL Expression in HEK293T Cells (dMFI)** | **% of Full-Length 4-1BBL Expression** |
|---|---|---|
| Murine 4-1BBL, Full-length | 4.06E+05 | 100.00 |
| Murine 4-1BBL, Cytoplasmic domain deleted | 5.43E+04 | 13.37 |
| Murine 4-1BBL, Cytoplasmic domain truncated | 2.56E+05 | 63.05 |
| Murine 4-1BBL, Cytoplasmic domain truncated + Myc tag | 7.54E+04 | 18.57 |

### Example 20

### Anti-CTLA-4 scFv-Fc Demonstrates Superior Blockade of the CD80/CTLA-4 and CD86/CTLA-4 Interactions Compared to Anti-CTLA-4 scFv

An scFv-Fc specific for human CTLA-4 (see, SEQ ID NOs:401 and 402 for nucleic acid and protein sequences, respectively) was designed using the amino acid sequence of ipilimumab. Ipilimumab is a fully human IgG1κ monoclonal antibody that specifically binds human CTLA-4 (see, *e.g.,* the antibody designated 10D1 in U.S. Patent Publication No. 2002/0086014 and in U.S. Patent No. 6,984,720), blocking CTLA-4's immune inhibiting interaction with CD80 (also known as B7.1 or B7-1) and CD86 (also known as B7.2 or B7-2).

To generate the ipilimumab scFv antibody fragment (see, SEQ ID NO:403), the variable light chain (V_{L}) and variable heavy chain (V_{H}) of ipilimumab were linked with a 20 amino acid long glycine-serine (GS) linker ((GGGGS)₄). To generate the scFv-Fc antibody fragment (see, SEQ ID NO:402), the variable heavy chain of the ipilimumab scFv was linked to a human IgG1 Fc, containing a mutation of the free cysteine in the hinge region to a serine (at position 272 in SEQ ID NO:402). The leader sequence (METPAQLLFLLLLWLPDTTG; corresponding to residues 1-20 in SEQ ID NO:402) was derived from the sequence of the human immunoglobulin kappa variable 3-20 (IGKV3-20) protein. The sequence was codon optimized using the GenScrip GenSmart^{™} Codon Optimization tool.

The neutralizing ability of the anti-CTLA-4 scFv-Fc was compared to that of the anti-CTLA-4 scFv (lacking the human IgG1 Fc portion) using competitive ELISAs to measure the ability of each of the antibody fragments to block the interactions between CTLA-4 and its ligands, CD80 and CD86. HEK293T cells were transfected with 3 micrograms of DNA encoding the anti-CTLA-4 scFv-Fc or the anti-CTLA-4 scFv antibody fragment constructs, using the FuGENE^{®} transfection reagent (Promega), at the proper reagent:DNA ratios. Forty-eight hours post-transfection, HEK293T cell-free culture supernatants were harvested, filtered, and used in a competitive ELISA to assess the blockade activity of the anti-CTLA-4 antibody fragments.

For the competitive ELISAs, mouse CD80 or CD86 recombinant proteins (R&D Systems) were coated overnight at 4 °C on a high protein-binding 96-well plate, at a concentration of 100 ng/ml. The wells were then washed one time with PBS 0.05% Tween-20, and the wells were blocked with ELISA blocking buffer for 1 hour at room temperature. The wells were then washed one time with PBS 0.05% Tween-20. HEK293T cell culture supernatants, containing each of the anti-CTLA-4 antibody fragments, were mixed with 10 ng/ml of a recombinant murine CTLA-4-human IgG1 Fc chimera (R&D Systems), and added to the wells and incubated for 2 hours at room temperature. The wells were then washed three times with PBS 0.05% Tween-20, and horseradish peroxidase (HRP)-conjugated anti-human IgG1 antibody was added to the wells (Jackson ImmunoResearch), and incubated for one hour at room temperature. The wells were then washed three times with PBS 0.05% Tween-20, and detection reagent (3,3',5,5'-tetramethylbenzidine (TMB), Thermo Fisher Scientific) was added to the wells. The enzymatic reaction was stopped with sulfuric acid (BioLegend), and the optical densities were read at 450 nm.

The results of the competitive ELISAs are summarized in the table below. The anti-CTLA-4 scFv-Fc blocked the binding of CTLA-4 to CD86 by 75.5%, and blocked the binding of CTLA-4 to CD80 by 40.6%, whereas the anti-CTLA-4 scFv blocked the binding of CTLA-4 to CD86 by 32.5%, and blocked the binding of CTLA-4 to CD80 by 7%. A higher degree of CD86/CTLA-4 blocking activity (compared with CD80/CTLA-4 blocking activity) was observed with both antibody fragments, and a superior neutralizing activity was observed with the anti-CTLA-4 scFv-Fc, when compared to the anti-CTLA-4 scFv.

### Competitive ELISA Results

| **Anti-CTLA-4 Antibody Fragment** | **% CD86 Blockade Activity** | **% CD80 Blockade Activity** |
|---|---|---|
| Anti-CTLA-4 scFv | 32.5 | 7.0 |
| Anti-CTLA-4 scFv-Fc | 75.5 | 40.6 |

Included among the immunostimulatory bacteria provided herein, are those that encode, on the plasmid, anti-CTLA-4 antibodies and fragments thereof, including the anti-CTLA-4 scFv antibody fragments and the anti-CTLA-4 scFv-Fc antibody fragments, as provided herein, as well as combinations with nucleic acid molecules encoding other therapeutic products.

### Example 21

### Strain YS1646Δasd/ΔFLG/ΔpagP/ΔansB/ΔcsgD is Restricted to the Phagocytic Myeloid Immune Cell Compartment In Vivo

Strain YS1646Δ*asd*/ΔFLG is deficient for non-phagocytic cell uptake, as demonstrated above (see, Example 4). To confirm that strain YS1646Δ*asd*/ΔFLG/Δ*pagP*/Δ*ansB*/Δ*csgD* also is deficient for non-phagocytic cell uptake, a strain of YS1646Δ*asd*/ΔFLG/Δ*pagP*/Δ*ansB*/Δ*csgD*, constitutively expressing mCherry (a red fluorescent protein) under the bacterial *rpsM* promoter, was IV administered to EMT6 orthotopic tumor-bearing mice.

For this experiment, 6-8 week-old female Balb/c mice (4 mice per group) received orthotopic implantation of EMT6 cells (2 x10⁵ cells in 100 µL PBS). Mice bearing large established flank tumors were IV injected on day 10 with 3x10⁶ CFUs of the YS1646Δ*asd*/ΔFLG/Δ*pagP*/Δ*ansB*/Δ*csgD-*mCherry strain. Tumors were resected 8 days post IV dosing, and cut into 2-3 mm pieces into gentleMACS^{™} C tubes (Miltenyi Biotec) filled with 2.5 mL enzyme mix (RPMI-1640 containing 10% FBS with 1 mg/mL Collagenase IV and 20 µg/mL DNase I). The tumor pieces were dissociated using OctoMACS^{™} (Miltenyi Biotec) specific dissociation program (mouse implanted tumors), and the whole cell preparation was incubated with agitation for 45 minutes at 37 °C. After the 45 minute incubation, a second round of dissociation was performed using the OctoMACS^{™} (mouse implanted tumor) program, and the resulting single cell suspensions were filtered through a 70 µM nylon mesh into a 50 mL tube. The nylon mesh was washed once with 5 mL of RPMI-1640 containing 10% FBS, and the cells were filtered a second time using a new 70 µM nylon mesh, into a new 50 mL tube. The nylon mesh was washed with 5 mL of RPMI-1640 containing 10% FBS, and the filtered cells were then centrifuged at 1000 RPM for 7 minutes. The resulting dissociated cells were resuspended in PBS and kept on ice before the staining process.

For the flow-cytometry staining, 100 µL of the single cell suspensions were seeded in wells of a V-bottom 96-well plate. PBS containing a dead/live stain (Zombie Aqua^{™}, BioLegend) and Fc Blocking reagents (BD Biosciences) were added at 100 µL per well, and the plate was incubated on ice for 30 minutes in the dark. After 30 minutes, cells were washed twice with PBS + 2% FBS by centrifugation at 1300 RPM for 3 minutes. Cells were then resuspended in PBS + 2% FBS containing fluorochrome-conjugated antibodies (CD4 FITC clone RM4-5; CD8a BV421 clone 53-6.7; F4/80 APC clone BM8; CD11b PE-Cy7 clone M1/70; CD45 BV570 clone 30-F11; CD3 PE clone 145-2C11; Ly6C BV785 clone HK1.4; I-A/I-E APC-Cy7 clone M5/114.15.2; Ly6G BV605 clone 1A8; and CD24 PercP-Cy5.5 clone M1/69; all from BioLegend), and incubated on ice for 30 minutes in the dark. After 30 minutes, cells were washed twice with PBS + 2% FBS by centrifugation at 1300 RPM for 3 minutes, and resuspended in flow cytometry fixation buffer (Thermo Fisher Scientific). Flow cytometry data were acquired using the NovoCyte^{®} Flow Cytometer (ACEA Biosciences, Inc.) and analyzed using the FlowJo^{™} software (Tree Star, Inc.).

The results (see tables below) demonstrated that, in tumors from mice IV injected with the YS1646Δ*asd*/ΔFLG/Δ*pagP*/Δ*ansB*/Δ*csgD-*mCherry strain, 1.95% of tumor-infiltrating monocytes and 3.36% of tumor-associated neutrophils (TANs) in the tumor microenvironment were positive for mCherry expression, whereas tumors from mice IV injected with PBS showed background staining/fluorescence of 0.64% for tumor-infiltrating monocytes, and 0.01% for TANs. 2.92% of the tumor-associated macrophage (TAM) population, and 4.34% of the tumor-infiltrating dendritic cells (DCs) had taken up the YS1646Δ*asd*/ΔFLG/Δ*pagP*/Δ*ansB*/Δ*csgD-*mCherry strain, while background fluorescence in tumors from mice injected with PBS was 0.70% and 0.50% for TAMs and DCs, respectively. 2.39% of M1 TAMs, and 0.53% of M2 TAMs stained positive for YS1646Δ*asd*/ΔFLG/Δ*pagP*/Δ*ansB*/Δ*csgD-*mCherry, while tumors from PBS-injected mice had background signals of 0.20% in M1 TAMs and 0.50% in M2 TAMs. In contrast, within the CD45⁻ population, corresponding to stromal and tumor cells *(i.e.,* cells that are not tumor-resident immune/myeloid cells), only 0.081% showed positivity for mCherry expression (compared to 0.002% background staining, from PBS-injected mice).

### % mCherry Expression in Tumors from Mice IV Injected with the YS1646Δasd/ΔFLG/ΔpagP/ΔansB/ΔcsgD-mCherry Strain

| **Experiment #** | **% mCherry Expression by Cell Type** | | | | | | |
|---|---|---|---|---|---|---|---|
| | **CD45⁻ Cells** | **DCs** | **M1 TAMs** | **M2 TAMs** | **TAMs** | **Mono-cytes** | **TANs** |
| 1 | 0.00248 | 0.00000 | 0.28000 | 0.29000 | 0.57000 | 0.33000 | 0.00000 |
| 2 | 0.20000 | 10.50000 | 5.84000 | 1.06000 | 6.90000 | 4.37000 | 7.61000 |
| 3 | 0.09100 | 6.02000 | 3.25000 | 0.52000 | 3.77000 | 2.23000 | 5.55000 |
| 4 | 0.02900 | 0.82000 | 0.20000 | 0.25000 | 0.45000 | 0.88000 | 0.29000 |
| **Average** | **0.08062** | **4.33500** | **2.39250** | **0.53000** | **2.92250** | **1.95250** | **3.36250** |
| SD | 0.08781 | 4.89877 | 2.70125 | 0.37283 | 3.07408 | 1.79852 | 3.81108 |

| | | | | | | | |
|---|---|---|---|---|---|---|---|
| SD = standard deviation; DCs = dendritic cells; TAMs = tumor-associated macrophages; TANs = tumor-associated neutrophils | | | | | | | |

### % mCherry Expression in Tumors from Mice IV Injected with PBS

| **Experiment #** | **% mCherry Expression by Cell Type** | | | | | | |
|---|---|---|---|---|---|---|---|
| | **CD45⁻ Cells** | **DCs** | **M1 TAMS** | **M2 TAMs** | **TAMs** | **Mono-cytes** | **TANs** |
| 1 | 0.00307 | 0.19000 | 0.15000 | 0.43000 | 0.58000 | 0.28000 | 0.01300 |
| 2 | 0.00129 | 0.58000 | 0.10000 | 0.64000 | 0.74000 | 1.38000 | 0.01100 |
| 3 | 0.00283 | 0.97000 | 0.36000 | 0.60000 | 0.96000 | 0.69000 | 0.00350 |
| 4 | 0.00169 | 0.25000 | 0.19000 | 0.34000 | 0.53000 | 0.21000 | 0.00599 |
| **Average** | **0.00222** | **0.49750** | **0.20000** | **0.50250** | **0.70250** | **0.64000** | **0.00837** |
| SD | 0.00086 | 0.35864 | 0.11284 | 0.14151 | 0.25435 | 0.53684 | 0.00439 |

| | | | | | | | |
|---|---|---|---|---|---|---|---|
| SD = standard deviation; DCs = dendritic cells; TAMs = tumor-associated macrophages; TANs = tumor-associated neutrophils | | | | | | | |

These data indicate that the deletion of *pagP*, *ansB*, and *csgD* from the flagella-eliminated strain, does not significantly impact the specificity of bacterial uptake by the phagocytic immune cell compartment of the tumor microenvironment *(i.e.,* the tumor-resident immune/myeloid cells). As discussed above and elsewhere herein, the flagella and its downstream signaling impact on SPI-1 are required for epithelial cell infectivity, and the lack thereof restricts uptake of the bacteria to only the tumor-resident immune/myeloid cells. Eliminating the flagella confers numerous benefits to the immunostimulatory bacteria, including the *S. typhimurium* strains. These benefits include eliminating TLR5-induced inflammatory cytokines that suppress adaptive immunity, reducing macrophage pyroptosis, as well as maintaining (or enhancing) tumor-specific enrichment upon systemic administration, where uptake is confined to tumor-resident phagocytic cells.

It is shown herein that elimination of *pagP, ansB*, and *csgD* from the flagella-eliminated strain does not significantly impact the specificity of bacterial uptake by tumor-resident immune cells, and confers additional benefits, including, for example, reducing TLR4-induced pro-inflammatory cytokines, reducing immunogenicity and enhancing tolerability (*ΔpagP*); restoring T-cell function (Δ*ansB*); and improving the intracellular uptake of the bacteria, which enhances plasmid delivery of encoded immunomodulatory proteins *in vivo* and improves therapeutic efficacy *(ΔcsgD).*

### Example 22

### Salmonella purI and msbB Clean Deletion Gene Knockout Strain Engineering and Characterization

The *purI* gene in strain YS1646 (VNP20009) was not deleted; it was disrupted by a transposon (Tn10) insertion that resulted in a 16.6 kbp (kilobase pair) genomic inversion event, whereby two insertion sequence (IS) elements were subsequently incorporated into the genome, one within the p*urI* (*purM*) gene, and the other 16.6 kbp upstream, in the intergenic region directly flanking the 3'-end of the *acrD* gene. The region between the two IS elements is inverted and contains 18 genes, including *yffB,* DC51_2568, *upp,* uraA, *yfgE*, *yfgD*, DC51_2573, *perM*, *purC,* and others. The insertion sequence element in the intergenic region encodes a fully functional transposase, and represents a potential genetic stability concern (see, *e*.*g*., Broadway et al. (2014) J. Biotechnology 192:177-178). The presence of the complete genetic sequence of the *purI* gene, disrupted by means of a chromosomal reengagement, leaves open the possibility of reversion to a wild-type gene.

The *msbB* gene in strain YS1646 also was not fully deleted, but was disrupted by a genetically engineered 511 bp deletion (of the 972 bp gene) that resulted in an extension of the *pykA* gene (encoding pyruvate kinase), replacing the last 5 amino acid codons with 13 new codons (see, *e.g.,* Broadway et al. (2014) J. Biotechnology 192:177-178).

To eliminate any possible reversion of the *purI* and *msbB* genes to their respective wild-type genes, and to determine the effects of the clean deletions of these genes, strain YS1646Δ*asd*/ΔFLG/Δ*pagP*/Δ*ansB*/Δ*csgD* was further modified to delete the remaining *purI* and *msbB* gene sequence fragments, as well as two transposon-associated insertion sequence elements, and a *pykA* gene extension.

### Deletion of purI Gene Fragments and Transposon-Associated Insertion Sequence Elements

A first region, located between the yffB and *purN* genes, and containing: 1) a 1,209 bp transposon insertion sequence element, annotated as DC51_2586 in the sequence obtained from Broadway *et. al* (2014) (see, GenBank Accession numbers CP007804 and CP008745); and 2) 740 bp of the remaining 891 bp *purI* gene fragment (referred to herein as the large *purI* gene fragment), was targeted for deletion, using modifications of the method of Datsenko and Wanner (see, Proc. Natl. Acad. Sci. U.S.A. 97:6640-6645 (2000)). A small 151 bp portion of the 891 bp (large) *purI* gene fragment was left intact to avoid affecting the adjacent, downstream gene, *purN* (encoding phosphoribosylglycinamide formyltransferase). A plasmid, pSL0165, containing 284 and 262 bps of homology to the left hand and right hand regions, respectively, of the DC51_2586 insertion sequence element and the large *purI* gene fragment, was transformed into DH5-alpha competent cells (Thermo Fisher Scientific). A kanamycin gene cassette flanked by loxP sites was cloned into this plasmid, and the resulting vector was designated pSL0174. The DC51_2586 insertion sequence element and the large *purI* gene fragment knockout cassette then was PCR amplified using primers purm-1 and purm-2 (SEQ ID NOs: 410 and 411, respectively; see Table 2), gel purified, and introduced into strain YS1646Δ*asd*/ΔFLG/Δ*pagP*/Δ*ansB*/Δ*csgD* carrying the temperature sensitive lambda red recombination plasmid pKD46 by electroporation. The kanamycin resistance gene then was cured by cre-mediated recombination as described above, and the temperature-sensitive plasmids were cured by growth at non-permissive temperature. The DC51_2586 insertion sequence element and large *purI* gene fragment knockout sequences were confirmed by PCR using primers purm-3 and purm-4 (SEQ ID NOs: 412 and 413, respectively; see Table 2), and verified by DNA sequencing. The resulting mutant derivative of parental strain YS1646Δ*asd*/ΔFLG/Δ*pagP*/Δ*ansB*/Δ*csgD* was designated YS1646Δ*asd*/ΔFLG/Δ*pagP*/Δ*ansB*/Δ*csgD*/ΔpurI_{(large clean)}.

A second region, located between the *acrD* and DC51_2568 genes, and containing: 1) a 1,209 bp transposon insertion sequence element, annotated as DC51_2566 in the sequence obtained from Broadway et. al (2014) (see, GenBank Accession numbers CP007804 and CP008745); and the remaining 231 bp *purI* gene fragment (referred to herein as the small *purI* gene fragment), was targeted for deletion, using modifications of the method of Datsenko and Wanner (see, Proc. Natl. Acad. Sci. U.S.A. 97:6640-6645 (2000)). A plasmid, pSL0210, containing 241 and 265 bps of homology to the left hand and right hand regions, respectively, of the DC51_2566 insertion sequence element and the small *purI* gene fragment, was transformed into DH5-alpha competent cells (Thermo Fisher Scientific). A kanamycin gene cassette flanked by loxP sites was cloned into this plasmid, and the resulting vector was designated pSL0212. The DC51_2566 insertion sequence element and the small *purI* gene fragment knockout cassette then was PCR amplified using primers acrd-1 and purm-5 (SEQ ID NOs: 416 and 414, respectively; see Table 2), gel purified, and introduced into strain YS1646Δ*asd*/ΔFLG/Δ*pagP*/Δ*ansB*/Δ*csgD*/ΔpurI_{(large clean)} carrying the temperature sensitive lambda red recombination plasmid pKD46 by electroporation. The kanamycin resistance gene then was cured by cre-mediated recombination as described above, and the temperature-sensitive plasmids were cured by growth at non-permissive temperature. The DC51_2566 insertion sequence element and small *purI* gene fragment knockout sequences were confirmed by PCR using primers purm-6 and acrd-3 (SEQ ID NOs: 415 and 417, respectively; see Table 2), and verified by DNA sequencing. The resulting mutant derivative of parental strain YS1646Δ*asd*/ΔFLG/Δ*pagP*/Δ*ansB*/Δ*csgD*/Δ*purI*_{(large clean)} was designated as YS1646Δ*asd*/ΔFLG/Δ*pagP*/Δ*ansB*/Δ*csgD*/Δ*purI*_{(clean)}*.*

### Deletion of msbB Gene Fragment and pykA Extension

The *msbB* gene in strain YS1646 was disrupted by a genetically engineered 511 bp deletion (of the 972 bp gene) that resulted in an extension of the *pykA* gene, replacing the last 5 amino acid codons with 13 new codons (see, *e.g.,* Broadway et al. (2014) J. Biotechnology 192:177-178). The region containing the remaining *msbB* gene fragment and the sequence encoding the last 13 amino acid codons in *pykA* was targeted for deletion using modifications of the method of Datsenko and Wanner (see, Proc. Natl. Acad. Sci. U.S.A. 97:6640-6645 (2000)). A plasmid, SL0209 (see, SEQ ID NO:408), containing 200 and 196 bp of homology to the left hand and right hand regions, respectively, of the remaining *msbB* gene fragment and the *pykA* gene extension, was transformed into DH5-alpha competent cells (Thermo Fisher Scientific). A kanamycin gene cassette flanked by loxP sites was cloned into this plasmid, and the resulting vector was designated pSL0211 (see, SEQ ID NO:409). The *msbB* gene fragment and *pykA* gene extension knockout cassette then was PCR amplified using primers msbB-1 and msbB-2 (SEQ ID NOs: 418 and 419, respectively; see, Table 2), gel purified, and introduced into strain YS1646Δ*asd*/ΔFLG/Δ*pagP*/Δ*ansB*/Δ*csgD*/ΔpurI_{(clean}), carrying the temperature sensitive lambda red recombination plasmid pKD46 by electroporation. The kanamycin resistance gene then was cured by cre-mediated recombination as described above, and the temperature-sensitive plasmids were cured by growth at non-permissive temperature. The *msbB* gene fragment and *pykA* gene extension knockout sequences were confirmed by PCR using primers msbB-3 and msbB-4 (SEQ ID NOs: 420 and 421, respectively; see, Table 2), and verified by DNA sequencing. The resulting mutant derivative of parental strain YS1646Δ*asd*/ΔFLG/Δ*pagP*/Δ*ansB*/Δ*csgD*/ΔpurI_{(clean)} was designated as YS1646Δ*asd*/ΔFLG/Δ*pagP*/Δ*ansB*/Δ*csgD*/Δ*purI*_{(clean)}/Δ*msbB*_{(clean)}*.*

**Table 2. Primer Sequence Information**

| **Primer** | **Primer Sequence** | **Primer Description** | **SEQ ID NO.** |
|---|---|---|---|
| purm-1 | ccagatgtggcttgtttatacttcgc | Fwd primer for amplifying the DC51_2586 insertion sequence element and the large *purI* (740 bp) fragment gene KO cassette | 410 |
| purm-2 | ccttattgctgaatactgccctgag | Rev primer for amplifying the DC51_2586 insertion sequence element and the large *purI* (740 bp) gene fragment KO cassette | 411 |
| purm-3 | cgtgtcgcaattcttaatgccatag | Fwd primer for checking DC51_2586 insertion sequence element and the large *purI* (740 bp) gene fragment KO | 412 |
| purm-4 | caaacatcggactcagaatacgc | Rev primer for checking DC51_2586 insertion sequence element and the large *purI* (740 bp) gene fragment KO | 413 |
| purm-5 | caggcgataaaggctaacaaccg | Rev primer for amplifying the DC51_2566 insertion sequence element and the small *purI* (231 bp) gene fragment KO cassette | 414 |
| purm-6 | ttgacctggatcaaccaaaagcg | Rev primer for checking the DC51_2566 insertion sequence element and the small *purI* (231 bp) gene fragment KO | 415 |
| acrd-1 | cctgcgaccgatactgatgac | Fwd primer for amplifying the DC51_2566 insertion sequence element and the small *purI* (231 bp) gene fragment KO cassette | 416 |
| acrd-3 | gctactcgctacctggatgc | Fwd primer for checking the DC51_2566 insertion sequence element and the small *purI* (231 bp) gene KO | 417 |
| msbB-1 | ggtcagatcgtcgtgaatacctg | Fwd primer for amplifying the *msbB* gene fragment and *pykA* gene extension KO cassette | 418 |
| msbB-2 | atgaacgcacgctgaacctg | Rev primer for amplifying the *msbB* gene fragment and *pykA* gene extension KO cassette | 419 |
| msbB-3 | acaccacacgttacatgcacttg | Fwd primer for checking the *msbB* gene fragment and *pykA* gene extension KO | 420 |
| msbB-4 | aagccattgccatgtctgcg | Rev primer for checking the *msbB* gene fragment and *pykA* gene extension KO | 421 |

| | | | |
|---|---|---|---|
| Fwd= forward; Rev = reverse; KO = knockout | | | |

### Plasmid Information

| **Gene Sequence** | **Synthetic DNA Vector** | **Vector Name after Kanamycin Cloning** | **Homology Arm Length** | |
|---|---|---|---|---|
| | | | **Left Hand** | **Right Hand** |
| *purI* large (740 bp) fragment | pSL0165 | pSL0174 | 284 bp | 262 bp |
| *purI* small (231 bp) fragment | pSL0210 | pSL0212 | 241 bp | 265 bp |
| *msbB* fragment | pSL0209 | pSL0211 | 200 bp | 196 bp |

### The YS1646Δasd/ΔFLG/ΔpagP/ΔansB/ΔcsgD/ΔpurI_{(clean)}/ΔmsbB_{(clean)} Strain Displays Enhanced Growth in Broth Media

To evaluate the effects of the deletions of the remaining *purI* and *msbB* gene fragments, the transposon-associated insertion sequence elements, and the *pykA* gene extension, from the bacterial genome, on the *in vitro* fitness of the immunostimulatory bacteria, the broth growth profiles of strains YS1646Δ*asd*/ΔFLG/Δ*pagP*/Δ*ansB*/Δ*csgD*/Δ*purI*_{(clean)}/Δ*msbB*_{(clean)} and YS1646Δ*asd*/ΔFLG/Δ*pagP*/Δ*ansB*/Δ*csgD*, expressing the same plasmid encoding nanoLuciferase^{®} under control of a CMV promoter (a plasmid designated ADN-256), were compared directly by broth growth assay. Overnight stationary phase cultures were adjusted to OD₆₀₀ₙₘ = 1 by dilution in LB media, and 5 µl of the resulting normalized culture was used to inoculate 250 µl of LB media in a 96-well plate, in technical duplicate. The plate was incubated with shaking at 37 °C, and the OD₆₀₀ₙₘ value was monitored in 15 minute intervals over the course of 16 hours. OD₆₀₀ₙₘ values were plotted to construct growth curves, and the slope of the log phase of growth was calculated and used to determine doubling time for each strain. Four independent growth curves were performed, and the results, depicted in the table below, show that strain YS1646Δ*asd*/ΔFLG/Δ*pagP*/Δ*ansB*/Δ*csgD-*(ADN-256) had an average doubling time of 67.74 minutes (standard deviation (SD) = 3.21), while strain YS1646Δ*asd*/ΔFLG/Δ*pagP*/Δ*ansB*/Δ*csgD*/Δ*purI*_{(clean)}/Δ*msbB*_{(clean)} -(ADN-256) had an average doubling time of 60.13 minutes (SD = 3.08).

### Effects of Clean Deletions of purI and msbB on Growth of Immunostimulatory Bacteria in Broth Media

| **Strain** | **Doubling Time (minutes)** | | | | **Average Doubling Time (mins)** | **SD** |
|---|---|---|---|---|---|---|
| | **Growth Curve 1** | **Growth Curve 2** | **Growth Curve 3** | **Growth Curve 4** | | |
| YS1646Δ*asd*/ΔFLG/Δ*pagP*/Δ*ansB*/Δ*csgD-*(ADN-256) | 65.39 | 72.20 | 65.39 | 67.96 | 67.74 | 3.21 |
| YS1646Δ*asd*/ΔFLG/Δ*pagP*/Δ*ansB*/Δ*csgD*/Δ*purI*_{(clean)}/Δ*msbB*_{(clean)}- (ADN-256) | 58.25 | 64.18 | 57.28 | 60.80 | 60.13 | 3.08 |

| | | | | | | |
|---|---|---|---|---|---|---|
| SD = Standard Deviation | | | | | | |

Strains YS1646Δ*asd*/ΔFLG/Δ*pagP*/Δ*ansB*/Δ*csgD* and YS1646Δ*asd*/ΔFLG/Δ*pagP*/Δ*ansB*/Δ*csgD*/Δ*purI*_{(clean)}/Δ*msbB*_{(clean)}, containing the same plasmid (ADN-480, encoding murine 4-1BBLΔcyt, murine IL-12p70, and a modified human STING chimeric protein), were evaluated for broth growth in large, highly aerated shaker flask conditions with rich media (4XYT). 50 µl of overnight stationary phase cultures of equivalent OD₆₀₀ₙₘ optical densities (ODs) were used to inoculate 12.5 ml of 4XYT media in 125 ml baffled shaker flasks with vented caps, and the cultures were incubated at 37 °C with shaking at 225 RPM. The OD₆₀₀ₙₘ was monitored in approximately 1 hour intervals, over the course of 18 hours. OD₆₀₀ₙₘ values were plotted to construct growth curves, and the slope of the log phase of growth was calculated and used to determine the doubling time for each strain.

The results showed that strain YS1646Δ*asd*/ΔFLG/Δ*pagP*/Δ*ansB*/Δ*csgD*/Δ*purI*_{(clean)}/Δ*msbB*_{(clean)}-(ADN-480), which had a doubling time of 41.83 minutes, grew slightly faster and reached stationary phase quicker than strain YS1646Δ*asd*/ΔFLG/Δ*pagP*/Δ*ansB*/Δ*csgD*-(ADN-480), which had a doubling time of 48.33 minutes. This data demonstrates an enhanced growth profile for strain YS1646Δ*asd*/ΔFLG/Δ*pagP*/Δ*ansB*/Δ*csgD*/Δ*purI*_{(clean)}/Δ*msbB*_{(clean)}, compared to the parental strain, YS1646Δ*asd*/ΔFLG/Δ*pagP*/Δ*ansB*/Δ*csgD.* Strain YS1646Δ*asd*/ΔFLG/Δ*pagP*/Δ*ansB*/Δ*csgD*/Δ*purI*_{(clean)}/Δ*msbB*_{(clean)} grew with faster doubling times in two independent growth curve assays (LB media in a 96-well plate, and rich 4XYT media in a 250 ml shaker flask), indicating that the genomic deletions of *purI* and *msbB* not only alleviate a potential genetic stability issue, but also confer a metabolic benefit which allows for faster growth and superior fitness.

### Strain YS1646Δasd/ΔFLG/ΔpagP/ΔansB/ΔcsgD/ΔpurI_{(clean)}/ΔmsbB_{(clean)} Displays Enhanced Injection Stock Cell Viability

To evaluate the effects of the deletions of the remaining *purI* and *msbB* gene fragments, the transposon-associated insertion sequence elements, and the *pykA* gene extension, from the bacterial genome, on the *in vitro* fitness of the immunostimulatory bacteria, the cell viability of strains YS1646Δ*asd*/ΔFLG/Δ*pagP*/Δ*ansB*/Δ*csgD*/Δ*purI*_{(clean)}/Δ*msbB*_{(clean)} and YS1646Δ*asd*/ΔFLG/Δ*pagP*/Δ*ansB*/Δ*csgD*, containing the same plasmid (ADN-542, encoding a modified human STING chimeric protein), was evaluated by directly comparing viable CFUs after culture processing for frozen injection stocks. 100 µl of overnight stationary phase cultures of equivalent OD₆₀₀ₙₘ values were used to inoculate 25 ml of 4XYT media in 250 ml baffled shaker flasks with vented caps, and the cultures were incubated at 37 °C shaking at 225 RPM for approximately 6 hours. The cultures were harvested at stationary phase at equivalent OD₆₀₀ₙₘ values (11.4 for strain YS1646Δ*asd*/ΔFLG/Δ*pagP*/Δ*ansB*/Δ*csgD*-(ADN-542), and 11.7 for strain YS1646Δ*asd*/ΔFLG/Δ*pagP*/Δ*ansB*/Δ*csgD*/Δ*purI*_{(clean)}/Δ*msbB*_{(clean)}-(ADN-542)), washed twice, adjusted to OD₆₀₀ₙₘ = 2, aliquoted, and frozen at -80 °C. The following day, two aliquots of each strain were thawed, the OD₆₀₀ₙₘ values were measured, and the cultures were plated on agar plates to determine titer and viability. % viability was determined by calculating the ratio of OD₆₀₀ₙₘ to CFUs/ml. The viability of the YS1646Δ*asd*/ΔFLG/Δ*pagP*/Δ*ansB*/Δ*csgD-*(ADN-542) strain injection stock preparation was determined to be 52%, and the viability of the YS1646Δ*asd*/ΔFLG/Δ*pagP*/Δ*ansB*/Δ*csgD*/Δ*purI*_{(clean)}/Δ*msbB*_{(clean)}-(ADN-542) strain injection stock preparation was 96%.

This data demonstrates an enhanced fitness profile for strain YS1646Δ*asd*/ΔFLG/Δ*pagP*/Δ*ansB*/Δ*csgD*/Δ*purI*_{(clean)}/Δ*msbB*_{(clean)}, compared to the parental strain, YS1646Δ*asd*/ΔFLG/Δ*pagP*/Δ*ansB*/Δ*csgD.* Strain YS1646Δ*asd*/ΔFLG/Δ*pagP*/Δ*ansB*/Δ*csgD*/Δ*purI*_{(clean)}/Δ*msbB*_{(clean)} displayed an enhanced viability following frozen injection stock preparation, indicating that the genomic deletions of *purI* and *msbB* not only diminish a potential genetic stability issue, but also confer a metabolic benefit, which allows for increased cell viability.

### Strain YS1646Δasd/ΔFLG/ΔpagP/ΔansB/ΔcsgD/ΔpurI_{(clean)}/ΔmsbB_{(clean)} Displays Enhanced Plasmid Payload Delivery And Ectopic Gene Expression in the Tumor Microenvironment

To evaluate plasmid delivery and subsequent ectopic gene expression in the tumor microenvironment, strains YS1646Δ*asd*/ΔFLG/Δ*pagP*/Δ*ansB*/Δ*csgD* and YS1646Δ*asd*/ΔFLG/Δ*pagP*/Δ*ansB*/Δ*csgD*/Δ*purI*_{(clean)}/Δ*msbB*_{(clean)}, containing the same plasmid (ADN-480), encoding murine IL-12 (muIL-12p70) (as well as murine 4-1BBLΔCyt, and a modified human STING chimeric protein), were evaluated in an orthotopic EMT6 mammary carcinoma model *in vivo.* BALB/c mice received orthotopic implantation with 5×10⁵ EMT6 cells, followed by intravenous injection of 1×10⁷ CFUs of strain YS1646Δ*asd*/ΔFLG/Δ*pagP*/Δ*ansB*/Δ*csgD*-(ADN-480), or strain YS1646Δ*asd*/ΔFLG/Δ*pagP*/Δ*ansB*/Δ*csgD*/Δ*purI*_{(clean)}/Δ*msbB*_{(clean)}-(ADN-480), or PBS, 10 days post implantation. Four days post injection, mice were sacrificed, and the tumors were harvested and processed for analysis of murine IL-12p70 expression by reverse transcription quantitative real-time PCR (RT-qPCR).

Tumors were resected four days post IV dosing, and cut into 2-3 mm pieces into gentleMACS^{™} C tubes (Miltenyi Biotec) filled with 2.5 mL enzyme mix (RPMI-1640 containing 10% FBS with 1 mg/mL Collagenase IV and 20 µg/mL DNase I). The tumor pieces were dissociated using OctoMACS^{™} (Miltenyi Biotec) specific dissociation program (mouse implanted tumors), and the whole cell preparation was incubated with agitation for 45 minutes at 37 °C. After the 45 minute incubation, a second round of dissociation was performed using the OctoMACS^{™} (mouse implanted tumor) program, and the resulting single cell suspensions were filtered through a 70 µM nylon mesh into a 50 mL tube. The nylon mesh was washed once with 5 mL of RPMI-1640 containing 10% FBS, and the cells were filtered a second time using a new 70 µM nylon mesh, into a new 50 mL tube. The nylon mesh was washed with 5 mL of RPMI-1640 containing 10% FBS, and the filtered cells were then centrifuged at 1000 RPM for 7 minutes. The resulting dissociated cells were resuspended in PBS, then lysed with 200 µL RNA Lysis Buffer (Zymo Research), and RNA extraction was performed using the Zymo Research *Quick-*RNA^{™} 96 Kit, according to the manufacturer's protocol. Total RNA concentration was measured using a NanoDrop^{™} 2000 UV-Vis Spectrophotometer (Thermo Fisher Scientific). The purity of each sample also was assessed from the A₂₆₀/A₂₃₀ absorption ratio.

Synthesis of cDNA was performed from 0.5-1 µg of template RNA using a CFX96^{™} Real-Time PCR Detection System (Bio-Rad) and iScript^{™} Reverse Transcription Supermix for RT-qPCR (Bio-Rad) in a 20 µL reaction, according to the manufacturer's instructions. A PrimePCR^{™} Probe Assay for murine IL-12p70 (muIL-12p70) was purchased from Bio-Rad. The qPCR reaction (20 µL) was conducted per protocol, using the iQ^{™} Multiplex Powermix (Bio-Rad). The standard thermocycling program on the Bio-Rad CFX96^{™} Real-Time PCR Detection System consisted of a 95 °C denaturation for 150 seconds, followed by 39 cycles of 95 °C for 15 seconds and 60 °C for 55 seconds. All samples were run in triplicate, and the mean C_{q} values were calculated. Quantification of the target mRNA was normalized using actin reference mRNA (Bio-Rad, assay ID: qHsaCEP0036280). ΔC_{q} was calculated as the difference between the target and reference gene. ΔΔC_{q} was obtained by normalizing the ΔC_{q} values of the YS1646Δ*asd*/ΔFLG/Δ*pagP*/Δ*ansB*/Δ*csgD*-(ADN-480) and YS1646Δ*asd*/ΔFLG/Δ*pagP*/Δ*ansB*/Δ*csgD*/Δ*purI*_{(clean)}/Δ*msbB*_{(clean)}-(ADN-480) treated groups to the average ΔC_{q} value of the PBS-treated control group, and the fold increase in murine IL-2p70 expression relative to PBS was calculated as 2^-ΔΔC_{q}. Murine IL-12p70 expression in PBS-treated tumors was normalized to a value of 1.0, based on the average ΔC_{q} values.

As shown in the table below, IV injection of strain YS1646Δ*asd*/ΔFLG/Δ*pagP*/Δ*ansB*/Δ*csgD*-(ADN-480) resulted in an average fold increase of muIL-12p70 expression relative to PBS (2^-ΔΔC_{q}) of 5.19, while IV injection of strain YS1646Δ*asd*/ΔFLG/Δ*pagP*/Δ*ansB*/Δ*csgD*/Δ*purI*_{(clean)}/Δ*msbB*_{(clean)}-(ADN-480) resulted in an average fold increase of muIL-12p70 expression relative to PBS (2^-ΔΔC_{q}) of 6.52.

These data demonstrate an enhanced capability for plasmid delivery and induction of ectopic gene expression by strain YS1646Δ*asd*/ΔFLG/Δ*pagP*/Δ*ansB*/Δ*csgD*/Δ*purI*_{(clean)}/Δ*msbB*_{(clean)}, compared to the parental strain, YS1646Δ*asd*/ΔFLG/Δ*pagP*/Δ*ansB*/Δ*csgD.* Strain YS1646Δ*asd*/ΔFLG/Δ*pagP*/Δ*ansB*/Δ*csgD*/Δ*purI*_{(clean)}/Δ*msbB*_{(clean)} displayed increased plasmid delivery and ectopic muIL-12p70 gene expression within the tumor microenvironment, indicating that the genomic deletions of *purI* and *msbB* not only diminish a potential genetic stability issue, but also confer an enhanced ability for payload delivery and increased potential for therapeutic efficacy.

### Effects of Clean Deletions of purI and msbB on Gene Expression in Tumors

| **Strain** | **Tumor Murine IL-12p70 Expression** | | | | | **Average Fold Increase** | **SD** |
|---|---|---|---|---|---|---|---|
| | **Tumor 1** | **Tumor 2** | **Tumor 3** | **Tumor 4** | **Tumor 5** | | |
| PBS | 1 | 1 | 1 | 1 | 1 | 1 | 0 |
| YS1646Δ*asd*/ΔFLG/Δ*pagP*/Δ*ansB*/Δ*csgD-*( ADN-480) | 6.69 | 2.51 | 2.93 | 7.59 | 6.25 | 5.19 | 2.31 |
| YS1646Δ*asd*/ΔFLG/Δ*pagP*/Δ*ansB*/Δ*csgD*/Δ*purI*_{(clean)}/Δ*msbB*_{(clean)} -(ADN-480) | 7.17 | 6.64 | 10.8 | 2.97 | 5.04 | 6.52 | 2.90 |

| | | | | | | | |
|---|---|---|---|---|---|---|---|
| SD = Standard Deviation | | | | | | | |

### Example 23

### Infection with Immunostimulatory Bacteria, and with Immunostimulatory Bacteria Transformed with Plasmids Encoding Immunomodulatory Proteins, Converts Human M2 Phenotype Macrophages into M1 Phenotype Macrophages

It was determined herein whether primary human M2 macrophages, infected with the YS1646Δ*asd*/ΔFLG/Δ*pagP*/Δ*ansB*/Δ*csgD* strain containing a plasmid encoding wild-type (WT) *asd*, or with the YS1646Δ*asd*/ΔFLG/Δ*pagP*/Δ*ansB*/Δ*csgD* strain containing a plasmid encoding immunostimulatory proteins (such as STING variants, IL-12, IL-15, and IL-21), could be converted to an M1 phenotype (or M1-like phenotype), and express pro-inflammatory surface receptors, such as CD80 and CCR7, and cytokines/chemokines, such as IFNγ and CXCL10 (see, *e.g.,* Gerrick et al. (2018) PLoS ONE 13(12):e0208602).

Frozen human monocytes, isolated from healthy human donors, were thawed in complete medium (RPMI-1640 + 10% FBS), and washed by centrifugation for 10 minutes at 600xg at room temperature. To generate primary human M2 macrophages, the monocytes were resuspended in ImmunoCult^{™}-SF Macrophage Medium (StemCell Technologies), containing 100 ng/mL human macrophage colony-stimulating factor (M-CSF) + 10 ng/mL human IL-4 + 10 ng/mL human IL-10. Monocytes (7e5 per well) were then seeded in a 24-well plate, with a final volume of 500 µL. Three days later (on day 3), 500 µL of ImmunoCult^{™}-SF Macrophage Medium containing 200 ng/mL human M-CSF + 20 ng/mL human IL-4 + 20 ng/mL human IL-10 was added per well, and the cells were incubated for 72 hours.

On day 6, triplicate wells were infected at an MOI of 20, for one hour in RPMI, with the following strains: YS1646Δ*asd*/ΔFLG/Δ*pagP*/Δ*ansB*/Δ*csgD*, containing a plasmid encoding a human (hu) STING variant with the GOF mutations N154S/R284G, and with the C-terminal tail (CTT) of huSTING replaced with the CTT of Tasmanian devil STING (huSTING N154S/R284G tazCTT; SEQ ID NO:397); YS1646Δ*asd*/ΔFLG/Δ*pagP*/Δ*ansB*/Δ*csgD*, containing a plasmid encoding wild-type (WT) huIL-12 and a huSTING N154S/R284G tazCTT variant; YS1646Δ*asd*/ΔFLG/Δ*pagP*/Δ*ansB*/Δ*csgD*, containing a plasmid encoding WT huIL-15; or YS1646Δ*asd*/ΔFLG/Δ*pagP*/Δ*ansB*/Δ*csgD*, containing a plasmid encoding WT huIL-21. The cells were then washed 3 times with PBS, and resuspended in RPMI + 100 µg/mL gentamicin (Sigma), to kill any extracellular bacteria (gentamicin cannot permeate into macrophages). As controls, human M1 macrophages, generated in 10 ng/mL LPS + 50 ng/mL IFNγ; human M2 macrophages treated with the STING agonist 3'5' RpRp c-di-AMP (InvivoGen), an analog of the clinical compound ADU-S100; human M2 macrophages re-polarized in 10 ng/mL LPS + 50 ng/mL IFNγ; and M2 macrophages infected with strain YS1646Δ*asd*/ΔFLG/Δ*pagP*/Δ*ansB*/Δ*csgD* not containing a plasmid; were included.

After 48 hours, supernatants were harvested and evaluated for downstream signaling differences using a custom human M2/M1 U-PLEX panel (Meso Scale Discovery), according to the manufacturer's protocol. Cytokine secretion was measured, and the average of the triplicate measurements was calculated. Fold increase in average cytokine secretion was calculated compared to untreated M2 macrophages, in which the average cytokine secretion was set as 1.00.

To measure the levels of expression of the proinflammatory surface receptors CD80 and CCR7, the cells were lysed with 200 µL RNA Lysis Buffer (Zymo Research), and RNA extraction was performed using the Zymo Research *Quick-*RNA^{™} 96 Kit, according to the manufacturer's protocol. Total RNA concentration was measured using a NanoDrop^{™} 2000 UV-Vis Spectrophotometer (Thermo Fisher Scientific). The purity of each sample also was assessed from the A₂₆₀/A₂₃₀ absorption ratio. Synthesis of cDNA was performed from 0.5-1 µg of template RNA using a CFX96^{™} Real-Time PCR Detection System (Bio-Rad) and iScript^{™} Reverse Transcription Supermix for RT-qPCR (Bio-Rad) in a 20 µL reaction, according to the manufacturer's instructions. qPCR was performed with a CFX96^{™} Real-Time PCR Detection System (Bio-Rad). The PrimePCR^{™} Probe Assay for human CD80 (Assay ID: qHsaCIP0026764) and human CCR7 (Assay ID: qHsaCIP0033364) were purchased from Bio-Rad. The qPCR reaction (20 µL) was conducted per protocol, using the iQ^{™} Multiplex Powermix (Bio-Rad). The standard thermocycling program on the Bio-Rad CFX96^{™} Real-Time PCR Detection System consisted of a 95 °C denaturation for 150 seconds, followed by 39 cycles of 95 °C for 15 seconds and 60 °C for 55 seconds. All samples were run in triplicate, and the mean C_{q} values were calculated. Quantification of the target mRNA was normalized using actin reference mRNA (Bio-Rad, Assay ID: qHsaCEP0036280). ΔC_{q} was calculated as the difference between the target and reference gene. ΔΔC_{q} was obtained by normalizing the ΔC_{q} values for the treatments to the ΔC_{q} values for the non-treatment control. Fold increase was calculated as 2^-ΔΔC_{q}. The values are shown in the table below, as the average of the triplicate wells.

As shown in the table below, compared to the untreated M2 macrophages control, in which the average cytokine secretion was set as 1.00, all strains of YS1646Δ*asd*/ΔFLG/Δ*pagP*/Δ*ansB*/Δ*csgD* induced high levels of secretion of IFNγ, CXCL10, and CXCL11. The YS1646Δ*asd*/ΔFLG/Δ*pagP*/Δ*ansB*/Δ*csgD* strains, transformed with plasmids encoding the huSTING N154S/R284G tazCTT variant; or WT huIL-12 and the huSTING N154S/R284G tazCTT variant; or WT huIL-15, induced higher levels of CXCL10 and CXCL11 secretion than the YS1646ΔFLG/Δ*pagP*/Δ*ansB*/Δ*csgD* strain not containing a plasmid. In particular, the YS1646Δ*asd*/ΔFLG/Δ*pagP*/Δ*ansB*/Δ*csgD* strain containing a plasmid encoding WT huIL-15 induced significantly higher levels of CXCL10 and CXCL11 than the STING agonist-treated M2 macrophages and the untreated M1 macrophages. In addition, the YS1646Δ*asd*/ΔFLG/Δ*pagP*/Δ*ansB*/Δ*csgD* strains, containing a plasmid encoding the huSTING N154S/R284G tazCTT variant; or WT huIL-12 and the huSTING N154S/R284G tazCTT variant; or WT huIL-15, induced higher levels of CD80 and CCR7 expression than the YS1646ΔFLG/Δ*pagP*/Δ*ansB*/Δ*csgD* strain not containing a plasmid. While the YS1646Δ*asd*/ΔFLG/Δ*pagP*/Δ*ansB*/Δ*csgD* strains containing plasmids encoding the huSTING N154S/R284G tazCTT variant; or WT huIL-12 and the huSTING N154S/R284G tazCTT variant; or WT huIL-15, induced higher levels of CXCL10, CXCL11, CD80 and CCR7, they induced less IFNγ than the YS1646ΔFLG/Δ*pagP*/Δ*ansB*/Δ*csgD* strain not containing a plasmid. The YS1646Δ*asd*/ΔFLG/Δ*pagP*/Δ*ansB*/Δ*csgD* strain, containing a plasmid encoding WT huIL-21, induced significantly higher levels of CD80 expression than the other YS1646Δ*asd*/ΔFLG/Δ*pagP*/Δ*ansB*/Δ*csgD* strains, but induced lower levels of IFNγ, CXCL10, CXCL11, and CCR7 expression.

These data demonstrate the ability of a strain, such as the strain designated as YS1646Δ*asd*/ΔFLG/Δ*pagP*/Δ*ansB*/Δ*csgD*, alone, or such strain containing plasmids encoding one or more immunostimulatory proteins, to convert a human primary, immunosuppressive M2 phenotype macrophage, into an M1 or M1-like phenotype macrophage, with immunosuppressive properties reduced or eliminated, and immune-stimulating, anti-tumor, or anti-viral properties enhanced or added.

### Expression of IFNy, CXCL10, CXCL11, CD80 and CCR7 in M2 Macrophages Infected with Immunostimulatory Bacteria

| **Treatments** | **Fold Cytokine/Chemokine Secretion Over Uninfected M2 Control** | | | **Fold Receptor Expression Over Uninfected M2 Control** | |
|---|---|---|---|---|---|
| | **IFNγ** | **CXCL10** | **CXCL11** | **CD80** | **CCR7** |
| M1 Macrophages, Untreated | 25131.7 | 106.6 | 89.5 | 335.0 | 2186.7 |
| M2 + STING Agonist | 5.2 | 106.9 | 199.3 | 54.0 | 47.8 |
| M2 + LPS + IFNγ | 25065.8 | 92.5 | 186.3 | 81.9 | 1002.3 |
| M2 + YS1646ΔFLG/Δ*pagP*/Δ*ansB*/Δ*csgD* | 732.2 | 46.6 | 45.1 | 0.8 | 0.7 |
| M2 + YS1646Δasd/ΔFLG/Δ*pagP*/Δ*ansB*/Δ*csgD* + STING variant | 657.1 | 93.9 | 65.7 | 6.3 | 2.9 |
| M2 + YS1646Δasd/ΔFLG/Δ*pagP*/Δ*ansB*/Δ*csgD* + STING variant + IL-12 | 398.4 | 111.2 | 84.0 | 2.1 | 79.0 |
| M2 + YS1646Δasd/ΔFLG/Δ*pagP*/Δ*ansB*/Δ*csgD* + IL-15 | 383.5 | 144.3 | 223.8 | 5.4 | 31.3 |
| M2 + YS1646Δasd/ΔFLG/Δ*pagP*/Δ*ansB*/Δ*csgD* + IL-21 | 176.7 | 15.8 | 14.5 | 55.9 | 0.3 |

| | | | | | |
|---|---|---|---|---|---|
| STING Agonist = 3'5' RpRp c-di-AMP; LPS = lipopolysaccharide; STING variant = huSTING N154S/R284G tazCTT | | | | | |

### Example 24

### IV-Delivered Strains of YS1646Δasd/ΔFLG/ΔpagP/ΔansB/ΔcsgD, Containing Plasmids Encoding IL-15, or Encoding the Combination of 4-1BBL and IL-12, Demonstrate Enhanced Efficacy and Induce Durable Anti-tumor Immunity in a Mouse Model of Colorectal Carcinoma

The anti-tumor efficacy of the YS1646Δ*asd*/ΔFLG/Δ*pagP*/Δ*ansB*/Δ*csgD* strain, containing plasmids encoding murine IL-15 (muIL-15Rα-IL-15sc); or murine IL-12p70 (muIL-12p70); or murine 4-1BBL containing a cytoplasmic domain deletion (mu4-1BBL(Δcyt)); or a combination of muIL-12p70 and mu4-1BBL(Δcyt), was assessed in the subcutaneous flank MC38 colorectal adenocarcinoma model. For this study, 6-8 week-old female C57BL/6 mice (8 mice per group) were inoculated SC in the right flank with MC38 cells (5x10⁵ cells in 100 µL PBS). Mice bearing established flank tumors were IV injected on day 7 with 2x10⁷ CFUs of strain YS1646Δ*asd*/ΔFLG/Δ*pagP*/Δ*ansB*/Δ*csgD-*muIL-15Rα-IL-15sc, or strain YS1646Δ*asd*/ΔFLG/Δ*pagP*/Δ*ansB*/Δ*csgD-*muIL-12p70, or strain YS1646Δ*asd*/ΔFLG/Δ*pagP*/Δ*ansB*/Δ*csgD-*mu-1BBL(Δcyt), or strain YS1646Δ*asd*/ΔFLG/Δ*pagP*/Δ*ansB*/Δ*csgD-*muIL-12p70 + 4-1BBL(Δcyt), or with PBS vehicle control. Tumor measurements and body weights were recorded twice weekly.

The results revealed that the YS1646Δ*asd*/ΔFLG/Δ*pagP*/Δ*ansB*/Δ*csgD-*muIL-15Rα-IL-15sc strain demonstrated significant tumor growth inhibition (TGI) compared to PBS (70.9% TGI, day 24), with a cure rate of 4 out of 8 mice (50%). The YS1646Δ*asd*/ΔELG|Δ*pag*P|Δ*ans*B/Δ*csg*D-muIL-12p70 strain demonstrated significant TGI compared to PBS (81.1% TGI, day 24), with a cure rate of 2 out of 8 mice (25%). The YS1646Δ*asd*lΔFLG/Δ*pag*P/Δ*ansB*/Δ*csg*D-mu4-1BBL(ΔCyt) strain also demonstrated a high degree of TGI compared to PBS (81.0% TGI, day 24), and also resulted in a cure rate of 2 out of 8 mice (25%). The strain expressing the combination of muIL-12p70 and mu4-1BBL(Δcyt) demonstrated the highest TGI (90.6% TGI, day 24), with 4 out of 8 mice achieving complete cures (50% cure rate). Thus, the immunostimulatory bacterial strains expressing muIL-15Rα-IL-15sc, or the combination of 4-1BBL(Δcyt) and IL-12p70, more potently inhibit tumor growth inhibition than the strains expressing 4-1BBL(Δcyt) or IL-12p70 alone, and result in a high complete response rate (50% cure rate) in a model of colorectal carcinoma.

All cured mice continued to remain tumor-free, and on day 66 post-tumor implantation, the mice were re-challenged on the opposite flank with MC38 cells (5x10⁵ cells in 100 µL PBS), and compared to naive (no previous tumor implantation), age-matched mice. Compared to the naïve mice, which had reached an average tumor size of 1384.7 mm³ by day 26 post-tumor implantation, each of the previously cured (and re-challenged) mice remained tumor free. These data demonstrate the ability of the mice treated with any of strains YS1646Δ*asd*/ΔFLG/Δ*pag*P/Δ*ans*B/Δ*csg*D-muIL-15Rα-IL-15sc, YS1646Δ*asd*/ΔFLG/Δ*pagP*/Δ*ansB*/Δ*csgD*-muIL-12p70, YS1646Δ*asd*/ΔFLG/Δ*pag*P/Δ*ans*B/Δ*csg*D-mu4-1BBL(Δcyt), or YS1646Δ*asd*/ΔFLG/Δ*pagP*/Δ*ansB*/Δ*csgD*-muIL-12p70 + mu4-1BBL(Δcyt), to generate durable anti-tumor immunity that prevents tumor recurrence, and protects from tumor re-challenge.

### Example 25

### Deletion and/or Disruption of csgD, lppAB, pagP, and FLG, and their Combination, Results in Strains with Significantly Higher Viability in Human Serum, Compared to Strain YS1646

Strain YS1646 exhibits limited tumor colonization in humans after systemic administration. It is shown herein that strain YS1646 is inactivated by complement factors in human blood (see, Example 5). In this Example, strains YS1646 and *E*. *coli* D10B were compared to exemplary immunostimulatory bacteria provided herein, that contain additional mutations that alter the surface of the bacteria. These exemplary modified strains were YS1646Δ*asd*/ΔFLG/Δ*pagP*, YS1646Δ*asd*/ΔFLG/Δ*pagP*/Δ*ansB*/Δ*csgD*, and YS1646Δ*asd*/ΔFLG/Δ*pagP*/Δ*ansB*/Δ*csgD*/Δ*lppAB*, each containing the plasmid ADN-256, which encodes a secreted NanoLuciferase^{®} (secNanoLuc^{®}), under the control of a CMV promoter. These three strains, in addition to YS1646 and *E. coli* D10B cultures, were incubated in technical triplicate with serum, or heat-inactivated (HI) serum, from healthy human donors (n=3), for 3 hours at 37 °C. After incubation with serum, bacteria were serially diluted and plated on LB agar plates, and the colony forming units (CFUs) were determined. % survival was determined by calculating the CFUs present in whole serum, versus the CFUs present in heat-inactivated serum.

The results showed that all strains were 100% viable in the heat-inactivated human serum. The *E*. *coli* D10B strain was completely eliminated after 3 hours in whole human serum, while strain YS1646 exhibited an average of only 12.22% survival, demonstrating that tumor colonization of the YS1646 clinical strain was limited due to complement inactivation in human blood. Strain YS1646Δ*asd*/ΔFLG/Δ*pagP* demonstrated an average of 74.56% survival after incubation with human serum for 3 hours, while strains YS1646Δ*asd*/ΔFLG/Δ*pagP*/Δ*ansB*/Δ*csgD* and YS1646Δ*asd*/ΔFLG/Δ*pagP*/Δ*ansB*/Δ*csgD*/Δ*lppAB* displayed average % survivals of 129.56% and 158.33%, respectively. The YS1646Δ*asd*/ΔFLG/Δ*pagP*/Δ*ansB*/Δ*csgD* and YS1646Δ*asd*/ΔFLG/Δ*pagP*/Δ*ansB*/Δ*csgD*/Δ*lppAB* strains demonstrated survival greater than 100%, indicating that they are completely resistant to complement inactivation in human serum.

These data explain why strain YS1646 (VNP20009) has very low tumor colonization when systemically administered. It is shown herein that strain YS1646 is highly sensitive to complement inactivation in human serum. The *fljB*/*fliC* (FLG), *pagP, csgD,* and *lppAB* deletions/disruptions, or the combination of these mutations, partially or completely rescues this phenotype. Thus, the enhanced stability observed in human serum with the YS1646Δ*asd*/ΔFLG/Δ*pagP*, YS1646Δ*asd*/ΔFLG/*ΔpagP* /*ΔansB*/*ΔcsgD,* and YS1646Δ*asd*/ΔFLG/Δ*pagP*/Δ*ansB*/Δ*csgD*/Δ*lppAB* strains provides for increased human tumor colonization.

### Example 26

### Combinations of Therapeutic Products Encoded on the Plasmids, Including Cytokines, STING Variants, Anti-CTLA-4 Antibodies, 4-1BBL, and Anti-4-1BBL Agonistic Antibodies, Potently Induce the Secretion of CXCL10 from Myeloid Cells and Induce the Activation of T-Cells

The impact of the expression of the immunomodulatory payloads and their combinations, encoded on the plasmids in the immunostimulatory bacteria, on the activation and function of T-cells and myeloid cells was determined. The secretion of cytokines and chemokines, such as IFN-γ and CXCL10, by myeloid cells and T-cells, in response to the expression of various immunomodulatory payloads, was measured as an indicator of protective anti-tumor immunity. The secretion of the pro-tumor, inflammatory cytokine IL-6, also was monitored in parallel.

This Example describes and demonstrates the impact of the delivery of various immunomodulatory payload combinations by the immunostimulatory bacteria herein on the activation of antigen-specific T-cells, and on the secretion of CXCL10, a key chemokine involved in the recruitment of anti-tumor T-cells, by myeloid cells. This was assessed by transfecting mouse dendritic cells with plasmids encoding various combinations of payloads, co-culturing the transfected dendritic cells with autologous mouse T-cells, and identifying and quantifying the cytokines secreted by each cell type.

The plasmids encoding the immunomodulatory payloads/proteins included those encoding single payloads, as well as those encoding combinations of payloads. For example, as shown in the table below, single payloads included secreted NanoLuc^{®}; murine IL-12p70 (muIL-12p70); STING R284G tazCTT (a chimeric protein containing human STING with the mutation R284G and a replacement of the C-terminal tail (CTT) of human STING with the CTT of Tasmanian devil STING); a murine anti-CTLA-4 scFv (clone 9D9); murine 4-1BBL with a deleted cytoplasmic domain (mu4-1BBL(Δcyt)); murine IL-18 (muIL-18); and murine IL-21 (muIL-21).

Combinations of payloads, including two or three payloads, were expressed on a single plasmid using T2A and/or P2A peptides, such that multiple proteins were encoded under the control of the same promoter. The combinations of payloads included, for example: 1) murine IL-18 and murine IL-12p70; 2) murine IL-21 and murine IL-12p70; 3) murine IL-12p70 and STING R284G tazCTT; 4) murine IL-12, an anti-murine CTLA-4 scFv, and STING R284G tazCTT; 5) an anti-murine CTLA-4 scFv, murine IL-12p70, and STING R284G tazCTT; 6) an anti-murine CTLA-4 scFv and murine IL-12p70; 7) an anti-murine CTLA-4 scFv, and STING R284G tazCTT; 8) murine IL-21, murine IL-12p70, and STING R284G tazCTT; 9) murine 4-1BBL(Δcyt) and STING R284G tazCTT; 10) murine 4-1BBL(ΔCyt) and murine IL-12p70; and 11) murine 4-1BBL(ΔCyt), murine IL-12p70, and STING R284G tazCTT.

Bone marrow-derived dendritic cells (BMDCs) were generated from Goldenticket mice, which are STING deficient, and were transfected with plasmids encoding various combinations of the investigated payloads. Twenty-four hours post-transfection, supernatants were harvested and the levels of secreted CXCL10 were measured in BMDC culture supernatants, using a U-Plex assay platform from Meso Scale Discovery, according to the manufacturer's protocol.

To measure CD8⁺ T-cell activation, transfected BMDCs were pulsed with chicken ovalbumin (OVA) SIINFEKL (OVA₂₅₇₋₂₆₄) peptide, a major histocompatibility complex (MHC) class I (H-2Kb)-restricted peptide epitope recognized by CD8⁺ T-cells. Splenic T-cells, isolated from Rag1^{-/-} OT-I mice, which express T-cell receptors (TCRs) that are specific for SIINFEKL presented by the MHC class I molecule H-2Kb, were added to the BMDCs for co-culture. After 72 hours of BMDC/T-cell co-culture, supernatants were harvested, and the levels of secreted IFN-γ were measured in the cell culture supernatants, using a cytometric bead array (CBA) kit.

The results are summarized in the table below, which shows the levels of CXCL10 secreted by BMDCs in response to transfection with plasmids encoding the various single and combination payloads, as well as the levels of IFN-γ secreted by CD8⁺ T-cells, following co-culture with the transfected BMDCs.

The results show the specific combinations of encoded payloads that induce high levels of secretion of CXCL10 by BMDCs, and show that a synergy is observed when combining payloads. For example, synergy was observed between muIL-12p70 and STING R284G tazCTT expression. The combinations of mu4-1BBL(Δcyt) or an anti-murine CTLA-4 antibody fragment, with STING R284G tazCTT expression, also results in higher levels of secretion of CXCL10 by BMDCs. The results also show that the expression of muIL-12p70, as well as several of the combinations of payloads, such as muIL-18 and muIL-12p70; muIL-21 and muIL-12p70; muIL-12p70 and STING R284G tazCTT; muIL-12p70, the anti-murine CTLA-4 scFV, and STING R284G tazCTT; the anti-murine CTLA-4 scFV and muIL-12p70; and mu4-1BBL(Δcyt) and muIL-12p70, by BMDCs, induces the activation of CD8⁺ T-cell responses, and the secretion of IFN-γ.

### Effects of Delivery of Single and Combination Payloads on Secretion of CXCL10 by BMDCs and on Activation of CD8⁺ T-cells

| **BMDC Treatments (Transfections)** | **CXCL10 Secreted by BDMCs (pg/ml)** | | **IFN-γ Secreted by CD8⁺ T-Cells (pg/ml)** | |
|---|---|---|---|---|
| | **Mean** | **SEM** | **Mean** | **SEM** |
| Untransfected BMDCs (control) | 70 | 14 | 1195 | 9 |
| Secreted NanoLuc^{®} | 142 | 36 | 2003 | 157 |
| muIL-12p70 | 155 | 27 | 15149 | 987 |
| STING R284G tazCTT | 253 | 49 | 1686 | 52 |
| Anti-murine CTLA-4 scFv | 80 | 3 | 1873 | 27 |
| mu4-1BBL(Δcyt) | 236 | 66 | 1450 | 330 |
| muIL-18 | 69 | 20 | 1891 | 149 |
| muIL-21 | 84 | 6 | 2028 | 188 |
| muIL-18_T2A_muIL-12p70 | 95 | 7 | 6730 | 879 |
| muIL-21_T2A_muIL-12p70 | 143 | 19 | 7944 | 342 |
| muIL-12p70_T2A_STING R284G tazCTT | 1843 | 248 | 11669 | 1399 |
| muIL-12p70_T2A_anti-murine CTLA-4 scFv_P2A_STING R284G tazCTT | 1508 | 34 | 11652 | 1351 |
| anti-murine CTLA-4 scFv_T2A_muIL-12p70_P2A_STING R284G tazCTT | 157 | 42 | 2675 | 326 |
| anti-murine CTLA-4 scFv_T2A_muIL-I2p70 | 128 | 45 | 6696 | 888 |
| anti-murine CTLA-4 scFv_T2A_STING R284G tazCTT | 604 | 100 | 1891 | 96 |
| muIL-21_T2A_muIL-12p70_P2A_STING R284G tazCTT | 164 | 23 | 2209 | 305 |
| mu4-1BBL(Δcyt)_T2A_STING R284G tazCTT | 469 | 38 | 1648 | 271 |
| mu4-1BBL(Δcyt)_T2A_muIL-12p70 | 98 | 12 | 6105 | 482 |
| mu4-1BBL(Δcyt)_T2A_muIL-12p70_P2A_STING R284G tazCTT | 213 | 64 | 2218 | 936 |

| | | | | |
|---|---|---|---|---|
| SEM = Standard Error of the Mean | | | | |

The same biological responses were investigated when combining the payloads listed above with one or more of murine (mu) IL-36γ, muIL-23, muOX40L, and muIFN-α2. BMDCs were isolated from STING-deficient Goldenticket mice, and were transfected with plasmids encoding various combinations of payloads, as shown in the table below. Twenty-four hours post-transfection, the levels of secreted CXCL10, IFN-γ, and IL-6 were measured in BMDC culture supernatants using a cytometric bead array (CBA) kit. As described above, BMDCs were pulsed with ovalbumin SIINFEKL antigenic peptides, before the addition of splenic T-cells from Rag1^{-/-} OT-I mice (OT-I cells). After 30 hours of BMDC/T-cell co-culture, the levels of secreted IFN-γ were measured in the cell culture supernatants using a CBA kit. T-cells were stained with an APC-conjugated anti-murine 4-1BB antibody (clone 17B5, BioLegend), to monitor the expression of the activation marker 4-1BB.

The results, which are summarized in the table below, show that specific combinations of payloads, such as muIL-36γ + muIL-12p70 + STING R284G TazCTT, and muIL-36γ + muIL-23 + STING R284G TazCTT, induce high levels of secretion of CXCL10 by BMDCs. The combination of muIL-36γ with muIL-12p70 and STING R284G tazCTT also induces high levels of secretion of IFN-γ by BMDCs, but relatively low levels of IL-6 secretion, representing a therapeutically useful combination for the induction of anti-tumor immunity.

The results also show that many of the combinations listed in the table below induce the activation of CD8⁺ T-cell responses, as assessed by 4-1BB expression and secretion of IFN-γ. For example, the combination of muIL-36γ + muIL-12p70 + STING R284G TazCTT induced the highest levels of IFN-γ secretion by the CD8⁺ T-cells, and induced a high level of 4-1BB expression, indicating efficient activation and functionality of CD8⁺ T-cells, which is critical for the generation of proper anti-tumor immunity.

### Cytokine Secretion by BMDCs and CD8⁺ T-Cell Activation Following Transfection of BMDCs with Plasmids Encoding Combinations of Immunomodulatory Payloads

| **BMDC Treatments (Transfection)** | **Cytokine Secretion by BMDCs (pg/ml)** | | | **IFN-γ Secretion by CD8⁺ T Cells (pg/ml)** | | **% Expression** |
|---|---|---|---|---|---|---|
| | **CXCL10** | **IFN-γ** | **IL-6** | **IFN-γ** | **SEM** | **4-1BB** |
| Untransfected BMDCs (control) | 26 | 0 | 6 | 1616 | 46 | 12 |
| Secreted NanoLuc^{®} | 99 | 0 | 16 | 1606 | 48 | 16 |
| STING R284G tazCTT | 576 | 0 | 22 | 1920 | 49 | 17 |
| muIL-12p70 | 181 | 14 | 21 | 7077 | 932 | 30 |
| mu4-1BBL(Δcyt) | 181 | 0 | 11 | 1628 | 53 | 26 |
| muIFN-α2 | 347 | 0 | 47 | 1841 | 350 | 23 |
| muIL-36γ | 216 | 0 | 18 | 1532 | 8 | 26 |
| muIL-23 | 143 | 1 | 15 | 1352 | 83 | 29 |
| muOX40L | 125 | 0 | 18 | 1827 | 140 | 29 |
| muIL-36γ_T2A_muIL-12p70 | 131 | 14 | 18 | 3550 | 587 | 37 |
| muIL-36γ_T2A_muIL-12p70_P2A STING R284G tazCTT | 2788 | 49 | 33 | 4286 | 51 | 33 |
| muIL-36γ_T2A_muIL-23 | 848 | 0 | 85 | 1880 | 56 | 28 |
| muIL-36γ_T2A_STING R284G tazCTT | 637 | 0 | 266 | 1341 | 37 | 33 |
| muIL-36γ_T2A muIL-23 P2A STING R284G tazCTT | 1270 | 1 | 49 | 1375 | 28 | 30 |
| muIL-36γ_T2A_muIL-23 P2A_muOX40L | 101 | 0 | 39 | 1554 | 25 | 34 |
| muIL-36γ_T2A_muIL-12p70_P2A_muOX40L | 240 | 11 | 39 | 3939 | 268 | 35 |
| muIFN-α2_T2A_muIL-36γ | 339 | 0 | 31 | 1409 | 142 | 28 |
| muIL-36γ_T2A_muIFN-α2 | 208 | 0 | 26 | 1361 | 84 | 31 |
| mu4-1BBL(Δcyt)_T2A_muIL-36γ | 177 | 0 | 12 | 1492 | 13 | 15 |

| | | | | | | |
|---|---|---|---|---|---|---|
| SEM = Standard Error of the Mean | | | | | | |

The effects on T-cell function of various combinations of payloads and receptor-ligand interactions also were investigated by treating murine pooled CD4⁺ and CD8⁺ T-cells with recombinant cytokines and agonistic antibodies. Splenic T-cells (CD4⁺ and CD8⁺) from BALB/c mice were negatively isolated, and then treated with 2.5 nM of one or more recombinant cytokines (*e*.*g*., muIL-12p70; muIL-15 complex (muIL-15Rα-IL-15sc); muIL-21; muIL-36γ; muIFN-α2; and combinations thereof), and/or 1.5 µg/ml of an anti-murine 4-1BB agonistic antibody (Clone 17B5, BioLegend). After 24 hours of treatment, the levels of secreted IFN-γ and IL-6 were measured in the T-cell culture supernatants using a CBA kit.

The results, which are shown in the table below, demonstrate that several combinations of cytokines, with or without the addition of an anti-murine 4-1BB agonist antibody, activate T-cells. For example, the combinations of muIL-12p70 + muIL-15 complex (muIL-15Rα-IL-15sc); muIL-12p70 + muIL-15 complex + muIFN-α2; muIL-12p70 + muIL-15 complex + anti-murine 4-1BB agonistic antibody; muIL-12p70 + muIL-15 complex + muIL-36γ; muIL-12p70 + muIL-15 complex + muIL-21; muIL-12p70 + muIL-21 + muIL-36γ; muIL-12p70 + muIL-36γ + muIFN-α2; muIL-12p70 + muIL-36γ + anti-murine 4-1BB agonistic antibody; muIL-15 complex + muIL-36γ + muIFN-α2; and muIL-15 complex + muIL-36γ + anti-murine 4-1BB agonistic antibody, result in the secretion of high levels of IFN-γ, but relatively low levels of IL-6, from T-cells, making them ideal combinations for optimal T-cell activation, for the induction of anti-tumor immunity in the tumor microenvironment.

### Effects of Treatment with Various Cytokines and/or Anti-Murine 4-1BB Agonist Antibody and/or Combinations Thereof on the Activation of T-Cells

| **Treatments** | **Secretion by Murine CD4⁺ and CD8⁺ T-Cells** | | | |
|---|---|---|---|---|
| | **IFN-γ (pg/ml)** | | **IL-6 (pg/ml)** | |
| | **Mean** | **SEM** | **Mean** | **SEM** |
| No cytokine (untreated control) | 1 | 0 | 125 | 8 |
| muIL-12p70 | 83 | 8 | 151 | 2 |
| muIL-15 complex (muIL-15Rα-IL-15sc) | 21 | 2 | 187 | 22 |
| muIL-21 | 1 | 0 | 135 | 20 |
| muIL-36γ | 5 | 3 | 496 | 23 |
| muIFN-α2 | 3 | 1 | 129 | 19 |
| Anti-murine 4-1BB agonistic antibody | 1 | 0 | 139 | 15 |
| muIL-12p70 + muIL-15 complex | 1808 | 93 | 120 | 23 |
| muIL-12p70 + muIL-21 | 122 | 33 | 140 | 23 |
| muIL-12p70 + anti-murine 4-1BB agonistic antibody | 100 | 9 | 160 | 1 |
| muIL-12p70 + muIL-36γ | 6655 | 115 | 467 | 61 |
| muIL-12p70 + muIFN-α2 | 84 | 2 | 140 | 10 |
| muIL-15 complex + muIFN-α2 | 97 | 12 | 57 | 1 |
| muIL-15 complex + mulL-21 | 23 | 1 | 108 | 1 |
| muIL-15 complex + muIL-36γ | 2790 | 262 | 377 | 29 |
| muIL-15 complex + anti-murine 4-1BB agonistic antibody | 40 | 5 | 134 | 23 |
| muIL-21 + muIFN-α2 | 2 | 0 | 77 | 15 |
| muIL-21 + muIL-36γ | 62 | 13 | 304 | 31 |
| muIL-21 + anti-murine 4-1BB agonistic antibody | 10 | 5 | 85 | 4 |
| muIL-3 6γ + muIFN-α2 | 55 | 8 | 234 | 14 |
| muIL-36γ + anti-murine 4-1BB agonistic antibody | 18 | 1 | 393 | 69 |
| muIFN-α2 + anti-murine 4-1BB agonistic antibody | 1 | 0 | 58 | 1 |
| muIL-12p70 + muIL-15 complex + muIFN-α2 | 1655 | 542 | 64 | 5 |
| muIL-12p70 + mulL-15 complex + anti-murine 4-1BB agonistic antibody | 3703 | 1104 | 127 | 22 |
| muIL-12p70 + muIL-15 complex + muIL-36γ | 24293 | 3266 | 260 | 13 |
| muIL-12p70 + muIL-15 complex + muIL-21 | 1496 | 83 | 74 | 4 |
| muIL-12p70 + muIL-21 + muIFN-α2 | 61 | 7 | 56 | 13 |
| muIL-12p70 + muIL-21 + anti-murine 4-1BB agonistic antibody | 91 | 10 | 84 | 8 |
| muIL-12p70 + muIL-21 + muIL-3 6γ | 6587 | 538 | 243 | 12 |
| muIL-12p70 + muIL-36γ + muIFN-α2 | 5559 | 470 | 243 | 7 |
| muIL-12p70 + muIL-36γ + anti-murine 4-1BB agonistic antibody | 10191 | 2139 | 191 | 9 |
| muIL-12p70 + muIFN-α2 + anti-murine 4-1BB agonistic antibody | 99 | 3 | 48 | 0 |
| muIL-15 complex + muIL-21 + muIFN-α2 | 70 | 3 | 44 | 5 |
| muIL-15 complex + muIL-36γ + muIFN-α2 | 1539 | 24 | 145 | 11 |
| muIL-15 complex + muIL-36γ + anti-murine 4-1BB agonistic antibody | 2764 | 672 | 261 | 2 |
| muIL-15 complex + muIFN-α2 + anti-murine 4-1BB agonistic antibody | 104 | 3 | 49 | 15 |
| muIL-15 complex + muIL-21 + anti-murine 4-1BB agonistic antibody | 37 | 3 | 50 | 1 |
| muIL-21 + muIL-36γ + muIFN-α2 | 62 | 7 | 111 | 3 |
| muIL-21 + muIL-36γ + anti-murine 4-1BB agonistic antibody | 25 | 8 | 175 | 11 |
| muIL-36γ + muIFN-α2 + anti-murine 4-1BB agonistic antibody | 47 | 7 | 158 | 21 |

| | | | | |
|---|---|---|---|---|
| SEM = Standard Error of the Mean; IL-15 complex = muIL-15Rα-IL-15sc | | | | |

The effects of treatment with one or more recombinant human cytokines and/or an anti-human 41BB antibody (clone 4B4-1, BioLegend), on the activation of human T-cells, was assessed. Human CD4⁺ and CD8⁺ T-cells were negatively isolated from human peripheral blood mononuclear cells (PBMCs) and treated with 1 nM of recombinant cytokines, with or without T-cell receptor (TCR) and 4-1BB stimulation, using coated agonistic anti-human CD3ε (clone OKT3, BioLegend) and anti-human 4-1BB (clone 4B4-1, BioLegend) antibodies, respectively. After 24 hours and 72 hours of treatment, the supernatants were harvested, and the levels of secreted IFN-γ were measured in the cell culture supernatants using a CBA kit. T-cells also were stained with a phycoerythrin (PE)-conjugated anti-human CD25 antibody (clone BC96, BioLegend), to monitor the expression of the activation marker CD25.

The results, which are summarized in the two tables below, show that several combinations of cytokines (IL-12p70, IL-15, IL-21, and IL-36y) and 4-1BB engagement, activate T-cells to secrete high levels of IFN-γ, with and without TCR stimulation by an anti-CD3ε agonistic antibody, for CD4⁺ and CD8⁺ T-cells. CD4⁺ and CD8⁺ T-cells expressed the activation marker CD25, in response to treatment with various combinations of human cytokines and/or an anti-human 4-1BB agonistic antibody, particularly after stimulation with an anti-CD3ε agonistic antibody. The expression of CD25 was much more pronounced in CD8⁺ T-cells, with a percentage of cells positive for CD25 of above 90% for all treated groups (which is why the Mean fluorescence intensity (MFI) is provided below as a measure of the CD25 expression with CD3ε stimulation in CD8⁺ T-cells). This is because CD25 is a well-established activation marker for CD8⁺ T-cells (more so than for CD4⁺ T-cells), and CD8⁺ T-cells are more reactive to the stimuli for that activation marker.

### Effects of Treatment with Various Human Cytokines and/or an Anti-Human 4-1BB Agonist Antibody and/or Combinations Thereof on the Secretion of IFN-γ by T-Cells

| **Treatments** | **24 Hours, Unstimulated** | | **24 Hours, with CD3ε Stimulation** | | **72 Hours, Unstimulated** | | **72 Hours, with CD3ε Stimulation** | |
|---|---|---|---|---|---|---|---|---|
| | **Mean Secreted IFN-γ (pg/ml)** | **SEM** | **Mean Secreted IFN-γ (pg/ml)** | **SEM** | **Mean Secreted IFN-γ (pg/ml)** | **SEM** | **Mean Secreted IFN-γ (pg/ml)** | **SEM** |
| No cytokine (untreated control) | 11 | 0 | 17335 | 7241 | 10 | 2 | 2123 | 852 |
| IL-12p70 | 10916 | 194 | 98371 | 2829 | 14060 | 1786 | 25143 | 89 |
| IL-15 | 1596 | 514 | 40517 | 104 | 1823 | 1027 | 9373 | 143 |
| IL-21 | 154 | 47 | 17968 | 437 | 79 | 14 | 1393 | 82 |
| IL-36γ | 30 | 19 | 12217 | 2075 | 12 | 0 | 2104 | 420 |
| Anti-4-1BB agonistic antibody | 19 | 10 | 19362 | 3461 | 14 | 5 | 3330 | 788 |
| IL-12p70 + IL-15 | 75903 | 9641 | 131979 | 613 | 165517 | 7174 | 48166 | 1724 |
| IL-12p70 + IL-21 | 19165 | 2843 | 95734 | 3754 | 19830 | 4954 | 28214 | 758 |
| IL-12p70 + IL-36γ | 20079 | 6046 | 91096 | 610 | 29878 | 10848 | 26385 | 2795 |
| IL-12p70 + anti-4-1BB agonistic antibody | 15052 | 1557 | 99653 | 1322 | 30903 | 2424 | 29248 | 260 |
| IL-15 + IL-21 | 5394 | 817 | 29765 | 7463 | 5312 | 241 | 5897 | 2085 |
| IL-15 + IL-36γ | 5788 | 1281 | 40881 | 238 | 5954 | 721 | 10295 | 146 |
| IL-15 + anti-4-1BB agonistic antibody | 1708 | 259 | 37833 | 5210 | 5208 | 3977 | 9015 | 1679 |
| IL-21 + IL-36γ | 638 | 176 | 22612 | 2 | 356 | 72 | 1776 | 157 |
| IL-21 + anti-4-1BB agonistic antibody | 128 | 64 | 19102 | 76 | 95 | 33 | 1348 | 196 |
| IL-36γ + anti-4-1BB agonistic antibody | 10 | 1 | 12714 | 1165 | 8 | 0 | 2116 | 356 |
| IL-12p70 + IL-15 + IL-21 | 64847 | 1957 | 124755 | 6662 | 126040 | 7660 | 49433 | 2130 |
| IL-12p70 + IL-15 + IL-36γ | 70049 | 3270 | 122175 | 1139 | 158389 | 3106 | 49668 | 21 |
| IL-12p70 + IL-15 + anti-4-1BB agonistic antibody | 99206 | 2970 | 132011 | 3779 | 208361 | 6574 | 45361 | 817 |
| IL-12p70 + IL-21 + IL36γ | 14321 | 237 | 88340 | 2001 | 18015 | 2207 | 27797 | 2142 |
| IL-12p70 + IL-21 + anti-4-1BB agonistic antibody | 4394 | 395 | 95440 | 2439 | 4816 | 541 | 28580 | 17 |
| IL-12p70 + IL-36y + anti-4-1BB agonistic antibody | 8012 | 2959 | 98147 | 2938 | 11193 | 4687 | 31059 | 2344 |
| IL-15 + IL-21 + IL-36γ | 5401 | 2501 | 41892 | 2614 | 4797 | 2145 | 7411 | 812 |
| IL-15 + IL-21 + anti-4-1BB agonistic antibody | 6398 | 2097 | 47003 | 5628 | 5522 | 2046 | 8950 | 999 |
| IL-15 + IL-36γ + anti-4-1BB agonistic antibody | 1337 | 1 | 39746 | 4726 | 1767 | 381 | 7416 | 600 |
| IL-21 + IL-36γ + anti-4-1BB agonistic antibody | 228 | 42 | 21012 | 2668 | 142 | 22 | 1485 | 153 |

| | | | | | | | | |
|---|---|---|---|---|---|---|---|---|
| SEM = Standard Error of the Mean | | | | | | | | |

### Expression of CD25 on CD4⁺ and CD8⁺ T-Cells Following Treatment with Various Combinations of Human Cytokines and/or an Anti-Human 4-1BB Agonistic Antibody

| **Treatments** | **CD4⁺ T-Cells** | | **CD8⁺ T-Cells** | |
|---|---|---|---|---|
| | **% CD25 Expression, Unstimulated** | **% CD25 Expression, with CD3ε Stimulation** | **% CD25 Expression, Unstimulated** | **CD25 Expression, with CD3ε Stimulation (MFI*)** |
| No cytokine (untreated control) | 2 | 37 | 1 | 8356 |
| IL-12p70 | 2 | 43 | 2 | 17025 |
| IL-15 | 13 | 78 | 16 | 21442 |
| IL-21 | 2 | 41 | 2 | 8842 |
| IL-36γ | 3 | 37 | 1 | 7641 |
| Anti-4-1BB agonistic antibody | 2 | 37 | 1 | 9331 |
| IL-12p70 + IL-15 | 18 | 83 | 25 | 24511 |
| IL-12p70 + IL-21 | 3 | 53 | 3 | 17009 |
| IL-12p70 + IL-36γ | 2 | 49 | 2 | 15348 |
| IL-12p70 + anti-4-1BB agonistic antibody | 2 | 43 | 2 | 15420 |
| IL-15 + IL-21 | 14 | 83 | 22 | 22054 |
| IL-15 + IL-36γ | 14 | 75 | 23 | 20800 |
| IL-15 + anti-4-1BB agonistic antibody | 13 | 76 | 19 | 20008 |
| IL-21 + IL-36γ | 3 | 43 | 2 | 8619 |
| IL-21 + anti-4-1BB agonistic antibody | 2 | 41 | 2 | 8284 |
| IL-36γ + anti-4-1BB agonistic antibody | 2 | 40 | 1 | 8551 |
| IL-12p70 + IL-15 + IL-21 | 18 | 87 | 25 | 23757 |
| IL-12p70 + IL-15 + IL-36γ | 18 | 85 | 26 | 22447 |
| IL-12p70 + IL-15 + anti-4-1BB agonistic antibody | 19 | 83 | 29 | 22262 |
| IL-12p70 + IL-21 + IL-36γ | 3 | 54 | 2 | 15683 |
| IL-12p70 + IL-21 + anti-4-1BB agonistic antibody | 2 | 54 | 2 | 16589 |
| IL-12p70 + IL-36γ + anti-4-1BB agonistic antibody | 2 | 49 | 1 | 15660 |
| IL-15 + IL-21 + IL-36γ | 14 | 86 | 24 | 21383 |
| IL-15 + IL-21 + anti-4-1BB agonistic antibody | 14 | 81 | 23 | 22358 |
| IL-15 + IL-36γ + anti-4-1BB agonistic antibody | 12 | 73 | 16 | 19611 |
| IL-21 + IL-36γ + anti-4-1BB agonistic antibody | 2 | 42 | 1 | 7297 |

| | | | | |
|---|---|---|---|---|
| SEM = Standard Error of the Mean *MFI values provided because the % of CD25 expression with CD3ε stimulation was above 90% for all groups. | | | | |

The ability of the immunomodulatory payloads to increase the activation of human antigen-experienced CD8⁺ T-cells was assessed. Human monocyte-derived dendritic cells (ModDCs) were generated and transfected with plasmids encoding secreted NanoLuc^{®}; human STING with the replacements N154S/R284G (STING N154S/R184G; SEQ ID NO:398); human STING with the replacements N154S/R284G and with a replacement of the C-terminal tail (CTT) of human STING with the CTT of Tasmanian devil STING (STING N154S/R284G tazCTT; SEQ ID NO:397); or IL-12p70. Five hours post-transfection, the human dendritic cells were pulsed with HIV-1 (negative control), CEF, or CMV MHC-I restricted peptides, to stimulate antigen-specific CD8⁺ T-cells with a broad array of viral peptides. Human CD8⁺ T-cells were isolated from the same donor and were co-cultured with the pulsed dendritic cells for 48 hours. After 48 hours of co-culture, the supernatants were harvested, and the levels of secreted IFN-γ were measured in the cell culture supernatants using a CBA kit (BioLegend).

The results, summarized in the table below, demonstrate the potent effect of IL-12p70, as well as the STING variants with the N154S and R284G GOF mutations, with or without replacement of the human STING CTT with the Tasmanian devil STING CTT, in increasing the antigen-specific activation of human CD8⁺ T-cells, measured in terms of IFN-γ secretion from the T-cells.

### Antigen-Specific Activation of Human CD8⁺ T-Cells Following Treatment with Immunomodulatory Proteins

| **Treatments** | **IFN-γ Secretion (pg/ml)** | | | | | |
|---|---|---|---|---|---|---|
| | **HIV Peptide Stimulation** | | **CEF Peptide Stimulation** | | **CMV Peptide Stimulation** | |
| | **Mean** | **SEM** | **Mean** | **SEM** | **Mean** | **SEM** |
| Untransfected control | 7 | 1 | 904 | 126 | 61 | 23 |
| Secreted NanoLuc^{®} | 352 | 7 | 3287 | 632 | 577 | 85 |
| STING N154S/R284G | 1122 | 95 | 7226 | 393 | 1579 | 201 |
| STING N154S/R284G tazCTT | 952 | 21 | 6178 | 640 | 1724 | 547 |
| IL-12p70 #1 | 841 | 10 | 5678 | 232 | 1404 | 231 |
| IL-12p70 #2 | 846 | 32 | 4815 | 110 | 1328 | 3 |

| | | | | | | |
|---|---|---|---|---|---|---|
| SEM = Standard Error of the Mean | | | | | | |

### Example 27

### Plasmid Transfer Following Immunostimulatory Bacterial Cell Death Enables Durable Protein Production in Human Primary M2 Macrophages

The relative efficiencies of transfection (*i.e.,* direct transfer of plasmid DNA) vs. bactofection (*i.e*., transfer of plasmid DNA by infection with the immunostimulatory bacterial strains herein), in primary human M2 macrophages, for expression of a reporter gene, were compared. The transfer of plasmids encoding gene expression cassettes from strain YS1646Δ*asd*/ΔFLG/Δ*pagP*/Δ*ansB*/Δ*csgD*, upon bacterial infection, to immunosuppressive phagocytic cells (*i.e.,* bactofection), was assessed. Human M2 macrophages were generated from healthy human donors, and then infected with a strain of YS 1646Δ*asd*/ΔFLG/Δ*pagP*/Δ*ansB*/Δ*csgD* encoding a NanoLuciferase^{®} (NanoLuc^{®}, Promega) reporter gene. Transfection (*i.e*., direct transfer of plasmid DNA) experiments also were performed as a control, and used to determine the efficiency of gene expression in comparison to bactofection (*i.e*., transfer of plasmid DNA by the immunostimulatory bacterial strains herein).

Human M2 macrophages were generated from negatively isolated human monocytes using ImmunoCult^{™}-SF Macrophage Medium (StemCell Technologies). Monocytes (5x10⁵ cells per well) were seeded in a 24-well plate, with a final volume of 500 µL containing 100 ng/ml of human macrophage colony-stimulating factor (M-CSF). Three days later, 500 µL of ImmunoCult^{™}-SF Macrophage Medium, containing 200 ng/mL of human M-CSF + 20 ng/mL of human IL-4 + 20 ng/mL of human IL-10, was added per well, and the cells were incubated for three more days. On day 6, M2 macrophages were infected, at an MOI of 150, with strain YS1646Δ*asd*/ΔFLG/Δ*pagP*/Δ*ansB*/Δ*csgD* containing a plasmid encoding secreted NanoLuc^{®} (secNanoLuc^{®}) under control of a CMV promoter (referred to herein as strain YS1646Δ*asd*/ΔFLG/Δ*pagP*/Δ*ansB*/Δ*csgD*-NanoLuc^{®}). Cell culture supernatants were harvested every 24 hours for a period of five days, and the levels of secreted NanoLuc^{®} were measured using a Luciferase Assay Detection Kit (Promega).

The results are summarized in the table below. As shown in the table below, the amount of secreted NanoLuc^{®}, measured in terms of relative light units (RLUs) detected from the luciferase activity assay, increased at every time point measured, from 24 hours, until 120 hours, for the infected M2 macrophages, compared to the uninfected M2 macrophages (control, which gave a background luminescence of 60.0 RLUs). The NanoLuc^{®} signals significantly increased with time, indicating an efficient delivery of plasmid DNA and a sustained expression of mRNA encoding protein, over a period of several days, upon infection of M2 macrophages with strain YS1646Δ*asd*/ΔFLG/Δ*pagP*/Δ*ansB*/Δ*csgD*-NanoLuc^{®}*.* These data demonstrate efficient plasmid transfer from the bacteria to the M2 macrophages, upon bacterial infection, and following bacterial cell death inside the M2 macrophages.

### Protein Secretion Following Infection of M2 Macrophages with Immunostimulatory Bacteria Containing Plasmid Encoding Reporter Gene

| **Treatments (Infections)** | **Time Post-Infection** | **Luciferase Activity (RLUs)** |
|---|---|---|
| Uninfected M2 Macrophages (control) | 24 hours | 60.0 |
| YS1646Δ*asd*/ΔFLG/Δ*pagP*/Δ*ansB*/Δ*csgD*-NanoLuc^{®} (infection) | 24 hours | 63.7 |
| YS1646Δ*asd*/ΔFLG/Δ*pagP*/Δ*ansB*/Δ*csgD*-NanoLuc^{®} (infection) | 48 hours | 193.2 |
| YS1646Δ*asd*/ΔFLG/Δ*pagP*/Δ*ansB*/Δ*csgD*-NanoLuc^{®} (infection) | 72 hours | 308.2 |
| YS1646Δ*asd*/ΔFLG/Δ*pagP*/Δ*ansB*/Δ*csgD*-NanoLuc^{®} (infection) | 96 hours | 415.7 |
| YS1646Δ*asd*/ΔFLG/Δ*pagP*/Δ*ansB*/Δ*csgD*-NanoLuc^{®} (infection) | 120 hours | 515.2 |

In a parallel experiment, M2 macrophages were transfected with 1 µg of the same secNanoLuc^{®} expression plasmid, using Viromer^{®} RED mRNA and plasmid DNA transfection reagent (OriGene), or were infected with strain YS1646Δ*asd*/ΔFLG/Δ*pagP*/Δ*ansB*/Δ*csgD*-NanoLuc^{®}*,* as described above. Every 24 hours for a period of five days, and for every time point, the infected and transfected M2 macrophages were lysed with RNA Lysis Buffer (Zymo Research), and RNA extraction was performed using the Zymo Research *Quick-*RNA^{™} 96 Kit, according to the manufacturer's protocol. Synthesis of cDNA was performed from template RNA using the iScript^{™} Reverse Transcription Supermix for RT-qPCR (Bio-Rad) in a 20 µL reaction, according to the manufacturer's instructions. qPCR for the NanoLuc^{®} was performed with a CFX96^{™} Real-Time PCR Detection System (Bio-Rad) using the iQ^{™} Multiplex Powermix (Bio-Rad). The standard thermocycling program on the Bio-Rad CFX96^{™} Real-Time PCR Detection System consisted of a 95 °C denaturation for 150 seconds, followed by 39 cycles of 95 °C for 15 seconds and 60 °C for 55 seconds. All samples were run in triplicates, and the mean C_{q} values were calculated. Quantification of the NanoLuc^{®} mRNA was normalized using actin reference mRNA (Bio-Rad, assay ID: qHsaCEP0036280). ΔC_{q} was calculated as the difference between the target (*i.*e.*,* NanoLuc^{®}) and reference gene (*i.e*., actin). ΔΔC_{q} was obtained by normalizing the ΔC_{q} values of the YS1646Δ*asd*/ΔFLG/Δ*pagP*/Δ*ansB*/Δ*csgD*-NanoLuc^{®} infected M2 macrophages, and the M2 macrophages transfected with the plasmid encoding NanoLuc^{®}, to the average ΔC_{q} value of the untransfected control group. NanoLuc^{®} expression in transfected M2 macrophages was normalized to a value of 1.0, based on the average ΔC_{q} values, and the fold increase in NanoLuc^{®} expression from infected M2 macrophages (*i.e*., bactofection), relative to NanoLuc^{®} expression from transfected M2 macrophages, was calculated as 2^-ΔΔC_{q}.

The results are summarized in the table below, which shows that a higher level of NanoLuc^{®} expression was detected by RT-qPCR at 24 hours, in the M2 macrophages infected with strain YS1646Δ*asd*/ΔFLG/Δ*pagP*/Δ*ansB*/Δ*csgD-*NanoLuc^{®}, compared to the level of NanoLuc^{®} expression in the M2 macrophages transfected with the plasmid encoding NanoLuc^{®}. This indicates a higher degree of plasmid delivery upon infection of M2 macrophages with the immunostimulatory bacteria, as compared to direct transfection of the cells with plasmid DNA.

### Gene Expression Levels Following Bactofection or Transfection of M2 Macrophages

| **Treatments to M2 Macrophages** | **Time Post-Infection** | **NanoLuc^{®} mRNA Expression from Infected Cells Relative to mRNA from Transfected Cells (Mean Fold Increase)** | |
|---|---|---|---|
| | | **Mean** | **SEM** |
| Transfection with Plasmid Encoding NanoLuc^{®} | 24 hours | 1 | - |
| Infection with YS1646Δ*asd*/ΔFLG/Δ*pagP*/Δ*ansB*/Δ*csgD-*NanoLuc^{®} | 24 hours | 4.8 | 0.4 |
| Infection with YS1646Δ*asd*/ΔFLG/Δ*pagP*/Δ*ansB*/Δ*csgD-*NanoLuc^{®} | 48 hours | 4.9 | 0.3 |
| Infection with YS1646Δ*asd*/ΔFLG/Δ*pagP*/Δ*ansB*/Δ*csgD-*NanoLuc^{®} | 72 hours | 4.4 | 0.3 |
| Infection with YS1646Δ*asd*/ΔFLG/Δ*pagP*/Δ*ansB*/Δ*csgD-*NanoLuc^{®} | 96 hours | 4.3 | 0.4 |
| Infection with YS1646*Δasd*/ΔFLG/Δ*pagP*/*ΔansB*/*ΔcsgD-*NanoLuc^{®} | 120 hours | 4.8 | 0.4 |

| | | | |
|---|---|---|---|
| SEM = Standard Error of Mean | | | |

### Example 28

### Positioning of Encoding Nucleic Acids, in Plasmids Encoding A Plurality of Heterologous Products, Can Enhance the Production of Detectable Protein in Human Cells

This example shows that the expression of a plurality of therapeutic products, encoded on plasmids in the immunostimulatory bacteria provided herein, can be improved by virtue of the position of the nucleic acid molecule encoding one product, relative to the positions of nucleic acid molecules encoding other products. To demonstrate this, and to identify a position for each gene that encodes a particular product (payload) in the expression cassette on the combination plasmids that results in the highest level of protein expression in target cells, various combinations of cytokines and other immunostimulatory proteins were cloned into a plasmid containing one or more eukaryotic promoters. The plasmids contained two separate open reading frames (ORFs), each under the control of a different promoter; an EF-1α promoter, and a CMV promoter (*i.e.,* a dual promoter system), or contained 2A peptides (*e*.*g*., T2A and/or P2A) within one ORF, and one promoter to drive the expression of two or more genes (*i.e.,* a single promoter system). The plasmids were transfected into HEK293T cells, and the expression levels for the various encoded products (payloads) in the cell culture supernatants were determined.

The payloads encoded on the combination plasmids include, but are not limited to, for example, murine STING with the GOF (gain-of-function) mutation C205Y (muSTING C205Y); human STING with the GOF mutation R284G and with a replacement of the human STING C-terminal tail (CTT) with the Tasmanian devil STING CTT (huSTING R284G tazCTT); human STING with the GOF mutations N154S and R284G and with a replacement of the human STING CTT with the Tasmanian devil STING CTT (huSTING N154S/R284G tazCTT); human STING with the GOF mutations N154S and R284G (huSTING N154S/R284G); murine IL-12p70 (muIL-12p70); murine IL-15Rα-IL-15sc (muIL-15Rα-IL-15sc); murine IL-21 (muIL-21); murine IL-18 (muIL-18); murine CXCL10 (muCXCL10); anti-murine CTLA-4 scFv (anti-muCTLA-4 scFv; SEQ ID NO:404); anti-murine CTLA-4 scFv-Fc (anti-muCTLA-4 scFv-Fc; SEQ ID NO:405); murine 4-1BBLΔCyt (mu4-1BBLΔCyt, where ΔCyt indicates a deletion of the cytoplasmic domain); murine soluble TGFβ receptor II fused with a mouse IgG2a Fc (mu sTGFβRII-Fc; SEQ ID NO:406); human soluble TGFβ receptor II fused with a human IgG1 Fc (hu sTGFβRII-Fc; SEQ ID NO:407); murine IFN-alpha2 (muIFN-α2); murine IFN-beta (muIFN-β); murine IL-36 gamma (muIL-36γ); murine IL-23 (muIL-23); murine OX40L (muOX40L); and various combinations of two or more thereof. The sequences were confirmed by Sanger sequencing.

The anti-murine CTLA-4 scFv (SEQ ID NO:404) anti-murine CTLA-4 scFv-Fc (SEQ ID NO:405) were derived from the 9D9 clone. The scFv contains an IgK leader mouse sequence, and the V_{L} and V_{H} domains from clone 9D9, linked via a (Gly₄Ser)₃ linker. The scFv-Fc also contains a mouse IgG2a Fc linked to the V_{H} domain.
HEK293T STING Null Cells (293-Dual^{™} Null Cells; InvivoGen), which do not contain endogenous STING, and express secreted embryonic alkaline phosphatase (SEAP), placed under the control of the endogenous IFN-stimulated response element (ISRE) promoter, where the coding sequence of ISRE is replaced by the SEAP ORF using knock-in technology, were used. HEK293T STING Null cells (293-Dual^{™} Null Cells; InvivoGen) were seeded in 24-well plates coated with poly-L-lysine at 200,000 cells per well, and incubated overnight at 37 °C in a 5% CO₂ incubator, to achieve 80% confluency. The following day, 500 ng of each plasmid DNA was diluted in serum-free media and added to FuGENE^{®} transfection reagent (Promega), at the proper reagent:DNA ratios, with untransfected wells serving as negative controls (in duplicates). Cell culture supernatants from each sample were collected 48 hours post-transfection.

STING activity of each of the encoded STING variants (muSTING C205Y, huSTING N154S/R284G, huSTING R284G tazCTT, and huSTING N154S/R284G tazCTT) was evaluated with the HEK 293T STING Null ISRE-SEAP reporter cell line (293-Dual^{™} Null Cells; InvivoGen). Using these cells, the type I interferon (IFN) activity (induced by STING) is assessed by monitoring type I IFN-stimulated SEAP production in the cell supernatants. 20 µL of cell culture supernatant was added to 180 µL of QUANTI-Blue^{™} reagent (InvivoGen), which is used for measuring SEAP. Type I interferon activation was determined by measuring ISRE-induced SEAP activity on a SpectraMax^{®} M3 Spectrophotometer (Molecular Devices), at an absorbance of 650 nm.

Cell culture supernatants also were assessed for the expression of encoded cytokines by using ELISAs specific for each cytokine. For the muIL-15Rα-IL-15sc constructs, the murine IL-15Rα-IL-15sc ELISA (Thermo Fisher Scientific) was used, per kit instructions. ELISAs specific for murine IL-18 (Invitrogen), murine IL-21 (Invitrogen), murine IL-12p70 (BioLegend), murine IL-23 (BioLegend), murine CXCL10 (BioLegend), and murine IL-36γ (RayBiotech), were used to measure the levels of each of these cytokines in the cell culture supernatants. Murine IFN-α2 and murine IFN-β were measured using the appropriate U-PLEX Assays (Meso Scale Discovery).
Direct ELISAs with mouse TGF-β1 or human TGF-β1 (R&D Systems) were performed on the cell culture supernatants of HEK293T cells transfected with plasmids encoding the murine or human soluble TGFβ receptor II fusions with Fc, respectively, to measure the expression levels of these proteins. Direct ELISAs with mouse CTLA-4-Fc (R&D Systems) were performed on the cell culture supernatants of cells transfected with plasmids encoding anti-murine CTLA-4 scFv and anti-murine CTLA-4 scFv-Fc, to measure the expression levels of these proteins.

The expression of the murine cell surface ligands 4-1BBLΔcyt and OX40L was assessed by flow cytometry, 48 hours post-transfection. For the cells transfected with the plasmids encoding mu4-1BBLΔcyt, the cells were washed twice with PBS + 2% FBS by centrifugation at 1300 RPM for 3 minutes. The cells then were resuspended in PBS + 2% FBS, and stained with biotin-conjugated anti-murine 4-1BBL antibody (clone TKS-1, BioLegend). After incubation for 30 minutes, the cells were washed twice with PBS + 2% FBS by centrifugation at 1300 RPM for 3 minutes, resuspended in PBS + 2% FBS, and stained with APC-conjugated streptavidin (BioLegend). After incubation for 30 minutes, the cells were washed twice with PBS + 2% FBS by centrifugation at 1300 RPM for 3 minutes, and resuspended in PBS + 2% FBS with DAPI (dead/live stain). Flow cytometry data were acquired using the ACEA NovoCyte^{®} flow cytometer (ACEA Biosciences, Inc.) and analyzed using the FlowJo^{™} software (Tree Star, Inc.).

Alternatively, cells expressing muOX40L were washed twice with PBS + 2% FBS by centrifugation at 1300 RPM for 3 minutes. The cells were then resuspended in PBS + 2% FBS, and stained with APC-conjugated anti-mouse OX40L antibody (clone RM134L, BioLegend). After incubation for 30 minutes, the cells were washed twice with PBS + 2% FBS by centrifugation at 1300 RPM for 3 minutes, and resuspended in PBS + 2% FBS with DAPI (dead/live stain). Flow cytometry data were acquired using the ACEA NovoCyte^{®} flow cytometer (ACEA Biosciences, Inc.) and analyzed using the FlowJo^{™} software (Tree Star, Inc.).

The expression of individual payloads in the combination plasmids are summarized in the table below, using the symbols -, +, ++, and +++ to indicate expression levels. - denotes no expression; + denotes some expression; ++ denotes good expression; and +++ denotes high expression. The symbol +/- denotes expression levels between + and -, *i.e.,* low expression; +/++ denotes expression levels between + and ++, *i.e.,* moderate expression; and ++/+++ denotes expression levels between ++ and +++ expression, *i.e.,* high expression.

For the single promoter systems, in which the plasmid encodes two or more payloads under the control of a single CMV promoter, with a 2A peptide (T2A or P2A) encoded between each pair of genes or ORFs, X_T2A denotes that the payload being assessed *(i.e.,* "X") is encoded before the T2A peptide on a plasmid with a CMV promoter; T2A_X denotes that the payload being assessed is encoded after the T2A peptide on a plasmid with a CMV promoter; P2A_X denotes that the payload being assessed is encoded after the P2A peptide on a plasmid with a CMV promoter; and P2A_X_T2A_X*, where X* is the payload being assessed, denotes that the payload being assessed is encoded after the T2A peptide that is encoded after the P2A peptide and the payload (X) following the P2A polypeptide, on a plasmid with a CMV promoter.
For the single promoter systems, in which the plasmid encodes two or more payloads under the control of a single EF-1α promoter, with a 2A peptide (T2A or P2A) encoded between each pair of genes or ORFs, X^{#}_ T2A and T2A _X^{#}, where X^{#} is the payload that is assessed, denote that the payload is expressed before or after the T2A peptide, respectively, on a plasmid with an EF-1α promoter. For example, in a plasmid encoding CMV mu4-1BBLΔcyt_T2A_muIL-12p70_P2A_mu sTGFβRII-Fc_T2A_huSTING N154S/R284G tazCTT, X_T2A represents mu4-1BBLΔcyt; T2A_X represents muIL-12p70; P2A_X represents mu sTGFβRII-Fc; and P2A_X_T2A_X* represents huSTING N154S/R284G tazCTT. Similarly, in a plasmid encoding CMV muIL-12p70_T2A_muIL-21 + EF-1α mu4-1BBLΔcyt_T2A_muSTING C205Y, X_T2A represents muIL-12p70; T2A_X represents muIL-21; X^{#}_ T2A represents mu4-1BBLΔcyt; and T2A _X^{#} represents muSTING C205Y.

For the plasmids encoding each payload under the control of a separate promoter (or only one payload with one promoter; *i.e.,* single expressors), CMV_X and EF-1α_X^{#} denote that the payload being assessed is encoded after a CMV promoter (payload X), or after an EF-1α promoter (payload X^{#}), respectively.
As shown in the table below, single expressors generally show the highest expression levels. Expression tends to decrease for each payload as more proteins are encoded on the plasmid, whether it is a dual promoter plasmid, or a single promoter plasmid. Expression of the payload located in the first position of a combination plasmid (*e*.*g*., before the first T2A), generally was higher than expression of the same payload in other positions on the plasmid. In combination plasmids containing more than one payload, higher expression was often seen in the first position after the CMV promoter. Murine 4-1BBLΔCyt was found to only express well when encoded in the first position, after the CMV promoter, in combination plasmids.

### Expression Levels of Single and Combination Payloads

| **Encoded Payloads** | **CMV Promoter** | | | | **EF-1α Promoter** | | **Single Expressors** | |
|---|---|---|---|---|---|---|---|---|
| | **X_ T2A** | **T2A_ X** | **P2A _X** | **P2A _X_ T2A X*** | **X^{#}_ T2A** | **T2A _X^{#}** | **CMV _X** | **EF-1α _X^{#}** |
| CMV muSTING C205Y WPRE SV40pA | | | | | | | + | |
| CMV muSTING C205Y HPRE bGHpA | | | | | | | + | |
| EF-1α muSTING C205Y WPRE SV40pA | | | | | | | | +++ |
| CMV huSTING R284G tazCTT HPRE bGHpA | | | | | | | +++ | |
| CMV huSTING N154S/R284G HPRE bGHpA | | | | | | | +++ | |
| CMV huSTING N154S/R284G tazCTT HPRE bGHpA | | | | | | | +++ | |
| CMV muIL-12p70 WPRE SV40pA | | | | | | | +++ | |
| CMV muIL-12p70 HPRE bGHpA | | | | | | | +++ | |
| CMV muIL-15Rα-IL-15sc HPRE bGHpA | | | | | | | ++ | |
| CMV muIL-21 HPRE bGHpA | | | | | | | ++ | |
| CMV muIL-18 HPRE bGHpA | | | | | | | +++ | |
| EF-1α muCXCL10 WPRE SV40pA | | | | | | | | +++ |
| CMV anti-muCTLA-4 scFv WPRE SV40pA | | | | | | | +++ | |
| EF-1α anti-muCTLA-4 scFv WPRE SV40pA | | | | | | | | +++ |
| CMV anti-muCTLA-4 scFv HPRE bGHpA | | | | | | | +++ | |
| CMV anti-muCTLA-4 scFv-Fc WPRE-SV40pA | | | | | | | +++ | |
| EF-1α mu4-1BBLΔCyt WPRE SV40pA | | | | | | | | +++ |
| CMV mu4-1BBLΔCyt HPRE bGHpA | | | | | | | ++ | |
| CMV mu sTGFβRII-Fc WPRE SV40pA | | | | | | | +++ | |
| CMV mu sTGFβRII-Fc HPRE bGHpA | | | | | | | +++ | |
| CMV hu sTGFβRII-Fc HPRE bGHpA | | | | | | | +++ | |
| CMV muIFN-α2 HPRE bGHpA | | | | | | | +++ | |
| CMV muIFN-β HPRE bGHpA | | | | | | | +++ | |
| CMV muIL-36γ HPRE bGHpA | | | | | | | +++ | |
| CMV muIL-23 HPRE bGHpA | | | | | | | +++ | |
| CMV muOX40L HPRE bGHpA | | | | | | | +++ | |
| CMV mulL-12p70 HPRE bGHpA + EF-1α muSTING C205Y WPRE SV40pA | | | | | | | +++ | ++ |
| CMV muSTING C205Y HPRE bGHpA + EF-1α muIL-12p70 WPRE SV40pA | | | | | | | + | + |
| CMV muIL-12p70 HPRE bGHpA + EF-1α muCXCL10 WPRE SV40pA | | | | | | | +++ | ++ |
| CMV muIL-12p70_T2A_muIL-15Rα-IL-15sc HPRE bGHpA + EF-1α muSTING C205Y WPRE SV40pA | ++ | +++ | | | | | | +++ |
| CMV muIL-12p70_T2A_muIL-18 HPRE bGHpA + EF-1α muSTING C205Y WPRE SV40pA | ++ | ++ | | | | | | +++ |
| CMV muIL-12p70_T2A_muIL-21 HPRE bGHpA + EF-1α muSTING C205Y WPRE SV40pA | + | ++ | | | | | | +++ |
| CMV muIL-12p70_T2A**_**muIL-18 HPRE | ++ | ++ | | | | | | ++ |
| bGHpA + EF-1α muCXCL10 WPRE SV40pA | | | | | | | | |
| CMV muIL-12p70_T2A_muIL-21 HPRE bGHpA + EF-1α muCXCL10 WPRE SV40pA | ++ | ++ | | | | | | ++ |
| CMV muIL-12p70_T2A_ muIL-15Rα-IL-15sc HPRE bGHpA + EF-1α muCXCL10 WPRE SV40pA | ++ | +++ | | | | | | ++ |
| CMV muIL-12p70_T2A_muSTING C205Y HPRE bGHpA + EF-1α muIL-18 WPRE SV40pA | ++ | ++ | | | | | | + |
| CMV muIL-12p70_T2A_muSTING C205Y HPRE bGHpA + EF-1α anti-muCTLA-4 scFv WPRE SV40pA | + | + | | | | | | + |
| CMV mulL-12p70_T2A_muSTING C205Y HPRE bGHpA + EF-1α mu4-1BBLΔCyt WPRE SV40pA | +++ | ++ | | | | | | ++ |
| CMV muIL-12p70_T2A_ mu4-1BBLΔCyt HPRE bGHpA + EF-1α muSTING C205Y WPRE SV40pA | ++ | + | | | | | | + |
| CMV muIL-12p70_T2A_ muIL-15Rα-IL-15sc HPRE bGHpA + EF-1α mu4-1BBLΔCyt_ T2A_muSTING C205Y WPRE SV40pA | ++ | +++ | | | +/- | + | | |
| CMV muIL-12p70_T2A_muIL-21 HPRE bGHpA + EF-1α mu4-1BBLΔCyt_T2A_muSTING C205Y WPRE SV40pA | + | + | | | +/- | + | | |
| CMV muIL-12p70_T2A_muSTING C205Y HPRE bGHpA + EF-1α anti-muCTLA-4 scFv_T2A _mu4-1BBLΔCyt WPRE SV40pA | + | + | | | +/- | +/- | | |
| CMV muIL-12p70_T2A_ muIL-15Rα-IL-15sc HPRE bGHpA + EF-1α anti-muCTLA-4 scFv_T2A_muSTING C205Y WPRE SV40pA | + | ++ | | | +/- | ++ | | |
| CMV muIL-12p70_T2A_muIL-18 HPRE bGHpA + EF-1α anti-muCTLA-4 scFv_T2A _muSTING C205Y WPRE SV40pA | + | + | | | +/- | + | | |
| CMV muIL-12p70_T2A_muIL-21 HPRE bGHpA + EF-1α anti-muCTLA-4 scFv_T2A_muSTING C205Y WPRE SV40pA | + | + | | | +/- | + | | |
| CMV muIL-12p70_T2A_muSTING C205Y WPRE SV40pA | ++ | ++ | | | | | | |
| CMV muIL-12p70_T2A_muSTING C205Y HPRE bGHpA | ++ | ++ | | | | | | |
| CMV muIL-12p70_T2A_ muIL-15Rα-IL-15scHPRE bGHpA | ++ | ++ | | | | | | |
| CMV muIL-15Rα-IL-15sc_T2A_muIL-12p70 HPRE bGHpA | ++ | ++ | | | | | | |
| CMV muIL-12p70_T2A_muIL-18 HPRE bGHpA | ++ | ++ | | | | | | |
| CMV muIL-18_T2A_muIL-12p70 HPRE bGHpA | ++ | + | | | | | | |
| CMV muIL-12p70_T2A_muIL-21 HPRE bGHpA | ++ | ++ | | | | | | |
| CMV muIL-21_T2A_muIL-12p70 HPRE bGHpA | +++ | ++ | | | | | | |
| CMV muIL-12p70_T2A_huSTING R284G tazCTT HPRE bGHpA | + | ++/+++ | | | | | | |
| CMV mu4-1BBLΔCyt_T2A_huSTING R284G tazCTT HPRE bGHpA | + | +++ | | | | | | |
| CMV mu4-1BBLΔCyt_T2A_muIL-12p70 HPRE bGHpA | ++ | ++ | | | | | | |
| CMV anti-muCTLA-4 scFv_T2A muIL-12p70 HPRE bGHpA | + | + | | | | | | |
| CMV anti-muCTLA-4 scFv_T2A_huSTING R284G tazCTT HPRE bGHpA | +/- | ++ | | | | | | |
| CMV anti-muCTLA-4 scFv-Fc_T2A_muIL-12p70 HPRE bGHpA | +++ | + | | | | | | |
| CMV anti-muCTLA-4 scFv-Fc_T2A_huSTING R284G tazCTT HPRE bGHpA | +++ | ++ | | | | | | |
| CMV muIFN-α2_T2A_muIL-12p70 HPRE bGHpA | + | ++ | | | | | | |
| CMV muIFN-α2_T2A_muIL-36γ HPRE bGHpA | + | +++ | | | | | | |
| CMV muIFN-α2_T2A_muIFN-β HPRE bGHpA | + | +++ | | | | | | |
| CMV muIFN-α2_T2A_huSTING R284G tazCTT HPRE bGHpA | ++ | +++ | | | | | | |
| CMV mu4-1BBLΔCyt _T2A_mu sTGFβRII-Fc HPRE bGHpA | +++ | +++ | | | | | | |
| CMV mud-1BBLΔCyt_T2A_ muIL-15Rα-IL-15sc HPRE bGHpA | +++ | +++ | | | | | | |
| CMV mu4-1BBLΔCyt _T2A_muIFN-α2 HPRE bGHpA | +++ | ++ | | | | | | |
| CMV mu4-1BBLΔCyt_T2A_muIL-36y HPRE | +++ | +++ | | | | | | |
| CMV muIL-21_T2A_muIL-15Rα-IL-15sc HPRE bGHpA | +++ | ++ | | | | | | |
| CMV muIL-21_T2A_huSTING R284G tazCTT HPRE bGHpA | ++ | ++ | | | | | | |
| CMV muIL-15Rα-IL-15sc_T2A_huSTING R284G tazCTT HPRE bGHpA | ++ | +++ | | | | | | |
| CMV muIL-36γ_T2A_muIL-12p70 HPRE bGHpA | ++ | ++ | | | | | | |
| CMV muIL-36γ_T2A_huSTING R284G tazCTT HPRE bGHpA | +++ | +++ | | | | | | |
| CMV muIL-36γ_T2A_muIFN-α2 HPRE bGHpA | ++ | ++ | | | | | | |
| CMV muIL-36γ_T2A_muIL-23 HPRE bGHpA | ++ | +++ | | | | | | |
| CMV mu sTGFβRII-Fc_T2A_huSTING R284G tazCTT HPRE bGHpA | ++ | +++ | | | | | | |
| CMV muIL-12p70_T2A_anti-muCTLA-4 scFv_P2A_huSTING R284G tazCTT HPRE bGHpA | + | +/- | +++ | | | | | |
| CMV anti-muCTLA-4 scFv_T2A_muIL-12p70_P2A_huSTING R284G tazCTT HPRE bGHpA | +/- | + | +++ | | | | | |
| CMV muIL-12p70_T2A_anti-muCTLA-4 scFv-Fc_P2A_ huSTING R284G tazCTT HPRE bGHpA | + | ++ | ++ | | | | | |
| CMV anti-muCTLA-4 scFv-Fc_T2A_muIL-12p70_P2A_ huSTING R284G tazCTT HPRE bGHpA | +++ | + | + | | | | | |
| CMV muIL-21_T2A_muIL-12p70_P2A_huSTING R284G tazCTT HPRE bGHpA | ++ | + | +++ | | | | | |
| CMV muIL-21_T2A_muIL-12p70 _P2A_muIFN-α2 HPRE bGHpA | +++ | + | + | | | | | |
| CMV muIL-21_T2A_muIL-15Rα-IL-15sc _P2A_huSTING R284G tazCTT HPRE bGHpA | ++ | ++ | +++ | | | | | |
| CMV muIL-12p70_T2A_ muIL-15Rα-IL-15sc _P2A_ huSTING R284G tazCTT HPRE bGHpA | + | + | ++ | | | | | |
| CMV muIL-12p70_T2A_ muIL-15Rα-IL-15sc _P2A_muIFN-α2 HPRE bGHpA | + | + | + | | | | | |
| CMV muIL-36y_T2A_muIL-12p70_P2A_huSTING R284G tazCTT HPRE bGHpA | ++ | + | ++ | | | | | |
| CMV muIL-36y_T2A_muIL-12p70_P2A_muOX40L HPRE bGHpA | ++ | + | ++ | | | | | |
| CMV muIL-36y_T2A_muIL-23 _P2A_ muOX40L HPRE bGHpA | ++ | +++ | ++ | | | | | |
| CMV muIL-36y_T2A_muIL-23 _P2A_ huSTING R284G tazCTT HPRE bGHpA | ++ | +++ | ++ | | | | | |
| CMV mu4-1BBLΔCyt_T2A_muIL-12p70_P2A_huSTING R284G tazCTT HPRE bGHpA | ++ | + | +++ | | | | | |
| CMV mu4-1BBLACyt_T2A_muIL-12p70 _P2A_ huSTING N154S/R284G tazCTT HPRE bGHpA | ++ | + | +++ | | | | | |
| CMV mu4-1BBLΔCyt_T2A_muIL-12p70_P2A_huSTING N154S/R284G tazCTT HPRE bGHpA | ++ | + | +++ | | | | | |
| CMV mu4-1BBLΔCyt_T2A_mu sTGFβRII-Fc_P2A_ huSTING R284G tazCTT HPRE bGHpA | +++ | ++ | ++ | | | | | |
| CMV muIL-15Rα-IL-15sc _T2A_muIL-12p70_P2A_huSTING R284G tazCTT HPRE bGHpA | + | + | +++ | | | | | |
| CMV mu sTGFβRII-Fc_T2A_muIL-12p70_P2A_ huSTING R284G tazCTT HPRE bGHpA | + | + | +++ | | | | | |
| CMV mu4-1BBLΔCyt_T2A_hu sTGFβRII-Fc_P2A_muIL-12p70_T2A_huSTING N154S/R284G tazCTT HPRE bGHpA | ++ | ++ | + | +++ | | | | |
| CMV mu4-1BBLΔCyt_T2A-muIL-12p70_P2A_hu sTGFβRII-Fc_T2A_huSTING N154S/R284G tazCTT HPRE bGHpA | ++ | + | ++ | +++ | | | | |

| | | | | | | | | |
|---|---|---|---|---|---|---|---|---|
| HPRE = Hepatitis B virus Posttranscriptional Regulatory Element; WPRE = Woodchuck Hepatitis Virus (WHP) Posttranscriptional Regulatory Element; bGHpA = bovine growth hormone poly A; SV40pA = simian virus 40 poly A. | | | | | | | | |

### Example 29

### Biodistribution, Clearance, Tumor Colonization, and Ectopic Gene Expression Studies in Strains YS1646Δasd/ΔFLG/ΔpagP/ΔansB/ΔcsgD and YS1646Δasd/ΔFLG/ΔpagP/ΔansB/ΔcsgD/ΔlppAB

The biodistribution of strains YS1646Δ*asd*/Δ*FLG*/Δ*pagP*/Δ*ansB*/Δ*csgD* and YS1646Δ*asd*/Δ*ELG*/Δ*pagP*/Δ*ansB*/Δ*csgD*/Δ*lppAB* in various tissues in non-tumor bearing mice, including the liver, spleen, heart, lungs, kidney, intestine, muscle, bone marrow, and lymph nodes, and the clearance therefrom, was assessed and compared to the biodistribution and clearance of parental strain YS1646, following systemic administration. The colonization of the strains in tumors was compared to colonization of non-tumor tissues in mice bearing tumors, and the expression of a heterologous gene product, encoded on a plasmid in the bacteria, in tumors vs. non-tumor tissues, also was determined.

### A. The YS1646Δasd/ΔFLG/ΔpagP/ΔansB/ΔcsgD and YS1646Δasd/ΔFLG/ΔpagP/ΔansB/ΔcsgD/ΔlppAB Deletion Strains are Cleared Rapidly from Naive Mice, and Ectopic Gene Expression in Healthy Tissue is not Observed

A biodistribution study was performed to compare the bacterial clearance from tissues over time in non-tumor bearing mice, between the parental YS 1646 strain, and strains YS1646Δ*asd*/ΔFLG/Δ*pagP*/Δ*ansB*/Δ*csgD* and YS1646Δ*asd*/Δ*ELG*/Δ*pagP*/Δ*ansB*/Δ*csgD*/Δ*lppAB.* A luciferase protein was encoded on plasmids in the YS1646Δ*asd*/Δ*FLG*/Δ*pagP*/Δ*ansB*/Δ*csgD* and YS1646*Δasd*/Δ*FLG*/Δ*pagP*/Δ*ansB*/Δ*csgD*/Δ*lppAB* strains, and expression of the luciferase in tissues also was assessed, in order to determine the levels of ectopic gene expression in healthy, non-tumor tissues, as described below. For this, 6-8 week-old female BALB/c mice (3 mice per group) were IV injected with a single dose of 2 x10⁶ CFUs of each bacterial strain, or with PBS vehicle control. At 2 hours, 24 hours, and 30 days post-dosing, the mice were euthanized, and the spleen, liver, heart, lungs, kidney, intestine, muscle, bone marrow, and lymph nodes were harvested. The tissues were homogenized using the GentleMACS^{™} Octo Dissociator and the M tubes (Miltenyi Biotec) molecule setting in 2 mL of PBS, and homogenates were plated on LB plates to enumerate the number of colony forming units (CFUs) per gram of tissue.

As shown in the tables below, all three strains demonstrated CFUs in the spleen and liver, and to a lesser extent, in the heart, lungs and kidneys, at 2 hours post-IV dosing. Unlike with the parental YS1646 strain, which continued to demonstrate high CFUs in the spleen and liver at 24 hours post-dosing, significantly lower CFUs were detected in the spleens and livers of the mice treated with the YS1646Δ*asd*/Δ*FLG*/Δ*pagP*/Δ*ansB*/Δ*csgD* and YS1646*Δasd*/Δ*FLG*/Δ*pagP*/Δ*ansB*/Δ*csgD*/Δ*lppAB* deletion strains. At day 30 post-dosing, strains YS1646Δ*asd*/Δ*FLG*/Δ*pagP*/Δ*ansB*/Δ*csgD* and YS1646Δ*asd*/Δ*FLG*/Δ*pagP*/Δ*ansB*/Δ*csgD*/Δ*lppAB* were cleared from all organs. The parental YS 1646 strain, in contrast, demonstrated colonization in many tissues at 24 hours post-doing, including colonization that was detected in the spleen and liver at day 30 post-dosing.

### Biodistribution of Strain YS1646 at 2 Hours, 24 Hours, and 30 Days Post IV-Dosing in Non-Tumor Bearing Mice

| **Tissue** | **Time Post-IV Dosing** | | | | | |
|---|---|---|---|---|---|---|
| | **2 Hours** | | **24 Hours** | | **30 Days** | |
| | **Mean CFUs** | **SD** | **Mean CFUs** | **SD** | **Mean CFUs** | **SD** |
| Spleen | 23981 | 6909.2 | 14214 | 2199.9 | 428 | 261.2 |
| Liver | 6914 | 1784.7 | 8881 | 2060.3 | 54 | 27.2 |
| Heart | 1334 | 693.8 | 768 | 264.1 | <LOD | <LOD |
| Lungs | 1821 | 629.4 | 981 | 137.2 | 27 | 27.5 |
| Kidney | 1868 | 595.9 | 668 | 394.0 | <LOD | <LOD |
| Intestine | 88 | 31.3 | 41 | 50.6 | 21 | 1.5 |
| Muscle | 148 | 45.3 | 188 | 31.3 | <LOD | <LOD |
| Bone Marrow | 214 | 194.9 | 834 | 300.0 | <LOD | <LOD |
| Lymph Node | 48 | 11.6 | 14 | 4.7 | <LOD | <LOD |

| | | | | | | |
|---|---|---|---|---|---|---|
| SD = standard deviation; LOD = limit of detection | | | | | | |

### Biodistribution of Strain YS1646Δasd/ΔFLG/ΔpagP/ΔansB/ΔcsgD at 2 Hours, 24 Hours, and 30 Days Post IV-Dosing in Non-Tumor Bearing Mice

| **Tissue** | **Time Post-IV Dosing** | | | | | |
|---|---|---|---|---|---|---|
| | **2 Hours** | | **24 Hours** | | **30 Davs** | |
| | **Mean CFUs** | **SD** | **Mean CFUs** | **SD** | **Mean CFUs** | **SD** |
| Spleen | 19881 | 6322.1 | 621 | 308.4 | 20 | 16.2 |
| Liver | 8154 | 5820.9 | 341 | 396.7 | <LOD | <LOD |
| Heart | 374 | 302.5 | <LOD | <LOD | <LOD | <LOD |
| Lungs | 308 | 111.1 | 101 | 154.2 | <LOD | <LOD |
| Kidney | 68 | 38.7 | <LOD | <LOD | <LOD | <LOD |
| Intestine | 20 | 16.2 | <LOD | <LOD | <LOD | <LOD |
| Muscle | 21 | 15.9 | <LOD | <LOD | <LOD | <LOD |
| Bone Marrow | <LOD | <LOD | <LOD | <LOD | <LOD | <LOD |
| Lymph Node | <LOD | <LOD | <LOD | <LOD | <LOD | <LOD |

| | | | | | | |
|---|---|---|---|---|---|---|
| SD = Standard deviation; LOD = limit of detection | | | | | | |

### Biodistribution of Strain YS1646Δasd/ΔFLG/ΔpagP/ΔansB/ΔcsgD/ΔlppAB at 2 Hours, 24 Hours, and 30 Days Post IV-Dosing in Non-Tumor Bearing Mice

| **Tissue** | **Time Post-IV Dosing** | | | | | |
|---|---|---|---|---|---|---|
| | **2 Hours** | | **24 Hours** | | **30 Days** | |
| | **Mean CFUs** | **SD** | **Mean CFUs** | **SD** | **Mean CFUs** | **SD** |
| Spleen | 2247981 | 3768949.6 | 7674 | 2366.3 | 14 | 4.7 |
| Liver | 208648 | 235899.1 | 4768 | 2011.0 | <LOD | <LOD |
| Heart | 701 | 563.8 | 141 | 102.7 | <LOD | <LOD |
| Lungs | 1434 | 812.7 | 301 | 72.7 | <LOD | <LOD |
| Kidnev | 794 | 744.5 | 114 | 81.7 | <LOD | <LOD |
| Intestine | 47 | 62.4 | <LOD | <LOD | <LOD | <LOD |
| Muscle | 41 | 16.2 | 34 | 12.1 | <LOD | <LOD |
| Bone Marrow | 107 | 91.6 | 61 | 33.9 | <LOD | <LOD |
| Lymph Node | <LOD | <LOD | <LOD | <LOD | <LOD | <LOD |

| | | | | | | |
|---|---|---|---|---|---|---|
| SD = Standard deviation; LOD = limit of detection | | | | | | |

These data demonstrate that the deletion or disruption of gene(s) encoding the flagella, as well as the genes *asd, pagP, ansB, csgD,* and, optionally, *lppAB,* results in the faster clearance of the immunostimulatory bacterial strains from healthy, non-tumor tissues, than parental strain YS1646 (VNP20009, which is *msbB⁻*/*purI⁻*), and results in the complete clearance of the bacteria from non-tumor tissues within 24 hours to 30 days; whereas strain YS1646 (VNP20009) still can be detected in non-tumor tissues 24 hours post-dosing, and in the liver and spleen at 30 days post-dosing. This indicates that the immunostimulatory bacteria with genome modifications provided herein are safer to administer and are better tolerated than strain VNP20009. Thus, higher doses of the immunostimulatory bacteria provided herein can be administered.

In order to measure plasmid delivery by the bacteria to the various non-tumor tissues, and the subsequent heterologous gene expression and protein secretion, the activity of a secreted luciferase protein (NanoLuciferase^{®}, Promega, abbreviated as secNanoLuc^{®}), was measured. The IV administered YS1646Δ*asd*/Δ*FLG*/Δ*pagP*/Δ*ansB*/Δ*csgD* and YS1646Δ*asd*/Δ*FLG*/Δ*pagP*/Δ*ansB*/Δ*csgD*/Δ*lppAB* strains each contained a plasmid encoding secNanoLuc^{®}, under the control of the eukaryotic CMV promoter. Following homogenization of the mouse tissues, as described above, the homogenates were spun down at 1300 RPM for 10 minutes, and the supernatant was collected and assayed for luciferase activity using the NanoGlo^{®} detection reagent (Promega), and luminescence was measured using a SpectraMax^{®} M3 Spectrophotometer/Luminometer (Molecular Devices).

In all tissues, and at all time points, no luciferase activity was detected above background, despite observing CFUs in the spleen and liver at 24 hours post-dosing. These data demonstrate the ability of the YS1646Δ*asd*/Δ*FLG*/Δ*pagP*/Δ*ansB*/Δ*csgD* and YS1646Δ*asd*/Δ*FLG*/Δ*pagP*/Δ*ansB*/Δ*csgD*/Δ*lppAB* deletion strains to rapidly clear from healthy tissues in non-tumor bearing mice, and, unlike the parental YS1646 strain, to not require antibiotics for bacterial clearance at day 30 post-dosing. Importantly, ectopic gene expression was not observed in healthy tissues at any time point.

### B. The YS1646Δasd/ΔFLG/ΔpagP/ΔansB/ΔcsgD and YS1646Δasd/ΔFLG/ΔpagP/ΔansB/ΔcsgD/ΔlppAB Deletion Strains Preferentially Accumulate in Tumor Tissue, and Ectopic Gene Expression is Exclusively Tumor-Specific

To determine the relative tissue colonization, and tumor-specific gene expression of the secNanoLuc^{®} plasmid-containing deletion strains, as compared to the parental YS1646 strain, a biodistribution study was performed in tumor-bearing mice. For this, 6-8 week-old female BALB/c mice (4 mice per group) were inoculated orthotopically in the 4^{th} mammary fat pad with 4T1 mammary carcinoma cells (2x10⁵ cells in 100 µL PBS). Mice bearing 10-day established flank tumors were IV injected with a single dose of 2 x10⁶ CFUs of the YS1646Δ*asd*/Δ*FLG*/Δ*pagP*/Δ*ansB*/Δ*csgD* strain, or the YS1646Δ*asd*/Δ*FLG*/Δ*pagP*/Δ*ansB*/Δ*csgD*/Δ*lppAB* strain, each containing the plasmid encoding secNanoLuc^{®}, or with a single dose of 2 x10⁶ CFUs of the parental YS1646 strain. At day 1, day 4, and day 8 post IV-dosing of the bacteria, mice were euthanized, and the tumors, spleen, liver, heart, lungs, kidney, intestine, muscle, bone marrow, and lymph nodes were harvested and processed as described above, to enumerate the number of colony forming units (CFUs), and to measure the average luminescence (in relative light units (RLUs)) per gram of tumor tissue. In addition, mice were bled at these time points and serum was collected, to determine the levels of systemic pro-inflammatory cytokines induced in response to the administration of the different strains of bacteria, as discussed below.

As shown in the tables below, all three strains demonstrated preferential colonization (as determined by mean CFUs) in tumors, relative to other tissues, at day 1 post-dosing, and demonstrated increasing tumor colonization at day 4 and day 8. Strain YS1646Δ*asd*/Δ*FLG*/Δ*pagP*/Δ*ansB*/Δ*csgD* demonstrated a faster and higher level of tumor colonization than strain YS1646Δ*asd*/Δ*FLG*/Δ*pagP*/Δ*ansB*/Δ*csgD*/Δ*lppAB.* Additionally, for the YS1646Δ*asd*/Δ*FLG*/Δ*pagP*/Δ*ansB*/Δ*csgD* strain, and, to a greater degree, for the YS1646Δ*asd*/Δ*FLG*/Δ*pagP*/Δ*ansB*/Δ*csgD*/Δ*lppAB* strain, very few colonies were observed in tissues other than the tumor, spleen, and liver. In contrast, colonization of the parental YS1646 strain was observed in healthy, non-tumor tissues throughout the mouse, even at day 8 post-dosing.

### Biodistribution of Strain YS1646 in Tumors vs. Healthy Tissues at 1, 4, and 8 Days Post IV-Dosing in Tumor-Bearing Mice

| **Tissue** | **Davs Post-IV Dosing** | | | | | |
|---|---|---|---|---|---|---|
| | **Dav 1** | | **Day 4** | | **Day 8** | |
| | **Mean CFUs** | **SD** | **Mean CFUs** | **SD** | **Mean CFUs** | **SD** |
| Tumor | 30851 | 54138.9 | 12999981 | 7262671 | 58999981 | 11489106.3 |
| Spleen | 7621 | 2683.4 | 85981 | 11981 | 127981 | 14947.6 |
| Liver | 4466 | 2693.3 | 124981 | 73836.7 | 5969981 | 5161208.2 |
| Heart | 246 | 201.3 | 356 | 215.6 | 2096 | 222.9 |
| Lungs | 121 | 9.3 | 2016 | 247.0 | 6081 | 3983.4 |
| Kidney | 106 | 126.7 | 471 | 127.5 | 5951 | 4618.0 |
| Intestine | 131 | 203.0 | 126 | 31.0 | 176 | 145.8 |
| Muscle | <LOD | <LOD | 196 | 163.5 | 21 | 9.5 |
| Bone Marrow | 16 | 0.4 | 131 | 152.9 | 266 | 36.1 |
| Lymph Node | <LOD | <LOD | 86 | 49.5 | 4346 | 947.7 |

| | | | | | | |
|---|---|---|---|---|---|---|
| SD = Standard deviation; LOD = limit of detection | | | | | | |

### Biodistribution of Strain YS1646Δasd/ΔFLG/ΔpagP/ΔansB/ΔcsgD in Tumors vs. Healthy Tissues at 1, 4, and 8 Days post IV-Dosing in Tumor-Bearing Mice

| **Tissue** | **Days Post-IV Dosing** | | | | | |
|---|---|---|---|---|---|---|
| | **Day 1** | | **Day 4** | | **Day 8** | |
| | **Mean CFUs** | **SD** | **Mean CFUs** | **SD** | **Mean CFUs** | **SD** |
| Tumor | 26374 | 26240.6 | 2940281 | 3402144.3 | 3694981 | 2787829.6 |
| Spleen | 3991 | 1050.0 | 17231 | 5894.5 | 17831 | 2563.6 |
| Liver | 13011 | 3178.9 | 55981 | 16554.1 | 16756 | 5120.0 |
| Heart | 371 | 330.3 | 26 | 11.0 | <LOD | <LOD |
| Lungs | 61 | 40.2 | 816 | 185.2 | 176 | 104.7 |
| Kidney | 71 | 31.3 | 121 | 208.2 | <LOD | <LOD |
| Intestine | 10 | 1.5 | <LOD | <LOD | <LOD | <LOD |
| Muscle | <LOD | <LOD | 170 | 321.5 | 96 | 171.3 |
| Bone Marrow | <LOD | <LOD | 136 | 129.2 | 331 | 145.5 |
| Lymph Node | <LOD | <LOD | 11 | 1.0 | 371 | 428.3 |

| | | | | | | |
|---|---|---|---|---|---|---|
| SD = Standard deviation; LOD = limit of detection | | | | | | |

### Biodistribution of Strain YS1646Δasd/ΔFLG/ΔpagP/ΔansB/ΔcsgD/ΔlppAB in Tumors vs. Healthy Tissues at 1, 4, and 8 Days Post IV-Dosing in Tumor-Bearing Mice

| **Tissue** | **Days Post-IV Dosing** | | | | | |
|---|---|---|---|---|---|---|
| | **Day 1** | | **Day 4** | | **Day 8** | |
| | **Mean CFUs** | **SD** | **Mean CFUs** | **SD** | **Mean CFUs** | **SD** |
| Tumor | 4406 | 8433.7 | 295331 | 372484 | 596881 | 406961.5 |
| Spleen | 1926 | 642.6 | 1516 | 330.6 | 2026 | 699.8 |
| Liver | 2241 | 880.9 | 2111 | 213.4 | 2286 | 545.4 |
| Heart | 36 | 22.5 | <LOD | <LOD | <LOD | <LOD |
| Lungs | 146 | 156.7 | 16 | 0.7 | 76 | 92.2 |
| Kidney | <LOD | <LOD | <LOD | <LOD | <LOD | <LOD |
| Intestine | <LOD | <LOD | <LOD | <LOD | <LOD | <LOD |
| Muscle | <LOD | <LOD | <LOD | <LOD | <LOD | <LOD |
| Bone Marrow | <LOD | <LOD | <LOD | <LOD | <LOD | <LOD |
| Lymph Node | <LOD | <LOD | <LOD | <LOD | <LOD | <LOD |

| | | | | | | |
|---|---|---|---|---|---|---|
| SD = Standard deviation; LOD = limit of detection | | | | | | |

To determine the level of heterologous gene expression and protein secretion in the tumors and in the various non-tumor mouse tissues, the activity of the secNanoLuc^{®}, encoded on the plasmids in the bacteria, was measured in the supernatant from the tissue homogenates using a luciferase activity assay, as described above. As shown in the tables below, expression of secNanoLuc^{®} in both strains was confined to the tumor tissue, despite bacterial CFUs being observed in other tissues (as shown above). The RLUs observed in the bone marrow of mice dosed with the YS1646Δ*asd*/Δ*FLG*/Δ*pagP*/Δ*ansB*/Δ*csgD*/Δ*lppAB* deletion strain were determined to be from a contamination, and were not observed in a subsequent experiment. The YS1646Δ*asd*/Δ*FLG*/Δ*pagP*/Δ*αnsB*/Δ*csgD*/Δ*lppAB* deletion strain showed delayed tumor secNanoLuc^{®} expression, as compared to the YS1646Δ*asd*/Δ*FLG*/Δ*pagP*/Δ*ansB*/Δ*csgD* deletion strain, due to a delay in tumor colonization (as shown in the tables above).

### Tumor-Specific Expression of secNanoLuc^{®} Encoded on Plasmid in Strain YS1646Δasd/ΔFLG/ΔpagP/ΔansB/ΔcsgD

| **Tissue** | **Days Post-Dosing** | | | | | |
|---|---|---|---|---|---|---|
| | **Day 1** | | **Day 4** | | **Day 8** | |
| | **Mean RLUs** | **SD** | **. Mean RLUs** | **SD** | **Mean RLUs** | **SD** |
| Tumor | 39 | -5.0 | 7014 | 7606.8 | 29790 | 14706.8 |
| Spleen | <LOD | <LOD | <LOD | <LOD | <LOD | <LOD |
| Liver | <LOD | <LOD | <LOD | <LOD | <LOD | <LOD |
| Heart | <LOD | <LOD | <LOD | <LOD | <LOD | <LOD |
| Lungs | <LOD | <LOD | <LOD | <LOD | <LOD | <LOD |
| Kidney | <LOD | <LOD | <LOD | <LOD | <LOD | <LOD |
| Intestine | <LOD | <LOD | <LOD | <LOD | <LOD | <LOD |
| Muscle | <LOD | <LOD | <LOD | <LOD | <LOD | <LOD |
| Bone Marrow | <LOD | <LOD | <LOD | <LOD | <LOD | <LOD |
| Lymph Node | <LOD | <LOD | <LOD | <LOD | <LOD | <LOD |

| | | | | | | |
|---|---|---|---|---|---|---|
| SD = Standard deviation; LOD = limit of detection | | | | | | |

### Tumor-Specific Expression of secNanoLuc^{®} Encoded on Plasmid in Strain YS1646Δasd/ΔFLG/ΔpagP/ΔansB/ΔcsgD/ΔlppAB

| **Tissue** | **Days Post-Dosing** | | | | | |
|---|---|---|---|---|---|---|
| | **Day 1** | | **Day 4** | | **Dav 8** | |
| | **Mean RLUs** | **SD** | **Mean RLUs** | **SD** | **Mean RLUs** | **SD** |
| Tumor | <LOD | <LOD | 85 | 80.6 | 5503 | 4465.9 |
| Spleen | <LOD | <LOD | <LOD | <LOD | <LOD | <LOD |
| Liver | <LOD | <LOD | <LOD | <LOD | <LOD | <LOD |
| Heart | <LOD | <LOD | <LOD | <LOD | <LOD | <LOD |
| Lungs | <LOD | <LOD | <LOD | <LOD | <LOD | <LOD |
| Kidney | <LOD | <LOD | <LOD | <LOD | <LOD | <LOD |
| Intestine | <LOD | <LOD | <LOD | <LOD | <LOD | <LOD |
| Muscle | <LOD | <LOD | <LOD | <LOD | <LOD | <LOD |
| Bone Marrow | <LOD | <LOD | <LOD | <LOD | 285 | 70.1 |
| Lymph Node | <LOD | <LOD | <LOD | <LOD | <LOD | <LOD |

| | | | | | | |
|---|---|---|---|---|---|---|
| SD = Standard deviation; LOD = limit of detection | | | | | | |

As discussed above, tumor-bearing mice were bled at days 1, 4, and 8 post IV-dosing with the immunostimulatory bacterial strains, and serum was collected. The serum was assessed for the levels of the systemic pro-inflammatory cytokines IL-6, TNF-α, IFN-γ, IL-2, and IL-10, on day 1 (D1), day 4 (D4), and day 8 (D8) post-IV dosing, by cytometric bead array (Mouse Inflammation CBA, BD Biosciences).

As shown in the tables below, on day 1 post-IV dosing of the bacteria, the serum concentrations of all of the cytokines were lower from the mice dosed with strains YS1646Δ*asd*/Δ*FLG*/Δ*pagP*/Δ*ansB*/Δ*csgD* and YS1646Δ*asd*/Δ*FLG*/Δ*pagP*/Δ*ansB*/Δ*csgD*/Δ*lppAB,* compared to the serum concentrations from the mice dosed with the parental YS1646 strain, with the exception of slightly higher levels of IL-6 for the YS1646Δ*asd*/Δ*FLG*/Δ*pagP*/Δ*ansB*/Δ*csgD* strain. The serum concentrations of IFN-γ, IL-2, and IL-10, from the mice dosed with strains YS1646Δ*asd*/Δ*FLG*/Δ*pagP*/Δ*ansB*/Δ*csgD* and YS1646Δ*asd*/ΔFLG/Δ*pagP*/Δ*ansB*/Δ*csgD*/Δ*lppAB* were the same as PBS control on day 1 post-dosing *(i.e.,* 0 pg/mL). The parental strain, YS1646, demonstrated peak systemic cytokine levels on day 4 for IL-6, IFN-γ, and IL-2, while the serum cytokine levels after administration of each of the deletion strains were well below those observed after administration of the parental strain, and closer to the PBS vehicle control measurements. The high serum concentration of IL-10, which is a direct target of TLR2 signaling, on day 1 from dosing with the parental strain, was absent after dosing with each of the deletion strains, which contain deletions or modifications of TLR2 agonists (e.g., lipoproteins).

These data demonstrate that the genomic deletions that modify TLR2, TLR4, and TLR5 signaling in the YS1646Δ*asd*/ΔELG/Δ*pagP*/Δ*ansB*/Δ*csgD* and YS1646Δ*asd*/ΔFLG/Δ*pagP*/Δ*ansB*/Δ*csgD*/Δ*lppAB* deletion strains, largely abrogate the production of systemic pro-inflammatory cytokines, despite robust bacterial tumor colonization. As a result, strains YS1646Δ*asd*/ΔFLG/Δ*pagP*/Δ*ansB*/Δ*csgD* and YS1646*Δasd*/Δ*FLG*/Δ*pagP*/Δ*ansB*/Δ*csgD*/Δ*lppAB* are well-tolerated, and, if needed, can be dosed at higher concentrations than parental strain YS1646, leading to enhanced therapeutic efficacy.

### Serum Levels of IL-6, TNF-α and IFN-γ in Response to IV-Dosing of Tumor-Bearing Mice with Immunostimulatory Bacterial Strains

| **Strain** | **IL-6 (pg/mL)** | | | **TNF-α (pg/mL)** | | | **IFN-γ (pg/mL)** | | |
|---|---|---|---|---|---|---|---|---|---|
| | **D1** | **D4** | **D8** | **D1** | **D4** | **D8** | **D1** | **D4** | **D8** |
| PBS Control | 2.7 | 1.4 | 1.4 | 1.7 | 1.2 | 4.2 | 0.0 | 15.2 | 0.6 |
| YS1646 | 48.7 | 309.1 | 65.9 | 4.1 | 2.1 | 95.6 | 149 | 145.8 | 52.6 |
| YS1646Δ*asd*/ΔFLG/ Δ*pagP*/Δ*ansB*/Δ*csgD* | 77.2 | 28.1 | 26.0 | 3.0 | 1.2 | 17.8 | 0.0 | 9.1 | 25.1 |
| YS1646Δ*asd*/ΔFLG/ Δ*pagP*/Δ*ansB*/Δ*csgD* /Δ*lppAB* | 15.5 | 1.8 | 22.8 | 2.4 | 1.2 | 0.8 | 0.0 | 4.1 | 6.6 |

### Serum Levels of IL-2 and IL-10 in Response to IV-Dosing of Tumor-Bearing Mice with Immunostimulatory Bacterial Strains

| **Strain** | **IL-2 (pg/mL)** | | | **IL-10 (pg/mL)** | | |
|---|---|---|---|---|---|---|
| | **D1** | **D4** | **D8** | **D1** | **D4** | **D8** |
| PBS Control | 0.0 | 25.0 | 14.2 | 0.0 | 1.8 | 1.8 |
| YS1646 | 4.1 | 212.7 | 201.0 | 202.1 | 2.2 | 8.0 |
| YS1646Δ*asd*/ΔFLG/Δ*pagP*/Δ*ansB*/Δ*csgD* | 0.0 | 33.2 | 61.5 | 0.0 | 1.8 | 8.8 |
| YS1646Δ*asd*/Δ*FLG*/Δ*pagP*/Δ*ansB*/Δ*csgD*/ Δ*lppAB* | 0.0 | 15.9 | 22.1 | 0.0 | 1.8 | 1.8 |

These data show that the YS1646Δ*asd*/Δ*FLG*/Δ*pagP*/Δ*ansB*/Δ*csgD* and YS1646Δ*asd*/ΔFLG/Δ*pagP*/Δ*ansB*/Δ*csgD*/Δ*lppAB* deletion strains are rapidly cleared from naive mice following IV dosing, and do not require antibiotic clearance. In naive and tumor-bearing mice, the deletion strains preferentially colonize tumors compared to healthy tissues, and the expression of plasmid-encoded payloads is tumor-specific. Further, unlike the parental YS1646 strain, the YS1646Δ*asd*/ΔFLG/Δ*pagP*/Δ*ansB*/Δ*csgD* and YS1646Δ*asd*/Δ*FLG*/Δ*pagP*/Δ*ansB*/Δ*csgD*/Δ*lppAB* deletion strains do not elicit pro-inflammatory systemic cytokines that are toxic and immunosuppressive, despite robust tumor colonization, indicating that these strains are well-tolerated *in vivo,* and, if necessary, can be dosed at higher concentrations than strain YS1646 (VNP20009), resulting in higher therapeutic efficacy.

### Example 30

### Modifications to the Delivery Plasmid Encoding Immunomodulatory Payloads

Delivery plasmids encode genes and regulatory elements that are required for bacterial fitness and function, as well as for the proper expression of encoded complex polycistronic eukaryotic payloads. The switch between such evolutionarily divergent organisms introduces challenges for proper functioning in both. In bacteria, transcriptional leakiness from eukaryotic promoters, such as the CMV promoter, is observed. A promoter is considered "leaky" where there is always some level of basal transcription in bacteria from the eukaryotic promoter. This promoter leakiness, combined with large eukaryotic genes and regulatory sequences, when encoded (e.g., on plasmids) in bacteria, results in reduced bacterial fitness that manifests in low injection stock viability, and reduced growth rate in broth culture. It was found herein that bacterial fitness was affected by the leakiness from the eukaryotic promoter. The leaky promoter can affect bacterial fitness for several reasons, such as the increased transcriptional/translational load, and due to partial expression of normally secreted eukaryotic proteins that can be toxic to the bacterial cells as they accumulate.

To decrease CMV promoter leakiness and improve bacterial growth and fitness, it is shown herein that bacterial CMV leakiness can be decreased or inhibited, for example, by including bacterial-specific terminator sequences, such as the phage *T4, trpA,* and BBa_B0015 terminators, and/or by placing a strong bacterial promoter in the opposing orientation (*i.e.,* including a reverse promoter). For example, a reverse promoter (a promoter in the antisense orientation), referred to herein as RevMTL*, is a strong promoter that is derived from the MTL promoter (available from Molecular Technologies Laboratories), that is in turn derived from *the proD* promoter (see, *e.g.,* Davis et al. (2011) Nucleic Acids Research 39(3):1131-1141), and which was mutated to remove all antisense ATGs in the sequence, to eliminate or reduce the expression of eukaryotic genes.

It is shown herein that engineered mutations, that alter the predicted bacterial promoter sequences within a eukaryotic promoter, also can be used to generate variants with decreased CMV promoter leakiness. In addition to CMV promoter leakiness, robust *asd* expression readthrough (in *asd⁻* strains containing plasmids incorporating an *asd* complementation system) can cause leakiness through the expression cassette of the eukaryotic payloads. It is shown herein that strategies to block or decrease *asd* expression readthrough include placing the expression cassette in a reverse orientation, and/or inserting a bacterial terminator sequence between the *asd* gene and the payload expression cassette.

To minimize the negative impacts to bacterial fitness, while maintaining high ectopic gene/payload expression in mammalian cells, the delivery plasmid was systematically modified in four distinct ways, as follows:
1) cryptic bacterial promoter sequences, encoded within the CMV promoter enhancer region, were identified using PromoterHunter (available online at phisite.org/promoterhunter/; see, *e.g.,* Klucar et al. (2010) Nucleic Acids Res. 38(Database issue):D366-D370), and putative promoter sequences were then replaced with CREB-binding sites and partial CREB-binding sites to promote efficient plasmid delivery;
2) to inhibit transcriptional leakiness from the CMV promoter, a number of bacterial terminators were inserted into the 5' untranslated region (UTR) of open reading frame (ORF) 1, to inhibit expression in bacteria (see table below);
3) to reduce the level of readthrough transcription from the origin of replication, the orientation of the expression cassette, from upstream of the CMV promoter to the end of the polyadenylation signal, was reversed, with and without a transcription terminator inserted between the expression cassette and the origin of replication; and
4) modifications from among 1)-3) above, that resulted in increased injection stock viability, with enhanced *in vitro* genetic payload expression, were combined for further improvement.

To measure the expression of encoded payloads from bacterially-infected eukaryotic cells, THP-1 human macrophage cells (ATCC catalog # 202165) were infected with strains of S. *typhimurium* YS1646Δ*asd*/Δ*fliC*/Δ*fljB*/Δ*pagP*/Δ*ansB*/Δ*csgD,* containing plasmids encoding a functional *asd* gene to ensure plasmid maintenance, as well as a complex eukaryotic expression cassette (CMV muIL-12p70_T2A_muIL-18 HPRE bGHpolyA + EF-1α muSTING C205Y WPRE SV40polyA), with the modifications discussed above, and listed in the table below. Plasmid ADN-287 contains the eukaryotic expression cassette and no further modifications. Plasmids ADN-397, ADN-398, and ADN-399 each contain one unique CMV cryptic promoter replacement (with a CREB-binding site or partial CREB-binding site); plasmids ADN-400, ADN-401, and ADN-402 each contain two CMV cryptic promoter replacements; and plasmid ADN-403 contains three CMV cryptic promoter replacements. Plasmid ADN-355 contains the eukaryotic expression cassette from plasmid ADN-287, in the reverse orientation; plasmid ADN-358 contains the expression cassette from plasmid ADN-287 with a BBa_B0015 terminator placed downstream of the polyadenylation site, and the expression cassette is in the reverse orientation; plasmid ADN-325 contains the T4 bacterial terminator between the CMV promoter and the ADN-287 expression cassette; plasmid ADN-382 is the same as plasmid ADN-325, but the expression cassette is placed in the reverse orientation; plasmid ADN-373 contains the T4 bacterial terminator between the CMV promoter and the ADN-287 expression cassette, which is followed by the BBa_B0015 terminator, and the expression cassette is in the reverse orientation; plasmid ADN-327 contains the *trpA* bacterial terminator between the CMV promoter and the ADN-287 expression cassette; plasmid ADN-381 is the same as plasmid ADN-327, but the expression cassette is in the reverse orientation; plasmid ADN-372 contains the *trpA* bacterial terminator between the CMV promoter and the ADN-287 expression cassette, which is followed by the BBa_B0015 terminator, and the expression cassette is in the reverse orientation; plasmid ADN-329 contains the RevMTL* promoter (see description above) between the CMV promoter and the ADN-287 expression cassette; plasmid ADN-383 is the same as plasmid ADN-329, but the expression cassette is in the reverse orientation; plasmid ADN-374 contains the RevMTL* promoter between the CMV promoter and the ADN-287 expression cassette, which is followed by the BBa_B0015 terminator, and the expression cassette is in the reverse orientation; and plasmid ADN-328 contains the BBa_B0015 terminator between the CMV promoter and the ADN-287 expression cassette. Plasmid ADN-257 contains the gene encoding muIL12-p70, under control of the CMV promoter, and no further modifications.

5x10⁵ cells were placed in each well of a 24-well dish, with RPMI and 10% FBS. Cells were infected with washed, stationary phase cultures of *S. typhimurium* strain YS1646Δ*asd*/Δ*fliC*/Δ*fljB*/Δ*pagP*/Δ*ansB*/Δ*csgD* (containing one of the plasmids described above and listed in the table below), for 1 hour at an MOI of 200 CFUs per cell, and then the cells were washed with PBS, and the media was replaced with media containing 200 µg/mL of gentamicin to kill extracellular bacteria. After 24 hours, the cells were lysed with 350 µL of Buffer RLT with β-mercaptoethanol (β-ME) (Qiagen), and RNA extraction was performed using the Qiagen RNeasy Mini Kit, with the following modifications. A genomic DNA elimination step, using an RNase-Free DNase kit (Qiagen) was included in the kit to remove genomic DNA from the total RNA. Total RNA concentration was measured using a NanoDrop^{™} One^{c} UV-Vis Spectrophotometer (Thermo Fisher Scientific). RNA was stored at -80 °C without freeze-thawing until reverse-transcription was performed. cDNA synthesis was performed using 0.4-1 µg of template RNA using a C1000 Touch^{™} Thermal Cycler (Bio-Rad) and SuperScript^{™} VILO^{™} Master Mix (Invitrogen) in a 30 µL reaction, according to the manufacturer's instructions.

qPCR (quantitative polymerase chain reaction) was performed with a CFX96^{™} Real-Time PCR Detection System (Bio-Rad). SYBR^{®} primers for murine IL-12 (Assay ID: qMmuCID0015668) were purchased from Bio-Rad. The qPCR reaction (20 µL) was conducted per protocol, using the iTaq^{™} Universal SYBR^{®} Green Supermix (Bio-Rad). The standard thermocycling program on the Bio-Rad CFX96^{™} Real-Time PCR Detection System consisted of a 95 °C denaturation for 30 seconds, followed by 40 cycles of 95 °C for 5 seconds and 60 °C for 30 seconds. Reactions with template-free control were included for each set of primers on each plate. All samples were run in duplicate, and the mean C_{q} values were calculated. Quantification of the target mRNA was normalized using beta-actin reference mRNA (Bio-Rad, qMmuCED0027505). ΔC_{q} was calculated as the difference between target *(i.e.,* muIL-12p70) and reference *(i.e.,* beta-actin) gene. ΔΔC_{q} was obtained by normalizing the ΔC_{q} values of the individual strains, to the ΔC_{q} values of the strain carrying the unmodified plasmid (ADN-287; see table below). Fold expression was calculated as 2^-ΔΔC_{q}.

The doubling time for each of the individual bacterial strains was calculated from broth growth curves, which were performed by diluting OD₆₀₀-normalized stationary cultures to OD₆₀₀ = 0.05, and growing at 37 °C with shaking overnight, and then using a SpectraMax^{®} M3 Spectrophotometer (Molecular Devices) to read the OD₆₀₀ every 15 minutes. Doubling time (G) was derived from the exponential growth phase, using the formula G = t/n, where t= time interval (t_{final}-tₛₜₐᵣₜ) in minutes, and n = (logOD_{600(final)}- logOD₆₀₀₍ₛₜₐᵣₜ₎)/log2.

Injection stocks were prepared by washing a 25 ml overnight stationary culture of each *S. typhimurium* YS1646Δ*asd*/Δ*fliC*/Δ*fljB*/Δ*pagP*/Δ*ansB*/Δ*csgD* strain, grown in 4XYT medium in an ice-cold solution of 10% glycerol in PBS three times, normalizing to an OD₆₀₀ = 2, and flask-freezing 1 ml aliquots in dry ice. Stocks were stored at -80 °C. Injection stock viability was determined by titering thawed injection stock vials, by dividing the colony forming units (CFUs) per vial by the expected CFUs based on OD₆₀₀. The results for the muIL-12p70 expression levels, bacterial doubling times, injection stock viabilities, and average stationary phase ending OD₆₀₀ readings, for the YS1646Δ*asd*/Δ*fliC*/Δ*fljB*/Δ*pagP*/Δ*ansB*/Δ*csgD* strains containing each of the described plasmids (with and without the discussed modifications), are shown in the table below.

### Effects of Plasmid Modifications on Payload Expression Levels and Bacterial Fitness

| **Plasmid** | **Description** | **Average Fold muIL-12 Expression (RT-gPCR)** | **Average Doubling Time (mins)** | **Injection Stock Viability** | **Average Stationary Phase Ending OD₆₀₀** |
|---|---|---|---|---|---|
| **ADN-287** | CMV-muIL-12p70_T2A_muIL-18 + EF-1α_muSTING C205Y | 1.0 | 125 | 22% | 3.62 |
| **ADN-397** | CMV cryptic promoter replacement 1 (ADN-287) | 1.3 | 146 | 23% | 3.54 |
| **ADN-398** | CMV cryptic promoter replacement 2 (ADN-287) | 1.5 | 150 | 23% | 4.14 |
| **ADN-399** | CMV cryptic promoter replacement 3 (ADN-287) | 7.2 | 145 | 22% | 3.78 |
| **ADN-400** | CMV cryptic promoter replacements 1 + 2 (ADN-287) | 1.4 | 136 | 16% | 3.88 |
| **ADN-401** | CMV cryptic promoter replacements 1 + 3 (ADN-287) | 1.3 | 147 | 18% | 4.16 |
| **ADN-402** | CMV cryptic promoter replacements 2 + 3 (ADN-287) | 1.0 | 138 | 26% | 2.58 |
| **ADN-403** | CMV cryptic promoter replacements 1 + 2 + 3 (ADN-287) | 0.6 | 139 | 17% | 3.96 |
| **ADN-355** | (ADN-287 Cassette) Reverse orientation | 2.0 | 110 | 31% | 4.08 |
| **ADN-358** | (ADN-287 Cassette-BBa_B0015 terminator) Reverse orientation | 1.7 | 108 | 24% | 4.22 |
| **ADN-325** | CMV-T4 terminator-ADN-287 Cassette | 0.7 | 96 | 41% | 3.82 |
| **ADN-382** | (CMV-T4 terminator-ADN-287 Cassette) Reverse orientation | 6.1 | 99 | 13% | 3.96 |
| **ADN-373** | **(CMV-T4 terminator-ADN-287 Cassette-BBa_B0015 terminator) Reverse orientation** | 1.5 | 101 | 41% | 3.98 |
| **ADN-327** | CMV*-trpA* terminator-ADN-287 Cassette | 1.4 | 105 | 40% | 3.80 |
| **ADN-381** | (CMV*-trpA* terminator-ADN-287 Cassette) Reverse orientation | 2.5 | 112 | 27% | 2.58 |
| **ADN-372** | (CMV-*trpA* terminator-ADN-287 Cassette-BBa_B0015 terminator) Reverse orientation | 11.6 | 105 | 13% | 3.59 |
| **ADN-329** | CMV-RevMTL* promoter-ADN-287 Cassette | 0.9 | 105 | 36% | 3.92 |
| **ADN-383** | (CMV-RevMTL* promoter-ADN-287 Cassette) Reverse orientation | 1.8 | 121 | 24% | 3.56 |
| **ADN-374** | (CMV-RevMTL* promoter-ADN-287 Cassette-BBa_B0015 terminator) Reverse orientation | 0.8 | 110 | 15% | 3.17 |
| **ADN-328** | CMV-BBa_B0015 terminator-ADN-287 Cassette | 3.8 | 108 | 10% | 2.87 |
| **ADN-257** | CMV- muIL-12p70 | 4.1 | 98 | - | - |

| | | | | | |
|---|---|---|---|---|---|
| RevMTL* is reverse promoter (a promoter in the antisense orientation) that is a strong bacterial promoter, derived from the MTL promoter (Molecular Technologies Laboratories), which is derived from the *proD* promoter, and which was mutated to remove all antisense ATGs to reduce or eliminate the expression of eukaryotic genes. | | | | | |

The results show that, when the expression cassette was reversed on the plasmid and followed by the BBa_B0015 terminator, and the T4 terminator was inserted downstream of the CMV promoter ((CMV-T4 terminator-ADN-287 Cassette-BBa_B0015 terminator) Reverse orientation; see results for plasmid ADN-373), there was an increase in the bacterial cell viability, compared to the strain carrying the parental plasmid, ADN-287 (41% injection stock viability compared to 22%). The strain carrying this plasmid also expressed higher levels of muIL-12p70 *in vitro* (1.5 compared to 1.0), had a reduced doubling time (101 min compared to 125 min), and grew to a higher stationary OD₆₀₀ (3.98 compared to 3.62), compared to the same strain carrying the parental plasmid, ADN-287. No other plasmid resulted in such improvements across all metrics.

Thus, where a plasmid encodes complex polycistronic eukaryotic payloads, the combination of 1) inserting a bacterial terminator, such as the T4 bacterial terminator, downstream of the CMV promoter; 2) following the expression cassette with another terminator, such as the bacterial BBa_B00015 terminator; and 3) reversing the orientation of the eukaryotic expression cassette on the plasmid, increases the efficiency of encoded payload expression and improves bacterial fitness.

Since modifications will be apparent to those of skill in the art, it is intended that this invention be limited only by the scope of the appended claims.

The present disclosure also includes the following items.
1. A nucleic acid construct, comprising nucleic acid encoding a plurality of anti-cancer products as a polycistronic sequence under control of a single promoter.
2. The construct of item 1, wherein the polycistronic sequence comprises a 2A peptide between each open reading frame (ORF) encoding each product.
3. The construct of item 1 or item 2, wherein the anti-cancer products are proteins.
4. The construct of item 2 or item 3, wherein the 2A peptide is one or more of T2A, P2A, E2A, or F2A.
5. The construct of any of items 1-4, wherein the encoded products are one or more immunostimulatory protein(s) that confer(s) or contributes to an anti-tumor immune response in a tumor microenvironment.
6. The construct of item 5, wherein an immunostimulatory protein that confers or contributes to an anti-tumor immune response in the tumor microenvironment is selected from among one or more of: IL-2, IL-7, IL-12p70 (IL-12p40 + IL-12p35), IL-15, IL-2 that has attenuated binding to IL-2Ra, IL-15/IL-15R alpha chain complex, IL-18, IL-21, IL-23, IL-36γ, IL-2 modified so that it does not bind to IL-2Ra, CXCL9, CXCL10, CXCL11, interferon-α, interferon-β, interferon-γ, CCL3, CCL4, CCL5, proteins that are involved in or that effect or potentiate the recruitment and/or persistence of T-cells, CD40, CD40 ligand (CD40L), CD28, OX40, OX40 ligand (OX40L), 4-1BB, 4-1BB ligand (4-1BBL), 4-1BBL that has a cytoplasmic domain deletion or truncation to eliminate immunosuppressive reverse signaling, members of the B7-CD28 family, CD47 antagonists, an anti-IL6 antibody or IL-6 binding decoy receptor, TGF-beta polypeptide antagonists, and members of the tumor necrosis factor receptor (TNFR) superfamily.
7. The construct of item 5, wherein an immunostimulatory protein that confers or contributes to an anti-tumor immune response in the tumor microenvironment is selected from among one or more of: IFN-α, IFN-β, GM-CSF, IL-2, IL-7, IL-12, IL-15, IL-18, IL-21, IL-23, IL-12p70 (IL-12p40 + IL-12p35), IL-15/IL-15R alpha chain complex, IL-36 gamma, IL-2 that has attenuated binding to IL-2Ra, IL-2 that is modified so that it does not bind to IL-2Ra, CXCL9, CXCL10 (IP-10), CXCL11, CCL3, CCL4, CCL5, molecules involved in the potential recruitment and/or persistence of T-cells, CD40, CD40 ligand (CD40L), OX40, OX40 ligand (OX40L), 4-1BB, 4-1BB ligand (4-1BBL), 4-1BBL with a deleted cytoplasmic domain (4-1BBLΔcyt) or with a partially deleted cytoplasmic domain, which the cytoplasmic domain is deleted or truncated to eliminate the immunosuppressive reverse signaling, members of the B7-CD28 family, and members of the tumor necrosis factor receptor (TNFR) superfamily.
8. The construct of any of items 1-7, comprising nucleic acid encoding 4-1BBL with a deleted, or partially deleted, cytoplasmic domain, or a partially deleted cytoplasmic domain and optionally including amino acid modifications, whereby the resulting 4-1BBL assumes the proper orientation when expressed in a cell.
9. The construct of any of items 1-8, comprising nucleic acid encoding a 4-1BBL variant with a deleted or partially deleted cytoplasmic domain, or a modified 4-1BBL with a truncated and modified cytoplasmic domain, wherein the sequence of the 4-1BBL is set forth in SEQ ID NO:390, SEQ ID NO:391, and SEQ ID NO:392.
10. The construct of any of items 1-9, comprising nucleic acid encoding any of the following products and combinations of products:
   one or more of IL-12, or IL-15, or IL12p70, or IL-15/IL-15R alpha chain complex;
   a cytokine and a Stimulator of Interferon Genes (STING) pathway agonist;
   a cytokine, a STING pathway agonist, and either a costimulatory receptor ligand or an immune checkpoint inhibitor;
   a cytokine, a STING pathway agonist, and a TGF-beta polypeptide antagonist;
   a cytokine, a STING pathway agonist, a TGF-beta polypeptide antagonist, and either a co-stimulatory receptor ligand or an immune checkpoint inhibitor,
   wherein a STING pathway agonist is any product that increases type I interferon expression via activation of the STING pathway.
11. The construct of any of items 1-10, comprising nucleic acid encoding a Stimulator of Interferon Genes (STING) polypeptide.
12. The construct of any of items 1-11, comprising nucleic acid encoding a combination of therapeutic products selected from among the following combinations:
   an anti-CTLA-4 antibody and a STING polypeptide,
   IL-15 and a STING polypeptide,
   4-1BBL and a STING polypeptide,
   A TGF-beta receptor decoy or antagonist polypeptide, and a STING polypeptide,
   IL-12 and a STING polypeptide,
   an anti-CTLA-4 antibody, IL-15, and a STING polypeptide,
   4-1BBL, IL-15, and a STING polypeptide,
   A TGF-beta receptor decoy or antagonist polypeptide, and IL-15, and a STING polypeptide,
   an anti-CTLA-4 antibody, and IL-12, and a STING polypeptide,
   4-1BBL, IL-12, and a STING polypeptide,
   a TGF-beta receptor decoy or polypeptide antagonist, IL-12, and a STING polypeptide,
   an anti-CTLA-4 antibody, IL-15, a TGF-beta receptor decoy or polypeptide antagonist, and a STING polypeptide,
   4-1BBL, IL-15, a TGF-beta receptor decoy or polypeptide antagonist, and a STING polypeptide,
   an anti-CTLA-4 antibody, IL-12, a TGF-beta receptor decoy or polypeptide antagonist, and a STING polypeptide,
   4-1BBL, IL-12, a TGF-beta receptor decoy or polypeptide antagonist, and a STING polypeptide,
   an anti-CTLA-4 antibody, IL-12, IL-15, and a STING polypeptide,
   4-1BBL, IL-12, IL-15, and a STING polypeptide,
   a TGF-beta receptor decoy or polypeptide antagonist, IL-12, IL-15, and a STING polypeptide,
   a TGF-beta receptor decoy or polypeptide antagonist, IL-12, IL-15, and a STING polypeptide,
   an anti-CTLA-4 antibody, IL-12, IL-15, a TGF-beta receptor decoy or polypeptide antagonist, and aSTING polypeptide,
   4-1BBL, IL-12, IL-21, a TGF-beta receptor decoy or polypeptide antagonist, and a STING polypeptide,
   an anti-CTLA-4 antibody, IL-12, IL-15, and a TGF-beta receptor decoy or polypeptide antagonist,
   4-1BBL, IL-12, IL-21, and a TGF-beta receptor decoy or polypeptide antagonist,
   IL-12, IL-15, and a STING polypeptide,
   IL-15, IL-21, and a STING polypeptide,
   IL-12, IL-21, and a STING polypeptide,
   an anti-CTLA-4 antibody, IL-15, IL-21, and a STING polypeptide,
   an anti-CTLA-4 antibody, IL-12, IL-21, and a STING polypeptide,
   4-1BBL, IL-15, IL-21, and a STING polypeptide,
   4-1BBL, IL-12, IL-21, and a STING polypeptide,
   an anti-CTLA-4 antibody, and IL-15,
   an anti-CTLA-4 antibody, IL-15, and a TGF-beta receptor decoy or polypeptide antagonist,
   4-1BBL and IL-15,
   4-1BBL, IL-15, and a TGF-beta receptor decoy or polypeptide antagonist,
   an anti-CTLA-4 antibody, and IL-12,
   an anti-CTLA-4 antibody, IL-12, and a TGF-beta receptor decoy or polypeptide antagonist,
   4-1BBL, and IL-12,
   4-1BBL, IL-12, and a TGF-beta receptor decoy or polypeptide antagonist,
   an anti-CTLA-4 antibody, and a TGF-beta receptor decoy or polypeptide antagonist,
   4-1BBL, and a TGF-beta receptor decoy or polypeptide antagonist,
   IL-15, and a TGF-beta receptor decoy or polypeptide antagonist,
   IL-12, and a TGF-beta receptor decoy or polypeptide antagonist,
   IL-12, IL-15, and a TGF-beta receptor decoy or polypeptide antagonist, and
   IL-15, IL-21, and a TGF-beta receptor decoy or polypeptide antagonist, wherein:
      4-1BBL is 4-1BBL with a deleted cytoplasmic domain, 4-1BBL with a modified cytoplasmic domain, 4-1BBL with a truncated cytoplasmic domain, or 4-1BBL with a truncated and modified cytoplasmic domain;
      an anti-CTLA-4 antibody is an scFv or an scFv-Fc; and
      a STING polypeptide is a wild-type STING, or a variant STING polypeptide, or a chimeric STING polypeptide, or a chimeric STING polypeptide with amino acid replacements.
13. The construct of any of items 1-11 that encodes a combination of therapeutic products selected from among:
   IL-2 and IL-12p70;
   IL-2 and IL-21;
   IL-2, IL-12p70, and a STING gain-of-function (GOF) variant;
   IL-2, IL-21, and a STING GOF variant;
   IL-2, IL-12p70, a STING GOF variant, and 4-1BBL (including 4-1BBLΔcyt), where Δcyt is a deleted cytoplasmic domain;
   IL-2, IL-21, a STING GOF variant, and 4-1BBL (including 4-1BBLΔcyt);
   IL-15/IL-15Rα, and a STING GOF variant;
   IL-15/IL-15Rα, a STING GOF variant, and 4-1BBL (including 4-1BBLΔcyt);
   IL-15/IL-15Rα and IL-12p70;
   IL-15/IL-15Rα and IL-21;
   IL-15/IL-15Rα, IL-12p70, and a STING GOF variant;
   IL-15/IL-15Rα, IL-21, and a STING GOF variant;
   IL-15/IL-15Rα, IL-12p70, a STING GOF variant, and 4-1BBL (including 4-1BBLΔcyt);
   IL-15/IL-15Rα, IL-21, a STING GOF variant, and 4-1BBL (including 4-1BBLΔcyt);
   IL-12p70 and IL-21;
   IL-12p70, IL-21, and a STING GOF variant;
   IL-12p70, IL-21, a STING GOF variant, and 4-1BBL (including 4-1BBLΔcyt);
   IL-12p70 and a STING GOF variant;
   IL-12p70, a STING GOF variant, and 4-1BBL (including 4-1BBLΔcyt);
   IL-12p70 and IL-18,
   IL-12p70, IL-18, and a STING GOF variant;
   IL-12p70, IL-18, a STING GOF variant, and 4-1BBL (including 4-1BBLΔcyt);
   a TGF-β decoy receptor or a TGF-β polypeptide antagonist, IL-2, and IL-12p70;
   a TGF-β decoy receptor or a TGF-β polypeptide antagonist, IL-2, and IL-21,
   a TGF-β decoy receptor or a TGF-β polypeptide antagonist, IL-2, IL-12p70, and a STING GOF variant;
   a TGF-β decoy receptor or a TGF-β polypeptide antagonist, IL-2, IL-21, and a STING GOF variant;
   a TGF-β decoy receptor or a TGF-β polypeptide antagonist, IL-2, IL-12p70, a STING GOF variant, and 4-1BBL (including 4-1BBLΔcyt);
   a TGF-β decoy receptor or a TGF-β polypeptide antagonist, IL-2, IL-21, a STING GOF variant, and 4-1BBL (including 4-1BBLΔcyt);
   a TGF-β decoy receptor or a TGF-β polypeptide antagonist, IL-15/IL-15Rα, and a STING GOF variant;
   a TGF-β decoy receptor or a TGF-β polypeptide antagonist, IL-15/IL-15Rα, a STING GOF variant, and 4-1BBL (including 4-1BBLΔcyt);
   a TGF-β decoy receptor or a TGF-β polypeptide antagonist, IL-15/IL-15Rα, and IL-12p70;
   a TGF-β decoy receptor or a TGF-β polypeptide antagonist, IL-15/IL-15Rα, and IL-21;
   a TGF-β decoy receptor or a TGF-β polypeptide antagonist, IL-15/IL-15Rα, IL-12p70, and a STING GOF variant;
   a TGF-β decoy receptor or a TGF-β polypeptide antagonist, IL-15/IL-15Rα, IL-21, and a STING GOF variant;
   a TGF-β decoy receptor or a TGF-β polypeptide antagonist, IL-15/IL-15Rα, IL-12p70, a STING GOF variant, and 4-1BBL (including 4-1BBLΔcyt);
   a TGF-β decoy receptor or a TGF-β polypeptide antagonist, IL-15/IL-15Rα, IL-21, a STING GOF variant, and 4-1BBL (including 4-1BBLΔcyt);
   a TGF-β decoy receptor or a TGF-β polypeptide antagonist, IL-12p70, and IL-21;
   a TGF-β decoy receptor or a TGF-β polypeptide antagonist, IL-12p70, IL-21, and a STING GOF variant;
   a TGF-β decoy receptor or a TGF-β polypeptide antagonist, IL-12p70, IL-21, a STING GOF variant, and 4-1BBL (including 4-1BBLΔcyt);
   a TGF-β decoy receptor or a TGF-β polypeptide antagonist, and IL-12p70;
   a TGF-β decoy receptor or a TGF-β polypeptide antagonist, IL-12p70, and a STING GOF variant;
   a TGF-β decoy receptor or a TGF-β polypeptide antagonist, IL-12p70, a STING GOF variant, and 4-1BBL (including 4-1BBLΔcyt);
   a TGF-β decoy receptor or a TGF-β polypeptide antagonist, IL-12p70, and IL-18;
   a TGF-β decoy receptor or a TGF-β polypeptide antagonist, IL-12p70, IL-18, and a STING GOF variant;
   a TGF-β decoy receptor or a TGF-β polypeptide antagonist, IL-12p70, IL-18, a STING GOF variant, and 4-1BBL (including 4-1BBLΔcyt);
   a TGF-β decoy receptor or a TGF-β polypeptide antagonist, and a STING GOF variant;
   an anti-CTLA-4 antibody, IL-2, and IL-12p70;
   an anti-CTLA-4 antibody, IL-2, and IL-21;
   an anti-CTLA-4 antibody, IL-2, IL-12p70, and a STING GOF variant;
   an anti-CTLA-4 antibody, IL-2, IL-21, and a STING GOF variant;
   an anti-CTLA-4 antibody, IL-2, IL-12p70, a STING GOF variant, and 4-1BBL (including 4-1BBLΔcyt);
   an anti-CTLA-4 antibody, IL-2, IL-21, a STING GOF variant, and 4-1BBL (including 4-1BBLΔcyt);
   an anti-CTLA-4 antibody, IL-15/IL-15Rα, and a STING GOF variant;
   an anti-CTLA-4 antibody, IL-15/IL-15Rα, a STING GOF variant, and 4-1BBL (including 4-1BBLΔcyt);
   an anti-CTLA-4 antibody, IL-15/IL-15Rα, and IL-12p70;
   an anti-CTLA-4 antibody, IL-15/IL-15Rα, and IL-21;
   an anti-CTLA-4 antibody, IL-15/IL-15Rα, IL-12p70, and a STING GOF variant;
   an anti-CTLA-4 antibody, IL-15/IL-15Rα, IL-21, and a STING GOF variant;
   an anti-CTLA-4 antibody, IL-15/IL-15Rα, IL-12p70, a STING GOF variant, and 4-1BBL (including 4-1BBLΔcyt);
   an anti-CTLA-4 antibody, IL-15/IL-15Rα, IL-21, a STING GOF variant, and 4-1BBL (including 4-1BBLΔcyt);
   an anti-CTLA-4 antibody, IL-12p70, and IL-21;
   an anti-CTLA-4 antibody, IL-12p70, IL-21, and a STING GOF variant;
   an anti-CTLA-4 antibody, IL-12p70, IL-21, a STING GOF variant, and 4-1BBL (including 4-1BBLΔcyt);
   an anti-CTLA-4 antibody and IL-12p70;
   an anti-CTLA-4 antibody, IL-12p70, and a STING GOF variant;
   an anti-CTLA-4 antibody, IL-12p70, a STING GOF variant, and 4-1BBL (including 4-1BBLΔcyt);
   an anti-CTLA-4 antibody, IL-12p70, and IL-18,
   an anti-CTLA-4 antibody, IL-12p70, IL-18, and a STING GOF variant;
   an anti-CTLA-4 antibody, IL-12p70, IL-18, a STING GOF variant, and 4-1BBL (including 4-1BBLΔcyt);
   an anti-CTLA-4 antibody and a STING GOF variant;
   a CD40 agonist, IL-2, and IL-12p70;
   a CD40 agonist, IL-2, and IL-21;
   a CD40 agonist, IL-2, IL-12p70, and a STING GOF variant;
   a CD40 agonist, IL-2, IL-21, and a STING GOF variant;
   a CD40 agonist, IL-2, IL-12p70, a STING GOF variant, and 4-1BBL (including 4-1BBLΔcyt);
   a CD40 agonist, IL-2, IL-21, a STING GOF variant, and 4-1BBL (including 4-1BBLΔcyt);
   a CD40 agonist, IL-15/IL-15Rα, and a STING GOF variant;
   a CD40 agonist, IL-15/IL-15Rα, a STING GOF variant, and 4-1BBL (including 4-1BBLΔcyt);
   a CD40 agonist, IL-15/IL-15Rα, and IL-12p70;
   a CD40 agonist, IL-15/IL-15Rα, and IL-21;
   a CD40 agonist, IL-15/IL-15Rα, IL-12p70, and a STING GOF variant;
   a CD40 agonist, IL-15/IL-15Rα, IL-21, and a STING GOF variant;
   a CD40 agonist, IL-15/IL-15Rα, IL-12p70, a STING GOF variant, and 4-1BBL (including 4-1BBLΔcyt);
   a CD40 agonist, IL-15/IL-15Rα, IL-21, a STING GOF variant, and 4-1BBL (including 4-1BBLΔcyt);
   a CD40 agonist, IL-12p70, and IL-21;
   a CD40 agonist, IL-12p70, IL-21, and a STING GOF variant;
   a CD40 agonist, IL-12p70, IL-21, a STING GOF variant, and 4-1BBL (including 4-1BBLΔcyt);
   a CD40 agonist and IL-12p70;
   a CD40 agonist, IL-12p70, and a STING GOF variant;
   a CD40 agonist, IL-12p70, a STING GOF variant, and 4-1BBL (including 4-1BBLΔcyt);
   a CD40 agonist, IL-12p70, and IL-18;
   a CD40 agonist, IL-12p70, IL-18, and a STING GOF variant;
   a CD40 agonist, IL-12p70, IL-18, a STING GOF variant, and 4-1BBL (including 4-1BBLΔcyt); and
   a CD40 agonist and a STING GOF variant, wherein:
      4-1BBL is 4-1BBL with a deleted cytoplasmic domain (4-1BBLΔcyt), 4-1BBL with a modified cytoplasmic domain, 4-1BBL with a truncated cytoplasmic domain, or 4-1BBL with a truncated and modified cytoplasmic domain; and
      an anti-CTLA-4 antibody is an scFv or an scFv-Fc,
14. The construct of any of items 1-13, wherein:
   the STING polypeptide is modified to result in increased or constitutive expression of a type I interferon (IFN), or is a chimeric polypeptide comprising a human STING polypeptide with a C-terminal tail (CTT) from a different species that has lower NF-κB signaling activity than the NF-κB signaling activity of human STING, and wherein:
   the TRAF6 binding site in the CTT optionally is deleted; and
   the human STING protein has the sequence set forth in any of SEQ ID NOs:305-309.
15. The construct of any of items 1-14, wherein the encoded therapeutic proteins comprise IL-12p70, and a chimeric human STING polypeptide with a CTT from Tasmanian devil and an amino acid replacement that results in increased or constitutive expression of type I interferon, or is a STING polypeptide with an amino acid replacement that results in increased or constitutive expression of type I interferon, where a mutation that results in increased or constitutive expression of type I interferon is a gain-of-function (GOF) mutation.
16. The construct of item 15, wherein the amino replacement in the STING polypeptide corresponds to R284G with reference, for alignment, to any of SEQ ID NOs: 305-309.
17. The construct of any of items 11-16, wherein the STING polypeptide further comprises the replacement N154S.
18. The construct of any of items 1-17, comprising nucleic acid encoding IL-36γ.
19. The construct of any of items 1-18 that encodes an immune checkpoint inhibitor antibody, or an antigen-binding portion thereof.
20. The construct of item 19, wherein the immune checkpoint is CTLA-4, or PD-1, or PD-L1.
21. The construct of any of items 1-20, wherein the encoded product is an antibody that is scFv, or that is an scFv-Fc two-chain polypeptide.
22. A plasmid, comprising the construct of any of items 1-21.
23. The plasmid of item 22 that is a bacterial plasmid, wherein the construct is operatively linked to eukaryotic transcriptional regulatory sequences.
24. The plasmid of item 23, wherein the transcriptional regulatory sequences comprise a eukaryotic promoter.
25. A composition, comprising the mixture of anti-cancer protein products encoded by the construct or plasmid of any of items 1-24.
26. An immunostimulatory bacterium, comprising the construct or plasmid of any of items 1-24.
27. The immunostimulatory bacterium of item 26, wherein the genome of the bacterium is modified so that the resulting bacterium is *msbB⁻*/*purI⁻.*
28. The immunostimulatory bacterium of item 26 or item 27, wherein the bacterium is *msbB⁻* and *purI⁻,* whereby the full length of at least the coding portion of the *msbB⁻* and *purI⁻* genes is deleted.
29. The immunostimulatory bacterium of any of items 26-28, wherein the genome of the bacterium is modified, whereby the bacterium lacks flagella.
30. The immunostimulatory bacterium of any of items 26-29 that comprises genome modifications, whereby the bacterium is *msbB⁻*/*pagP⁻.*
31. The immunostimulatory bacterium of any of items 26-30 that comprises genome modifications, whereby the bacterium does not express L-asparaginase II, so that the bacterium is *ansB⁻,*
32. An immunostimulatory bacterium, comprising a plasmid encoding a plurality of therapeutic products, wherein the genome of the bacterium is modified so that the bacterium is *msbB⁻* and *purI⁻,* whereby the full length of at least the coding portion of the *msbB* and *purI* genes is deleted.
33. An immunostimulatory bacterium, comprising a plasmid encoding a therapeutic product under control of a eukaryotic promoter, wherein the genome of the immunostimulatory bacterium is modified by deletion or disruption of all or of a sufficient portion of a gene or genes, whereby the bacterium does not activate the synthesis of secreted asparaginase.
34. The immunostimulatory bacterium of item 33, wherein the asparaginase is L-asparaginase II, encoded by the gene *asnB.*
35. The immunostimulatory bacterium of item 34 that has genome modifications, whereby the bacterium lacks flagella, and is *pagP⁻, ansB⁻,* and *csgD⁻*
36. The immunostimulatory bacterium of item 35 that is Δ*asd*/Δ*FLG*/Δ*pagP*/Δ*ansB*/Δ*csgD.*
37. The immunostimulatory bacterium of any of items 33-36 that has genome modifications, whereby the bacterium is *msbB⁻.*
38. The immunostimulatory bacterium of any of items item 33-37 that has genome modifications, whereby the bacterium is an adenosine auxotroph, or is an adenosine and adenine auxotroph.
39. The immunostimulatory bacterium of any of items item 33-38 that has genome modifications, whereby the bacterium is *purI⁻.*
40. The immunostimulatory bacterium of any of items 33-39, wherein the plasmid encodes aspartate-semialdehyde dehydrogenase (*asd*)*.*
41. The immunostimulatory bacterium of any of items 33-40 that is *purI⁻.*
42. An immunostimulatory bacterium, comprising a plasmid encoding a plurality of therapeutic products under control of a eukaryotic promoter, wherein:
   the genome of the immunostimulatory bacterium is modified by deletion or disruption of all or of a sufficient portion of a gene or genes, whereby the bacterium has been modified to generate penta-acylated lipopolysaccharide (LPS);
   hexa-acylated lipopolysaccharide is substantially reduced, by at least 10-fold, compared to the wild-type bacterium, or is absent; and
   the plasmid encodes a plurality of complementary anti-cancer therapeutic products under control of a single promoter, wherein each of the complementary therapeutic products treats a different aspect of a cancer, or acts via a different mechanism, so that the combined effect is at least additive of the effect of each product separately.
43. An immunostimulatory bacterium, comprising a plasmid encoding a therapeutic product under control of a eukaryotic promoter, wherein:
   the genome of the immunostimulatory bacterium is modified by deletion or disruption of all or of a sufficient portion of a gene or genes, whereby the bacterium has attenuated recognition by TLR2, TLR4, and TLR5; and
   the plasmid encodes a plurality of complementary therapeutic products under control of a single promoter,
44. An immunostimulatory bacterium, comprising a plasmid encoding a therapeutic product under control of a eukaryotic promoter, wherein:
   the genome of the immunostimulatory bacterium is modified by deletion or disruption of all or of a sufficient portion of a gene or genes, whereby the bacterium does not activate the synthesis of curli fimbriae and/or cellulose; and
   the plasmid encodes a plurality of complementary therapeutic products under control of a single promoter.
45. An immunostimulatory bacterium, comprising a plasmid encoding a therapeutic product under control of a eukaryotic promoter, wherein:
   the genome of the immunostimulatory bacterium is modified by deletion or disruption of all or of a sufficient portion of a gene or genes, whereby the bacterium is auxotrophic for purines, adenosine, or ATP, and is modified by deletion or disruption of all or of a sufficient portion of a gene or genes, whereby the bacterium lacks flagella; and
   the plasmid encodes a plurality of therapeutic products under control of a single eukaryotic promoter.
46. An immunostimulatory bacterium, comprising a plasmid encoding a therapeutic product under control of a eukaryotic promoter, wherein the genome of the immunostimulatory bacterium is modified by deletion or disruption of all or of a sufficient portion of a gene or genes, whereby the bacterium lacks flagella; and
   the plasmid encodes a plurality of therapeutic products under control of a single promoter.
47. An immunostimulatory bacterium, comprising a plasmid encoding a therapeutic product under control of a eukaryotic promoter, wherein the genome of the immunostimulatory bacterium is modified by deletion or disruption of all or of a sufficient portion of a gene or genes, whereby the bacterium has been modified to specifically infect tumor-resident myeloid cells; and
   the plasmid encodes a plurality of therapeutic products under control of a single promoter.
48. An immunostimulatory bacterium, comprising a plasmid encoding a therapeutic product under control of a eukaryotic promoter, wherein the genome of the immunostimulatory bacterium is modified by deletion or disruption of all or of a sufficient portion of a gene or genes, whereby the bacterium has been modified to specifically infect tumor-resident myeloid cells, and is unable to replicate in tumor-resident myeloid cells.
49. An immunostimulatory bacterium, comprising a plasmid encoding a therapeutic product under control of a eukaryotic promoter, wherein the genome of the immunostimulatory bacterium comprises two or more modifications selected from among:
   a) deletion or disruption of all or of a sufficient portion of a gene or genes, whereby the bacterium has been modified to generate penta-acylated lipopolysaccharide, wherein:
      the genome of the immunostimulatory bacterium is modified by deletion or disruption of all or of a sufficient portion of a gene or genes, whereby the bacterium has been modified to generate penta-acylated lipopolysaccharide; and
      hexa-acylated lipopolysaccharide is substantially reduced, by at least 10-fold compared to the wild-type bacterium, or is absent;
   b) deletion or disruption of all or of a sufficient portion of a gene or genes, whereby the bacterium has attenuated recognition by one or more of TLR2, TLR4, and TLR5;
   c) deletion or disruption of all or of a sufficient portion of a gene or genes, whereby the bacterium does not activate the synthesis of curli fimbriae and/or cellulose;
   d) deletion or disruption of all or of a sufficient portion of a gene or genes, whereby the bacterium does not activate the synthesis of secreted asparaginase;
   e) deletion or disruption of all or of a sufficient portion of a gene or genes, whereby the bacterium is auxotrophic for purines, adenosine, or ATP;
   f) deletion or disruption of all or of a sufficient portion of a gene or genes, whereby the bacterium lacks flagella;
   g) deletion or disruption of all or of a sufficient portion of a gene or genes, whereby the bacterium has been modified to specifically infect tumor-resident myeloid cells;
   h) deletion or disruption of all or of a sufficient portion of a gene or genes, whereby the bacterium has been modified to specifically infect tumor-resident myeloid cells, and is unable to replicate in tumor-resident myeloid cells; and
   i) deletion or disruption of either or both of *lppA* and *IppB* to decrease or eliminate lipoprotein expression in the membrane, whereby expression of an encoded therapeutic protein is increased in the tumor microenvironment and/or in tumor-resident immune cells.
50. The immunostimulatory bacterium of item 49 comprising modifications a), d) and f), or comprising modifications c) and d).
51. The immunostimulatory bacterium of item 49, comprising modifications a), c), d), e) and f).
52. The immunostimulatory bacterium of item 49, comprising modifications a), c), d), e), f) and i), or comprising modifications a), d) f), and i), or comprising modifications c), d) and i), or comprising modifications f) and i), or comprising modifications a)-i).
53. The immunostimulatory bacterium of item 49, comprising modifications a), b), d) and f), or comprising modifications a), b), c) and d).
54. The immunostimulatory bacterium of any of items 26-53, wherein *lppA* and *lppB* are deleted.
55. The immunostimulatory bacterium of any of items 26-54 that lacks flagella and is *msbB⁻*/*pagP⁻,* wherein the plasmid encodes a combination of two or more therapeutic proteins selected from among a co-stimulatory molecule, a cytokine, a STING pathway agonist, and an immune checkpoint inhibitor antibody.
56. An immunostimulatory bacterium, comprising a plasmid encoding a therapeutic product, wherein infection of a macrophage by the bacterium converts a human M2 macrophage to an M1 phenotype macrophage.
57. An immunostimulatory bacterium, comprising a plasmid encoding a therapeutic product, wherein:
   the immunostimulatory bacterium comprises modifications in its genome, whereby the bacterium infects tumor-resident macrophages, and does not infect epithelial cells; and
   expression of the therapeutic product in a macrophage converts human M2 macrophages to an M1 or M1-like phenotype.
58. The immunostimulatory bacterium of any of items 26-57, comprising a plasmid encoding a therapeutic product, where expression of the therapeutic product in a macrophage converts human M2 macrophages to an M1 or M1-like phenotype.
59. The immunostimulatory bacterium of any of items 26-58 wherein the therapeutic product is part of a cytosolic DNA/RNA sensor pathway that leads to expression of type I interferon (IFN).
60. The immunostimulatory bacterium of item 59, wherein the expression of the type I IFN is constitutive.
61. The immunostimulatory bacterium of item 59 or item 60, wherein the therapeutic product is a gain-of-function (GOF) variant of the therapeutic product that is part of the cytosolic DNA/RNA sensor pathway, wherein the GOF variant product does not require cytosolic nucleic acids, nucleotides, dinucleotides, or cyclic dinucleotides to result in expression of type I IFN.
62. The immunostimulatory bacterium of any of items 26-61, wherein the therapeutic product is a variant STING protein.
63. The immunostimulatory bacterium of any of items 26-62, wherein infection by the bacterium converts human M2 macrophages into M1-like, type I IFN producing cells.
64. The immunostimulatory bacterium of any of items 26-63 that lacks flagella, wherein the wild-type bacterium has flagella, and the immunostimulatory bacteriaum is *pagP⁻*/*msbB⁻.*
65. The immunostimulatory bacterium of any of items 26-64, wherein the immunostimulatory bacterium contains genome modifications, whereby the bacterium lacks flagella and does not produce L-asparaginase II (*ansB*).
66. The immunostimulatory bacterium of item 65, wherein the immunostimulatory bacterium also has genome modifications whereby the bacterium is *msbB⁻* and*pagP⁻.*
67. The immunostimulatory bacterium of any of items 26-66, wherein immunostimulatory bacterium has modifications of the genome, whereby the bacterial phenotype conferred by the genome is Δ*asd*/ΔFLG/Δ*pagP*/Δ*ansB*/Δ*csgD* or Δ*asd*/ΔFLG/Δ*pagP*/Δ*ansB*/Δ*csgD*/Δ*msbB*/Δ*purI,* wherein the plasmid optionally encodes aspartate-semialdehyde dehydrogenase (*asd*)*.*
68. The immunostimulatory bacterium of any of items 26-67, comprising a plasmid that encodes a STING polypeptide.
69. The immunostimulatory bacterium of item 68, wherein the STING polypeptide is a variant STING polypeptide that results in increased or constitutive expression of type I interferon.
70. The immunostimulatory bacterium of item 69, wherein the STING polypeptide is a human chimeric STING polypeptide that comprises a C-terminal tail (CTT) from Tasmanian devil STING.
71. The immunostimulatory bacterium of item 69 or item 70, wherein the STING polypeptide comprises the replacement corresponding to R284, with reference, for alignment, to any of SEQ ID NOs:305-309.
72. The immunostimulatory bacterium of item 71, further comprising a replacement corresponding to N154S, with reference to any of SEQ ID NOs:305-309.
73. The immunostimulatory bacterium of any of items 26-72, comprising a plasmid encoding one or more of IL-12, IL-15, and IL-21.
74. The immunostimulatory bacterium of any of items 26-73 that is a *Salmonella* strain.
75. The immunostimulatory bacterium of any of items 26-74, wherein the therapeutic product is an anti-cancer therapeutic.
76. The immunostimulatory bacterium of any of items 26-75, wherein the bacterium encodes a therapeutic product that is an immunostimulatory protein.
77. The immunostimulatory bacterium of item 76, wherein the immunostimulatory protein is a Stimulator of Interferon Genes (STING) protein, a modified STING protein, a cytokine, a chemokine, or a co-stimulatory receptor or ligand.
78. The immunostimulatory bacterium of any of items 26-77, wherein the bacterium comprises a genomic modification whereby it lacks flagella.
79. The immunostimulatory bacterium of any of items 26-78, wherein the bacterium comprises a genomic modification whereby it is *pagP⁻* or *msbB⁻*/*pagP⁻*.
80. The immunostimulatory bacterium of any of items 26-79, wherein:
   the bacterium comprises genomic modifications whereby it does not express asparaginase or activate the synthesis of secreted asparaginase; and/or
   the genome of the immunostimulatory bacterium is modified by deletion or disruption of all or of a sufficient portion of the gene *ansB* encoding L-asparaginase II, whereby the bacterium is *ansB⁻* and does not express active L-asparaginase II.
81. An immunostimulatory bacterium, comprising a plasmid encoding a therapeutic product under control of a eukaryotic promoter, wherein the genome of the immunostimulatory bacterium is modified by deletion or disruption of all or of a sufficient portion of *csgD,* whereby the bacterium is *csgD⁻,* and does not activate the synthesis of curli fimbriae.
82. An immunostimulatory bacterium, comprising a plasmid encoding a therapeutic product under control of a eukaryotic promoter, wherein the genome of the immunostimulatory bacterium is modified by deletion or disruption of all or of a sufficient portion of the gene *ansB,* encoding L-asparaginase II, whereby the bacterium is *ansB⁻* and does not express active L-asparaginase II.
83. An immunostimulatory bacterium, comprising a plasmid encoding a therapeutic product under control of a eukaryotic promoter, wherein the genome of the immunostimulatory bacterium is modified by deletion or disruption of all or of a sufficient portion of the gene *ansB,* encoding L-asparaginase II, and by deletion or disruption of all or of a sufficient portion of the gene *csgD,* whereby the bacterium is *ansB⁻* and does not express active L-asparaginase II, and is *csgD⁻* and does not activate the synthesis of curli fimbriae.
84. The immunostimulatory bacterium of any of items 26-83, further comprising deletion of or disruption of all or of a sufficient portion of the genes encoding the flagella, whereby the bacterium is flagellin⁻ (*fliC⁻*/*fljB⁻*) and does not produce flagella, wherein the wild-type bacterium has flagella.
85. An immunostimulatory bacterium, comprising a plasmid encoding a therapeutic product under control of a eukaryotic promoter, wherein the genome of the immunostimulatory bacterium is modified by deletion of or disruption of all or of a sufficient portion of the genes *lppA* and *lppB,* encoding the bacterial lipoprotein, whereby the lipoprotein is not produced, and wherein the therapeutic product encoded on the plasmid is an anti-cancer therapeutic.
86. The immunostimulatory bacterium of item 85, wherein the genome of the bacterium is further modified by deletion or disruption of all or of a sufficient portion of the gene *csgD,* whereby the bacterium is *csgD⁻,* or has another or additional modification in the genome whereby biofilm formation is impaired.
87. The immunostimulatory bacterium of any of items 82-86, wherein the genome of the bacterium is further modified by deletion or disruption of all or of a sufficient portion of genes, whereby the bacterium is *csgD⁻*/*msbB⁻*/*pagP⁻.*
88. The immunostimulatory bacterium of any of items 82, 83, and 85-87, wherein the bacterium comprises modifications of the genome, whereby the bacterium lacks flagella.
89. An immunostimulatory bacterium, comprising genome modifications whereby the bacterium lacks flagella, and is *lppA⁻*/*lppB⁻,* and optionally is *csgD⁻,* or lacks flagella, is *ansB⁻,* and optionally is *csgD⁻.*
90. The immunostimulatory bacterium of any of items 26-89, wherein the bacterium is auxotrophic for purines.
91. The immunostimulatory bacterium of any of items 26-89, wherein the bacterium is auxotrophic for adenosine.
92. The immunostimulatory bacterium of any of items 26-89 that is auxotrophic for adenosine, adenine, and ATP.
93. The immunostimulatory bacterium of any of items 26-92, wherein the bacterium is *purI⁻.*
94. The immunostimulatory bacterium of any of items 26-93, wherein the bacterium is *pagP⁻.*
95. The immunostimulatory bacterium of any of items 26-94, wherein the bacterium is *asd⁻.*
96. The immunostimulatory bacterium of any of items 26-95 that is aspartate-semialdehyde dehydrogenase- (*asd⁻*), wherein the bacterium is *asd⁻* by virtue of disruption of or deletion of all or a portion of the endogenous gene encoding aspartate-semialdehyde dehydrogenase (*asd*)*,* whereby endogenous *asd* is not expressed.
97. The immunostimulatory bacterium of any of items 26-96 that encodes aspartate-semialdehyde dehydrogenase (*asd*) on the plasmid under control of a bacterial promoter.
98. The immunostimulatory bacterium of any of items 26-97, wherein the bacterium is *msbB⁻.*
99. The immunostimulatory bacterium of any of items 26-98 that comprises genome modifications, whereby the bacterium is *asd⁻, purI⁻, msbB⁻,* flagellin⁻, and *pagP⁻,* wherein the wild-type bacterium has flagella.
100. The immunostimulatory bacterium of any of items 26-98 that is *asd⁻, csgD⁻, purI⁻, msbB⁻,* flagellin⁻ and *pagP⁻,* wherein the wild-type bacterium has flagella.
101. The immunostimulatory bacterium of any of items 26-98 that is *ansB⁻ , asd⁻, csgD⁻, purI⁻, msbB⁻,* flagellin⁻ (*fliC⁻*/*fljB⁻*), and *pagP⁻,* wherein the wild-type bacterium has flagella.
102. The immunostimulatory bacterium of any of items 96-100, wherein the bacterium is flagellin⁻ (*fliC⁻*/*fljB⁻*), wherein the wild-type bacterium has flagella.
103. The immunostimulatory bacterium of any of items 26-102, wherein the genome of the bacterium is modified by deletion or disruption of all or of a sufficient portion of the gene *lppA* and/or *lppB,* whereby the bacterium is *lppA⁻* and/or *lppB⁻,* whereby expression in the tumor microenvironment and/or tumor-resident macrophages of the therapeutic protein encoded on the plasmid is increased, compared to the same immunostimulatory bacterium except with intact *lppA* and/or *lppB.*
104. An immunostimulatory bacterium, comprising a plasmid encoding a therapeutic product under control of a eukaryotic promoter, wherein the genome of the immunostimulatory bacterium is modified by deletion or disruption of all or of a sufficient portion of a gene or genes, whereby the bacterium is *ansB⁻, asd⁻, csgD⁻, purI⁻, msbB⁻,* flagellin⁻, and *pagP⁻,* wherein the wild-type bacterium has flagella.
105. An immunostimulatory bacterium, comprising a plasmid encoding a therapeutic product under control of a eukaryotic promoter, wherein the genome of the immunostimulatory bacterium is modified by deletion or disruption of all or of a sufficient portion of a gene or genes, whereby the bacterium is *ansb⁻, asd⁻, csgD⁻, purI⁻, msbB⁻,* flagellin⁻, wherein the wild-type bacterium has flagella, and *pagP⁻.*
106. The immunostimulatory bacterium of item 104 or item 105, wherein the bacterium is flagellin⁻ (*fliC⁻*/*fljB⁻*).
107. The immunostimulatory bacterium of item 105 or 106, wherein the bacterial genome comprises further modifications, whereby the bacterium is *ansB⁻, asd⁻, csgD⁻, purI⁻, msbB⁻,* flagellin⁻ (*fliC⁻*/*fljB⁻*)*, pagP , lppA⁻,* and *lppB⁻.*
108. The immunostimulatory bacterium of any of items 26-107, wherein the therapeutic product is an anti-cancer therapeutic.
109. The immunostimulatory bacterium of any of items 26-108, wherein nucleic acid encoding the therapeutic product is operably linked to nucleic acid encoding a secretion signal, whereby, when expressed, the therapeutic product is secreted.
110. The immunostimulatory bacterium of any of items 26-109, wherein one or more genes or operons involved in SPI-1 invasion are deleted or inactivated, whereby the immunostimulatory bacterium does not invade or infect epithelial cells.
111. The immunostimulatory bacterium of item 110, wherein one or more of *avrA, hilA, hilD, invA, invB, invC, invE, invF, invG, invH, invI, invJ, iacP, iagB, spaO, spaQ, spaR, spas, orgA, orgB, orgC, prgH, prgI, prgJ, prgK, sicA, sicP, sipA, sipB, sipC, sipD, sirC, sopB, sopD, sopE, sopE2, sprB,* and *sptP* is deleted or inactivated.
112. The immunostimulatory bacterium of any of items 26-111, wherein the plasmid is present in low copy number, or in medium copy number.
113. The immunostimulatory bacterium of any of items 26-111, wherein the plasmid comprises a medium-to-low copy number origin of replication.
114. The immunostimulatory bacterium of any of items 26-111, wherein the plasmid comprises a low copy number origin of replication.
115. The immunostimulatory bacterium of any of items 26-111 and 114, wherein the plasmid is present in low copy number.
116. The immunostimulatory bacterium of any of items 26-111, wherein the plasmid is present in a copy number of 150 or less.
117. The immunostimulatory bacterium of any of items 26-111, where in the plasmid is present in a copy number greater than 150.
118. The immunostimulatory bacterium of any of items 26-111, wherein the plasmid is present in high copy number.
119. The immunostimulatory bacterium of any of items 26-111, wherein the plasmid is present in medium copy number, which is between 20 and 150, inclusive, is less than 150 or less than about 150 and more than 20 or about 20, or is between 20 and 150 copies.
120. The immunostimulatory bacterium of any of items 26-111, wherein the number of copies of the plasmid is greater than 150.
121. The immunostimulatory bacterium of item 116, wherein the number of copies of the plasmid is 150 copies or fewer, or is less than or equal to 150.
122. The immunostimulatory bacterium of any of items 26-115, wherein the plasmid is present in low copy number, and low copy number is less than 25, or less than 20, or less than about 25, or less than about 20 copies.
123. The immunostimulatory bacterium of any of items 26-122, wherein the therapeutic product is a nucleic acid or a protein.
124. The immunostimulatory bacterium of item 123, wherein the therapeutic product is a protein.
125. The immunostimulatory bacterium of any of items 26-124, wherein the plasmid encodes two or more therapeutic products.
126. The immunostimulatory bacterium of any of items 26-125, wherein the encoded therapeutic product(s) on the plasmid is/are anti-cancer treatment(s).
127. The immunostimulatory bacterium of any of items 26-126, wherein the bacterium encodes two or more products selected from among a cytokine, a protein that constitutively induces a type I IFN, and a co-stimulatory receptor or molecule.
128. The immunostimulatory bacterium of item 127, wherein the co-stimulatory molecule lacks a cytoplasmic domain, or has a modified truncated cytoplasmic domain to ensure that the encoded co-stimulatory molecule is expressed in the correct orientation in a cell, or has a truncated cytoplasmic domain whereby immunosuppressive reverse signaling is eliminated or reduced.
129. The immunostimulatory bacterium of any of items 26-128, wherein the nucleic acid encoding one or more of the therapeutic product or products comprises nucleic acid encoding a signal for secretion of the therapeutic product(s) from a cell comprising the bacterium or the plasmid.
130. The immunostimulatory bacterium of any of items 26-129, wherein the nucleic acid encoding the product on the plasmid is operatively linked to regulatory sequences recognized by a eukaryotic host.
131. The immunostimulatory bacterium of any of items 26-130, wherein the immunostimulatory bacterium encodes two or more products, and expression of each product is under control of a separate promoter, or expression of all is under control of a single promoter, and each product is separated by nucleic acid encoding a 2A peptide to effect separate translation of each encoded therapeutic product.
132. The immunostimulatory bacterium of item 131, wherein the nucleic acid encodes a T2A, F2A, E2A, or P2A peptide to effect separate expression of therapeutic products expressed under control of a single promoter.
133. The immunostimulatory bacterium of any of items 26-132, wherein the eukaryotic promoter is an RNA polymerase II promoter, or an RNA polymerase III promoter.
134. The immunostimulatory bacterium of item 133, wherein the promoter is an RNA polymerase II promoter that is a viral promoter or a mammalian RNA polymerase II promoter.
135. The immunostimulatory bacterium of item 134, wherein the promoter is a viral promoter selected from among a cytomegalovirus (CMV) promoter, an SV40 promoter, an Epstein Barr virus (EBV) promoter, a herpes virus promoter, and an adenovirus promoter.
136. The immunostimulatory bacterium of any of items 26-134, wherein the promoter that controls expression of one or more of the encoded therapeutic products or heterologous proteins on the plasmid is an elongation factor-1 (EF-1) alpha promoter, or an MND promoter, or a UBC promoter, or a PGK promoter, or a CAG promoter.
137. The immunostimulatory bacterium of any of items 26-134, wherein the promoter that controls expression of one or more of the encoded therapeutic products or heterologous proteins on the plasmid is an EF-1 alpha, an adenovirus 2 or 5 late, a CMV, an SV40, an MND, a PGK, an EIF4A1, a CAG, or a CD68 promoter.
138. The immunostimulatory bacterium of any of items 26-134, wherein the promoter that controls expression of one or more of the encoded therapeutic products or heterologous proteins on the plasmid is a viral promoter that is a late promoter.
139. The immunostimulatory bacterium of any of items 26-138, wherein the plasmid comprises regulatory sequences that comprise a terminator and/or promoters selected from among SV40, hGH, BGH, MND, chicken beta-globulin, and rbGlob (rabbit globulin) genes, to control expression of the therapeutic product(s).
140. The immunostimulatory bacterium of any of items 26-139, wherein the encoded therapeutic product(s) is/are operatively linked to a signal sequence for secretion from a cell containing the plasmid.
141. The immunostimulatory bacterium of any of items 26-140, wherein the plasmid that encodes the therapeutic product comprises a construct that includes an enhancer, a promoter, the open reading frame encoding the therapeutic product or heterologous protein, and a polyA tail.
142. The immunostimulatory bacterium of any of items 26-141, wherein the plasmid comprises a construct that includes an enhancer, a promoter, an IRES, the open reading frame encoding the therapeutic product or heterologous protein, and a polyA tail.
143. The immunostimulatory bacterium of any of (items 26-142, wherein the plasmid comprises a construct that includes an enhancer, a promoter, an IRES, a localization sequence, the open reading frame encoding the therapeutic product, and a polyA tail.
144. The immunostimulatory bacterium of any of items 26-143, wherein the plasmid comprises a construct that includes a bacterial terminator positioned to decrease read-through from a bacterial promoter on the plasmid.
145. The immunostimulatory bacterium of any of items 26-144, wherein the eukaryotic promoter on the plasmid is oriented in the opposite direction from a bacterial promoter on the plasmid.
146. The immunostimulatory bacterium of item 145, wherein the bacterial promoter controls expression of the *asd* gene.
147. The immunostimulatory bacterium of any of items 26-146 wherein the construct on the plasmid encoding the therapeutic product or heterologous protein comprises a Woodchuck Hepatitis Virus (WHP) Posttranscriptional Regulatory Element (WPRE), or a Hepatitis B virus Posttranscriptional Regulatory Element (HPRE).
148. The immunostimulatory bacterium of any of items 26-147, wherein the plasmid contains nucleic acid encoding a therapeutic product that is part of a cytosolic DNA/RNA sensor pathway that leads to expression of type I interferon (IFN), or is a variant thereof.
149. The immunostimulatory bacterium of item 148, wherein the therapeutic product in its unmodified form senses or interacts directly or indirectly with cytosolic nucleic acids, nucleotides, dinucleotides, or cyclic dinucleotides, to induce expression of type I IFN, and the variant protein induces expression of type I IFN in the absence of the sensing or interacting with the cytosolic nucleic acids, nucleotides, dinucleotides, or cyclic dinucleotides.
150. The immunostimulatory bacterium of item 148, wherein the therapeutic product is a variant that, when expressed in a subject, leads to constitutive expression of type I IFN.
151. The immunostimulatory bacterium of item 148, wherein the therapeutic product is a gain-of-function (GOF) variant that does not require cytosolic nucleic acids, nucleotides, dinucleotides, or cyclic dinucleotides to result in expression of type I IFN.
152. The immunostimulatory bacterium of any of items 26-151, wherein the therapeutic product is selected from among STING, RIG-I, MDA-5, IRF-3, IRF-7, TRIM56, RIP1, Sec5, TRAF3, TRAF2, TRAF6, STAT1, LGP2, DDX3, DHX9, DDX1, DDX9, DDX21, DHX15, DHX33, DHX36, DDX60, and SNRNP200, and variants thereof that increase activity or increase activity so that type I interferon activity is increased or constitutive.
153. The immunostimulatory bacterium of any of items 26-152, wherein the therapeutic product is selected from among TRIM56, RIP1, Sec5, TRAF3, TRAF2, TRAF6, STAT1, LGP2, DDX3, DHX9, DDX1, DDX9, DDX21, DHX15, DHX33, DHX36, DDX60, and SNRNP200, and variants thereof that increase activity or increase activity so that type I interferon activity is increased or constitutive.
154. The immunostimulatory bacterium of any of items 26-153, wherein the therapeutic product is a variant protein that has increased activity that results in increased expression of type I interferon (IFN), or results in constitutive expression of type I IFN.
155. The immunostimulatory bacterium of any of items 26-154, wherein the plasmid encodes a gain-of-function, constitutively active variant of a protein that, in humans, promotes or causes interferonopathies.
156. The immunostimulatory bacterium of any of items 62-80 and 148-155, wherein the therapeutic product is a variant that comprises a mutation that eliminates a phosphorylation site in a STING protein to thereby reduce nuclear factor kappa-light-chain-enhancer of activated B-cell (NF-κB) signaling.
157. The immunostimulatory bacterium of any of items 148-156, wherein the therapeutic product that induces type I IFN is STING, RIG-I, IRF-3, or MDA5, or a variant thereof.
158. The immunostimulatory bacterium of any of items 148-157, wherein:
   the therapeutic product that induces expression of type I IFN is a variant thereof that has increased activity or constitutive activity; and
   the therapeutic product is STING, RIG-I, IRF-3, or MDA5, or a variant thereof.
159. The immunostimulatory bacterium of any of items 148-158, wherein the therapeutic product is a variant of STING, RIG-I, IRF-3, or MDA5 that comprises a gain-of-function mutation resulting in increased expression of type I IFN.
160. The immunostimulatory bacterium of any of items 148-159, wherein the therapeutic product is a variant of STING, RIG-I, IRF-3, or MDA5, in which one or more serine (S) or threonine (T) residue(s) that is/are phosphorylated as a consequence of viral infection, is/are replaced with an aspartic acid (D), whereby the resulting variant is a phosphomimetic that constitutively induces type I IFN.
161. The immunostimulatory bacterium of any of items 148-160, wherein:
   the therapeutic product is IRF-3 that has one or more replacement(s) at residues at positions 396, 398, 402, 404 and 405, with reference, for alignment to SEQ ID NO:312; and
   the residues are replaced with aspartic acid residues.
162. The immunostimulatory bacterium of item 161, wherein IRF-3 comprises the replacement S396D with reference, for alignment, to SEQ ID NO:312.
163. The immunostimulatory bacterium of item 161, wherein IRF-3 comprises the replacements S396D/S398D/S402D/T404D/S405D with reference, for alignment, to SEQ ID NO:312.
164. The immunostimulatory bacterium of any of items 148-163, wherein the therapeutic product is selected from among STING, RIG-I, MDA-5, IRF-3, IRF-7, TRIM56, RIP1, Sec5, TRAF3, TRAF2, TRAF6, STAT1, LGP2, DDX3, DHX9, DDX1, DDX9, DDX21, DHX15, DHX33, DHX36, DDX60, and SNRNP200, and variants thereof that increase activity or increase activity so that type I interferon activity is increased or constitutive.
165. The immunostimulatory bacterium of any of items 148-164, wherein:
   the therapeutic product that senses cytosolic DNA/RNA is a variant STING, MDA5, RIG-I or IRF-3; and
   unmodified STING has the sequence set forth in any of SEQ ID NOs: 305-309, unmodified MDA5 has the sequence set forth in SEQ ID NO:310, unmodified RIG-I has the sequence set forth in SEQ ID NO 311, and unmodified IRF-3 has the sequence set forth in SEQ ID NO:312.
166. The immunostimulatory bacterium of any of items 148-165, wherein the therapeutic product is selected from among STING, MDA5, IRF-3, and RIG-I, and comprises a gain-of-function mutation(s) that renders the STING, MDA5, IRF-3, or RIG-I constitutively active, whereby expression of type I IFN is constitutive.
167. The immunostimulatory bacterium of item 165 or item 166, wherein the mutations are selected as follows:
   a) in STING, with reference, for alignment, to SEQ ID NOs: 305-309, one or more selected from among: S102P, V147L, V147M, N154S, V155M, G166E, C206Y, G207E, S102P/F279L, F279L, R281Q, R284G, R284S, R284M, R284K, R284T, R197A, D205A, R310A, R293A, T294A, E296A, R197A/D205A, S272A/Q273A, R310A/E316A, E316A, E316N, E316Q, S272A, R293A/T294A/E296A, D231A, R232A, K236A, Q273A, S358A/E360A/S366A, D231A/R232A/K236A/R238A, S358A, E360A, S366A, R238A, R375A, N154S/R284G, and S324A/S326A;
   b) in MDA5, with reference, for alignment, to SEQ ID NO:310, one or more of: T331I, T331R, A489T, R822Q, G821S, A946T, R337G, D393V, G495R, R720Q, R779H, R779C, L372F, and A452T;
   c) in RIG-I, with reference, for alignment, to SEQ ID NO:311, one or both of E373A and C268F; and
   d) in IRF-3, with reference, for alignment, to SEQ ID NO:312, S396D.
168. The immunostimulatory bacterium of any of items 148-167, wherein the therapeutic product is a variant STING that contains one or more amino replacement(s) selected, with reference, for alignment, to any of SEQ ID NOs: 305-309, from among: S102P, V147L, V147M, N154S, V155M, G166E, C206Y, G207E, S102P/F279L, F279L, R281Q, R284G, R284S, R284M, R284K, R284T, R197A, D205A, R310A, R293A, T294A, E296A, R197A/D205A, S272A/Q273A, R310A/E316A, E316A, E316N, E316Q, S272A, R293A/T294A/E296A, D231A, R232A, K236A, Q273A, S358A/E360A/S366A, D231A/R232A/K236A/R238A, S358A, E360A, S366A, R238A, R375A, S324A/S326A, and N154S/R284G, and conservative replacements thereof.
169. The immunostimulatory bacterium of any of items 26-168, wherein: the therapeutic protein increases or induces expression of a type I IFN; and
   the type I IFN is an interferon-a or interferon-β.
170. The immunostimulatory bacterium of any of items 26-169, wherein one or more genes or operons involved in SPI-1 invasion or SPI-1 independent invasion are deleted, disrupted, or inactivated, whereby the immunostimulatory bacterium does not invade or infect epithelial cells, or has a reduced ability to invade or infect epithelial cells.
171. The immunostimulatory bacterium of item 170, wherein one or more of *avrA, hilA, hilD, invA, invB, invC, invE, invF, invG, invH, invI, invJ, iacP, iagB, spaO, spaP, spaQ, spaR, spaS, orgA, orgB, orgC, prgH, prgI, prgJ, prgK, sicA, sicP, sipA, sipB, sipC, sipD, sirC, sopB, sopD, sopE, sopE2, sprB,* and *sptP* is deleted, disrupted, or inactivated.
172. The immunostimulatory bacterium of item 171, wherein the one or more genes is/are selected from among *pagN*, *hlyE, pefI, srgD, srgA, srgB,* and *srgC.*
173. The immunostimulatory bacterium of any of claims 26-172 that has a deletion or disruption of a gene encoding a protein in the SPI-2 complex.
174. The immunostimulatory bacterium of any of items 26-173, wherein the plasmid encodes an immunostimulatory protein that confers or contributes to an anti-tumor immune response in the tumor microenvironment.
175. The immunostimulatory bacterium of (item 174, wherein the immunostimulatory protein that confers or contributes to an anti-tumor immune response in the tumor microenvironment is selected from among one or more of: IL-2, IL-7, IL-12p70 (IL-12p40 + IL-12p35), IL-15, IL-2 that has attenuated binding to IL-2Ra, IL-15/IL-15R alpha chain complex, IL-18, IL-21, IL-23, IL-36γ, IL-2 modified so that it does not bind to IL-2Ra, CXCL9, CXCL10, CXCL11, interferon-α, interferon-β, interferon-γ, CCL3, CCL4, CCL5, proteins that are involved in or that effect or potentiate the recruitment and/or persistence of T-cells, CD40, CD40 ligand (CD40L), CD28, OX40, OX40 ligand (OX40L), 4-1BB, 4-1BB ligand (4-1BBL), 4-1BBl that has a deleted or truncated or otherwise modified cytoplasmic domain to eliminate the immunosuppressive reverse signaling, members of the B7-CD28 family, CD47 antagonists, an anti-IL6 antibody or IL-6 binding decoy receptor, TGF-beta polypeptide antagonists, and members of the tumor necrosis factor receptor (TNFR) superfamily.
176. The immunostimulatory bacterium of item 174 or item 175, wherein the plasmid encodes a modified 4-1BBL, whose sequence is set forth in SEQ ID NO:390, SEQ ID NO:391, or SEQ ID NO:392.
177. The immunostimulatory bacterium of item 175 or item 176, further comprising a tag linked to the co-stimulatory protein to facilitate purification or expression thereof.
178. The immunostimulatory bacterium of item 177, wherein the tag is a c-myc tag comprising the sequence MEQKLISEEDL, set forth as residues 1-11 of SEQ ID NO:392.
179. The immunostimulatory bacterium of any of items 174-178, wherein the plasmid encodes a 4-1BBL polypeptide and at least one additional therapeutic protein.
180. The immunostimulatory bacterium of item 179, wherein the additional therapeutic protein(s) is/are IL-15Rα-IL-15sc, or IL-12, or IL-15Rα-IL-15sc and IL-12.
181. The immunostimulatory bacterium of any of items 174-180, comprising a 1-4BBL polypeptide and one or more additional therapeutic polypeptides encoded in a polycistronic nucleic acid under control of a single promoter, wherein the 1-4BBL polypeptide is the first polypeptide in the polycistron.
182. The immunostimulatory bacterium of any of items , 174-181, wherein:
   the immunostimulatory protein is a co-stimulatory molecule selected from among CD40, CD40 ligand (CD40L), CD28, OX40, OX40 ligand (OX40L), 4-1BB, and a 4-1BB ligand (4-1BBL) that optionally is truncated in the cytoplasmic domain or that lacks a cytoplasmic domain, for expression on an antigen-presenting cell (APC), to eliminate the immunosuppressive reverse signaling; and
   the truncated gene product is capable of constitutive immunostimulatory signaling to a T-cell through co-stimulatory receptor engagement, and is unable to counter-regulatory signal to the antigen-presenting cell (APC) due to the truncated or deleted cytoplasmic domain.
183. The immunostimulatory bacterium of any of items 26-182, wherein the encoded product comprises one or more of a STING protein, a modified STING protein, a chimeric STING protein, an anti-CTLA-4 antibody, IL-15, 4-1BBL and modified or truncated forms thereof, a TGF-beta receptor decoy or polypeptide antagonist, IL-12, and IL-21, and/or any of the following:
   one or more of IL-12, or IL-15, or IL12p70, or IL-15/IL-15R alpha chain complex;
   a cytokine, and a STING pathway agonist;
   a cytokine, a STING pathway agonist, and either a co-stimulatory molecule or an immune checkpoint inhibitor;
   a cytokine, a STING pathway agonist, and a TGF-beta receptor decoy or polypeptide antagonist; and/or
   a cytokine, a STING pathway agonist, a TGF-beta receptor decoy or polypeptide antagonist, and either a co-stimulatory molecule or an immune checkpoint inhibitor.
184. The immunostimulatory bacterium of any of items 26-183, wherein the plasmid includes nucleic acid encoding a product that induces tumor cell apoptosis or is cytotoxic to tumor cells.
185. The immunostimulatory bacterium of item 184, wherein:
   the product is a nucleic acid; and
   the nucleic acid encoding the product includes nucleic acid encoding a secretion signal whereby the product is secreted.
186. The immunostimulatory bacterium of item 183 or item 184, wherein the product induces apoptosis.
187. The immunostimulatory bacterium of item 186, wherein the product that induces apoptosis is azurin.
188. The immunostimulatory bacterium of any of items 174-187, wherein the plasmid encodes an immunostimulatory protein that confers or contributes to an anti-tumor immune response in the tumor microenvironment, and the immunostimulatory protein is a cytokine or a chemokine.
189. The immunostimulatory bacterium of any of items 174-188, wherein the plasmid encodes an immunostimulatory protein that confers or contributes to an anti-tumor immune response in the tumor microenvironment that is a co-stimulatory molecule or cytoplasmic domain-deleted or truncated or otherwise modified form thereof.
190. The immunostimulatory bacterium of item 189, wherein the immunostimulatory protein that confers or contributes to an anti-tumor immune response in the tumor microenvironment is selected from among 4-1BBL, CD80, CD86, CD27L, B7RP1, and OX40L, and cytoplasmic domain deleted or truncated or truncated and modified forms thereof, where the modifications promote correct orientation when the protein is expressed in a cell, and the cytoplasmic deletions, truncations and/or modifications eliminate or reduce immunosuppressive reverse signaling.
191. The immunostimulatory bacterium of any of items 26-190, wherein the plasmid encodes a therapeutic product that is a TGF-beta polypeptide antagonist.
192. The immunostimulatory bacterium of item 191, wherein the TGF-beta polypeptide antagonist is selected from among an anti-TGF-beta antibody or a fragment thereof, an anti-TGF-beta receptor antibody or a fragment thereof, a soluble TGF-beta antagonist polypeptide, and a TGF-beta binding decoy receptor.
193. The immunostimulatory bacterium of item 191 or item 192, wherein nucleic acid encoding the TGF-beta polypeptide antagonist comprises nucleic acid encoding a signal sequence for secretion of the encoded polypeptide.
194. The immunostimulatory bacterium of any of items 26-193, wherein the therapeutic product is an antibody or antigen-binding fragment thereof.
195. The immunostimulatory bacterium of item 194, wherein the antibody or antigen-binding fragment thereof is an antigen-binding fragment that is selected from among a Fab, Fab', F(ab')₂, single-chain Fv (scFv), Fv, dsFv, nanobody, diabody fragment, a single-chain antibody, and an scFv-Fc two-chain antibody.
196. The immunostimulatory bacterium of item 195, wherein the antibody is an scFV.
197. The immunostimulatory bacterium of item 195 or item 196, wherein the antibody comprises an Fc, whereby the resulting antibody comprises two chains.
198. The immunostimulatory bacterium of any of items 195-197, wherein the antibody is encoded by nucleic acid comprising nucleic acid encoding the variable light chain, a linker, the variable heavy chain, and an IgG Fc, whereby the encoded antibody is an scFv-Fc.
199. The immunostimulatory bacterium of any of items 195-198, wherein the antibody is an anti-CTLA-4 antibody.
200. The immunostimulatory bacterium of any of items 194-199, wherein the antibody or antigen-binding fragment thereof is humanized or is human.
201. The immunostimulatory bacterium of any of items 194-200, wherein the antibody or antigen-binding fragment thereof is an antagonist of PD-1, PD-L1, CTLA-4, VEGF, VEGFR2, or IL-6.
202. The immunostimulatory bacterium of any of items 26-201, wherein the plasmid encodes two or more therapeutic products selected from among:
   a) an immunostimulatory protein that confers or contributes to an anti-tumor immune response in the tumor microenvironment;
   b) one or more of a protein that is part of a cytosolic DNA/RNA sensor pathway that leads to expression of type I interferon (IFN), or a variant thereof that has increased activity to increase expression of type I IFN, or a variant thereof that results in constitutive expression of a type I IFN; and
   c) an anti-cancer antibody or antigen-binding portion thereof.
203. The immunostimulatory bacterium of item 202, wherein the immunostimulatory protein is a co-stimulatory molecule that lacks a cytoplasmic domain or a sufficient portion thereof, for expression on an antigen-presenting cell (APC), whereby the truncated co-stimulatory molecule is capable of constitutive immunostimulatory signaling to a T-cell through co-stimulatory receptor engagement and is unable to counter-regulatory signal to the antigen presenting cell (APC).
204. An immunostimulatory bacterium, comprising a plasmid that encodes two or more therapeutic products under control of a single promoter, wherein:
   the therapeutic products are selected from among:
      a) an immunostimulatory protein that confers or contributes to an anti-tumor immune response in the tumor microenvironment;
      b) one or more of a protein that is part of a cytosolic DNA/RNA sensor pathway that leads to expression of type I interferon (IFN), or a variant thereof that has increased activity to increase expression of type I IFN, or a variant thereof that results in constitutive expression of a type I IFN; and
      c) an anti-cancer antibody or antigen-binding portion thereof; and
   the encoding nucleic acids are separated by an IRES sequence or 2A peptides, and each nucleic acid encoding each product is optionally operatively linked to nucleic acid encoding a signal sequence, whereby, upon translation of the encoded mRNA, each product is separately expressed and secreted from a cell comprising the bacterium and/or plasmid.
205. The immunostimulatory bacterium of item 204, wherein the immunostimulatory protein is a co-stimulatory molecule that lacks a cytoplasmic domain or a sufficient portion thereof, for expression on an antigen-presenting cell (APC), whereby the truncated co-stimulatory molecule is capable of constitutive immunostimulatory signaling to a T-cell through co-stimulatory receptor engagement and is unable to counter-regulatory signal to the antigen presenting cell (APC).
206. The immunostimulatory bacterium of any of items 26-205, wherein the plasmid encodes at least two therapeutic products selected from among a cytokine, a protein that constitutively induces a type I IFN, a co-stimulatory molecule, and an anti-cancer antibody or antigen-binding portion thereof.
207. The immunostimulatory bacterium of any of items 26-206, wherein:
   the plasmid encodes two or more therapeutic products under control of a single promoter; and
   expression of the nucleic acid encoding at least two or all of the products is under control of a single promoter, and the nucleic acid encoding each product is separated by nucleic acid encoding 2A peptides, whereby, upon translation, each product is separately expressed.
208. The immunostimulatory bacterium of any of items 202-207, wherein nucleic acid encoding one or more of the therapeutic products is operatively linked to nucleic acid encoding a sequence that directs secretion of the expressed product(s).
209. The immunostimulatory bacterium of any of items 26-208, wherein:
   the therapeutic product is a co-stimulatory molecule with a cytoplasmic domain deletion or truncation or other modification for expression on an antigen-presenting cell (APC); and
   the truncated gene product is capable of constitutive immunostimulatory signaling to a T-cell through co-stimulatory receptor engagement, and is unable to counter-regulatory signal to the APC due to the cytoplasmic domain deletion, truncation or other modification.
210. The immunostimulatory bacterium of item 209, wherein the co-stimulatory molecule with the deleted, truncated or otherwise modified cytoplasmic domain is 4-1BBL, CD80, CD86, CD27L, B7RP1, or OX40L, and variants that increase correct orientation of the protein when expressed in a cells.
211. The immunostimulatory bacterium of any of items 26-210 that encodes two or more therapeutic products, wherein at least one product is selected from a) and at least one is selected from b), wherein:
   a) is IL-2, IL-7, IL-12p70 (IL-12p40 + IL-12p35), IL-15, IL-23, IL-36 gamma, IL-2 that has attenuated binding to IL-2Ra, IL-15/IL-15R alpha chain complex, IL-18, IL-2 that is modified so that it does not bind to IL-2Ra, CXCL9, CXCL10, CXCL11, interferon-α, interferon-β, CCL3, CCL4, CCL5, proteins that are involved in or that effect or potentiate recruitment/persistence of T cells, CD40, CD40 Ligand (CD40L), OX40, OX40 Ligand (OX40L), 4-1BB, 4-1BB Ligand (4-1BBL), members of the B7-CD28 family, TGF-beta polypeptide antagonists, or members of the tumor necrosis factor receptor (TNFR) superfamily; and
   b) is STING, RIG-I, MDA-5, IRF-3, IRF-5, IRF-7, TRIM56, RIP1, Sec5, TRAF3, TRAF2, TRAF6, STAT1, LGP2, DDX3, DHX9, DDX1, DDX9, DDX21, DHX15, DHX33, DHX36, DDX60, or SNRNP200.
212. The immunostimulatory bacterium of any of items 26-211 that encodes or further encodes one or more of a TGF-beta inhibitory antibody or antigen-binding fragment thereof, a TGF-beta binding decoy receptor, an anti-IL6 antibody or antigen-binding fragment thereof, and an IL-6 binding decoy receptor.
213. The immunostimulatory bacterium of any of items 26-212 that encodes one or more of the following combinations of therapeutic products:
   IL-2 and IL-12p70;
   IL-2 and IL-21;
   IL-2, IL-12p70, and a STING gain-of-function (GOF) variant;
   IL-2, IL-21, and a STING GOF variant;
   IL-2, IL-12p70, a STING GOF variant, and 4-1BBL (including 4-1BBLΔcyt), where Δcyt is a deleted cytoplasmic domain;
   IL-2, IL-21, a STING GOF variant, and 4-1BBL (including 4-1BBLΔcyt);
   IL-15/IL-15Rα, and a STING GOF variant;
   IL-15/IL-15Rα, a STING GOF variant, and 4-1BBL (including 4-1BBLΔcyt);
   IL-15/IL-15Rα and IL-12p70;
   IL-15/IL-15Rα and IL-21;
   IL-15/IL-15Rα, IL-12p70, and a STING GOF variant;
   IL-15/IL-15Rα, IL-21, and a STING GOF variant;
   IL-15/IL-15Rα, IL-12p70, a STING GOF variant, and 4-1BBL (including 4-1BBLΔcyt);
   IL-15/IL-15Rα, IL-21, a STING GOF variant, and 4-1BBL (including 4-1BBLΔcyt);
   IL-12p70 and IL-21;
   IL-12p70, IL-21, and a STING GOF variant;
   IL-12p70, IL-21, a STING GOF variant, and 4-1BBL (including 4-1BBLΔcyt);
   IL-12p70 and a STING GOF variant;
   IL-12p70, a STING GOF variant, and 4-1BBL (including 4-1BBLΔcyt);
   IL-12p70 and IL-18;
   IL-12p70, IL-18, and a STING GOF variant;
   IL-12p70, IL-18, a STING GOF variant, and 4-1BBL (including 4-1BBLΔcyt);
   a TGF-β decoy receptor or polypeptide antagonist, IL-2, and IL-12p70;
   a TGF-β decoy receptor or polypeptide antagonist, IL-2, and IL-21;
   a TGF-β decoy receptor or polypeptide antagonist, IL-2, IL-12p70, and a STING GOF variant;
   a TGF-β decoy receptor or polypeptide antagonist, IL-2, IL-21, and a STING GOF variant;
   a TGF-β decoy receptor or polypeptide antagonist, IL-2, IL-12p70, a STING GOF variant, and 4-1BBL (including 4-1BBLΔcyt);
   a TGF-β decoy receptor or polypeptide antagonist, IL-2, IL-21, a STING GOF variant, and 4-1BBL (including 4-1BBLΔcyt);
   a TGF-β decoy receptor or polypeptide antagonist, IL-15/IL-15Rα, and a STING GOF variant;
   a TGF-β decoy receptor or polypeptide antagonist, IL-15/IL-15Rα, a STING GOF variant, and 4-1BBL (including 4-1BBLΔcyt);
   a TGF-β decoy receptor or polypeptide antagonist, IL-15/IL-15Rα, and IL-12p70;
   a TGF-β decoy receptor or polypeptide antagonist, IL-15/IL-15Rα, and IL-21;
   a TGF-β decoy receptor or polypeptide antagonist, IL-15/IL-15Rα, IL-12p70, and a STING GOF variant;
   a TGF-β decoy receptor or polypeptide antagonist, IL-15/IL-15Rα, IL-21, and a STING GOF variant;
   a TGF-β decoy receptor or polypeptide antagonist, IL-15/IL-15Rα, IL-12p70, a STING GOF variant, and 4-1BBL (including 4-1BBLΔcyt);
   a TGF-β decoy receptor or polypeptide antagonist, IL-15/IL-15Rα, IL-21, a STING GOF variant, and 4-1BBL (including 4-1BBLΔcyt);
   a TGF-β decoy receptor or polypeptide antagonist, IL-12p70, and IL-21;
   a TGF-β decoy receptor or polypeptide antagonist, IL-12p70, IL-21, and a STING GOF variant;
   a TGF-β decoy receptor or polypeptide antagonist, IL-12p70, IL-21, a STING GOF variant, and 4-1BBL (including 4-1BBLΔcyt);
   a TGF-β decoy receptor or polypeptide antagonist, and IL-12p70;
   a TGF-β decoy receptor or polypeptide antagonist, IL-12p70, and a STING GOF variant;
   a TGF-β decoy receptor or polypeptide antagonist, IL-12p70, a STING GOF variant, and 4-1BBL (including 4-1BBLΔcyt);
   a TGF-β decoy receptor or polypeptide antagonist, IL-12p70, and IL-18;
   a TGF-β decoy receptor or polypeptide antagonist, IL-12p70, IL-18, and a STING GOF variant;
   a TGF-β decoy receptor or polypeptide antagonist, IL-12p70, IL-18, a STING GOF variant, and 4-1BBL (including 4-1BBLΔcyt);
   a TGF-β decoy receptor or polypeptide antagonist, and a STING GOF variant;
   an anti-CTLA-4 antibody, IL-2, and IL-12p70;
   an anti-CTLA-4 antibody, IL-2, and IL-21;
   an anti-CTLA-4 antibody, IL-2, IL-12p70, and a STING GOF variant;
   an anti-CTLA-4 antibody, IL-2, IL-21, and a STING GOF variant;
   an anti-CTLA-4 antibody, IL-2, IL-12p70, a STING GOF variant, and 4-1BBL (including 4-1BBLΔcyt);
   an anti-CTLA-4 antibody, IL-2, IL-21, a STING GOF variant, and 4-1BBL (including 4-1BBLΔcyt);
   an anti-CTLA-4 antibody, IL-15/IL-15Rα, and a STING GOF variant;
   an anti-CTLA-4 antibody, IL-15/IL-15Rα, a STING GOF variant, and 4-1BBL (including 4-1BBLΔcyt);
   an anti-CTLA-4 antibody, IL-15/IL-15Rα, and IL-12p70;
   an anti-CTLA-4 antibody, IL-15/IL-15Rα, and IL-21;
   an anti-CTLA-4 antibody, IL-15/IL-15Rα, IL-12p70, and a STING GOF variant;
   an anti-CTLA-4 antibody, IL-15/IL-15Rα, IL-21, and a STING GOF variant;
   an anti-CTLA-4 antibody, IL-15/IL-15Rα, IL-12p70, a STING GOF variant, and 4-1BBL (including 4-1BBLΔcyt);
   an anti-CTLA-4 antibody, IL-15/IL-15Rα, IL-21, a STING GOF variant, and 4-1BBL (including 4-1BBLΔcyt);
   an anti-CTLA-4 antibody, IL-12p70, and IL-21;
   an anti-CTLA-4 antibody, IL-12p70, IL-21, and a STING GOF variant;
   an anti-CTLA-4 antibody, IL-12p70, IL-21, a STING GOF variant, and 4-1BBL (including 4-1BBLΔcyt);
   an anti-CTLA-4 antibody and IL-12p70;
   an anti-CTLA-4 antibody, IL-12p70, and a STING GOF variant;
   an anti-CTLA-4 antibody, IL-12p70, a STING GOF variant, and 4-1BBL (including 4-1BBLΔcyt);
   an anti-CTLA-4 antibody, IL-12p70, and IL-18;
   an anti-CTLA-4 antibody, IL-12p70, IL-18, and a STING GOF variant;
   an anti-CTLA-4 antibody, IL-12p70, IL-18, a STING GOF variant, and 4-1BBL (including 4-1BBLΔcyt);
   an anti-CTLA-4 antibody and a STING GOF variant;
   a CD40 agonist, IL-2, and IL-12p70;
   a CD40 agonist, IL-2, and IL-21;
   a CD40 agonist, IL-2, IL-12p70, and a STING GOF variant;
   a CD40 agonist, IL-2, IL-21, and a STING GOF variant;
   a CD40 agonist, IL-2, IL-12p70, a STING GOF variant, and 4-1BBL (including 4-1BBLΔcyt);
   a CD40 agonist, IL-2, IL-21, a STING GOF variant, and 4-1BBL (including 4-1BBLΔcyt);
   a CD40 agonist, IL-15/IL-15Rα, and a STING GOF variant;
   a CD40 agonist, IL-15/IL-15Rα, a STING GOF variant, and 4-1BBL (including 4-1BBLΔcyt);
   a CD40 agonist, IL-15/IL-15Rα, and IL-12p70;
   a CD40 agonist, IL-15/IL-15Rα, and IL-21;
   a CD40 agonist, IL-15/IL-15Rα, IL-12p70, and a STING GOF variant;
   a CD40 agonist, IL-15/IL-15Rα, IL-21, and a STING GOF variant;
   a CD40 agonist, IL-15/IL-15Rα, IL-12p70, a STING GOF variant, and 4-1BBL (including 4-1BBLΔcyt);
   a CD40 agonist, IL-15/IL-15Rα, IL-21, a STING GOF variant, and 4-1BBL (including 4-1BBLΔcyt);
   a CD40 agonist, IL-12p70, and IL-21;
   a CD40 agonist, IL-12p70, IL-21, and a STING GOF variant;
   a CD40 agonist, IL-12p70, IL-21, a STING GOF variant, and 4-1BBL (including 4-1BBLΔcyt);
   a CD40 agonist and IL-12p70; a CD40 agonist, IL-12p70, and a STING GOF variant;
   a CD40 agonist, IL-12p70, a STING GOF variant, and 4-1BBL (including 4-1BBLΔcyt);
   a CD40 agonist, IL-12p70, and IL-18;
   a CD40 agonist, IL-12p70, IL-18, and a STING GOF variant;
   a CD40 agonist, IL-12p70, IL-18, a STING GOF variant, and 4-1BBL (including 4-1BBLΔcyt); and
   a CD40 agonist and a STING GOF variant, wherein:
      4-1BBL is 4-1BBL with a deleted cytoplasmic domain, 4-1BBL with a modified cytoplasmic domain, 4-1BBL with a truncated cytoplasmic domain, or 4-1BBL with a truncated and modified cytoplasmic domain; and
      an anti-CTLA-4 antibody is an scFv or an scFv-Fc; and
214. The immunostimulatory bacterium of any of items 26-213, the comprises nucleic acid encoding a combination of therapeutic products selected from among the following combinations:
   an anti-CTLA-4 antibody and STING,
   IL-15 and STING,
   4-1BBL and STING,
   TGF-beta decoy receptor or polypeptide antagonist, and STING,
   IL-12 and STING,
   an anti-CTLA-4 antibody, IL-15, and STING,
   4-1BBL, IL-15, and STING,
   TGF-beta decoy receptor or polypeptide antagonist, and IL-15, and STING, an anti-CTLA-4 antibody, and IL-12, and STING,
   4-1BBL, IL-12, and STING,
   TGF-beta decoy receptor or antagonist polypeptide, IL-12, and STING,
   an anti-CTLA-4 antibody, IL-15, a TGF-beta decoy receptor or polypeptide antagonist, and STING,
   4-1BBL, and IL-15, a TGF-beta decoy receptor or polypeptide antagonist, and STING,
   an anti-CTLA-4 antibody, and IL-12, and a TGF-beta decoy receptor or polypeptide antagonist, and STING,
   4-1BBL, and IL-12, a TGF-beta decoy receptor or polypeptide antagonist, and STING,
   an anti-CTLA-4 antibody, IL-12, IL-15, and STING,
   4-1BBL, IL-12, IL-15, and STING,
   a TGF-beta decoy receptor or polypeptide antagonist, IL-12, IL-15, and STING,
   a TGF-beta decoy receptor or polypeptide antagonist, IL-12, IL-15, and STING,
   an anti-CTLA-4 antibody, IL-12, IL-15, a TGF-beta decoy receptor or polypeptide antagonist, and STING,
   4-1BBL, IL-12, IL-21, a TGF-beta decoy receptor or polypeptide antagonist, and STING,
   an anti-CTLA-4 antibody, IL-12, IL-15, and a TGF-beta decoy receptor or polypeptide antagonist,
   4-1BBL, IL-12, IL-21, and a TGF-beta decoy receptor or polypeptide antagonist,
   IL-12, IL-15, and STING,
   IL-15, IL-21, and STING,
   IL-12, IL-21, and STING,
   an anti-CTLA-4 antibody, IL-15, IL-21, and STING,
   an anti-CTLA-4 antibody, IL-12, IL-21, and STING,
   4-1BBL, IL-15, IL-21, and STING,
   4-1BBL, IL-12, IL-21, and STING,
   an anti-CTLA-4 antibody, and IL-15,
   an anti-CTLA-4 antibody, IL-15, and a TGF-beta decoy receptor or polypeptide antagonist,
   4-1BBL and IL-15,
   4-1BBL, IL-15, and TGF-beta decoy receptor or polypeptide antagonist,
   an anti-CTLA-4 antibody and IL-12,
   an anti-CTLA-4 antibody, and IL-12, and a TGF-beta decoy receptor or polypeptide antagonist,
   4-1BBL and IL-12,
   4-1BBL, IL-12, and a TGF-beta decoy receptor or polypeptide antagonist,
   an anti-CTLA-4 antibody, and a TGF-beta decoy receptor or polypeptide antagonist,
   4-1BBL and a TGF-beta decoy receptor or polypeptide antagonist,
   IL-15 and a TGF-beta decoy receptor or polypeptide antagonist,
   IL-12 and a TGF-beta decoy receptor or polypeptide antagonist,
   IL-12, IL-15, and a TGF-beta decoy receptor or polypeptide antagonist, and
   IL-15, and IL-21, and a TGF-beta decoy receptor or polypeptide antagonist, wherein:
      IL-15 is IL-15 or IL-15/IL-15R alpha chain complex;
      STING is a STING polypeptide, or a variant STING polypeptide, or a chimeric STING polypeptide or a chimeric STING with amino acid replacements;
      TGF-beta is a soluble TGF-beta decoy or TGF-beta antagonist;
      4-1BBL is 4-1BBL with a deleted cytoplasmic domain, 4-1BBL with a modified cytoplasmic domain, 4-1BBL with a truncated cytoplasmic domain, or 4-1BBL with a truncated and modified cytoplasmic domain; and
      an anti-CTLA-4 antibody is an scFv or an scFv-Fc.
215. The immunostimulatory bacterium of item 214, wherein the TGF-beta decoy comprises an Fc fusion or is an anti-TGF antibody or an antigen-binding fragment thereof.
216. The immunostimulatory bacterium of item 214 or item 215, wherein the 4-1BBL is full-length, or is full-length with amino acid replacements at Ser5, and/or Ser8, whereby immunosuppressive reverse signaling is reduced or eliminated, or is 1-4BBL with a deleted cytoplasmic domain, or a truncated cytoplasmic domain that eliminates or reduces immunosuppressive reverse signaling, or is 4-1BBL that has amino acid replacements in the truncated cytoplasmic domain, whereby the 4-1BBL is in the correct orientation when expressed in a cell.
217. The immunostimulatory bacterium of any of items 214-215, wherein the STING polypeptide comprises the replacement R284G, or comprises the replacements N154S/R284, or is a chimeric STING polypeptide that is a chimeric human STING polypeptide with a CTT from Tasmanian Devil, or is a chimeric human STING polypeptide with a CTT from Tasmanian Devil and the replacement corresponding to R284G or the replacements corresponding to N154S/R284G, all with reference, for alignment, to SEQ ID NOs:305-309.
218. The immunostimulatory bacterium of any of items 213-215, wherein the STING gain-of-function (GOF) variant is/are selected from among any set forth in item 167 or item 168.
219. An immunostimulatory bacterium or a cell, encoding one or more of the following combinations of therapeutic products:
   IL-2 and IL-12p70;
   IL-2 and IL-21;
   IL-2, IL-12p70, and a STING GOF variant;
   IL-2, IL-21, and a STING GOF variant;
   IL-2, IL-12p70, a STING GOF variant, and 4-1BBL (including 4-1BBLΔcyt), where Δcyt is a deleted cytoplasmic domain;
   IL-2, IL-21, a STING GOF variant, and 4-1BBL (including 4-1BBLΔcyt);
   IL-15/IL-15Rα, and a STING GOF variant;
   IL-15/IL-15Rα, a STING GOF variant, and 4-1BBL (including 4-1BBLΔcyt);
   IL-15/IL-15Rα and IL-12p70;
   IL-15/IL-15Rα and IL-21;
   IL-15/IL-15Rα, IL-12p70, and a STING GOF variant;
   IL-15/IL-15Rα, IL-21, and a STING GOF variant;
   IL-15/IL-15Rα, IL-12p70, a STING GOF variant, and 4-1BBL (including 4-1BBLΔcyt);
   IL-15/IL-15Rα, IL-21, a STING GOF variant, and 4-1BBL (including 4-1BBLΔcyt);
   IL-12p70 and IL-21;
   IL-12p70, IL-21, and a STING GOF variant;
   IL-12p70, IL-21, a STING GOF variant, and 4-1BBL (including 4-1BBLΔcyt);
   IL-12p70 and a STING GOF variant;
   IL-12p70, a STING GOF variant, and 4-1BBL (including 4-1BBLΔcyt);
   IL-12p70 and IL-18;
   IL-12p70, IL-18, and a STING GOF variant;
   IL-12p70, IL-18, a STING GOF variant, and 4-1BBL (including 4-1BBLΔcyt);
   a TGF-β decoy receptor, IL-2, and IL-12p70;
   a TGF-β decoy receptor, IL-2, and IL-21;
   a TGF-β decoy receptor, IL-2, IL-12p70, and a STING GOF variant;
   a TGF-β decoy receptor, IL-2, IL-21, and a STING GOF variant;
   a TGF-β decoy receptor, IL-2, IL-12p70, a STING GOF variant, and 4-1BBL (including 4-1BBLΔcyt);
   a TGF-β decoy receptor, IL-2, IL-21, a STING GOF variant, and 4-1BBL (including 4-1BBLΔcyt);
   a TGF-β decoy receptor, IL-15/IL-15Rα, and a STING GOF variant;
   a TGF-β decoy receptor, IL-15/IL-15Rα, a STING GOF variant, and 4-1BBL (including 4-1BBLΔcyt);
   a TGF-β decoy receptor, IL-15/IL-15Rα, and IL-12p70;
   a TGF-β decoy receptor, IL-15/IL-15Rα, and IL-21;
   a TGF-β decoy receptor, IL-15/IL-15Rα, IL-12p70, and a STING GOF variant;
   a TGF-β decoy receptor, IL-15/IL-15Rα, IL-21, and a STING GOF variant;
   a TGF-β decoy receptor, IL-15/IL-15Rα, IL-12p70, a STING GOF variant, and 4-1BBL (including 4-1BBLΔcyt);
   a TGF-β decoy receptor, IL-15/IL-15Rα, IL-21, a STING GOF variant, and 4-1BBL (including 4-1BBLΔcyt);
   a TGF-β decoy receptor, IL-12p70, and IL-21;
   a TGF-β decoy receptor, IL-12p70, IL-21, and a STING GOF variant;
   a TGF-β decoy receptor, IL-12p70, IL-21, a STING GOF variant, and 4-1BBL (including 4-1BBLΔcyt);
   a TGF-β decoy receptor and IL-12p70;
   a TGF-β decoy receptor, IL-12p70, and a STING GOF variant;
   a TGF-β decoy receptor, IL-12p70, a STING GOF variant, and 4-1BBL (including 4-1BBLΔcyt);
   a TGF-β decoy receptor, IL-12p70, and IL-18;
   a TGF-β decoy receptor, IL-12p70, IL-18, and a STING GOF variant;
   a TGF-β decoy receptor, IL-12p70, IL-18, a STING GOF variant, and 4-1BBL (including 4-1BBLΔcyt);
   a TGF-β decoy receptor and a STING GOF variant;
   an anti-CTLA-4 antibody, IL-2, and IL-12p70;
   an anti-CTLA-4 antibody, IL-2, and IL-21;
   an anti-CTLA-4 antibody, IL-2, IL-12p70, and a STING GOF variant;
   an anti-CTLA-4 antibody, IL-2, IL-21, and a STING GOF variant;
   an anti-CTLA-4 antibody, IL-2, IL-12p70, a STING GOF variant, and 4-1BBL (including 4-1BBLΔcyt);
   an anti-CTLA-4 antibody, IL-2, IL-21, a STING GOF variant, and 4-1BBL (including 4-1BBLΔcyt);
   an anti-CTLA-4 antibody, IL-15/IL-15Rα, and a STING GOF variant;
   an anti-CTLA-4 antibody, IL-15/IL-15Rα, a STING GOF variant, and 4-1BBL (including 4-1BBLΔcyt);
   an anti-CTLA-4 antibody, IL-15/IL-15Rα, and IL-12p70;
   an anti-CTLA-4 antibody, IL-15/IL-15Rα, and IL-21;
   an anti-CTLA-4 antibody, IL-15/IL-15Rα, IL-12p70, and a STING GOF variant;
   an anti-CTLA-4 antibody, IL-15/IL-15Rα, IL-21, and a STING GOF variant;
   an anti-CTLA-4 antibody, IL-15/IL-15Rα, IL-12p70, a STING GOF variant, and 4-1BBL (including 4-1BBLΔcyt);
   an anti-CTLA-4 antibody, IL-15/IL-15Rα, IL-21, a STING GOF variant, and 4-1BBL (including 4-1BBLΔcyt);
   an anti-CTLA-4 antibody, IL-12p70, and IL-21;
   an anti-CTLA-4 antibody, IL-12p70, IL-21, and a STING GOF variant;
   an anti-CTLA-4 antibody, IL-12p70, IL-21, a STING GOF variant, and 4-1BBL (including 4-1BBLΔcyt);
   an anti-CTLA-4 antibody and IL-12p70;
   an anti-CTLA-4 antibody, IL-12p70, and a STING GOF variant;
   an anti-CTLA-4 antibody, IL-12p70, a STING GOF variant, and 4-1BBL (including 4-1BBLΔcyt);
   an anti-CTLA-4 antibody, IL-12p70, and IL-18;
   an anti-CTLA-4 antibody, IL-12p70, IL-18, and a STING GOF variant;
   an anti-CTLA-4 antibody, IL-12p70, IL-18, a STING GOF variant, and 4-1BBL (including 4-1BBLΔcyt);
   an anti-CTLA-4 antibody and a STING GOF variant;
   a CD40 agonist, IL-2, and IL-12p70;
   a CD40 agonist, IL-2, and IL-21;
   a CD40 agonist, IL-2, IL-12p70, and a STING GOF variant;
   a CD40 agonist, IL-2, IL-21, and a STING GOF variant;
   a CD40 agonist, IL-2, IL-12p70, a STING GOF variant, and 4-1BBL (including 4-1BBLΔcyt);
   a CD40 agonist, IL-2, IL-21, a STING GOF variant, and 4-1BBL (including 4-1BBLΔcyt);
   a CD40 agonist, IL-15/IL-15Rα, and a STING GOF variant;
   a CD40 agonist, IL-15/IL-15Rα, a STING GOF variant, and 4-1BBL (including 4-1BBLΔcyt);
   a CD40 agonist, IL-15/IL-15Rα, and IL-12p70;
   a CD40 agonist, IL-15/IL-15Rα, and IL-21;
   a CD40 agonist, IL-15/IL-15Rα, IL-12p70, and a STING GOF variant;
   a CD40 agonist, IL-15/IL-15Rα, IL-21, and a STING GOF variant;
   a CD40 agonist, IL-15/IL-15Rα, IL-12p70, a STING GOF variant, and 4-1BBL (including 4-1BBLΔcyt);
   a CD40 agonist, IL-15/IL-15Rα, IL-21, a STING GOF variant, and 4-1BBL (including 4-1BBLΔcyt);
   a CD40 agonist, IL-12p70, and IL-21;
   a CD40 agonist, IL-12p70, IL-21, and a STING GOF variant;
   a CD40 agonist, IL-12p70, IL-21, a STING GOF variant, and 4-1BBL (including 4-1BBLΔcyt);
   a CD40 agonist and IL-12p70;
   a CD40 agonist, IL-12p70, and a STING GOF variant;
   a CD40 agonist, IL-12p70, a STING GOF variant, and 4-1BBL (including 4-1BBLΔcyt);
   a CD40 agonist, IL-12p70, and IL-18;
   a CD40 agonist, IL-12p70, IL-18, and a STING GOF variant;
   a CD40 agonist, IL-12p70, IL-18, a STING GOF variant, and 4-1BBL (including 4-1BBLΔcyt); and
   a CD40 agonist and a STING GOF variant.
220. The immunostimulatory bacterium or cell of item 219, wherein the STING GOF variant is selected from among any set forth in item 167 or item 168.
221. The immunostimulatory bacterium any of items 26-220, wherein an encoded therapeutic product comprises an Fc domain.
222. The immunostimulatory bacterium of any of items 26-221, wherein an encoded therapeutic product comprises the B7 protein transmembrane domain.
223. The immunostimulatory bacterium of any of items 26-222, wherein an encoded therapeutic product is GPI-anchored.
224. The immunostimulatory bacterium of any of items 26-223, wherein an encoded therapeutic product comprises human serum albumin or its derivatives thereof that increase serum half-life of the encoded product.
225. The immunostimulatory bacterium of any of items 26-224, wherein an encoded therapeutic product comprises a fusion to collagen.
226. The immunostimulatory bacterium of any of items 26-225, wherein the bacterium is a Gram-negative bacterium.
227. The immunostimulatory bacterium of any of items 26-226,
   wherein the bacterium is a strain of *Salmonella, Shigella, E. coli, Bifidobacteriae, Rickettsia, Vibrio, Listeria, Klebsiella, Bordetella, Neisseria, Aeromonas, Francisella, Cholera, Corynebacterium, Citrobacter, Chlamydia, Haemophilus, Brucella, Mycobacterium, Mycoplasma, Legionella, Rhodococcus, Pseudomonas, Helicobacter, Bacillus,* or *Erysipelothrix,* or an attenuated strain thereof or a modified strain thereof of any of the preceding list of bacterial strains.
228. The immunostimulatory bacterium of any of items 26-226, wherein the bacterium is *Rickettsia rickettsiae, Rickettsia prowazekii, Rickettsia tsutsugamuchi, Rickettsia mooseri, Rickettsia sibirica, Bordetella bronchiseptica, Neisseria meningitidis, Neisseria gonorrhoeae, Aeromonas eucrenophila, Aeromonas salmonicida, Francisella tularensis, Corynebacterium pseudotuberculosis, Citrobacter freundii, Chlamydia pneumoniae, Haemophilus somnus, Brucella abortus, Mycobacterium intracellulare, Legionella pneumophila, Rhodococcus equi, Pseudomonas aeruginosa, Helicobacter mustelae, Vibrio cholerae, Bacillus subtilis, Erysipelothrix rhusiopathiae, Yersinia enterocolitica, Rochalimaea quintana,* or *Agrobacterium tumerfacium.*
229. The immunostimulatory bacterium of any of items 26-228 that is an attenuated bacterium or is a Gram-negative bacterium.
230. The immunostimulatory bacterium of any of items 26-229 that is a strain of *Salmonella.*
231. The immunostimulatory bacterium of item 230 that is a *Salmonella typhimurium* strain.
232. The immunostimulatory bacterium of item 230 or item 231, wherein the unmodified *Salmonella* is a wild-type strain.
233. The immunostimulatory bacterium of item 230 or item 231, wherein the unmodified *Salmonella* strain is attenuated.
234. The immunostimulatory bacterium of any of items 26-233, wherein the immunostimulatory bacterium is derived from strain AST-100 (VNP20009 or YS1646), or strain ATCC 14028, or a strain having all of the identifying characteristics of strain ATCC 14028.
235. The immunostimulatory bacterium of any of items 227-233 that is *ansB⁻, asd⁻, csgD⁻, purI⁻, msbB⁻,* flagellin⁻ (*fliC⁻*/*fljB⁻*), and *pagP⁻.*
236. The immunostimulatory bacterium of any of items 26-235, wherein the bacterium encodes and expresses the gene resistance to complement killing (*rck*)*.*
237. The immunostimulatory bacterium of item 236, wherein the *rck* gene is a *Salmonella rck* gene.
238. The immunostimulatory bacterium of any of items 26-237 that, when intravenously administered at a therapeutic dose, induces less than 150 pg/ml of each of serum IL-6, serum TNF-alpha, and serum IL-10, when measures at 7 days posttreatment.
239. An immunostimulatory bacterium that, when intravenously administered at a therapeutic dose, induces less than 150 pg/ml of each of serum IL-6, serum TNF-alpha, and serum IL-10, when measured at 7 days post treatment.
240. The immunostimulatory bacterium of item 238 or item 239, wherein the dose is 1x10⁸ CFUs (colony forming units).
241. The immunostimulatory bacterium of item 238 or item 239, wherein the dose is 1x10⁸ CFUs - 1x10⁹ CFUs.
242. An immunostimulatory bacterium, comprising a plasmid that encodes a therapeutic product, and that is modified to encode and express the gene resistance to complement killing (*rck*), wherein the wild-type bacterium does not encode *rck.*
243. The immunostimulatory bacterium of item 242 that is an *E. coli* strain.
244. A delivery vehicle encoding a truncated co-stimulatory molecule with a full or partial cytoplasmic domain deletion, for expression on an antigen-presenting cell (APC), wherein the truncated gene product is capable of constitutive immunostimulatory signaling to a T-cell through co-stimulatory receptor engagement, and is unable to counter-regulatory signal to the APC due to the deleted cytoplasmic domain.
245. The delivery vehicle of item 244, wherein the co-stimulatory molecule is 4-1BBL, CD80, CD86, CD27L, B7RP1, or OX40L, or a variant thereof with a deleted or truncated cytoplasmic domain and/or mutations that confer proper orientation of the protein when expressed in a cell.
246. The delivery vehicle of item 244 or item 245 that is an immunostimulatory bacterium, an exosome, a nanoparticle, an oncolytic virus, or a cell.
247. The delivery vehicle of any of items 244-246 that is a cell.
248. The delivery vehicle of item 247, wherein the cell is a stem cell.
249. The delivery vehicle of item 248, wherein the stem cell is a mesenchymal stem cell (MSC).
250. The delivery vehicle of item 249, wherein the MSC is genetically modified to express a combination of immunomodulatory cytokines.
251. The delivery vehicle of item 250, wherein the cytokines are Interleukin 12 (IL-12) and Interleukin 21 (IL-21).
252. An isolated cell, comprising the delivery vehicle of any of items 244-251.
253. An isolated cell, comprising the immunostimulatory bacterium of any of items 26-243.
254. The cell of item 252 or item 253 that is an immune cell, a stem cell, a tumor cell, or a primary cell line.
255. The cell of item 254 that is a hematopoietic cell.
256. The cell of item 255 that is a T-cell.
257. The cell of any of items 252-256 that is produced *ex vivo* by infecting the cell with the delivery vehicle or immunostimulatory bacterium.
258. The cell of any of items 252-254 that is a stem cell that is not of embryonic origin.
259. The cell of any of items 252-254, wherein the cell is a stem cell.
260. The cell of item 259, wherein the stem cell is a mesenchymal stem cell (MSC).
261. The cell of item 260, wherein the MSC is genetically modified to express a combination of immunomodulatory cytokines.
262. The cell of item 261, wherein the cytokines are Interleukin 12 (IL-12) and Interleukin 21 (IL-21).
263. A pharmaceutical composition, comprising the immunostimulatory bacterium of any of items 25-243, or the delivery vehicle of any of items 244-251, or the cell of any of items 252-262, in a pharmaceutically acceptable vehicle.
264. A pharmaceutical composition, comprising the immunostimulatory bacterium of item 84 in a pharmaceutically acceptable vehicle.
265. The pharmaceutical composition of item 263 or item 264 that is formulated for administration without dilution.
266. The pharmaceutical composition of any of items 263-265 that is formulated for systemic administration.
267. The pharmaceutical composition of any of items 263-266 that is formulated for parenteral administration.
268. The pharmaceutical composition of item 267 that is formulated for intravenous administration.
269. The pharmaceutical composition of item 267 that is formulated for intratumoral administration.
270. The pharmaceutical composition of item 267 that is formulated for intraperitoneal administration.
271. The pharmaceutical composition of item 266 that is formulated for oral administration.
272. A method of treatment of cancer that comprises a solid tumor or a hematological malignancy in a subject, comprising administering the immunostimulatory bacterium of any of items 26-243, or the delivery vehicle of any of items 244-251, or the cell of any of items 252-262, or the pharmaceutical composition of any of items 263-271.
273. Use of the immunostimulatory bacterium of any of items 26-244, or the delivery vehicle of any of items 244-251, or the cell of any of items 252-262, or the pharmaceutical composition of any of items 263-271, for the treatment of a cancer that comprises a solid tumor or a hematological malignancy in a subject.
274. The immunostimulatory bacterium of any of items 26-244, or the delivery vehicle of any of items 244-251, or the cell of any of items 252-262, or the pharmaceutical composition of any of items 263-271, for use for the treatment of a cancer that comprises a solid tumor or a hematological malignancy in a subject.
275. The method, use, immunostimulatory bacterium, delivery vehicle, cell, or pharmaceutical composition of any of items 272-274, wherein the subject is a human.
276. The method, use, immunostimulatory bacterium, delivery vehicle, cell, or pharmaceutical composition of any of items 272-275, wherein the cancer comprises a solid tumor.
277. The method, use, immunostimulatory bacterium, delivery vehicle, cell, or pharmaceutical composition of any of items 272-275, wherein the cancer comprises a hematological malignancy.
278. The method, use, immunostimulatory bacterium, delivery vehicle, cell, or pharmaceutical composition of any of items 272-277, wherein the treatment comprises combination therapy in which a second anti-cancer agent or treatment is administered.
279. The method, use, immunostimulatory bacterium, delivery vehicle, cell, or pharmaceutical composition of item 278, wherein the second anti-cancer agent or treatment is administered before, concomitantly with, after, or intermittently with, the immunostimulatory bacterium, delivery vehicle, cell, or pharmaceutical composition.
280. The method, use, immunostimulatory bacterium, delivery vehicle, cell, or pharmaceutical composition of item 278 or item 279, wherein the second anti-cancer agent or treatment is an immunotherapy.
281. The method, use, immunostimulatory bacterium, delivery vehicle, cell, or pharmaceutical composition of any of items 272-280, wherein administration of the immunostimulatory bacterium, delivery vehicle, cell, or pharmaceutical composition is parenteral.
282. The method, use, immunostimulatory bacterium, delivery vehicle, cell, or pharmaceutical composition of any of items 272-280, wherein administration of the immunostimulatory bacterium, delivery vehicle, cell, or pharmaceutical composition is oral, or rectal, or by aerosol into the lung, or intratumoral.
283. The method, use, immunostimulatory bacterium, delivery vehicle, cell, or pharmaceutical composition of any of items 272-280, wherein administration of the immunostimulatory bacterium, delivery vehicle, cell, or pharmaceutical composition is intravenously, intramuscularly, or subcutaneously.
284. The method, use, immunostimulatory bacterium, delivery vehicle, cell, or pharmaceutical composition of any of items 272-283, wherein the cancer is selected from among leukemia; lymphoma; gastric cancer; and cancer of the breast, heart, lung, small intestine, colon, spleen, kidney, bladder, head and neck, colorectum, ovary, prostate, brain, pancreas, skin, bone, bone marrow, blood, thymus, uterus, testicles, cervix, and liver.
285. The method, use, immunostimulatory bacterium, delivery vehicle, cell, or pharmaceutical composition of item 280, wherein the immunotherapy comprises administration of an anti-PD-1, or anti-PD-L1, or anti-CTLA-4 antibody, or antigen-binding portions or forms thereof.
286. The method, use, immunostimulatory bacterium, delivery vehicle, cell, or pharmaceutical composition of any of items 272-285, wherein the immunostimulatory bacterium is a *Salmonella, Shigella, Listeria,* or *E*. *coli* species.
287. The method, use, immunostimulatory bacterium, delivery vehicle, cell, or pharmaceutical composition of any of items 272-286, wherein the immunostimulatory bacterium is a *Salmonella* species.
288. The method, use, immunostimulatory bacterium, delivery vehicle, cell, or pharmaceutical composition of any of items 272-287, wherein the immunostimulatory bacterium is a *Salmonella typhimurium* strain.
289. The method, use, immunostimulatory bacterium, delivery vehicle, cell, or pharmaceutical composition of any of items 272-288, wherein administration of the immunostimulatory bacterium is by intraperitoneal or intratumoral administration.
290. The method, use, immunostimulatory bacterium, delivery vehicle, cell, or pharmaceutical composition of any of items 272-289, wherein the subject has metastatic cancer.
291. The method, use, immunostimulatory bacterium, delivery vehicle, cell, or pharmaceutical composition of any of items 272-290, comprising administering a second anti-cancer treatment, wherein the second treatment is selected from among anti-PD-1, anti-CTLA-4, anti-PD-L1, anti-IL-6, anti-Siglec-15, anti-VEGF, anti-CD73, and anti-CD38 antibodies, or antigen-binding portions or forms thereof.
292. The method, use, immunostimulatory bacterium, delivery vehicle, cell, or pharmaceutical composition of any of items 272-291, comprising administering a second or further anti-cancer treatment, wherein the second or further treatment is selected from among a poly (ADP-ribose) polymerase (PARP) inhibitor, a histone deacetylase (HDAC) inhibitor, a chemotherapy agent, an anti-EGFR antibody, a CAR-T cell, an anti-Her2 antibody, an anti-mesothelin antibody, and an anti-B-cell maturation antigen (BCMA) antibody.
293. The method, use, immunostimulatory bacterium, delivery vehicle, cell, or pharmaceutical composition of any of items 272-292, wherein the immunostimulatory bacterium is *ansB⁻, asd⁻, csgD⁻, purI⁻, msbB⁻,* flagellin- (*fliC⁻*/*fljB⁻* ), and*pagP⁻.*
294. A method of selecting a subject for treatment with the immunostimulatory bacterium of any of items 26-244, or the delivery vehicle of any of items 244-251, or the cell of any of items 252-262, or the pharmaceutical composition of any of items 263-271, comprising:
   obtaining a biological sample from the subject; and
   detecting one or more biomarkers that indicate an immune-excluded or immune-desert tumor phenotype.
295. The method of item 294, wherein the biomarker is selected from among:
   a test indicative of T-cell or tumor-infiltrating lymphocyte (TIL) infiltration into the tumor microenvironment;
   a biomarker that measures the restriction of T-cells or TILs to the invasive margin of the tumor and tumor core;
   the level of TILs;
   an adenosine signature (Nanostring) indicative of CXCL1, CXCL2, CXCL3, CXCL5, CXCL8, THBS1, IL-6, CSF-3, IL-1beta, CCL2, CCL3, or CCL7;
   a myeloid signature (Nanostring) indicative of CXXCL1, CXCL2, CXCL3, CXCL8, IL-6, or PTGS2; and
   levels of one or more of CD3, CD8, CD73, CD39, TNAP (tissue-nonspecific alkaline phosphatase), CD38, CD45, CD68, PD-L1, and FoxP3.
296. A method of monitoring therapy with the immunostimulatory bacterium of any of items 26-244, or the delivery vehicle of any of items 244-251, or the cell of any of items 252-262, or the pharmaceutical composition of any of items 263-271, comprising:
   obtaining a biological sample from a subject, and detecting a change in the level of a biomarker, wherein:
   an increase in a biomarker indicative of an anti-cancer phenotype indicates that treatment is effective; and
   the biomarkers are any that indicate a cytokine response, activation of Type I Interferon, or activation of Type II Interferon.
297. The method of item 296, wherein:
   the biomarker is selected from among the level of one or more of CXCL10/IP-10, CXCL9, Interferon-alpha, Interferon-beta, the pro-inflammatory serum cytokines IL-6, TNF-α, MCP-1, or CCL2, and IL-18 binding protein; and
   an increase in the level of one or more of the biomarkers indicates that treatment is effective.
298. The method of any of items 294-297, wherein the biological sample is a tumor biopsy, or a sample of a body fluid.
299. The method of any of items 294-298, further comprising administering the immunostimulatory bacterium of any of items 26-244, or the delivery vehicle of any of items 244-251, or the cell of any of items 252-262, or the pharmaceutical composition of any of items 263-271.
300. A modified Stimulator of Interferon Genes (STING) protein from a non-human species, where the non-human STING is one that has lower NF-κB signaling activity compared to human STING, and, optionally, higher type I interferon (IFN) pathway signaling activity compared to human STING, wherein:
   the non-human STING protein is modified to include a mutation or mutations so that it has increased activity or acts constitutively in the absence of cytosolic nucleic acids;
   the mutations are insertions, deletions, and/or replacements of amino acids; and
   the STING protein optionally has a deletion or disruption of the TRAF6 binding site.
301. A modified Stimulator of Interferon Genes (STING) protein from a non-human species, or a chimeric human STING protein and modifications thereof, comprising one or more mutation(s) associated with gain-of-function (GOF) that result in the constitutive activation of the encoded STING protein and/or enhanced sensitivity, or increased affinity or binding to endogenous ligands, whereby the STING protein is modified by one or more of an insertion, deletion, and replacement of an amino acid or amino acids;
   the STING protein has IFN-beta signaling activity, and attenuated nuclear factor kappa-light-chain-enhancer of activated B cell (NF-κB) signaling activity, compared to human STING; and
   the mutation or mutations result in increased STING activity or constitutive activity in inducing IFN-beta production.
302. The modified STING protein of item 300 or item 301, wherein the human STING protein comprises the sequence set forth in any of SEQ ID NOs:305-309, or is a human allelic variant thereof with at least 98% sequence identity to the sequence of amino acids set forth in any of SEQ ID NOs:305-309.
303. The modified STING protein of any of items 300-302, wherein:
   the STING protein is a chimera comprising replacement of a C-terminal tail (CTT) region in a STING protein from a first species, with the CTT of a STING protein from a second species;
   the STING protein of the second species has lower NF-κB signaling activity than the NF-κB signaling activity of human STING; and
   the TRAF6 binding site in the CTT optionally is deleted.
304. The modified STING protein of any of items 300-303, wherein the mutation or mutations is/are any that correspond to those associated with the auto-inflammatory disease STING-associated vasculopathy (SAVI).
305. A modified Stimulator of Interferon Genes (STING) protein that is a chimera, comprising replacement of the CTT (C-terminal tail) region in a STING protein from a first species, with the CTT of a STING protein from a second species, wherein:
   the STING protein of the second species has lower NF-κB signaling activity than the NF-κB signaling activity of human STING; and
   the TRAF6 binding site in the CTT optionally is deleted.
306. The modified STING protein of any of items 303-305, wherein the human STING protein comprises the sequence set forth in any of SEQ ID NOs:305-309, or is a human allelic variant thereof with at least 98% sequence identity to the sequence of amino acids set forth in any of SEQ ID NOs:305-309.
307. The modified STING protein of any of items 303-306, wherein the first species is human, and the second species is selected from among Tasmanian devil, marmoset, cattle, cat, ostrich, boar, bat, manatee, crested ibis, coelacanth, mouse, and ghost shark.
308. The modified STING protein of any of items 300-307, wherein the type I IFN signaling activity is at least or at least about 30% that of a wild type human STING protein.
309. The modified STING protein of any of items 300-308, wherein the NF-κB signaling activity is less than 30%, less than 20%, less than 15%, less than 10%, or less than 5% that of wild type human STING NF-κB signaling activity.
310. The modified STING protein of any of items 300-309, wherein the non-human species or second species is selected from among Tasmanian devil, marmoset, cattle, cat, ostrich, boar, bat, manatee, crested ibis, coelacanth, mouse, and ghost shark.
311. The modified STING protein of any of items 300-310, wherein the modification of STING is a mutation or mutations that correspond, by reference to and alignment with human STING, to a mutation that occurs in an interferonopathy, wherein the sequence of human STING with which alignment is effected is set forth in any of SEQ ID NOs:305-309.
312. The modified STING protein of any of items 300-311 that comprises replacement of the C-terminal tail (CTT) with the CTT from a STING protein that has reduced NF-κB signaling activity compared to the NF-κB signaling activity of human STING.
313. The modified STING protein of item 312, wherein the replacing CTT is from a Tasmanian devil, marmoset, cattle, cat, ostrich, boar, bat, manatee, crested ibis, coelacanth, mouse, or ghost shark STING protein.
314. The modified STING protein of item 312, wherein the replacing CTT is from a Tasmanian devil, marmoset, cattle, cat, ostrich, boar, bat, manatee, crested ibis, coelacanth, mouse, or ghost shark STING protein, and it replaces the human STING CTT.
315. The modified STING protein of item 313 or item 314, wherein the replacing CTT is selected from among the following species, and has a sequence: Tasmanian devil RQEEFAIGPKRAMTVTTSSTLSQEPQLLISGMEQPLSLRTDGF SEQ ID NO:371,
   Marmoset EEEEVTVGSLKTSEVPSTSTMSQEPELLISGMEKPLPLRSDLF SEQ ID NO:372,
   Cow EREVTMGSTETSVMPGSSVLSQEPELLISGLEKPLPLRSDVF SEQ ID NO:373,
   Cat EREVTVGSVGTSMVRNPSVLSQEPNLLISGMEQPLPLRTDVF SEQ ID NO:374,
   Ostrich RQEEYTVCDGTLCSTDLSLQISESDLPQPLRSDCL SEQ ID NO:375,
   Boar EREVTMGSAETSVVPTSSTLSQEPELLISGMEQPLPLRSDIF SEQ ID NO:376,
   Bat EKEEVTVGTVGTYEAPGSSTLHQEPELLISGMDQPLPLRTDIF SEQ ID NO:377,
   Manatee EREEVTVGSVGTSVVPSPSSPSTSSLSQEPKLLISGMEQPLPLRTDVF SEQ ID NO:378,
   Crested ibis CHEEYTVYEGNQPHNPSTTLHSTELNLQISESDLPQPLRSDCF SEQ ID NO:379,
   Coelacanth
      (variant 1) QKEEYFMSEQTQPNSSSTSCLSTEPQLMISDTDAPHTLKRQVC SEQ ID NO:380,
   Coelacanth
      (variant 2) QKEEYFMSEQTQPNSSSTSCLSTEPQLMISDTDAPHTLKSGF SEQ ID NO:381,
   Ghost shark LTEYPVAEPSNANETDCMSSEPHLMISDDPKPLRSYCP SEQ ID NO:383, and
   Mouse EKEEVTMNAPMTSVAPPPSVLSQEPRLLISGMDQPLPLRTDLI SEQ ID NO:384,
   or allelic variants of each of these sequences, having at least 98% sequence identity thereto.
316. The modified STING protein of item 314 or item 315, wherein the human CTT comprises the sequence EKEEVTVGSLKTSA VPSTSTMSQEPELLISGMEKPLPLRTDFS (SEQ ID NO:370), or is an allelic variant having at least 98% sequence identity thereto.
317. The modified STING protein of any of items 305-316, wherein the modified STING protein is a chimera in which the human STING CTT is replaced with a CTT from the Tasmanian devil STING.
318. The modified STING protein of item 317, wherein the C-terminal tail (CTT) from the Tasmanian devil STING comprises the sequence: RQEEFAIGPKRAMTVTTSSTLSQEPQLLISGMEQPLSLRTDGF (SEQ ID NO:371), or is an allelic variant having at least 98% sequence identity thereto.
319. The modified STING protein of any of items 300-318, comprising a deletion or disruption of the TRAF6 binding site.
320. The modified STING protein of item 319, wherein the STING protein is a human STING protein, and the TRAF6 binding site comprises the amino acid residues DFS at the C-terminus.
321. The modified STING protein of any of items 300-320, comprising a modification that increases type I interferon signaling activity, or that renders the activity constitutive in the absence of cytosolic nucleic acids.
322. The modified STING protein of item 321, wherein the modification corresponds, by reference to and alignment with human STING, to a mutation that occurs in an interferonopathy, wherein the human STING protein has the sequence set forth in any of SEQ ID NOs:305-309.
323. The modified STING protein of any of items 300-322, wherein the modification is one or more amino acid replacements that correspond(s) to one or more of S102P, V147L, V147M, N154S, V155M, G166E, C206Y, G207E, S102P/F279L, F279L, R281Q, R284G, R284S, R284M, R284K, R284T, R197A, D205A, R310A, R293A, T294A, E296A, R197A/D205A, S272A/Q273A, R310A/E316A, E316A, E316N, E316Q, S272A, R293A/T294A/E296A, D231A, R232A, K236A, Q273A, S358A/E360A/S366A, D231A/R232A/K236A/R238A, S358A, E360A, S366A, R238A, R375A, and S324A/S326A, with reference, for alignment, to the sequence of human STING, as set forth in any of SEQ ID NOs:305-309.
324. The modified STING protein of item 323 that comprises a replacement corresponding to C206Y or R284G, with reference, for alignment, to the sequence of human STING, as set forth in any of SEQ ID NOs:305-309.
325. The modified STING protein of item 324 that comprises replacements corresponding to N154S/R284G
326. The modified STING protein of item 300 that is a Tasmanian devil, marmoset, cattle, cat, ostrich, boar, bat, manatee, crested ibis, coelacanth, mouse, or ghost shark STING protein.
327. A delivery vehicle, comprising nucleic acids encoding the modified STING protein of any of items 300-326.
328. A delivery vehicle, comprising nucleic acids encoding a STING protein from a non-human species, wherein the STING protein has type I IFN signaling activity, and attenuated NF-κB signaling activity compared to the NF-xB signaling activity of human STING.
329. The delivery vehicle of item 328, wherein the human STING protein comprises the sequence set forth in any of SEQ ID NOs:305-309, or is a human allelic variant thereof with at least 98% sequence identity to the sequence of amino acids set forth in any of SEQ ID NOs:305-309.
330. The delivery vehicle of item 328 or item 329, wherein the type I IFN signaling activity is at least or at least about 30% of the type I IFN signaling activity of wild-type human STING.
331. The delivery vehicle of any of items 327-330, wherein the NF-xB signaling activity of the non-human STING protein is less than 30%, less than 20%, less than 15%, less than 10%, or less than 5% of the NF-κB signaling activity of human STING.
332. The delivery vehicle of any of items 327-331, wherein the non-human species is Tasmanian devil, marmoset, cattle, cat, ostrich, boar, bat, manatee, crested ibis, coelacanth, mouse, or ghost shark.
333. The delivery vehicle of any of items 327-332 that is a cell, an exosome, an oncolytic virus or viral vector, a liposome or other lipid-based vehicle, or an immunostimulatory bacterium.
334. The delivery vehicle of item 333, wherein the delivery vehicle is a cell that is a stem cell or an immune cell.
335. The delivery vehicle of item 333, wherein the delivery vehicle is the immunostimulatory bacterium of any of items 26-243.
336. The delivery vehicle of item 335, wherein the immunostimulatory bacterium is *ansB⁻, asd⁻, csgD⁻, purI⁻, msbB⁻,* flagellin⁻ (*fliC⁻*/*fljB⁻*), and *pagP⁻.*
337. The delivery vehicle of item 335, wherein the immunostimulatory bacterium is *ansB⁻, asd⁻, csgD⁻, purI⁻, msbB⁻,* flagellin⁻ (*fliC⁻*/*fljB⁻*), *pagP⁻, lppA⁻* and *lppB⁻.*
338. An immunostimulatory bacterium, comprising a plasmid encoding the modified STING protein of any of claims 300-326, or encoding a STING protein from a non-human species, wherein the encoded STING protein has type I IFN signaling activity, and attenuated NF-κB signaling activity compared to the NF-κB signaling activity of human STING.
339. The immunostimulatory bacterium of item 338, that is *ansB⁻, asd⁻, csgD⁻, purI⁻, msbB⁻,* flagellin⁻, wherein the wild-type bacterium has flagella, and *pagP⁻*
340. The immunostimulatory bacterium of item 338, that is *ansB⁻, asd⁻, csgD⁻, purI⁻, msbB⁻,* flagellin⁻ (*fliC⁻*/*fljB⁻*), wherein the wild-type bacterium has flagella, *pagP⁻, lppA⁻,* and *lppB⁻.*
341. The immunostimulatory bacterium of any of items 338-340, wherein the non-human species is selected from among Tasmanian devil, marmoset, cattle, cat, ostrich, boar, bat, manatee, crested ibis, coelacanth, mouse, and ghost shark.
342. The immunostimulatory bacterium of any of items 338-341, or the delivery vehicle of any of claims 295-297, wherein the immunostimulatory bacterium is a Gram-negative bacterium.
343. The immunostimulatory bacterium of any of items 338-342, or the delivery vehicle of any of claims 295-297, wherein the immunostimulatory bacterium is a strain of *Salmonella, Shigella, E. coli, Bifidobacteriae, Rickettsia, Vibrio, Listeria, Klebsiella, Bordetella, Neisseria, Aeromonas, Francisella, Cholera, Corynebacterium, Citrobacter, Chlamydia, Haemophilus, Brucella, Mycobacterium, Mycoplasma, Legionella, Rhodococcus, Pseudomonas, Helicobacter, Bacillus,* or *Erysipelothrix,* or an attenuated strain thereof, or a modified strain thereof of any of the preceding list of bacterial strains.
344. The immunostimulatory bacterium of any of items 338-342, or the delivery vehicle of any of ,items ; 327-337, wherein the immunostimulatory bacterium is *Rickettsia rickettsiae, Rickettsia prowazekii, Rickettsia tsutsugamuchi, Rickettsia mooseri, Rickettsia sibirica, Bordetella bronchiseptica, Neisseria meningitidis, Neisseria gonorrhoeae, Aeromonas eucrenophila, Aeromonas salmonicida, Francisella tularensis, Corynebacterium pseudotuberculosis, Citrobacter freundii, Chlamydia pneumoniae, Haemophilus somnus, Brucella abortus, Mycobacterium intracellulare, Legionella pneumophila, Rhodococcus equi, Pseudomonas aeruginosa, Helicobacter mustelae, Vibrio cholerae, Bacillus subtilis, Erysipelothrix rhusiopathiae, Yersinia enterocolitica, Rochalimaea quintana,* or *Agrobacterium tumerfacium,* or an attenuated strain thereof, or a modified strain thereof of any of the preceding list of bacterial strains.
345. The immunostimulatory bacterium of any of items 338-342, or the delivery vehicle of any of items 327-337 wherein the immunostimulatory bacterium is an attenuated bacterium.
346. The immunostimulatory bacterium of any of items 338-342, or the delivery vehicle of any of items 327-337, wherein the bacterium is a strain of *Salmonella.*
347. The immunostimulatory bacterium or delivery vehicle of item 346, wherein the bacterium is a *Salmonella typhimurium* strain.
348. The immunostimulatory bacterium or delivery vehicle of item 346 or item 347, wherein the unmodified *Salmonella* is a wild-type strain.
349. The immunostimulatory bacterium or delivery vehicle of item 346 or item 347, wherein the unmodified *Salmonella* strain is attenuated.
350. The immunostimulatory bacterium or delivery vehicle of any of items 327-349, wherein the immunostimulatory bacterium is derived from strain AST-100 (VNP20009 or YS1646), or strain ATCC 14028, or a strain having all of the identifying characteristics of strain ATCC 14028.
351. A pharmaceutical composition, comprising the modified STING protein of any of items 300-326, or the delivery vehicle of any of items 327-337, or the immunostimulatory bacterium of any of items 338-350, in a pharmaceutically acceptable vehicle.
352. An isolated cell, comprising the modified STING protein of any of items 300-326, or the delivery vehicle of any of items 327-337, or the immunostimulatory bacterium of any of items 338-350, wherein the cell is not a zygote of a fertilized human egg.
353. The cell of item 352 that is an immune cell, a stem cell, a tumor cell, or a primary cell line.
354. An isolated cell or cultured cells, comprising the immunostimulatory bacterium of any of items 338-350.
355. The cell of item 353 or item 354 that is a hematopoietic cell.
356. The cell of item 355 that is a T-cell.
357. The cell of any of items 352-356 that is produced *ex vivo* by infecting the cell with the immunostimulatory bacterium or delivery vehicle.
358. The cell of item 357 that is a T-cell, or a hematopoietic cell.
359. A method of treatment of cancer, comprising administering the modified STING protein of any of items 300-326, or the delivery vehicle of any of items 327-337, or the immunostimulatory bacterium of any of items 348-350, or the pharmaceutical composition of item 351, or the cell of any of items 352-358, to a subject with a cancer that comprises a solid tumor or is a hematological malignancy.
360. The method of item 359, wherein the cancer comprises a solid tumor.
361. The method of item 359 or item 360, wherein the cancer is metastatic.
362. The method of any of items 359-361, wherein the cancer is selected from among lymphoma; leukemia; gastric cancer; and cancer of the breast, heart, lung, small intestine, colon, spleen, kidney, bladder, head and neck, colorectum, ovary, prostate, brain, pancreas, skin, bone, bone marrow, blood, thymus, uterus, testicles, cervix, and liver.
363. Use of the modified STING protein of any of items 300-326, or the delivery vehicle of any of items 327-337, or the immunostimulatory bacterium of any of items 338-350, or the pharmaceutical composition of item 351, or the cell of any of items 352-358, for the treatment of cancer.
364. The use of item 363, wherein the cancer comprises a solid tumor or a hematological malignancy.
365. The use of item 363 or item 364, wherein the cancer is metastatic.
366. The use of any of items 363-365, wherein the cancer is selected from among lymphoma; leukemia; gastric cancer; and cancer of the breast, heart, lung, small intestine, colon, spleen, kidney, bladder, head and neck, colorectum, ovary, prostate, brain, pancreas, skin, bone, bone marrow, blood, thymus, uterus, testicles, cervix, and liver.
367. The modified STING protein, delivery vehicle, immunostimulatory bacterium, pharmaceutical composition, cell, method, or use of any of items 300-366, wherein the non-human STING protein has the sequence of amino acids set forth in any of SEQ ID NOs: 349, 356, or 359-369, or is an allelic variant of the STING protein of each species, having at least 98% sequence identity to the sequence of amino acids set forth in any of SEQ ID NOs: 349, 356, or 359-369.
368. The modified STING protein, delivery vehicle, immunostimulatory bacterium, pharmaceutical composition, cell, method, or use of any of items 300-366, wherein the non-human STING protein or chimera has the sequence of amino acids set forth in any of SEQ ID NOs: 350-355, 357, or 358.
369. A method of converting an M2 macrophage to an M1 or M1-like phenotype, comprising administering the immunostimulatory bacterium of any of items 26-343 and 338-350 to a subject with a condition, disease, or disorder treated by enhancing an anti-tumor immune response.
370. Use of an a immunostimulatory bacterium of any of items 25-343 and 338-350 or the immunostimulatory bacterium of any of items 25-343 and 338-350 for use to convert an M2 macrophage to an M1 or M1-like phenotype macrophage in a subject with cancer, whereby an anti-tumor immune response is induced or enhanced.
371. The method of item 369 or use or bacterium of item 370, wherein the disease, disorder, or condition is a cancer that comprises a solid tumor.
372. An isolated cell the comprises an immunostimulatory bacterium of any of items 26-243 and 338-350.
373. The cell of item 372 that is an immune cell or a stem cell.
374. The cell of item 373, wherein the cell is a stem cell is not an embryonic stem cell.
375. The cell of item 373 that is a T-cell.
376. The cell of item . 375 that is a CAR-T cell.
377. A method of treatment of cancer, comprising administering the cell of any of items 372-376.
378. The cell of any of items 372-376 for use for the treatment of cancer.
379. Use of the cell of any of items 372-376 for the treatment of cancer.

## Claims

1. An immunostimulatory bacterium, comprising a plasmid encoding one or more therapeutic product(s) under control of a eukaryotic promoter or promoters, wherein the genome of the immunostimulatory bacterium is modified by deletion or disruption of all or of a sufficient portion of a gene or genes, whereby the bacterium does not activate the synthesis of secreted asparaginase or does not express L-asparaginase II so that the bacterium is *ansB⁻.*

2. The immunostimulatory bacterium of claim 1, wherein the genome of the immunostimulatory bacterium is modified by deletion or disruption of all or of a sufficient portion of the gene *ansB,* encoding L-asparaginase II, and by deletion or disruption of all or of a sufficient portion of the gene *csgD,* whereby the bacterium is *ansB⁻* and does not express active L-asparaginase II, and is *csgD⁻* and does not activate the synthesis of curli fimbriae.

3. The immunostimulatory bacterium of claim 1 or claim 2, wherein a therapeutic product contributes to an anti-tumor immune response in a tumor microenvironment.

4. The immunostimulatory bacterium of any of claims 1-3, wherein:
(a) the product is a protein that is part of a cytosolic DNA/RNA sensor pathway; optionally:
the product is Stimulator of Interferon Genes (STING), RIG-I, MDA-5, IRF-3, or IRF-7; or
the product is a modified Stimulator of Interferon Genes (STING) protein that constitutively induces type I interferon (IFN), optionally, the modified STING has lower NF-κB signaling activity compared to human STING; and/or
(b) the product is a variant protein that is part of a cytosolic DNA/RNA sensor pathway that leads to expression of type I interferon (IFN) that is a variant of STING, MDA5, RIG-1, or IRF-3, wherein the variant protein comprises mutations that confer constitutive activity selected from among:
i) in STING, with reference, for alignment, to SEQ ID NOs: 305-309, one or more selected from: S102P, V147L, V147M, N154S, V155M, G166E, C206Y, G207E, S102P/F279L, F279L, R281Q, R284G, R284S, R284M, R284K, R284T, R197A, D205A, R310A, R293A, T294A, E296A, R197A/D205A, S272A/Q273A, R310A/E316A, E316A, E316N, E316Q, S272A, R293A/T294A/E296A, D231A, R232A, K236A, Q273A, S358A/E360A/S366A, D231A/R232A/K236A/R238A, S358A, E360A, S366A, R238A, R375A, N154S/R284G and S324A/S326A;
ii) in MDA5, with reference, for alignment, to SEQ ID NO:310, one or more of: T3311, T331R, A489T, R822Q, G821S, A946T, R337G, D393V, G495R, R720Q, R779H, R779C, L372F, and A452T;
iii) in RIG-I, with reference, for alignment, to SEQ ID NO:311, one or both of E373A and C268F; and
iv) in IRF-3, with reference, for alignment, to SEQ ID NO:312, S396D.

5. The immunostimulatory bacterium of any of claims 1-4, wherein:
the plasmid encodes a plurality of the products as a polycistronic sequence under control of a single promoter;
optionally, the polycistronic sequence comprises a peptide between each open reading frame (ORF) encoding each product; or
optionally the 2A peptide is one or more of T2A, P2A, E2A, or F2A.

6. The immunostimulatory bacterium of any of claims 1-5, wherein:
the encoded products comprise at least two proteins;
one protein is part of a cytosolic DNA/RNA sensor pathway that leads to expression of type I interferon (IFN), comprising one or more modifications, whereby activity of the protein constitutively induces expression of type I interferon (IFN); and
the second protein is different from the first and is a protein(s) that confer(s) or contributes to an anti-tumor immune response in a tumor microenvironment.

7. The immunostimulatory bacterium of any of claims 1-6, wherein:
an encoded product is a modified STING protein; and
the modified STING protein has constitutive activity to induce expression of type I interferon, and lower NF-κB signaling activity compared to unmodified human STING.

8. The immunostimulatory bacterium of any of claims 1-7, wherein a product encoded on the plasmid is a modified STING protein that is selected from among:
a) a modified non-human STING protein, wherein:
the non-human STING protein is one that has lower NF-κB signaling, and, optionally, higher type I interferon activation activity compared to the wild-type (WT) human STING protein; and
the non-human STING protein comprises a mutation, whereby the STING protein constitutively induces type I interferon (IFN);
b) a modified chimeric STING protein in which the C-terminal tail (CTT) region in a STING protein is replaced with the CTT from a STING protein of another species that has lower NF-κB signaling activity than human STING, and the modified chimeric STING protein further comprises a mutation whereby the chimeric STING protein constitutively induces type I IFN;
c) a STING protein that is modified to constitutively induce type I IFN and that comprises a mutation that eliminates a phosphorylation site to thereby reduce NF-κB signaling activity, optionally, wherein the phosphorylation site occurs at residues corresponding to 324-326 with reference to and alignment with the sequence of human STING as set forth in any of SEQ ID NOs: 305-309;
d) the modified STING protein of a), b), or c), wherein the TRAF6 binding site in the CTT of the STING protein is deleted;
e) the chimeric STING protein of b) that comprises a human STING polypeptide with a CTT from Tasmanian devil and an amino acid replacement that results in constitutive expression of type I interferon; optionally the chimeric STING has the sequence of amino acids set forth in SEQ ID NO: 354 or 397, or a sequence having at least 95% sequence identity therewith and including the replacement that renders the STING constitutively active and having lower NF-κB signaling activity compared to unmodified human STING;
f) the chimeric STING protein of b) that comprises a STING from a first species and the CTT from a second species that has lower NF-κB signaling activity than human STING replacing the CTT from the first species, wherein:
the first species is human; and
the replacing CTT is from a Tasmanian devil, marmoset, cattle, cat, ostrich, boar, bat, manatee, crested ibis, coelacanth, or ghost shark STING protein; and
g) the chimeric STING protein of b) that comprises a CTT from a species that has lower NF-κB signaling activity compared to human STING to replace the native CTT, wherein the replacing CTT is selected from among the following species, and has a sequence:
Tasmanian devil RQEEFAIGPKRAMTVTTSSTLSQEPQLLISGMEQPLSLRTDGF SEQ ID NO:371,
Marmoset EEEEVTVGSLKTSEVPSTSTMSQEPELLISGMEKPLPLRSDLF SEQ ID NO:372,
Cow EREVTMGSTETSVMPGSSVLSQEPELLISGLEKPLPLRSDVF SEQ ID NO:373,
Cat EREVTVGSVGTSMVRNPSVLSQEPNLLISGMEQPLPLRTDVF SEQ ID NO:374,
Ostrich RQEEYTVCDGTLCSTDLSLQISESDLPQPLRSDCL SEQ ID NO:375,
Boar EREVTMGSAETSVVPTSSTLSQEPELLISGMEQPLPLRSDIF SEQ ID NO:376,
Bat EKEEVTVGTVGTYEAPGSSTLHQEPELLISGMDQPLPLRTDIF SEQ ID NO:377,
Manatee EREEVTVGSVGTSVVPSPSSPSTSSLSQEPKLLISGMEQPLPLRTDVF SEQ ID NO:378,
Crested ibis CHEEYTVYEGNQPHNPSTTLHSTELNLQISESDLPQPLRSDCF SEQ ID NO:379,
Coelacanth
(variant 1) QKEEYFMSEQTQPNSSSTSCLSTEPQLMISDTDAPHTLKRQVC SEQ ID NO:380,
Coelacanth
(variant 2) QKEEYFMSEQTQPNSSSTSCLSTEPQLMISDTDAPHTLKSGF SEQ ID NO:381, and
Ghost shark LTEYPVAEPSNANETDCMSSEPHLMISDDPKPLRSYCP SEQ ID NO:383,
or variants of each of these sequences, having at least 98% sequence identity thereto.

9. The immunostimulatory bacterium of claim 7 or claim 8, wherein the encoded product is a modified STING that comprises a modification that renders the STING activity constitutive and the modification is selected from:
a) one or more amino acid replacements that correspond(s) to S102P, V147L, V147M, N154S, V155M, G166E, C206Y, G207E, S102P/F279L, F279L, R281Q, R284G, R284S, R284M, R284K, R284T, R197A, D205A, R310A, R293A, T294A, E296A, R197A/D205A, S272A/Q273A, R310A/E316A, E316A, E316N, E316Q, S272A, R293A/T294A/E296A, D231A, R232A, K236A, Q273A, S358A/E360A/S366A, D231A/R232A/K236A/R238A, S358A, E360A, S366A, R238A, R375A, and S324A/S326A, with reference, for alignment, to the sequence of human STING, as set forth in any of SEQ ID NOs:305-309; or
b) a replacement corresponding to C206Y or R284G, with reference, for alignment, to the sequence of human STING, as set forth in any of SEQ ID NOs:305-309; or
c) the replacements corresponding to N154S/R284G with reference, for alignment, to the sequence of human STING, as set forth in any of SEQ ID NOs:305-309.

10. The immunostimulatory bacterium of any of claims 7-9, wherein:
unmodified STING protein has the sequence set forth in any of SEQ ID NOs:305-309 or is variant thereof with at least 98% sequence identity to the sequence of amino acids set forth in any of SEQ ID NOs:305-309; and/or
the non-human STING protein is selected from among Tasmanian devil, marmoset, cattle, cat, ostrich, boar, bat, manatee, crested ibis, coelacanth, and ghost shark STING protein.

11. The immunostimulatory bacterium of any of claims 1-10, wherein at least one product encoded on the plasmid is a cytokine.

12. The immunostimulatory bacterium of any of claims 1-11, wherein at least one product encoded on the plasmid is a protein selected from among:
(a) one or more of: IL-2, IL-7, IL-12p70 (IL-12p40 + IL-12p35), IL-15, IL-2 that has attenuated binding to IL-2Ra, IL-15/IL-15R alpha chain complex, IL-18, IL-21, IL-23, IL-36γ, IL-2 modified so that it does not bind to IL-2Ra, CXCL9, CXCL10, CXCL11, interferon-α, interferon-β, interferon-γ, CCL3, CCL4, CCL5, proteins that are involved in or that effect or potentiate the recruitment and/or persistence of T-cells, CD40, CD40 ligand (CD40L), CD28, OX40, OX40 ligand (OX40L), 4-1BB, 4-1BB ligand (4-1BBL), 4-1BBL that has a cytoplasmic domain deletion or truncation to eliminate immunosuppressive reverse signaling, members of the B7-CD28 family, CD47 antagonists, an anti-IL6 antibody or IL-6 binding decoy receptor, TGF-beta polypeptide antagonists, bi-specific T-cell engager antibodies, and members of the tumor necrosis factor receptor (TNFR) superfamily; and/or
(b) one or more of: IFN-α, IFN-β, GM-CSF, IL-2, IL-7, IL-12, IL-15, IL-18, IL-21, IL-23, IL-12p70 (IL-12p40 + IL-12p35), IL-15/IL-15R alpha chain complex, IL-36 gamma, IL-2 that has attenuated binding to IL-2Ra, IL-2 that is modified so that it does not bind to IL-2Ra, CXCL9, CXCL10 (IP-10), CXCL11, CCL3, CCL4, CCL5, molecules involved in the potential recruitment and/or persistence of T-cells, CD40, CD40 ligand (CD40L), OX40, OX40 ligand (OX40L), 4-1BB, 4-1BB ligand (4-1BBL), 4-1BBL with a deleted cytoplasmic domain (4-1BBLΔcyt) or with a partially deleted cytoplasmic domain, which the cytoplasmic domain is deleted or truncated to eliminate the immunosuppressive reverse signaling, members of the B7-CD28 family, and members of the tumor necrosis factor receptor (TNFR) superfamily.

13. The immunostimulatory bacterium of any of claims 1-12, where an encoded product comprises:
(a) 4-1BBL with a deleted, or partially deleted, cytoplasmic domain, or a partially deleted cytoplasmic domain and optionally including amino acid modifications, whereby the resulting 4-1BBL assumes the proper orientation when expressed in a cell; and/or
(b) 4-1BBL variant with a deleted or partially deleted cytoplasmic domain, or a modified 4-1BBL with a truncated and modified cytoplasmic domain, wherein the sequence of the 4-1BBL is set forth in SEQ ID NO:390, SEQ ID NO:391, and SEQ ID NO:392.

14. The immunostimulatory bacterium of any of claims 1-13, wherein the encoded products comprise a modified STING that is constitutively active and IL-15 or IL-15/IL-15R alpha chain complex.

15. The immunostimulatory bacterium of any of claims 1-14, wherein:
an encoded protein is a modified STING;
the modified STING comprises a mutation or mutations whereby the STING protein is constitutively active; and
the TRAF6 binding site in the CTT of the STING protein optionally is deleted.

16. The immunostimulatory bacterium of any of claims 1-15, wherein the encoded products comprise modified STING that is constitutively active and IL-12p70.

17. The immunostimulatory bacterium of any of claims 1-16, comprising nucleic acid encoding a plurality of therapeutic products as a polycistronic sequence under control of a single promoter, wherein the nucleic acid encodes one of the following combinations of products:
one or more of IL-12, or IL-15, or IL12p70, or IL-15/IL-15R alpha chain complex;
IL-2 and IL-12p70;
IL-2 and IL-21;
a cytokine and a Stimulator of Interferon Genes (STING) pathway agonist;
a cytokine, a STING pathway agonist, and either a costimulatory receptor ligand or an immune checkpoint inhibitor;
a cytokine, a STING pathway agonist, and a TGF-beta polypeptide antagonist;
a cytokine, a STING pathway agonist, a TGF-beta polypeptide antagonist, and either a co-stimulatory receptor ligand or an immune checkpoint inhibitor;
an anti-CTLA-4 antibody, IL-15, and a TGF-beta receptor decoy or polypeptide antagonist;
4-1BBL and IL-15;
4-1BBL, IL-15, and a TGF-beta receptor decoy or polypeptide antagonist;
an anti-CTLA-4 antibody, and IL-12;
an anti-CTLA-4 antibody, IL-12, and a TGF-beta receptor decoy or polypeptide antagonist;
4-1BBL, and IL-12;
4-1BBL, IL-12, and a TGF-beta receptor decoy or polypeptide antagonist;
an anti-CTLA-4 antibody, and a TGF-beta receptor decoy or polypeptide antagonist;
4-1BBL, and a TGF-beta receptor decoy or polypeptide antagonist;
IL-15, and a TGF-beta receptor decoy or polypeptide antagonist;
IL-12, and a TGF-beta receptor decoy or polypeptide antagonist;
IL-12, IL-15, and a TGF-beta receptor decoy or polypeptide antagonist; and
IL-15, IL-21, and a TGF-beta receptor decoy or polypeptide antagonist,
wherein a STING pathway agonist is a product that increases type I interferon expression via activation of the STING pathway.

18. The immunostimulatory bacterium any of claims 1-17, comprising nucleic acid encoding one of the following combinations of products;
an anti-CTLA-4 antibody and a STING polypeptide;
IL-15 and a STING polypeptide;
4-1BBL and a STING polypeptide;
a TGF-beta receptor decoy or antagonist polypeptide, and a STING polypeptide;
IL-12 and a STING polypeptide;
an anti-CTLA-4 antibody, IL-15, and a STING polypeptide;
4-1BBL, IL-15, and a STING polypeptide;
a TGF-beta receptor decoy or antagonist polypeptide, IL-15, and a STING polypeptide;
an anti-CTLA-4 antibody, IL-12, and a STING polypeptide;
4-1BBL, IL-12, and a STING polypeptide;
a TGF-beta receptor decoy or polypeptide antagonist, IL-12, and a STING polypeptide;
an anti-CTLA-4 antibody, IL-15, a TGF-beta receptor decoy or polypeptide antagonist, and a STING polypeptide;
4-1BBL, IL-15, a TGF-beta receptor decoy or polypeptide antagonist, and a STING polypeptide;
an anti-CTLA-4 antibody, IL-12, a TGF-beta receptor decoy or polypeptide antagonist, and a STING polypeptide;
4-1BBL, IL-12, a TGF-beta receptor decoy or polypeptide antagonist, and a STING polypeptide;
an anti-CTLA-4 antibody, IL-12, IL-15, and a STING polypeptide;
4-1BBL, IL-12, IL-15, and a STING polypeptide;
a TGF-beta receptor decoy or polypeptide antagonist, IL-12, IL-15, and a STING polypeptide;
a TGF-beta receptor decoy or polypeptide antagonist, IL-12, IL-15, and a STING polypeptide;
an anti-CTLA-4 antibody, IL-12, IL-15, a TGF-beta receptor decoy or polypeptide antagonist, and a STING polypeptide;
4-1BBL, IL-12, IL-21, a TGF-beta receptor decoy or polypeptide antagonist, and a STING polypeptide;
an anti-CTLA-4 antibody, IL-12, IL-15, and a TGF-beta receptor decoy or polypeptide antagonist;
4-1BBL, IL-12, IL-21, and a TGF-beta receptor decoy or polypeptide antagonist;
IL-12, IL-15, and a STING polypeptide;
IL-15, IL-21, and a STING polypeptide;
IL-12, IL-21, and a STING polypeptide;
an anti-CTLA-4 antibody, IL-15, IL-21, and a STING polypeptide;
an anti-CTLA-4 antibody, IL-12, IL-21, and a STING polypeptide;
4-1BBL, IL-15, IL-21, and a STING polypeptide;
4-1BBL, IL-12, IL-21, and a STING polypeptide;
an anti-CTLA-4 antibody, and IL-15, wherein:
4-1BBL is 4-1BBL with a deleted cytoplasmic domain, 4-1BBL with a modified cytoplasmic domain, 4-1BBL with a truncated cytoplasmic domain, or 4-1BBL with a truncated and modified cytoplasmic domain;
an anti-CTLA-4 antibody is an scFv or an scFv-Fc; and
a STING polypeptide is a variant STING polypeptide, or a chimeric STING polypeptide, or a chimeric STING polypeptide with amino acid replacements; and
the STING polypeptide has constitutive activity.

19. The immunostimulatory bacterium of any of claims 1-18 that encodes a combination of therapeutic products selected from among:
IL-2, IL-12p70, and a STING gain-of-function (GOF) variant;
IL-2, IL-21, and a STING GOF variant;
IL-2, IL-12p70, a STING GOF variant, and 4-1BBL (including 4-1BBLΔcyt), where Δcyt is a deleted cytoplasmic domain;
IL-2, IL-21, a STING GOF variant, and 4-1BBL (including 4-1BBLΔcyt);
IL-15/IL-15Rα, and a STING GOF variant;
IL-15/IL-15Rα, a STING GOF variant, and 4-1BBL (including 4-1BBLΔcyt);
IL-15/IL-15Rα and IL-12p70;
IL-15/IL-15Rα and IL-21;
IL-15/IL-15Rα, IL-12p70, and a STING GOF variant;
IL-15/IL-15Rα, IL-21, and a STING GOF variant;
IL-15/IL-15Rα, IL-12p70, a STING GOF variant, and 4-1BBL (including 4-1BBLΔcyt);
IL-15/IL-15Rα, IL-21, a STING GOF variant, and 4-1BBL (including 4-1BBLΔcyt);
IL-12p70 and IL-21;
IL-12p70, IL-21, and a STING GOF variant;
IL-12p70, IL-21, a STING GOF variant, and 4-1BBL (including 4-1BBLΔcyt);
IL-12p70 and a STING GOF variant;
IL-12p70, a STING GOF variant, and 4-1BBL (including 4-1BBLΔcyt);
IL-12p70 and IL-18;
IL-12p70, IL-18, and a STING GOF variant;
IL-12p70, IL-18, a STING GOF variant, and 4-1BBL (including 4-1BBLΔcyt);
a TGF-β decoy receptor or a TGF-β polypeptide antagonist, IL-2, and IL-12p70;
a TGF-β decoy receptor or a TGF-β polypeptide antagonist, IL-2, and IL-21;
a TGF-β decoy receptor or a TGF-β polypeptide antagonist, IL-2, IL-12p70, and a STING GOF variant;
a TGF-β decoy receptor or a TGF-β polypeptide antagonist, IL-2, IL-21, and a STING GOF variant;
a TGF-β decoy receptor or a TGF-β polypeptide antagonist, IL-2, IL-12p70, a STING GOF variant, and 4-1BBL (including 4-1BBLΔcyt);
a TGF-β decoy receptor or a TGF-β polypeptide antagonist, IL-2, IL-21, a STING GOF variant, and 4-1BBL (including 4-1BBLΔcyt);
a TGF-β decoy receptor or a TGF-β polypeptide antagonist, IL-15/IL-15Rα, and a STING GOF variant;
a TGF-β decoy receptor or a TGF-β polypeptide antagonist, IL-15/IL-15Rα, a STING GOF variant, and 4-1BBL (including 4-1BBLΔcyt);
a TGF-β decoy receptor or a TGF-β polypeptide antagonist, IL-15/IL-15Rα, and IL-12p70;
a TGF-β decoy receptor or a TGF-β polypeptide antagonist, IL-15/IL-15Rα, and IL-21;
a TGF-β decoy receptor or a TGF-β polypeptide antagonist, IL-15/IL-15Rα, IL-12p70, and a STING GOF variant;
a TGF-β decoy receptor or a TGF-β polypeptide antagonist, IL-15/IL-15Rα, IL-21, and a STING GOF variant;
a TGF-β decoy receptor or a TGF-β polypeptide antagonist, IL-15/IL-15Rα, IL-12p70, a STING GOF variant, and 4-1BBL (including 4-1BBLΔcyt);
a TGF-β decoy receptor or a TGF-β polypeptide antagonist, IL-15/IL-15Rα, IL-21, a STING GOF variant, and 4-1BBL (including 4-1BBLΔcyt);
a TGF-β decoy receptor or a TGF-β polypeptide antagonist, IL-12p70, and IL-21;
a TGF-β decoy receptor or a TGF-β polypeptide antagonist, IL-12p70, IL-21, and a STING GOF variant;
a TGF-β decoy receptor or a TGF-β polypeptide antagonist, IL-12p70, IL-21, a STING GOF variant, and 4-1BBL (including 4-1BBLΔcyt);
a TGF-β decoy receptor or a TGF-β polypeptide antagonist, and IL-12p70;
a TGF-β decoy receptor or a TGF-β polypeptide antagonist, IL-12p70, and a STING GOF variant;
a TGF-β decoy receptor or a TGF-β polypeptide antagonist, IL-12p70, a STING GOF variant, and 4-1BBL (including 4-1BBLΔcyt);
a TGF-β decoy receptor or a TGF-β polypeptide antagonist, IL-12p70, and IL-18;
a TGF-β decoy receptor or a TGF-β polypeptide antagonist, IL-12p70, IL-18, and a STING GOF variant;
a TGF-β decoy receptor or a TGF-β polypeptide antagonist, IL-12p70, IL-18, a STING GOF variant, and 4-1BBL (including 4-1BBLΔcyt);
a TGF-β decoy receptor or a TGF-β polypeptide antagonist, and a STING GOF variant;
an anti-CTLA-4 antibody, IL-2, and IL-12p70;
an anti-CTLA-4 antibody, IL-2, and IL-21;
an anti-CTLA-4 antibody, IL-2, IL-12p70, and a STING GOF variant;
an anti-CTLA-4 antibody, IL-2, IL-21, and a STING GOF variant;
an anti-CTLA-4 antibody, IL-2, IL-12p70, a STING GOF variant, and 4-1BBL (including 4-1BBLΔcyt);
an anti-CTLA-4 antibody, IL-2, IL-21, a STING GOF variant, and 4-1BBL (including 4-1BBLΔcyt);
an anti-CTLA-4 antibody, IL-15/IL-15Rα, and a STING GOF variant;
an anti-CTLA-4 antibody, IL-15/IL-15Rα, a STING GOF variant, and 4-1BBL (including 4-1BBLΔcyt);
an anti-CTLA-4 antibody, IL-15/IL-15Rα, and IL-12p70;
an anti-CTLA-4 antibody, IL-15/IL-15Rα, and IL-21;
an anti-CTLA-4 antibody, IL-15/IL-15Rα, IL-12p70, and a STING GOF variant;
an anti-CTLA-4 antibody, IL-15/IL-15Rα, IL-21, and a STING GOF variant;
an anti-CTLA-4 antibody, IL-15/IL-15Rα, IL-12p70, a STING GOF variant, and 4-1BBL (including 4-1BBLΔcyt);
an anti-CTLA-4 antibody, IL-15/IL-15Rα, IL-21, a STING GOF variant, and 4-1BBL (including 4-1BBLΔcyt);
an anti-CTLA-4 antibody, IL-12p70, and IL-21;
an anti-CTLA-4 antibody, IL-12p70, IL-21, and a STING GOF variant;
an anti-CTLA-4 antibody, IL-12p70, IL-21, a STING GOF variant, and 4-1BBL (including 4-1BBLΔcyt);
an anti-CTLA-4 antibody and IL-12p70;
an anti-CTLA-4 antibody, IL-12p70, and a STING GOF variant;
an anti-CTLA-4 antibody, IL-12p70, a STING GOF variant, and 4-1BBL (including 4-1BBLΔcyt);
an anti-CTLA-4 antibody, IL-12p70, and IL-18;
an anti-CTLA-4 antibody, IL-12p70, IL-18, and a STING GOF variant;
an anti-CTLA-4 antibody, IL-12p70, IL-18, a STING GOF variant, and 4-1BBL (including 4-1BBLΔcyt);
an anti-CTLA-4 antibody and a STING GOF variant;
a CD40 agonist, IL-2, and IL-12p70;
a CD40 agonist, IL-2, and IL-21;
a CD40 agonist, IL-2, IL-12p70, and a STING GOF variant;
a CD40 agonist, IL-2, IL-21, and a STING GOF variant;
a CD40 agonist, IL-2, IL-12p70, a STING GOF variant, and 4-1BBL (including 4-1BBLΔcyt);
a CD40 agonist, IL-2, IL-21, a STING GOF variant, and 4-1BBL (including 4-1BBLΔcyt);
a CD40 agonist, IL-15/IL-15Rα, and a STING GOF variant;
a CD40 agonist, IL-15/IL-15Rα, a STING GOF variant, and 4-1BBL (including 4-1BBLΔcyt);
a CD40 agonist, IL-15/IL-15Rα, and IL-12p70;
a CD40 agonist, IL-15/IL-15Rα, and IL-21;
a CD40 agonist, IL-15/IL-15Rα, IL-12p70, and a STING GOF variant;
a CD40 agonist, IL-15/IL-15Rα, IL-21, and a STING GOF variant;
a CD40 agonist, IL-15/IL-15Rα, IL-12p70, a STING GOF variant, and 4-1BBL (including 4-1BBLΔcyt);
a CD40 agonist, IL-15/IL-15Rα, IL-21, a STING GOF variant, and 4-1BBL (including 4-1BBLΔcyt);
a CD40 agonist, IL-12p70, and IL-21;
a CD40 agonist, IL-12p70, IL-21, and a STING GOF variant;
a CD40 agonist, IL-12p70, IL-21, a STING GOF variant, and 4-1BBL (including 4-1BBLΔcyt);
a CD40 agonist and IL-12p70;
a CD40 agonist, IL-12p70, and a STING GOF variant;
a CD40 agonist, IL-12p70, a STING GOF variant, and 4-1BBL (including 4-1BBLΔcyt);
a CD40 agonist, IL-12p70, and IL-18;
a CD40 agonist, IL-12p70, IL-18, and a STING GOF variant;
a CD40 agonist, IL-12p70, IL-18, a STING GOF variant, and 4-1BBL (including 4-1BBLΔcyt); and
a CD40 agonist and a STING GOF variant, wherein:
4-1BBL is 4-1BBL with a deleted cytoplasmic domain (4-1BBLΔcyt), 4-1BBL with a modified cytoplasmic domain, 4-1BBL with a truncated cytoplasmic domain, or 4-1BBL with a truncated and modified cytoplasmic domain;
a STING GOF variant is a variant STING that has constitutive activity; and
an anti-CTLA-4 antibody is an scFv or an scFv-Fc.

20. The immunostimulatory bacterium of any of claims 1-19, comprising:
(a) nucleic acid encoding IL-36γ; and/or
(b) nucleic acid encoding an immune checkpoint inhibitor antibody, or an antigen-binding portion thereof; and/or
(c) nucleic acid encoding a product that is an antibody that is scFv, or that is an scFv-Fc two-chain polypeptide; and/or
(d) nucleic acid encoding an immune checkpoint inhibitor antibody, or an antigen-binding portion thereof, wherein the immune checkpoint is CTLA-4, or PD-1, or PD-L1.

21. The immunostimulatory bacterium of any of claims 1-20, wherein the genome of the bacterium is modified, whereby:
a. the bacterium is flagellin- and lacks flagella, wherein the wild-type bacterium has flagella; or
b. the bacterium comprises genome modifications, whereby the bacterium is *msbB⁻* /*pagP⁻*; or
c. the bacterium is flagellin⁻ and lacks flagella, and is *pagP⁻, ansB⁻,* and *csgD⁻,* wherein the wild-type bacterium has flagella; and/or
d. the bacterium is flagellin⁻ and lacks flagella, and is *msbB⁻, pagP⁻, ansB⁻,* and *csgD⁻,* wherein the wild-type bacterium has flagella; or
e. the bacterium is flagellin⁻ and lacks flagella, and is *asd⁻, csgD⁻, purI⁻, msbB⁻,* and *pagP⁻,* wherein the wild-type bacterium has flagella; or
f. the bacterium is flagellin⁻ and lacks flagella, and is *csgD⁻, purI⁻, msbB⁻,* and *pagP⁻,* wherein the wild-type bacterium has flagella; or
g. the bacterium comprises genome modifications whereby the bacterium is flagellin⁻ and lacks flagella and is *msbB⁻*/*pagP⁻.*

22. The immunostimulatory bacterium of any of claims 1-21, wherein:
the genome of the bacterium is modified to be *asd⁻* and the plasmid encodes aspartate-semialdehyde dehydrogenase (*asd*); and/or
the bacterium is auxotrophic for adenosine.

23. The immunostimulatory bacterium of any of claims 1-22, wherein the immunostimulatory bacterium has modifications of the genome, whereby the bacterial phenotype conferred by the genome is Δ*asd*/Δ*FLG*/Δ*pagP*/Δ*ansB*/Δ*csgD* or Δ*asd*/ΔFLG/Δ*pagP*/Δ*ansB*/Δ*csgD*/*ΔmsbB*/*ΔpurI,* wherein the plasmid optionally encodes aspartate-semialdehyde dehydrogenase (*asd*).

24. The immunostimulatory bacterium of any of claims 1-23, wherein:
the bacterium is a Gram-negative bacterium; or
the bacterium is a strain of *Salmonella, Shigella, E. coli, Bifidobacteriae, Rickettsia, Vibrio, Listeria, Klebsiella, Bordetella, Neisseria, Aeromonas, Francisella, Cholera, Corynebacterium, Citrobacter, Chlamydia, Haemophilus, Brucella, Mycobacterium, Mycoplasma, Legionella, Rhodococcus, Pseudomonas, Helicobacter, Bacillus,* or *Erysipelothrix,* or an attenuated strain thereof or a modified strain thereof of any of the preceding list of bacterial strains; or
the bacterium is *Rickettsia rickettsiae, Rickettsia prowazekii, Rickettsia tsutsugamuchi, Rickettsia mooseri, Rickettsia sibirica, Bordetella bronchiseptica, Neisseria meningitidis, Neisseria gonorrhoeae, Aeromonas eucrenophila, Aeromonas salmonicida, Francisella tularensis, Corynebacterium pseudotuberculosis, Citrobacter freundii, Chlamydia pneumoniae, Haemophilus somnus, Brucella abortus, Mycobacterium intracellulare, Legionella pneumophila, Rhodococcus equi, Pseudomonas aeruginosa, Helicobacter mustelae, Vibrio cholerae, Bacillus subtilis, Erysipelothrix rhusiopathiae, Yersinia enterocolitica, Rochalimaea quintana,* or *Agrobacterium tumefaciens,* or an attenuated strain thereof or a modified strain thereof.

25. The immunostimulatory bacterium of any of claims 1-23, wherein the bacterium is a strain of *Salmonella* or an attenuated strain thereof or a modified strain thereof; optionally
the bacterium is a *Salmonella typhimurium* strain, or an attenuated strain thereof or a modified strain thereof; or
the bacterium is derived from strain YS1646, or strain ATCC 14028, or a strain having all of the identifying characteristics of strain ATCC 14028.

26. The immunostimulatory bacterium of any of claims 1-25 for use for treatment of cancer.

27. A pharmaceutical composition, comprising the immunostimulatory bacterium of any of claims 1-25 for use for treating cancer; optionally wherein the cancer is selected from among leukemia; lymphoma; gastric cancer; and cancer of the breast, heart, lung, small intestine, colon, spleen, kidney, bladder, head and neck, colorectum, ovary, prostate, brain, pancreas, skin, bone, bone marrow, blood, thymus, uterus, testicles, cervix, and liver.
